(19) Europäisches Patentamt
European Patent Office
Office européen des brevets

(11) EP 4 644 381 A1

(12) EUROPEAN PATENT APPLICATION
published in accordance with Art. 153(4) EPC

(43) Date of publication:
**05.11.2025 Bulletin 2025/45**

(21) Application number: **23910562.0**

(22) Date of filing: **26.12.2023**

(51) International Patent Classification (IPC):
***C07D 401/14*** (2006.01) ***C07D 401/04*** (2006.01)
***C07D 413/14*** (2006.01) ***C07D 491/107*** (2006.01)
***C07D 487/10*** (2006.01) ***C07D 498/10*** (2006.01)
***C07D 419/14*** (2006.01) ***C07D 417/14*** (2006.01)
***C07D 409/14*** (2006.01) ***C07D 405/14*** (2006.01)
***A61K 31/45*** (2006.01) ***A61K 31/454*** (2006.01)
***A61K 31/4545*** (2006.01) ***A61K 31/497*** (2006.01)
***A61K 31/538*** (2006.01) ***A61P 35/00*** (2006.01)
***A61P 37/00*** (2006.01) ***A61P 25/00*** (2006.01)
***A61P 29/00*** (2006.01)

(52) Cooperative Patent Classification (CPC):
**A61K 31/45; A61K 31/454; A61K 31/4545; A61K 31/497; A61K 31/538; A61P 25/00; A61P 29/00; A61P 35/00; A61P 37/00; C07D 401/04; C07D 401/14; C07D 405/14; C07D 409/14; C07D 413/14; C07D 417/14;** (Cont.)

(86) International application number:
**PCT/CN2023/141786**

(87) International publication number:
**WO 2024/140637 (04.07.2024 Gazette 2024/27)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **27.12.2022 CN 202211685361**

(71) Applicant: **Gluetacs Therapeutics (Shanghai) Co., Ltd.**
**Shanghai 201306 (CN)**

(72) Inventors:
- **YANG, Xiaobao**
**Shanghai 201306 (CN)**
- **SUN, Renhong**
**Shanghai 201306 (CN)**
- **ZHOU, Yuedong**
**Shanghai 201306 (CN)**
- **ZHAO, Baoyin**
**Shanghai 201306 (CN)**
- **REN, Chaowei**
**Shanghai 201306 (CN)**

(74) Representative: **August Debouzy**
**7, rue de Téhéran**
**75008 Paris (FR)**

# (54) OXOISOINDOLINYL SUBSTITUTED PIPERIDINEDIONE DERIVATIVE AND USE THEREOF

(57) The present disclosure provides a compound of Formula (I) or salts, enantiomers, stereoisomers, solvates, or polymorphs thereof and uses thereof. The present disclosure also provides pharmaceutical compositions comprising, as an active ingredient, the compound of Formula (I) or salts, enantiomers, stereoisomers, solvates, or polymorphs thereof and uses thereof. The series of compounds designed and synthesized in the present disclosure can effectively prevent and/or treat diseases or disorders associated with the cereblon protein.

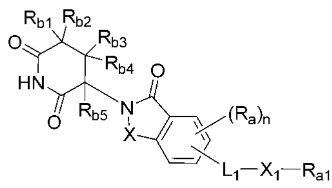

Formula (I)

EP 4 644 381 A1

(52) Cooperative Patent Classification (CPC): (Cont.)
**C07D 419/14; C07D 487/10; C07D 491/107; C07D 498/10**

## Description

## Technical Field

The present disclosure relates to a compound of Formula (I) or salts, enantiomers, stereoisomers, solvates, or polymorphs thereof and uses thereof, especially their use in the prevention and/or treatment of diseases or disorders associated with cereblon protein (CRBN).

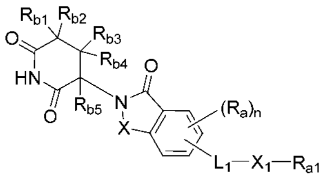

Formula (I)

## Background

Although Thalidomide, lenalidomide and other phthalimide immunomodulatory drugs (IMiDs) have shown significant efficacy in the treatment of multiple myeloma and autoimmune diseases, it was not until 2010 that the E3 ubiquitin ligase cereblon (CRBN) was identified as a direct target of IMiDs. Subsequent research confirmed that these drugs function as molecular glue, inducing the interaction between transcription factors IKZF1/3 and CRBN protein, ultimately leading to their ubiquitination and degradation. Compared with traditional small molecule inhibitors, molecular glue protein degraders have natural mechanism advantages: the former works by occupying the functional domain of the target protein for a long time to inhibit its function, while the protein degraders directly degrade and eliminate the entire target protein, often having a much greater efficacy than traditional small molecule inhibitors. The protein degraders can target "non-drugable" targets and have low requirements for binding force, catalytic capacity and low concentration, which can overcome the clinical drug resistance problem of traditional small molecules. Moreover, molecular glue degraders usually have small molecular weights and desirable druggability, so the research and development of such drugs have received great attention. Based on the CRBN E3 ubiquitin ligase and molecular glue degradation mechanism, a series of compounds have been developed, such as pomalidomide, which has been launched and CC-122 of Bristol-Myers Squibb (BMS), which is undergoing clinical trials, CC-220, CC-90009, CC-99282 and CC-92480, DKY709 from Novartis and CFT7455 from C4 Therapeutics. The degradation substrates of these molecular glues have also expanded from the initially discovered transcription factors IKZF1/3 to include casein kinase 1$\alpha$ (CK1$\alpha$), zinc finger protein 91 (ZFP91) and translation factor GSPT1. The degradation of these protein substrates will enable the molecular glues to exert immune regulatory, anti-inflammatory and anti-tumor pharmaceutical activities. Currently, the number of identified molecular glue candidate compounds is quite limited. Moreover, there is a wide variety of pathogenic proteins that can theoretically serve as degradation substrates for the development of molecular glues. Therefore, it is necessary to design and develop more molecular glues through rationalization and diversification approaches to degrade and eliminate more pathogenic proteins, and to apply them to the treatment of diseases associated with these pathogenic proteins.

All the compounds listed above contain the common fused phenyl-glutarimide skeleton shared by thalidomide-based IMiDs. Avadomide (CC-122), a novel molecular glue developed by Bristol-Myers Squibb (BMS), features distinct structural differences in its core scaffold while demonstrating remarkable pharmacological efficacy through CRBN binding, which induces pronounced degradation of IKZF1 and IKZF3 to downregulate cytokines such as TNF-$\alpha$, thereby exhibiting both potent antitumor and immunomodulatory activities. Currently, Avadomide has progressed to Phase II clinical trials (NCT02406742, NCT02859324, NCT03834623) evaluating its therapeutic potential against various relevant tumors. Building upon Avadomide's quinazolinyl-substituted glutarimide scaffold, the present invention employs rational design strategies to develop novel molecular glue degraders with enhanced efficacy, reduced toxicity, improved stability, and lower production costs to address unmet clinical needs.

## Summary of Invention

In view of the above, the objectives of the present disclosure are to provide novel molecular gluetype protein degraders based on an oxoisoindolinyl-substituted piperidinedione scaffold, their applications and usage methods.

To achieve the above objectives and other related goals, in one aspect, the present disclosure provides a compound of Formula (I):

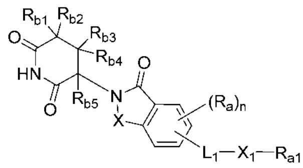

Formula (I)

or salts, stereoisomers (including enantiomers and diastereoisomer), solvates, or polymorphs thereof,

wherein $R_{b1}$, $R_{b2}$, $R_{b3}$, $R_{b4}$ and $R_{b5}$ each independently represent H, D or $C_{1-3}$ alkyl;
X represents C(O) or $CH_2$;
$(R_a)_n$ indicates that the benzene ring in Formula (I) is optionally substituted with n $R_a$, where $R_a$ represents deuterium, halogen, hydroxy, mercapto, nitro, amino, cyano, optionally deuterated $C_{1-6}$ alkyl, optionally deuterated $C_{1-6}$ alkoxy, halogenated $C_{1-6}$ alkoxy or halogenated $C_{1-6}$ alkyl, and n represents an integer of 0, 1, 2 or 3; and
Li represents C(O), alkynylene, alkenylene, optionally substituted $C_{1-5}$ alkylene, -CH= or $N(R_{c1})$, where $R_{c1}$ represents H or $C_{1-3}$ alkyl; or
Li represents Formula $-L_2-L_3-*$, where $L_2$ represents optionally substituted phenylene, $L_3$ represents optionally substituted $C_{1-2}$ alkylene, and symbol * indicates the point of attachment to $X_1$; or
Li represents a bond;
$X_1$ represents optionally substituted cycloalkylene, optionally substituted heterocyclylene, optionally substituted heteroarylene, or $N(R_{c2})$, where $R_{c2}$ represents H or $C_{1-3}$ alkyl; or
$X_1$ represents a bond;
$R_{a1}$ represents the structure of Formula (II):

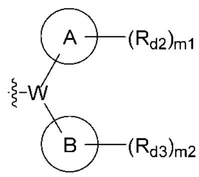

Formula (II)

wherein W represents N or $CR_{d1}$, where $R_{d1}$ represents H, halogen, $C_{1-3}$ alkyl or halogenated $C_{1-3}$ alkyl;

ring A represents aryl, cycloalkyl, heterocyclyl, or heteroaryl, $(R_{d2})_{m1}$ indicates that ring A is optionally substituted with m1 $R_{d2}$, where m1 represents an integer of 0 to 10, and each $R_{d2}$ independently represents deuterium, hydroxy, amino, mercapto, halogen, cyano, optionally deuterated $C_{1-6}$ alkyl, optionally deuterated $C_{3-6}$ cycloalkyl, optionally deuterated $C_{1-6}$ alkoxy, halogenated $C_{1-6}$ alkyl or $-C_{1-6}$ alkylene-$NH_2$; and
ring B represents aryl, cycloalkyl, heterocyclyl, or heteroaryl, and $(Rd_3)_{m2}$ indicates that ring B is optionally substituted with m2 $R_{d3}$, where m2 represents an integer of 0 to 10, and each $R_{d3}$ independently represents deuterium, hydroxy, amino, mercapto, halogen, cyano, optionally deuterated $C_{1-6}$ alkyl, optionally deuterated $C_{3-6}$ cycloalkyl, optionally deuterated $C_{1-6}$ alkoxy, halogenated $C_{1-6}$ alkyl or $-C_{1-6}$ alkylene-$NH_2$; or

$R_{a1}$ represents the structure of Formula (III):

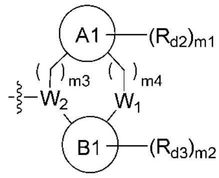

Formula (III)

wherein m3 and m4 each independently represent an integer of 0, 1 or 2, $W_1$ represents O, S or NH, or $W_1$ represents a bond;
$W_2$ represents N or $CR_{d1}$, where $R_{d1}$ represents H, halogen, $C_{1-3}$ alkyl or halogenated $C_{1-3}$ alkyl;

ring A1 represents an aryl ring, a cycloalkyl ring, a heterocyclyl ring or a heteroaryl ring, and $(R_{d2})_{m1}$ indicates that ring A is optionally substituted with m1 $R_{d2}$, where m1 represents an integer of 0 to 10, and each $R_{d2}$ independently represents deuterium, hydroxy, amino, mercapto, halogen, cyano, optionally deuterated $C_{1-6}$ alkyl, optionally deuterated $C_{3-6}$ cycloalkyl, optionally deuterated $C_{1-6}$ alkoxy, halogenated $C_{1-6}$ alkyl, halogenated $C_{1-6}$ alkoxy or -$C_{1-6}$ alkylene-$NH_2$; and

ring B1 represents an aryl ring, a cycloalkyl ring, a heterocyclyl ring or a heteroaryl ring, and $(Rd_3)_{m2}$ indicates that ring B is optionally substituted with m2 $R_{d3}$, where m2 represents an integer of 0 to 10, and each $R_{d3}$ independently represents deuterium, hydroxy, amino, mercapto, halogen, cyano, optionally deuterated $C_{1-6}$ alkyl, optionally deuterated $C_{3-6}$ cycloalkyl, optionally deuterated $C_{1-6}$ alkoxy, halogenated $C_{1-6}$ alkyl, halogenated $C_{1-6}$ alkoxy or -$C_{1-6}$ alkylene-$NH_2$; or

$R_{a1}$ represents the structure of Formula (IV):

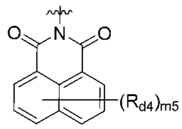

Formula (IV) ;

wherein $(R_{d4})_{m5}$ indicates that the structure of Formula (IV) is optionally substituted with m5 $R_{d4}$, where m5 represents an integer of 0 to 6, and each $R_{d4}$ independently represents hydroxy, amino, mercapto, halogen, cyano, optionally deuterated $C_{1-6}$ alkyl, optionally deuterated $C_{3-6}$ cycloalkyl, optionally deuterated $C_{1-6}$ alkoxy, halogenated $C_{1-6}$ alkyl or -$C_{1-6}$ alkylene-$NH_2$;

with the provisos that:

$L_1$ and $X_1$ are not simultaneously bonds; and
when W represents N and $X_1$ represents $N(R_{c2})$, $L_1$ is not $N(R_{c1})$.

In another aspect, the present disclosure provides a pharmaceutical composition comprising the compound of Formula (I) or pharmaceutically acceptable salts, stereoisomers (including enantiomers, diastereoisomers), solvates, isotopically enriched analogs, prodrugs, or polymorphs thereof, and at least one pharmaceutically acceptable carrier.

In a further aspect, the present disclosure further provides a medicine kit or reagent kit comprising: the compound of Formula (I) or a pharmaceutically acceptable salt thereof, or the pharmaceutical composition comprising the same.

In a further aspect, the present disclosure provides the compound of Formula (I) or pharmaceutically acceptable salts, enantiomers, diastereoisomers, solvates, prodrugs, or polymorphs thereof, for use as a medicament.

In a further aspect, the present disclosure provides the compound of Formula (I) or pharmaceutically acceptable salts, stereoisomers (including enantiomers, diastereoisomers), solvates, isotopically enriched analogs, prodrugs, or polymorphs thereof, or the pharmaceutical composition of the present disclosure for use in the prevention or treatment of diseases or disorders associated with cereblon protein.

In a further aspect, the present disclosure provides use of the compound of Formula (I) or pharmaceutically acceptable salts, stereoisomers (including enantiomers, diastereoisomers), solvates, isotopically enriched analogs, prodrugs, or polymorphs thereof, or the pharmaceutical composition of the present disclosure for the manufacture of a medicament for the prevention or treatment of diseases or disorders associated with cereblon protein.

In a further aspect, the present disclosure provides a method for treating or preventing diseases or disorders associated with cereblon protein, comprising administering to a subject a therapeutically effective amount of the compound of Formula (I) or pharmaceutically acceptable salts, stereoisomers (including enantiomers, diastereoisomers), solvates, isotopically enriched analogs, prodrugs, or polymorphs thereof, or the pharmaceutical composition.

## Description of Drawings

Figure 1 illustrates the results of Western blot analysis.

## Detailed Description of the Invention

The following detailed description is provided as exemplary specific embodiments to assist those skilled in the art in understanding and practicing the present disclosure. It should be appreciated, however, that such description is not

intended to limit the scope of the present disclosure, and that various modifications and changes may be made to the specific embodiments described in the present disclosure without departing from the spirit and scope of the present disclosure. Such changes and modifications are to be understood as being included within the scope of the present invention as defined by the appended claims.

## I. Compounds

### Compounds of Formula (I)

The present disclosure provides a compound of Formula (I) or salts (including pharmaceutically acceptable salts), stereoisomers (including enantiomers, diastereoisomers), solvates, isotopically enriched analogs, prodrugs, or polymorphs thereof:

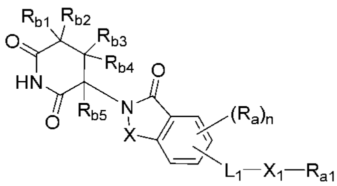

Formula (I)

wherein $R_{b1}$, $R_{b2}$, $R_{b3}$, $R_{b4}$, $R_{b5}$, X, $(R_a)_n$, $L_1$, $X_1$, and $R_{a1}$ are as defined in the compounds of Formula (I) above and its various embodiments herein.

In some embodiments of the present disclosure, $R_{b1}$, $R_{b2}$, $R_{b3}$, $R_{b4}$, and $R_{b5}$ each independently represent H, D or $C_{1-3}$ alkyl (e.g., methyl, ethyl, or propyl).

In some embodiments of the present disclosure, $R_{b5}$ represents H or D.

In some embodiments of the present disclosure, $R_{b1}$, $R_{b2}$, $R_{b3}$, and $R_{b4}$ are the same or different and each independently represent H, D or $C_{1-3}$ alkyl (e.g., methyl, ethyl, or propyl). In some sub-embodiments of the present disclosure, $R_{b1}$, $R_{b2}$, $R_{b3}$, and $R_{b4}$ are the same or different and each independently represent H.

In some embodiments of the present disclosure, $R_{b1}$, $R_{b2}$, $R_{b3}$, $R_{b4}$ and $R_{b5}$ all represent H.

In some embodiments of the present disclosure, X represents C(O).

In some embodiments of the present disclosure, X represents $CH_2$.

In some embodiments of the present disclosure, $(R_a)_n$ indicates that the benzene ring in Formula (I) is optionally substituted with n $R_a$, where $R_a$ represents deuterium, halogen (e.g., fluorine, chlorine, bromine, or iodine), hydroxy, mercapto, nitro, amino, cyano, optionally deuterated $C_{1-6}$ alkyl, optionally deuterated $C_{1-6}$ alkoxy, halogenated $C_{1-6}$ alkoxy or halogenated $C_{1-6}$ alkyl, and n represents an integer of 0, 1, 2 or 3. Optionally, in some sub-embodiments of the present disclosure, n represents an integer of 0, 1 or 2. Optionally, $R_a$ represents deuterium, halogen (e.g., fluorine, chlorine, bromine, or iodine), hydroxy, mercapto, nitro, amino, cyano, optionally deuterated $C_{1-4}$ alkyl, optionally deuterated $C_{1-4}$ alkoxy, halogenated $C_{1-4}$ alkoxy or halogenated $C_{1-4}$ alkyl. Further optionally, $R_a$ represents deuterium, halogen (e.g., fluorine, chlorine, bromine, or iodine), hydroxy, mercapto, nitro, amino, cyano, $C_{1-4}$ alkyl (e.g., methyl, ethyl, propyl or tert-butyl), $C_{1-4}$ alkoxy (e.g., methoxy), halogenated $C_{1-4}$ alkoxy (e.g., trifluoromethoxy) or halogenated $C_{1-4}$ alkyl (e.g., trifluoromethyl).

In embodiments of the present disclosure, the number of substituents is not limited in principle, or is automatically limited by the size of the building units.

In some embodiments of the present disclosure, Li represents C(O), alkynylene, alkenylene, optionally substituted $C_{1-5}$ alkylene, -CH= or $N(R_{c1})$, where $R_{c1}$ represents H or $C_{1-3}$ alkyl.

In some embodiments of the present disclosure, Li represents C(O) or -CH=.

In some embodiments of the present disclosure, Li represents alkynylene. In some sub-embodiments of the present disclosure, exemplary alkynylene groups include, but are not limited to, $C_{2-6}$ alkynylene, $C_{2-5}$ alkynylene, $C_{2-4}$ alkynylene, $C_{2-3}$ alkynylene and ethynylene. Examples of alkynylene include, but are not limited to, ethynylene, propynylene, and butynylene.

In some embodiments of the present disclosure, Li represents alkenylene. In some sub-embodiments of the present disclosure, exemplary alkenylene groups include, but are not limited to, $C_{2-6}$ alkenylene, $C_{2-5}$ alkenylene, $C_{2-4}$ alkenylene, $C_{2-3}$ alkenylene, and vinylene. Examples of alkenylene include, but are not limited to: vinylene, 1-propenylene, allylidene, 1-butenylene, 2-butenylene, 3-butenylene, isobutenylene, pentenylene, n-penta-2,4-dienylene, 1-methyl-but-1-enylene, 2-methyl-but-1-enylene, 3-methyl-but-1-enylene, 1-methyl-but-2-enylene, 2-methyl-but-2-enylene, 3-methyl-but-2-enylene, 1-methyl-but-3-enylene, 2-methyl-but-3-enylene, 3-methyl-but-3-enylene, and hexenylene.

In some embodiments of the present disclosure, Li represents optionally substituted $C_{1-5}$ alkylene. In some sub-embodiments of the present disclosure, exemplary optionally substituted $C_{1-5}$ alkylene groups include, but are not limited to, optionally substituted $C_{1-4}$ alkylene and optionally substituted $C_{1-3}$ alkylene. Examples of $C_{1-5}$ alkylene include, but are not limited to: methylene, ethylene, propylene, isopropylene, butylene, isobutylene, sec-butylene, tert-butylene, n-pentylene, isopentylene, neopentylidene, and tertamylene. $C_{1-5}$ alkylene is optionally substituted with one or more (e.g., 1-5, 1-4, 1-3, 1-2 or 1) substituents selected from the group consisting of: deuterium, hydroxy, amino, mercapto, halogen, cyano, oxo, $C_1$-$C_3$ alkoxy, halogenated $C_1$-$C_3$ alkyl, $C_{3-6}$ cycloalkyl or any combination thereof. In some sub-embodiments of the present disclosure, Li optionally represents methylene, ethylene, propylene, or -CH(OH)-.

In some embodiments of the present disclosure, Li represents $N(R_{c1})$, where $R_{c1}$ represents H or $C_{1-3}$ alkyl. In some sub-embodiments of the present disclosure, Li represents NH, $N(CH_3)$ or $N(CH_2CH_3)$.

In some embodiments of the present disclosure, Li represents a bond.

In some embodiments of the present disclosure, Li represents Formula $-L_2-L_3-*$, where $L_2$ represents optionally substituted phenylene, $L_3$ represents optionally substituted $C_{1-2}$ alkylene, and symbol * indicates the point of attachment to $X_1$. The phenylene is optionally substituted with one or more (e.g., 1-4, 1-3, 1-2 or 1) substituents selected from the group consisting of: hydroxy, amino, mercapto, halogen, cyano, oxo, $C_1$-$C_3$ alkyl, $C_1$-$C_3$ alkoxy, halogenated $C_1$-$C_3$ alkyl, $C_{3-6}$ cycloalkyl or any combination thereof. Examples of $C_{1-2}$ alkylene include, but are not limited to: methylene and ethylene. $C_{1-2}$ alkylene is optionally substituted with one or more (e.g., 1-5, 1-4, 1-3, 1-2 or 1) substituents selected from the group consisting of: deuterium, hydroxy, amino, mercapto, halogen, cyano, oxo, $C_1$-$C_3$ alkoxy, halogenated $C_1$-$C_3$ alkyl, $C_{3-6}$ cycloalkyl and any combination thereof. In some sub-embodiments of the present disclosure, Li optionally represents: -phenylene-methylene-*, where symbol * indicates the point of attachment to $X_1$.

In some embodiments of the present disclosure, $X_1$ represents a bond.

In embodiments of the present disclosure, $L_1$ and $X_1$ are not simultaneously bonds.

In some embodiments of the present disclosure, $X_1$ represents optionally substituted cycloalkylene, optionally substituted heterocyclylene, optionally substituted heteroarylene, or $N(R_{c2})$, where $R_{c2}$ represents H or $C_{1-3}$ alkyl.

In some embodiments of the present disclosure, $X_1$ represents $N(R_{c2})$, where $R_{c2}$ represents H or $C_{1-3}$ alkyl. In some embodiments of the present disclosure, $X_1$ represents NH or $N(CH_3)$.

In some embodiments of the present disclosure, $X_1$ represents optionally substituted cycloalkylene. Exemplary cycloalkylene groups include, but are not limited to, optionally substituted $C_{3-30}$ cycloalkylene, optionally substituted $C_{3-20}$ cycloalkylene and optionally substituted $C_{3-15}$ cycloalkylene. Representative examples of cycloalkylene include, but are not limited to: cyclopropylene, cyclobutylene, cyclopentylene, cyclopentenylene, cyclohexylene, cyclohexenylene, cycloheptylene, cyclooctylene, decalinylene, octahydropentalenylene, octahydro-1H-indenylene, spiro-cycloalkylene (e.g., $C_3$-$C_{15}$ spiro-cycloalkylene, such as spiro[3.3]heptylene, spiro[2.5]octylene, spiro[3.5]nonylene, spiro[4.4]nonylene, spiro[4.5]decylene, and spiro[5.5]undecylene), p-menthanylene, m-menthanylene, and bridged cycloalkylene (e.g., $C_3$-$C_{15}$ bridged cycloalkylene, such as adamantanylene, noradamantanylene, bornylene, bicyclo[2.2.1]heptanylene, 2-oxobicyclo[2.2.1]heptanylene, and bicyclo[2.2.1]heptentylene). The cycloalkylene is optionally substituted with one or more (e.g., 1-10, 1-9, 1-7, 1-6, 1-5, 1-4, 1-3, 1-2 or 1) substituents selected from the group consisting of: D (deuterium), optionally deuterated $C_{1-4}$ alkyl, hydroxy, amino, mercapto, halogen, cyano, oxo, $C_{1-4}$ alkoxy, $C_{1-4}$ alkyl-NH-, halogenated $C_{1-4}$ alkyl, $NH_2$-$C_{1-4}$ alkylene-, $C_{1-4}$ alkyl-NHC(O)-, $C_{1-4}$ alkyl-C(O)NH- and any combination thereof.

In some embodiments of the present disclosure, $X_1$ represents optionally substituted heterocyclylene. Exemplary heterocyclylene groups include, but are not limited to, optionally substituted 4- to 30-membered heterocyclylene, optionally substituted 4- to 20-membered heterocyclylene and optionally substituted 4- to 15-membered heterocyclylene. Representative examples of heterocyclylene include, but are not limited to, azetidinylene, pyrrolidinylene, imidazolidylene, pyrazolidylene, oxazolidinylene, thiazolidinylene, piperidinylene, piperazinylene, tetrahydropyridylene, dihydroxypiperidinylene, difluoropiperidinylene, morpholinylene, thiomorpholinylene, azacycloheptanylene, azacyclooctylene, diazacycloheptanylene, diazacyclooctylene, bridged heterocyclylene (e.g., 4- to 15-membered bridged heterocyclylene, such as 3-azabicyclo[3.1.0]hexylene, 3-azabicyclo[3.1.1]heptanylene, 2-azabicyclo[2.2.1]heptanylene, 6-azabicyclo [3.1.1]heptanylene, 2-azabicyclo[2.2.2]octanylene, 2,5-diazabicyclo[2.2.1]heptanylene, 3,6-diazabicyclo[3.1.1]heptanylene, 3-azabicyclo[3.2.1]octanylene, 3,8-diazabicyclo[3.2.1]octanylene, 2,5-diazabicyclo[2.2.2]octanylene, and quinuclidinylene), spiro-heterocyclylene (e.g., 4- to 15-membered spiro-heterocyclylene, such as 2,6-diazaspiro[3.3]heptanylene, 2,7-diazaspiro[3.5]nonylene, 2,8-diazaspiro[4.5]decylene, 3,9-diazaspiro[5.5]undecylene, 3-azaspiro[5.5]undecylene, and 7-azaspiro[3.5]nonylene), and octahydropyrrolo[3,4-c]pyrrolylene. The heterocyclylene is optionally substituted with one or more (e.g., 1-10, 1-9, 1-7, 1-6, 1-5, 1-4, 1-3, 1-2 or 1) substituents selected from the group consisting of: D (deuterium), optionally deuterated $C_{1-4}$ alkyl, hydroxy, amino, mercapto, halogen, cyano, oxo, $C_{1-4}$ alkoxy, $C_{1-4}$ alkyl-NH-, halogenated $C_{1-4}$ alkyl, $NH_2$-$C_{1-4}$ alkylene-, $C_{1-4}$ alkyl-NHC(O)-, $C_{1-4}$ alkyl-C(O)NH-, and any combination thereof.

In some embodiments of the present disclosure, $X_1$ represents optionally substituted heteroarylene. Exemplary heteroarylene groups include, but are not limited to, optionally substituted 5- to 30-membered heteroarylene, optionally substituted 5- to 20-membered heteroarylene, and optionally substituted 5- to 15-membered heteroarylene. Representative examples of heteroarylene include, but are not limited to, furanylene, oxazolylene, isoxazolylene, oxadiazolylene,

thienylene, thiazolylene, isothiazolylene, thiadiazolylene, pyrrolylene, imidazolylene, pyrazolylene, triazolylene, pyridylene, pyrimidinylene, pyridazinylene, pyrazinylene, indolylene, isoindolylene, benzofuranylene, isobenzofuranylene, benzothienylene, indazolylene, benzimidazolylene, benzoxazolylene, benzisoxazolylene, benzothiazolylene, benzisothiazolylene, benzotriazolylene, benzo[2,1,3]oxadiazolylene, benzo[2,1,3]thiadiazolylene, benzo[1,2,3]thiadiazolylene, quinolinylene, isoquinolinylene, naphthyridinylene, cinnolinylene, quinazolinylene, quinoxalinylene, phthalazinylene, pyrazolo[1,5-a]pyridylene, pyrazolo[1,5-a]pyrimidinylene, imidazo[1,2-a]pyridylene, 1H-pyrrolo[3,2-b]pyridylene, 1H-pyrrolo[2,3-b]pyridylene, 4H-fluoro[3,2-b]pyrrolylene, pyrrolo[2,1-b]thiazolylene, and imidazo[2,1-b]thiazolylene. The heteroarylene is optionally substituted with one or more (e.g., 1-10, 1-9, 1-7, 1-6, 1-5, 1-4, 1-3, 1-2 or 1) substituents selected from the group consisting of: D (deuterium), optionally deuterated $C_{1-4}$ alkyl, hydroxy, amino, mercapto, halogen, cyano, $C_{1-4}$ alkoxy, $C_{1-4}$ alkyl-NH-, halogenated $C_{1-4}$ alkyl, $NH_2$-$C_{1-4}$ alkylene-, $C_{1-4}$ alkyl-NHC(O)-, $C_{1-4}$ alkyl-C(O)NH-, and any combination thereof.

In some embodiments of the present disclosure, $X_1$ represents the following bivalent groups:

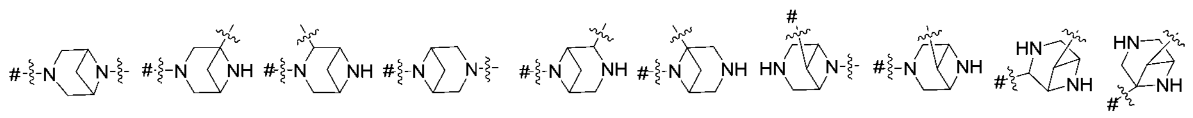

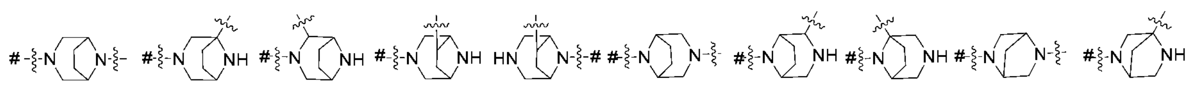

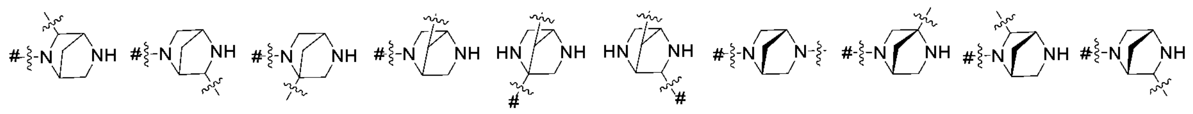

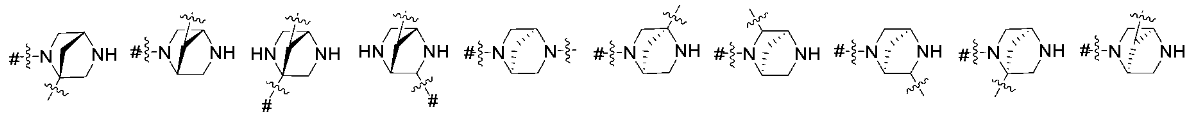

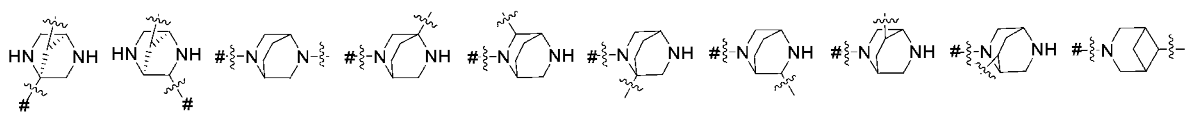

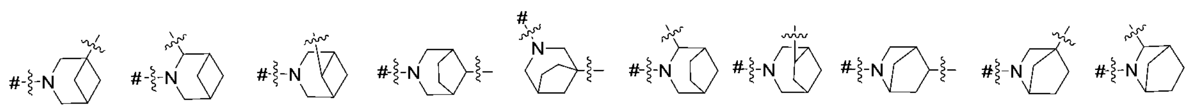

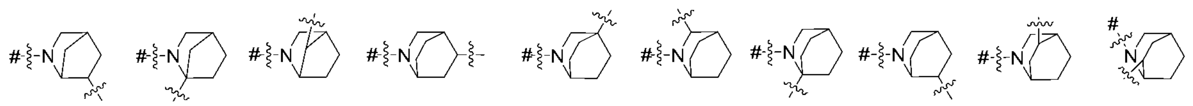

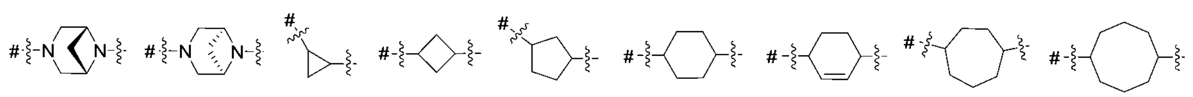

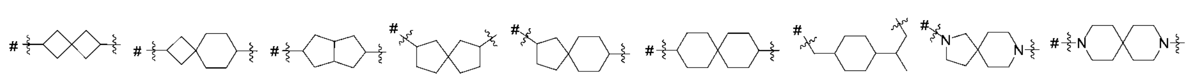

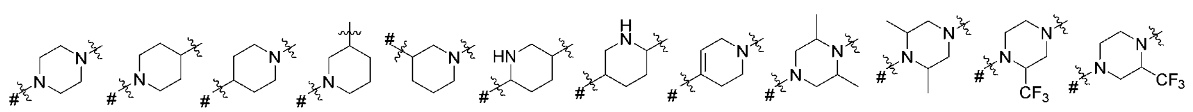

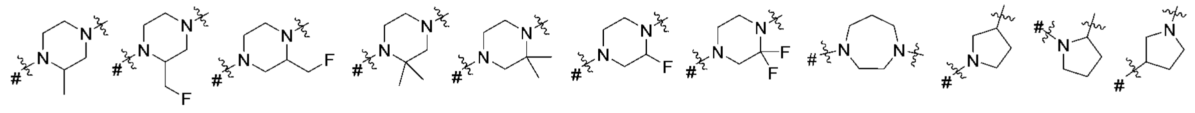
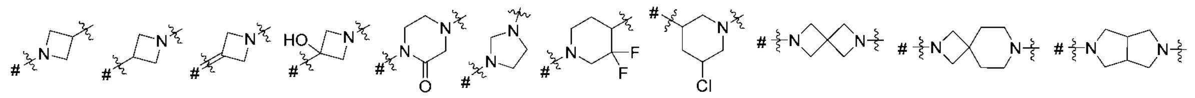
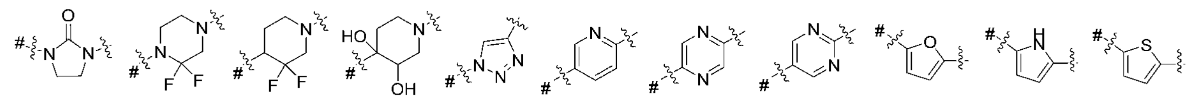
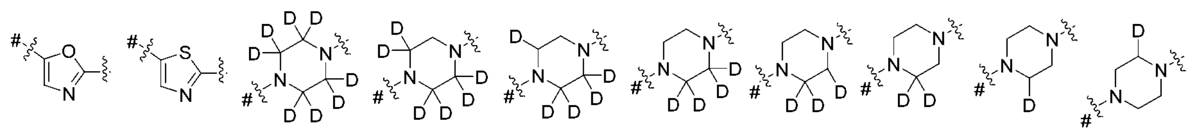
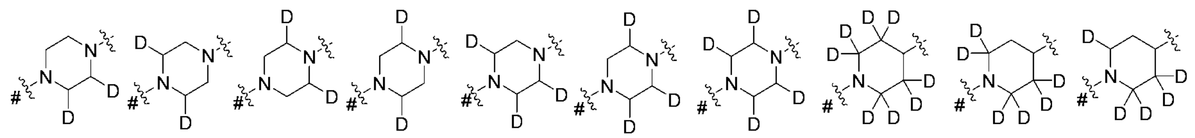
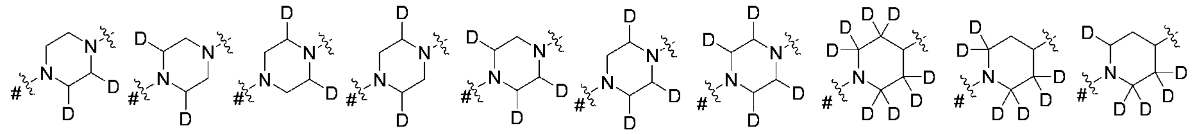
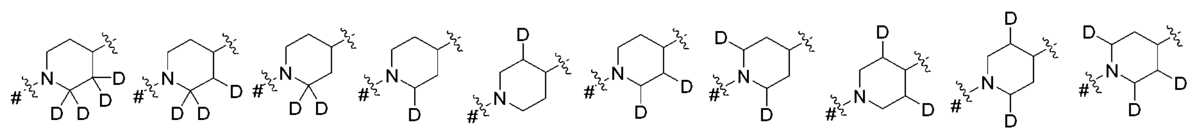
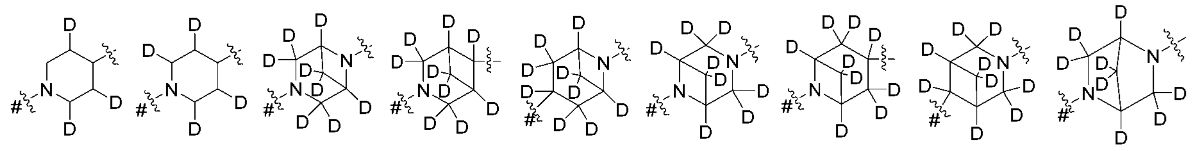
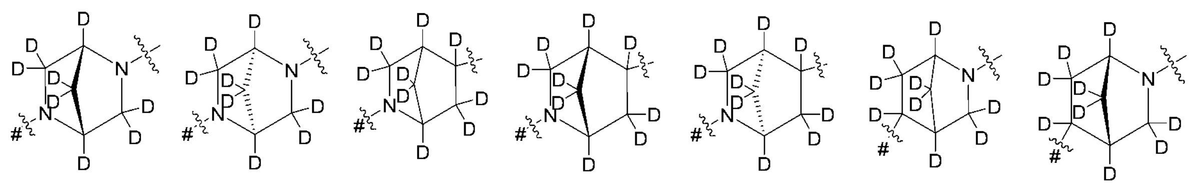
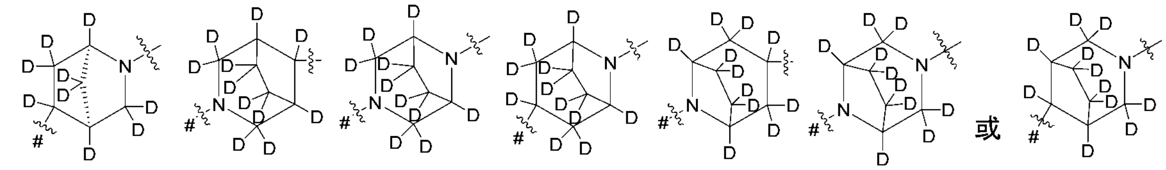

或 ,

wherein symbol # indicates the point of attachment to $L_1$.

In some embodiments of the present disclosure, $R_{a1}$ represents the structure of Formula (II):

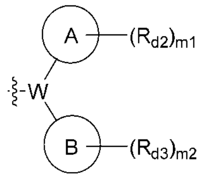

Formula (II)

wherein W represents N or $CR_{d1}$, where $R_{d1}$ represents H, halogen, $C_{1-3}$ alkyl, or halogenated $C_{1-3}$ alkyl;

ring A represents aryl, cycloalkyl, heterocyclyl, or heteroaryl, and $(R_{d2})_{m1}$ indicates that ring A is optionally substituted with m1 $R_{d2}$, where m1 represents an integer of 0 to 10, and each $R_{d2}$ independently represents deuterium, hydroxy, amino, mercapto, halogen, cyano, optionally deuterated $C_{1-6}$ alkyl, optionally deuterated $C_{3-6}$ cycloalkyl, optionally deuterated $C_{1-6}$ alkoxy, halogenated $C_{1-6}$ alkyl, halogenated $C_{1-6}$ alkoxy, or -$C_{1-6}$ alkylene-$NH_2$; and

ring B represents aryl, cycloalkyl, heterocyclyl, or heteroaryl, and $(R_{d3})_{m2}$ indicates that ring B is optionally substituted with m2 $R_{d3}$, where m2 represents an integer of 0 to 10, and each $R_{d3}$ independently represents deuterium, hydroxy, amino, mercapto, halogen, cyano, optionally deuterated $C_{1-6}$ alkyl, optionally deuterated $C_{3-6}$ cycloalkyl, optionally deuterated $C_{1-6}$ alkoxy, halogenated $C_{1-6}$ alkyl, halogenated $C_{1-6}$ alkoxy, or -$C_{1-6}$ alkylene-$NH_2$.

In embodiments of the present disclosure, when W represents N and $X_1$ represents $N(R_{c2})$, $L_1$ is not $N(R_{c1})$.

In some embodiments of the present disclosure, W in the structure of Formula (II) represents N.

In some embodiments of the present disclosure, W in the structure of Formula (II) represents $CR_{d1}$, where $R_{d1}$ represents H, halogen, $C_{1-3}$ alkyl or halogenated $C_{1-3}$ alkyl. In some embodiments of the present disclosure, W in the structure of Formula (II) represents CH.

In some embodiments of the present disclosure, ring A in the structure of Formula (II) represents aryl. Exemplary aryl groups include, but are not limited to, $C_{5-30}$ aryl, $C_{5-25}$ aryl, $C_{5-20}$ aryl, and $C_{5-15}$ aryl. Representative examples of aryl include, but are not limited to, phenyl and naphthyl. The aryl is optionally substituted with m1 $R_{d2}$, where each $R_{d2}$ independently represents deuterium, hydroxy, amino, mercapto, halogen, cyano, optionally deuterated $C_{1-6}$ alkyl, optionally deuterated $C_{3-6}$ cycloalkyl, optionally deuterated $C_{1-6}$ alkoxy, halogenated $C_{1-6}$ alkyl, halogenated $C_{1-6}$ alkoxy or -$C_{1-6}$ alkylene-$NH_2$, and m1 represents an integer of 0 to 10 (e.g., an integer of 1-10, 0-9, 0-7, 1-6, 1-5, 1-4, 1-3, 1-2 or 1).

In some embodiments of the present disclosure, ring A in the structure of Formula (II) represents cycloalkyl. Exemplary cycloalkyl groups include, but are not limited to, $C_{3-30}$ cycloalkyl, $C_{3-25}$ cycloalkyl, $C_{3-20}$ cycloalkyl, and $C_{3-15}$cycloalkyl. Representative examples of cycloalkyl include, but are not limited to, cyclopropyl, cyclobutyl, cyclopentyl, cyclopentenyl, cyclohexyl, cyclohexenyl, cycloheptyl, cyclooctyl, decalinyl, octahydropentalenyl, octahydro-1H-indenyl, spiro-cycloalkyl (e.g., $C_3$-$C_{15}$ spiro-cycloalkyl or $C_7$-$C_{15}$ spiro-cycloalkyl or $C_5$-$C_{15}$ spiro-cycloalkyl, such as spiro[3.3]heptanyl, spiro[2.5]octyl, spiro[3.5]nonyl, spiro[4.4]nonyl, spiro[4.5]decyl, and spiro[5.5]undecyl), p-menthanyl, m-menthanyl, and bridged cycloalkyl (e.g., $C_3$-$C_{15}$ bridged cycloalkyl or $C_7$-$C_{15}$ bridged cycloalkyl or $C_5$-$C_{15}$ bridged cycloalkyl, such as adamantanyl, noradamantanyl, bornyl, bicyclo[2.2.1]heptanyl, 2-oxobicyclo[2.2.1]heptanyl, or bicyclo[2.2.1]heptentyl). The cycloalkyl is optionally substituted with m1 $R_{d2}$, where each $R_{d2}$ independently represents deuterium, hydroxy, amino, mercapto, halogen, cyano, optionally deuterated $C_{1-6}$ alkyl, optionally deuterated $C_{3-6}$ cycloalkyl, optionally deuterated $C_{1-6}$ alkoxy, halogenated $C_{1-6}$ alkyl, halogenated $C_{1-6}$ alkoxy or -$C_{1-6}$ alkylene-$NH_2$, and m1 represents an integer of 0 to 10 (e.g., an integer of 1-10, 0-9, 0-7, 1-6, 1-5, 1-4, 1-3, 1-2 or 1).

In some embodiments of the present disclosure, ring A in the structure of Formula (II) represents heterocyclyl. Exemplary heterocyclyl groups include, but are not limited to, 4- to 30-membered heterocyclyl, 4- to 25-membered heterocyclyl, 4- to 20-membered heterocyclyl, and 4- to 15-membered heterocyclyl. Representative examples of heterocyclyl include, but are not limited to, azetidinyl, pyrrolidinyl, imidazolidinyl, pyrazolidyl, oxazolidinyl, thiazolidinyl, piperidinyl, piperazinyl, tetrahydropyridyl, dihydroxypiperidinyl, difluoropiperidinyl, morpholinyl, thiomorpholinyl, azacycloheptanyl, azacyclooctyl, diazacycloheptanyl, diazacyclooctyl, bridged heterocyclyl (e.g., 4- to 15-membered bridged heterocyclyl, such as 3-azabicyclo[3.1.0]hexyl, 3-azabicyclo[3.1.1]heptanyl, 2-azabicyclo[2.2.1]heptanyl, 6-azabicyclo[3.1.1]heptanyl, 2-azabicyclo[2.2.2]octanyl, 2,5-diazabicyclo[2.2.1]heptanyl, 3,6-diazabicyclo[3.1.1]heptanyl, 3-azabicyclo[3.2.1]octanyl, 3,8-diazabicyclo[3.2.1]octanyl, 2,5-diazabicyclo[2.2.2]octanyl, and quinuclidinyl), spiro-heterocyclyl (e.g., 4- to 15-membered spiro-heterocyclyl, such as 2,6-diazaspiro[3.3]heptanyl, 2,7-diazaspiro[3.5]nonyl, 2,8-diazaspiro[4.5]decyl, 3,9-diazaspiro[5.5]undecyl, 3-azaspiro[5.5]undecyl, and 7-azaspiro[3.5]nonyl) and octahydropyrrolo[3,4-c]pyrrolyl. The heterocyclyl is optionally substituted with m1 $R_{d2}$, where each $R_{d2}$ independently represents deuterium, hydroxy, amino, mercapto, halogen, cyano, optionally deuterated $C_{1-6}$ alkyl, optionally deuterated $C_{3-6}$ cycloalkyl, optionally deuterated $C_{1-6}$ alkoxy, halogenated $C_{1-6}$ alkyl, halogenated $C_{1-6}$ alkoxy or -$C_{1-6}$ alkylene-$NH_2$, and m1 represents an integer of 0 to 10 (e.g., an integer of 1-10, 0-9, 0-7, 1-6, 1-5, 1-4, 1-3, 1-2 or 1).

In some embodiments of the present disclosure, ring A in the structure of Formula (II) represents heteroaryl. Exemplary heteroaryl groups include, but are not limited to, 5- to 30-membered heteroaryl, 5-to 25-membered heteroaryl, 5- to 20-membered heteroaryl, and 5- to 15-membered heteroaryl. Representative examples of heteroaryl include, but are not limited to, furanyl, oxazolyl, isoxazolyl, oxadiazolyl, thienyl, thiazolyl, isothiazolyl, thiadiazolyl, pyrrolyl, imidazolyl, pyrazolyl, triazolyl, pyridyl, pyrimidinyl, pyridazinyl, pyrazinyl, indolyl, isoindolyl, benzofuranyl, isobenzofuranyl, benzothienyl, indazolyl, benzimidazolyl, benzoxazolyl, benzisoxazolyl, benzothiazolyl, benzisothiazolyl, benzotriazolyl, benzo[2,1,3]oxadiazolyl, benzo[2,1,3]thiadiazolyl, benzo[1,2,3]thiadiazolyl, quinolinyl, isoquinolinyl, naphthyridinyl, cinnolinyl, quinazolinyl, quinoxalinyl, phthalazinyl, pyrazolo[1,5-a]pyridyl, pyrazolo[1,5-a]pyrimidinyl, imidazo[1,2-a]pyridyl, 1H-pyrrolo[3,2-b]pyridyl, 1H-pyrrolo[2,3-b]pyridyl, 4H-fluoro[3,2-b]pyrrolyl, pyrrolo[2,1-b]thiazolyl, and imidazo[2,1-b]thiazolyl. The heteroaryl is optionally substituted with m1 $R_{d2}$, where each $R_{d2}$ independently represents deuterium, hydroxy, amino, mercapto, halogen, cyano, optionally deuterated $C_{1-6}$ alkyl, optionally deuterated $C_{3-6}$ cycloalkyl, optionally deuterated $C_{1-6}$ alkoxy, halogenated $C_{1-6}$ alkyl, halogenated $C_{1-6}$ alkoxy or -$C_{1-6}$ alkylene-$NH_2$, and m1 represents an integer of 0 to 10 (e.g., an integer of 1-10, 0-9, 0-7, 1-6, 1-5, 1-4, 1-3, 1-2 or 1).

In some embodiments of the present disclosure, ring B in the structure of Formula (II) represents aryl. Exemplary aryl groups include, but are not limited to, $C_{5-30}$ aryl, $C_{5-25}$ aryl, $C_{5-20}$ aryl, and $C_{5-15}$ aryl. Representative examples of aryl include, but are not limited to, phenyl and naphthyl. The aryl is optionally substituted with m2 $R_{d3}$, where each $R_{d3}$ independently represents deuterium, hydroxy, amino, mercapto, halogen, cyano, optionally deuterated $C_{1-6}$ alkyl, optionally deuterated $C_{3-6}$ cycloalkyl, optionally deuterated $C_{1-6}$ alkoxy, halogenated $C_{1-6}$ alkyl, halogenated $C_{1-6}$ alkoxy or -$C_{1-6}$ alkylene-$NH_2$, and m2 represents an integer of 0 to 10 (e.g., an integer of 1-10, 0-9, 0-7, 1-6, 1-5, 1-4, 1-3, 1-2 or 1).

In some embodiments of the present disclosure, ring B in the structure of Formula (II) represents cycloalkyl. Exemplary cycloalkyl groups include, but are not limited to, $C_{3-30}$ cycloalkyl, $C_{3-25}$ cycloalkyl, $C_{3-20}$ cycloalkyl, and $C_{3-15}$ cycloalkyl. Representative examples of cycloalkyl include, but are not limited to, cyclopropyl, cyclobutyl, cyclopentyl, cyclopentenyl, cyclohexyl, cyclohexenyl, cycloheptyl, cyclooctyl, decalinyl, octahydropentalenyl, octahydro-1H-indenyl, spiro-cycloalkyl (e.g., $C_3$-$C_{15}$ spiro-cycloalkyl or $C_7$-$C_{15}$ spiro-cycloalkyl or $C_5$-$C_{15}$ spiro-cycloalkyl, such as spiro[3.3]heptanyl, spiro[2.5]octyl, spiro[3.5]nonyl, spiro[4.4]nonyl, spiro[4.5]decyl, and spiro[5.5]undecyl), p-menthanyl, m-menthanyl, and bridged cycloalkyl (e.g., $C_3$-$C_{15}$ bridged cycloalkyl or $C_7$-$C_{15}$ bridged cycloalkyl or $C_5$-$C_{15}$ bridged cycloalkyl, such as adamantanyl, noradamantanyl, bornyl, bicyclo[2.2.1]heptanyl, 2-oxobicyclo[2.2.1]heptanyl, and bicyclo[2.2.1]heptentyl). The cycloalkyl is optionally substituted with m2 $R_{d3}$, where each $R_{d3}$ independently represents deuterium, hydroxy, amino, mercapto, halogen, cyano, optionally deuterated $C_{1-6}$ alkyl, optionally deuterated $C_{3-6}$ cycloalkyl, optionally deuterated $C_{1-6}$ alkoxy, halogenated $C_{1-6}$ alkyl, halogenated $C_{1-6}$ alkoxy or -$C_{1-6}$ alkylene-$NH_2$, and m2 represents an integer of 0 to 10 (e.g., an integer of 1-10, 0-9, 0-7, 1-6, 1-5, 1-4, 1-3, 1-2 or 1).

In some embodiments of the present disclosure, ring B in the structure of Formula (II) represents heterocyclyl. Exemplary heterocyclyl groups include, but are not limited to, 4- to 30-membered heterocyclyl, 4- to 25-membered heterocyclyl, 4- to 20-membered heterocyclyl, and 4- to 15-membered heterocyclyl. Representative examples of heterocyclyl include, but are not limited to, azetidinyl, pyrrolidinyl, imidazolidinyl, pyrazolidyl, oxazolidinyl, thiazolidinyl, piperidinyl, piperazinyl, tetrahydropyridyl, dihydroxypiperidinyl, difluoropiperidinyl, morpholinyl, thiomorpholinyl, azacycloheptanyl, azacyclooctyl, diazacycloheptanyl, diazacyclooctyl, bridged heterocyclyl (e.g., 4- to 15-membered bridged heterocyclyl, such as 3-azabicyclo[3.1.0]hexyl, 3-azabicyclo[3.1.1]heptanyl, 2-azabicyclo[2.2.1]heptanyl, 6-azabicyclo[3.1.1]heptanyl, 2-azabicyclo[2.2.2]octanyl, 2,5-diazabicyclo[2.2.1]heptanyl, 3,6-diazabicyclo[3.1.1]heptanyl, 3-azabicyclo[3.2.1]octanyl, 3,8-diazabicyclo[3.2.1]octanyl, 2,5-diazabicyclo[2.2.2]octanyl, and quinuclidinyl), spiro-heterocyclyl (e.g., 4- to 15-membered spiro-heterocyclyl, such as 2,6-diazaspiro[3.3]heptanyl, 2,7-diazaspiro[3.5]nonyl, 2,8-diazaspiro[4.5]decyl, 3,9-diazaspiro[5.5]undecyl, 3-azaspiro[5.5]undecyl, and 7-azaspiro[3.5]nonyl), and octahydropyrrolo[3,4-c]pyrrolyl. The heterocyclyl is optionally substituted with m2 $R_{d3}$, where each $R_{d3}$ independently represents deuterium, hydroxy, amino, mercapto, halogen, cyano, optionally deuterated $C_{1-6}$ alkyl, optionally deuterated $C_{3-6}$ cycloalkyl, optionally deuterated $C_{1-6}$ alkoxy, halogenated $C_{1-6}$ alkyl, halogenated $C_{1-6}$ alkoxy or -$C_{1-6}$ alkylene-$NH_2$, and m2 represents an integer of 0 to 10 (e.g., an integer of 1-10, 0-9, 0-7, 1-6, 1-5, 1-4, 1-3, 1-2 or 1).

In some embodiments of the present disclosure, ring B in the structure of Formula (II) represents heteroaryl. Exemplary heteroaryl groups include, but are not limited to, 5- to 30-membered heteroaryl, 5-to 25-membered heteroaryl, 5- to 20-membered heteroaryl, and 5- to 15-membered heteroaryl. Representative examples of heteroaryl include, but are not limited to, furanyl, oxazolyl, isoxazolyl, oxadiazolyl, thienyl, thiazolyl, isothiazolyl, thiadiazolyl, pyrrolyl, imidazolyl, pyrazolyl, triazolyl, pyridyl, pyrimidinyl, pyridazinyl, pyrazinyl, indolyl, isoindolyl, benzofuranyl, isobenzofuranyl, benzothienyl, indazolyl, benzimidazolyl, benzoxazolyl, benzisoxazolyl, benzothiazolyl, benzisothiazolyl, benzotriazolyl, benzo[2,1,3]oxadiazolyl, benzo[2,1,3]thiadiazolyl, benzo[1,2,3]thiadiazolyl, quinolinyl, isoquinolinyl, naphthyridinyl, cinnolinyl, quinazolinyl, quinoxalinyl, phthalazinyl, pyrazolo[1,5-a]pyridyl, pyrazolo[1,5-a]pyrimidinyl, imidazo[1,2-a]pyridyl, 1H-pyrrolo[3,2-b]pyridyl, 1H-pyrrolo[2,3-b]pyridyl, pyrrolo[2,1-b]thiazolyl, and imidazo[2,1-b]thiazolyl. The heteroaryl is optionally substituted with m2 $R_{d3}$, where each $R_{d3}$ independently represents deuterium, hydroxy, amino, mercapto, halogen, cyano, optionally deuterated $C_{1-6}$ alkyl, optionally deuterated $C_{3-6}$ cycloalkyl, optionally deuterated $C_{1-6}$ alkoxy, halogenated $C_{1-6}$ alkyl, halogenated $C_{1-6}$ alkoxy or -$C_{1-6}$ alkylene-$NH_2$, and m2 represents an integer of 0 to 10 (e.g., an

integer of 1-10, 0-9, 0-7, 1-6, 1-5, 1-4, 1-3, 1-2 or 1).

In some embodiments of the present disclosure, $R_{a1}$ represents the following group:

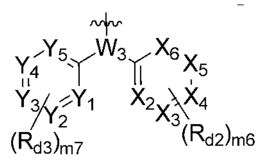

wherein $W_3$ represents CH or N;

any one or two of $X_2$, $X_3$, $X_4$, $X_5$ and $X_6$ represent CH or N, and the remaining ones represent CH;

any one or two of $Y_1$, $Y_2$, $Y_3$, $Y_4$ and $Y_5$ represent CH or N, and the remaining ones represent CH;

$(R_{d2})_{m6}$ represents m6 substituents $R_{d2}$, where m6 represents an integer of 0 to 5 (e.g., an integer of 0, 1, 2, 3, 4 or 5), and each $R_{d2}$ independently represents hydroxy, amino, mercapto, halogen, cyano, optionally deuterated $C_{1-6}$ alkyl, optionally deuterated $C_{3-6}$ cycloalkyl, optionally deuterated $C_{1-6}$ alkoxy, halogenated $C_{1-6}$ alkyl, halogenated $C_{1-6}$ alkoxy or $-C_{1-6}$ alkylene-$NH_2$; and

$(R_{d3})_{m7}$ represents m7 substituents $R_{d3}$, where m7 represents an integer of 0 to 5 (e.g., an integer of 0, 1, 2, 3, 4 or 5), and each $R_{d3}$ independently represents hydroxy, amino, mercapto, halogen, cyano, optionally deuterated $C_{1-6}$ alkyl, optionally deuterated $C_{3-6}$ cycloalkyl, optionally deuterated $C_{1-6}$ alkoxy, halogenated $C_{1-6}$ alkyl, halogenated $C_{1-6}$ alkoxy or $-C_{1-6}$ alkylene-$NH_2$.

In embodiments of the present disclosure, when $W_3$ represents N and $X_1$ represents $N(R_{c2})$, $L_1$ is not $N(R_{c1})$.

In some embodiments of the present disclosure, any one of $X_2$, $X_3$, $X_4$, $X_5$ and $X_6$ represents N, and the remaining ones represent CH.

In some embodiments of the present disclosure, any two of $X_2$, $X_3$, $X_4$, $X_5$ and $X_6$ represent N, and the remaining ones represent CH.

In some embodiments of the present disclosure, $X_2$, $X_3$, $X_4$, $X_5$ and $X_6$ all represent CH.

In some embodiments of the present disclosure, any one of $Y_1$, $Y_2$, $Y_3$, $Y_4$ and $Y_5$ represents N, and the remaining ones represent CH.

In some embodiments of the present disclosure, any two of $Y_1$, $Y_2$, $Y_3$, $Y_4$ and $Y_5$ represent N, and the remaining ones represent CH.

In some embodiments of the present disclosure, $Y_1$, $Y_2$, $Y_3$, $Y_4$ and $Y_5$ all represent CH.

In some embodiments of the present disclosure, $R_{a1}$ represents the structure of Formula (III) (a fused ring system):

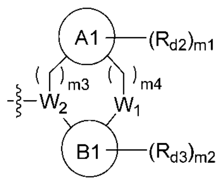

Formula (III)

wherein m3 and m4 each independently represent an integer of 0, 1 or 2, $W_1$ represents O, S or NH, or $W_1$ represents a bond; preferably, m3 and m4 each independently represent an integer of 0 or 1, $W_1$ represents O, S or NH, or $W_1$ represents a bond;

$W_2$ represents N or $CR_{d1}$, where $R_{d1}$ represents H, halogen, $C_{1-3}$ alkyl or halogenated $C_{1-3}$ alkyl;

ring A1 represents an aryl ring, a cycloalkyl ring, a heterocyclyl ring or a heteroaryl ring, and $(R_{d2})_{m1}$ indicates that ring A1 is optionally substituted with m1 $R_{d2}$, where m1 represents an integer of 0 to 10, and each $R_{d2}$ independently represents deuterium, hydroxy, amino, mercapto, halogen, cyano, optionally deuterated $C_{1-6}$ alkyl, optionally deuterated $C_{3-6}$ cycloalkyl, optionally deuterated $C_{1-6}$ alkoxy, halogenated $C_{1-6}$ alkyl, halogenated $C_{1-6}$ alkoxy or $-C_{1-6}$ alkylene-$NH_2$; and

ring B1 represents an aryl ring, a cycloalkyl ring, a heterocyclyl ring or a heteroaryl ring, and $(R_{d3})_{m2}$ indicates that ring B1 is optionally substituted with m2 $R_{d3}$, where m2 represents an integer of 0 to 10, and each $R_{d3}$ independently represents deuterium, hydroxy, amino, mercapto, halogen, cyano, optionally deuterated $C_{1-6}$ alkyl, optionally deuterated $C_{3-6}$ cycloalkyl, optionally deuterated $C_{1-6}$ alkoxy, halogenated $C_{1-6}$ alkyl, halogenated $C_{1-6}$ alkoxy or $-C_{1-6}$ alkylene-$NH_2$.

In some embodiments of the present disclosure, m3 and m4 each independently represent an integer of 0, $W_1$ represents a bond, i.e., $R_{a1}$ represents the structure of the following formula:

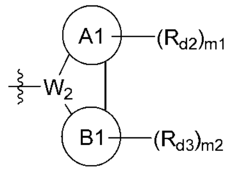

$W_2$, ring A1, ring B1, $(R_{d2})_{m1}$, and $(R_{d3})_{m2}$ are as defined in the structure of Formula (III).

In some embodiments of the present disclosure, m3 and m4 each independently represent an integer of 0, $W_1$ represents O, S or NH, i.e., $R_{a1}$ represents the structure of the following formula:

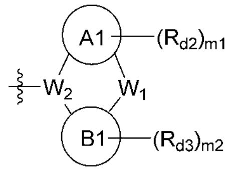

$W_2$, ring A1, ring B1, $(R_{d2})_{m1}$, and $(R_{d3})_{m2}$ are as defined in the structure of Formula (III).

In embodiments of the present disclosure, when $W_2$ represents N and Xi represents $N(R_{c2})$, $L_1$ is not $N(R_{c1})$.

In some embodiments of the present disclosure, $W_2$ in the structure of Formula (III) represents N.

In some embodiments of the present disclosure, $W_2$ in the structure of Formula (III) represents $CR_{d1}$, where $R_{a1}$ represents H, halogen, $C_{1-3}$ alkyl or halogenated $C_{1-3}$ alkyl. In some embodiments of the present disclosure, $W_2$ in the structure of Formula (III) represents CH.

In some embodiments of the present disclosure, ring A1 in the structure of Formula (III) represents aryl ring. Exemplary aryl ring includes, but is not limited to, $C_{5-30}$ aryl ring, $C_{5-25}$ aryl ring, $C_{5-20}$ aryl ring, and $C_{5-15}$ aryl ring. Representative examples of aryl ring include, but are not limited to, phenyl ring, and naphthyl ring. The aryl ring is optionally substituted with m1 $R_{d2}$, where each $R_{d2}$ independently represents deuterium, hydroxy, amino, mercapto, halogen, cyano, optionally deuterated $C_{1-6}$ alkyl, optionally deuterated $C_{3-6}$ cycloalkyl, optionally deuterated $C_{1-6}$ alkoxy, halogenated $C_{1-6}$ alkyl, halogenated $C_{1-6}$ alkoxy or -$C_{1-6}$ alkylene-$NH_2$, and m1 represents an integer of 0 to 10 (e.g., an integer of 1-10, 0-9, 0-7, 1-6, 1-5, 1-4, 1-3, 1-2 or 1).

In some embodiments of the present disclosure, ring A1 in the structure of Formula (III) represents cycloalkyl ring. Exemplary cycloalkyl ring includes, but is not limited to, $C_{3-30}$ cycloalkyl ring, $C_{3-25}$ cycloalkyl ring, $C_{3-20}$ cycloalkyl ring, and $C_{3-15}$ cycloalkyl ring. Representative examples of cycloalkyl ring include, but are not limited to, cyclopropyl ring, cyclobutyl ring, cyclopentyl ring, cyclopentenyl ring, cyclohexyl ring, cyclohexenyl ring, cycloheptyl ring, cyclooctyl ring, decalinyl ring, octahydropentalenyl ring, octahydro-1H-indenyl ring, spiro-cycloalkyl ring (e.g., $C_3$-$C_{15}$ spiro-cycloalkyl ring or $C_5$-$C_{15}$ spiro-cycloalkyl ring or $C_7$-$C_{15}$ spiro-cycloalkyl ring, such as spiro[3.3]heptanyl ring, spiro[2.5]octyl ring, spiro[3.5]nonyl ring, spiro[4.4]nonyl ring, spiro[4.5]decyl ring, and spiro[5.5]undecyl ring), p-menthanyl ring, m-menthanyl ring, and bridged cycloalkyl ring (e.g., $C_3$-$C_{15}$ bridged cycloalkyl ring, or $C_5$-$C_{15}$ bridged cycloalkyl ring, or $C_7$-$C_{15}$ bridged cycloalkyl ring, such as adamantanyl ring, noradamantanyl ring, bornyl ring, bicyclo[2.2.1]heptanyl ring, 2-oxobicyclo[2.2.1]heptanyl ring, and bicyclo[2.2.1]heptentyl ring). The cycloalkyl ring is optionally substituted with m1 $R_{d2}$, where each $R_{d2}$ independently represents deuterium, hydroxy, amino, mercapto, halogen, cyano, optionally deuterated $C_{1-6}$ alkyl, optionally deuterated $C_{3-6}$ cycloalkyl, optionally deuterated $C_{1-6}$ alkoxy, halogenated $C_{1-6}$ alkyl, halogenated $C_{1-6}$ alkoxy or -$C_{1-6}$ alkylene-$NH_2$, and m1 represents an integer of 0 to 10 (e.g., an integer of 1-10, 0-9, 0-7, 1-6, 1-5, 1-4, 1-3, 1-2 or 1).

In some embodiments of the present disclosure, ring A1 in the structure of Formula (III) represents heterocyclyl ring. Exemplary heterocyclyl ring includes, but is not limited to, 4- to 30-membered heterocyclyl ring, 4- to 25-membered heterocyclyl ring, 4- to 20-membered heterocyclyl ring, and 4- to 15-membered heterocyclyl ring. Representative examples of heterocyclyl ring include, but are not limited to, azetidinyl ring, pyrrolidinyl ring, imidazolidinyl ring, pyrazolidyl ring, oxazolidinyl ring, thiazolidinyl ring, piperidinyl ring, piperazinyl ring, tetrahydropyridyl ring, dihydroxypiperidinyl ring, difluoropiperidinyl ring, morpholinyl ring, thiomorpholinyl ring, azacycloheptanyl ring, azacyclooctyl ring, diazacycloheptanyl ring, diazacyclooctyl ring, bridged heterocyclyl ring (e.g., 4- to 15-membered bridged heterocyclyl ring, or 6- to 15-membered bridged heterocyclyl ring, or 7- to 15-membered bridged heterocyclyl ring, such as 3-azabicyclo[3.1.0]hexyl ring, 3-azabicyclo[3.1.1]heptanyl ring, 2-azabicyclo[2.2.1]heptanyl ring, 6-azabicyclo[3.1.1]heptanyl ring, 2-azabicyclo[2.2.2]octanyl ring, 2,5-diazabicyclo[2.2.1]heptanyl ring, 3,6-diazabicyclo[3.1.1]heptanyl ring, 3-azabicyclo[3.2.1]octanyl ring, 3,8-diazabicyclo[3.2.1]octanyl ring, 2,5-diazabicyclo[2.2.2]octanyl ring, or quinuclidinyl ring), spiro-heterocyclyl ring (e.g., 4- to 15-membered spiro-heterocyclyl ring, or 5- to 15-membered spiro-heterocyclyl ring, or 7- to 15-membered

spiro-heterocyclyl ring, such as 2,6-diazaspiro[3.3]heptanyl ring, 2,7-diazaspiro[3.5]nonyl ring, 2,8-diazaspiro[4.5]decyl ring, 3,9-diazaspiro[5.5]undecyl ring, 3-azaspiro[5.5]undecyl ring, and 7-azaspiro[3.5]nonyl ring), and octahydropyrrolo [3,4-c]pyrrolyl ring. The heterocyclyl ring is optionally substituted with m1 $R_{d2}$, where each $R_{d2}$ independently represents deuterium, hydroxy, amino, mercapto, halogen, cyano, optionally deuterated $C_{1-6}$ alkyl, optionally deuterated $C_{3-6}$ cycloalkyl, optionally deuterated $C_{1-6}$ alkoxy, halogenated $C_{1-6}$ alkyl, halogenated $C_{1-6}$ alkoxy or -$C_{1-6}$ alkylene-$NH_2$, and m1 represents an integer of 0 to 10 (e.g., an integer of 1-10, 0-9, 0-7, 1-6, 1-5, 1-4, 1-3, 1-2 or 1).

In some embodiments of the present disclosure, ring A1 in the structure of Formula (III) represents heteroaryl ring. Exemplary heteroaryl ring includes, but is not limited to, 5- to 30-membered heteroaryl ring, 5- to 25-membered heteroaryl ring, 5- to 20-membered heteroaryl ring, and 5- to 15-membered heteroaryl ring. Representative examples of heteroaryl ring include, but are not limited to, furanyl ring, oxazolyl ring, isoxazolyl ring, oxadiazolyl ring, thienyl ring, thiazolyl ring, isothiazolyl ring, thiadiazolyl ring, pyrrolyl ring, imidazolyl ring, pyrazolyl ring, triazolyl ring, pyridyl ring, pyrimidinyl ring, pyridazinyl ring, pyrazinyl ring, indolyl ring, isoindolyl ring, benzofuranyl ring, isobenzofuranyl ring, benzothienyl ring, indazolyl ring, benzimidazolyl ring, benzoxazolyl ring, benzisoxazolyl ring, benzothiazolyl ring, benzisothiazolyl ring, benzotriazolyl ring, benzo[2,1,3]oxadiazolyl ring, benzo[2,1,3]thiadiazolyl ring, benzo[1,2,3]thiadiazolyl ring, quinolinyl ring, isoquinolinyl ring, naphthyridinyl ring, cinnolinyl ring, quinazolinyl ring, quinoxalinyl ring, phthalazinyl ring, pyrazolo[1,5-a]pyridyl ring, pyrazolo[1,5-a]pyrimidinyl ring, imidazo[1,2-a]pyridyl ring, 1H-pyrrolo[3,2-b] pyridyl ring, 1H-pyrrolo[2,3-b]pyridyl ring, pyrrolo[2,1-b]thiazolyl ring, and imidazo[2,1-b]thiazolyl ring. The heteroaryl ring is optionally substituted with m1 $R_{d2}$, where each $R_{d2}$ independently represents deuterium, hydroxy, amino, mercapto, halogen, cyano, optionally deuterated $C_{1-6}$ alkyl, optionally deuterated $C_{3-6}$ cycloalkyl, optionally deuterated $C_{1-6}$ alkoxy, halogenated $C_{1-6}$ alkyl, halogenated $C_{1-6}$ alkoxy or -$C_{1-6}$ alkylene-$NH_2$, and m1 represents an integer of 0 to 10 (e.g., an integer of 1-10, 0-9, 0-7, 1-6, 1-5, 1-4, 1-3, 1-2 or 1).

In some embodiments of the present disclosure, ring B1 in the structure of Formula (III) represents aryl ring. Exemplary aryl ring includes, but is not limited to, $C_{5-30}$ aryl ring, $C_{5-25}$ aryl ring, $C_{5-20}$ aryl ring, and $C_{5-15}$ aryl ring. Representative examples of aryl ring include, but are not limited to, phenyl ring, and naphthyl ring. The aryl ring is optionally substituted with m2 $R_{d3}$, where each $R_{d3}$ independently represents deuterium, hydroxy, amino, mercapto, halogen, cyano, optionally deuterated $C_{1-6}$ alkyl, optionally deuterated $C_{3-6}$ cycloalkyl, optionally deuterated $C_{1-6}$ alkoxy, halogenated $C_{1-6}$ alkyl, halogenated $C_{1-6}$ alkoxy or -$C_{1-6}$ alkylene-$NH_2$, and m2 represents an integer of 0 to 10 (e.g., an integer of 1-10, 0-9, 0-7, 1-6, 1-5, 1-4, 1-3, 1-2 or 1).

In some embodiments of the present disclosure, ring B1 in the structure of Formula (III) represents cycloalkyl ring. Exemplary cycloalkyl ring includes, but is not limited to, $C_{3-30}$ cycloalkyl ring, $C_{3-25}$ cycloalkyl ring, $C_{3-20}$ cycloalkyl ring, and $C_{3-15}$ cycloalkyl ring. Representative examples of cycloalkyl ring include, but are not limited to, cyclopropyl ring, cyclobutyl ring, cyclopentyl ring, cyclopentenyl ring, cyclohexyl ring, cyclohexenyl ring, cycloheptyl ring, cyclooctyl ring, decalinyl ring, octahydropentalenyl ring, octahydro-1H-indenyl ring, spiro-cycloalkyl ring (e.g., $C_3$-$C_{15}$ spiro-cycloalkyl ring, such as spiro[3.3]heptanyl ring, spiro[2.5]octyl ring, spiro[3.5]nonyl ring, spiro[4.4]nonyl ring, spiro[4.5]decyl ring, and spiro[5.5] undecyl ring), p-menthanyl ring, m-menthanyl ring, and bridged cycloalkyl ring (e.g., $C_3$-$C_{15}$ bridged cycloalkyl ring, such as adamantanyl ring, noradamantanyl ring, bornyl ring, bicyclo[2.2.1]heptanyl ring, 2-oxobicyclo[2.2.1]heptanyl ring, or bicyclo[2.2.1]heptentyl ring). The cycloalkyl ring is optionally substituted with m2 $R_{d3}$, where each $R_{d3}$ independently represents deuterium, hydroxy, amino, mercapto, halogen, cyano, optionally deuterated $C_{1-6}$ alkyl, optionally deuterated $C_{3-6}$ cycloalkyl, optionally deuterated $C_{1-6}$ alkoxy, halogenated $C_{1-6}$ alkyl, halogenated $C_{1-6}$ alkoxy or -$C_{1-6}$ alkylene-$NH_2$, and m2 represents an integer of 0 to 10 (e.g., an integer of 1-10, 0-9, 0-7, 1-6, 1-5, 1-4, 1-3, 1-2 or 1).

In some embodiments of the present disclosure, ring B1 in the structure of Formula (III) represents heterocyclyl ring. Exemplary heterocyclyl ring includes, but is not limited to, 4- to 30-membered heterocyclyl ring, 4- to 25-membered heterocyclyl ring, 4- to 20-membered heterocyclyl ring, and 4- to 15-membered heterocyclyl ring. Representative examples of heterocyclyl ring include, but are not limited to, azetidinyl ring, pyrrolidinyl ring, imidazolidinyl ring, pyrazolidyl ring, oxazolidinyl ring, thiazolidinyl ring, piperidinyl ring, piperazinyl ring, tetrahydropyridyl ring, dihydroxypiperidinyl ring, difluoropiperidinyl ring, morpholinyl ring, thiomorpholinyl ring, azacycloheptanyl ring, azacyclooctyl ring, diazacycloheptanyl ring, diazacyclooctyl ring, bridged heterocyclyl ring (e.g., 4- to 15-membered bridged heterocyclyl ring, such as 3-azabicyclo[3.1.0]hexyl ring, 3-azabicyclo[3.1.1]heptanyl ring, 2-azabicyclo[2.2.1]heptanyl ring, 6-azabicyclo[3.1.1]heptanyl ring, 2-azabicyclo[2.2.2]octanyl ring, 2,5-diazabicyclo[2.2.1]heptanyl ring, 3,6-diazabicyclo[3.1.1]heptanyl ring, 3-azabicyclo[3.2.1]octanyl ring, 3,8-diazabicyclo[3.2.1]octanyl ring, 2,5-diazabicyclo[2.2.2]octanyl ring, and quinuclidinyl ring), spiro-heterocyclyl ring (e.g., 4- to 15-membered spiro-heterocyclyl ring, such as 2,6-diazaspiro[3.3]heptanyl ring, 2,7-diazaspiro[3.5]nonyl ring, 2,8-diazaspiro[4.5]decyl ring, 3,9-diazaspiro[5.5]undecyl ring, 3-azaspiro[5.5]undecyl ring, and 7-azaspiro[3.5]nonyl ring), and octahydropyrrolo[3,4-c]pyrrolyl ring. The heterocyclyl ring is optionally substituted with m2 $R_{d3}$, where each $R_{d3}$ independently represents deuterium, hydroxy, amino, mercapto, halogen, cyano, optionally deuterated $C_{1-6}$ alkyl, optionally deuterated $C_{3-6}$ cycloalkyl, optionally deuterated $C_{1-6}$ alkoxy, halogenated $C_{1-6}$ alkyl, halogenated $C_{1-6}$ alkoxy or -$C_{1-6}$ alkylene-$NH_2$, and m2 represents an integer of 0 to 10 (e.g., an integer of 1-10, 0-9, 0-7, 1-6, 1-5, 1-4, 1-3, 1-2 or 1).

In some embodiments of the present disclosure, ring B1 in the structure of Formula (III) represents heteroaryl

ring. Exemplary heteroaryl ring includes, but is not limited to, 5- to 30-membered heteroaryl ring, 5- to 25-membered heteroaryl ring, 5- to 20-membered heteroaryl ring, and 5- to 15-membered heteroaryl ring. Representative examples of heteroaryl ring include, but are not limited to, furanyl ring, oxazolyl ring, isoxazolyl ring, oxadiazolyl ring, thienyl ring, thiazolyl ring, isothiazolyl ring, thiadiazolyl ring, pyrrolyl ring, imidazolyl ring, pyrazolyl ring, triazolyl ring, pyridyl ring, pyrimidinyl ring, pyridazinyl ring, pyrazinyl ring, indolyl ring, isoindolyl ring, benzofuranyl ring, isobenzofuranyl ring, benzothienyl ring, indazolyl ring, benzimidazolyl ring, benzoxazolyl ring, benzisoxazolyl ring, benzothiazolyl ring, benzisothiazolyl ring, benzotriazolyl ring, benzo[2,1,3]oxadiazolyl ring, benzo[2,1,3]thiadiazolyl ring, benzo[1,2,3]thiadiazolyl ring, quinolinyl ring, isoquinolinyl ring, naphthyridinyl ring, cinnolinyl ring, quinazolinyl ring, quinoxalinyl ring, phthalazinyl ring, pyrazolo[1,5-a]pyridyl ring, pyrazolo[1,5-a]pyrimidinyl ring, imidazo[1,2-a]pyridyl ring, 1H-pyrrolo[3,2-b]pyridyl ring, 1H-pyrrolo[2,3-b]pyridyl ring, 4H-fluoro[3,2-b]pyrrolyl ring, pyrrolo[2,1-b]thiazolyl ring, and imidazo[2,1-b]thiazolyl ring. The heteroaryl ring is optionally substituted with m2 $R_{d3}$, where each $R_{d3}$ independently represents deuterium, hydroxy, amino, mercapto, halogen, cyano, optionally deuterated $C_{1}$-$_{6}$ alkyl, optionally deuterated $C_{3-6}$ cycloalkyl, optionally deuterated $C_{1-6}$ alkoxy, halogenated $C_{1-6}$ alkyl, halogenated $C_{1-6}$ alkoxy or -$C_{1-6}$ alkylene-$NH_2$, and m2 represents an integer of 0 to 10 (e.g., an integer of 1-10, 0-9, 0-7, 1-6, 1-5, 1-4, 1-3, 1-2 or 1).

In some embodiments of the present disclosure, $R_{a1}$ represents the following groups:

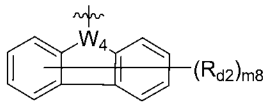 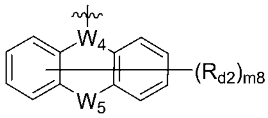

wherein $W_4$ represents CH or N, and $W_5$ represents O, S, NH or $CH_2$; and

each $(R_{d2})_{m8}$ independently represents m8 substituents $R_{d2}$, where m8 represents an integer of 0 to 8 (e.g., an integer of 1-8, 0-7, 1-6, 1-5, 1-4, 1-3, 1-2 or 1), and each $R_{d2}$ independently represents hydroxy, amino, mercapto, halogen, cyano, optionally deuterated $C_{1-6}$ alkyl, optionally deuterated $C_{3-6}$ cycloalkyl, optionally deuterated $C_{1-6}$ alkoxy, halogenated $C_{1-6}$ alkyl, halogenated $C_{1-6}$ alkoxy or - $C_{1-6}$ alkylene-$NH_2$.

In embodiments of the present disclosure, when $W_4$ represents N and $X_1$ represents $N(R_{c2})$, $L_1$ is not $N(R_{c1})$.

In some embodiments of the present disclosure, $W_4$ represents CH. In some embodiments of the present disclosure, $W_4$ represents N.

In some embodiments of the present disclosure, $W_5$ represents NH or $CH_2$.

In some embodiments of the present disclosure, $R_{a1}$ represents the structure of Formula (IV):

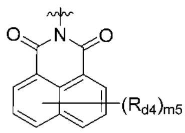

Formula (IV) ;

wherein $(R_{d4})_{m5}$ indicates that the structure of Formula (IV) is optionally substituted with m5 $R_{d4}$, where m5 represents 0 to 6 an integer of (e.g., an integer of 1-6, 0-5, 1-4, 1-3, 1-2 or 1), and each $R_{d4}$ independently represents hydroxy, amino, mercapto, halogen, cyano, optionally deuterated $C_{1-6}$ alkyl, optionally deuterated $C_{3-6}$ cycloalkyl, optionally deuterated $C_{1-6}$ alkoxy, halogenated $C_{1-6}$ alkyl, halogenated $C_{1-6}$ alkoxy or -$C_{1-6}$ alkylene-$NH_2$.

In embodiments of the present disclosure, when $R_{a1}$ represents the structure of Formula (IV) above, $X_1$ is not $N(R_{c2})$ and Li is not $N(R_{c1})$.

In some embodiments of the present disclosure, $R_{a1}$ represents the following group:

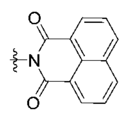.

In some embodiments of the present disclosure, the compound of Formula (I) is also of Formula (I-1) or Formula (I-2):

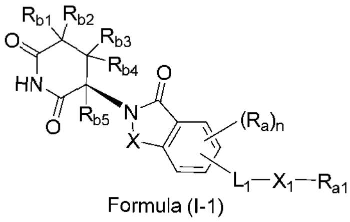

Formula (I-1)

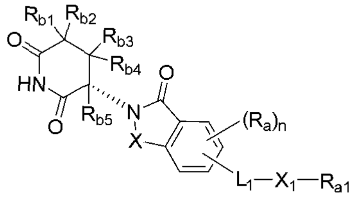

Formula (I-2)

wherein $R_{b1}$, $R_{b2}$, $R_{b3}$, $R_{b4}$, $R_{b5}$, $(R_a)_n$, X, $L_1$, $X_1$ and $R_{a1}$ are as defined in the compounds of Formula (I) above and its various embodiments described herein.

Preferably the compounds of the present invention and their salts (especially pharmaceutically acceptable salts, such as hydrochloride, etc.), enantiomers, diastereomers, solvates, or polymorphs thereof in Table 1 below are provided:

Table 1. The compounds of the present invention

| Compound No. | Structure of the compounds | The compounds' name | Chinese translation of the compounds' name |
|---|---|---|---|
| GT-03245 | 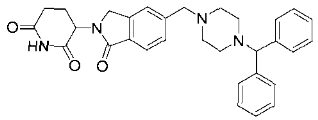 | 3-(5-((4-benzhydrylpiperazin-1-yl)methyl)-1-oxoisoindolin-2-yl)piperidine-2,6-dione | 3-(5-((4-benzhydrylpiperazin-1-yl)methyl)-1-oxoisoindolin-2-yl)piperidine-2,6-dione |
| GT-03466 | 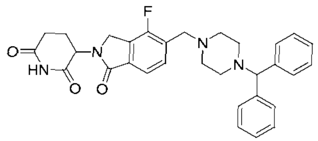 | 3-(5-((4-benzhydrylpiperazin-1-yl)methyl)-4-fluoro-1-oxoisoindolin-2-vl)piperidine-2,6-dione | 3-(5-((4-benzhydrylpiperazin-1-yl)methyl)-4-fluoro-1-oxoisoindolin-2-vl)piperidine-2,6-dione |
| GT-03700 | 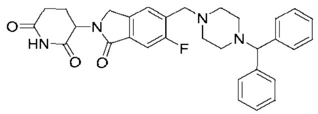 | 3-(5-((4-benzhydrylpiperazin-1-yl)methyl)-6-fluoro-1-oxoisoindolin-2-yl)piperidine-2,6-dione | 3-(5-((4-benzhydrylpiperazin-1-yl)methyl)-6-fluoro-1-oxoisoindolin-2-yl)piperidine-2,6-dione |
| GT-03467 | 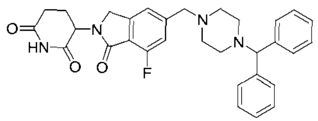 | 3-(5-((4-benzhydrylpiperazin-1-yl)methyl)-7-fluoro-1-oxoisoindolin-2-yl)piperidine-2,6-dione | 3-(5-((4-benzhydrylpiperazin-1-yl)methyl)-7-fluoro-1-oxoisoindolin-2-yl)piperidine-2,6-dione |
| GT-03646 | 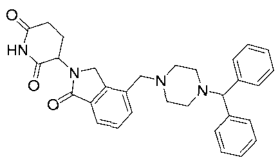 | 3-(4-((4-benzhydrylpiperazin-1-yl)methyl)-1-oxoisoindolin-2-yl)piperidine-2,6-dione | 3-(4-((4-benzhydrylpiperazin-1-yl)methyl)-1-oxoisoindolin-2-vl)piperidine-2,6-dione |
| GT-05686 | 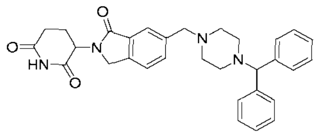 | 3-(6-((4-benzhydrylpiperazin-1-yl)methyl)-1-oxoisoindolin-2-yl)piperidine-2,6-dione | 3-(6-((4-benzhydrylpiperazin-1-yl)methyl)-1-oxoisoindolin-2-yl)piperidine-2,6-dione |
| GT-05687 | 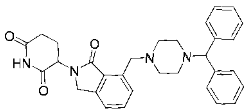 | 3-(7-((4-benzhydrylpiperazin-1-yl)methyl)-1-oxoisoindolin-2-yl)piperidine-2,6-dione | 3-(7-((4-benzhydrylpiperazin-1-yl)methyl)-1-oxoisoindolin-2-yl)piperidine-2,6-dione |

(continued)

| Compound No. | Structure of the compounds | The compounds' name | Chinese translation of the compounds' name |
|---|---|---|---|
| GT-05696 | 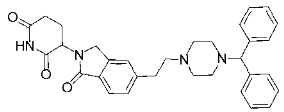 | 3-(5-(2-(4-benzhydrylpiperazin-1-yl)ethyl)-1-oxoisoindolin-2-vl)piperidine-2,6-dione | 3-(5-(2-(4-benzhydrylpiperazin-1-yl)ethyl)-1-oxoisoindolin-2-yl)piperidine-2,6-dione |
| GT-05688 | 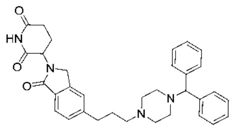 | 3-(5-(3-(4-benzhydrylpiperazin-1-yl)propyl)-1-oxoisoindolin-2-yl)piperidine-2,6-dione | 3-(5-(3-(4-benzhydrylpiperazin-1-yl)propyl)-1-oxoisoindolin-2-yl)piperidine-2,6-dione |
| GT-05867 | 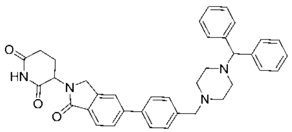 | 3-(5-(4-((4-benzhydrylpiperazin-1-yl)methyl)phenyl)-1-oxoisoindolin-2-yl)piperidine-2,6-dione | 3-(5-(4-((4-benzhydrylpiperazin-1-yl)methyl)phenyl)-1-oxoisoindolin-2-yl)piperidine-2,6-dione |
| GT-05744 | 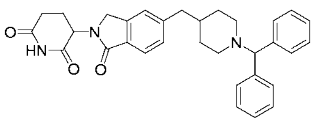 | 3-(5-((1-benzhydrylpiperidin-4-yl)methyl)-1-oxoisoindolin-2-yl)piperidine-2,6-dione | 3-(5-((1-benzhydrylpiperidin-4-yl)methyl)-1-oxoisoindolin-2-vl)piperidine-2,6-dione |
| GT-05864 | 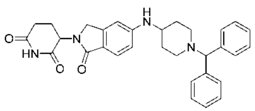 | 3-(5-((1-benzhydrylpiperidin-4-yl)amino)-1-oxoisoindolin-2-yl)piperidine-2,6-dione | 3-(5-((1-benzhydrylpiperidin-4-yl)amino)-1-oxoisoindolin-2-yl)piperidine-2,6-dione |
| GT-05743 | 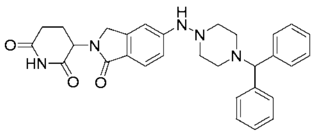 | 3-(5-((4-benzhydrylpiperazin-1-yl)amino)-1-oxoisoindolin-2-yl)piperidine-2,6-dione | 3-(5-((4-benzhydrylpiperazin-1-yl)amino)-1-oxoisoindolin-2-yl)piperidine-2,6-dione |
| | 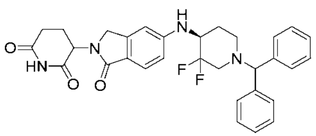 | 3-(5-(((S)-1-benzhydryl-3,3-difluoropiperidin-4-yl)amino)-1-oxoisoindolin-2-yl)piperidine-2,6-dione | 3-(5-(((S)-1-benzhydryl-3,3-difluoropiperidin-4-yl)amino)-1-oxoisoindolin-2-yl)piperidine-2,6-dione |

(continued)

| Compou nd No. | Structure of the compounds | The compounds' name | Chinese translation of the compounds' name |
|---|---|---|---|
| GT-05859 | 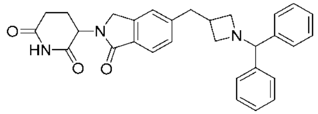 | 3-(5-((1-benzhydrylazetidin-3-yl)methyl)-1-oxoisoindolin-2-yl)piperidine-2,6-dione | 3-(5-((1-benzhydrylazetidin-3-yl)methyl)-1-oxoisoindolin-2-vl)piperidine-2,6-dione |
| | 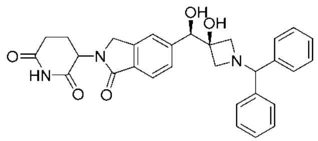 | 3-(5-((R)-(1-benzhydryl-3-hydroxyazetidin-3-yl)(hydroxy) methyl)-1-oxoisoindolin-2-yl)piperidine-2,6-dione | 3-(5-((R)-(1-benzhydryl-3-hydroxyazetidin-3-yl)(hydroxy) methyl)-1-oxoisoindolin-2-yl)piperidine-2,6-dione |
| GT-05745 | 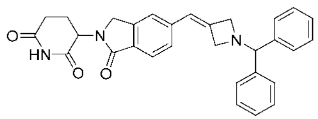 | 3-(5-((1-benzhydrylazetidin-3-ylidene)methyl)-1-oxoisoindolin-2-yl)piperidine-2,6-dione | 3-(5-((1-benzhydrylazetidin-3-ylidene)methyl)-1-oxoisoindolin-2-vl)piperidine-2,6-dione |
| | 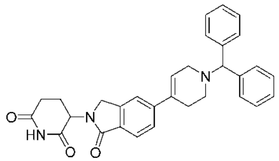 | 3-(5-(1-benzhydryl-1,2,3,6-tetrahydropyridin-4-yl)-1-oxoisoindolin-2-yl)piperidine-2,6-dione | 3-(5-(1-benzhydryl-1,2,3,6-tetrahydropyridin-4-yl)-1-oxoisoindolin-2-yl)piperidine-2,6-dione |
| GT-05868 | 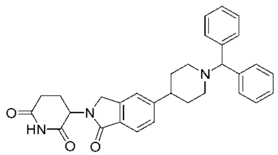 | 3-(5-(1-benzhydrylpiperidin-4-yl)-1-oxoisoindolin-2-yl)piperidine-2,6-dione | 3-(5-(1-benzhydrylpiperidin-4-yl)-1-oxoisoindolin-2-yl)piperidine-2,6-dione |
| | 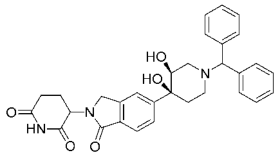 | 3-(5-((3S,4S)-1-benzhydryl-3,4-dihydroxypiperidin-4-yl)-1-oxoisoindolin-2-yl)piperidine-2,6-dione | 3-(5-((3S,4S)-1-benzhydryl-3,4-dihydroxypiperidin-4-yl)-1-oxoisoindolin-2-yl)piperidine-2,6-dione |

(continued)

| Compound No. | Structure of the compounds | The compounds' name | Chinese translation of the compounds' name |
|---|---|---|---|
| GT-06198 | 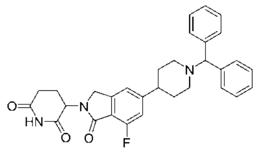 | 3-(5-(1-benzhydrylpiperidin-4-yl)-7-fluoro-1-oxoisoindolin-2-yl)piperidine-2,6-dione | 3-(5-(1-benzhydrylpiperidin-4-yl)-7-fluoro-1-oxoisoindolin-2-yl)piperidine-2,6-dione |
| GT-06136 | 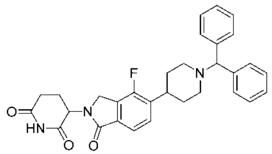 | 3-(5-(1-benzhydrylpiperidin-4-yl)-4-fluoro-1-oxoisoindolin-2-yl)piperidine-2,6-dione | 3-(5-(1-benzhydrylpiperidin-4-yl)-4-fluoro-1-oxoisoindolin-2-yl)piperidine-2,6-dione |
| GT-06072 | 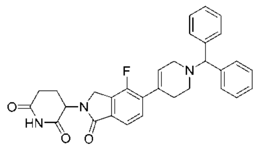 | 3-(5-(1-benzhydryl-1,2,3,6-tetrahydropyridin-4-yl)-4-fluoro-1-oxoisoindolin-2-yl)piperidine-2,6-dione | 3-(5-(1-benzhydryl-1,2,3,6-tetrahydropyridin-4-yl)-4-fluoro-1-oxoisoindolin-2-yl)piperidine-2,6-dione |
| GT-05865 | 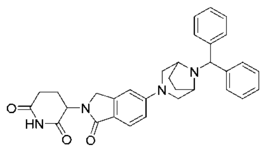 | 3-(5-(8-benzhydryl-3,8-diazabicyclo[3.2.1]octan-3-yl)-1-oxoisoindolin-2-yl)piperidine-2,6-dione | 3-(5-(8-benzhydryl-3,8-diazabicyclo[3.2.1]octan-3-yl)-1-oxoisoindolin-2-yl)piperidine-2,6-dione |
| GT-05866 | 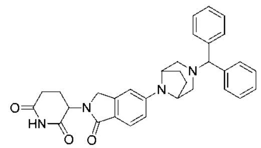 | 3-(5-(3-benzhydryl-3,8-diazabicyclo[3.2.1]octan-8-yl)-1-oxoisoindolin-2-yl)piperidine-2,6-dione | 3-(5-(3-benzhydryl-3,8-diazabicyclo[3.2.1]octan-8-yl)-1-oxoisoindolin-2-yl)piperidine-2,6-dione |
| | 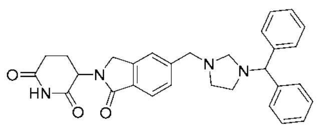 | 3-(5-((3-benzhydrylimidazolidin-1-yl)methyl)-1-oxoisoindolin-2-yl)piperidine-2,6-dione | 3-(5-((3-benzhydrylimidazolidin-1-yl)methyl)-1-oxoisoindolin-2-vl)piperidine-2,6-dione |

(continued)

| Compou nd No. | Structure of the compounds | The compounds' name | Chinese translation of the compounds' name |
|---|---|---|---|
| | 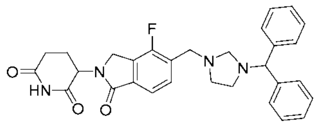 | 3-(5-((3-benzhydrylimidazolidin-1-yl)methyl)-4-fluoro-1-oxoisoindolin-2-yl)piperidine-2,6-dione | 3-(5-((3-benzhydrylimidazolidin-1-yl)methyl)-4-fluoro-1-oxoisoindolin-2-yl)piperidine-2,6-dione |
| | 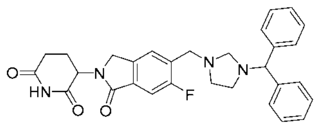 | 3-(5-((3-benzhydrylimidazolidin-1-yl)methyl)-6-fluoro-1-oxoisoindolin-2-vl)piperidine-2,6-dione | 3-(5-((3-benzhydrylimidazolidin-1-yl)methyl)-6-fluoro-1-oxoisoindolin-2-vl)piperidine-2,6-dione |
| | 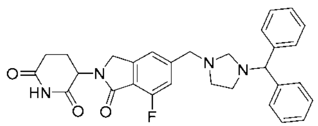 | 3-(5-((3-benzhydrylimidazolidin-1-yl)methyl)-7-fluoro-1-oxoisoindolin-2-vl)piperidine-2,6-dione | 3-(5-((3-benzhydrylimidazolidin-1-yl)methyl)-7-fluoro-1-oxoisoindolin-2-vl)piperidine-2,6-dione |
| | 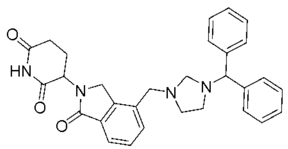 | 3-(4-((3-benzhydrylimidazolidin-1-yl)methyl)-1-oxoisoindolin-2-yl)piperidine-2,6-dione | 3-(4-((3-benzhydrylimidazolidin-1-yl)methyl)-1-oxoisoindolin-2-vl)piperidine-2,6-dione |
| GT-03994 | 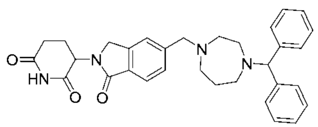 | 3-(5-((4-benzhydryl-1,4-diazepan-1-yl)methyl)-1-oxoisoindolin-2-yl)piperidine-2,6-dione | 3-(5-((4-benzhydryl-1,4-diazepan-1-yl)methyl)-1-oxoisoindolin-2-yl)piperidine-2,6-dione |
| GT-04164 | 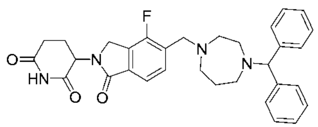 | 3-(5-((4-benzhydryl-1,4-diazepan-1-yl)methyl)-4-fluoro-1-oxoisoindolin-2-vl)piperidine-2,6-dione | 3-(5-((4-benzhydryl-1,4-diazepan-1-yl)methyl)-4-fluoro-1-oxoisoindolin-2-yl)piperidine-2,6-dione |
| GT-05738 | 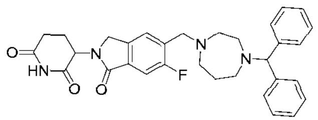 | 3-(5-((4-benzhydryl-1,4-diazepan-1-yl)methyl)-6-fluoro-1-oxoisoindolin-2-vl)piperidine-2,6-dione | 3-(5-((4-benzhydryl-1,4-diazepan-1-yl)methyl)-6-fluoro-1-oxoisoindolin-2-vl)piperidine-2,6-dione |

(continued)

| Compou nd No. | Structure of the compounds | The compounds' name | Chinese translation of the compounds' name |
|---|---|---|---|
| | 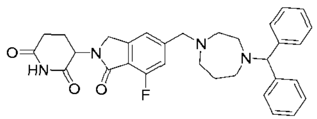 | 3-(5-((4-benzhydryl-1,4-diazepan-1-yl)methyl)-7-fluoro-1-oxoisoindolin-2-yl)piperidine-2,6-dione | 3-(5-((4-benzhydryl-1,4-diazepan-1-yl)methyl)-7-fluoro-1-oxoisoindolin-2-yl)piperidine-2,6-dione |
| | 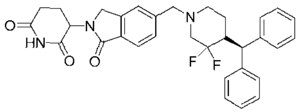 | 3-(5-(((R)-4-benzhydryl-3,3-difluoropiperidin-1-yl) methyl)-1-oxoisoindolin-2-yl)piperidine-2,6-dione | 3-(5-(((R)-4-benzhydryl-3,3-difluoropiperidin-1-yl) methyl)-1-oxoisoindolin-2-yl)piperidine-2,6-dione |
| | 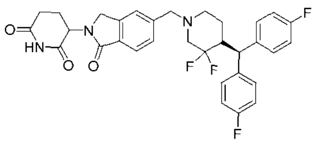 | 3-(5-(((R)-4-(bis(4-fluorophenyl)methyl)-3,3-difluoropiperidin-1-yl)methyl)-1-oxoisoindolin-2-yl)piperidine-2,6-dione | 3-(5-(((R)-4-(bis(4-fluorophenyl)methyl)-3,3-difluoropiperidin-1-yl)methyl)-1-oxoisoindolin-2-vl)piperidine-2,6-dione |
| | 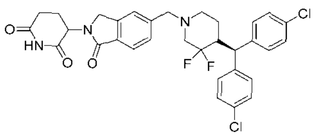 | 3-(5-(((R)-4-(bis(4-chlorophenyl)methyl)-3,3-difluoropiperidin-1-yl)methyl)-1-oxoisoindolin-2-yl)piperidine-2,6-dione | 3-(5-(((R)-4-(bis(4-chlorophenyl)methyl)-3,3-difluoropiperidin-1-yl)methyl)-1-oxoisoindolin-2-vl)piperidine-2,6-dione |
| | 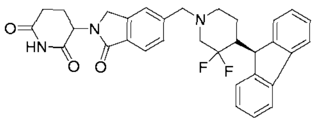 | 3-(5-(((R)-4-(9H-fluoren-9-yl)-3,3-difluoropiperidin-1-yl) methyl)-1-oxoisoindolin-2-yl)piperidine-2,6-dione | 3-(5-(((R)-4-(9H-fluoren-9-yl)-3,3-difluoropiperidin-1-yl) methyl)-1-oxoisoindolin-2-yl)piperidine-2,6-dione |
| GT-05901 | 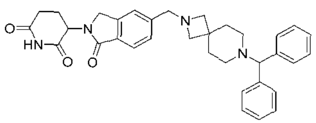 | 3-(5-((7-benzhydryl-2,7-diazaspiro[3.5]nonan-2-yl) methyl)-1-oxoisoindolin-2-yl)piperidine-2,6-dione | 3-(5-((7-benzhydryl-2,7-diazaspiro[3.5]nonan-2-yl) methyl)-1-oxoisoindolin-2-vl)piperidine-2,6-dione |
| GT-05902 | 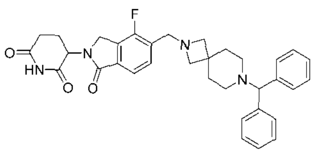 | 3-(5-((7-benzhydryl-2,7-diazaspiro[3.5]nonan-2-yl) methyl)-4-fluoro-1-oxoisoindolin-2-yl)piperidine-2,6-dione | 3-(5-((7-benzhydryl-2,7-diazaspiro[3.5]nonan-2-yl) methyl)-4-fluoro-1-oxoisoindolin-2-yl)piperidine-2,6-dione |

(continued)

| Compound No. | Structure of the compounds | The compounds' name | Chinese translation of the compounds' name |
|---|---|---|---|
| GT-05903 | 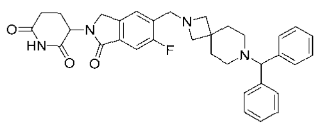 | 3-(5-((7-benzhydryl-2,7-diazaspiro[3.5]nonan-2-yl) methyl)-6-fluoro-1-oxoisoindolin-2-yl)piperidine-2,6-dione | 3-(5-((7-benzhydryl-2,7-diazaspiro[3.5]nonan-2-yl) methyl)-6-fluoro-1-oxoisoindolin-2-yl)piperidine-2,6-dione |
| GT-05904 | 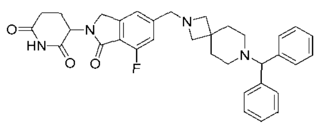 | 3-(5-((7-benzhydryl-2,7-diazaspiro[3.5]nonan-2-yl) methyl)-7-fluoro-1-oxoisoindolin-2-yl)piperidine-2,6-dione | 3-(5-((7-benzhydryl-2,7-diazaspiro[3.5]nonan-2-yl) methyl)-7-fluoro-1-oxoisoindolin-2-vl)piperidine-2,6-dione |
| GT-05905 | 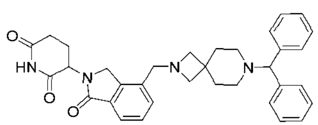 | 3-(4-((7-benzhydryl-2,7-diazaspiro[3.5]nonan-2-yl) methyl)-1-oxoisoindolin-2-yl)piperidine-2,6-dione | 3-(4-((7-benzhydryl-2,7-diazaspiro[3.5]nonan-2-yl) methyl)-1-oxoisoindolin-2-yl)piperidine-2,6-dione |
| GT-05969 | 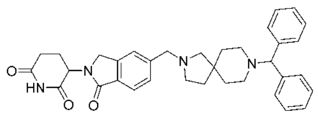 | 3-(5-((8-benzhydryl-2,8-diazaspiro[4.5]decan-2-yl) methyl)-1-oxoisoindolin-2-yl)piperidine-2,6-dione | 3-(5-((8-benzhydryl-2,8-diazaspiro[4.5]decan-2-yl) methyl)-1-oxoisoindolin-2-yl)piperidine-2,6-dione |
| GT-05970 | 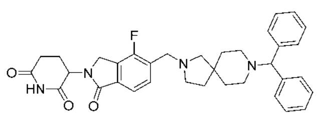 | 3-(5-((8-benzhydryl-2,8-diazaspiro[4.5]decan-2-yl) methyl)-4-fluoro-1-oxoisoindolin-2-vl)piperidine-2,6-dione | 3-(5-((8-benzhydryl-2,8-diazaspiro[4.5]decan-2-yl) methyl)-4-fluoro-1-oxoisoindolin-2-yl)piperidine-2,6-dione |
| GT-05971 | 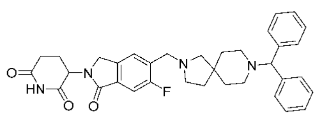 | 3-(5-((8-benzhydryl-2,8-diazaspiro[4.5]decan-2-yl) methyl)-6-fluoro-1-oxoisoindolin-2-vl)piperidine-2,6-dione | 3-(5-((8-benzhydryl-2,8-diazaspiro[4.5]decan-2-yl) methyl)-6-fluoro-1-oxoisoindolin-2-vl)piperidine-2,6-dione |
| GT-05972 | 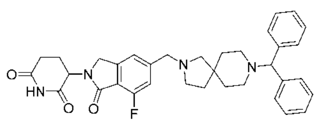 | 3-(5-((8-benzhydryl-2,8-diazaspiro[4.5]decan-2-yl) methyl)-7-fluoro-1-oxoisoindolin-2-yl)piperidine-2,6-dione | 3-(5-((8-benzhydryl-2,8-diazaspiro[4.5]decan-2-yl) methyl)-7-fluoro-1-oxoisoindolin-2-yl)piperidine-2,6-dione |
| GT-05973 | 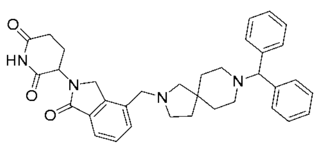 | 3-(4-((8-benzhydryl-2,8-diazaspiro[4.5]decan-2-yl) methyl)-1-oxoisoindolin-2-yl)piperidine-2,6-dione | 3-(4-((8-benzhydryl-2,8-diazaspiro[4.5]decan-2-yl) methyl)-1-oxoisoindolin-2-vl)piperidine-2,6-dione |

(continued)

| Compound No. | Structure of the compounds | The compounds' name | Chinese translation of the compounds' name |
|---|---|---|---|
| GT-06002 | 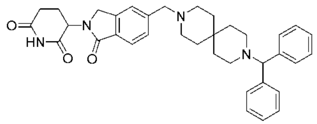 | 3-(5-((9-benzhydryl-3,9-diazaspiro[5 .5]undecan-3-yl) methyl)-1-oxoisoindolin-2-yl)piperidine-2,6-dione | 3-(5-((9-benzhydryl-3,9-diazaspiro[5.5]undecan-3-yl) methyl)-1-oxoisoindolin-2-vl)piperidine-2,6-dione |
| GT-06024 | 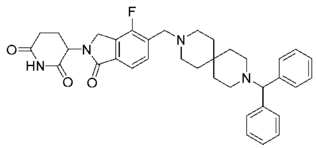 | 3-(5-((9-benzhydryl-3,9-diazaspiro[5 .5]undecan-3-yl) methyl)-4-fluoro-1-oxoisoindolin-2-vl)piperidine-2,6-dione | 3-(5-((9-benzhydryl-3,9-diazaspiro[5.5]undecan-3-yl) methyl)-4-fluoro-1-oxoisoindolin-2-vl)piperidine-2,6-dione |
| GT-06025 | 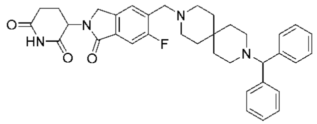 | 3-(5-((9-benzhydryl-3,9-diazaspiro[5 .5]undecan-3-yl) methyl)-6-fluoro-1-oxoisoindolin-2-yl)piperidine-2,6-dione | 3-(5-((9-benzhydryl-3,9-diazaspiro[5.5]undecan-3-yl) methyl)-6-fluoro-1-oxoisoindolin-2-yl)piperidine-2,6-dione |
| GT-06026 | 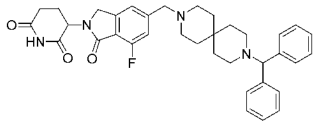 | 3-(5-((9-benzhydryl-3,9-diazaspiro[5 .5]undecan-3-yl) methyl)-7-fluoro-1-oxoisoindolin-2-vl)piperidine-2,6-dione | 3-(5-((9-benzhydryl-3,9-diazaspiro[5.5]undecan-3-yl) methyl)-7-fluoro-1-oxoisoindolin-2-vl)piperidine-2,6-dione |
| GT-06027 | 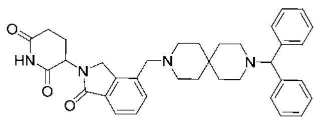 | 3-(4-((9-benzhydryl-3,9-diazaspiro[5 .5]undecan-3-yl) methyl)-1-oxoisoindolin-2-yl)piperidine-2,6-dione | 3-(4-((9-benzhydryl-3,9-diazaspiro[5.5]undecan-3-yl) methyl)-1-oxoisoindolin-2-vl)piperidine-2,6-dione |
| GT-06050 | 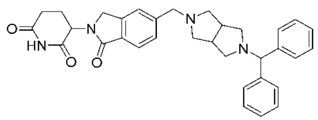 | 3-(5-((5-benzhydrylhexahydropyrrolo [3,4-c]pyrrol-2(l-H)-yl)methyl)-1-oxoisoindolin-2-yl)piperidine-2,6-dione | 3-(5-((5-benzhydrylhexahydropyrrol o[3,4-c]pyrrol-2(1H)-yl)methyl)-1-oxoisoindolin-2-vl)piperidine-2,6-dione |
| GT-06044 | 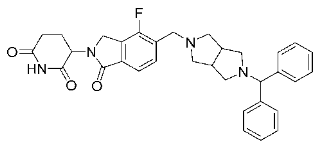 | 3-(5-((5-benzhydrylhexahydropyrrolo [3,4-c]pyrrol-2(1H)-yl)methyl)-4-fluoro-1-oxoisoindolin-2-yl)piperidine-2,6-dione | 3-(5-((5-benzhydrylhexahydropyrrol o[3,4-c]pyrrol-2(1H)-yl)methyl)-4-fluoro-1-oxoisoindolin-2-yl)piperi-dine-2,6-dione |

(continued)

| Compou nd No. | Structure of the compounds | The compounds' name | Chinese translation of the compounds' name |
|---|---|---|---|
| GT-06051 | 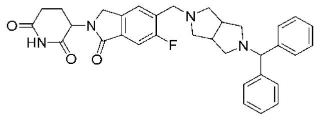 | 3-(5-((5-benzhydrylhexahydropyrrolo [3,4-c]pyrrol-2(1H)-yl)methyl)-6-fluoro-1-oxoisoindolin-2-yl)piperidine-2,6-dione | 3-(5-((5-benzhydrylhexahydropyrrol o[3,4-c]pyrrol-2(1H)-yl)methyl)-6-fluoro-1-oxoisoindolin-2-yl)piperidine-2,6-dione |
| GT-06045 | 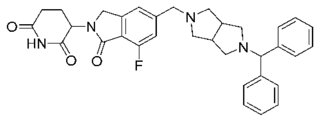 | 3-(5-((5-benzhydrylhexahydropyrrolo [3,4-c]pyrrol-2(1H)-yl)methyl)-7-fluoro-1-oxoisoindolin-2-yl)piperidine-2,6-dione | 3-(5-((5-benzhydrylhexahydropyrrol o[3,4-c]pyrrol-2(1H)-yl)methyl)-7-fluoro-1-oxoisoindolin-2-yl)piperidine-2,6-dione |
| GT-06052 | 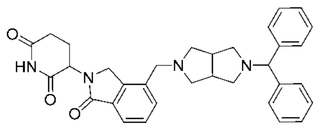 | 3-(4-((5-benzhydrylhexahydropyrrolo [3,4-c]pyrrol-2(1H)-yl)methyl)-1-oxoisoindolin-2-yl)piperidine-2,6-dione | 3-(4-((5-benzhydrylhexahydropyrrol o[3,4-c]pyrrol-2(1H)-yl)methyl)-1-oxoisoindolin-2-yl)piperidine-2,6-dione |
| GT-03569 | 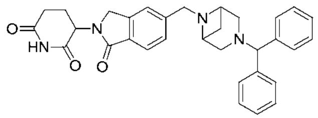 | 3-(5-((3-benzhydryl-3,6-diazabicyclo [3 .1.1]heptan-6-yl) methyl)-1-oxoisoindolin-2-yl)piperidine-2,6-dione | 3-(5-((3-benzhydryl-3,6-diazabicyclo[3.1.1]heptan-6-yl) methyl)-1-oxoisoindolin-2-yl)piperidine-2,6-dione |
| GT-03572 | 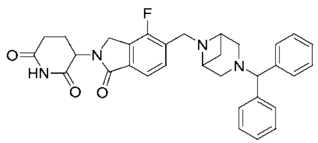 | 3-(5-((3-benzhydryl-3,6-diazabicyclo [3 .1.1]heptan-6-yl) methyl)-4-fluoro-1-oxoisoindolin-2-yl)piperidine-2,6-dione | 3-(5-((3-benzhydryl-3,6-diazabicyclo[3.1.1]heptan-6-yl) methyl)-4-fluoro-1-oxoisoindolin-2-yl)piperidine-2,6-dione |
| GT-05737 | 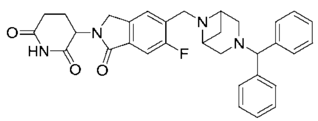 | 3-(5-((3-benzhydryl-3,6-diazabicyclo [3 .1.1]heptan-6-yl) methyl)-6-fluoro-1-oxoisoindolin-2-yl)piperidine-2,6-dione | 3-(5-((3-benzhydryl-3,6-diazabicyclo[3.1.1]heptan-6-yl) methyl)-6-fluoro-1-oxoisoindolin-2-yl)piperidine-2,6-dione |
| GT-03575 | 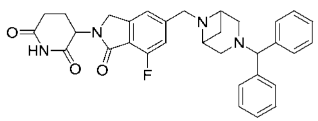 | 3-(5-((3-benzhydryl-3,6-diazabicyclo [3 .1.1]heptan-6-yl) methyl)-7-fluoro-1-oxoisoindolin-2-yl)piperidine-2,6-dione | 3-(5-((3-benzhydryl-3,6-diazabicyclo[3.1.1]heptan-6-yl) methyl)-7-fluoro-1-oxoisoindolin-2-yl)piperidine-2,6-dione |

(continued)

| Compound No. | Structure of the compounds | The compounds' name | Chinese translation of the compounds' name |
|---|---|---|---|
| GT-03570 | 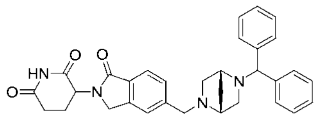 | 3-(5-(((1R,4R)-5-benzhydryl-2,5-diazabicyclo[2.2.1]heptan-2-yl)methyl)-1-oxoisoindolin-2-yl)piperidine-2,6-dione | 3-(5-(((1R,4R)-5-benzhydryl-2,5-diazabicyclo[2.2.1]heptan-2-yl)methyl)-1-oxoisoindolin-2-yl)piperidine-2,6-dione |
| GT-03573 | 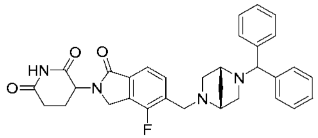 | 3-(5-(((1R,4R)-5-benzhydryl-2,5-diazabicyclo[2.2.1]heptan-2-yl)methyl)-4-fluoro-1-oxoisoindolin-2-yl)piperidine-2,6-dione | 3-(5-(((1R,4R)-5-benzhydryl-2,5-diazabicyclo[2.2.1]heptan-2-yl)methyl)-4-fluoro-1-oxoisoindolin-2-yl)piperidine-2,6-dione |
| GT-05958 | 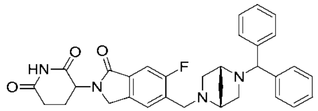 | 3-(5-(((1R,4R)-5-benzhydryl-2,5-diazabicyclo[2.2.1]heptan-2-yl)methyl)-6-fluoro-1-oxoisoindolin-2-yl)piperidine-2,6-dione | 3-(5-(((1R,4R)-5-benzhydryl-2,5-diazabicyclo[2.2.1]heptan-2-yl)methyl)-6-fluoro-1-oxoisoindolin-2-yl)piperidine-2,6-dione |
| GT-03576 | 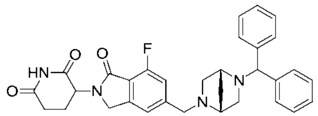 | 3-(5-(((1R,4R)-5-benzhydryl-2,5-diazabicyclo[2.2.1]heptan-2-yl)methyl)-7-fluoro-1-oxoisoindolin-2-yl)piperidine-2,6-dione | 3-(5-(((1R,4R)-5-benzhydryl-2,5-diazabicyclo[2.2.1]heptan-2-yl)methyl)-7-fluoro-1-oxoisoindolin-2-yl)piperidine-2,6-dione |
| GT-03721 | 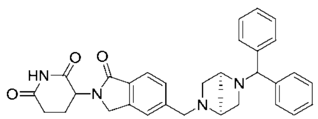 | 3-(5-(((1S,4S)-5-benzhydryl-2,5-diazabicyclo[2.2.1]heptan-2-yl)methyl)-1-oxoisoindolin-2-yl)piperidine-2,6-dione | 3-(5-(((1S,4S)-5-benzhydryl-2,5-diazabicyclo[2.2.1]heptan-2-yl)methyl)-1-oxoisoindolin-2-yl)piperidine-2,6-dione |
| GT-03724 | 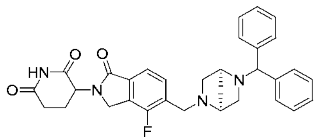 | 3-(5-(((1S,4S)-5-benzhydryl-2,5-diazabicyclo[2.2.1]heptan-2-yl)methyl)-4-fluoro-1-oxoisoindolin-2-yl)piperidine-2,6-dione | 3-(5-(((1S,4S)-5-benzhydryl-2,5-diazabicyclo[2.2.1]heptan-2-yl)methyl)-4-fluoro-1-oxoisoindolin-2-yl)piperidine-2,6-dione |
| GT-05894 | 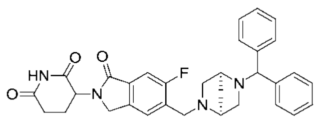 | 3-(5-(((1S,4S)-5-benzhydryl-2,5-diazabicyclo[2.2.1]heptan-2-yl)methyl)-6-fluoro-1-oxoisoindolin-2-yl)piperidine-2,6-dione | 3-(5-(((1S,4S)-5-benzhydryl-2,5-diazabicyclo[2.2.1]heptan-2-yl)methyl)-6-fluoro-1-oxoisoindolin-2-yl)piperidine-2,6-dione |

(continued)

| Compound No. | Structure of the compounds | The compounds' name | Chinese translation of the compounds' name |
|---|---|---|---|
| GT-05895 | 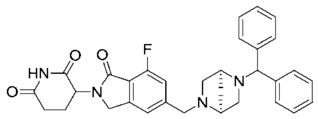 | 3-(5-(((1S,4S)-5-benzhydryl-2,5-diazabicyclo[2.2.1]heptan-2-yl)methyl)-7-fluoro-1-oxoisoindolin-2-yl)piperidine-2,6-dione | 3-(5-(((1S,4S)-5-benzhydryl-2,5-diazabicyclo[2.2.1]heptan-2-yl)methyl)-7-fluoro-1-oxoisoindolin-2-yl)piperidine-2,6-dione |
| GT-03837 | 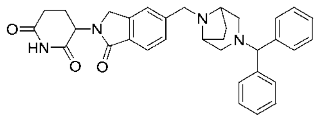 | 3-(5-((3-benzhydryl-3,8-diazabicyclo[3.2.1]octan-8-yl)methyl)-1-oxoisoindolin-2-yl)piperidine-2,6-dione | 3-(5-((3-benzhydryl-3,8-diazabicyclo[3.2.1]octan-8-yl)methyl)-1-oxoisoindolin-2-yl)piperidine-2,6-dione |
| GT-03838 | 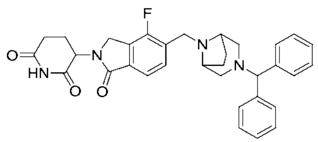 | 3-(5-((3-benzhydryl-3,8-diazabicyclo[3.2.1]octan-8-yl)methyl)-4-fluoro-1-oxoisoindolin-2-yl)piperidine-2,6-dione | 3-(5-((3-benzhydryl-3,8-diazabicyclo[3.2.1]octan-8-yl)methyl)-4-fluoro-1-oxoisoindolin-2-yl)piperidine-2,6-dione |
| GT-03839 | 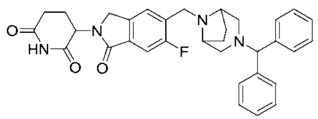 | 3-(5-((3-benzhydryl-3,8-diazabicyclo[3.2.1]octan-8-yl)methyl)-6-fluoro-1-oxoisoindolin-2-yl)piperidine-2,6-dione | 3-(5-((3-benzhydryl-3,8-diazabicyclo[3.2.1]octan-8-yl)methyl)-6-fluoro-1-oxoisoindolin-2-yl)piperidine-2,6-dione |
| GT-03840 | 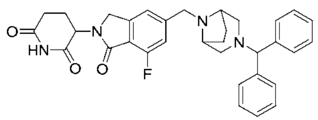 | 3-(5-((3-benzhydryl-3,8-diazabicyclo[3.2.1]octan-8-yl)methyl)-7-fluoro-1-oxoisoindolin-2-yl)piperidine-2,6-dione | 3-(5-((3-benzhydryl-3,8-diazabicyclo[3.2.1]octan-8-yl)methyl)-7-fluoro-1-oxoisoindolin-2-yl)piperidine-2,6-dione |
| GT-03722 | 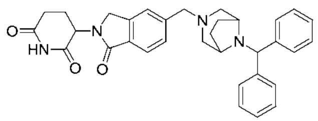 | 3-(5-((8-benzhydryl-3,8-diazabicyclo[3.2.1]octan-3-yl)methyl)-1-oxoisoindolin-2-yl)piperidine-2,6-dione | 3-(5-((8-benzhydryl-3,8-diazabicyclo[3.2.1]octan-3-yl)methyl)-1-oxoisoindolin-2-yl)piperidine-2,6-dione |
| GT-03725 | 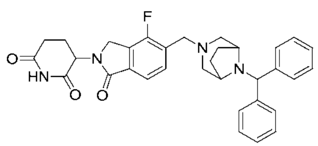 | 3-(5-((8-benzhydryl-3,8-diazabicyclo[3.2.1]octan-3-yl)methyl)-4-fluoro-1-oxoisoindolin-2-yl)piperidine-2,6-dione | 3-(5-((8-benzhydryl-3,8-diazabicyclo[3.2.1]octan-3-yl)methyl)-4-fluoro-1-oxoisoindolin-2-yl)piperidine-2,6-dione |

(continued)

| Compound No. | Structure of the compounds | The compounds' name | Chinese translation of the compounds' name |
|---|---|---|---|
| GT-05896 | 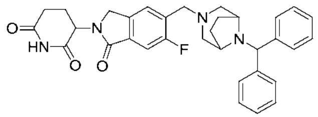 | 3-(5-((8-benzhydryl-3,8-diazabicyclo[3.2.1]octan-3-yl) methyl)-6-fluoro-1-oxoisoindolin-2-yl)piperidine-2,6-dione | 3-(5-((8-benzhydryl-3,8-diazabicyclo[3.2.1]octan-3-yl) methyl)-6-fluoro-1-oxoisoindolin-2-yl)piperidine-2,6-dione |
| GT-05897 | 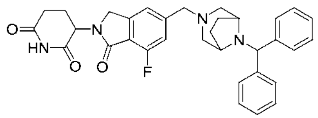 | 3-(5-((8-benzhydryl-3,8-diazabicyclo[3.2.1]octan-3-yl) methyl)-7-fluoro-1-oxoisoindolin-2-yl)piperidine-2,6-dione | 3-(5-((8-benzhydryl-3,8-diazabicyclo[3.2.1]octan-3-yl) methyl)-7-fluoro-1-oxoisoindolin-2-yl)piperidine-2,6-dione |
| GT-03571 | 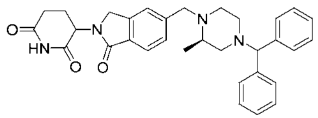 | 3-(5-(((R)-4-benzhydryl-2-methylpiperazin-1-yl)methyl)-1-oxoisoindolin-2-yl)piperidine-2,6-dione | 3-(5-(((R)-4-benzhydryl-2-methylpiperazin-1-yl)methyl)-1-oxoisoindolin-2-yl)piperidine-2,6-dione |
| GT-03574 | 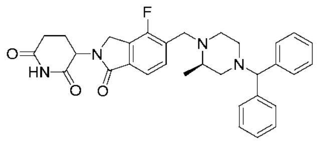 | 3-(5-(((R)-4-benzhydryl-2-methylpiperazin-1-yl)methyl)-4-fluoro-1-oxoisoindolin-2-yl)piperidine-2,6-dione | 3-(5-(((R)-4-benzhydryl-2-methylpiperazin-1-yl)methyl)-4-fluoro-1-oxoisoindolin-2-yl)piperidine-2,6-dione |
| GT-06046 | 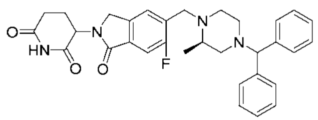 | 3-(5-(((R)-4-benzhydryl-2-methylpiperazin-1-yl)methyl)-6-fluoro-1-oxoisoindolin-2-yl)piperidine-2,6-dione | 3-(5-(((R)-4-benzhydryl-2-methylpiperazin-1-yl)methyl)-6-fluoro-1-oxoisoindolin-2-yl)piperidine-2,6-dione |
| GT-03577 | 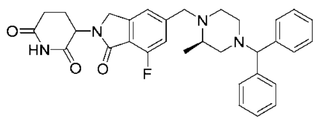 | 3-(5-(((R)-4-benzhydryl-2-methylpiperazin-1-yl)methyl)-7-fluoro-1-oxoisoindolin-2-yl)piperidine-2,6-dione | 3-(5-(((R)-4-benzhydryl-2-methylpiperazin-1-yl)methyl)-7-fluoro-1-oxoisoindolin-2-yl)piperidine-2,6-dione |
| GT-03723 | 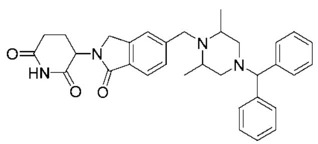 | 3-(5-((4-benzhydryl-2,6-dimethylpiperazin-1-yl)methyl)-1-oxoisoindolin-2-yl)piperidine-2,6-dione | 3-(5-((4-benzhydryl-2,6-dimethylpiperazin-1-yl)methyl)-1-oxoisoindolin-2-yl)piperidine-2,6-dione |

(continued)

| Compound No. | Structure of the compounds | The compounds' name | Chinese translation of the compounds' name |
|---|---|---|---|
| GT-03726 | 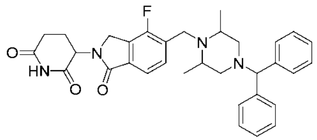 | 3-(5-((4-benzhydryl-2,6-dimethylpiperazin-1-yl)methyl)-4-fluoro-1-oxoisoindolin-2-yl)piperidine-2,6-dione | 3-(5-((4-benzhydryl-2,6-dimethylpiperazin-1-yl)methyl)-4-fluoro-1-oxoisoindolin-2-yl)piperidine-2,6-dione |
| GT-03873 | 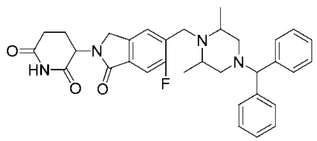 | 3-(5-((4-benzhydryl-2,6-dimethylpiperazin-1-yl)methyl)-6-fluoro-1-oxoisoindolin-2-yl)piperidine-2,6-dione | 3-(5-((4-benzhydryl-2,6-dimethylpiperazin-1-yl)methyl)-6-fluoro-1-oxoisoindolin-2-yl)piperidine-2,6-dione |
| GT-03874 | 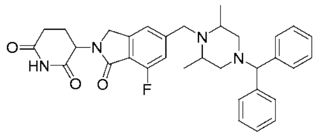 | 3-(5-((4-benzhydryl-2,6-dimethylpiperazin-1-yl)methyl)-7-fluoro-1-oxoisoindolin-2-yl)piperidine-2,6-dione | 3-(5-((4-benzhydryl-2,6-dimethylpiperazin-1-yl)methyl)-7-fluoro-1-oxoisoindolin-2-yl)piperidine-2,6-dione |
| GT-03872 | 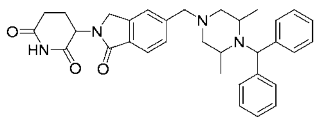 | 3-(5-((4-benzhydryl-3,5-dimethylpiperazin-1-yl)methyl)-1-oxoisoindolin-2-yl)piperidine-2,6-dione | 3-(5-((4-benzhydryl-3,5-dimethylpiperazin-1-yl)methyl)-1-oxoisoindolin-2-yl)piperidine-2,6-dione |
| GT-05965 | 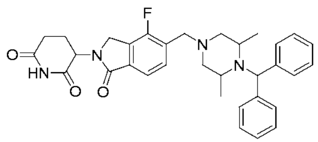 | 3-(5-((4-benzhydryl-3,5-dimethylpiperazin-1-yl)methyl)-4-fluoro-1-oxoisoindolin-2-yl)piperidine-2,6-dione | 3-(5-((4-benzhydryl-3,5-dimethylpiperazin-1-yl)methyl)-4-fluoro-1-oxoisoindolin-2-yl)piperidine-2,6-dione |
| GT-05966 | 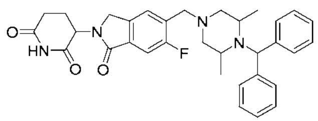 | 3-(5-((4-benzhydryl-3,5-dimethylpiperazin-1-yl)methyl)-6-fluoro-1-oxoisoindolin-2-yl)piperidine-2,6-dione | 3-(5-((4-benzhydryl-3,5-dimethylpiperazin-1-yl)methyl)-6-fluoro-1-oxoisoindolin-2-yl)piperidine-2,6-dione |
| GT-05967 | 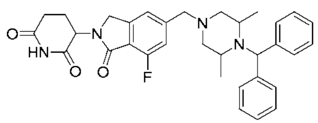 | 3-(5-((4-benzhydryl-3,5-dimethylpiperazin-1-yl)methyl)-7-fluoro-1-oxoisoindolin-2-yl)piperidine-2,6-dione | 3-(5-((4-benzhydryl-3,5-dimethylpiperazin-1-yl)methyl)-7-fluoro-1-oxoisoindolin-2-yl)piperidine-2,6-dione |

(continued)

| Compound No. | Structure of the compounds | The compounds' name | Chinese translation of the compounds' name |
|---|---|---|---|
| GT-03993 | 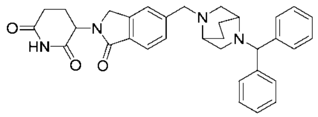 | 3-(5-((5-benzhydryl-2,5-diazabicyclo[2.2.2]octan-2-yl) methyl)-1-oxoisoindolin-2-yl)piperidine-2,6-dione | 3-(5-((5-benzhydryl-2,5-diazabicyclo[2.2.2]octan-2-yl) methyl)-1-oxoisoindolin-2-yl)piperidine-2,6-dione |
| GT-04163 | 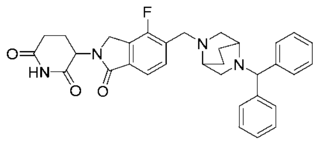 | 3-(5-((5-benzhydryl-2,5-diazabicyclo[2.2.2]octan-2-yl) methyl)-4-fluoro-1-oxoisoindolin-2-yl)piperidine-2,6-dione | 3-(5-((5-benzhydryl-2,5-diazabicyclo[2.2.2]octan-2-yl) methyl)-4-fluoro-1-oxoisoindolin-2-yl)piperidine-2,6-dione |
| GT-05963 | 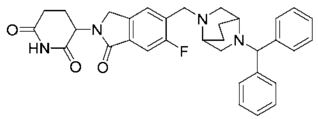 | 3-(5-((5-benzhydryl-2,5-diazabicyclo[2.2.2]octan-2-yl) methyl)-6-fluoro-1-oxoisoindolin-2-yl)piperidine-2,6-dione | 3-(5-((5-benzhydryl-2,5-diazabicyclo[2.2.2]octan-2-yl) methyl)-6-fluoro-1-oxoisoindolin-2-yl)piperidine-2,6-dione |
| GT-05964 | 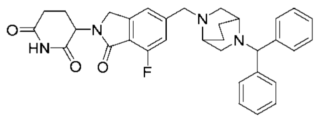 | 3-(5-((5-benzhydryl-2,5-diazabicyclo[2.2.2]octan-2-yl) methyl)-7-fluoro-1-oxoisoindolin-2-yl)piperidine-2,6-dione | 3-(5-((5-benzhydryl-2,5-diazabicyclo[2.2.2]octan-2-yl) methyl)-7-fluoro-1-oxoisoindolin-2-yl)piperidine-2,6-dione |
| | 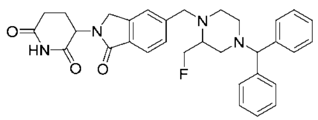 | 3-(5-((4-benzhydryl-2-(fluoromethyl)piperazin-1-yl) methyl)-1-oxoisoindolin-2-yl)piperidine-2,6-dione | 3-(5-((4-benzhydryl-2-(fluoromethyl)piperazin-1-yl) methyl)-1-oxoisoindolin-2-yl)piperidine-2,6-dione |
| | 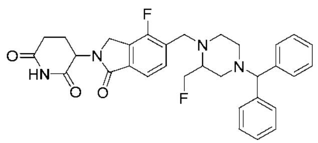 | 3-(5-((4-benzhydryl-2-(fluoromethyl)piperazin-1-yl) methyl)-4-fluoro-1-oxoisoindolin-2-yl)piperidine-2,6-dione | 3-(5-((4-benzhydryl-2-(fluoromethyl)piperazin-1-yl) methyl)-4-fluoro-1-oxoisoindolin-2-yl)piperidine-2,6-dione |
| | 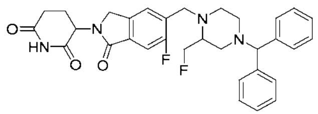 | 3-(5-((4-benzhydryl-2-(fluoromethyl)piperazin-1-yl) methyl)-6-fluoro-1-oxoisoindolin-2-yl)piperidine-2,6-dione | 3-(5-((4-benzhydryl-2-(fluoromethyl)piperazin-1-yl) methyl)-6-fluoro-1-oxoisoindolin-2-yl)piperidine-2,6-dione |

(continued)

| Compou nd No. | Structure of the compounds | The compounds' name | Chinese translation of the compounds' name |
|---|---|---|---|
| | 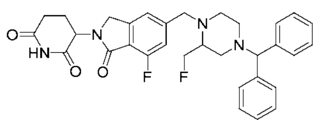 | 3-(5-((4-benzhydryl-2-(fluoromethyl)piperazin-1-yl) methyl)-7-fluoro-1-oxoisoindolin-2-yl)piperidine-2,6-dione | 3-(5-((4-benzhydryl-2-(fluoromethyl)piperazin-1-yl) methyl)-7-fluoro-1-oxoisoindolin-2-yl)piperidine-2,6-dione |
| GT-06095 | 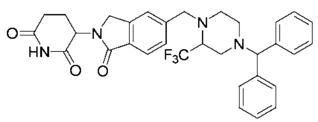 | 3-(5-((4-benzhydryl-2-(trifluoromethyl)piperazin-1-yl) methyl)-1-oxoisoindolin-2-yl)piperidine-2,6-dione | 3-(5-((4-benzhydryl-2-(trifluoromethyl)piperazin-1-yl) methyl)-1-oxoisoindolin-2-yl)piperidine-2,6-dione |
| GT-06096 | 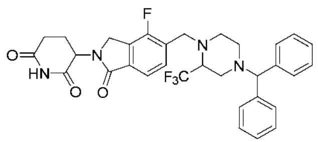 | 3-(5-((4-benzhydryl-2-(trifluoromethyl)piperazin-1-yl) methyl)-4-fluoro-1-oxoisoindolin-2-yl)piperidine-2,6-dione | 3-(5-((4-benzhydryl-2-(trifluoromethyl)piperazin-1-yl) methyl)-4-fluoro-1-oxoisoindolin-2-yl)piperidine-2,6-dione |
| GT-06097 | 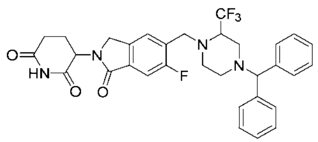 | 3-(5-((4-benzhydryl-2-(trifluoromethyl)piperazin-1-yl) methyl)-6-fluoro-1-oxoisoindolin-2-yl)piperidine-2,6-dione | 3-(5-((4-benzhydryl-2-(trifluoromethyl)piperazin-1-yl) methyl)-6-fluoro-1-oxoisoindolin-2-yl)piperidine-2,6-dione |
| GT-06098 | 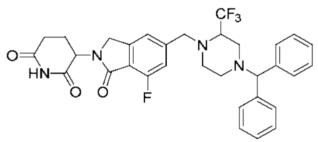 | 3-(5-((4-benzhydryl-2-(trifluoromethyl)piperazin-1-yl) methyl)-7-fluoro-1-oxoisoindolin-2-yl)piperidine-2,6-dione | 3-(5-((4-benzhydryl-2-(trifluoromethyl)piperazin-1-yl) methyl)-7-fluoro-1-oxoisoindolin-2-yl)piperidine-2,6-dione |
| GT-04165 | 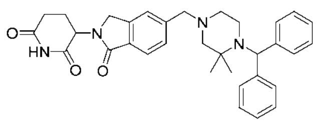 | 3-(5-((4-benzhydryl-3,3-dimethylpiperazin-1-yl)methyl)-1-oxoisoindolin-2-yl)piperidine-2,6-dione | 3-(5-((4-benzhydryl-3,3-dimethylpiperazin-1-yl)methyl)-1-oxoisoindolin-2-yl)piperidine-2,6-dione |
| GT-04166 | 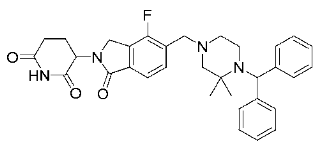 | 3-(5-((4-benzhydryl-3,3-dimethylpiperazin-1-yl)methyl)-4-fluoro-1-oxoisoindolin-2-yl)piperidine-2,6-dione | 3-(5-((4-benzhydryl-3,3-dimethylpiperazin-1-yl)methyl)-4-fluoro-1-oxoisoindolin-2-yl)piperidine-2,6-dione |

(continued)

| Compound No. | Structure of the compounds | The compounds' name | Chinese translation of the compounds' name |
|---|---|---|---|
| GT-05960 | 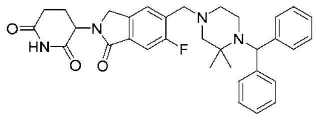 | 3-(5-((4-benzhydryl-3,3-dimethylpiperazin-1-yl)methyl)-6-fluoro-1-oxoisoindolin-2-yl)piperidine-2,6-dione | 3-(5-((4-benzhydryl-3,3-dimethylpiperazin-1-yl)methyl)-6-fluoro-1-oxoisoindolin-2-yl)piperidine-2,6-dione |
| GT-05961 | 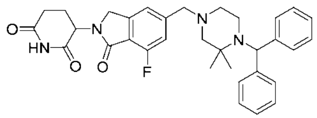 | 3-(5-((4-benzhydryl-3,3-dimethylpiperazin-1-yl)methyl)-7-fluoro-1-oxoisoindolin-2-yl)piperidine-2,6-dione | 3-(5-((4-benzhydryl-3,3-dimethylpiperazin-1-yl)methyl)-7-fluoro-1-oxoisoindolin-2-yl)piperidine-2,6-dione |
| | 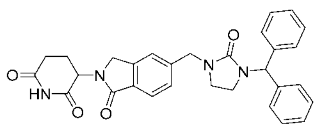 | 3-(5-((3-benzhydryl-2-oxoimidazolidin-1-yl)methyl)-1-oxoisoindolin-2-yl)piperidine-2,6-dione | 3-(5-((3-benzhydryl-2-oxoimidazolidin-1-yl)methyl)-1-oxoisoindolin-2-yl)piperidine-2,6-dione |
| | 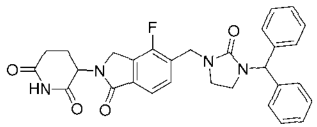 | 3-(5-((3-benzhydryl-2-oxoimidazolidin-1-yl)methyl)-4-fluoro-1-oxoisoindolin-2-yl)piperidine-2,6-dione | 3-(5-((3-benzhydryl-2-oxoimidazolidin-1-yl)methyl)-4-fluoro-1-oxoisoindolin-2-yl)piperidine-2,6-dione |
| | 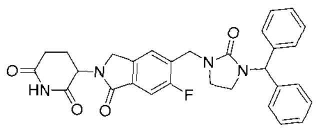 | 3-(5-((3-benzhydryl-2-oxoimidazolidin-1-yl)methyl)-6-fluoro-1-oxoisoindolin-2-yl)piperidine-2,6-dione | 3-(5-((3-benzhydryl-2-oxoimidazolidin-1-yl)methyl)-6-fluoro-1-oxoisoindolin-2-yl)piperidine-2,6-dione |
| | 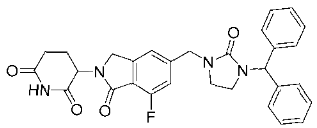 | 3-(5-((3-benzhydryl-2-oxoimidazolidin-1-yl)methyl)-7-fluoro-1-oxoisoindolin-2-yl)piperidine-2,6-dione | 3-(5-((3-benzhydryl-2-oxoimidazolidin-1-yl)methyl)-7-fluoro-1-oxoisoindolin-2-yl)piperidine-2,6-dione |
| | 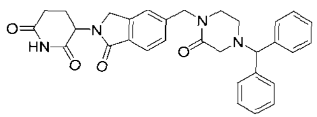 | 3-(5-((4-benzhydryl-2-oxopiperazin-1-yl)methyl)-1-oxoisoindolin-2-yl)piperidine-2,6-dione | 3-(5-((4-benzhydryl-2-oxopiperazin-1-yl)methyl)-1-oxoisoindolin-2-yl)piperidine-2,6-dione |

(continued)

| Compound No. | Structure of the compounds | The compounds' name | Chinese translation of the compounds' name |
|---|---|---|---|
| GT-03835 | 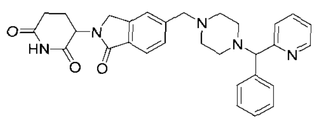 | 3-(1-oxo-5-((4-(phenyl(pyridin-2-yl)methyl)piperazin-1-yl) methyl)isoindolin-2-yl)piperidine-2,6-dione | 3-(1-oxo-5-((4-(phenyl(pyridin-2-yl)methyl)piperazin-1-yl) methyl)isoindolin-2-yl)piperidine-2,6-dione |
| GT-04523 | 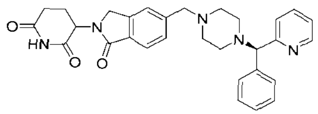 | 3-(1-oxo-5-((4-((R)-phenyl(pyridin-2-yl)methyl)piperazin-1-yl)methyl)isoindolin-2-yl)piperidine-2,6-dione | 3-(1-oxo-5-((4-((R)-phenyl(pyridin-2-yl)methyl)piperazin-1-yl)methyl)isoindolin-2-yl)piperidine-2,6-dione |
| GT-04524 | 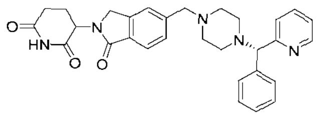 | 3-(1-oxo-5-((4-((S)-phenyl(pyridin-2-yl)methyl)piperazin-1-yl)methyl)isoindolin-2-yl)piperidine-2,6-dione | 3-(1-oxo-5-((4-((S)-phenyl(pyridin-2-yl)methyl)piperazin-1-yl)methyl)isoindolin-2-yl)piperidine-2,6-dione |
| GT-03832 | 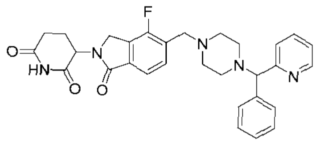 | 3-4-fluoro-1-oxo-5-((4-(phenyl(pyridin-2-yl)methyl)piperazin-1-yl)methyl)isoindolin-2-yl)piperidine-2,6-dione | 3-(4-fluoro-1-oxo-5-((4-(phenyl(pyridin-2-yl)methyl)piperazin-1-yl)methyl)isoindolin-2-yl)piperidine-2,6-dione |
| GT-03833 | 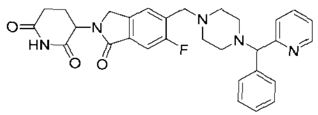 | 3-(6-fluoro-1-oxo-5-((4-(phenyl(pyridin-2-yl)methyl)piperazin-1-yl)methyl)isoindolin-2-yl)piperidine-2,6-dione | 3-(6-fluoro-1-oxo-5-((4-(phenyl(pyridin-2-yl)methyl)piperazin-1-yl)methyl)isoindolin-2-yl)piperidine-2,6-dione |
| GT-03834 | 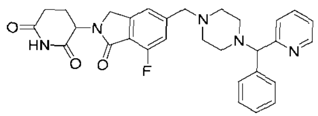 | 3-(7-fluoro-1-oxo-5-((4-(phenyl(pyridin-2-yl)methyl)piperazin-1-yl)methyl)isoindolin-2-yl)piperidine-2,6-dione | 3-(7-fluoro-1-oxo-5-((4-(phenyl(pyridin-2-yl)methyl)piperazin-1-yl)methyl)isoindolin-2-yl)piperidine-2,6-dione |
| GT-03836 | 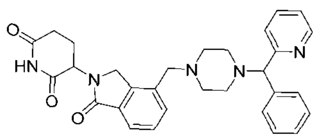 | 3-(1-oxo-4-((4-(phenyl(pyridin-2-yl)methyl)piperazin-1-yl) methyl)isoindolin-2-yl)piperidine-2,6-dione | 3-(1-oxo-4-((4-(phenyl(pyridin-2-yl)methyl)piperazin-1-yl) methyl)isoindolin-2-yl)piperidine-2,6-dione |
| GT-04249 | 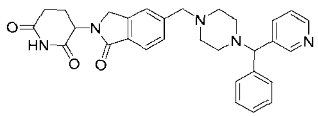 | 3-(1-oxo-5-((4-(phenyl(pyridin-3-yl)methyl)piperazin-1-yl) methyl)isoindolin-2- | 3-(1-oxo-5-((4-(phenyl(pyridin-3-yl)methyl)piperazin-1-yl) methyl)isoindolin-2- |

(continued)

| Compound No. | Structure of the compounds | The compounds' name | Chinese translation of the compounds' name |
|---|---|---|---|
| | | yl)piperidine-2,6-dione | yl)piperidine-2,6-dione |
| GT-04250 | 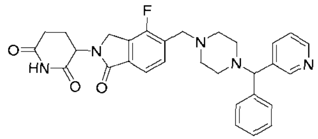 | 3-(4-fluoro-1-oxo-5-((4-(phenyl(pyridin-3-yl)methyl)piperazin-1-yl)methyl)isoindolin-2-yl)piperidine-2,6-dione | 3-(4-fluoro-1-oxo-5-((4-(phenyl(pyridin-3-yl)methyl)piperazin-1-yl)methyl)isoindolin-2-yl)piperidine-2,6-dione |
| GT-04251 | 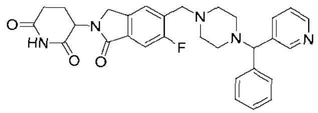 | 3-(6-fluoro-1-oxo-5-((4-(phenyl(pyridin-3-yl)methyl)piperazin-1-yl)methyl)isoindolin-2-yl)piperidine-2,6-dione | 3-(6-fluoro-1-oxo-5-((4-(phenyl(pyridin-3-yl)methyl)piperazin-1-yl)methyl)isoindolin-2-yl)piperidine-2,6-dione |
| GT-06111 | 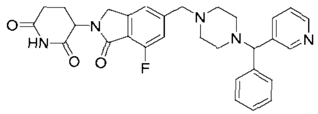 | 3-(7-fluoro-1-oxo-5-((4-(phenyl(pyridin-3-yl)methyl)piperazin-1-yl)methyl)isoindolin-2-yl)piperidine-2,6-dione | 3-(7-fluoro-1-oxo-5-((4-(phenyl(pyridin-3-yl)methyl)piperazin-1-yl)methyl)isoindolin-2-yl)piperidine-2,6-dione |
| GT-06113 | 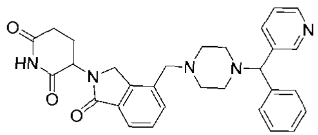 | 3-(1-oxo-4-((4-(phenyl(pyridin-3-yl)methyl)piperazin-1-yl)methyl)isoindolin-2-yl)piperidine-2,6-dione | 3-(1-oxo-4-((4-(phenyl(pyridin-3-yl)methyl)piperazin-1-yl)methyl)isoindolin-2-yl)piperidine-2,6-dione |
| GT-04205 | 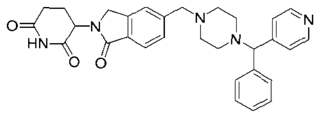 | 3-(1-oxo-5-((4-(phenyl(pyridin-4-yl)methyl)piperazin-1-yl)methyl)isoindolin-2-yl)piperidine-2,6-dione | 3-(1-oxo-5-((4-(phenyl(pyridin-4-yl)methyl)piperazin-1-yl)methyl)isoindolin-2-yl)piperidine-2,6-dione |
| GT-04247 | 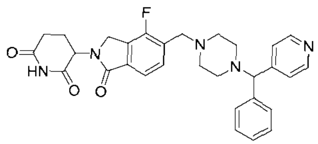 | 3-(4-fluoro-1-oxo-5-((4-(phenyl(pyridin-4-yl)methyl)piperazin-1-yl)methyl)isoindolin-2-yl)piperidine-2,6-dione | 3-(4-fluoro-1-oxo-5-((4-(phenyl(pyridin-4-yl)methyl)piperazin-1-yl)methyl)isoindolin-2-yl)piperidine-2,6-dione |
| GT-04248 | 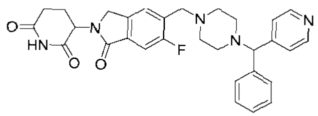 | 3-(6-fluoro-1-oxo-5-((4-(phenyl(pyridin-4-yl)methyl)piperazin-1-yl)methyl)isoindolin-2-yl)piperidine-2,6-dione | 3-(6-fluoro-1-oxo-5-((4-(phenyl(pyridin-4-yl)methyl)piperazin-1-yl)methyl)isoindolin-2-yl)piperidine-2,6-dione |

(continued)

| Compound No. | Structure of the compounds | The compounds' name | Chinese translation of the compounds' name |
|---|---|---|---|
| GT-06135 | 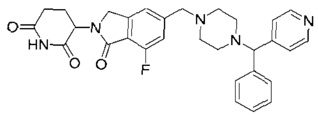 | 3-(7-fluoro-1-oxo-5-((4-(phenyl(pyridin-4-yl)methyl)piperazin-1-yl)methyl)isoindolin-2-yl)piperidine-2,6-dione | 3-(7-fluoro-1-oxo-5-((4-(phenyl(pyridin-4-yl)methyl)piperazin-1-yl)methyl)isoindolin-2-yl)piperidine-2,6-dione |
| GT-04945 | 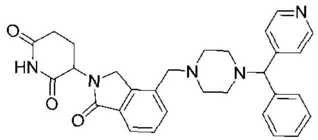 | 3-(1-oxo-4-((4-(phenyl(pyridin-4-yl)methyl)piperazin-1-yl)methyl)isoindolin-2-yl)piperidine-2,6-dione | 3-(1-oxo-4-((4-(phenyl(pyridin-4-yl)methyl)piperazin-1-yl)methyl)isoindolin-2-yl)piperidine-2,6-dione |
| GT-04383 | 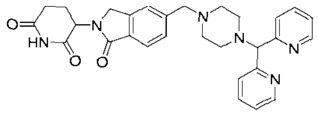 | 3-(5-((4-(di(pyridin-2-yl)methyl)piperazin-1-yl)methyl)-1-oxoisoindolin-2-yl)piperidine-2,6-dione | 3-(5-((4-(di(pyridin-2-yl)methyl)piperazin-1-yl)methyl)-1-oxoisoindolin-2-yl)piperidine-2,6-dione |
| GT-05713 | 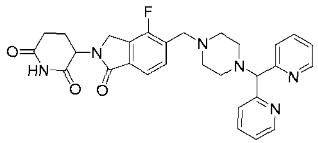 | 3-(5-((4-(di(pyridin-2-yl)methyl)piperazin-1-yl)methyl)-4-fluoro-1-oxoisoindolin-2-yl)piperidine-2,6-dione | 3-(5-((4-(di(pyridin-2-yl)methyl)piperazin-1-yl)methyl)-4-fluoro-1-oxoisoindolin-2-yl)piperidine-2,6-dione |
| GT-04384 | 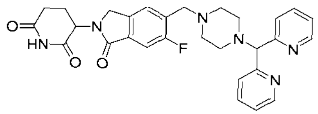 | 3-(5-((4-(di(pyridin-2-yl)methyl)piperazin-1-yl)methyl)-6-fluoro-1-oxoisoindolin-2-yl)piperidine-2,6-dione | 3-(5-((4-(di(pyridin-2-yl)methyl)piperazin-1-yl)methyl)-6-fluoro-1-oxoisoindolin-2-yl)piperidine-2,6-dione |
| GT-05714 | 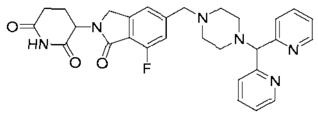 | 3-(5-((4-(di(pyridin-2-yl)methyl)piperazin-1-yl)methyl)-7-fluoro-1-oxoisoindolin-2-yl)piperidine-2,6-dione | 3-(5-((4-(di(pyridin-2-yl)methyl)piperazin-1-yl)methyl)-7-fluoro-1-oxoisoindolin-2-yl)piperidine-2,6-dione |
| GT-05715 | 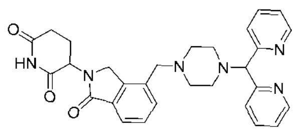 | 3-(4-((4-(di(pyridin-2-yl)methyl)piperazin-1-yl)methyl)-1-oxoisoindolin-2-yl)piperidine-2,6-dione | 3-(4-((4-(di(pyridin-2-yl)methyl)piperazin-1-yl)methyl)-1-oxoisoindolin-2-yl)piperidine-2,6-dione |
| GT-04385 | 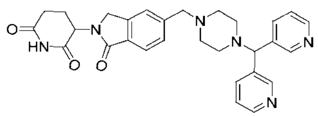 | 3-(5-((4-(di(pyridin-3-yl)methyl)piperazin-1-yl)methyl)-1-oxoisoindolin-2-yl)piperidine-2,6-dione | 3-(5-((4-(di(pyridin-3-yl)methyl)piperazin-1-yl)methyl)-1-oxoisoindolin-2-yl)piperidine-2,6-dione |

(continued)

| Compound No. | Structure of the compounds | The compounds' name | Chinese translation of the compounds' name |
|---|---|---|---|
| GT-05716 | 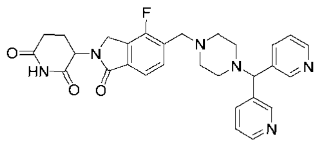 | 3-(5-((4-(di(pyridin-3-yl)methyl)piperazin-1-yl)methyl)-4-fluoro-1-oxoisoindolin-2-yl)piperidine-2,6-dione | 3-(5-((4-(di(pyridin-3-yl)methyl)piperazin-1-yl)methyl)-4-fluoro-1-oxoisoindolin-2-yl)piperidine-2,6-dione |
| GT-04386 | 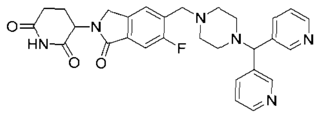 | 3-(5-((4-(di(pyridin-3-yl)methyl)piperazin-1-yl)methyl)-6-fluoro-1-oxoisoindolin-2-yl)piperidine-2,6-dione | 3-(5-((4-(di(pyridin-3-yl)methyl)piperazin-1-yl)methyl)-6-fluoro-1-oxoisoindolin-2-yl)piperidine-2,6-dione |
| GT-05717 | 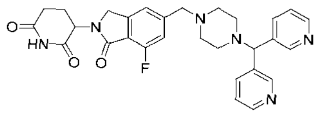 | 3-(5-((4-(di(pyridin-3-yl)methyl)piperazin-1-yl)methyl)-7-fluoro-1-oxoisoindolin-2-yl)piperidine-2,6-dione | 3-(5-((4-(di(pyridin-3-yl)methyl)piperazin-1-yl)methyl)-7-fluoro-1-oxoisoindolin-2-yl)piperidine-2,6-dione |
| GT-05718 | 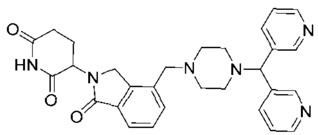 | 3-(4-((4-(di(pyridin-3-yl)methyl)piperazin-1-yl)methyl)-1-oxoisoindolin-2-yl)piperidine-2,6-dione | 3-(4-((4-(di(pyridin-3-yl)methyl)piperazin-1-yl)methyl)-1-oxoisoindolin-2-yl)piperidine-2,6-dione |
| | 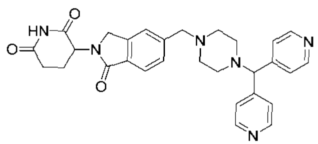 | 3-(5-((4-(di(pyridin-4-yl)methyl)piperazin-1-yl)methyl)-1-oxoisoindolin-2-yl)piperidine-2,6-dione | 3-(5-((4-(di(pyridin-4-yl)methyl)piperazin-1-yl)methyl)-1-oxoisoindolin-2-yl)piperidine-2,6-dione |
| | 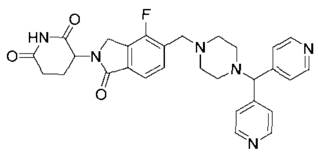 | 3-(5-((4-(di(pyridin-4-yl)methyl)piperazin-1-yl)methyl)-4-fluoro-1-oxoisoindolin-2-yl)piperidine-2,6-dione | 3-(5-((4-(di(pyridin-4-yl)methyl)piperazin-1-yl)methyl)-4-fluoro-1-oxoisoindolin-2-yl)piperidine-2,6-dione |
| | 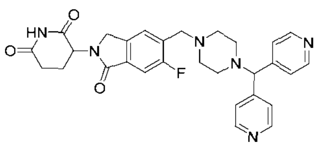 | 3-(5-((4-(di(pyridin-4-yl)methyl)piperazin-1-yl)methyl)-6-fluoro-1-oxoisoindolin-2-yl)piperidine-2,6-dione | 3-(5-((4-(di(pyridin-4-yl)methyl)piperazin-1-yl)methyl)-6-fluoro-1-oxoisoindolin-2-yl)piperidine-2,6-dione |

(continued)

| Compound No. | Structure of the compounds | The compounds' name | Chinese translation of the compounds' name |
|---|---|---|---|
| | 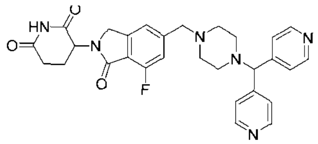 | 3-(5-((4-(di(pyridin-4-yl)methyl)piperazin-1-yl)methyl)-7-fluoro-1-oxoisoindolin-2-yl)piperidine-2,6-dione | 3-(5-((4-(di(pyridin-4-yl)methyl)piperazin-1-yl)methyl)-7-fluoro-1-oxoisoindolin-2-yl)piperidine-2,6-dione |
| | 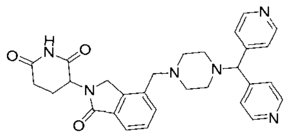 | 3-(4-((4-(di(pyridin-4-yl)methyl)piperazin-1-yl)methyl)-1-oxoisoindolin-2-yl)piperidine-2,6-dione | 3-(4-((4-(di(pyridin-4-yl)methyl)piperazin-1-yl)methyl)-1-oxoisoindolin-2-yl)piperidine-2,6-dione |
| | 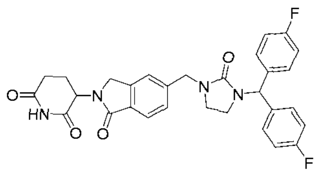 | 3-(5-((3-(bis(4-fluorophenyl)methyl)-2-oxoimidazolidin-1-yl)methyl)-1-oxoisoindolin-2-yl)piperidine-2,6-dione | 3-(5-((3-(bis(4-fluorophenyl)methyl)-2-oxoimidazolidin-1-yl)methyl)-1-oxoisoindolin-2-yl)piperidine-2,6-dione |
| | 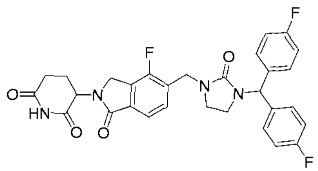 | 3-(5-((3-(bis(4-fluorophenyl)methyl)-2-oxoimidazolidin-1-yl)methyl)-4-fluoro-1-oxoisoindolin-2-yl)piperidine-2,6-dione | 3-(5-((3-(bis(4-fluorophenyl)methyl)-2-oxoimidazolidin-1-yl)methyl)-4-fluoro-1-oxoisoindolin-2-yl)piperidine-2,6-dione |
| | 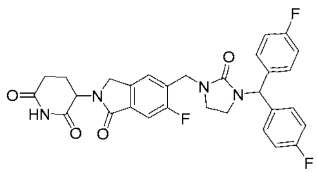 | 3-(5-((3-(bis(4-fluorophenyl)methyl)-2-oxoimidazolidin-1-yl)methyl)-6-fluoro-1-oxoisoindolin-2-yl)piperidine-2,6-dione | 3-(5-((3-(bis(4-fluorophenyl)methyl)-2-oxoimidazolidin-1-yl)methyl)-6-fluoro-1-oxoisoindolin-2-yl)piperidine-2,6-dione |
| | 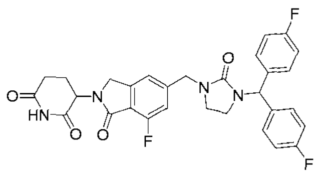 | 3-(5-((3-(bis(4-fluorophenyl)methyl)-2-oxoimidazolidin-1-yl)methyl)-7-fluoro-1-oxoisoindolin-2-yl)piperidine-2,6-dione | 3-(5-((3-(bis(4-fluorophenyl)methyl)-2-oxoimidazolidin-1-yl)methyl)-7-fluoro-1-oxoisoindolin-2-yl)piperidine-2,6-dione |

(continued)

| Compound No. | Structure of the compounds | The compounds' name | Chinese translation of the compounds' name |
|---|---|---|---|
| | 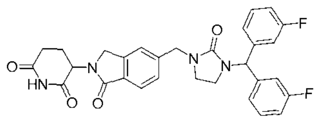 | 3-(5-((3-(bis(3-fluorophenyl)methyl)-2-oxoimidazolidin-1-yl)methyl)-1-oxoisoindolin-2-yl)piperidine-2,6-dione | 3-(5-((3-(bis(3-fluorophenyl)methyl)-2-oxoimidazolidin-1-yl)methyl)-1-oxoisoindolin-2-yl)piperidine-2,6-dione |
| | 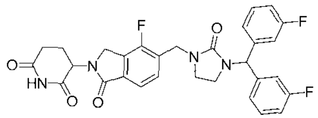 | 3-(5-((3-(bis(3-fluorophenyl)methyl)-2-oxoimidazolidin-1-yl)methyl)-4-fluoro-1-oxoisoindolin-2-yl)piperidine-2,6-dione | 3-(5-((3-(bis(3-fluorophenyl)methyl)-2-oxoimidazolidin-1-yl)methyl)-4-fluoro-1-oxoisoindolin-2-yl)piperidine-2,6-dione |
| | 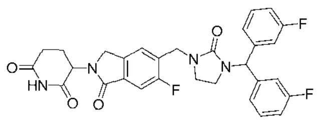 | 3-(5-((3-(bis(3-fluorophenyl)methyl)-2-oxoimidazolidin-1-yl)methyl)-6-fluoro-1-oxoisoindolin-2-yl)piperidine-2,6-dione | 3-(5-((3-(bis(3-fluorophenyl)methyl)-2-oxoimidazolidin-1-yl)methyl)-6-fluoro-1-oxoisoindolin-2-yl)piperidine-2,6-dione |
| | 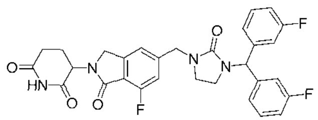 | 3-(5-((3-(bis(3-fluorophenyl)methyl)-2-oxoimidazolidin-1-yl)methyl)-7-fluoro-1-oxoisoindolin-2-yl)piperidine-2,6-dione | 3-(5-((3-(bis(3-fluorophenyl)methyl)-2-oxoimidazolidin-1-yl)methyl)-7-fluoro-1-oxoisoindolin-2-yl)piperidine-2,6-dione |
| | 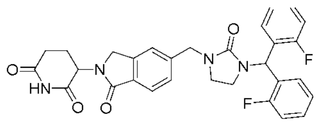 | 3-(5-((3-(bis(2-fluorophenyl)methyl)-2-oxoimidazolidin-1-yl)methyl)-1-oxoisoindolin-2-yl)piperidine-2,6-dione | 3-(5-((3-(bis(2-fluorophenyl)methyl)-2-oxoimidazolidin-1-yl)methyl)-1-oxoisoindolin-2-yl)piperidine-2,6-dione |
| | 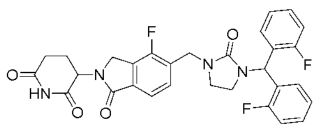 | 3-(5-((3-(bis(2-fluorophenyl)methyl)-2-oxoimidazolidin-1-yl)methyl)-4-fluoro-1-oxoisoindolin-2-yl)piperidine-2,6-dione | 3-(5-((3-(bis(2-fluorophenyl)methyl)-2-oxoimidazolidin-1-yl)methyl)-4-fluoro-1-oxoisoindolin-2-yl)piperidine-2,6-dione |
| | 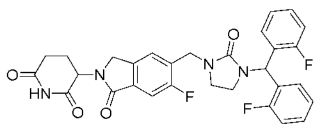 | 3-(5-((3-(bis(2-fluorophenyl)methyl)-2-oxoimidazolidin-1-yl)methyl)-6-fluoro-1-oxoisoindolin-2-yl)piperidine-2,6-dione | 3-(5-((3-(bis(2-fluorophenyl)methyl)-2-oxoimidazolidin-1-yl)methyl)-6-fluoro-1-oxoisoindolin-2-yl)piperidine-2,6-dione |

(continued)

| Compound No. | Structure of the compounds | The compounds' name | Chinese translation of the compounds' name |
|---|---|---|---|
| | 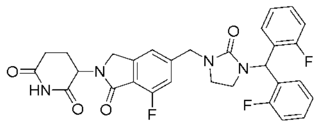 | 3-(5-((3-(bis(2-fluorophenyl)methyl)-2-oxoimidazolidin-1-yl)methyl)-7-fluoro-1-oxoisoindolin-2-yl)piperidine-2,6-dione | 3-(5-((3-(bis(2-fluorophenyl)methyl)-2-oxoimidazolidin-1-yl)methyl)-7-fluoro-1-oxoisoindolin-2-yl)piperidine-2,6-dione |
| | 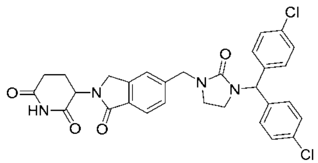 | 3-(5-((3-(bis(4-chlorophenyl)methyl)-2-oxoimidazolidin-1-yl)methyl)-1-oxoisoindolin-2-yl)piperidine-2,6-dione | 3-(5-((3-(bis(4-chlorophenyl)methyl)-2-oxoimidazolidin-1-yl)methyl)-1-oxoisoindolin-2-yl)piperidine-2,6-dione |
| | 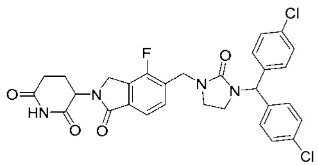 | 3-(5-((3-(bis(4-chlorophenyl)methyl)-2-oxoimidazolidin-1-yl)methyl)-4-fluoro-1-oxoisoindolin-2-yl)piperidine-2,6-dione | 3-(5-((3-(bis(4-chlorophenyl)methyl)-2-oxoimidazolidin-1-yl)methyl)-4-fluoro-1-oxoisoindolin-2-yl)piperidine-2,6-dione |
| | 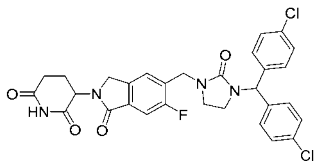 | 3-(5-((3-(bis(4-chlorophenyl)methyl)-2-oxoimidazolidin-1-yl)methyl)-6-fluoro-1-oxoisoindolin-2-yl)piperidine-2,6-dione | 3-(5-((3-(bis(4-chlorophenyl)methyl)-2-oxoimidazolidin-1-yl)methyl)-6-fluoro-1-oxoisoindolin-2-yl)piperidine-2,6-dione |
| | 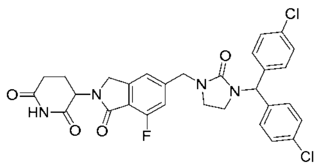 | 3-(5-((3-(bis(4-chlorophenyl)methyl)-2-oxoimidazolidin-1-yl)methyl)-7-fluoro-1-oxoisoindolin-2-yl)piperidine-2,6-dione | 3-(5-((3-(bis(4-chlorophenyl)methyl)-2-oxoimidazolidin-1-yl)methyl)-7-fluoro-1-oxoisoindolin-2-yl)piperidine-2,6-dione |
| GT-06640 | 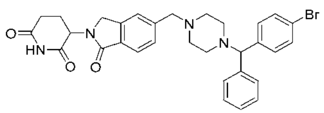 | 3-(5-((4-((4-bromophenyl)(phenyl)methyl )piperazin-1-yl) methyl)-1-oxoisoindolin-2-yl)piperidine-2,6-dione | 3-(5-((4-((4-bromophenyl)(phenyl)meth yl)piperazin-1-yl) methyl)-1-oxoisoindolin-2-yl)piperidine-2,6-dione |

(continued)

| Compou nd No. | Structure of the compounds | The compounds' name | Chinese translation of the compounds' name |
|---|---|---|---|
| GT-03386 | 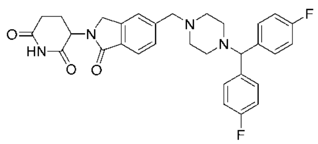 | 3-(5-((4-(bis(4-fluorophenyl)methyl)piperazi n-1-yl) methyl)-1-oxoisoindolin-2-yl)piperidine-2,6-dione | 3-(5-((4-(bis(4-fluorophenyl)methyl)pipera zin-1-yl) methyl)-1-oxoisoindolin-2-yl)piperidine-2,6-dione |
| GT-03564 | 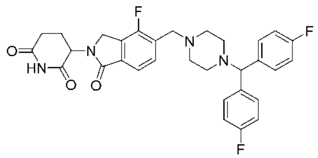 | 3-(5-((4-(bis(4-fluorophenyl)methyl)piperazi n-1-yl) methyl)-4-fluoro-1-oxoisoindolin-2-yl)piperidine-2,6-dione | 3-(5-((4-(bis(4-fluorophenyl)methyl)pipera zin-1-yl) methyl)-4-fluoro-1-oxoisoindolin-2-yl)piperidine-2,6-dione |
| GT-03698 | 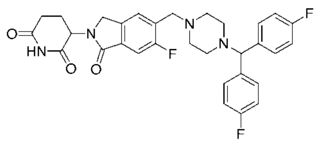 | 3-(5-((4-(bis(4-fluorophenyl)methyl)piperazi n-1-yl) methyl)-6-fluoro-1-oxoisoindolin-2-yl)piperidine-2,6-dione | 3-(5-((4-(bis(4-fluorophenyl)methyl)pipera zin-1-yl) methyl)-6-fluoro-1-oxoisoindolin-2-yl)piperidine-2,6-dione |
| GT-03565 | 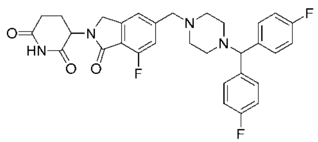 | 3-(5-((4-(bis(4-fluorophenyl)methyl)piperazi n-1-yl) methyl)-7-fluoro-1-oxoisoindolin-2-yl)piperidine-2,6-dione | 3-(5-((4-(bis(4-fluorophenyl)methyl)pipera zin-1-yl) methyl)-7-fluoro-1-oxoisoindolin-2-yl)piperidine-2,6-dione |
| GT-03647 | 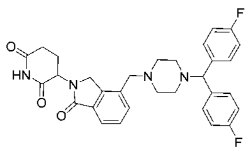 | 3-(4-((4-(bis(4-fluorophenyl)methyl)piperazi n-1-yl) methyl)-1-oxoisoindolin-2-yl)piperidine-2,6-dione | 3-(4-((4-(bis(4-fluorophenyl)methyl)pipera zin-1-yl) methyl)-1-oxoisoindolin-2-yl)piperidine-2,6-dione |
| GT-05719 | 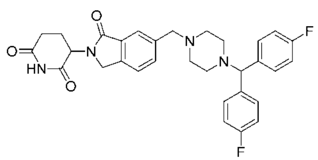 | 3-(6-((4-(bis(4-fluorophenyl)methyl)piperazi n-1-yl) methyl)-1-oxoisoindolin-2-yl)piperidine-2,6-dione | 3-(6-((4-(bis(4-fluorophenyl)methyl)pipera zin-1-yl) methyl)-1-oxoisoindolin-2-yl)piperidine-2,6-dione |

(continued)

| Compou nd No. | Structure of the compounds | The compounds' name | Chinese translation of the compounds' name |
|---|---|---|---|
| GT-05720 | 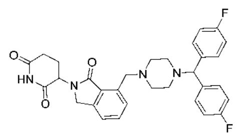 | 3-(7-((4-(bis(4-fluorophenyl)methyl)piperazi n-1-yl) methyl)-1-oxoisoindolin-2-yl)piperidine-2,6-dione | 3-(7-((4-(bis(4-fluorophenyl)methyl)pipera zin-1-yl) methyl)-1-oxoisoindolin-2-yl)piperidine-2,6-dione |
| GT-03702 | 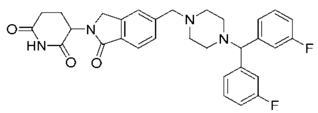 | 3-(5-((4-(bis(3-fluorophenyl)methyl)piperazi n-1-yl) methyl)-1-oxoisoindolin-2-yl)piperidine-2,6-dione | 3-(5-((4-(bis(3-fluorophenyl)methyl)pipera zin-1-yl) methyl)-1-oxoisoindolin-2-yl)piperidine-2,6-dione |
| GT-03703 | 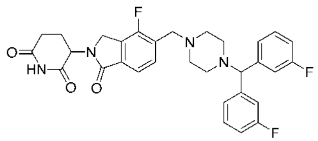 | 3-(5-((4-(bis(3-fluorophenyl)methyl)piperazi n-1-yl) methyl)-4-fluoro-1-oxoisoindolin-2-yl)piperidine-2,6-dione | 3-(5-((4-(bis(3-fluorophenyl)methyl)pipera zin-1-yl) methyl)-4-fluoro-1-oxoisoindolin-2-yl)piperidine-2,6-dione |
| GT-03701 | 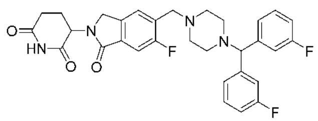 | 3-(5-((4-(bis(3-fluorophenyl)methyl)piperazi n-1-yl) methyl)-6-fluoro-1-oxoisoindolin-2-yl)piperidine-2,6-dione | 3-(5-((4-(bis(3-fluorophenyl)methyl)pipera zin-1-yl) methyl)-6-fluoro-1-oxoisoindolin-2-yl)piperidine-2,6-dione |
| GT-03704 | 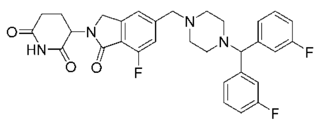 | 3-(5-((4-(bis(3-fluorophenyl)methyl)piperazi n-1-yl) methyl)-7-fluoro-1-oxoisoindolin-2-yl)piperidine-2,6-dione | 3-(5-((4-(bis(3-fluorophenyl)methyl)pipera zin-1-yl) methyl)-7-fluoro-1-oxoisoindolin-2-yl)piperidine-2,6-dione |
| GT-03875 | 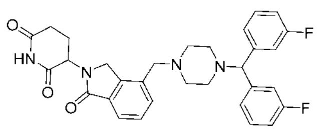 | 3-(4-((4-(bis(3-fluorophenyl)methyl)piperazi n-1-yl) methyl)-1-oxoisoindolin-2-yl)piperidine-2,6-dione | 3-(4-((4-(bis(3-fluorophenyl)methyl)pipera zin-1-yl) methyl)-1-oxoisoindolin-2-yl)piperidine-2,6-dione |
| GT-06116 | 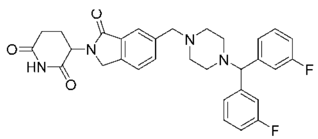 | 3-(6-((4-(bis(3-fluorophenyl)methyl)piperazi n-1-yl) methyl)-1-oxoisoindolin-2-yl)piperidine-2,6-dione | 3-(6-((4-(bis(3-fluorophenyl)methyl)pipera zin-1-yl) methyl)-1-oxoisoindolin-2-yl)piperidine-2,6-dione |

(continued)

| Compou nd No. | Structure of the compounds | The compounds' name | Chinese translation of the compounds' name |
|---|---|---|---|
| GT-06117 | 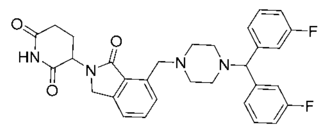 | 3-(7-((4-(bis(3-fluorophenyl)methyl)piperazi n-1-yl) methyl)-1-oxoisoindolin-2-yl)piperidine-2,6-dione | 3-(7-((4-(bis(3-fluorophenyl)methyl)pipera zin-1-yl) methyl)-1-oxoisoindolin-2-yl)piperidine-2,6-dione |
| GT-06084 | 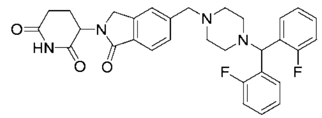 | 3-(5-((4-(bis(2-fluorophenyl)methyl)piperazi n-1-yl) methyl)-1-oxoisoindolin-2-yl)piperidine-2,6-dione | 3-(5-((4-(bis(2-fluorophenyl)methyl)pipera zin-1-yl) methyl)-1-oxoisoindolin-2-yl)piperidine-2,6-dione |
| GT-06085 | 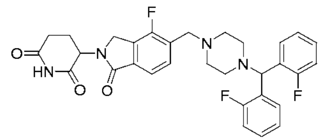 | 3-(5-((4-(bis(2-fluorophenyl)methyl)piperazi n-1-yl) methyl)-4-fluoro-1-oxoisoindolin-2-yl)piperidine-2,6-dione | 3-(5-((4-(bis(2-fluorophenyl)methyl)pipera zin-1-yl) methyl)-4-fluoro-1-oxoisoindolin-2-yl)piperidine-2,6-dione |
| GT-06086 | 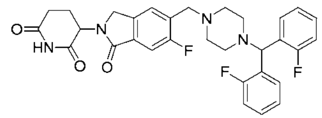 | 3-(5-((4-(bis(2-fluorophenyl)methyl)piperazi n-1-yl) methyl)-6-fluoro-1-oxoisoindolin-2-yl)piperidine-2,6-dione | 3-(5-((4-(bis(2-fluorophenyl)methyl)pipera zin-1-yl) methyl)-6-fluoro-1-oxoisoindolin-2-yl)piperidine-2,6-dione |
| GT-06087 | 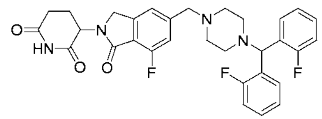 | 3-(5-((4-(bis(2-fluorophenyl)methyl)piperazi n-1-yl) methyl)-7-fluoro-1-oxoisoindolin-2-yl)piperidine-2,6-dione | 3-(5-((4-(bis(2-fluorophenyl)methyl)pipera zin-1-yl) methyl)-7-fluoro-1-oxoisoindolin-2-yl)piperidine-2,6-dione |
| GT-06199 | 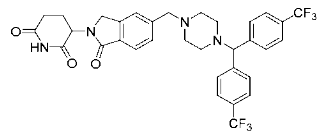 | 3-(5-((4-(bis(4-(trifluoromethyl)phenyl)meth yl)piperazin-1-yl)methyl)-1-oxoisoindolin-2-yl)piperidine-2,6-dione | 3-(5-((4-(bis(4-(trifluoromethyl)phenyl)me thyl)piperazin-1-yl)methyl)-1-oxoisoindolin-2-yl)piperidine-2,6-dione |
| GT-06200 | 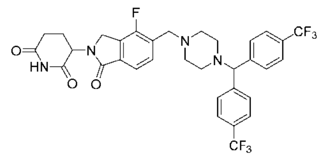 | 3-(5-((4-(bis(4-(trifluoromethyl)phenyl)meth yl)piperazin-1-yl)methyl)-4-fluoro-1-oxoisoindolin-2-yl)piperidine-2,6-dione | 3-(5-((4-(bis(4-(trifluoromethyl)phenyl)me thyl)piperazin-1-yl)methyl)-4-fluoro-1-oxoisoindolin-2-yl)piperidine-2,6-dione |

(continued)

| Compou nd No. | Structure of the compounds | The compounds' name | Chinese translation of the compounds' name |
|---|---|---|---|
| GT-06201 | 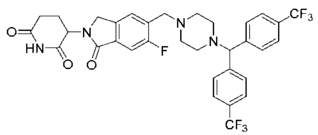 | 3-(5-((4-(bis(4-(trifluoromethyl)phenyl)meth yl)piperazin-1-yl)methyl)-6-fluoro-1-oxoisoindolin-2-yl)piperidine-2,6-dione | 3-(5-((4-(bis(4-(trifluoromethyl)phenyl)me thyl)piperazin-1-yl)methyl)-6-fluoro-1-oxoisoindolin-2-yl)piperidine-2,6-dione |
| GT-06202 | 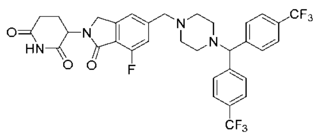 | 3-(5-((4-(bis(4-(trifluoromethyl)phenyl)meth yl)piperazin-1-yl)methyl)-7-fluoro-1-oxoisoindolin-2-yl)piperidine-2,6-dione | 3-(5-((4-(bis(4-(trifluoromethyl)phenyl)me thyl)piperazin-1-yl)methyl)-7-fluoro-1-oxoisoindolin-2-yl)piperidine-2,6-dione |
| | 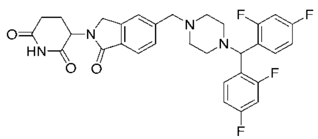 | 3-(5-((4-(bis(2,4-difluorophenyl)methyl)pipera zin-1-yl) methyl)-1-oxoisoindolin-2-yl)piperidine-2,6-dione | 3-(5-((4-(bis(2,4-difluorophenyl)methyl)pipe razin-1-yl) methyl)-1-oxoisoindolin-2-yl)piperidine-2,6-dione |
| | 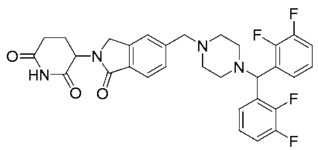 | 3-(5-((4-((2,3-difluorophenyl)(3,4-difluorophenyl)methyl) pipera zin-1-yl)methyl)-1-oxoisoindolin-2-yl)piperidine-2,6-dione | 3-(5-((4-((2,3-difluorophenyl)(3,4-difluorophenyl)methyl) pipe razin-1-yl)methyl)-1-oxoisoindolin-2-yl)piperidine-2,6-dione |
| GT-06387 | 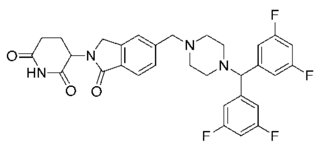 | 3-(5-((4-(bis(3,5-difluorophenyl)methyl)pipera zin-1-yl) methyl)-1-oxoisoindolin-2-yl)piperidine-2,6-dione | 3-(5-((4-(bis(3,5-difluorophenyl)methyl)pipe razin-1-yl) methyl)-1-oxoisoindolin-2-yl)piperidine-2,6-dione |
| | 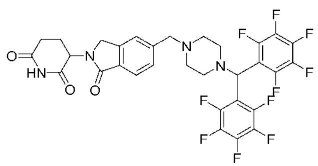 | 3-(5-((4-(bis(perfluorophenyl)methyl) piperazin-1-yl) methyl)-1-oxoisoindolin-2-yl)piperidine-2,6-dione | 3-(5-((4-(bis(perfluorophenyl)methy 1)piperazin-1-yl) methyl)-1-oxoisoindolin-2-yl)piperidine-2,6-dione |

(continued)

| Compou nd No. | Structure of the compounds | The compounds' name | Chinese translation of the compounds' name |
|---|---|---|---|
| | 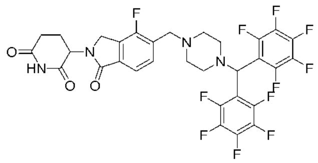 | 3-(5-((4-(bis(perfluorophenyl)methyl) piperazin-1-yl) methyl)-4-fluoro-1-oxoisoindolin-2-yl)piperidine-2,6-dione | 3-(5-((4-(bis(perfluorophenyl)methy 1)piperazin-1-yl) methyl)-4-fluoro-1-oxoisoindolin-2-yl)piperidine-2,6-dione |
| | 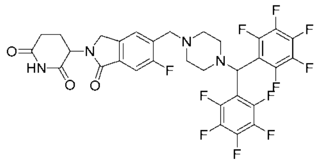 | 3-(5-((4-(bis(perfluorophenyl)methyl) piperazin-1-yl) methyl)-6-fluoro-1-oxoisoindolin-2-yl)piperidine-2,6-dione | 3-(5-((4-(bis(perfluorophenyl)methy l)piperazin-1-yl) methyl)-6-fluoro-1-oxoisoindolin-2-yl)piperidine-2,6-dione |
| | 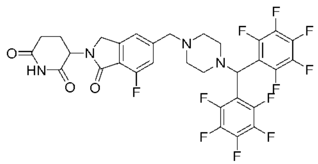 | 3-(5-((4-(bis(perfluorophenyl)methyl) piperazin-1-yl) methyl)-7-fluoro-1-oxoisoindolin-2-yl)piperidine-2,6-dione | 3-(5-((4-(bis(perfluorophenyl)methy 1)piperazin-1-yl) methyl)-7-fluoro-1-oxoisoindolin-2-yl)piperidine-2,6-dione |
| GT-03566 | 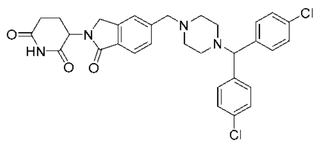 | 3-(5-((4-(bis(4-chlorophenyl)methyl)piperazi n-1-yl) methyl)-1-oxoisoindolin-2-yl)piperidine-2,6-dione | 3-(5-((4-(bis(4-chlorophenyl)methyl)piper azin-1-yl) methyl)-1-oxoisoindolin-2-yl)piperidine-2,6-dione |
| GT-03567 | 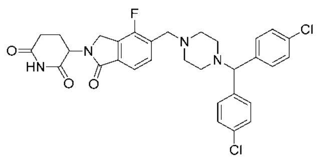 | 3-(5-((4-(bis(4-chlorophenyl)methyl)piperazi n-1-yl) methyl)-4-fluoro-1-oxoisoindolin-2-yl)piperidine-2,6-dione | 3-(5-((4-(bis(4-chlorophenyl)methyl)piper azin-1-yl) methyl)-4-fluoro-1-oxoisoindolin-2-yl)piperidine-2,6-dione |
| GT-03699 | 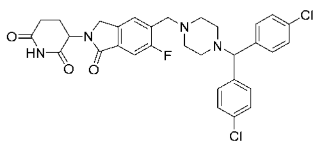 | 3-(5-((4-(bis(4-chlorophenyl)methyl)piperazi n-1-yl) methyl)-6-fluoro-1-oxoisoindolin-2-yl)piperidine-2,6-dione | 3-(5-((4-(bis(4-chlorophenyl)methyl)piper azin-1-yl) methyl)-6-fluoro-1-oxoisoindolin-2-yl)piperidine-2,6-dione |

(continued)

| Compou nd No. | Structure of the compounds | The compounds' name | Chinese translation of the compounds' name |
|---|---|---|---|
| GT-03568 | 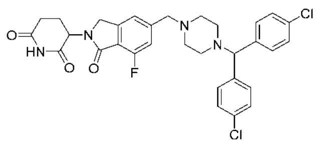 | 3-(5-((4-(bis(4-chlorophenyl)methyl)piperazi n-1-yl) methyl)-7-fluoro-1-oxoisoindolin-2-yl)piperidine-2,6-dione | 3-(5-((4-(bis(4-chlorophenyl)methyl)piper azin-1-yl) methyl)-7-fluoro-1-oxoisoindolin-2-yl)piperidine-2,6-dione |
| GT-03648 | 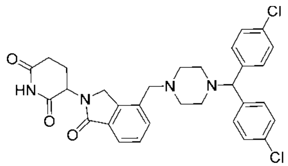 | 3-(4-((4-(bis(4-chlorophenyl)methyl)piperazi n-1-yl) methyl)-1-oxoisoindolin-2-yl)piperidine-2,6-dione | 3-(4-((4-(bis(4-chlorophenyl)methyl)piper azin-1-yl) methyl)-1-oxoisoindolin-2-yl)piperidine-2,6-dione |
| GT-05735 | 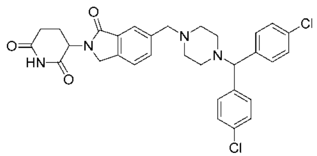 | 3-(6-((4-(bis(4-chlorophenyl)methyl)piperazi n-1-yl) methyl)-1-oxoisoindolin-2-yl)piperidine-2,6-dione | 3-(6-((4-(bis(4-chlorophenyl)methyl)piper azin-1-yl) methyl)-1-oxoisoindolin-2-yl)piperidine-2,6-dione |
| GT-05736 | 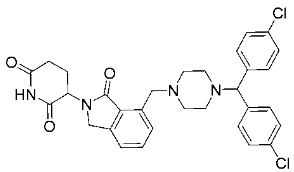 | 3-(7-((4-(bis(4-chlorophenyl)methyl)piperazi n-1-yl) methyl)-1-oxoisoindolin-2-yl)piperidine-2,6-dione | 3-(7-((4-(bis(4-chlorophenyl)methyl)piper azin-1-yl) methyl)-1-oxoisoindolin-2-yl)piperidine-2,6-dione |
| | 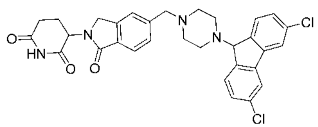 | 3-(5-((4-(3,6-dichloro-9H-fluoren-9-yl)piperazin-1-yl) methyl)-1-oxoisoindolin-2-yl)piperidine-2,6-dione | 3-(5-((4-(3,6-dichloro-9H-fluoren-9-yl)piperazin-1-yl) methyl)-1-oxoisoindolin-2-yl)piperidine-2,6-dione |
| GT-06048 | 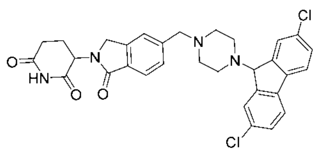 | 3-(5-((4-(2,7-dichloro-9H-fluoren-9-yl)piperazin-1-yl) methyl)-1-oxoisoindolin-2-yl)piperidine-2,6-dione | 3-(5-((4-(2,7-dichloro-9H-fluoren-9-yl)piperazin-1-yl) methyl)-1-oxoisoindolin-2-yl)piperidine-2,6-dione |

(continued)

| Compound No. | Structure of the compounds | The compounds' name | Chinese translation of the compounds' name |
|---|---|---|---|
| GT-05907 | 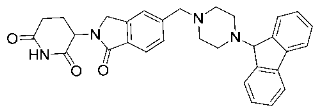 | 3-(5-((4-(9H-fluoren-9-yl)piperazin-1-yl)methyl)-1-oxoisoindolin-2-yl)piperidine-2,6-dione | 3-(5-((4-(9H-fluoren-9-yl)piperazin-1-yl)methyl)-1-oxoisoindolin-2-yl)piperidine-2,6-dione |
| GT-04244 | 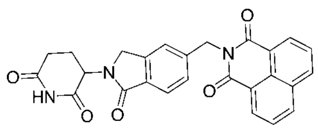 | 2-((2-(2,6-dioxopiperidin-3-yl)-1-oxoisoindolin-5-yl) methyl)-1H-benzo[de]isoquinoline-1,3(2H)-dione | 2-((2-(2,6-dioxopiperidin-3-yl)-1-oxoisoindolin-5-yl) methyl)-1H-benzo[de]isoquinoline-1,3(2H)-dione |
| | 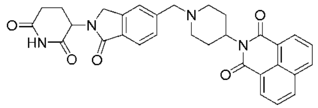 | 2-(1-((2-(2,6-dioxopiperidin-3-yl)-1-oxoisoindolin-5-yl) methyl)piperidin-4-yl)-1H-benzo[de]isoquinoline-1,3(2H)-dione | 2-(1-((2-(2,6-dioxopiperidin-3-yl)-1-oxoisoindolin-5-yl) methyl)piperidin-4-yl)-1H-benzo[de]isoquinoline-1,3(2H)-dione |
| | 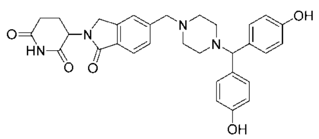 | 3-(5-((4-(bis(4-hydroxyphenyl)methyl)piper azin-1-yl) methyl)-1-oxoisoindolin-2-yl)piperidine-2,6-dione | 3-(5-((4-(bis(4-hydroxyphenyl)methyl)pip erazin-1-yl) methyl)-1-oxoisoindolin-2-yl)piperidine-2,6-dione |
| | 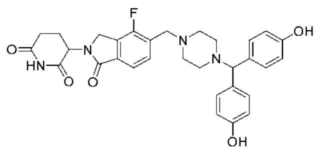 | 3-(5-((4-(bis(4-hydroxyphenyl)methyl)piper azin-1-yl) methyl)-4-fluoro-1-oxoisoindolin-2-yl)piperidine-2,6-dione | 3-(5-((4-(bis(4-hydroxyphenyl)methyl)pip erazin-1-yl) methyl)-4-fluoro-1-oxoisoindolin-2-yl)piperidine-2,6-dione |
| | 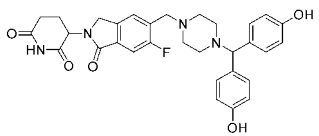 | 3-(5-((4-(bis(4-hydroxyphenyl)methyl)piper azin-1-yl) methyl)-6-fluoro-1-oxoisoindolin-2-yl)piperidine-2,6-dione | 3-(5-((4-(bis(4-hydroxyphenyl)methyl)pip erazin-1-yl) methyl)-6-fluoro-1-oxoisoindolin-2-yl)piperidine-2,6-dione |
| | 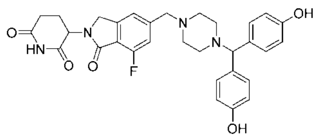 | 3-(5-((4-(bis(4-hydroxyphenyl)methyl)piper azin-1-yl) methyl)-7-fluoro-1-oxoisoindolin-2-yl)piperidine-2,6-dione | 3-(5-((4-(bis(4-hydroxyphenyl)methyl)pip erazin-1-yl) methyl)-7-fluoro-1-oxoisoindolin-2-yl)piperidine-2,6-dione |

(continued)

| Compound No. | Structure of the compounds | The compounds' name | Chinese translation of the compounds' name |
|---|---|---|---|
| | 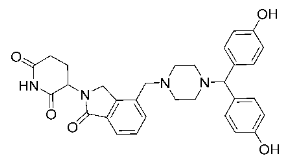 | 3-(4-((4-(bis(4-hydroxyphenyl)methyl)piper azin-1-yl) methyl)-1-oxoisoindolin-2-yl)piperidine-2,6-dione | 3-(4-((4-(bis(4-hydroxyphenyl)methyl)pip erazin-1-yl) methyl)-1-oxoisoindolin-2-yl)piperidine-2,6-dione |
| | 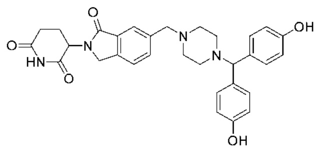 | 3-(6-((4-(bis(4-hydroxyphenyl)methyl)piper azin-1-yl) methyl)-1-oxoisoindolin-2-yl)piperidine-2,6-dione | 3-(6-((4-(bis(4-hydroxyphenyl)methyl)pip erazin-1-yl) methyl)-1-oxoisoindolin-2-yl)piperidine-2,6-dione |
| | 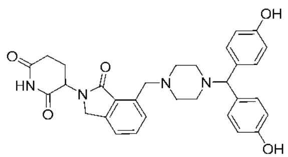 | 3-(7-((4-(bis(4-hydroxyphenyl)methyl)piper azin-1-yl) methyl)-1-oxoisoindolin-2-yl)piperidine-2,6-dione | 3-(7-((4-(bis(4-hydroxyphenyl)methyl)pip erazin-1-yl) methyl)-1-oxoisoindolin-2-yl)piperidine-2,6-dione |
| GT-06424 | 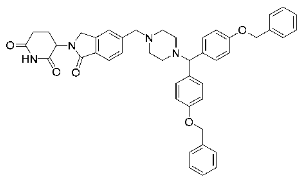 | 3-(5-((4-(bis(4-(benzyloxy)phenyl)methyl)pi perazin-1-yl) methyl)-1-oxoisoindolin-2-yl)piperidine-2,6-dione | 3-(5-((4-(bis(4-(benzyloxy)phenyl)methyl) piperazin-1-yl) methyl)-1-oxoisoindolin-2-yl)piperidine-2,6-dione |
| | 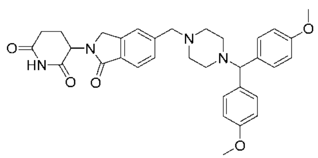 | 3-(5-((4-(bis(4-methoxyphenyl)methyl)piper azin-1-yl) methyl)-1-oxoisoindolin-2-yl)piperidine-2,6-dione | 3-(5-((4-(bis(4-methoxyphenyl)methyl)pip erazin-1-yl) methyl)-1-oxoisoindolin-2-yl)piperidine-2,6-dione |
| | 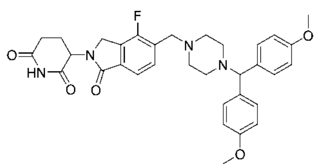 | 3-(5-((4-(bis(4-methoxyphenyl)methyl)piper azin-1-yl) methyl)-4-fluoro-1-oxoisoindolin-2-yl)piperidine-2,6-dione | 3-(5-((4-(bis(4-methoxyphenyl)methyl)pip erazin-1-yl) methyl)-4-fluoro-1-oxoisoindolin-2-yl)piperidine-2,6-dione |

(continued)

| Compou nd No. | Structure of the compounds | The compounds' name | Chinese translation of the compounds' name |
|---|---|---|---|
| | 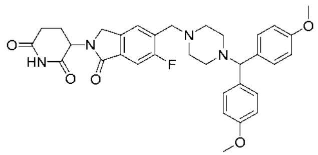 | 3-(5-((4-(bis(4-methoxyphenyl)methyl)piper azin-1-yl) methyl)-6-fluoro-1-oxoisoindolin-2-yl)piperidine-2,6-dione | 3-(5-((4-(bis(4-methoxyphenyl)methyl)pip erazin-1-yl) methyl)-6-fluoro-1-oxoisoindolin-2-yl)piperidine-2,6-dione |
| | 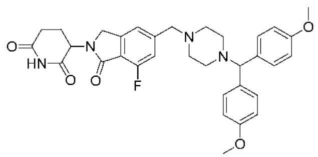 | 3-(5-((4-(bis(4-methoxyphenyl)methyl)piper azin-1-yl) methyl)-7-fluoro-1-oxoisoindolin-2-yl)piperidine-2,6-dione | 3-(5-((4-(bis(4-methoxyphenyl)methyl)pip erazin-1-yl) methyl)-7-fluoro-1-oxoisoindolin-2-yl)piperidine-2,6-dione |
| | 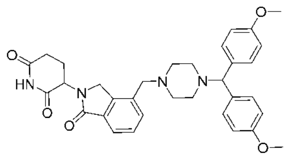 | 3-(4-((4-(bis(4-methoxyphenyl)methyl)piper azin-1-yl) methyl)-1-oxoisoindolin-2-yl)piperidine-2,6-dione | 3-(4-((4-(bis(4-methoxyphenyl)methyl)pip erazin-1-yl) methyl)-1-oxoisoindolin-2-yl)piperidine-2,6-dione |
| | 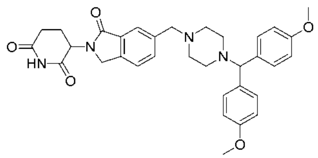 | 3-(6-((4-(bis(4-methoxyphenyl)methyl)piper azin-1-yl) methyl)-1-oxoisoindolin-2-yl)piperidine-2,6-dione | 3-(6-((4-(bis(4-methoxyphenyl)methyl)pip erazin-1-yl) methyl)-1-oxoisoindolin-2-yl)piperidine-2,6-dione |
| | 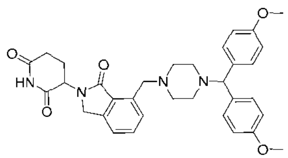 | 3-(7-((4-(bis(4-methoxyphenyl)methyl)piper azin-1-yl) methyl)-1-oxoisoindolin-2-yl)piperidine-2,6-dione | 3-(7-((4-(bis(4-methoxyphenyl)methyl)pip erazin-1-yl) methyl)-1-oxoisoindolin-2-yl)piperidine-2,6-dione |
| GT-04225 | 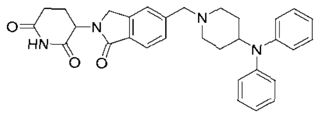 | 3-(5-((4-(diphenylamino)piperidin-1-yl)methyl)-1-oxoisoindolin-2-yl)piperidine-2,6-dione | 3-(5-((4-(diphenylamino)piperidin-1-yl)methyl)-1-oxoisoindolin-2-yl)piperidine-2,6-dione |

(continued)

| Compound No. | Structure of the compounds | The compounds' name | Chinese translation of the compounds' name |
|---|---|---|---|
| GT-04226 | 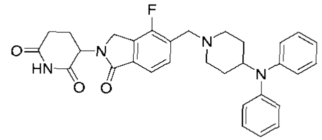 | 3-(5-((4-(diphenylamino)piperidin-1-yl)methyl)-4-fluoro-1-oxoisoindolin-2-yl)piperidine-2,6-dione | 3-(5-((4-(diphenylamino)piperidin-1-yl)methyl)-4-fluoro-1-oxoisoindolin-2-yl)piperidine-2,6-dione |
| GT-04227 | 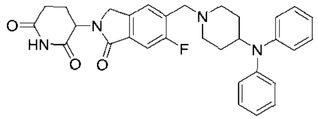 | 3-(5-((4-(diphenylamino)piperidin-1-yl)methyl)-6-fluoro-1-oxoisoindolin-2-yl)piperidine-2,6-dione | 3-(5-((4-(diphenylamino)piperidin-1-yl)methyl)-6-fluoro-1-oxoisoindolin-2-yl)piperidine-2,6-dione |
| GT-06133 | 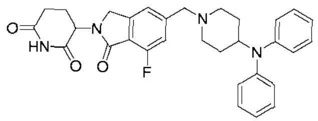 | 3-(5-((4-(diphenylamino)piperidin-1-yl)methyl)-7-fluoro-1-oxoisoindolin-2-yl)piperidine-2,6-dione | 3-(5-((4-(diphenylamino)piperidin-1-yl)methyl)-7-fluoro-1-oxoisoindolin-2-yl)piperidine-2,6-dione |
| GT-04992 | 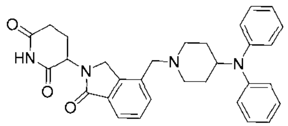 | 3-(4-((4-(diphenylamino)piperidin-1-yl)methyl)-1-oxoisoindolin-2-yl)piperidine-2,6-dione | 3-(4-((4-(diphenylamino)piperidin-1-yl)methyl)-1-oxoisoindolin-2-yl)piperidine-2,6-dione |
| GT-04525 | 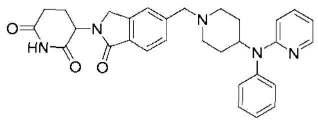 | 3-(1-oxo-5-((4-(phenyl(pyridin-2-yl)amino)piperidin-1-yl)methyl)isoindolin-2-yl)piperidine-2,6-dione | 3-(1-oxo-5-((4-(phenyl(pyridin-2-yl)amino)piperidin-1-yl)methyl)isoindolin-2-yl)piperidine-2,6-dione |
| GT-05856 | 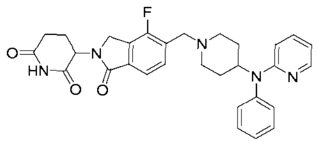 | 3-(4-fluoro-1-oxo-5-((4-(phenyl(pyridin-2-yl)amino)piperidin-1-yl)methyl)isoindolin-2-yl)piperidine-2,6-dione | 3-(4-fluoro-1-oxo-5-((4-(phenyl(pyridin-2-yl)amino)piperidin-1-yl)methyl)isoindolin-2-yl)piperidine-2,6-dione |
| GT-05857 | 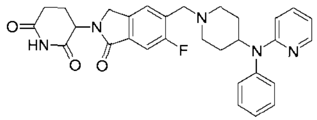 | 3-(6-fluoro-1-oxo-5-((4-(phenyl(pyridin-2-yl)amino)piperidin-1-yl)methyl)isoindolin-2-yl)piperidine-2,6-dione | 3-(6-fluoro-1-oxo-5-((4-(phenyl(pyridin-2-yl)amino)piperidin-1-yl)methyl)isoindolin-2-yl)piperidine-2,6-dione |

(continued)

| Compound No. | Structure of the compounds | The compounds' name | Chinese translation of the compounds' name |
|---|---|---|---|
| GT-05858 | 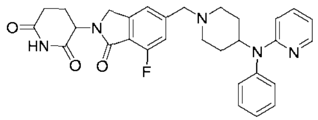 | 3-(7-fluoro-1-oxo-5-((4-(phenyl(pyridin-2-yl)amino)piperidin-1-yl)methyl)isoindolin-2-yl)piperidine-2,6-dione | 3-(7-fluoro-1-oxo-5-((4-(phenyl(pyridin-2-yl)amino)piperidin-1-yl)methyl)isoindolin-2-yl)piperidine-2,6-dione |
| GT-04948 | 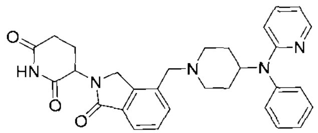 | 3-(1-oxo-4-((4-(phenyl(pyridin-2-yl)amino)piperidin-1-yl) methyl)isoindolin-2-yl)piperidine-2,6-dione | 3-(1-oxo-4-((4-(phenyl(pyridin-2-yl)amino)piperidin-1-yl) methyl)isoindolin-2-yl)piperidine-2,6-dione |
| GT-06806 | 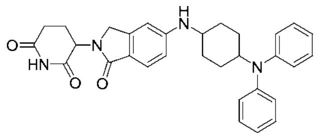 | 3-(5-((4-(diphenylamino)cyclohexyl)a mino)-1-oxoisoindolin-2-yl)piperidine-2,6-dione | 3-(5-((4-(diphenylamino)cyclohexyl )amino)- 1 -oxoisoindolin-2-yl)piperidine-2,6-dione |
| GT-05689 | 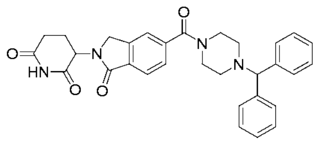 | 3-(5-(4-benzhydrylpiperazine-1-carbonyl)-1-oxoisoindolin-2-yl)piperidine-2,6-dione | 3-(5-(4-benzhydrylpiperazine-1-carbonyl)-1-oxoisoindolin-2-yl)piperidine-2,6-dione |
| | 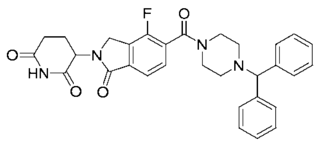 | 3-(5-(4-benzhydrylpiperazine-1-carbonyl)-4-fluoro-1-oxoisoindolin-2-yl)piperidine-2,6-dione | 3-(5-(4-benzhydrylpiperazine-1-carbonyl)-4-fluoro-1-oxoisoindolin-2-yl)piperidine-2,6-dione |
| | 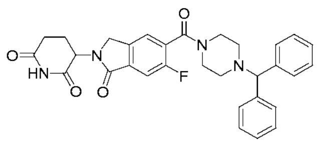 | 3-(5-(4-benzhydrylpiperazine-1-carbonyl)-6-fluoro-1-oxoisoindolin-2-yl)piperidine-2,6-dione | 3-(5-(4-benzhydrylpiperazine-1-carbonyl)-6-fluoro-1-oxoisoindolin-2-yl)piperidine-2,6-dione |
| GT-05850 | 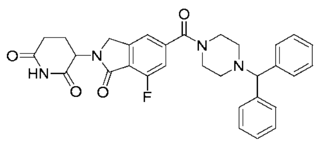 | 3-(5-(4-benzhydrylpiperazine-1-carbonyl)-7-fluoro-1-oxoisoindolin-2-yl)piperidine-2,6-dione | 3-(5-(4-benzhydrylpiperazine-1-carbonyl)-7-fluoro-1-oxoisoindolin-2-yl)piperidine-2,6-dione |

(continued)

| Compound No. | Structure of the compounds | The compounds' name | Chinese translation of the compounds' name |
|---|---|---|---|
| GT-05721 | 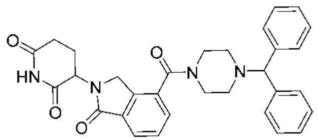 | 3-(4-(4-benzhydrylpiperazine-1-carbonyl)-1-oxoisoindolin-2-yl)piperidine-2,6-dione | 3-(4-(4-benzhydrylpiperazine-1-carbonyl)-1-oxoisoindolin-2-yl)piperidine-2,6-dione |
| GT-05851 | 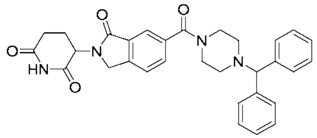 | 3-(6-(4-benzhydrylpiperazine-1-carbonyl)-1-oxoisoindolin-2-yl)piperidine-2,6-dione | 3-(6-(4-benzhydrylpiperazine-1-carbonyl)-1-oxoisoindolin-2-yl)piperidine-2,6-dione |
| GT-05852 | 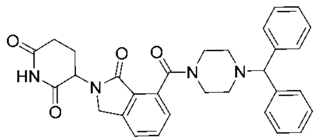 | 3-(7-(4-benzhydrylpiperazine-1-carbonyl)-1-oxoisoindolin-2-yl)piperidine-2,6-dione | 3-(7-(4-benzhydrylpiperazine-1-carbonyl)-1-oxoisoindolin-2-yl)piperidine-2,6-dione |
| | 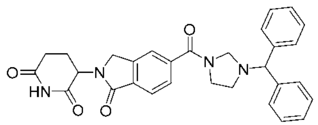 | 3-(5-(3-benzhydrylimidazolidine-1-carbonyl)-1-oxoisoindolin-2-yl)piperidine-2,6-dione | 3-(5-(3-benzhydrylimidazolidine-1-carbonyl)-1-oxoisoindolin-2-yl)piperidine-2,6-dione |
| | 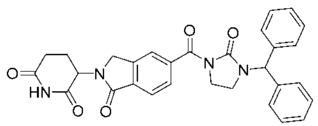 | 3-(5-(3-benzhydryl-2-oxoimidazolidine-1-carbonyl)-1-oxoisoindolin-2-yl)piperidine-2,6-dione | 3-(5-(3-benzhydryl-2-oxoimidazolidine-1-carbonyl)-1-oxoisoindolin-2-yl)piperidine-2,6-dione |
| GT-05892 | 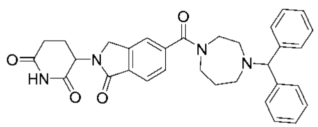 | 3-(5-(4-benzhydryl-1,4-diazepane-1-carbonyl)-1-oxoisoindolin-2-yl)piperidine-2,6-dione | 3-(5-(4-benzhydryl-1,4-diazepane-1-carbonyl)-1-oxoisoindolin-2-yl)piperidine-2,6-dione |
| | 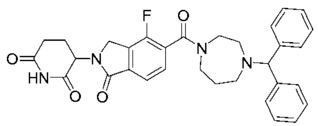 | 3-(5-(4-benzhydryl-1,4-diazepane-1-carbonyl)-4-fluoro-1-oxoisoindolin-2-yl)piperidine-2,6-dione | 3-(5-(4-benzhydryl-1,4-diazepane-1-carbonyl)-4-fluoro-1-oxoisoindolin-2-yl)piperidine-2,6-dione |

(continued)

| Compou nd No. | Structure of the compounds | The compounds' name | Chinese translation of the compounds' name |
|---|---|---|---|
| | 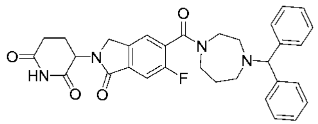 | 3-(5-(4-benzhydryl-1,4-diazepane-1-carbonyl)-6-fluoro-1-oxoisoindolin-2-yl)piperidine-2,6-dione | 3-(5-(4-benzhydryl-1,4-diazepane-1-carbonyl)-6-fluoro-1-oxoisoindolin-2-yl)piperidine-2,6-dione |
| | 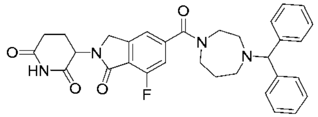 | 3-(5-(4-benzhydryl-1,4-diazepane-1-carbonyl)-7-fluoro-1-oxoisoindolin-2-yl)piperidine-2,6-dione | 3-(5-(4-benzhydryl-1,4-diazepane-1-carbonyl)-7-fluoro-1-oxoisoindolin-2-yl)piperidine-2,6-dione |
| GT-05988 | 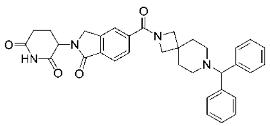 | 3-(5-(7-benzhydryl-2,7-diazaspiro[3.5]nonane-2-carbonyl)-1-oxoisoindolin-2-yl)piperidine-2,6-dione | 3-(5-(7-benzhydryl-2,7-diazaspiro[3.5]nonane-2-carbonyl)-1-oxoisoindolin-2-yl)piperidine-2,6-dione |
| GT-06063 | 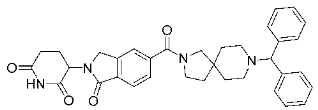 | 3-(5-(8-benzhydryl-2,8-diazaspiro[4.5]decane-2-carbonyl)-1-oxoisoindolin-2-yl)piperidine-2,6-dione | 3-(5-(8-benzhydryl-2,8-diazaspiro[4.5]decane-2-carbonyl)-1-oxoisoindolin-2-yl)piperidine-2,6-dione |
| GT-06065 | 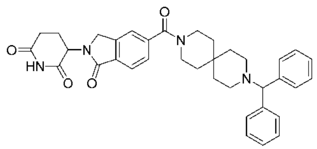 | 3-(5-(9-benzhydryl-3,9-diazaspiro[5.5]undecane-3-carbonyl)-1-oxoisoindolin-2-yl)piperidine-2,6-dione | 3-(5-(9-benzhydryl-3,9-diazaspiro[5.5]undecane-3-carbonyl)-1-oxoisoindolin-2-yl)piperidine-2,6-dione |
| GT-06067 | 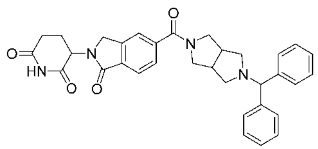 | 3-(5-(5-benzhydryloctahydropyrrolo[ 3,4-c]pyrrole-2-carbonyl)-1-oxoisoindolin-2-yl)piperidine-2,6-dione | 3-(5-(5-benzhydryloctahydropyrrol o[3,4-c]pyrrole-2-carbonyl)-1-oxoisoindolin-2-yl)piperidine-2,6-dione |
| GT-05891 | 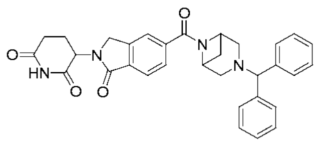 | 3-(5-(3-benzhydryl-3,6-diazabicyclo[3.1.1]heptane-6-carbonyl)-1-oxoisoindolin-2-yl)piperidine-2,6-dione | 3-(5-(3-benzhydryl-3,6-diazabicyclo[3.1.1]heptane-6-carbonyl)-1-oxoisoindolin-2-yl)piperidine-2,6-dione |

(continued)

| Compound No. | Structure of the compounds | The compounds' name | Chinese translation of the compounds' name |
|---|---|---|---|
| | 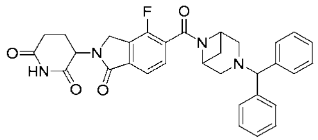 | 3-(5-(3-benzhydryl-3,6-diazabicyclo[3.1.1]heptane-6-carbonyl)-4-fluoro-1-oxoisoindolin-2-yl)piperidine-2,6-dione | 3-(5-(3-benzhydryl-3,6-diazabicyclo[3.1.1]heptane-6-carbonyl)-4-fluoro-1-oxoisoindolin-2-yl)piperidine-2,6-dione |
| | 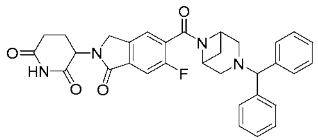 | 3-(5-(3-benzhydryl-3,6-diazabicyclo[3.1.1]heptane-6-carbonyl)-6-fluoro-1-oxoisoindolin-2-yl)piperidine-2,6-dione | 3-(5-(3-benzhydryl-3,6-diazabicyclo[3.1.1]heptane-6-carbonyl)-6-fluoro-1-oxoisoindolin-2-yl)piperidine-2,6-dione |
| GT-05916 | 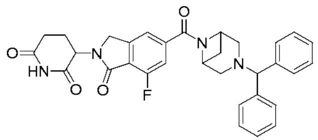 | 3-(5-(3-benzhydryl-3,6-diazabicyclo[3.1.1]heptane-6-carbonyl)-7-fluoro-1-oxoisoindolin-2-yl)piperidine-2,6-dione | 3-(5-(3-benzhydryl-3,6-diazabicyclo[3.1.1]heptane-6-carbonyl)-7-fluoro-1-oxoisoindolin-2-yl)piperidine-2,6-dione |
| GT-05990 | 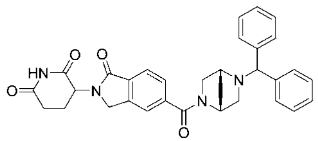 | 3-(5-((1R,4R)-5-benzhydryl-2,5-diazabicyclo[2.2.1]heptane-2-carbonyl)-1-oxoisoindolin-2-yl)piperidine-2,6-dione | 3-(5-((1R,4R)-5-benzhydryl-2,5-diazabicyclo[2.2.1]heptane-2-carbonyl)-1-oxoisoindolin-2-yl)piperidine-2,6-dione |
| | 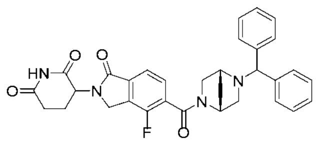 | 3-(5-((1R,4R)-5-benzhydryl-2,5-diazabicyclo[2.2.1]heptane-2-carbonyl)-4-fluoro-1-oxoisoindolin-2-yl)piperidine-2,6-dione | 3-(5-((1R,4R)-5-benzhydryl-2,5-diazabicyclo[2.2.1]heptane-2-carbonyl)-4-fluoro-1-oxoisoindolin-2-yl)piperidine-2,6-dione |
| | 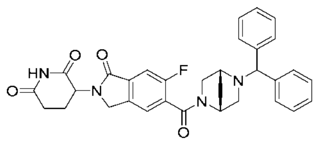 | 3-(5-((1R,4R)-5-benzhydryl-2,5-diazabicyclo[2.2.1]heptane-2-carbonyl)-6-fluoro-1-oxoisoindolin-2-yl)piperidine-2,6-dione | 3-(5-((1R,4R)-5-benzhydryl-2,5-diazabicyclo[2.2.1]heptane-2-carbonyl)-6-fluoro-1-oxoisoindolin-2-yl)piperidine-2,6-dione |
| GT-05991 | 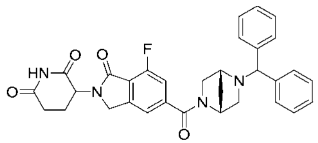 | 3-(5-((1R,4R)-5-benzhydryl-2,5-diazabicyclo[2.2.1]heptane-2-carbonyl)-7-fluoro-1-oxoisoindolin-2-yl)piperidine-2,6-dione | 3-(5-((1R,4R)-5-benzhydryl-2,5-diazabicyclo[2.2.1]heptane-2-carbonyl)-7-fluoro-1-oxoisoindolin-2-yl)piperidine-2,6-dione |

(continued)

| Compound No. | Structure of the compounds | The compounds' name | Chinese translation of the compounds' name |
|---|---|---|---|
| GT-05977 | 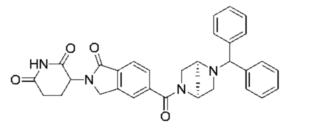 | 3-(5-((1S,4S)-5-benzhydryl-2,5-diazabicyclo[2.2.1]heptane-2-carbonyl)-1-oxoisoindolin-2-yl)piperidine-2,6-dione | 3-(5-((1S,4S)-5-benzhydryl-2,5-diazabicyclo[2.2.1]heptane-2-carbonyl)-1-oxoisoindolin-2-yl)piperidine-2,6-dione |
| | 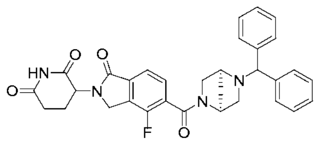 | 3-(5-((1S,4S)-5-benzhydryl-2,5-diazabicyclo[2.2.1]heptane-2-carbonyl)-4-fluoro-1-oxoisoindolin-2-yl)piperidine-2,6-dione | 3-(5-((1S,4S)-5-benzhydryl-2,5-diazabicyclo[2.2.1]heptane-2-carbonyl)-4-fluoro-1-oxoisoindolin-2-yl)piperidine-2,6-dione |
| | 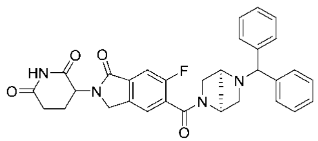 | 3-5-((1S,4S)-5-benzhydryl-2,5-diazabicyclo[2.2.1]heptane-2-carbonyl)-6-fluoro-1-oxoisoindolin-2-yl)piperidine-2,6-dione | 3-(5-((1S,4S)-5-benzhydryl-2,5-diazabicyclo[2.2.1]heptane-2-carbonyl)-6-fluoro-1-oxoisoindolin-2-yl)piperidine-2,6-dione |
| GT-05978 | 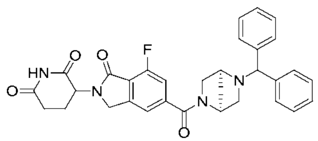 | 3-5-((1S,4S)-5-benzhydryl-2,5-diazabicyclo[2.2.1]heptane-2-carbonyl)-7-fluoro-1-oxoisoindolin-2-yl)piperidine-2,6-dione | 3-(5-((1S,4S)-5-benzhydryl-2,5-diazabicyclo[2.2.1]heptane-2-carbonyl)-7-fluoro-1-oxoisoindolin-2-yl)piperidine-2,6-dione |
| GT-05983 | 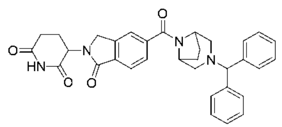 | 3-(5-(3-benzhydryl-3,8-diazabicyclo[3.2.1]octane-8-carbonyl)-1-oxoisoindolin-2-yl)piperidine-2,6-dione | 3-(5-(3-benzhydryl-3,8-diazabicyclo[3.2.1]octane-8-carbonyl)-1-oxoisoindolin-2-yl)piperidine-2,6-dione |
| | 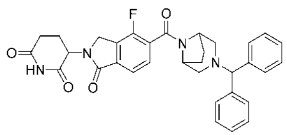 | 3-(5-(3-benzhydryl-3,8-diazabicyclo[3.2.1]octane-8-carbonyl)-4-fluoro-1-oxoisoindolin-2-yl)piperidine-2,6-dione | 3-(5-(3-benzhydryl-3,8-diazabicyclo[3.2.1]octane-8-carbonyl)-4-fluoro-1-oxoisoindolin-2-yl)piperidine-2,6-dione |
| | 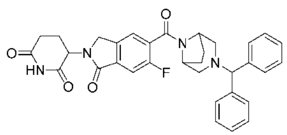 | 3-(5-(3-benzhydryl-3,8-diazabicyclo[3.2.1]octane-8-carbonyl)-6-fluoro-1-oxoisoindolin-2-yl)piperidine-2,6-dione | 3-(5-(3-benzhydryl-3,8-diazabicyclo[3.2.1]octane-8-carbonyl)-6-fluoro-1-oxoisoindolin-2-yl)piperidine-2,6-dione |

(continued)

| Compound No. | Structure of the compounds | The compounds' name | Chinese translation of the compounds' name |
|---|---|---|---|
| GT-06055 | 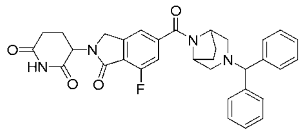 | 3-(5-(3-benzhydryl-3,8-diazabicyclo[3.2.1]octane-8-carbonyl)-7-fluoro-1-oxoisoindolin-2-yl)piperidine-2,6-dione | 3-(5-(3-benzhydryl-3,8-diazabicyclo[3.2.1]octane-8-carbonyl)-7-fluoro-1-oxoisoindolin-2-yl)piperidine-2,6-dione |
| GT-05980 | 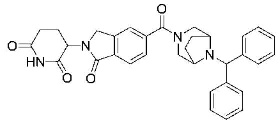 | 3-(5-(8-benzhydryl-3,8-diazabicyclo[3.2.1]octane-3-carbonyl)-1-oxoisoindolin-2-yl)piperidine-2,6-dione | 3-(5-(8-benzhydryl-3,8-diazabicyclo[3.2.1]octane-3-carbonyl)-1-oxoisoindolin-2-yl)piperidine-2,6-dione |
| | 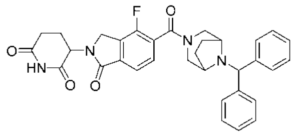 | 3-(5-(8-benzhydryl-3,8-diazabicyclo[3.2.1]octane-3-carbonyl)-4-fluoro-1-oxoisoindolin-2-yl)piperidine-2,6-dione | 3-(5-(8-benzhydryl-3,8-diazabicyclo[3.2.1]octane-3-carbonyl)-4-fluoro-1-oxoisoindolin-2-yl)piperidine-2,6-dione |
| | 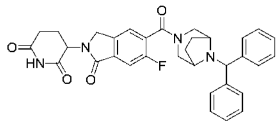 | 3-(5-(8-benzhydryl-3,8-diazabicyclo[3.2.1]octane-3-carbonyl)-6-fluoro-1-oxoisoindolin-2-yl)piperidine-2,6-dione | 3-(5-(8-benzhydryl-3,8-diazabicyclo[3.2.1]octane-3-carbonyl)-6-fluoro-1-oxoisoindolin-2-yl)piperidine-2,6-dione |
| GT-05981 | 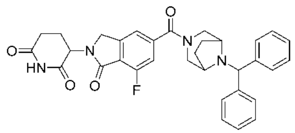 | 3-(5-(8-benzhydryl-3,8-diazabicyclo[3.2.1]octane-3-carbonyl)-7-fluoro-1-oxoisoindolin-2-yl)piperidine-2,6-dione | 3-(5-(8-benzhydryl-3,8-diazabicyclo[3.2.1]octane-3-carbonyl)-7-fluoro-1-oxoisoindolin-2-yl)piperidine-2,6-dione |
| GT-06092 | 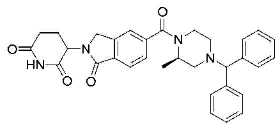 | 3-(5-((R)-4-benzhydryl-2-methylpiperazine-1-carbonyl)-1-oxoisoindolin-2-yl)piperidine-2,6-dione | 3-(5-((R)-4-benzhydryl-2-methylpiperazine-1-carbonyl)-1-oxoisoindolin-2-yl)piperidine-2,6-dione |
| | 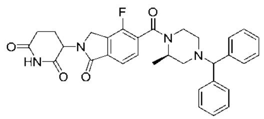 | 3-(5-((R)-4-benzhydryl-2-methylpiperazine-1-carbonyl)-4-fluoro-1-oxoisoindolin-2-yl)piperidine-2,6-dione | 3-(5-((R)-4-benzhydryl-2-methylpiperazine-1-carbonyl)-4-fluoro-1-oxoisoindolin-2-yl)piperidine-2,6-dione |

(continued)

| Compound No. | Structure of the compounds | The compounds' name | Chinese translation of the compounds' name |
|---|---|---|---|
| | 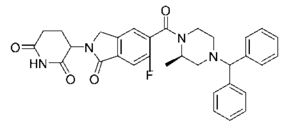 | 3-(5-((R)-4-benzhydryl-2-methylpiperazine-1-carbonyl)-6-fluoro-1-oxoisoindolin-2-yl)piperidine-2,6-dione | 3-(5-((R)-4-benzhydryl-2-methylpiperazine-1-carbonyl)-6-fluoro-1-oxoisoindolin-2-yl)piperidine-2,6-dione |
| GT-06093 | 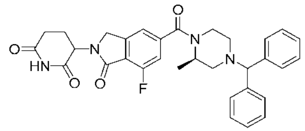 | 3-(5-((R)-4-benzhydryl-2-methylpiperazine-1-carbonyl)-7-fluoro-1-oxoisoindolin-2-yl)piperidine-2,6-dione | 3-(5-((R)-4-benzhydryl-2-methylpiperazine-1-carbonyl)-7-fluoro-1-oxoisoindolin-2-yl)piperidine-2,6-dione |
| GT-05920 | 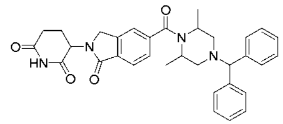 | 3-(5-(4-benzhydryl-2,6-dimethylpiperazine-1-carbonyl)-1-oxoisoindolin-2-yl)piperidine-2,6-dione | 3-(5-(4-benzhydryl-2,6-dimethylpiperazine-1-carbonyl)-1-oxoisoindolin-2-yl)piperidine-2,6-dione |
| | 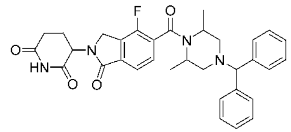 | 3-(5-(4-benzhydryl-2,6-dimethylpiperazine-1-carbonyl)-4-fluoro-1-oxoisoindolin-2-yl)piperidine-2,6-dione | 3-(5-(4-benzhydryl-2,6-dimethylpiperazine-1-carbonyl)-4-fluoro-1-oxoisoindolin-2-yl)piperidine-2,6-dione |
| | 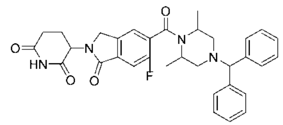 | 3-(5-(4-benzhydryl-2,6-dimethylpiperazine-1-carbonyl)-6-fluoro-1-oxoisoindolin-2-yl)piperidine-2,6-dione | 3-(5-(4-benzhydryl-2,6-dimethylpiperazine-1-carbonyl)-6-fluoro-1-oxoisoindolin-2-yl)piperidine-2,6-dione |
| GT-05921 | 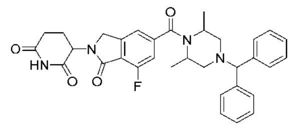 | 3-(5-(4-benzhydryl-2,6-dimethylpiperazine-1-carbonyl)-7-fluoro-1-oxoisoindolin-2-yl)piperidine-2,6-dione | 3-(5-(4-benzhydryl-2,6-dimethylpiperazine-1-carbonyl)-7-fluoro-1-oxoisoindolin-2-yl)piperidine-2,6-dione |
| GT-06059 | 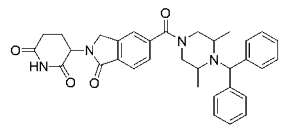 | 3-(5-(4-benzhydryl-3,5-dimethylpiperazine-1-carbonyl)-1-oxoisoindolin-2-yl)piperidine-2,6-dione | 3-(5-(4-benzhydryl-3,5-dimethylpiperazine-1-carbonyl)-1-oxoisoindolin-2-yl)piperidine-2,6-dione |

(continued)

| Compound No. | Structure of the compounds | The compounds' name | Chinese translation of the compounds' name |
|---|---|---|---|
| GT-06060 | 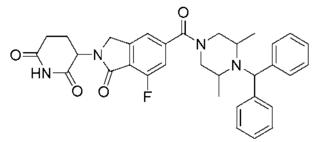 | 3-(5-(4-benzhydryl-3,5-dimethylpiperazine-1-carbonyl)-7-fluoro-1-oxoisoindolin-2-yl)piperidine-2,6-dione | 3-(5-(4-benzhydryl-3,5-dimethylpiperazine-1-carbonyl)-7-fluoro-1-oxoisoindolin-2-yl)piperidine-2,6-dione |
| GT-06056 | 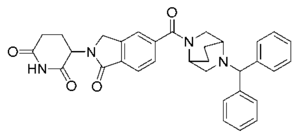 | 3-(5-(5-benzhydryl-2,5-diazabicyclo[2.2.2]octane-2-carbonyl)-1-oxoisoindolin-2-yl)piperidine-2,6-dione | 3-(5-(5-benzhydryl-2,5-diazabicyclo [2.2.2] octane-2-carbonyl)-1-oxoisoindolin-2-yl)piperidine-2,6-dione |
| | 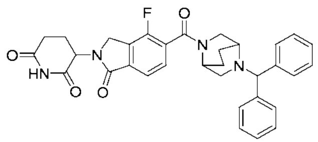 | 3-(5-(5-benzhydryl-2,5-diazabicyclo[2.2.2]octane-2-carbonyl)-4-fluoro-1-oxoisoindolin-2-yl)piperidine-2,6-dione | 3-(5-(5-benzhydryl-2,5-diazabicyclo [2.2.2] octane-2-carbonyl)-4-fluoro-1-oxoisoindolin-2-yl)piperidine-2,6-dione |
| | 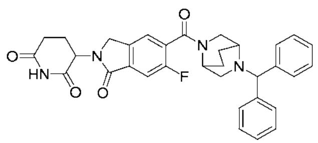 | 3-(5-(5-benzhydryl-2,5-diazabicyclo[2.2.2]octane-2-carbonyl)-6-fluoro-1-oxoisoindolin-2-yl)piperidine-2,6-dione | 3-(5-(5-benzhydryl-2,5-diazabicyclo [2.2.2]octane-2-carbonyl)-6-fluoro-1-oxoisoindolin-2-yl)piperidine-2,6-dione |
| GT-06057 | 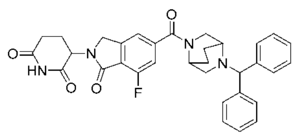 | 3-(5-(5-benzhydryl-2,5-diazabicyclo[2.2.2]octane-2-carbonyl)-7-fluoro-1-oxoisoindolin-2-yl)piperidine-2,6-dione | 3-(5-(5-benzhydryl-2,5-diazabicyclo [2.2.2]octane-2-carbonyl)-7-fluoro-1-oxoisoindolin-2-yl)piperidine-2,6-dione |
| | 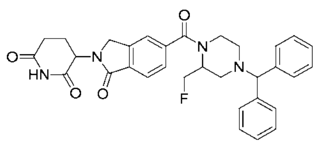 | 3-(5-(4-benzhydryl-2-(fluoromethyl)piperazine-1-carbonyl)-1-oxoisoindolin-2-yl)piperidine-2,6-dione | 3-(5-(4-benzhydryl-2-(fluoromethyl)piperazine-1-carbonyl)-1-oxoisoindolin-2-yl)piperidine-2,6-dione |
| | 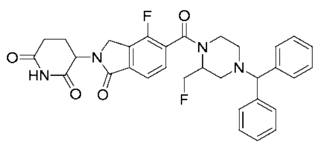 | 3-(5-(4-benzhydryl-2-(fluoromethyl)piperazine-1-carbonyl)-4-fluoro-1-oxoisoindolin-2-yl)piperidine-2,6-dione | 3-(5-(4-benzhydryl-2-(fluoromethyl)piperazine-1-carbonyl)-4-fluoro-1-oxoisoindolin-2-yl)piperidine-2,6-dione |

(continued)

| Compound No. | Structure of the compounds | The compounds' name | Chinese translation of the compounds' name |
|---|---|---|---|
| | 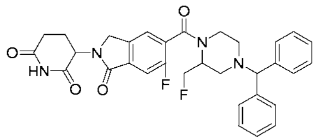 | 3-(5-(4-benzhydryl-2-(fluoromethyl)piperazine-1-carbonyl)-6-fluoro-1-oxoisoindolin-2-yl)piperidine-2,6-dione | 3-(5-(4-benzhydryl-2-(fluoromethyl)piperazine-1-carbonyl)-6-fluoro-1-oxoisoindolin-2-yl)piperidine-2,6-dione |
| | 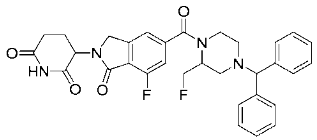 | 3-(5-(4-benzhydryl-2-(fluoromethyl)piperazine-1-carbonyl)-7-fluoro-1-oxoisoindolin-2-yl)piperidine-2,6-dione | 3-(5-(4-benzhydryl-2-(fluoromethyl)piperazine-1-carbonyl)-7-fluoro-1-oxoisoindolin-2-yl)piperidine-2,6-dione |
| GT-06071 | 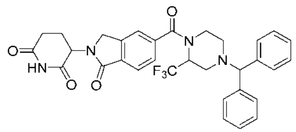 | 3-(5-(4-benzhydryl-2-(trifluoromethyl)piperazine-1-carbonyl)-1-oxoisoindolin-2-yl)piperidine-2,6-dione | 3-(5-(4-benzhydryl-2-(trifluoromethyl)piperazine -1-carbonyl)-1-oxoisoindolin-2-yl)piperidine-2,6-dione |
| | 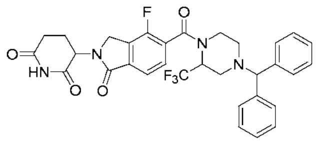 | 3-(5-(4-benzhydryl-2-(trifluoromethyl)piperazine-1-carbonyl)-4-fluoro-1-oxoisoindolin-2-yl)piperidine-2,6-dione | 3-(5-(4-benzhydryl-2-(trifluoromethyl)piperazine -1-carbonyl)-4-fluoro-1-oxoisoindolin-2-yl)piperidine-2,6-dione |
| | 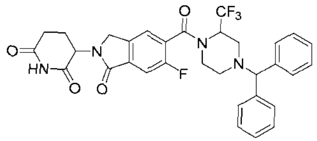 | 3-(5-(4-benzhydryl-2-(trifluoromethyl)piperazine-1-carbonyl)-6-fluoro-1-oxoisoindolin-2-yl)piperidine-2,6-dione | 3-(5-(4-benzhydryl-2-(trifluoromethyl)piperazine -1-carbonyl)-6-fluoro-1-oxoisoindolin-2-yl)piperidine-2,6-dione |
| GT-06090 | 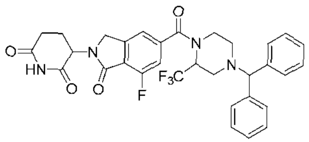 | 3-(5-(4-benzhydryl-2-(trifluoromethyl)piperazine-1-carbonyl)-7-fluoro-1-oxoisoindolin-2-yl)piperidine-2,6-dione | 3-(5-(4-benzhydryl-2-(trifluoromethyl)piperazine -1-carbonyl)-7-fluoro-1-oxoisoindolin-2-yl)piperidine-2,6-dione |
| GT-06061 | 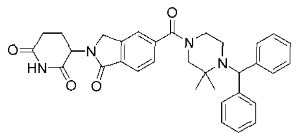 | 3-(5-(4-benzhydryl-3,3-dimethylpiperazine-1-carbonyl)-1-oxoisoindolin-2-yl)piperidine-2,6-dione | 3-(5-(4-benzhydryl-3,3-dimethylpiperazine-1-carbonyl)-1-oxoisoindolin-2-yl)piperidine-2,6-dione |

(continued)

| Compound No. | Structure of the compounds | The compounds' name | Chinese translation of the compounds' name |
|---|---|---|---|
| | 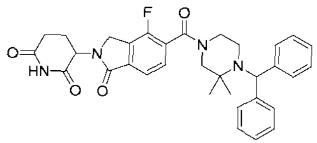 | 3-(5-(4-benzhydryl-3,3-dimethylpiperazine-1-carbonyl)-4-fluoro-1-oxoisoindolin-2-yl)piperidine-2,6-dione | 3-(5-(4-benzhydryl-3,3-dimethylpiperazine-1-carbonyl)-4-fluoro-1-oxoisoindolin-2-yl)piperidine-2,6-dione |
| | 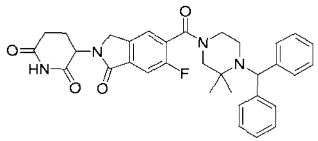 | 3-(5-(4-benzhydryl-3,3-dimethylpiperazine-1-carbonyl)-6-fluoro-1-oxoisoindolin-2-yl)piperidine-2,6-dione | 3-(5-(4-benzhydryl-3,3-dimethylpiperazine-1-carbonyl)-6-fluoro-1-oxoisoindolin-2-yl)piperidine-2,6-dione |
| GT-06062 | 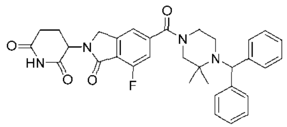 | 3-(5-(4-benzhydryl-3,3-dimethylpiperazine-1-carbonyl)-7-fluoro-1-oxoisoindolin-2-yl)piperidine-2,6-dione | 3-(5-(4-benzhydryl-3,3-dimethylpiperazine-1-carbonyl)-7-fluoro-1-oxoisoindolin-2-yl)piperidine-2,6-dione |
| GT-05854 | 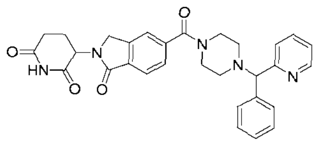 | 3-(1-oxo-5-(4-(phenyl(pyridin-2-yl)methyl)piperazine-1-carbonyl)isoindolin-2-yl)piperidine-2,6-dione | 3-(1-oxo-5-(4-(phenyl(pyridin-2-yl)methyl)piperazine-1-carbonyl)isoindolin-2-yl)piperidine-2,6-dione |
| | 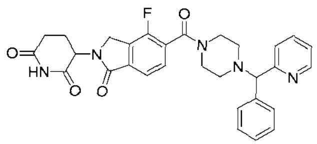 | 3-(4-fluoro-1-oxo-5-(4-(phenyl(pyridin-2-yl)methyl)piperazine-1-carbonyl)isoindolin-2-yl)piperidine-2,6-dione | 3-(4-fluoro-1-oxo-5-(4-(phenyl(pyridin-2-yl)methyl)piperazine-1-carbonyl)isoindolin-2-yl)piperidine-2,6-dione |
| | 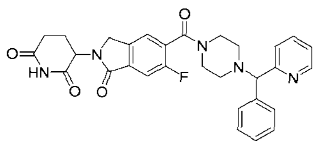 | 3-(6-fluoro-1-oxo-5-(4-(phenyl(pyridin-2-yl)methyl)piperazine-1-carbonyl)isoindolin-2-yl)piperidine-2,6-dione | 3-(6-fluoro-1-oxo-5-(4-(phenyl(pyridin-2-yl)methyl)piperazine-1-carbonyl)isoindolin-2-yl)piperidine-2,6-dione |
| GT-05877 | 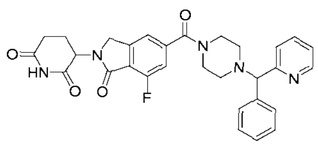 | 3-(7-fluoro-1-oxo-5-(4-(phenyl(pyridin-2-yl)methyl)piperazine-1-carbonyl)isoindolin-2-yl)piperidine-2,6-dione | 3-(7-fluoro-1-oxo-5-(4-(phenyl(pyridin-2-yl)methyl)piperazine-1-carbonyl)isoindolin-2-yl)piperidine-2,6-dione |

(continued)

| Compound No. | Structure of the compounds | The compounds' name | Chinese translation of the compounds' name |
|---|---|---|---|
| GT-05929 | 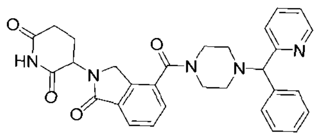 | 3-(1-oxo-4-(4-(phenyl(pyridin-2-yl)methyl)piperazine-1-carbonyl)isoindolin-2-yl)piperidine-2,6-dione | 3-(1-oxo-4-(4-(phenyl(pyridin-2-yl)methyl)piperazine-1-carbonyl)isoindolin-2-yl)piperidine-2,6-dione |
| GT-06142 | 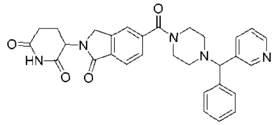 | 3-(1-oxo-5-(4-(phenyl(pyridin-3-yl)methyl)piperazine-1-carbonvl)isoindolin-2-yl)piperidine-2,6-dione | 3-(1-oxo-5-(4-(phenyl(pyridin-3-yl)methyl)piperazine-1-carbonyl)isoindolin-2-yl)piperidine-2,6-dione |
| GT-06144 | 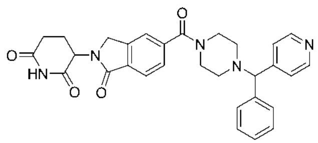 | 3-(1-oxo-5-(4-(phenyl(pyridin-4-yl)methyl)piperazine-1-carbonyl)isoindolin-2-yl)piperidine-2,6-dione | 3-(1-oxo-5-(4-(phenyl(pyridin-4-yl)methyl)piperazine-1-carbonyl)isoindolin-2-yl)piperidine-2,6-dione |
| GT-05881 | 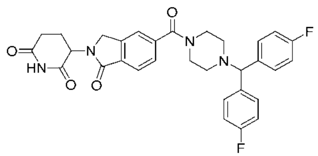 | 3-(5-(4-(bis(4-fluorophenyl)methyl)piperazi ne-1-carbonyl)-1-oxoisoindolin-2-yl)piperidine-2,6-dione | 3-(5-(4-(bis(4-fluorophenyl)methyl)pipera zine-1-carbonyl)-1-oxoisoindolin-2-yl)piperidine-2,6-dione |
| | 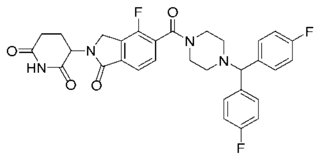 | 3-(5-(4-(bis(4-fluorophenyl)methyl)piperazi ne-1-carbonyl)-4-fluoro-1-oxoisoindolin-2-yl)piperidine-2,6-dione | 3-(5-(4-(bis(4-fluorophenyl)methyl)pipera zine-1-carbonyl)-4-fluoro-1-oxoisoindolin-2-yl)piperidine-2,6-dione |
| | 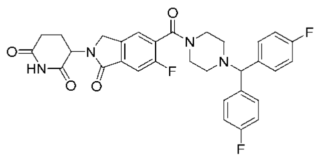 | 3-(5-(4-(bis(4-fluorophenyl)methyl)piperazi ne-1-carbonyl)-6-fluoro-1-oxoisoindolin-2-yl)piperidine-2,6-dione | 3-(5-(4-(bis(4-fluorophenyl)methyl)pipera zine-1-carbonyl)-6-fluoro-1-oxoisoindolin-2-yl)piperidine-2,6-dione |

(continued)

| Compou nd No. | Structure of the compounds | The compounds' name | Chinese translation of the compounds' name |
|---|---|---|---|
| GT-05882 | 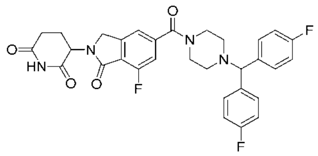 | 3-(5-(4-(bis(4-fluorophenyl)methyl)piperazi ne-1-carbonyl)-7-fluoro-1-oxoisoindolin-2-yl)piperidine-2,6-dione | 3-(5-(4-(bis(4-fluorophenyl)methyl)pipera zine-1-carbonyl)-7-fluoro-1-oxoisoindolin-2-yl)piperidine-2,6-dione |
| GT-05930 | 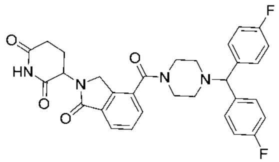 | 3-(4-(4-(bis(4-fluorophenyl)methyl)piperazi ne-1-carbonyl)-1-oxoisoindolin-2-yl)piperidine-2,6-dione | 3-(4-(4-(bis(4-fluorophenyl)methyl)pipera zine-1-carbonyl)-1-oxoisoindolin-2-yl)piperidine-2,6-dione |
| GT-05883 | 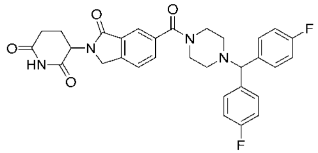 | 3-(6-(4-(bis(4-fluorophenyl)methyl)piperazi ne-1-carbonyl)-1-oxoisoindolin-2-yl)piperidine-2,6-dione | 3-(6-(4-(bis(4-fluorophenyl)methyl)pipera zine-1-carbonyl)-1-oxoisoindolin-2-yl)piperidine-2,6-dione |
| GT-05884 | 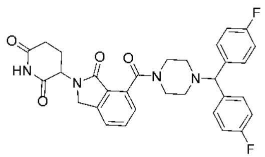 | 3-(7-(4-(bis(4-fluorophenyl)methyl)piperazi ne-1-carbonyl)-1-oxoisoindolin-2-yl)piperidine-2,6-dione | 3-(7-(4-(bis(4-fluorophenyl)methyl)pipera zine-1-carbonyl)-1-oxoisoindolin-2-yl)piperidine-2,6-dione |
| GT-06146 | 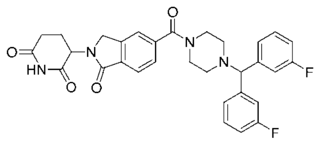 | 3-(5-(4-(bis(3-fluorophenyl)methyl)piperazi ne-1-carbonyl)-1-oxoisoindolin-2-yl)piperidine-2,6-dione | 3-(5-(4-(bis(3-fluorophenyl)methyl)pipera zine-1-carbonyl)-1-oxoisoindolin-2-yl)piperidine-2,6-dione |
|  | 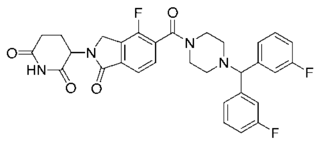 | 3-(5-(4-(bis(3-fluorophenyl)methyl)piperazi ne-1-carbonyl)-4-fluoro-1-oxoisoindolin-2-yl)piperidine-2,6-dione | 3-(5-(4-(bis(3-fluorophenyl)methyl)pipera zine-1-carbonyl)-4-fluoro-1-oxoisoindolin-2-yl)piperidine-2,6-dione |

(continued)

| Compou nd No. | Structure of the compounds | The compounds' name | Chinese translation of the compounds' name |
|---|---|---|---|
| | 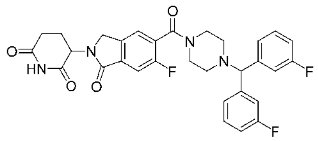 | 3-(5-(4-(bis(3-fluorophenyl)methyl)piperazi ne-1-carbonyl)-6-fluoro-1-oxoisoindolin-2-yl)piperidine-2,6-dione | 3-(5-(4-(bis(3-fluorophenyl)methyl)pipera zine-1-carbonyl)-6-fluoro-1-oxoisoindolin-2-yl)piperidine-2,6-dione |
| GT-06147 | 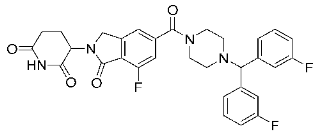 | 3-(5-(4-(bis(3-fluorophenyl)methyl)piperazi ne-1-carbonyl)-7-fluoro-1-oxoisoindolin-2-yl)piperidine-2,6-dione | 3-(5-(4-(bis(3-fluorophenyl)methyl)pipera zine-1-carbonyl)-7-fluoro-1-oxoisoindolin-2-yl)piperidine-2,6-dione |
| GT-06148 | 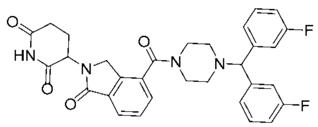 | 3-(4-(4-(bis(3-fluorophenyl)methyl)piperazi ne-1-carbonyl)-1-oxoisoindolin-2-yl)piperidine-2,6-dione | 3-(4-(4-(bis(3-fluorophenyl)methyl)pipera zine-1-carbonyl)-1-oxoisoindolin-2-yl)piperidine-2,6-dione |
| GT-06149 | 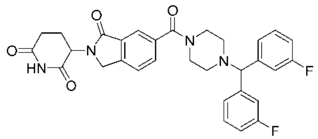 | 3-(6-(4-(bis(3-fluorophenyl)methyl)piperazi ne-1-carbonyl)-1-oxoisoindolin-2-yl)piperidine-2,6-dione | 3-(6-(4-(bis(3-fluorophenyl)methyl)pipera zine-1-carbonyl)-1-oxoisoindolin-2-yl)piperidine-2,6-dione |
| GT-06150 | 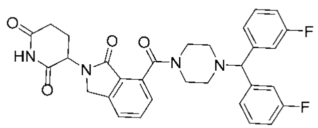 | 3-(7-(4-(bis(3-fluorophenyl)methyl)piperazi ne-1-carbonyl)-1-oxoisoindolin-2-yl)piperidine-2,6-dione | 3-(7-(4-(bis(3-fluorophenyl)methyl)pipera zine-1-carbonyl)-1-oxoisoindolin-2-yl)piperidine-2,6-dione |
| GT-06104 | 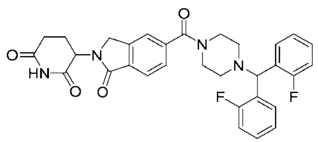 | 3-(5-(4-(bis(2-fluorophenyl)methyl)piperazi ne-1-carbonyl)-1-oxoisoindolin-2-yl)piperidine-2,6-dione | 3-(5-(4-(bis(2-fluorophenyl)methyl)pipera zine-1-carbonyl)-1-oxoisoindolin-2-yl)piperidine-2,6-dione |
| GT-06216 | 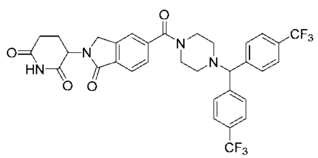 | 3-(5-(4-(bis(4-(trifluoromethyl)phenyl)meth yl)piperazine-1-carbonyl)-1-oxoisoindolin-2-yl)piperidine-2,6-dione | 3-(5-(4-(bis(4-(trifluoromethyl)phenyl)me thyl)piperazine-1-carbonyl)-1-oxoisoindolin-2-yl)piperidine-2,6-dione |

(continued)

| Compou nd No. | Structure of the compounds | The compounds' name | Chinese translation of the compounds' name |
|---|---|---|---|
| | 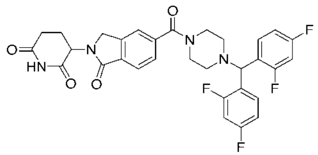 | 3-(5-(4-(bis(2,4-difluorophenyl)methyl)pipera zine-1-carbonyl)-1-oxoisoindolin-2-yl)piperidine-2,6-dione | 3-(5-(4-(bis(2,4-difluorophenyl)methyl)pipe razine-1-carbonyl)-1-oxoisoindolin-2-yl)piperidine-2,6-dione |
| | 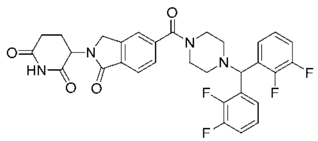 | 3-(5-(4-((2,3-difluorophenyl)(3,4-difluorophenyl)methyl)pipera zine-1-carbonyl)-1-oxoisoindolin-2-yl)piperidine-2,6-dione | 3-(5-(4-((2,3-difluorophenyl)(3,4-difluorophenyl)methyl) pipe razine-1-carbonyl)-1-oxoisoindolin-2-yl)piperidine-2,6-dione |
| GT-06597 | 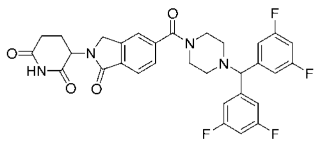 | 3-(5-(4-(bis(3,5-difluorophenyl)methyl)pipera zine-1-carbonyl)-1-oxoisoindolin-2-yl)piperidine-2,6-dione | 3-(5-(4-(bis(3,5-difluorophenyl)methyl)pipe razine-1-carbonyl)-1-oxoisoindolin-2-yl)piperidine-2,6-dione |
| | 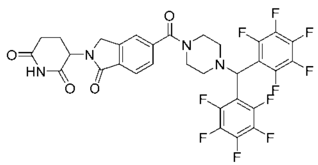 | 3-(5-(4-(bis(perfluorophenyl)methyl) piperazine-1-carbonyl)-1-oxoisoindolin-2-yl)piperidine-2,6-dione | 3-(5-(4-(bis(perfluorophenyl)methy l)piperazine-1-carbonyl)-1-oxoisoindolin-2-yl)piperidine-2,6-dione |
| GT-05886 | 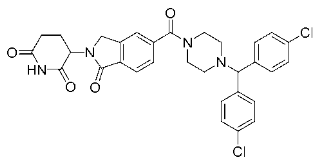 | 3-(5-(4-(bis(4-chlorophenyl)methyl)piperazi ne-1-carbonyl)-1-oxoisoindolin-2-yl)piperidine-2,6-dione | 3-(5-(4-(bis(4-chlorophenyl)methyl)piper azine-1-carbonyl)-1-oxoisoindolin-2-yl)piperidine-2,6-dione |
| | 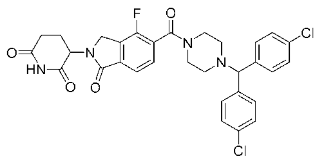 | 3-(5-(4-(bis(4-chlorophenyl)methyl)piperazi ne-1-carbonyl)-4-fluoro-1-oxoisoindolin-2-yl)piperidine-2,6-dione | 3-(5-(4-(bis(4-chlorophenyl)methyl)piper azine-1-carbonyl)-4-fluoro-1-oxoisoindolin-2-yl)piperidine-2,6-dione |

(continued)

| Compound No. | Structure of the compounds | The compounds' name | Chinese translation of the compounds' name |
|---|---|---|---|
| | 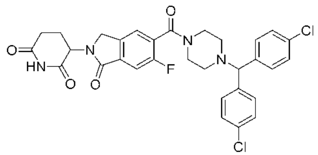 | 3-(5-(4-(bis(4-chlorophenyl)methyl)piperazi ne-1-carbonyl)-6-fluoro-1-oxoisoindolin-2-yl)piperidine-2,6-dione | 3-(5-(4-(bis(4-chlorophenyl)methyl)piper azine-1-carbonyl)-6-fluoro-1-oxoisoindolin-2-yl)piperidine-2,6-dione |
| GT-05887 | 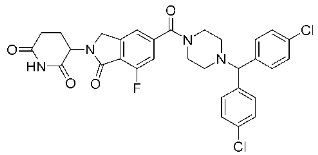 | 3-(5-(4-(bis(4-chlorophenyl)methyl)piperazi ne-1-carbonyl)-7-fluoro-1-oxoisoindolin-2-yl)piperidine-2,6-dione | 3-(5-(4-(bis(4-chlorophenyl)methyl)piper azine-1-carbonyl)-7-fluoro-1-oxoisoindolin-2-yl)piperidine-2,6-dione |
| GT-05985 | 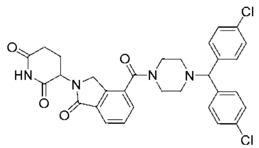 | 3-(4-(4-(bis(4-chlorophenyl)methyl)piperazi ne-1-carbonyl)-1-oxoisoindolin-2-yl)piperidine-2,6-dione | 3-(4-(4-(bis(4-chlorophenyl)methyl)piper azine-1-carbonyl)-1-oxoisoindolin-2-yl)piperidine-2,6-dione |
| GT-05888 | 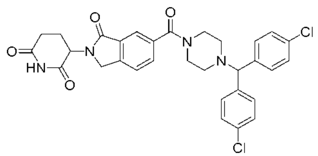 | 3-(6-(4-(bis(4-chlorophenyl)methyl)piperazi ne-1-carbonyl)-1-oxoisoindolin-2-yl)piperidine-2,6-dione | 3-(6-(4-(bis(4-chlorophenyl)methyl)piper azine-1-carbonyl)-1-oxoisoindolin-2-yl)piperidine-2,6-dione |
| GT-05889 | 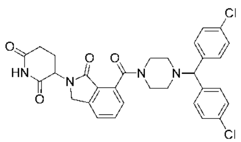 | 3-(7-(4-(bis(4-chlorophenyl)methyl)piperazi ne-1-carbonyl)-1-oxoisoindolin-2-yl)piperidine-2,6-dione | 3-(7-(4-(bis(4-chlorophenyl)methyl)piper azine-1-carbonyl)-1-oxoisoindolin-2-yl)piperidine-2,6-dione |
| | 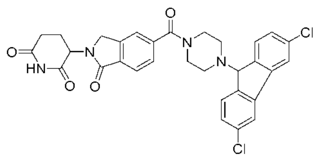 | 3-(5-(4-(3,6-dichloro-9H-fluoren-9-yl)piperazine-1-carbonyl)-1-oxoisoindolin-2-yl)piperidine-2,6-dione | 3-(5-(4-(3,6-dichloro-9H-fluoren-9-yl)piperazine-1-carbonyl)-1-oxoisoindolin-2-yl)piperidine-2,6-dione |

(continued)

| Compound No. | Structure of the compounds | The compounds' name | Chinese translation of the compounds' name |
|---|---|---|---|
| GT-06069 | 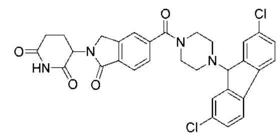 | 3-(5-(4-(2,7-dichloro-9H-fluoren-9-yl)piperazine-1-carbonyl)-1-oxoisoindolin-2-yl)piperidine-2,6-dione | 3-(5-(4-(2,7-dichloro-9H-fluoren-9-yl)piperazine-1-carbonyl)-1-oxoisoindolin-2-yl)piperidine-2,6-dione |
| GT-05986 | 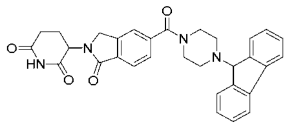 | 3-(5-(4-(9H-fluoren-9-yl)piperazine-1-carbonyl)-1-oxoisoindolin-2-yl)piperidine-2,6-dione | 3-(5-(4-(9H-fluoren-9-yl)piperazine-1-carbonyl)-1-oxoisoindolin-2-yl)piperidine-2,6-dione |
| | 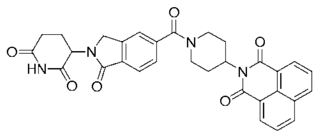 | 2-(1-(2-(2,6-dioxopiperidin-3-yl)-1-oxoisoindoline-5-carbonyl)piperidin-4-yl)-1H-benzo[de]isoquinoline-1,3(2H)-dione | 2-(1-(2-(2,6-dioxopiperidin-3-yl)-1-oxoisoindoline-5-carbonyl)piperidin-4-yl)-1H-benzo[de]isoquinoline-1,3(2H)-dione |
| | 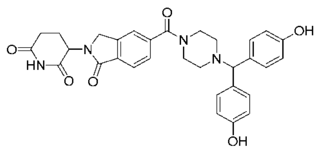 | 3-(5-(4-(bis(4-hydroxyphenyl)methyl)piper azine-1-carbonyl)-1-oxoisoindolin-2-yl)piperidine-2,6-dione | 3-(5-(4-(bis(4-hydroxyphenyl)methyl)pip erazine-1-carbonyl)-1-oxoisoindolin-2-yl)piperidine-2,6-dione |
| | 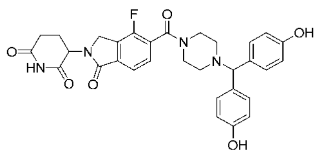 | 3-(5-(4-(bis(4-hydroxyphenyl)methyl)piper azine-1-carbonyl)-4-fluoro-1-oxoisoindolin-2-yl)piperidine-2,6-dione | 3-(5-(4-(bis(4-hydroxyphenyl)methyl)pip erazine-1-carbonyl)-4-fluoro-1-oxoisoindolin-2-yl)piperidine-2,6-dione |
| | 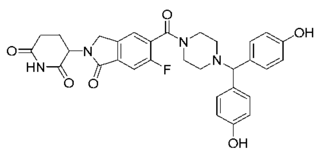 | 3-(5-(4-(bis(4-hydroxyphenyl)methyl)piper azine-1-carbonyl)-6-fluoro-1-oxoisoindolin-2-yl)piperidine-2,6-dione | 3-(5-(4-(bis(4-hydroxyphenyl)methyl)pip erazine-1-carbonyl)-6-fluoro-1-oxoisoindolin-2-yl)piperidine-2,6-dione |

(continued)

| Compound No. | Structure of the compounds | The compounds' name | Chinese translation of the compounds' name |
|---|---|---|---|
| | 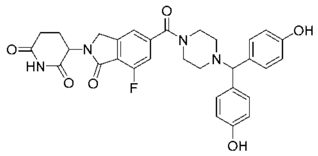 | 3-(5-(4-(bis(4-hydroxyphenyl)methyl)piper azine-1-carbonyl)-7-fluoro-1-oxoisoindolin-2-yl)piperidine-2,6-dione | 3-(5-(4-(bis(4-hydroxyphenyl)methyl)pip erazine-1-carbonyl)-7-fluoro-1-oxoisoindolin-2-yl)piperidine-2,6-dione |
| | 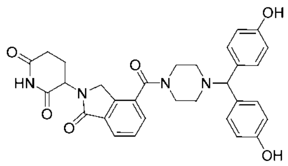 | 3-(4-(4-(bis(4-hydroxyphenyl)methyl)piper azine-1-carbonyl)-1-oxoisoindolin-2-yl)piperidine-2,6-dione | 3-(4-(4-(bis(4-hydroxyphenyl)methyl)pip erazine-1-carbonyl)-1-oxoisoindolin-2-yl)piperidine-2,6-dione |
| | 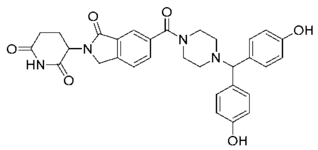 | 3-(6-(4-(bis(4-hydroxyphenyl)methyl)piper azine-1-carbonyl)-1-oxoisoindolin-2-yl)piperidine-2,6-dione | 3-(6-(4-(bis(4-hydroxyphenyl)methyl)pip erazine-1-carbonyl)-1-oxoisoindolin-2-yl)piperidine-2,6-dione |
| | 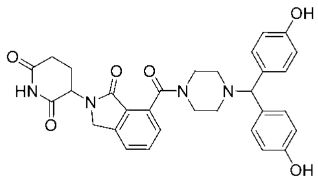 | 3-(7-(4-(bis(4-hydroxyphenyl)methyl)piper azine-1-carbonyl)-1-oxoisoindolin-2-yl)piperidine-2,6-dione | 3-(7-(4-(bis(4-hydroxyphenyl)methyl)pip erazine-1-carbonyl)-1-oxoisoindolin-2-yl)piperidine-2,6-dione |
| GT-06557 | 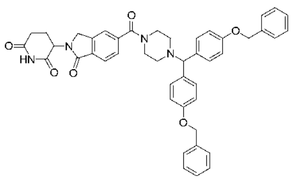 | 3-(5-(4-(bis(4-(benzyloxy)phenyl)methyl)pi perazine-1-carbonyl)-1-oxoisoindolin-2-yl)piperidine-2,6-dione | 3-(5-(4-(bis(4-(benzyloxy)phenyl)methyl) piperazine-1-carbonyl)-1-oxoisoindolin-2-yl)piperidine-2,6-dione |
| | 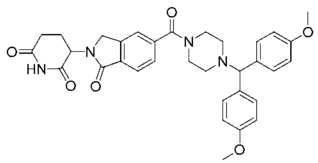 | 3-(5-(4-(bis(4-methoxyphenyl)methyl)piper azine-1-carbonyl)-1-oxoisoindolin-2-yl)piperidine-2,6-dione | 3-(5-(4-(bis(4-methoxyphenyl)methyl)pip erazine-1-carbonyl)-1-oxoisoindolin-2-yl)piperidine-2,6-dione |

(continued)

| Compou nd No. | Structure of the compounds | The compounds' name | Chinese translation of the compounds' name |
|---|---|---|---|
| | 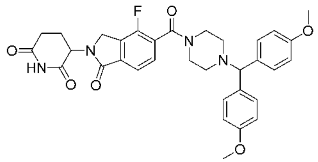 | 3-(5-(4-(bis(4-methoxyphenyl)methyl)piper azine-1-carbonyl)-4-fluoro-1-oxoisoindolin-2-yl)piperidine-2,6-dione | 3-(5-(4-(bis(4-methoxyphenyl)methyl)pip erazine-1-carbonyl)-4-fluoro-1-oxoisoindolin-2-yl)piperidine-2,6-dione |
| | 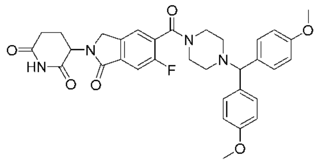 | 3-(5-(4-(bis(4-methoxyphenyl)methyl)piper azine-1-carbonyl)-6-fluoro-1-oxoisoindolin-2-yl)piperidine-2,6-dione | 3-(5-(4-(bis(4-methoxyphenyl)methyl)pip erazine-1-carbonyl)-6-fluoro-1-oxoisoindolin-2-yl)piperidine-2,6-dione |
| | 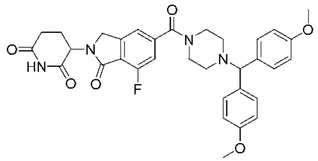 | 3-(5-(4-(bis(4-methoxyphenyl)methyl)piper azine-1-carbonyl)-7-fluoro-1-oxoisoindolin-2-yl)piperidine-2,6-dione | 3-(5-(4-(bis(4-methoxyphenyl)methyl)pip erazine-1-carbonyl)-7-fluoro-1-oxoisoindolin-2-yl)piperidine-2,6-dione |
| | 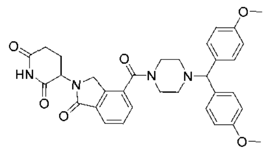 | 3-(4-(4-(bis(4-methoxyphenyl)methyl)piper azine-1-carbonyl)-1-oxoisoindolin-2-yl)piperidine-2,6-dione | 3-(4-(4-(bis(4-methoxyphenyl)methyl)pip erazine-1-carbonyl)-1-oxoisoindolin-2-yl)piperidine-2,6-dione |
| | 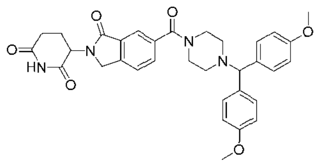 | 3-(6-(4-(bis(4-methoxyphenyl)methyl)piper azine-1-carbonyl)-1-oxoisoindolin-2-yl)piperidine-2,6-dione | 3-(6-(4-(bis(4-methoxyphenyl)methyl)pip erazine-1-carbonyl)-1-oxoisoindolin-2-yl)piperidine-2,6-dione |
| | 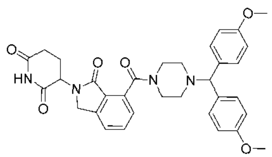 | 3-(7-(4-(bis(4-methoxyphenyl)methyl)piper azine-1-carbonyl)-1-oxoisoindolin-2-yl)piperidine-2,6-dione | 3-(7-(4-(bis(4-methoxyphenyl)methyl)pip erazine-1-carbonyl)-1-oxoisoindolin-2-yl)piperidine-2,6-dione |

(continued)

| Compou nd No. | Structure of the compounds | The compounds' name | Chinese translation of the compounds' name |
|---|---|---|---|
| GT-05926 | 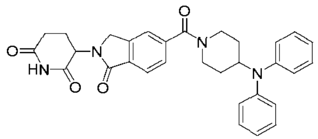 | 3-(5-(4-(diphenylamino)piperidine-1-carbonyl)-1-oxoisoindolin-2-yl)piperidine-2,6-dione | 3-(5-(4-(diphenylamino)piperidine-1-carbonyl)-1-oxoisoindolin-2-yl)piperidine-2,6-dione |
| | 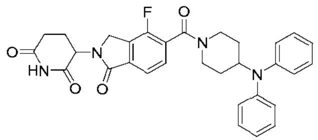 | 3-(5-(4-(diphenylamino)piperidine-1-carbonyl)-4-fluoro-1-oxoisoindolin-2-yl)piperidine-2,6-dione | 3-(5-(4-(diphenylamino)piperidine-1-carbonyl)-4-fluoro-1-oxoisoindolin-2-yl)piperidine-2,6-dione |
| | 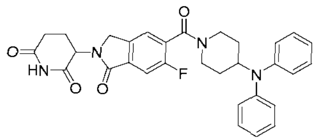 | 3-(5-(4-(diphenylamino)piperidine-1-carbonyl)-6-fluoro-1-oxoisoindolin-2-vl)piperidine-2,6-dione | 3-(5-(4-(diphenylamino)piperidine-1-carbonyl)-6-fluoro-1-oxoisoindolin-2-yl)piperidine-2,6-dione |
| GT-05927 | 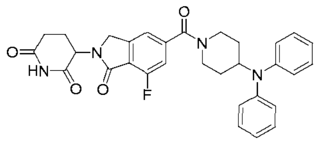 | 3-(5-(4-(diphenylamino)piperidine-1-carbonyl)-7-fluoro-1-oxoisoindolin-2-yl)piperidine-2,6-dione | 3-(5-(4-(diphenylamino)piperidine-1-carbonyl)-7-fluoro-1-oxoisoindolin-2-yl)piperidine-2,6-dione |
| GT-05923 | 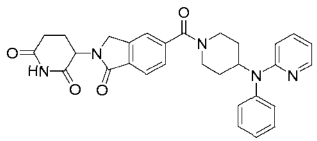 | 3-(1-oxo-5-(4-(phenyl(pyridin-2-yl)amino)piperidine-1-carbonyl)isoindolin-2-yl)piperidine-2,6-dione | 3-(1-oxo-5-(4-(phenyl(pyridin-2-yl)amino)piperidine-1-carbonyl)isoindolin-2-yl)piperidine-2,6-dione |
| GT-05924 | 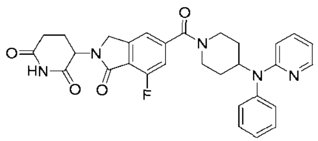 | 3-(7-fluoro-1-oxo-5-(4-(phenyl(pyridin-2-yl)amino)piperidine-1-carbonyl)isoindolin-2-yl)piperidine-2,6-dione | 3-(7-fluoro-1-oxo-5-(4-(phenyl(pyridin-2-yl)amino)piperidine-1-carbonyl)isoindolin-2-yl)piperidine-2,6-dione |
| GT-05690 | 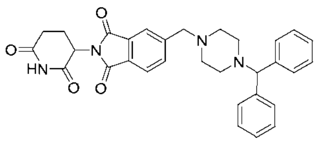 | 5-((4-benzhydrylpiperazin-1-yl)methyl)-2-(2,6-dioxopiperidin-3-yl)isoindoline-1,3-dione | 5-((4-benzhydrylpiperazin-1-yl)methyl)-2-(2,6-dioxopiperidin-3-yl)isoindoline-1,3-dione |

(continued)

| Compound No. | Structure of the compounds | The compounds' name | Chinese translation of the compounds' name |
|---|---|---|---|
| | 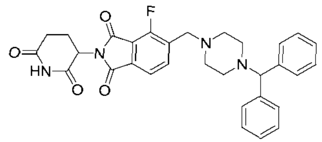 | 5-((4-benzhydrylpiperazin-1-yl)methyl)-2-(2,6-dioxopiperidin-3-yl)-4-fluoroisoindoline-1,3-dione | 5-((4-benzhydrylpiperazin-1-yl)methyl)-2-(2,6-dioxopiperidin-3-yl)-4-fluoroisoindoline-1,3-dione |
| | 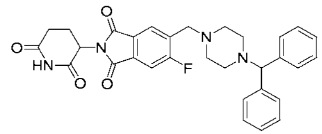 | 5-((4-benzhydrylpiperazin-1-yl)methyl)-2-(2,6-dioxopiperidin-3-yl)-6-fluoroisoindoline-1,3-dione | 5-((4-benzhydrylpiperazin-1-yl)methyl)-2-(2,6-dioxopiperidin-3-yl)-6-fluoroisoindoline-1,3-dione |
| | 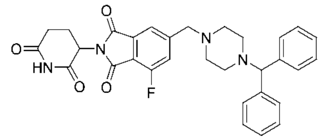 | 6-((4-benzhydrylpiperazin-1-yl)methyl)-2-(2,6-dioxopiperidin-3-yl)-4-fluoroisoindoline-1,3-dione | 6-((4-benzhydrylpiperazin-1-yl)methyl)-2-(2,6-dioxopiperidin-3-yl)-4-fluoroisoindoline-1,3-dione |
| GT-05722 | 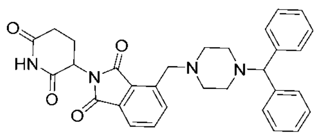 | 4-((4-benzhydrylpiperazin-1-yl)methyl)-2-(2,6-dioxopiperidin-3-yl)isoindoline-1,3-dione | 4-((4-benzhydrylpiperazin-1-yl)methyl)-2-(2,6-dioxopiperidin-3-yl)isoindoline-1,3-dione |
| | 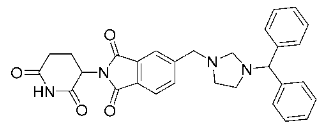 | 5-((3-benzhydrylimidazolidin-1-yl)methyl)-2-(2,6-dioxopiperidin-3-vl)isoindoline-1,3-dione | 5-((3-benzhydrylimidazolidin-1-yl)methyl)-2-(2,6-dioxopiperidin-3-vl)isoindoline-1,3-dione |
| GT-05739 | 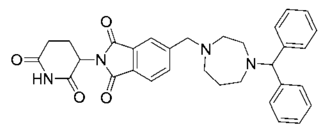 | 5-((4-benzhydryl-1,4-diazepan-1-yl)methyl)-2-(2,6-dioxopiperidin-3-vl)isoindoline-1,3-dione | 5-((4-benzhydryl-1,4-diazepan-1-yl)methyl)-2-(2,6-dioxopiperidin-3-vl)isoindoline-1,3-dione |
| GT-05906 | 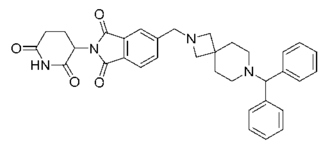 | 5-((7-benzhydryl-2,7-diazaspiro[3.5]nonan-2-yl) methyl)-2-(2,6-dioxopiperidin-3-vl)isoindoline-1,3-dione | 5-((7-benzhydryl-2,7-diazaspiro[3 .5]nonan-2-yl) methyl)-2-(2,6-dioxopiperidin-3-vl)isoindoline-1,3-dione |

(continued)

| Compou nd No. | Structure of the compounds | The compounds' name | Chinese translation of the compounds' name |
|---|---|---|---|
| GT-05974 | 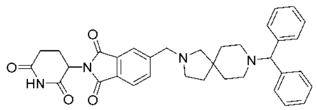 | 5-((8-benzhydryl-2,8-diazaspiro[4.5]decan-2-yl) methyl)-2-(2,6-dioxopiperidin-3-vl)isoindoline-1,3-dione | 5-((8-benzhydryl-2,8-diazaspiro[4.5]decan-2-yl) methyl)-2-(2,6-dioxopiperidin-3-vl)isoindoline-1,3-dione |
| GT-06028 | 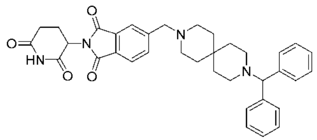 | 5-((9-benzhydryl-3,9-diazaspiro[5 .5]undecan-3-yl) methyl)-2-(2,6-dioxopiperidin-3-vl)isoindoline-1,3-dione | 5-((9-benzhydryl-3,9-diazaspiro[5.5]undecan-3-yl) methyl)-2-(2,6-dioxopiperidin-3-vl)isoindoline-1,3-dione |
| GT-06053 | 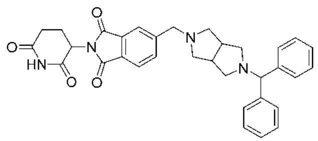 | 5-((5-benzhydrylhexahydropyrrolo [3,4-c]pyrrol-2(1H)-yl) methyl)-2-(2,6-dioxopiperidin-3-vl)isoindoline-1,3-dione | 5-((5-benzhydrylhexahydropyrrol o[3,4-c]pyrrol-2(1H)-yl) methyl)-2-(2,6-dioxopiperidin-3-vl)isoindoline-1,3-dione |
| GT-05728 | 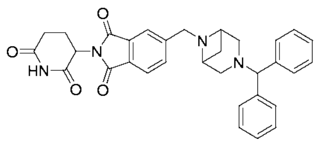 | 5-((3-benzhydryl-3,6-diazabicyclo[3.1.1]heptan-6-yl) methyl)-2-(2,6-dioxopiperidin-3-vl)isoindoline-1,3-dione | 5-((3-benzhydryl-3,6-diazabicyclo[3.1.1]heptan-6-yl) methyl)-2-(2,6-dioxopiperidin-3-vl)isoindoline-1,3-dione |
| GT-05959 | 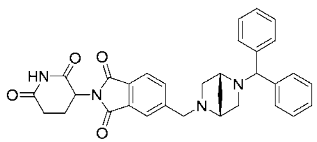 | 5-((1R,4R)-5-benzhydryl-2,5-diazabicyclo[2.2.1]heptan-2-yl)methyl)-2-(2,6-dioxopiperidin-3-vl)isoindoline-1,3-dione | 5-(((1R,4R)-5-benzhydryl-2,5-diazabicyclo[2.2.1]heptan-2-yl)methyl)-2-(2,6-dioxopiperidin-3-vl)isoindoline-1,3-dione |
| GT-05729 | 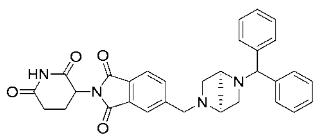 | 5-(((1S,4S)-5-benzhydryl-2,5-diazabicyclo[2.2.1]heptan-2-yl)methyl)-2-(2,6- | 5-(((1S,4S)-5-benzhydryl-2,5-diazabicyclo[2.2.1]heptan-2-yl)methyl)-2-(2,6- |
| | | dioxopiperidin-3-yl)isoindoline-1,3-dione | dioxopiperidin-3-yl)isoindoline-1,3-dione |

(continued)

| Compound No. | Structure of the compounds | The compounds' name | Chinese translation of the compounds' name |
| --- | --- | --- | --- |
| GT-05899 | 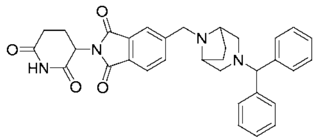 | 5-((3-benzhydryl-3,8-diazabicyclo[3.2.1]octan-8-yl) methyl)-2-(2,6-dioxopiperidin-3-vl)isoindoline-1,3-dione | 5-((3-benzhydryl-3,8-diazabicyclo[3.2.1]octan-8-yl) methyl)-2-(2,6-dioxopiperidin-3-vl)isoindoline-1,3-dione |
| GT-05898 | 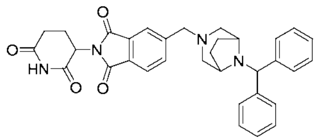 | 5-((8-benzhydryl-3,8-diazabicyclo[3.2.1]octan-3-yl) methyl)-2-(2,6-dioxopiperidin-3-vl)isoindoline-1,3-dione | 5-((8-benzhydryl-3,8-diazabicyclo[3.2.1]octan-3-yl) methyl)-2-(2,6-dioxopiperidin-3-vl)isoindoline-1,3-dione |
| GT-06047 | 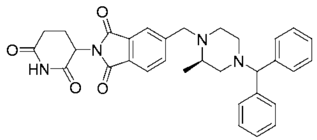 | 5-(((R)-4-benzhydryl-2-methylpiperazin-1-yl) methyl)-2-(2,6-dioxopiperidin-3-vl)isoindoline-1,3-dione | 5-(((R)-4-benzhydryl-2-methylpiperazin-1-yl) methyl)-2-(2,6-dioxopiperidin-3-vl)isoindoline-1,3-dione |
| GT-05740 | 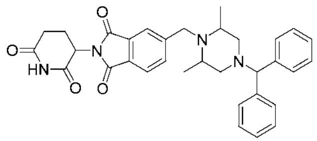 | 5-((4-benzhydryl-2,6-dimethylpiperazin-1-yl) methyl)-2-(2,6-dioxopiperidin-3-vl)isoindoline-1,3-dione | 5-((4-benzhydryl-2,6-dimethylpiperazin-1-yl) methyl)-2-(2,6-dioxopiperidin-3-vl)isoindoline-1,3-dione |
| GT-05968 | 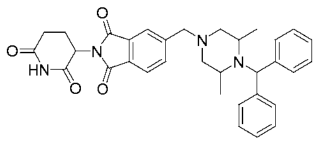 | 5-((4-benzhydryl-3,5-dimethylpiperazin-1-yl) methyl)-2-(2,6-dioxopiperidin-3-vl)isoindoline-1,3-dione | 5-((4-benzhydryl-3,5-dimethylpiperazin-1-yl) methyl)-2-(2,6-dioxopiperidin-3-vl)isoindoline-1,3-dione |
| GT-05730 | 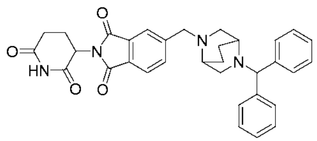 | 5-((5-benzhydryl-2,5-diazabicyclo[2.2.2]octan-2-yl) methyl)-2-(2,6-dioxopiperidin-3-vl)isoindoline-1,3-dione | 5-((5-benzhydryl-2,5-diazabicyclo[2.2.2]octan-2-yl) methyl)-2-(2,6-dioxopiperidin-3-vl)isoindoline-1,3-dione |
| | 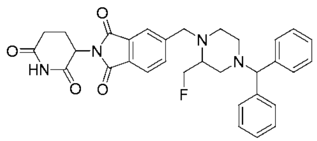 | 5-((4-benzhydryl-2-(fluoromethyl)piperazin-1-yl) methyl)-2-(2,6-dioxopiperidin-3-yl)isoindoline-1,3-dione | 5-((4-benzhydryl-2-(fluoromethyl)piperazin-1-yl) methyl)-2-(2,6-dioxopiperidin-3-yl)isoindoline-1,3-dione |

(continued)

| Compound No. | Structure of the compounds | The compounds' name | Chinese translation of the compounds' name |
|---|---|---|---|
| GT-06099 | 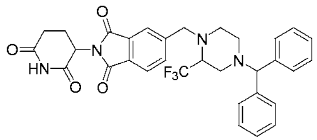 | 5-((4-benzhydryl-2-(trifluoromethyl)piperazin-1-yl) methyl)-2-(2,6-dioxopiperidin-3-vl)isoindoline-1,3-dione | 5-((4-benzhydryl-2-(trifluoromethyl)piperazin-1-yl) methyl)-2-(2,6-dioxopiperidin-3-vl)isoindoline-1,3-dione |
| GT-05962 | 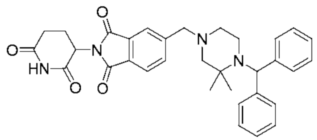 | 5-((4-benzhydryl-3,3-dimethylpiperazin-1-yl) methyl)-2-(2,6-dioxopiperidin-3-vl)isoindoline-1,3-dione | 5-((4-benzhydryl-3,3-dimethylpiperazin-1-yl) methyl)-2-(2,6-dioxopiperidin-3-vl)isoindoline-1,3-dione |
| | 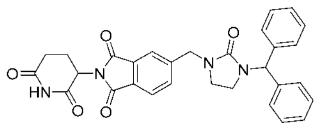 | 5-((3-benzhydryl-2-oxoimidazolidin-1-yl)methyl)-2-(2,6-dioxopiperidin-3-yl)isoindoline-1,3-dione | 5-((3-benzhydryl-2-oxoimidazolidin-1-yl)methyl)-2-(2,6-dioxopiperidin-3-yl)isoindoline-1,3-dione |
| GT-05723 | 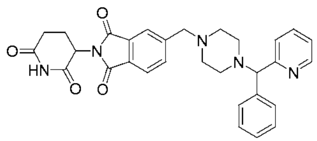 | 2-(2,6-dioxopiperidin-3-yl)-5-((4-(phenyl(pyridin-2-yl) methyl)piperazin-1-yl)methyl)isoindoline-1,3-dione | 2-(2,6-dioxopiperidin-3-yl)-5-((4-(phenyl(pyridin-2-yl) methyl)piperazin-1-yl)methyl)isoindoline-1,3-dione |
| GT-06114 | 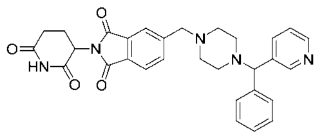 | 2-(2,6-dioxopiperidin-3-yl)-5-((4-(phenyl(pyridin-3-yl) methyl)piperazin-1-yl)methyl)isoindoline-1,3-dione | 2-(2,6-dioxopiperidin-3-yl)-5-((4-(phenyl(pyridin-3-yl) methyl)piperazin-1-yl)methyl)isoindoline-1,3-dione |
| GT-06115 | 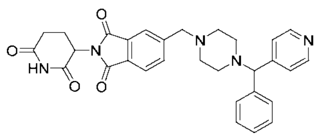 | 2-(2,6-dioxopiperidin-3-yl)-5-((4-(phenyl(pyridin-4-yl) methyl)piperazin-1-yl)methyl)isoindoline-1,3-dione | 2-(2,6-dioxopiperidin-3-yl)-5-((4-(phenyl(pyridin-4-yl) methyl)piperazin-1-yl)methyl)isoindoline-1,3-dione |
| GT-05724 | 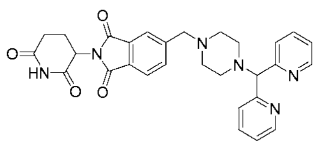 | 5-((4-(di(pyridin-2-yl)methyl)piperazin-1-yl)methyl)-2-(2,6-dioxopiperidin-3-vl)isoindoline-1,3-dione | 5-((4-(di(pyridin-2-yl)methyl)piperazin-1-yl)methyl)-2-(2,6-dioxopiperidin-3-yl)isoindoline-1,3-dione |

(continued)

| Compou nd No. | Structure of the compounds | The compounds' name | Chinese translation of the compounds' name |
|---|---|---|---|
| GT-05725 | 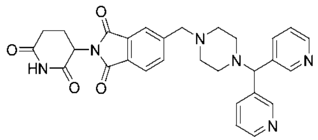 | 5-((4-(di(pyridin-3-yl)methyl)piperazin-1-yl)methyl)-2-(2,6-dioxopiperidin-3-yl)isoindoline-1,3-dione | 5-((4-(di(pyridin-3-yl)methyl)piperazin-1-yl)methyl)-2-(2,6-dioxopiperidin-3-yl)isoindoline-1,3-dione |
| GT-06639 | 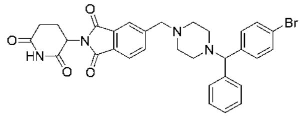 | 5-((4-((4-bromophenyl)(phenyl)methyl )piperazin-1-yl) methyl)-2-(2,6-dioxopiperidin-3-vl)isoindoline-1,3-dione | 5-((4-((4-bromophenyl)(phenyl)meth yl)piperazin-1-yl) methyl)-2-(2,6-dioxopiperidin-3-yl)isoindoline-1,3-dione |
| GT-05726 | 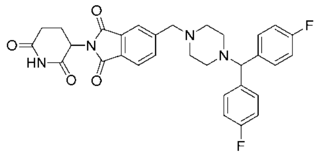 | 5-((4-(bis(4-fluorophenyl)methyl)piperazi n-1-yl) methyl)-2-(2,6-dioxopiperidin-3-vl)isoindoline-1,3-dione | 5-((4-(bis(4-fluorophenyl)methyl)pipera zin-1-yl) methyl)-2-(2,6-dioxopiperidin-3-vl)isoindoline-1,3-dione |
| GT-06118 | 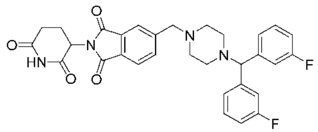 | 5-((4-(bis(3-fluorophenyl)methyl)piperazi n-1-yl) methyl)-2-(2,6-dioxopiperidin-3-vl)isoindoline-1,3-dione | 5-((4-(bis(3-fluorophenyl)methyl)pipera zin-1-yl) methyl)-2-(2,6-dioxopiperidin-3-vl)isoindoline-1,3-dione |
| GT-06088 | 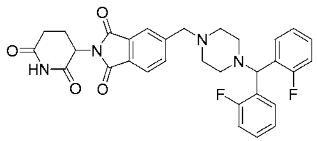 | 5-((4-(bis(2-fluorophenyl)methyl)piperazi n-1-yl) methyl)-2-(2,6-dioxopiperidin-3-yl)isoindoline-1,3-dione | 5-((4-(bis(2-fluorophenyl)methyl)pipera zin-1-yl) methyl)-2-(2,6-dioxopiperidin-3-yl)isoindoline-1,3-dione |
| GT-06203 | 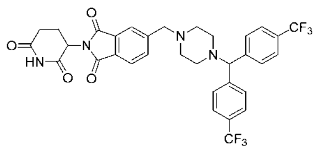 | 5-((4-(bis(4-(trifluoromethyl)phenyl)meth yl)piperazin-1-yl) methyl)-2-(2,6-dioxopiperidin-3-yl)isoindoline-1,3-dione | 5-((4-(bis(4-(trifluoromethyl)phenyl)me thyl)piperazin-1-yl) methyl)-2-(2,6-dioxopiperidin-3-vl)isoindoline-1,3-dione |

(continued)

| Compound No. | Structure of the compounds | The compounds' name | Chinese translation of the compounds' name |
|---|---|---|---|
| | 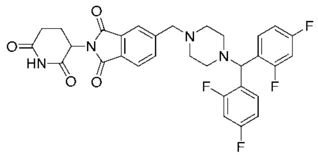 | 5-((4-(bis(2,4-difluorophenyl)methyl)pipera zin-1-yl) methyl)-2-(2,6-dioxopiperidin-3-vl)isoindoline-1,3-dione | 5-((4-(bis(2,4-difluorophenyl)methyl)pipe razin-1-yl) methyl)-2-(2,6-dioxopiperidin-3-vl)isoindoline-1,3-dione |
| GT-06596 | 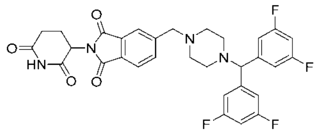 | 5-((4-(bis(3,5-difluorophenyl)methyl)pipera zin-1-yl) methyl)-2-(2,6-dioxopiperidin-3-vl)isoindoline-1,3-dione | 5-((4-(bis(3,5-difluorophenyl)methyl)pipe razin-1-yl) methyl)-2-(2,6-dioxopiperidin-3-vl)isoindoline-1,3-dione |
| | 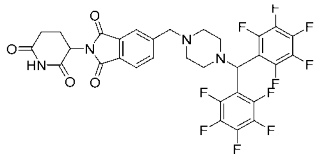 | 5-((4-(bis(perfluorophenyl)methyl) piperazin-1-yl) methyl)-2-(2,6-dioxopiperidin-3-yl)isoindoline-1,3-dione | 5-((4-(bis(perfluorophenyl)methy 1)piperazin-1-yl) methyl)-2-(2,6-dioxopiperidin-3-yl)isoindoline-1,3-dione |
| GT-05727 | 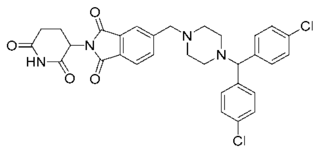 | 5-((4-(bis(4-chlorophenyl)methyl)piperazi n-1-yl) methyl)-2-(2,6-dioxopiperidin-3-vl)isoindoline-1,3-dione | 5-((4-(bis(4-chlorophenyl)methyl)piper azin-1-yl) methyl)-2-(2,6-dioxopiperidin-3-vl)isoindoline-1,3-dione |
| | 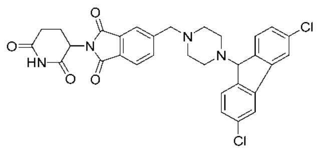 | 5-((4-(3,6-dichloro-9H-fluoren-9-yl)piperazin-1-yl) methyl)-2-(2,6-dioxopiperidin-3-vl)isoindoline-1,3-dione | 5-((4-(3,6-dichloro-9H-fluoren-9-yl)piperazin-1-yl) methyl)-2-(2,6-dioxopiperidin-3-vl)isoindoline-1,3-dione |
| GT-06049 | 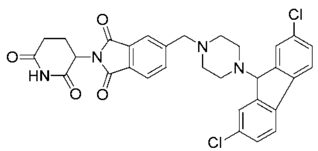 | 5-((4-(2,7-dichloro-9H-fluoren-9-yl)piperazin-1-yl) methyl)-2-(2,6-dioxopiperidin-3-vl)isoindoline-1,3-dione | 5-((4-(2,7-dichloro-9H-fluoren-9-yl)piperazin-1-yl) methyl)-2-(2,6-dioxopiperidin-3-vl)isoindoline-1,3-dione |

(continued)

| Compound No. | Structure of the compounds | The compounds' name | Chinese translation of the compounds' name |
|---|---|---|---|
| GT-05908 | 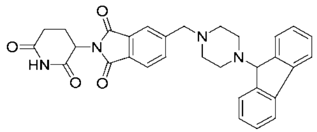 | 5-((4-(9H-fluoren-9-yl)piperazin-1-yl)methyl)-2-(2,6-dioxopiperidin-3-yl)isoindoline-1,3-dione | 5-((4-(9H-fluoren-9-yl)piperazin-1-yl)methyl)-2-(2,6-dioxopiperidin-3-yl)isoindoline-1,3-dione |
| | 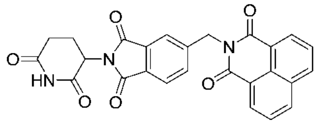 | 2-((2-(2,6-dioxopiperidin-3-yl)-1,3-dioxoisoindolin-5-yl) methyl)-1H-benzo[de]isoquinoline-1,3(2H)-dione | 2-((2-(2,6-dioxopiperidin-3-yl)-1,3-dioxoisoindolin-5-yl) methyl)-1H-benzo[de]isoquinoline-1,3(2H)-dione |
| | 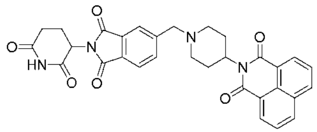 | 2-(1-((2-(2,6-dioxopiperidin-3-yl)-1,3-dioxoisoindolin-5-yl) methyl)piperidin-4-yl)-1H-benzo[de]isoquinoline-1,3(2H)-dione | 2-(1-((2-(2,6-dioxopiperidin-3-yl)-1,3-dioxoisoindolin-5-yl) methyl)piperidin-4-yl)-1H-benzo[de]isoquinoline-1,3(2H)-dione |
| | 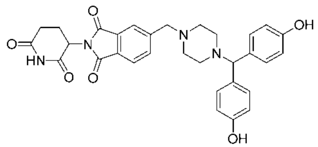 | 5-((4-(bis(4-hydroxyphenyl)methyl)piper azin-1-yl) methyl)-2-(2,6-dioxopiperidin-3-yl)isoindoline-1,3-dione | 5-((4-(bis(4-hydroxyphenyl)methyl)pip erazin-1-yl) methyl)-2-(2,6-dioxopiperidin-3-yl)isoindoline-1,3-dione |
| GT-06425 | 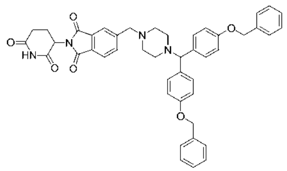 | 5-((4-(bis(4-(benzyloxy)phenyl)methyl)pi perazin-1-yl) methyl)-2-(2,6-dioxopiperidin-3-yl)isoindoline-1,3-dione | 5-((4-(bis(4-(benzyloxy)phenyl)methyl) piperazin-1-yl) methyl)-2-(2,6-dioxopiperidin-3-yl)isoindoline-1,3-dione |
| | 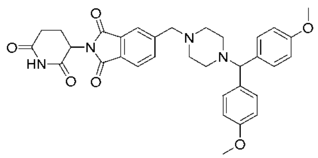 | 5-((4-(bis(4-methoxyphenyl)methyl)piper azin-1-yl) methyl)-2-(2,6-dioxopiperidin-3-yl)isoindoline-1,3-dione | 5-((4-(bis(4-methoxyphenyl)methyl)pip erazin-1-yl) methyl)-2-(2,6-dioxopiperidin-3-yl)isoindoline-1,3-dione |

(continued)

| Compound No. | Structure of the compounds | The compounds' name | Chinese translation of the compounds' name |
|---|---|---|---|
| GT-05741 | 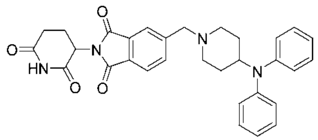 | 2-(2,6-dioxopiperidin-3-yl)-5-((4-(diphenylamino)piperidin-1-yl)methyl)isoindoline-1,3-dione | 2-(2,6-dioxopiperidin-3-yl)-5-((4-(diphenylamino)piperidin-1-yl)methyl)isoindoline-1,3-dione |
| GT-05742 | 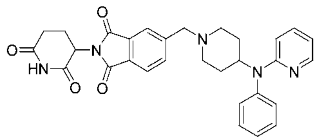 | 2-(2,6-dioxopiperidin-3-yl)-5-((4-(phenyl(pyridin-2-yl)amino)piperidin-1-yl)methyl)isoindoline-1,3-dione | 2-(2,6-dioxopiperidin-3-yl)-5-((4-(phenyl(pyridin-2-yl)amino)piperidin-1-yl)methyl)isoindoline-1,3-dione |
| GT-05691 | 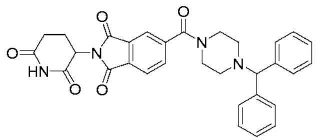 | 5-(4-benzhydrylpiperazine-1-carbonyl)-2-(2,6-dioxopiperidin-3-yl)isoindoline-1,3-dione | 5-(4-benzhydrylpiperazine-1-carbonyl)-2-(2,6-dioxopiperidin-3-yl)isoindoline-1,3-dione |
| | 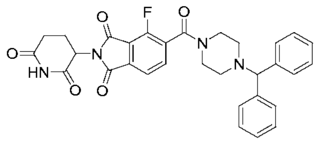 | 5-(4-benzhydrylpiperazine-1-carbonyl)-2-(2,6-dioxopiperidin-3-yl)-4-fluoroisoindoline-1,3-dione | 5-(4-benzhydrylpiperazine-1-carbonyl)-2-(2,6-dioxopiperidin-3-yl)-4-fluoroisoindoline-1,3-dione |
| | 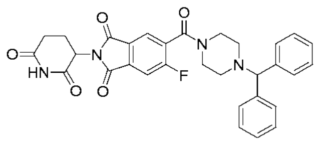 | 5-(4-benzhydrylpiperazine-1-carbonyl)-2-(2,6-dioxopiperidin-3-yl)-6-fluoroisoindoline-1,3-dione | 5-(4-benzhydrylpiperazine-1-carbonyl)-2-(2,6-dioxopiperidin-3-yl)-6-fluoroisoindoline-1,3-dione |
| | 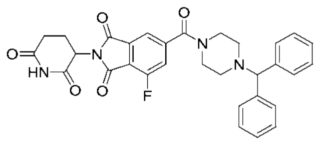 | 6-(4-benzhydrylpiperazine-1-carbonyl)-2-(2,6-dioxopiperidin-3-yl)-4-fluoroisoindoline-1,3-dione | 6-(4-benzhydrylpiperazine-1-carbonyl)-2-(2,6-dioxopiperidin-3-yl)-4-fluoroisoindoline-1,3-dione |
| GT-05853 | 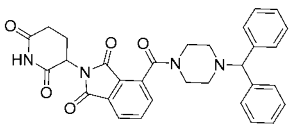 | 4-(4-benzhydrylpiperazine-1-carbonyl)-2-(2,6-dioxopiperidin-3-yl)isoindoline-1,3-dione | 4-(4-benzhydrylpiperazine-1-carbonyl)-2-(2,6-dioxopiperidin-3-yl)isoindoline-1,3-dione |

(continued)

| Compound No. | Structure of the compounds | The compounds' name | Chinese translation of the compounds' name |
|---|---|---|---|
| | 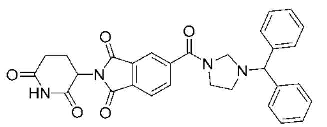 | 5-(3-benzhydrylimidazolidine-1-carbonyl)-2-(2,6-dioxopiperidin-3-vl)isoindoline-1,3-dione | 5-(3-benzhydrylimidazolidine-1-carbonyl)-2-(2,6-dioxopiperidin-3-vl)isoindoline-1,3-dione |
| GT-05919 | 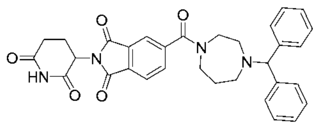 | 5-(4-benzhydryl-1,4-diazepane-1-carbonyl)-2-(2,6-dioxopiperidin-3-vl)isoindoline-1,3-dione | 5-(4-benzhydryl-1,4-diazepane-1-carbonyl)-2-(2,6-dioxopiperidin-3-yl)isoindoline-1,3-dione |
| GT-05989 | 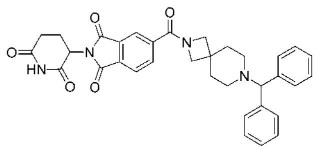 | 5-(7-benzhydryl-2,7-diazaspiro[3.5]nonane-2-carbonyl)-2-(2,6-dioxopiperidin-3-yl)isoindoline-1,3-dione | 5-(7-benzhydryl-2,7-diazaspiro[3.5]nonane-2-carbonyl)-2-(2,6-dioxopiperidin-3-yl)isoindoline-1,3-dione |
| GT-06064 | 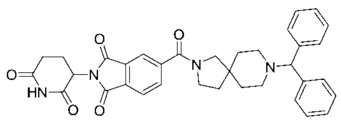 | 5-(8-benzhydryl-2,8-diazaspiro[4.5]decane-2-carbonyl)-2-(2,6-dioxopiperidin-3-yl)isoindoline-1,3-dione | 5-(8-benzhydryl-2,8-diazaspiro[4.5]decane-2-carbonyl)-2-(2,6-dioxopiperidin-3-yl)isoindoline-1,3-dione |
| GT-06066 | 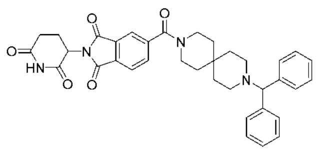 | 5-(9-benzhydryl-3,9-diazaspiro[5.5]undecane-3-carbonyl)-2-(2,6-dioxopiperidin-3-vl)isoindoline-1,3-dione | 5-(9-benzhydryl-3,9-diazaspiro[5.5]undecane-3-carbonyl)-2-(2,6-dioxopiperidin-3-yl)isoindoline-1,3-dione |
| GT-06068 | 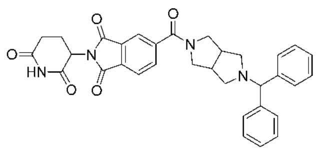 | 5-(5-benzhydryloctahydropyrrolo[ 3,4-c]pyrrole-2-carbonyl)-2-(2,6-dioxopiperidin-3-yl)isoindoline-1,3-dione | 5-(5-benzhydryloctahydropyrrol o[3,4-c]pyrrole-2-carbonyl)-2-(2,6-dioxopiperidin-3-vl)isoindoline-1,3-dione |
| GT-05918 | 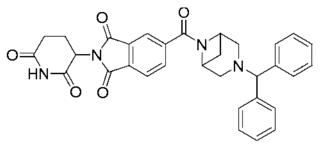 | 5-(3-benzhydryl-3,6-diazabicyclo[3.1.1]heptane-6-carbonyl)-2-(2,6-dioxopiperidin-3-yl)isoindoline-1,3-dione | 5-(3-benzhydryl-3,6-diazabicyclo[3.1.1]heptane-6-carbonyl)-2-(2,6-dioxopiperidin-3-yl)isoindoline-1,3-dione |

(continued)

| Compound No. | Structure of the compounds | The compounds' name | Chinese translation of the compounds' name |
|---|---|---|---|
| GT-05992 | 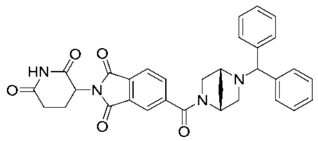 | 5-((1R,4R)-5-benzhydryl-2,5-diazabicyclo[2.2.1]heptane-2-carbonyl)-2-(2,6-dioxopiperidin-3-yl)isoindoline-1,3-dione | 5-((1R,4R)-5-benzhydryl-2,5-diazabicyclo[2.2.1]heptane-2-carbonyl)-2-(2,6-dioxopiperidin-3-yl)isoindoline-1,3-dione |
| GT-05979 | 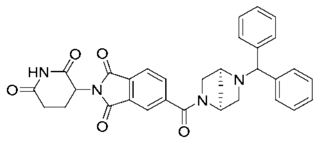 | 5-((1S,4S)-5-benzhydryl-2,5-diazabicyclo[2.2.1]heptane-2-carbonyl)-2-(2,6-dioxopiperidin-3-yl)isoindoline-1,3-dione | 5-((1S,4S)-5-benzhydryl-2,5-diazabicyclo[2.2.1]heptane-2-carbonyl)-2-(2,6-dioxopiperidin-3-yl)isoindoline-1,3-dione |
| GT-05984 | 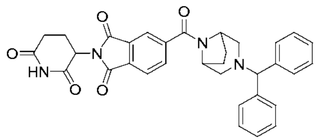 | 5-(3-benzhydryl-3,8-diazabicyclo[3.2.1]octane-8-carbonyl)-2-(2,6-dioxopiperidin-3-vl)isoindoline-1,3-dione | 5-(3-benzhydryl-3,8-diazabicyclo[3.2.1]octane-8-carbonyl)-2-(2,6-dioxopiperidin-3-yl)isoindoline-1,3-dione |
| GT-05982 | 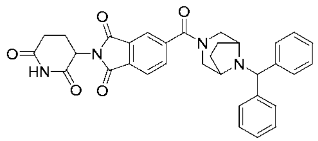 | 5-(8-benzhydryl-3,8-diazabicyclo[3.2.1]octane-3-carbonyl)-2-(2,6-dioxopiperidin-3-vl)isoindoline-1,3-dione | 5-(8-benzhydryl-3,8-diazabicyclo[3.2.1]octane-3-carbonyl)-2-(2,6-dioxopiperidin-3-yl)isoindoline-1,3-dione |
| GT-06094 | 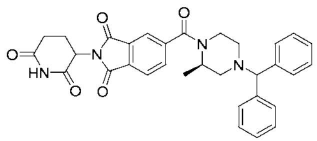 | 5-((R)-4-benzhydryl-2-methylpiperazine-1-carbonyl)-2-(2,6-dioxopiperidin-3-yl)isoindoline-1,3-dione | 5-((R)-4-benzhydryl-2-methylpiperazine-1-carbonyl)-2-(2,6-dioxopiperidin-3-yl)isoindoline-1,3-dione |
| GT-05922 | 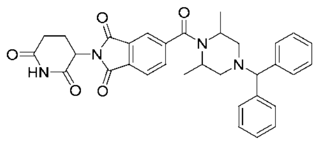 | 5-(4-benzhydryl-2,6-dimethylpiperazine-1-carbonyl)-2-(2,6-dioxopiperidin-3-vl)isoindoline-1,3-dione | 5-(4-benzhydryl-2,6-dimethylpiperazine-1-carbonyl)-2-(2,6-dioxopiperidin-3-yl)isoindoline-1,3-dione |
| GT-06386 | 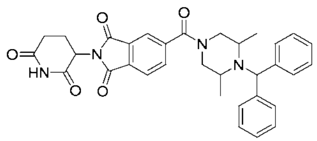 | 5-(4-benzhydryl-3,5-dimethylpiperazine-1-carbonyl)-2-(2,6-dioxopiperidin-3-vl)isoindoline-1,3-dione | 5-(4-benzhydryl-3,5-dimethylpiperazine-1-carbonyl)-2-(2,6-dioxopiperidin-3-yl)isoindoline-1,3-dione |

(continued)

| Compou nd No. | Structure of the compounds | The compounds' name | Chinese translation of the compounds' name |
|---|---|---|---|
| GT-06058 | 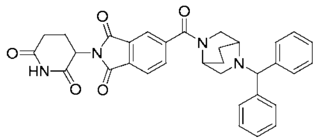 | 5-(5-benzhydryl-2,5-diazabicyclo[2.2.2]octane-2-carbonyl)-2-(2,6-dioxopiperidin-3-vl)isoindoline-1,3-dione | 5-(5-benzhydryl-2,5-diazabicyclo[2.2.2]octane-2-carbonyl)-2-(2,6-dioxopiperidin-3-yl)isoindoline-1,3-dione |
| | 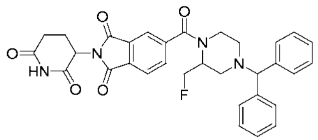 | 5-(4-benzhydryl-2-(fluoromethyl)piperazine-1-carbonyl)-2-(2,6-dioxopiperidin-3-yl)isoindoline-1,3-dione | 5-(4-benzhydryl-2-(fluoromethyl)piperazine-1-carbonyl)-2-(2,6-dioxopiperidin-3-yl)isoindoline-1,3-dione |
| GT-06091 | 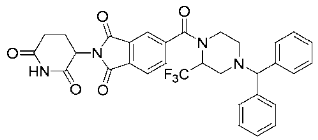 | 5-(4-benzhydryl-2-(trifluoromethyl)piperazine-1-carbonyl)-2-(2,6-dioxopiperidin-3-vl)isoindoline-1,3-dione | 5-(4-benzhydryl-2-(trifluoromethyl)piperazine -1-carbonyl)-2-(2,6-dioxopiperidin-3-yl)isoindoline-1,3-dione |
| GT-06278 | 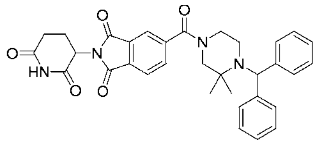 | 5-(4-benzhydryl-3,3-dimethylpiperazine-1-carbonyl)-2-(2,6-dioxopiperidin-3-yl)isoindoline-1,3-dione | 5-(4-benzhydryl-3,3-dimethylpiperazine-1-carbonyl)-2-(2,6-dioxopiperidin-3-yl)isoindoline-1,3-dione |
| GT-05878 | 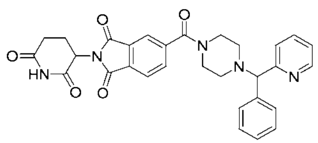 | 2-(2,6-dioxopiperidin-3-yl)-5-(4-(phenyl(pyridin-2-yl) methyl)piperazine-1-carbonyl)isoindoline-1,3-dione | 2-(2,6-dioxopiperidin-3-yl)-5-(4-(phenyl(pyridin-2-yl) methyl)piperazine-1-carbonyl)isoindoline-1,3-dione |
| GT-06143 | 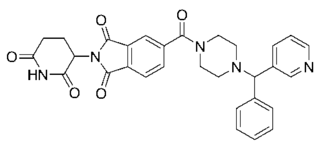 | 2-(2,6-dioxopiperidin-3-yl)-5-(4-(phenyl(pyridin-3-yl) methyl)piperazine-1-carbonyl)isoindoline-1,3-dione | 2-(2,6-dioxopiperidin-3-yl)-5-(4-(phenyl(pyridin-3-yl) methyl)piperazine-1-carbonyl)isoindoline-1,3-dione |
| GT-06145 | 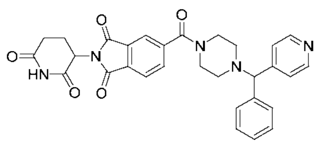 | 2-(2,6-dioxopiperidin-3-yl)-5-(4-(phenyl(pyridin-4-yl) methyl)piperazine-1-carbonyl)isoindoline-1,3-dione | 2-(2,6-dioxopiperidin-3-yl)-5-(4-(phenyl(pyridin-4-yl) methyl)piperazine-1-carbonyl)isoindoline-1,3-dione |

(continued)

| Compound No. | Structure of the compounds | The compounds' name | Chinese translation of the compounds' name |
|---|---|---|---|
| GT-05880 | 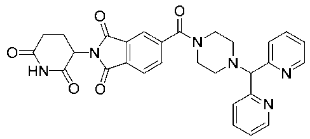 | 5-(4-(di(pyridin-2-yl)methyl)piperazine-1-carbonyl)-2-(2,6-dioxopiperidin-3-vl)isoindoline-1,3-dione | 5-(4-(di(pyridin-2-yl)methyl)piperazine-1-carbonyl)-2-(2,6-dioxopiperidin-3-yl)isoindoline-1,3-dione |
| GT-05855 | 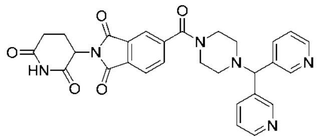 | 5-(4-(di(pyridin-3-yl)methyl)piperazine-1-carbonyl)-2-(2,6-dioxopiperidin-3-vl)isoindoline-1,3-dione | 5-(4-(di(pyridin-3-yl)methyl)piperazine-1-carbonyl)-2-(2,6-dioxopiperidin-3-vl)isoindoline-1,3-dione |
| GT-05885 | 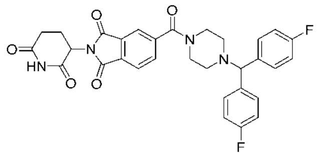 | 5-(4-(bis(4-fluorophenyl)methyl)piperazi ne-1-carbonyl)-2-(2,6-dioxopiperidin-3-yl)isoindoline-1,3-dione | 5-(4-(bis(4-fluorophenyl)methyl)pipera zine-1-carbonyl)-2-(2,6-dioxopiperidin-3-yl)isoindoline-1,3-dione |
| GT-06206 | 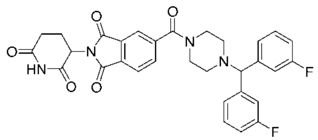 | 5-(4-(bis(3-fluorophenyl)methyl)piperazi ne-1-carbonyl)-2-(2,6-dioxopiperidin-3-yl)isoindoline-1,3-dione | 5-(4-(bis(3-fluorophenyl)methyl)pipera zine-1-carbonyl)-2-(2,6-dioxopiperidin-3-yl)isoindoline-1,3-dione |
| GT-06141 | 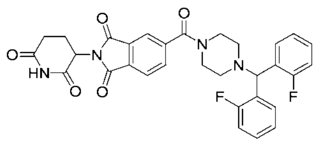 | 5-(4-(bis(2-fluorophenyl)methyl)piperazi ne-1-carbonyl)-2-(2,6-dioxopiperidin-3-yl)isoindoline-1,3-dione | 5-(4-(bis(2-fluorophenyl)methyl)pipera zine-1-carbonyl)-2-(2,6-dioxopiperidin-3-yl)isoindoline-1,3-dione |
| GT-06217 | 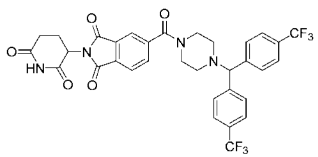 | 5-(4-(bis(4-(trifluoromethyl)phenyl)meth yl)piperazine-1-carbonyl)-2-(2,6-dioxopiperidin-3-yl)isoindoline-1,3-dione | 5-(4-(bis(4-(trifluoromethyl)phenyl)me thyl)piperazine-1-carbonyl)-2-(2,6-dioxopiperidin-3-yl)isoindoline-1,3-dione |

(continued)

| Compou nd No. | Structure of the compounds | The compounds' name | Chinese translation of the compounds' name |
|---|---|---|---|
| | 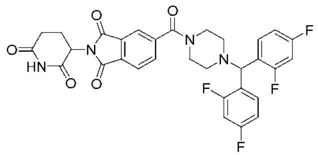 | 5-(4-(bis(2,4-difluorophenyl)methyl)pipera zine-1-carbonyl)-2-(2,6-dioxopiperidin-3-yl)isoindoline-1,3-dione | 5-(4-(bis(2,4-difluorophenyl)methyl)pipe razine-1-carbonyl)-2-(2,6-dioxopiperidin-3-yl)isoindoline-1,3-dione |
| GT-06598 | 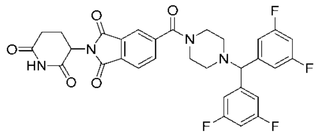 | 5-(4-(bis(3,5-difluorophenyl)methyl)pipera zine-1-carbonyl)-2-(2,6-dioxopiperidin-3-yl)isoindoline-1,3-dione | 5-(4-(bis(3,5-difluorophenyl)methyl)pipe razine-1-carbonyl)-2-(2,6-dioxopiperidin-3-yl)isoindoline-1,3-dione |
| | 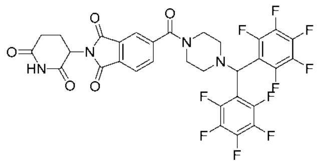 | 5-(4-(bis(perfluorophenyl)methyl) piperazine-1-carbonyl)-2-(2,6-dioxopiperidin-3-yl)isoindoline-1,3-dione | 5-(4-(bis(perfluorophenyl)methy 1)piperazine-1-carbonyl)-2-(2,6-dioxopiperidin-3-yl)isoindoline-1,3-dione |
| GT-05890 | 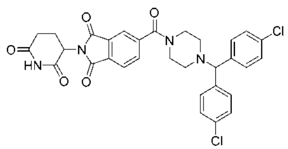 | 5-(4-(bis(4-chlorophenyl)methyl)piperazi ne-1-carbonyl)-2-(2,6-dioxopiperidin-3-vl)isoindoline-1,3-dione | 5-(4-(bis(4-chlorophenyl)methyl)piper azine-1-carbonyl)-2-(2,6-dioxopiperidin-3-vl)isoindoline-1,3-dione |
| | 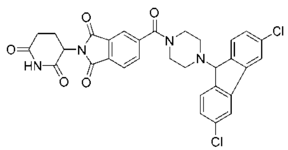 | 5-(4-(3,6-dichloro-9H-fluoren-9-yl)piperazine-1-carbonyl)-2-(2,6-dioxopiperidin-3-vl)isoindoline-1,3-dione | 5-(4-(3,6-dichloro-9H-fluoren-9-yl)piperazine-1-carbonyl)-2-(2,6-dioxopiperidin-3-vl)isoindoline-1,3-dione |
| GT-06070 | 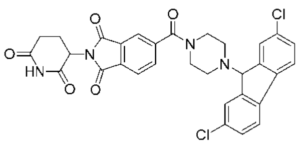 | 5-(4-(2,7-dichloro-9H-fluoren-9-yl)piperazine-1-carbonyl)-2-(2,6-dioxopiperidin-3-vl)isoindoline-1,3-dione | 5-(4-(2,7-dichloro-9H-fluoren-9-yl)piperazine-1-carbonyl)-2-(2,6-dioxopiperidin-3-vl)isoindoline-1,3-dione |

(continued)

| Compound No. | Structure of the compounds | The compounds' name | Chinese translation of the compounds' name |
|---|---|---|---|
| GT-05987 | 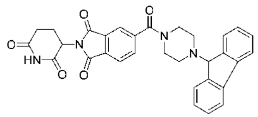 | 5-(4-(9H-fluoren-9-yl)piperazine-1-carbonyl)-2-(2,6-dioxopiperidin-3-yl)isoindoline-1,3-dione | 5-(4-(9H-fluoren-9-yl)piperazine-1-carbonyl)-2-(2,6-dioxopiperidin-3-yl)isoindoline-1,3-dione |
| | 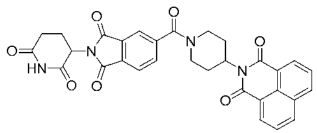 | 2-(1-(2-(2,6-dioxopiperidin-3-yl)-1,3-dioxoisoindoline-5-carbonyl)piperidin-4-yl)-1H-benzo[de]isoquinoline-1,3(2H)-dione | 2-(1-(2-(2,6-dioxopiperidin-3-yl)-1,3-dioxoisoindoline-5-carbonyl)piperidin-4-yl)-1H-benzo[de]isoquinoline-1,3(2H)-dione |
| | 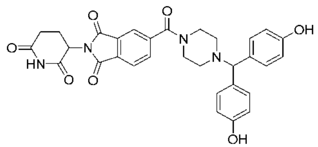 | 5-(4-(bis(4-hydroxyphenyl)methyl)piper azine-1-carbonyl)-2-(2,6-dioxopiperidin-3-vl)isoindoline-1,3-dione | 5-(4-(bis(4-hydroxyphenyl)methyl)pip erazine-1-carbonyl)-2-(2,6-dioxopiperidin-3-vl)isoindoline-1,3-dione |
| GT-06558 | 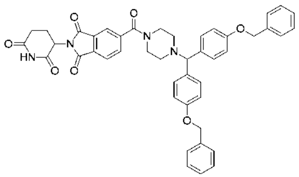 | 5-(4-(bis(4-(benzyloxy)phenyl)methyl)pi perazine-1-carbonyl)-2-(2,6-dioxopiperidin-3-yl)isoindoline-1,3-dione | 5-(4-(bis(4-(benzyloxy)phenyl)methyl) piperazine-1-carbonyl)-2-(2,6-dioxopiperidin-3-yl)isoindoline-1,3-dione |
| | 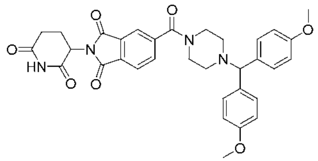 | 5-(4-(bis(4-methoxyphenyl)methyl)piper azine-1-carbonyl)-2-(2,6-dioxopiperidin-3-yl)isoindoline-1,3-dione | 5-(4-(bis(4-methoxyphenyl)methyl)pip erazine-1-carbonyl)-2-(2,6-dioxopiperidin-3-yl)isoindoline-1,3-dione |
| GT-05928 | 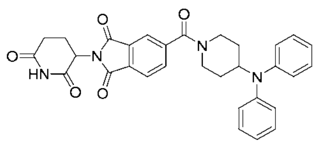 | 2-(2,6-dioxopiperidin-3-yl)-5-(4-(diphenylamino)piperidine-1-carbonyl)isoindoline-1,3-dione | 2-(2,6-dioxopiperidin-3-yl)-5-(4-(diphenylamino)piperidine-1-carbonyl)isoindoline-1,3-dione |

(continued)

| Compou nd No. | Structure of the compounds | The compounds' name | Chinese translation of the compounds' name |
|---|---|---|---|
| GT-05925 | 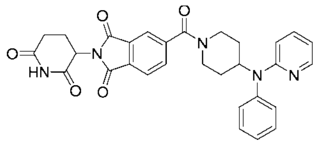 | 2-(2,6-dioxopiperidin-3-yl)-5-(4-(phenyl(pyridin-2-yl)amino)piperidine-1-carbonyl)isoindoline-1,3-dione | 2-(2,6-dioxopiperidin-3-yl)-5-(4-(phenyl(pyridin-2-yl)amino)piperidine-1-carbonyl)isoindoline-1,3-dione |
| GT-06208 | 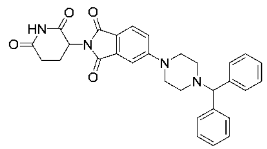 | 5-(4-benzhydrylpiperazin-1-yl)-2-(2,6-dioxopiperidin-3-yl) isoindoline-1,3-dione | 5-(4-benzhydrylpiperazin-1-yl)-2-(2,6-dioxopiperidin-3-yl) isoindoline-1,3-dione |
| GT-06209 | 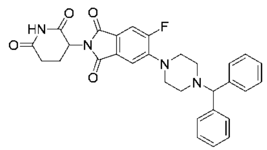 | 5-(4-benzhydrylpiperazin-1-yl)-2-(2,6-dioxopiperidin-3-yl)-6-fluoroisoindoline-1,3-dione | 5-(4-benzhydrylpiperazin-1-yl)-2-(2,6-dioxopiperidin-3-yl)-6-fluoroisoindoline-1,3-dione |
| GT-05937 | 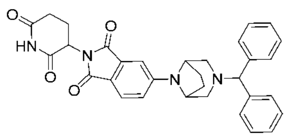 | 5-(3-benzhydryl-3,8-diazabicyclo[3.2.1]octan-8-yl)-2-(2,6-dioxopiperidin-3-yl)isoindoline-1,3-dione | 5-(3-benzhydryl-3,8-diazabicyclo[3.2.1]octan-8-yl)-2-(2,6-dioxopiperidin-3-yl)isoindoline-1,3-dione |
| GT-05975 | 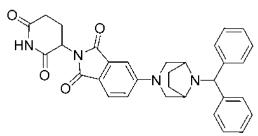 | 5-(8-benzhydryl-3,8-diazabicyclo[3.2.1]octan-3-yl)-2-(2,6-dioxopiperidin-3-yl)isoindoline-1,3-dione | 5-(8-benzhydryl-3,8-diazabicyclo[3.2.1]octan-3-yl)-2-(2,6-dioxopiperidin-3-yl)isoindoline-1,3-dione |
| GT-05938 | 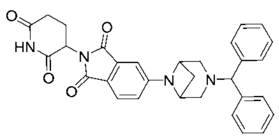 | 5-(3-benzhydryl-3,6-diazabicyclo [3 .1.1]heptan-6-yl)-2-(2,6-dioxopiperidin-3-yl)isoindoline-1,3-dione | 5-(3-benzhydryl-3,6-diazabicyclo[3.1.1]heptan-6-yl)-2-(2,6-dioxopiperidin-3-vl)isoindoline-1,3-dione |
| GT-05939 | 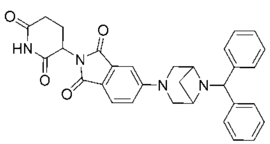 | 5-(6-benzhydryl-3,6-diazabicyclo[3.1.1]heptan-3-yl)-2-(2,6-dioxopiperidin-3-yl)isoindoline-1,3-dione | 5-(6-benzhydryl-3,6-diazabicyclo[3.1.1]heptan-3-yl)-2-(2,6-dioxopiperidin-3-yl)isoindoline-1,3-dione |

(continued)

| Compou nd No. | Structure of the compounds | The compounds' name | Chinese translation of the compounds' name |
|---|---|---|---|
| GT-06205 | 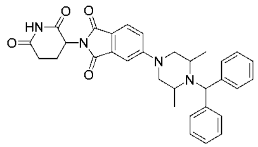 | 5-(4-benzhydryl-3,5-dimethylpiperazin-1-yl)-2-(2,6-dioxopiperidin-3-yl)isoindoline-1,3-dione | 5-(4-benzhydryl-3,5-dimethylpiperazin-1-yl)-2-(2,6-dioxopiperidin-3-yl)isoindoline-1,3-dione |
| GT-05976 | 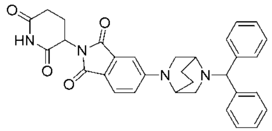 | 5-(5-benzhydryl-2,5-diazabicyclo[2.2.2]octan-2-yl)-2-(2,6-dioxopiperidin-3-yl)isoindoline-1,3-dione | 5-(5-benzhydryl-2,5-diazabicyclo[2.2.2]octan-2-yl)-2-(2,6-dioxopiperidin-3-yl)isoindoline-1,3-dione |
| GT-05932 | 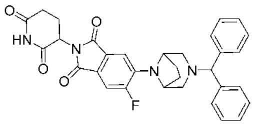 | 5-(3-benzhydryl-3,8-diazabicyclo[3.2.1]octan-8-yl)-2-(2,6-dioxopiperidin-3-yl)-6-fluoroisoindoline-1,3-dione | 5-(3-benzhydryl-3,8-diazabicyclo[3.2.1]octan-8-yl)-2-(2,6-dioxopiperidin-3-yl)-6-fluoroisoindoline-1,3-dione |
| GT-05933 | 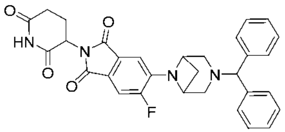 | 5-(3-benzhydryl-3,6-diazabicyclo [3.1.1]heptan-6-yl)-2-(2,6-dioxopiperidin-3-yl)-6-fluoroisoindoline-1,3-dione | 5-(3-benzhydryl-3,6-diazabicyclo[3.1.1]heptan-6-yl)-2-(2,6-dioxopiperidin-3-yl)-6-fluoroisoindoline-1,3-dione |
| GT-06211 | 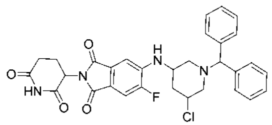 | 5-((1-benzhydryl-5-chloropiperidin-3-yl)amino)-2-(2,6-dioxopiperidin-3-yl)-6-fluoroisoindoline-1,3-dione | 5-((1-benzhydryl-5-chloropiperidin-3-yl)amino)-2-(2,6-dioxopiperidin-3-yl)-6-fluoroisoindoline-1,3-dione |
| GT-05934 | 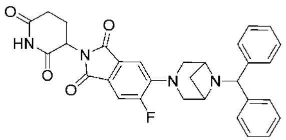 | 5-(6-benzhydryl-3,6-diazabicyclo[3.1.1]heptan-3-yl)-2-(2,6-dioxopiperidin-3-yl)-6-fluoroisoindoline-1,3-dione | 5-(6-benzhydryl-3,6-diazabicyclo[3.1.1]heptan-3-yl)-2-(2,6-dioxopiperidin-3-yl)-6-fluoroisoindoline-1,3-dione |
| GT-05931 | 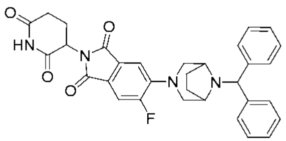 | 5-(8-benzhydryl-3,8-diazabicyclo[3.2.1]octan-3-yl)-2-(2,6-dioxopiperidin-3-yl)-6-fluoroisoindoline-1,3-dione | 5-(8-benzhydryl-3,8-diazabicyclo[3.2.1]octan-3-yl)-2-(2,6-dioxopiperidin-3-yl)-6-fluoroisoindoline-1,3-dione |

(continued)

| Compound No. | Structure of the compounds | The compounds' name | Chinese translation of the compounds' name |
|---|---|---|---|
| GT-05935 | 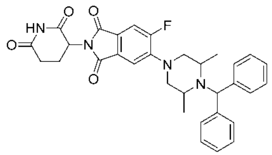 | 5-(4-benzhydryl-3,5-dimethylpiperazin-1-yl)-2-(2,6-dioxopiperidin-3-yl)-6-fluoroisoindoline-1,3-dione | 5-(4-benzhydryl-3,5-dimethylpiperazin-1-yl)-2-(2,6-dioxopiperidin-3-yl)-6-fluoroisoindoline-1,3-dione |
| GT-05936 | 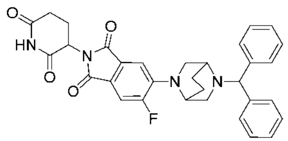 | 5-(5-benzhydryl-2,5-diazabicyclo[2.2.2]octan-2-yl)-2-(2,6-dioxopiperidin-3-yl)-6-fluoroisoindoline-1,3-dione | 5-(5-benzhydryl-2,5-diazabicyclo[2.2.2]octan-2-yl)-2-(2,6-dioxopiperidin-3-yl)-6-fluoroisoindoline-1,3-dione |
| GT-06210 | 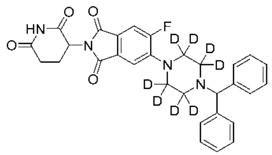 | 5-(4-benzhydrylpiperazin-1-yl-2,2,3,3,5,5,6,6-ds)-2-(2,6-dioxopiperidin-3-yl)-6-fluoroisoindoline-1,3-dione | 5-(4-benzhydrylpiperazin-1-yl-2,2,3,3,5,5,6,6-$d_8$)-2-(2,6-dioxopiperidin-3-yl)-6-fluoroisoindoline-1,3-dione |

**II. Other Forms of Compounds** (including salts, enantiomers, stereoisomers, solvates, isotopically enriched analogs, prodrugs, or polymorphs of compounds)

The compounds of the present disclosure have the structures of any one of Formula (I), Formula (I-1) or Formula (I-2). Unless otherwise specified, all references to the compounds of the present disclosure also include compounds of any one of Formula (I), Formula (I-1) or Formula (I-2) and specific compounds within the scope of these general formulae.
It should be recognized that compounds of the present disclosure (including compounds of Formula (I), Formula (I-1) or Formula (I-2)) may have a stereo-configuration and thus can exist in more than one stereoisomeric form. The present disclosure also relates to optically enriched compounds having a stereo-configuration, e.g., greater than about 90% enantiomeric/diastereomeric excess ("ee"), such as about 95% ee or 97% ee, or greater than 99% ee, and mixtures thereof, including racemic mixtures. As used herein, "optically enriched" means that a mixture of enantiomers consists of a significantly greater proportion of one enantiomer, and can be described by enantiomeric excess (ee%). Purification of isomers and separation of mixtures of isomers can be accomplished by standard techniques known in the art (e.g., column chromatography, preparative TLC, preparative HPLC, asymmetric synthesis (e.g., by using chiral intermediates) and/or or chiral resolution, etc.).
In some embodiments, polymorph forms or salts of the compounds of the present disclosure are also provided. Salts of the compounds of the present disclosure can be pharmaceutically acceptable salts including, but not limited to, hydrochlorides, sulfates, citrates, maleates, sulfonates, citrates, lactates, tartrates, fumarates, phosphates, dihydrophosphates, pyrophosphates, metaphosphates, oxalates, malonates, benzoates, mandelates, succinates, trifluoroacetates, hydroxyacetates, or p-toluenesulfonates, etc. The compounds of the present disclosure can exist as non-solvated or solvated forms in pharmaceutically acceptable solvents such as water, ethanol, and the like. In some embodiments, compounds of the present disclosure can be prepared as prodrugs or precursor drugs. Prodrugs can be converted into parent drugs in the body to play their role. In some embodiments, isotopically-labeled compounds of the present disclosure are also provided, examples of which include deuterium (D or $^2$H).

**III. Compositions/Formulations**

In some embodiments, the present disclosure provides a pharmaceutical composition comprising as an active ingredient the compound of the present disclosure or a pharmaceutically acceptable salt, solvate, isotopically enriched analog, polymorph, prodrug, stereoisomer (including enantiomer), or mixture of stereoisomers thereof, and at least one pharmaceutically acceptable carrier.
In some embodiments, pharmaceutically acceptable carriers include, but are not limited to, fillers, stabilizers, dispersants, suspending agents, diluents, excipients, thickeners, colorants, solvents, or encapsulating materials. Each carrier must be "acceptable" in the sense of being compatible with the other ingredients of the formulation (including the compounds useful in the present disclosure) and not injurious to the patient. Some examples of materials that can be used as pharmaceutically acceptable carriers include: sugars such as lactose, glucose, and sucrose; starches such as corn starch and potato starch; cellulose and its derivatives such as sodium carboxymethyl cellulose, ethyl cellulose and cellulose acetate; powdered tragacanth; malt; gelatin; talc; excipients such as cocoa butter and suppository wax; oils such as peanut oil, cottonseed oil, safflower oil, sesame oil, olive oil, corn oil and soybean oil; glycols such as propylene glycol; polyols such as glycerol, sorbitol, mannitol, and polyethylene glycol; esters such as ethyl oleate and ethyl laurate; agar; buffers such as magnesium hydroxide and aluminum hydroxide; surfactant phosphate buffer solution; polyethylene oxide, polyvinylpyrrolidone, polyacrylamide, poloxamer; and other common non-toxic compatible substances used in pharmaceutical formulations.
The pharmaceutical composition of the present disclosure can further comprise at least one second therapeutic agent, e.g., an anticancer agent. The second therapeutic agent may be used in combination with the compounds of Formula (I) of the present disclosure to treat the diseases or disorders as disclosed herein. The second therapeutic agent includes, but is not limited to, chemotherapeutic agents, immunotherapeutic agents, gene therapy agents, and the like.
The pharmaceutical composition of the present disclosure comprising, as an active ingredient, the compounds of Formula (I) of the present disclosure or a pharmaceutically acceptable salt thereof can be formulated into any suitable formulations such as sprays, patches, tablets (such as conventional tablets, dispersible tablets, orally disintegrating tablets), capsules (such as soft capsules, hard capsules, enteric-coated capsules), dragees, troches, powders, granules, powder injections, suppositories, or liquid formulations (such as suspensions (e.g., aqueous or oily suspensions), solutions, emulsions, or syrups), or conventional injection dosage forms such as injectable solutions (e.g., sterile injectable solutions formulated according to methods known in the art using water, Ringer's solution, or isotonic sodium chloride solution or the like as a vehicle or solvent) or lyophilized injectable formulation and the like, depending upon a suitable route of administration (including, but not limited to, nasal administration, inhalation administration, topical administration, oral administration, oral mucosal administration, rectal administration, intrapleural administration, intraperitoneal administration, vaginal administration, intramuscular administration, subcutaneous administration, transdermal administration, epidural administration, intrathecal administration, and intravenous administration). Those skilled in the art can also formulate the compounds of Formula (I) of the present disclosure into conventional, dispersible, chewable, orally disintegrating or rapidly dissolving formulations, or sustained-release capsules or controlled-release capsules as needed.

The compounds of Formula (I) of the present disclosure, as an active ingredient, is contained in the pharmaceutically acceptable carrier or diluent in an amount sufficient to deliver to a subject a therapeutically effective amount for the indication to be treated, without causing serious toxic effects in the subject treated. A dosage of the active compound for all diseases or disorders mentioned herein ranges, for example, from about 5ng/kg to 500mg/kg, from about 10ng/kg to 300mg/kg per day, such as from 0.1 to 100 mg/kg, or from 0.5 to about 25mg per kilogram body weight of the subject per day.

The compounds of Formula (I) of the present disclosure or pharmaceutically acceptable salts thereof can be conveniently administered in any suitable unit dosage form. Suitable unit dosage form specifications include, but are not limited to, less than 1mg, 1mg to 3000mg, 5mg to 1000mg, for example, 5 to 500mg, 25 to 250mg of active ingredient per unit dosage form.

## IV. Kits/Packaged Products

The compounds of Formula (I) or a pharmaceutically acceptable salt, solvate, isotopically enriched analog, polymorph, prodrug, stereoisomer (including enantiomer), or mixture of stereoisomers thereof is used as a medicament. The medicament of the present disclosure or the pharmaceutical composition of the present disclosure may be presented in a kit/packaged product. The kit/packaged product may include a package or container including, but not limited to, ampoules, blister packs, pharmaceutical plastic bottles, vials, pharmaceutical glass bottles, containers, syringes, laminated flexible packaging, co-extruded film infusion containers, test tubes and dispensing devices, and the like. The kit/packaged product may contain instructions for use of the product.

## V. Methods and Uses

The compounds of Formula (I) or a pharmaceutically acceptable salt, solvate, isotopically enriched analog, polymorph, prodrug, stereoisomer (including enantiomer), or mixture of stereoisomers thereof can be used as a medicament. Especially the compounds of Formula (I) or a pharmaceutically acceptable salt, solvate, isotopically enriched analog, polymorph, prodrug, stereoisomer (including enantiomer), or mixture of stereoisomers thereof can be used for the manufacture of a medicament for the prevention and/or treatment of diseases or disorders associated with cereblon protein. The diseases or disorders associated with cereblon protein comprise: tumor, infectious disease, inflammatory disease, autoimmune disease, anemia, hemorrhagic shock, transplant rejection, multiple organ dysfunction syndrome (MODS), sarcoidosis, adult respiratory distress syndrome, cardiovascular disease, Richter syndrome (RS), acute liver failure, or diabetes. In some embodiments, the diseases or disorders associated with cereblon protein include, but are not limited to, tumor, infectious disease, inflammatory disease, autoimmune disease, anemia, hemorrhagic shock, transplant rejection, multiple organ dysfunction syndrome (MODS), sarcoidosis, adult respiratory distress syndrome, cardiovascular disease, Richter syndrome (RS), acute liver failure, or diabetes. In some embodiments, the diseases or disorders associated with cereblon protein include, but are not limited to, myeloma, including multiple myeloma, plasma cell myeloma, smoldering myeloma, smoldering multiple myeloma; myelofibrosis; bone marrow disease; myelodysplastic syndrome (MDS); previously treated myelodysplastic syndrome; transplantation-related cancer; neutropenia; leukemia, including acute myeloid leukemia, chronic myelogenous leukemia, B-cell chronic lymphocytic leukemia, leukemia-associated anemia, acute myeloid leukemia (AML), acute B-cell lymphoblastic leukemia, acute T-cell lymphoblastic leukemia, chronic lymphocytic leukemia, lymphoma cell leukemia, T-cell lymphoblastic leukemia, monocytic leukemia, myelomonocytic leukemia; lymphoma, including diffuse large B-cell lymphoma, non-Hodgkin's lymphoma, Hodgkin's lymphoma, anaplastic lymphoma, anaplastic large cell lymphoma, CD20 positive lymphoma, mantle cell lymphoma, follicular lymphoma (FL), primary lymphoma, B-cell lymphoma, recurrent B-cell non-Hodgkin's lymphoma, recurrent diffuse large B-cell lymphoma, recurrent mediastinal (thymic) large B-cell lymphoma, primary mediastinal (thymic) large B-cell lymphoma, recurrent transformed non-Hodgkin's lymphoma, refractory B-cell non-Hodgkin's lymphoma, refractory diffuse large B-cell lymphoma, refractory primary mediastinal (thymic) large B-cell lymphoma, and refractory transformed non-Hodgkin's lymphoma; thyroid cancer; melanoma; lung cancer, including lung adenocarcinoma, lung squamous cell carcinoma, non-small cell lung cancer, and small cell lung cancer; inflammatory myofibroblastoma; colorectal cancer; intestinal cancer; brain glioma; astroblastoma; ovarian cancer; bronchial cancer; prostate cancer; breast cancer, including triple negative breast cancer, sporadic breast cancer, and patients with Cowden syndrome; pancreatic cancer; central nervous system tumor; neuroblastoma; neuroglioma; peripheral neuroepithelioma; extramedullary plasmacytoma; plasmacytoma; gastric cancer; gastrointestinal stromal tumors; esophageal cancer; colorectal adenocarcinoma; esophageal squamous cell carcinoma; liver cancer; renal cell carcinoma; bladder cancer; endometrial cancer; metrocarcinoma; head and neck cancer; brain cancer; oral cancer; sarcoma, including rhabdomyosarcoma, various lipogenic tumors, Ewing's sarcoma/primitive neuroectodermal tumors (Ewing/PNETs), and leiomyosarcoma; urothelial carcinoma; basal cell carcinoma; oral squamous cell carcinoma; cholangiocarcinoma; bone cancer; cervical cancer; skin cancer; Richter syndrome (RS); sepsis syndrome; autoimmune diseases, including rheumatoid arthritis, autoimmune encephalomyelitis, ankylosing spondylitis, psoriasis, systemic lupus erythematosus, multiple sclerosis, recurrent oral ulcers, Kawasaki disease, polymyositis/dermatomyositis, Sjogren's syndrome, and atopic dermatitis; keratoconjunctivitis; inflammatory diseases, including Crohn's disease and ulcerative colitis, pneumonia, osteoarthritis; synovitis, systemic inflammatory response syndrome, airway inflammation, and bronchitis; cerebral malaria; infectious diseases, including viral pneumonia, Acquired immunodeficiency syndrome (AIDS), COVID-19 novel coronavirus infection, gram-negative bacteria infection, gram-positive bacteria infection, tuberculosis, etc.; septic shock; tuberculosis; bacterial meningitis; chronic obstructive pulmonary disease; asthma; hemorrhagic shock; organ (including kidney, heart, lung) or tissue transplantation rejection; diabetes; sarcoidosis; adult respiratory distress syndrome; anemia; pediatric aplastic anemia; cardiovascular diseases (e.g., coronary heart disease, congestive heart failure, myocardial infarction, atherosclerosis); multiple organ dysfunction caused by cachexia and septic shock; or acute liver failure.

The present disclosure provides a method for preventing and/or treating diseases or disorders associated with cereblon protein, comprising administering to a subject a therapeutically effective amount of the compounds of Formula (I) of the present disclosure or a pharmaceutically acceptable salt thereof, or the pharmaceutical composition of the present disclosure comprising as an active ingredient the compounds of Formula (I) of the present disclosure or a pharmaceutically acceptable salt thereof. In some embodiments, the diseases or disorders associated with cereblon protein comprise: tumor, infectious disease, inflammatory disease, autoimmune disease, anemia, hemorrhagic shock, transplant rejection, multiple organ dysfunction syndrome (MODS), sarcoidosis, adult respiratory distress syndrome, cardiovascular disease, Richter syndrome (RS), acute liver failure, or diabetes. In some embodiments, the diseases or disorders associated with cereblon protein include, but are not limited to, myeloma, including multiple myeloma, plasma cell myeloma, smoldering myeloma, smoldering multiple myeloma; myelofibrosis; bone marrow disease; myelodysplastic syndrome (MDS); previously treated myelodysplastic syndrome; transplantation-related cancer; neutropenia; leukemia, including acute myeloid leukemia, chronic myelogenous leukemia, B-cell chronic lymphocytic leukemia, leukemia-associated anemia, acute myeloid leukemia (AML), acute B-cell lymphoblastic leukemia, acute T-cell lymphoblastic leukemia, chronic lymphocytic leukemia, lymphoma cell leukemia, T-cell lymphoblastic leukemia, monocytic leukemia, myelomonocytic leukemia; lymphoma, including diffuse large B-cell lymphoma, non-Hodgkin's lymphoma, Hodgkin's lymphoma, anaplastic lymphoma, anaplastic large cell lymphoma, CD20 positive lymphoma, mantle cell lymphoma, follicular lymphoma (FL), primary lymphoma, B-cell lymphoma, recurrent B-cell non-Hodgkin's lymphoma, recurrent diffuse large B-cell lymphoma, recurrent mediastinal (thymic) large B-cell lymphoma, primary mediastinal (thymic) large B-cell lymphoma, recurrent transformed non-Hodgkin's lymphoma, refractory B-cell non-Hodgkin's lymphoma, refractory diffuse large B-cell lymphoma, refractory primary mediastinal (thymic) large B-cell lymphoma, and refractory transformed non-Hodgkin's lymphoma; thyroid cancer; melanoma; lung cancer, including lung adenocarcinoma, lung squamous cell carcinoma, non-small cell lung cancer, and small cell lung cancer; inflammatory myofibroblastoma; colorectal cancer; intestinal cancer; brain glioma; astroblastoma; ovarian cancer; bronchial cancer; prostate cancer; breast cancer, including triple negative breast cancer, sporadic breast cancer, and patients with Cowden syndrome; pancreatic cancer; central nervous system tumor; neuroblastoma; neuroglioma; peripheral neuroepithelioma; extramedullary plasmacytoma; plasmacytoma; gastric cancer; gastrointestinal stromal tumors; esophageal cancer; colorectal adenocarcinoma; esophageal squamous cell carcinoma; liver cancer; renal cell carcinoma; bladder cancer; endometrial cancer; metrocarcinoma; head and neck cancer; brain cancer; oral cancer; sarcoma, including rhabdomyosarcoma, various lipogenic tumors, Ewing's sarcoma/-primitive neuroectodermal tumors (Ewing/PNETs), and leiomyosarcoma; urothelial carcinoma; basal cell carcinoma; oral squamous cell carcinoma; cholangiocarcinoma; bone cancer; cervical cancer; skin cancer; Richter syndrome (RS); sepsis syndrome; autoimmune diseases, including rheumatoid arthritis, autoimmune encephalomyelitis, ankylosing spondylitis, psoriasis, systemic lupus erythematosus, multiple sclerosis, recurrent oral ulcers, Kawasaki disease, polymyositis/dermatomyositis, Sjogren's syndrome, and atopic dermatitis; keratoconjunctivitis; inflammatory diseases, including Crohn's disease and ulcerative colitis, pneumonia, osteoarthritis; synovitis, systemic inflammatory response syndrome, airway inflammation, and bronchitis; cerebral malaria; infectious diseases, including viral pneumonia, Acquired immunodeficiency syndrome (AIDS), COVID-19 novel coronavirus infection, gram-negative bacteria infection, gram-positive bacteria infection, tuberculosis, etc.; septic shock; tuberculosis; bacterial meningitis; chronic obstructive pulmonary disease; asthma; hemorrhagic shock; organ (including kidney, heart, lung) or tissue transplantation rejection; diabetes; sarcoidosis; adult respiratory distress syndrome; anemia; pediatric aplastic anemia; cardiovascular diseases (e.g., coronary heart disease, congestive heart failure, myocardial infarction, atherosclerosis); multiple organ dysfunction caused by cachexia and septic shock; or acute liver failure.

In the method for preventing and/or treating diseases or disorders associated with cereblon protein, the compound of Formula (I) of the present disclosure or the pharmaceutical composition comprising as an active ingredient the compound of Formula (I) of the present disclosure is administered to the subject through at least one mode of

administration selected from the group consisting of nasal administration, inhalation administration, topical administration, oral administration, oral mucosal administration, rectal administration, pleural cavity administration, peritoneal administration, vaginal administration, intramuscular administration, subcutaneous, transdermal, epidural, intrathecal, and intravenous administration.

The term "treatment" or "treating" refers to the administration of the compound of Formula (I) or a pharmaceutically acceptable salt thereof according to the present disclosure, or the pharmaceutical composition containing, as an active ingredient, the compound of Formula (I) or a pharmaceutically acceptable salt thereof, to a subject to mitigate (alleviate) undesirable diseases or conditions, such as the development of a cancer or tumor. The beneficial or desired clinical results of the present disclosure include, but are not limited to: alleviating symptoms, reducing the severity of the disease, stabilizing the state of the disease, slowing down or delaying the progression of the disease, improving or alleviating the condition, and alleviating the disease.

A "therapeutic effective amount" of the compound of the present disclosure depends on a variety of factors, including the activity of the specific compound used, the metabolic stability of the compound and the duration of its action, the age, sex and weight of the patient, the patient's current medical condition, the route and duration of administration, the excretion rate, the combined administration of additional drugs, and the progression of the diseases or conditions of the patient being treated. Those skilled in the art will be able to determine appropriate dosages based on these and other factors.

It is to be understood that the choice of using one or more active compounds and/or compositions and their dosage depends on the basic situations of the individual (which should generally render the individual situation to achieve the best effect). Dosing and dosing regimens should be within the ability of those skilled in the art, and the appropriate dosage depends on many factors including the knowledge and ability of the physicians, veterinarians or researchers (see e.g., Jun Li (chief editor), "Clinical Pharmacology", 4th edition, People's Public Health Press, 2008).

As used herein, the term "patient" or "subject" to be treated refers to animal, for example mammal, including but not limited to primate (such as human being), cow, sheep, goat, horse, dog, cat, rabbit, guinea pig, rat, mice, etc.

VI. **Definitions**

Unless otherwise specified, the following words, phrases and symbols used herein generally have the meanings as described below.

In general, the nomenclature used herein (including the IUPAC nomenclature) and the laboratory procedures described below (including those used in cell culture, organic chemistry, analytical chemistry, and pharmacology, etc.) are those well-known and commonly used in the art. Unless otherwise defined, all scientific and technical terms used herein in connection with the present disclosure described herein have the same meaning as commonly understood by one skill in the art. In addition, the use of the word "a" or "an" when used in conjunction with the term "comprising" or a noun in the claims and/or the specification may mean "one", but it is also consistent with the meaning of "one or more", "at least one", and "one or more than one". Similarly, the terms "another" or "other" can mean at least a second or more.

It should be understood that whenever the term "comprise" or "include" is used herein to describe various aspects, other similar aspects described by "consisting of" and/or "consisting essentially of" are also provided.

As used herein, the term "about" used alone or in combination refers to approximately, roughly, nearly, or around. When the term "about" is used in conjunction with a numerical range, it modifies that range by extending the boundaries above and below the stated numerical value. In general, the term "about" can modify a numerical value above and below the stated value by an upward or downward (increasing or decreasing) variation, e.g., 10%, 5%, 2%, or 1%.

As used herein, the wording "...represents a bond" used alone or in combination means that the referenced group is a bond linker (that is, the referenced group is absent). For example, the wording "$X_1$ represents a bond" means that $X_1$ is a bond linker. In other words, when $X_1$ represents a bond, the group $R_{a1}$ in the structure of Formula (I) is directly connected to $L_1$ in the structure of Formula (I). For example, the wording "$W_1$ represents a bond" means that $W_1$ is a bond linker. In other words, when $W_1$ represents a bond, the group

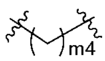

in the structure of Formula (III) is directly connected to ring A1 and ring B 1, respectively.

In this document, the term "optionally substituted" used alone or in combination means that the referenced group may be unsubstituted or substituted with one or more substituents as defined here. Herein, the wording "optionally substituted with..." and "unsubstituted or substituted" can be used interchangeably. The term "substituted" generally indicates that one or more hydrogens in the referenced structure are replaced by the same or different specific substituents. The number of substituents is not theoretically limited in any way, or is automatically limited by the size of the building units (i.e., the total number of replaceable hydrogen atoms in the building units), or as clearly defined herein.

Herein, a bond interrupted by a wavy line shows the point of attachment of the depicted group to the rest of the molecule. For example, the group $R_{a1}$ depicted below

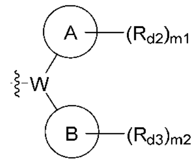

shows the point of attachment of W of said group $R_{a1}$ to $X_1$. For example, in the structure of Formula (IV) depicted below:

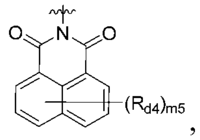

the nitrogen atom N is connected to group $X_1$, and substituent $R_{d4}$ may replace one or more hydrogen atoms on any carbon atom in the structure of Formula (IV), where the number m5 of $R_{d4}$ substituents is as defined herein.

As used herein, the term "oxo", used alone or in combination, refers to = O.

As used herein, the term "halogen atom" or "halogen", used alone or in combination, refers to fluorine, chlorine, bromine, or iodine.

As used herein, the term "alkyl", used alone or in combination, refers to a linear or branched alkyl group. The term "$C_x$-$C_y$ alkyl" or "$C_{x-y}$ alkyl" (x and y each being an integer) refers to a linear or branched alkyl group containing from x to y carbon atoms. The term "$C_{1-6}$ alkyl" used alone or in combination in the present disclosure refers to a linear or branched alkyl group containing from 1 to 6 carbon atoms, examples of which include $C_1$-$C_5$ alkyl, $C_1$-$C_4$ alkyl, $C_1$-$C_3$ alkyl and $C_1$-$C_2$ alkyl. Representative examples include methyl, ethyl, n-propyl, isopropyl, n-butyl, isobutyl, sec-butyl, tert-butyl, pentyl, isopentyl, neopentyl, tert-pentyl, and hexyl. The term "$C_{1-3}$ alkyl" or "$C_1$-$C_3$ alkyl" in the present disclosure refers to an alkyl group containing from 1 to 3 carbon atoms, and representative examples of which include methyl, ethyl, n-propyl, and isopropyl. In the present disclosure, the "alkyl" is optionally substituted with one or more substituents optionally selected from the group consisting of halogen, hydroxy, cyano, $C_{1-3}$ alkyl, $C_{1-3}$ alkoxy, halogenated $C_{1-6}$ alkoxy, halogenated $C_{1-6}$ alkyl (e.g., trifluoromethyl), $C_{3-6}$ cycloalkyl, 4- to 7-membered heterocyclyl, or a combination thereof.

As used herein, the term "halogenated alkyl" or "haloalkyl", used alone or in combination, refers to a linear or branched alkyl group substituted with one or more halogens, wherein one or more hydrogen atom(s) of the alkyl group are replaced with one or more halogens. The term "halogenated $C_{x-Cy}$ alkyl" or "halogenated $C_{x-y}$ alkyl" (x and y are each an integer) refers to a linear or branched alkyl containing from x to y carbon atoms substituted with one or more halogens. The term "halogenated $C_{1-10}$ alkyl" used alone or in combination in the present disclosure refers to a linear or branched alkyl group containing from 1 to 10 carbon atoms substituted with one or more halogens. Examples of the halogenated $C_{1-10}$ alkyl group of the present disclosure include halogenated $C_{1-9}$ alkyl, such as halogenated $C_{1-8}$ alkyl, halogenated $C_{2-8}$ alkyl, halogenated $C_{1-7}$ alkyl, halogenated $C_{1-6}$ alkyl, halogenated $C_{1-5}$ alkyl, or halogenated $C_{1-4}$ alkyl. Representative examples include halomethyl, haloethyl, halo-n-propyl, haloisopropyl, halo-n-butyl, haloisobutyl, halo-sec-butyl, halo-tert-butyl, halopentyl, haloisoamyl, haloneopentyl, halo-tert-pentyl, halohexyl, haloheptyl, halooctyl, halononyl, and halodecyl. The term "halo-$C_{1-3}$ alkyl" or " halogenated $C_1$-$C_3$ alkyl" of the present disclosure refers to an alkyl group containing from 1 to 3 carbon atoms substituted with one or more halogens, and its representative examples include halomethyl (e.g., trifluoromethyl), haloethyl, halo-n-propyl and haloisopropyl.

As used herein, the term "optionally deuterated alkyl", used alone or in combination, refers to a linear or branched alkyl group optionally substituted with one or more deuterium atoms, wherein one or more hydrogen atom(s) of the alkyl group are optionally replaced with one or more deuterium atoms. The term "optionally deuterated $C_{x-Cy}$ alkyl" or "optionally deuterated $C_{x-y}$ alkyl" (x and y are each an integer) refers to a linear or branched alkyl containing from x to y carbon atoms substituted with one or more deuterium atoms. The term "optionally deuterated $C_{1-6}$ alkyl" used alone or in combination in the present disclosure refers to a linear or branched alkyl group containing from 1 to 6 carbon atoms substituted with one or more deuterium atoms. Examples of the optionally deuterated $C_{1-6}$ alkyl group of the present disclosure include optionally deuterated $C_{1-5}$ alkyl group and optionally deuterated $C_{1-4}$ alkyl group. Representative examples include perdeuterated methyl ($CD_3$), perdeuterated ethyl ($CD_3CD_2$), perdeuterated n-propyl, perdeuterated isopropyl, perdeuterated n-butyl, perdeuterated isobutyl, perdeuterated sec-butyl, perdeuterated tert-butyl, perdeuterated pentyl, perdeuterated isopentyl, perdeuterated neopentyl, perdeuterated tert-pentyl, perdeuterated hexyl, methyl, ethyl, n-propyl, isopropyl, n-butyl, isobutyl, sec-butyl, tert-butyl, pentyl, isopentyl, neopentyl, tert-pentyl, and hexyl. The term "optionally deuterated $C_{1-3}$ alkyl" or "optionally deuterated $C_1$-$C_3$ alkyl" of the present disclosure refers to an alkyl group containing from 1 to 3 carbon

atoms substituted with one or more deuterium atoms, and its representative examples include perdeuterated methyl ($CD_3$), perdeuterated ethyl ($CD_3CD_2$), methyl, ethyl, and n-propyl.

As used herein, the term "alkylene" (which is used interchangeably with "alkylene chain"), used alone or in combination, refers to a substituent formed by removing one hydrogen atom from an alkyl group, specifically indicating a linear or branched divalent saturated hydrocarbon group composed of carbon and hydrogen atoms. The term "$C_x$-$C_y$ alkylene" or "$C_{x-y}$ alkylene" (x and y each being an integer) refers to a linear or branched alkylene group containing from x to y carbon atoms. The term "$C_1$-$C_6$ alkylene", used alone or in combination, refers to a linear or branched alkylene group containing from 1 to 6 carbon atoms, examples of which include $C_1$-$C_5$ alkylene, $C_1$-$C_4$ alkylene, $C_1$-$C_3$ alkylene, and $C_1$-$C_2$ alkylene. Representative examples include, but are not limited to, methylene, ethylene, propylene, isopropylene, butylene, isobutylene, sec-butylene, tert-butylene, n-pentylene, isopentylene, neopentylene, tert-pentylene, and hexylene. In the present disclosure, the "alkylene" is optionally substituted with one or more substituents optionally selected from $C_1$-$C_3$ alkyl, $C_{3-6}$ cycloalkyl, hydroxy, amino, mercapto, halogen, $C_1$-$C_3$ alkoxy, $C_1$-$C_3$ alkylamino, halogenated $C_1$-$C_3$ alkyl, amino$C_{1-3}$ alkylene, $C_{1-3}$ alkyl-NHC(O)-, $C_{1-3}$ alkyl-C(O)NH-, cyano or any combination thereof.

As used herein, the term "alkoxy", used alone or in combination, refers to a linear or branched alkoxy group having structural formula of alkyl-O-. Optionally, the alkyl portion of the alkoxy group may contain from 1 to 6 carbon atoms. Representative examples of "alkoxy" include, but are not limited to, methoxy, ethoxy, propoxy, isopropoxy, n-butoxy, isobutoxy, tert-butoxy, pentyloxy, 2-pentyloxy, isopentyloxy, neopentyloxy, hexyloxy, 2-hexyloxy, 3-hexyloxy, and 3-methylpentyloxy, etc. The term "$C_1$-$C_3$ alkoxy" or "$C_{1-3}$ alkoxy" refers to a linear or branched alkoxy group containing from 1 to 3 carbon atoms. Representative examples of $C_{1-3}$ alkoxy include, but are not limited to, methoxy, ethoxy, n-propoxy, and isopropoxy.

As used herein, the term "halogenated alkoxy", used alone or in combination, refers to an alkoxy group substituted with one or more halogen atoms. Optionally, the alkyl portion of the alkoxy group may contain from 1 to 6 carbon atoms. Examples of "halogenated alkoxy" include, but are not limited to, halogenated $C_{1-6}$ alkoxy or halogenated $C_{1-4}$ alkoxy. Representative examples include, but are not limited to, $F_3C$-O-, $FCH_2$-O-, $F_2CH$-O-, $ClCH_2$-O-, $Cl_2CH$-O-, $CF_3CF_2$-O-, $CF_3CHF$-O-, $CHF_2CF_2$-O-, $CHF_2CHF$-O-, $CF_3CH_2$-O-, or $CH_2ClCH_2$-O-.

As used herein, the term "alkyl-NH-", used alone or in combination, refers to a linear or branched alkyl-NH-, wherein alkyl is as defined above. Optionally, the alkyl portion of the alkyl-NH- group may contain 1-10 carbon atoms, i.e., $C_{1-10}$ alkyl-NH-. Representative examples of "alkyl-NH-" include, but are not limited to, methyl-NH-, ethyl-NH-, n-propyl-NH-, isopropyl-NH-, n-butyl-NH-, isobutyl-NH-, tert-butyl-NH-, pentyl-NH-, and hexyl-NH-, etc. The term "$C_1$-$C_3$ alkyl-NH-" or "$C_{1-3}$ alkyl-NH-" refers to a linear or branched alkyl-NH- group containing from 1 to 3 carbon atoms. Representative examples of $C_{1-3}$ alkyl-NH- include, but are not limited to, methyl-NH-, ethyl-NH-, n-propyl-NH-, and isopropyl-NH-.

As used herein, the term "$NH_2$-alkylene", used alone or in combination, refers to a linear or branched alkylene group substituted with amino, wherein alkylene is as defined above. Optionally, the alkylene portion of the "$NH_2$-alkylene" group may contain 1-10 carbon atoms. The term "$NH_2$-$C_{1-3}$ alkylene" or "amino-$C_{1-3}$ alkylene-" refers to a linear or branched alkylene group containing from 1 to 3 carbon atoms which is substituted with amino. Representative examples of $NH_2$-$C_{1-3}$ alkylene include, but are not limited to, $NH_2$-$CH_2$-, $NH_2$-$CH_2CH_2$-, and $NH_2$-$CH_2CH_2CH_2$-.

As used herein, the term "alkyl-NHC(O)-", used alone or in combination, refers to a linear or branched alkyl-NHC(O)- group, wherein alkyl is as defined above. Optionally, the alkyl portion of the alkyl-NHC(O)- group may contain 1-10 carbon atoms. The term "$C_{1-3}$ alkyl-NHC(O)-" or "$C_1$-$C_3$ alkyl-NHC(O)-" refers to a linear or branched alkyl-NHC(O)- group containing from 1 to 3 carbon atoms. Representative examples of $C_{1-3}$ alkyl-NHC(O)- include, but are not limited to, $CH_3$-NHC(O)-, $CH_3CH_2$-NHC(O)-, and $CH_3CH_2CH_2$-NHC(O)-.

As used herein, the term "alkyl-C(O)NH-", used alone or in combination, refers to a linear or branched alkyl-C(O)NH- group, wherein alkyl is as defined above. Optionally, the alkyl portion of the alkyl-C(O)NH- group may contain 1-10 carbon atoms. The term "$C_{1-3}$ alkyl-C(O)NH-" or "$C_1$-$C_3$ alkyl-C(O)NH-" refers to a linear or branched alkyl-C(O)NH- group containing from 1 to 3 carbon atoms. Representative examples of $C_{1-3}$ alkyl-C(O)NH- include, but are not limited to, $CH_3$-C(O)NH-, $CH_3CH_2$-C(O)NH-, and $CH_3CH_2CH_2$-C(O)NH-.

As used herein, the term "heteroaryl", used alone or in combination, refers to a 5- to 30-membered (optionally 5- to 20-membered, 5- to 15-membered, 5- to 12-membered, 5- to 11-membered, 5- to 10-membered, 5- to 9-membered, 5- to 8-membered, 5- to 7-membered, 5- to 6-membered, 6- to 15-membered, or 6- to 9-membered) monocyclic, bicyclic, or polycyclic cyclic radical containing at least one aromatic ring having one or more (e.g., from 1 to 6, or from 1 to 5, or from 1 to 4, or from 1 to 3) heteroatoms independently selected from the group consisting of oxygen, nitrogen, and sulfur. Bicyclic or polycyclic heteroaryl groups include bicyclic, tricyclic or tetracyclic heteroaryl groups, which contain one aromatic ring having one or more heteroatoms independently selected from O, S and N, and the remaining rings which may be a saturated, partially unsaturated or aromatic ring and can be carbocyclic ring or contain one or more heteroatoms independently selected from O, S and N. Examples of monocyclic heteroaryl groups include, but are not limited to, furanyl, oxazolyl, isoxazolyl, oxadiazolyl, thienyl, thiazolyl, isothiazolyl, thiadiazolyl, pyrrolyl, imidazolyl, pyrazolyl, triazolyl, pyridyl, pyrimidinyl, pyridazinyl, pyrazinyl, tetrazolyl, and triazinyl. Examples of bicyclic heteroaryl groups include, but are not limited to, indolyl, isoindolyl, isoindolinyl, benzofuranyl, isobenzofuranyl, benzothienyl, indazolyl, benzimidazolyl, benzoxazolyl, benzisoxazolyl, benzothiazolyl, benzisothiazolyl, benzotriazolyl, benzo[2,1,3]oxadiazolyl, benzo [2,1,3]thiadiazolyl, benzo[1,2,3]thiadiazolyl, quinolinyl, isoquinolinyl, naphthyridinyl, cinnolinyl, quinazolinyl, quinoxalinyl, phthalazinyl, oxazolopyridyl, furopyridyl, pteridyl, purinyl, pyridopyridyl, pyrazolo[1,5-a]pyridyl, pyrazolo[1,5-a]pyrimidinyl, imidazo[1,2-a]pyridyl, 1H-pyrrolo[3,2-b]pyridyl, 1H-pyrrolo[2,3-b]pyridyl, pyrrolo[2,1-b]thiazolyl and imidazo[2,1-b] thiazolyl. Examples of tricyclic or polycyclic heteroaryl groups include, but are not limited to, acridinyl, benzindolyl, carbazolyl, dibenzofuranyl, and xanthyl. The heteroaryl group may be unsubstituted or substituted. A substituted heteroaryl refers to heteroaryl substituted one or more times (e.g., 1-4, 1-3, or 1-2 times) by a substituent(s) optionally selected from the group consisting of $C_{1-3}$ alkyl, $C_{3-6}$ cycloalkyl, hydroxy, amino, mercapto, halogen, $C_{1-3}$ alkoxy, $C_{1-3}$ alkylamino, halogenated $C_{1-3}$ alkyl, amino $C_{1-3}$ alkylene, $C_{1-3}$ alkyl-NHC(O)-, $C_{1-3}$ alkyl-C(O)NH-, cyano, or any combination thereof.

As used herein, the term "heteroarylene", used alone or in combination, refers to a substituent formed by removing one hydrogen atom from a heteroaryl group, specifically indicating a 5- to 30-membered (optionally 5- to 20-membered, 5- to 15-membered, 5- to 12-membered, 5- to 11-membered, 5- to 10-membered, 5- to 9-membered, 5- to 8-membered, 5- to 7-membered, 5- to 6-membered, 6- to 15-membered, or 6- to 9-membered) bivalent monocyclic, bicyclic, or polycyclic cyclic radical containing at least one aromatic ring having one or more (e.g., from 1 to 6, or from 1 to 5, or from 1 to 4, or from 1 to 3) heteroatoms independently selected from the group consisting of oxygen, nitrogen, and sulfur. Bicyclic or polycyclic heteroarylene groups include bicyclic, tricyclic or tetracyclic heteroarylene groups, which contain one aromatic ring having one or more heteroatoms independently selected from O, S and N, and the remaining ring(s) which may be a saturated, partially unsaturated or aromatic ring and can be carbocyclic ring or contain one or more heteroatoms independently selected from O, S and N. Examples of monocyclic heteroarylene groups include, but are not limited to, furanylene, oxazolylene, isoxazolylene, oxadiazolylene, thienylene, thiazolylene, isothiazolylene, thiadiazolylene, pyrrolylene, imidazolylene, pyrazolylene, triazolylene, pyridylene, pyrimidinylene, pyridazinylene, pyrazinylene, tetrazolylene, and triazinylene. Examples of bicyclic heteroarylene groups include, but are not limited to, indolylene, isoindolylene, isoindolinylene, benzofuranylene, isobenzofuranylene, benzothienylene, indazolylene, benzimidazolylene, benzoxazolylene, benzisoxazolylene, benzothiazolylene, benzisothiazolylene, benzotriazolylene, benzo[2,1,3]oxadiazolylene, benzo[2,1,3]thiadiazolylene, benzo[1,2,3]thiadiazolylene, quinolinylene, isoquinolinylene, naphthyridinylene, cinnolinylene, quinazolinylene, quinoxalinylene, phthalazinylene, oxazolopyridylene, furopyridylene, pteridylene, purinylene, pyridopyridylene, pyrazolo[1,5-a]pyridylene, pyrazolo[1,5-a]pyrimidinylene, imidazo[1,2-a]pyridylene, 1H-pyrrolo[3,2-b]pyridylene, 1H-pyrrolo[2,3-b]pyridylene, pyrrolo[2,1-b]thiazolylene, and imidazo[2,1-b]thiazolylene. Examples of tricyclic heteroarylene groups include, but are not limited to, acridinylene, benzindolylene, carbazolylene, dibenzofuranylene, and xanthylene. The heteroarylene group may be unsubstituted or substituted. A substituted heteroarylene refers to heteroarylene substituted one or more times (e.g., 1-4, 1-3, or 1-2 times) by a substituent(s) optionally selected from the group consisting of $C_{1-3}$ alkyl, $C_{3-6}$ cycloalkyl, hydroxy, amino, mercapto, halogen, $C_{1-3}$ alkoxy, $C_{1-3}$ alkylamino, halogenated $C_{1-3}$ alkyl, amino-substituted $C_{1-3}$ alkylene, $C_{1-3}$ alkyl-NHC(O)-, $C_{1-3}$ alkyl-C(O)NH-, cyano, or any combination thereof.

As used herein, the term "aryl", used alone or in combination, refers to a monovalent aromatic hydrocarbon group containing from 5 to 30 (e.g., 5 to 20, 5 to 15, or 5 to 14) carbon atoms and optionally one or more fused rings, such as phenyl group, naphthyl group, or fluorenyl group. As used herein, the "aryl" is optionally substituted. A substituted aryl group refers to an aryl group substituted one or more times (e.g., 1-4, 1-3, or 1-2 times) with a substituent(s). For example, aryl is mono-, di-, or tri-substituted with a substituent(s) optionally selected from e.g., $C_{1-3}$ alkyl, $C_{3-6}$ cycloalkyl, hydroxy, amino, mercapto, halogen, $C_{1-3}$ alkoxy, $C_{1-3}$ alkylamino, halogenated $C_{1-3}$ alkyl, amino-substituted $C_{1-3}$ alkylene, $C_{1-3}$ alkyl-NHC(O)-, $C_{1-3}$ alkyl-C(O)NH-, cyano, or any combination thereof.

As used herein, the term "arylene", used alone or in combination, refers to a substituent formed by removing one hydrogen atom from an aryl group, specifically indicating a divalent aromatic hydrocarbon group containing from 5 to 30 (e.g., 5 to 20, 5 to 15, or 5 to 14) carbon atoms and optionally one or more fused rings, such as phenylene, naphthylene, or fluorenylene. As used herein, the "arylene" is optionally substituted. A substituted arylene refers to an arylene group optionally substituted one or more times (e.g., 1-4, 1-3, or 1-2 times) with a substituent(s). For example, arylene is mono-, di-, or tri-substituted with a substituent(s) optionally selected from e.g., $C_{1-3}$ alkyl, $C_{3-6}$ cycloalkyl, hydroxy, amino, mercapto, halogen, $C_{1-3}$ alkoxy, $C_{1-3}$ alkylamino, halogenated $C_{1-3}$ alkyl, amino-substituted $C_{1-3}$ alkylene, $C_{1-3}$ alkyl-NHC(O)-, $C_{1-3}$ alkyl-C(O)NH-, cyano, or any combination thereof.

As used herein, the term "cycloalkyl", used alone or in combination, refers to a saturated or partially unsaturated (i.e., containing one or more double bonds, but not having a fully conjugated $\pi$-electron system) monocyclic or bicyclic or polycyclic cyclic hydrocarbon radical, which in some embodiments contains from 3 to 30 carbon atoms (i.e., $C_{3-30}$ cycloalkyl), from 3 to 20 carbon atoms (i.e., $C_{3-20}$ cycloalkyl), or from 3 to 15 carbon atoms (i.e., $C_{3-15}$ cycloalkyl), or from 3 to 12 carbon atoms (i.e., $C_{3-12}$ cycloalkyl), or from 3 to 11 carbon atoms (i.e., $C_{3-11}$ cycloalkyl), or from 3 to 10 carbon atoms (i.e., $C_{3-10}$ cycloalkyl), or from 3 to 8 carbon atoms ( i.e., $C_{3-8}$ cycloalkyl), or from 3 to 7 carbon atoms (i.e., $C_{3-7}$ cycloalkyl), or from 3 to 6 carbon atoms (i.e., $C_{3-6}$ cycloalkyl). The term "cycloalkyl" includes monocyclic, bicyclic, tricyclic or polycyclic cyclic hydrocarbon radical having from 3 to 30 carbon atoms (e.g., from 3 to 20 carbon atoms). Representative examples of monocyclic cycloalkyl groups include, but are not limited to, cyclopropyl, cyclobutyl, cyclopentyl, cyclopentenyl, cyclohexyl, cyclohexenyl, cycloheptyl, and cyclooctyl. Bicyclic, tricyclic and polycyclic cycloalkyl groups include bridged cycloalkyl, fused cycloalkyl and spiro-cycloalkyl groups, examples of which include, but not limited to, decalinyl, octahydropentalenyl, octahydro-1H-indenyl, spiro-cycloalkyl, adamantanyl, noradamantanyl, bornyl, norbornyl (also named as bicyclo[2.2.1]heptyl by the IUPAC system). As used herein, the "cycloalkyl" is optionally mono- or poly-substituted, such as, but not limited to, 2,2-, 2,3-, 2,4-, 2,5-, or 2,6-disubstituted cyclohexyl. The substituents of the substituted "cycloalkyl" can be optionally one or more (e.g., 1-5, 1-4, 1-3, 1-2, or 1) substituents selected from the group consisting of $C_{1-3}$ alkyl, $C_{3-6}$ cycloalkyl, hydroxy, amino, mercapto, halogen, $C_{1-3}$ alkoxy, $C_{1-3}$ alkylamino, halogenated $C_{1-3}$ alkyl, amino-substituted $C_{1-3}$ alkylene, $C_{1-3}$ alkyl-NHC(O)-, $C_{1-3}$ alkyl-C(O)NH-, cyano, or any combination thereof. Examples of "$C_{3-6}$ cycloalkyl" include, but are not limited to, cyclopropyl, cyclobutyl, cyclopentyl, cyclopentenyl, cyclohexenyl, and cyclohexyl.

As used herein, the term "$C_{x-y}$ spiro-cycloalkyl" (x and y each being an integer), used alone or in combination, refers to a spiro-cycloalkyl group containing from x to y carbon atoms. As used herein, the term "$C_{5-15}$ spiro-cycloalkyl", used alone or in combination, refers to a spiro-cycloalkyl group containing from 5 to 15 carbon atoms (e.g., 7-15, 5-11, 5-10, and 7-9 carbon atoms). The term "$C_{5-15}$ spiro-cycloalkyl" includes "$C_{7-15}$ spiro-cycloalkyl". Representative examples of "$C_{5-15}$ spiro-cycloalkyl" include, but are not limited to, spiro[3.3]heptyl, spiro[2.5]octyl, spiro[3.5]nonyl, spiro[4.4]nonyl, spiro[4.5]decyl, or spiro[5.5]undecyl. The term "$C_{5-15}$ spiro-cycloalkyl" is optionally substituted with one or more substituents selected from the group consisting of $C_{1-3}$ alkyl, halogenated $C_{1-3}$ alkyl, $C_{1-3}$ alkoxy, deuterated $C_{1-3}$ alkoxy, $C_{1-3}$ alkylamino, amino, oxo, halogen, hydroxy, cyano or any combination thereof.

As used herein, the term "$C_{x-y}$ bridged cycloalkyl" (x and y each being an integer), used alone or in combination, refers to a bridged cycloalkyl group containing from x to y carbon atoms. As used herein, the term "$C_{5-15}$ bridged cycloalkyl", used alone or in combination, refers to a bridged cycloalkyl group containing from 5 to 15 carbon atoms (e.g., 7-15, 5-11, 5-10, and 7-9 carbon atoms). Representative examples of "$C_{5-15}$ bridged cycloalkyl" include, but are not limited to, adamantanyl, noradamantanyl, norbornyl (also named as bicyclo[2.2.1]heptyl by the IUPAC system), and cubanyl. The term "bridged cycloalkyl" is optionally substituted with 1-10 substituents selected from the group consisting of $C_{1-3}$ alkyl, deuterated $C_{1-3}$ alkyl, $C_{1-3}$ alkoxy, halogen, halogenated $C_{1-3}$ alkyl, $C_{1-3}$ alkylamino, amino, hydroxy, cyano, oxo, or any combination thereof.

As used herein, the term "cycloalkylene", used alone or in combination, refers to a substituent formed by removing one hydrogen atom from a cycloalkyl group, specifically indicating a saturated or partially unsaturated (i.e., containing one or more double bonds without full conjugation) divalent monocyclic or bicyclic or polycyclic cyclic hydrocarbon radical, which in some embodiments contains from 3 to 30 carbon atoms (i.e., $C_{3-30}$ cycloalkylene), from 3 to 20 carbon atoms (i.e., $C_{3-20}$ cycloalkylene), or from 3 to 15 carbon atoms (i.e., $C_{3-15}$ cycloalkylene), or from 3 to 12 carbon atoms (i.e., $C_{3-12}$ cycloalkylene), or from 3 to 11 carbon atoms (i.e., $C_{3-11}$ cycloalkylene), or from 3 to 10 carbon atoms (i.e., $C_{3-10}$ cycloalkylene), or from 3 to 8 carbon atoms ( i.e., $C_{3-8}$ cycloalkylene), or from 3 to 7 carbon atoms (i.e., $C_{3-7}$ cycloalkylene), or from 3 to 6 carbon atoms (i.e., $C_{3-6}$ cycloalkylene). The term "cycloalkylene" includes monocyclic, bicyclic, tricyclic and polycyclic divalent hydrocarbon groups having 3 to 30 (e.g., 3 to 12) carbon atoms. Representative examples of monocyclic cycloalkylene include, but are not limited to, cyclopropylene, cyclobutylene, cyclopentylene, cyclopentenylene, cyclohexylene, cyclohexenylene, cycloheptylene, and cyclooctylene. Bicyclic, tricyclic and polycyclic cycloalkylene groups include bridged cycloalkylene, fused cycloalkylene and spiro-cycloalkylene groups, examples of which include, but not limited to, decalinylene, octahydropentalenylene, octahydro-1H-indenylene, 2,3-dihydro-1H-indenylidene, spiro-cycloalkylene, adamantanylene, noradamantanylene, and norbornylene (also named as bicyclo [2.2.1]heptanylene by the IUPAC system). As used herein, the "cycloalkylene" is optionally mono- or poly-substituted, such as, but not limited to, 2,2-, 2,3-, 2,4-, 2,5-, or 2,6-disubstituted cyclohexyl. The substituents of the substituted "cycloalkylene" may optionally be one or more substituents selected from the group consisting of $C_{1-3}$ alkyl, $C_{3-6}$ cycloalkyl, hydroxy, amino, mercapto, halogen, $C_{1-3}$ alkoxy, $C_{1-3}$ alkylamino, halogenated $C_{1-3}$ alkyl, amino-substituted $C_{1-3}$ alkylene, $C_{1-3}$ alkyl-NHC(O)-, $C_{1-3}$ alkyl-C(O)NH-, cyano, or any combination thereof.

As used herein, the term "$C_{x-y}$ spiro-cycloalkylene" (x and y each being an integer), used alone or in combination, refers to a spiro-cycloalkylene group containing from x to y carbon atoms. As used herein, the term "$C_{5-15}$ spiro-cycloalkylene", used alone or in combination, refers to a spiro-cycloalkylene group containing from 5 to 15 carbon atoms (e.g., 7-15, 5-11, 5-10, and 7-9 carbon atoms). Representative examples of "$C_{5-15}$ spiro-cycloalkylene" include, but are not limited to, spiro[3.3]heptylene, spiro[2.5]octylene, spiro[3.5]nonylene, spiro[4.4]nonylene, spiro[4.5]decylene, or spiro [5.5]undecylene. The term "$C_{5-15}$ spiro-cycloalkylene" is optionally substituted with one or more substituents selected from the group consisting of $C_{1-3}$ alkyl, $C_{3-6}$ cycloalkyl, hydroxy, amino, mercapto, halogen, $C_{1-3}$ alkoxy, $C_{1-3}$ alkylamino, halogenated $C_{1-3}$ alkyl, amino-substituted $C_{1-3}$ alkylene, $C_{1-3}$ alkyl-NHC(O)-, $C_{1-3}$ alkyl-C(O)NH-, cyano, or any combination thereof.

As used herein, the term "$C_{x-y}$ bridged cycloalkylene" (x and y each being an integer), used alone or in combination, refers to a bridged cycloalkylene group containing from x to y carbon atoms. As used herein, the term "$C_{5-15}$ bridged cycloalkylene", used alone or in combination, refers to a bridged cycloalkylene group containing from 5 to 15 carbon atoms (e.g., 7-15, 5-11, 5-10, and 7-9 carbon atoms). Representative examples of "$C_{5-15}$ bridged cycloalkylene"

include, but are not limited to, adamantanylene, noradamantanylene, bornylene, bicyclo[2.2.1]heptanylene, 2-oxobicyclo [2.2.1]heptanylene, and bicyclo[2.2.1]heptentylene. The "$C_{5-15}$ bridged cycloalkyl" is optionally substituted with one or more substituents selected from the group consisting of $C_{1-3}$ alkyl, $C_{3-6}$ cycloalkyl, hydroxy, amino, mercapto, halogen, $C_{1-3}$ alkoxy, $C_{1-3}$ alkylamino, halogenated $C_{1-3}$ alkyl, amino-substituted $C_{1-3}$ alkylene, $C_{1-3}$ alkyl-NHC(O)-, $C_{1-3}$ alkyl-C(O) NH-, cyano, or any combination thereof.

As used herein, the term "heterocyclyl" or "heterocyclic group", used alone or in combination, refers to a 3- to 30-membered (optionally 4- to 30-membered, 3- to 20-membered, 4- to 20-membered, or 4- to 15-membered) saturated or partially unsaturated (i.e., containing one or more double bonds, but not having a fully conjugated $\pi$-electron system) monocyclic, bicyclic, tricyclic or polycyclic cyclic hydrocarbon group containing one or more (e.g., from 1 to 5, or from 1 to 4, from 1 to 3, from 1 to 2, or 1) heteroatoms independently selected from sulfur, oxygen, and nitrogen. Representative examples of the monocyclic heterocyclyl include, but are not limited to, azetidinyl, oxetanyl, pyrrolidinyl, imidazolidinyl, pyrazolidyl, tetrahydrofuranyl, tetrahydropyranyl, tetrahydrothienyl, tetrahydrothiopyranyl, oxazolidinyl, thiazolidinyl, piperidinyl, piperazinyl, morpholinyl, thiomorpholinyl, dioxacyclohexyl, azacycloheptyl, azacyclooctyl, diazacycloheptanyl (e.g., 1,4-diazacycloheptan-1-yl) and diazacyclooctyl. Bicyclic, tricyclic and polycyclic heterocyclyl groups include bridged heterocyclyl, fused heterocyclyl and spiro-heterocyclyl groups, representative examples of which include, but not limited to, 6-azabicyclo[3.1.1]heptan-3-yl, 2,5-diazabicyclo[2.2.1]heptan-2-yl, 3,6-diazabicyclo[3.1.1]heptan-3-yl, 3-azabicyclo[3.2.1]octan-8-yl, 3,8-diazabicyclo[3.2.1]octan-8-yl, 3,8-diazabicyclo[3.2.1]octan-3-yl, 2,5-diazabicyclo[2.2.2]octan-2-yl, and azaspirocycloalkyl (e.g., 3-azaspiro[5.5]undecan-3-yl). The heterocyclyl may be unsubstituted or substituted as explicitly defined (e.g., mono-, di-, tri-, or poly-substituted) by a substituent(s) optionally selected from the group consisting of deuterium, hydroxy, amino, mercapto, nitro, halogen, cyano, optionally deuterated $C_{1-6}$ alkyl, halogenated $C_{1-6}$ alkyl, optionally deuterated $C_{3-6}$ cycloalkyl, optionally deuterated $C_{1-6}$ alkoxy, optionally deuterated $C_{1-6}$ alkyl-NH-, $NH_2$-$C_{1-6}$ alkylene, optionally deuterated $C_{1-6}$ alkyl-NHC(O)-, optionally deuterated $C_{1-6}$ alkyl-C(O)NH- and any combination thereof.

As used herein, the term "heterocyclylene", used alone or in combination, refers to a 3- to 30-membered (optionally 4- to 30-membered, 3- to 20-membered, 4- to 20-membered, or 4- to 15-membered) saturated or partially unsaturated (i.e., containing one or more double bonds, but not having a fully conjugated $\pi$-electron system) monocyclic, bicyclic, tricyclic or polycyclic bivalent cyclic hydrocarbon group containing one or more (e.g., from 1 to 5, or from 1 to 4, from 1 to 3, from 1 to 2, or 1) heteroatoms independently selected from sulfur, oxygen, and nitrogen. Representative examples of the monocyclic heterocyclylene include, but are not limited to, azetidinylene, oxetanylene, pyrrolidinylene, imidazolidinylene, pyrazolidylene, tetrahydrofuranylene, tetrahydropyranylene, tetrahydrothienylene, tetrahydrothiopyranylene, oxazolidinylene, thiazolidinylene, piperidinylene, piperazinylene, morpholinylene, thiomorpholinylene, dioxacyclohexylene, and diazacycloheptanylene (e.g., 1,4-diazacycloheptanylene, 4,5-diazacycloheptanylene, 1,3-diazacycloheptanylene). Bicyclic, tricyclic and polycyclic heterocyclylene groups include bridged heterocyclylene, fused heterocyclylene and spiro-heterocyclylene groups, representative examples of which include, but not limited to, 6-azabicyclo [3.1.1]heptanylene, 2,5-diazabicyclo[2.2.1]heptanylene, 3,6-diazabicyclo[3.1.1]heptanylene, 3-azabicyclo[3.2.1]octanylene, 3,8-diazabicyclo[3.2.1]octanylene, 3,8-diazabicyclo[3.2.1]octanylene, 2,5-diazabicyclo[2.2.2]octanylene, and azaspirocycloalkylene (e.g., 3-azaspiro[5.5]undecanylene). The heterocyclylene may be unsubstituted or substituted as explicitly defined (e.g., mono-, di-, tri-, or poly-substituted) by a substituent(s) optionally selected from the group consisting of deuterium, hydroxy, amino, mercapto, nitro, halogen, cyano, optionally deuterated $C_{1-6}$ alkyl, halogenated $C_{1-6}$ alkyl, optionally deuterated $C_{3-6}$ cycloalkyl, optionally deuterated $C_{1-6}$ alkoxy, optionally deuterated $C_{1-6}$ alkyl-NH-, $NH_2$-$C_{1-6}$ alkylene, optionally deuterated $C_{1-6}$ alkyl-NHC(O)-, optionally deuterated $C_{1-6}$ alkyl-C(O)NH- and any combination thereof.

As used herein, the term "alkynylene", used alone or in combination, refers to a linear or branched divalent hydrocarbon group containing 2 to 8 (e.g., 2 to 6, 2 to 5, 2 to 4, preferably 2) carbon atoms with one or more (e.g., 1 to 3, 1 to 2, or 1) carbon-carbon triple bonds. Examples of alkynylene include $C_{2-8}$ alkynylene, $C_{2-6}$ alkynylene or $C_{2-4}$ alkynylene, with representative examples comprising, but not limited to, ethynylene, 1-propynylene, 1-butynylene, and 1,3-diynylene.

As used herein, the term "alkynyl", used alone or in combination, refers to a linear or branched monovalent hydrocarbon group containing from 2 to 8 (e.g., from 2 to 6, from 2 to 5, from 2 to 4, or preferably 2) carbon atoms and having one or more (e.g., from 1 to 3, from 1 to 2, or 1) carbon-carbon triple bonds. Examples of alkynyl include, but are not limited to, ethynyl, 1-propynyl, 1-butynyl, and 1,3-diynyl.

As used herein, the term "alkenylene", used alone or in combination, refers to a linear or branched divalent hydrocarbon group containing from 2 to 8 (e.g., from 2 to 6, from 2 to 5, from 2 to 4, from 2 to 3, or 2) carbon atoms and having one or more (e.g., from 1 to 3, from 1 to 2, or 1) carbon-carbon double bonds. Examples of alkenylene groups include $C_{2-8}$ alkenylene, $C_{2-6}$ alkenylene or $C_{2-4}$ alkenylene, with representative examples comprising, but not limited to, vinylene (e.g., -CH=CH-), 1-propenylene, allylidene, 1-butenylene, 2-butenylene, 3-butenylene, isobutenylene, pentenylene, n-pent-2,4-dienylene, 1-methyl-but-1-enylene, 2-methyl-but-1-enylene, 3-methyl-but-1-enylene, 1-methyl-but-2-enylene, 2-methyl-but-2-enylene, 3-methyl-but-2-enylene, 1-methyl-but-3-enylene, 2-methyl-but-3-enylene, 3-methyl-but-3-enylene, and hexenylene.

As used herein, the term "alkenyl", used alone or in combination, refers to a linear or branched monovalent hydrocarbon group containing from 2 to 8 (e.g., from 2 to 6, from 2 to 5, from 2 to 4, from 2 to 3, or 2) carbon atoms and having one or more (e.g., from 1 to 3, from 1 to 2, or 1) carbon-carbon double bonds. Examples of $C_{2-6}$ alkenyl group include, but are not limited to, vinyl (e.g., $CH_2$=CH-), 1-propenyl, allyl, 1-butenyl, 2-butenyl, 3-butenyl, isobutenyl, pentenyl, n-pent-2,4-dienyl, 1-methyl-but-1-enyl, 2-methyl-but-1-enyl, 3-methyl-but-1-enyl, 1-methyl-but-2-ylene, 2-methyl-but-2-ylene, 3-methyl-but-2-ylene, 1-methyl-but-3-enyl, 2-methyl-but-3-enyl, 3-methyl-but-3-enyl, and hexenyl.

As used herein, "bornylane" or "bornane" (also known as 1,7,7-trimethylbicyclo[2.2.1]heptane; camphane; bornylane) has a definition known to those skilled in the art. As used herein, "camphanyl" or "bornyl" refers to a monovalent group of bornane, i.e., the group remaining after any one of the hydrogens in bornane is removed. Representative examples of "bornyl" include, but are not limited to, 1,7,7-trimethylbicyclo[2.2.1]heptan-2-yl, 1,7,7-trimethylbicyclo[2.2.1]heptan-3-yl, 1,7,7-trimethylbicyclo[2.2.1]heptan-4-yl, 1,7,7-trimethylbicyclo[2.2.1]heptan-5-yl, 1,7,7-trimethylbicyclo[2.2.1]heptan-6-yl,

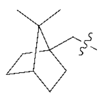 or 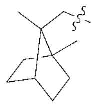 .

As used herein, "bicyclo[2.2.1]heptane" also known as "norbornane", has a definition known to those skilled in the art. As used herein, "bicyclo[2.2.1]heptyl" or "norbornyl" refers to a monovalent group of bicyclo[2.2.1]heptane, i.e., the group remaining after any hydrogen in bicyclo[2.2.1]heptane is removed. Representative examples of "bicyclo[2.2.1]heptyl" include, but are not limited to, bicyclo[2.2.1]heptan-2-yl, bicyclo[2.2.1]heptan-3-yl, bicyclo[2.2.1]heptan-4-yl, bicyclo[2.2.1]heptan-5-yl, or bicyclo[2.2.1]heptan-6-yl.

As used herein, the term "bicyclo[2.2.1]heptene" has a definition known to those skilled in the art. As used herein, "bicyclo[2.2.1]heptenyl" refers to a monovalent group of bicyclo[2.2.1]heptene, i.e., the group remaining after any hydrogen in bicyclo[2.2.1]heptene is removed. Representative examples of "bicyclo[2.2.1]heptenyl" include, but are not limited to, bicyclo[2.2.1]hept-5-en-2-yl, bicyclo[2.2.1]hept-5-en-3-yl, or bicyclo[2.2.1]hept-5-en-7-yl.

As used herein, "adamantane" (also known as tricyclo[3.3.1.1$^{3,7}$]decane) has a definition known to those skilled in the art, and its structural formula is e.g., as follows:

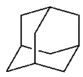 .

As used herein, "adamantanyl" refers to a monovalent group of adamantane, that is, the group remaining after any hydrogen in adamantane is removed. Representative examples of "adamantanyl" include, but are not limited to, 1-adamantanyl, 2-adamantanyl, 3-adamantanyl, 4-adamantanyl, 5-adamantanyl, 6-adamantanyl, 7-adamantanyl, 8-adamantanyl, 9-adamantanyl, or 10-adamantanyl.

As used herein, "noradamantane" (also known as octahydro-2,5-methanopentalen) has a definition known to those skilled in the art, and its structural formula is e.g., as follows:

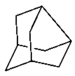 or 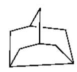 .

As used herein, "noradamantanyl" refers to a monovalent group of noradamantane, that is, the group remaining after any hydrogen in noradamantane is removed. Representative examples of "noradamantanyl" include, but are not limited to, 1-noradamantanyl, 2-noradamantanyl, 3-noradamantanyl, 4-noradamantanyl, 5-noradamantanyl, 6-noradamantanyl, 7-noradamantanyl, 8-noradamantanyl or 9-noradamantanyl.

As used herein, the term "adamantanamine" carries its conventional meaning as understood by persons skilled in the art, referring to adamantane bearing an amino substituent, wherein the amino group may replace any hydrogen atom on the adamantane carbon framework. One embodiment of "adamantanamine" may be adamantan-1-amine (with the English chemical name adamantan-1-amine or Tricyclo[3.3.1.1$^{3,7}$]decan-1-amine; CAS: 768-94-5), having the following structural formula:

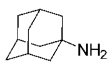 .

Salts or pharmaceutically acceptable salts, enantiomers, stereoisomers, solvates, polymorphs of the compounds of Formula (I), Formula (I-1) or Formula (I-2) of the present disclosure are also encompassed within the scope of the present disclosure.

In all embodiments of the present disclosure, the salts or pharmaceutically acceptable salts of the compounds of Formula (I), Formula (I-1) or Formula (I-2) refer to non-toxic inorganic or organic acid and/or base addition salts. Examples include: hydrochlorides, sulfates, citrates, maleates, sulfonates, citrates, lactates, tartrates, fumarates, phosphates, dihydrophosphates, pyrophosphates, metaphosphates, oxalates, malonates, benzoates, mandelates, succinates, trifluoroacetates, hydroxyacetates, or *p*-toluenesulfonates, etc

"Pharmaceutically acceptable carrier" refers to a pharmaceutically acceptable material, such as a filler, stabilizer, dispersant, suspending agent, diluent, excipient, thickener, solvent, or encapsulating material, with which the useful compounds according to the present disclosure are carried or transported into or administered to a patient so that they can perform their intended function. Generally, such constructs are carried or transported from one organ or part of the body to another organ or part of the body. The carrier is compatible with the other ingredients of the formulation, including the compounds useful in the present disclosure, and is not harmful to the patient, and the carrier must be "acceptable". Some examples of materials that can be used as pharmaceutically acceptable carriers include sugars such as lactose, glucose, and sucrose; starches such as corn starch and potato starch; cellulose and its derivatives such as sodium carboxymethyl cellulose, ethyl cellulose and cellulose acetate; powdered tragacanth; malt; gelatin; talc; excipients such as cocoa butter and suppository wax; oils such as peanut oil, cottonseed oil, safflower oil, sesame oil, olive oil, corn oil and soybean oil; glycols such as propylene glycol; polyols such as glycerol, sorbitol, mannitol, and polyethylene glycol; esters such as ethyl oleate and ethyl laurate; agar; buffers such as magnesium hydroxide and aluminum hydroxide; surfactant phosphate buffer solution; and other common non-toxic compatible substances used in pharmaceutical formulations.

As used herein, the term "room temperature" refers to the ambient temperature, such as 20-30°C.

As used herein, "stereoisomer" refers to a compound with the same chemical structural formula, but a different arrangement of atoms or groups in space. Stereoisomers include enantiomers, diastereomers, conformational isomers (rotational isomers), geometric isomers (cis/trans isomers), atropisomers, and so on.

As used herein, the term "solvate" refers to an association or complex formed by the interaction between one or more solvent molecules and compounds of the present invention. Examples of solvents include water, isopropanol, ethanol, methanol, DMSO, ethyl acetate, acetic acid and ethanolamine. The term "hydrate" means that a complex formed with water.

As used herein, the term "chiral" refers to a molecule that is nonsuperimposable on its mirror image; whereas "achiral" refers to a molecule that can be superimposed on its mirror image.

As used herein, the term "enantiomers" refers to two isomers of a compound that are nonsuperimposable mirror images.

As used herein, the term "diastereomers" refers to stereoisomers that have two or more chiral centers but are not mirror images of each other. The diastereomers have different physical properties, such as melting point, boiling point, spectral properties and reactivity. The diastereomeric mixture can be separated by high-resolution analytical operations such as electrophoresis and chromatography, such as HPLC.

As used herein, the term "C(O)" or "C(=O)" or "C=O", used alone or in combination, refers to carbonyl.

As used herein, "p-menthane" has the definitions known to those skilled in the art, and its structural formula is e.g., as follows:

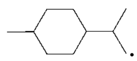

As used herein, "p-menthanyl" refers to a monovalent group of p-menthane, that is, the group remaining after any hydrogen in p-menthane is removed. Representative examples of "p-menthanyl" include, but are not limited to,

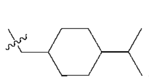, 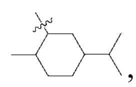,

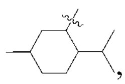, 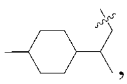, or 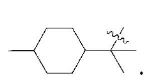.

As used herein, "m-menthane" has the definitions known to those skilled in the art, and its structural formula is e.g., as follows:

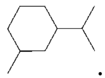.

As used herein, "m-menthanyl" refers to a monovalent group of m-menthane, that is, the group remaining after any hydrogen in m-menthane is removed. Representative examples of "m-menthanyl" include, but are not limited to,

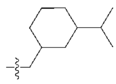, 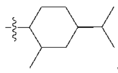, 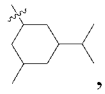,

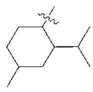, 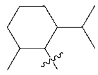, 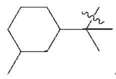, 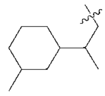, or 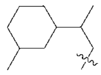.

As used herein, "Quinuclidine" (also known as 1-azabicyclo[2.2.2]octane) has the definitions known to those skilled in the art, and its structural formula is e.g., as follows:

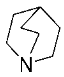.

As used herein, "quinuclidinyl" refers to a monovalent group of Quinuclidine, that is, the group remaining after any hydrogen in Quinuclidine is removed. Representative examples of "quinuclidinyl" include, but are not limited to,

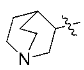, 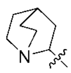, 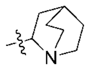, 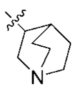, 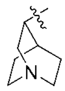, 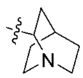, or 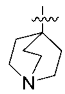.

## Examples

In the following description, numerous specific details are set forth in order to provide a thorough understanding of the present disclosure. The present disclosure may be practiced without some or all of these specific details. In other cases, well-known process operations have not been described in detail in order not to unnecessarily obscure the present disclosure. Although the present disclosure will be described in conjunction with specific embodiments, it should be understood that this is not intended to limit the present disclosure to these embodiments.

The following abbreviations are used throughout the specification and examples:

| | |
|---|---|
| AcOH | acetic acid |
| Boc | t-Butyloxy carbonyl |
| BINAP | 2,2'-Bis(diphenylphosphino)-1,1'-binaphthalene |
| Con. | Concentration |
| DCM | dichloromethane |
| DIAD | Diisopropyl azodicarboxylate |
| DIEA | N, N-diisopropylethylamine |
| DMAP | 4-dimethylaminopyridine |
| DMF | N,N-dimethylformamide |
| DMSO | dimethyl sulfoxide |
| DIPEA | N, N-diisopropylethylamine |
| EA | Ethyl acetate |
| EDCI | 1-(3-dimethylaminopropyl)-3-ethylcarbodiimide hydrochloride |
| ESI | electrospray ionization |
| equiv | equivalent |
| EtOH | ethanol |
| EtOAc | Ethyl acetate |
| HATU | 2-(7-azabenzotriazol-1-yl)-N,N,N',N'-tetramethyluronium hexafluorophosphate |
| HPLC | high performance liquid chromatography |

| | |
|---|---|
| HRMS | high resolution mass spectrometry |
| LC-MS | liquid chromatography-mass spectrometry |
| LRMS | low resolution mass spectrometry |
| LC | liquid chromatography |
| Me | methyl |
| MeCN | acetonitrile |
| MeOH | methanol |
| MS | mass spectrometry |
| MsO- | methanesulfonyloxy |
| $^1$H NMR | Proton nuclear magnetic resonance |
| HFIP | hexafluoroisopropanol |
| MeO- | methoxy |
| NMP | 1-methyl-2-pyrrolidinone |
| rt | room temperature |
| tBu | tert-butyl |
| TEA | triethylamine |
| TFA | trifluoroacetic acid |
| $T_f$O- | Trifluoromethanesulfonyloxy |
| TLC | thin layer chromatography |
| TMS | tetramethylsilane |
| TsO- | 4-methylbenzenesulfonyloxy |
| Xantphos | 4,5-bis(diphenylphosphino)-9,9-dimethylxanthene |
| X-Phos | 2-dicyclohexylphosphino-2',4',6'-triisopropylbiphenyl |

In the present disclosure, the $^1$H NMR spectrum was recorded on a Bruker-500MHz nuclear magnetic resonance instrument, by using, as a solvent, $CD_3OD$ ($\delta$ = 3.31 ppm) containing 0.1% TMS (as an internal standard); or using, as a solvent, $CDCl_3$ ($\delta$ = 7.26 ppm) containing 0.1% TMS (as an internal standard); or using, as a solvent, DMSO-$d_6$ ($\delta$ = 2.50 ppm) containing 0.03% TMS (as an internal standard). LRMS spectrum was recorded on an AB Triple 4600 mass spectrometer; HPLC preparation was measured on a SHIMADZU LC-20AP type instrument, and HPLC purity was measured on a SHIMADZU LC-30AP or Waters 1525 type instrument. Unless otherwise specified, all reactions were performed in the air atmosphere. The reactions were monitored via TLC or LC-MS.

Solvents and reagents are processed as follows:
the solvents used in the reactions such as DCM, DMF, anhydrous EtOH, and anhydrous MeOH were all purchased from Sinopharm Group; HPLC preparation uses preparation grade $CH_3CN$ and deionized water; Other reaction substrates, reagents and drugs may be commercially available without special instructions, or may be synthesized using or according to methods known in the art.

**General synthesis methods**

Compounds and/or pharmaceutically acceptable salts thereof of the present disclosure can be synthesized using commercially available raw materials by synthetic techniques known in the art. The synthetic schemes described below illustrate the preparation of most compounds. The starting materials or reagents used in each scheme can be purchased from commercial sources or prepared by methods known to those skilled in the art. One skilled in the art can prepare the salts, racemates, enantiomers, phosphates, sulfates, hydrochlorides and prodrug forms of the compounds of Formula (I) of the present disclosure according to routine techniques in the art.

Scheme 1:

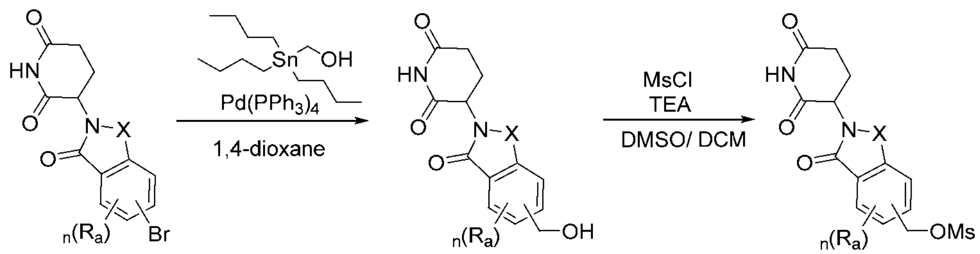


1-1 1-2 1-3

Scheme 1

In Scheme 1, the Br substituent may be positioned at the 4-, 5-, 6-, or 7-position of the benzene ring of isoindolinone, with the resulting hydroxymethyl group consequently occupying the corresponding 4-, 5-, 6-, or 7-position. $(R_a)_n$ indicates that the benzene ring is substituted by n $R_a$ groups, where each $R_a$ may be identical or different and independently represents deuterium, halogen, hydroxy, mercapto, nitro, amino, cyano, optionally deuterated $C_{1-6}$ alkyl, optionally deuterated $C_{1-6}$ alkoxy, halogenated $C_{1-6}$ alkoxy, or halogenated $C_{1-6}$ alkyl; n is an integer of 0, 1, 2, or 3. X is as defined in the compound of Formula (I) of the present disclosure and its various sub-embodiments.

The coupling reaction in Step 1 of Scheme 1 may employ conventional techniques and methods well-known to those skilled in the art. For example, the coupling reaction may be conducted at 40°C to 100°C in the presence of tetrakis(triphenylphosphine)palladium and 1,4-dioxane (or DMF). The esterification reaction in Step 2 of Scheme 1 may also utilize standard techniques and methods familiar to those skilled in the art. Alternatively, depending on requirements, the reaction substrate MsCl in Step 2 may be replaced with TsCl or NsCl, wherein compound 1-2 undergoes esterification with TsCl or NsCl to yield the corresponding target compounds bearing OTs or ONs groups.

For illustration, Scheme 1 may be executed as follows:

Step 1: to a solution of the brominated substrate 1-1 (1.0 eq.) and (tributylstannyl)methanol (1.5 eq.) in DMF was added tetrakis(triphenylphosphine)palladium (0.05 eq.). Under an $N_2$ atmosphere, the reaction solution was heated to 100°C and stirred for 48 hours. After monitoring the reaction via TLC and confirming its completion, the reaction solution was cooled to 60°C, and filtered while still hot to rapidly remove black solids. The filtrate was then concentrated under reduced pressure. To the residue was added dichloromethane, and the resulting mixture was filtered to yield the solid intermediate compound 1-2.

Step 2: to a solution of the solid intermediate compound 1-2 (1.0 eq.) in dimethyl sulfoxide/dichloromethane (1/9) were added sequentially triethylamine (30. eq.) and methanesulfonyl chloride (1.5 eq.). The reaction solution was stirred at room temperature for 1 hour. After monitoring the reaction via TLC and confirming its completion, water was added to the reaction solution. The resulting mixture was filtered, and the filter cake was washed with water and dried to obtain the target compound 1-3.

Scheme 2:

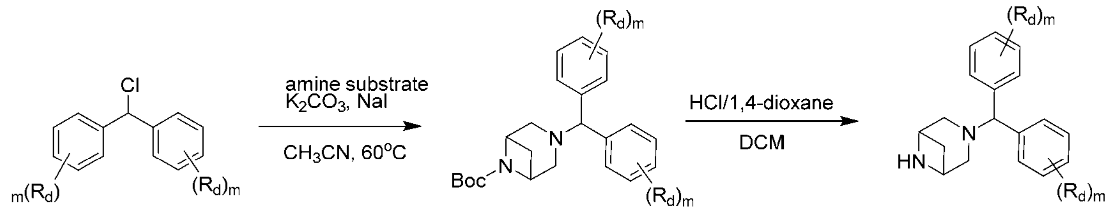


Scheme 2

In Scheme 2, $(R_d)_m$ indicates that the benzene ring is substituted by m $R_d$ groups, where each $R_d$ may be identical or different and independently represents deuterium, hydroxy, amino, mercapto, halogen, cyano, optionally deuterated $C_{1-6}$ alkyl, optionally deuterated $C_{3-6}$ cycloalkyl, optionally deuterated $C_{1-6}$ alkoxy, halogenated $C_{1-6}$ alkyl, halogenated $C_{1-6}$ alkoxy or $-C_{1-6}$ alkylene-$NH_2$; and m represents an integer of 0 to 10.

The amine alkylation reaction in Step 1 of Scheme 2 may employ conventional techniques and methods well-known to those skilled in the art. For example, the reaction may proceed in the presence of N,N-diisopropylethylamine (DIEA) and sodium iodide, or triethylamine and sodium iodide, at temperatures ranging from room temperature to 80°C. The Boc deprotection reaction in Step 2 of Scheme 2 may also employ standard techniques and methods familiar to those skilled in the art, such as using a solution of HCl in 1,4-dioxane.

For illustration, Scheme 2 may be performed as follows:

Step 1: To a solution of the halide substrate (1.0 equiv.) and amine substrate (1.5 equiv.) in acetonitrile were added potassium carbonate (3.0 equiv.) and sodium iodide (1.0 equiv.). The reaction mixture was heated to 60°C and stirred for 18 hours. After monitoring the reaction via TLC and confirming its completion, the mixture was cooled to room temperature, filtered, and the filtrate was concentrated under reduced pressure. The residue was washed with water and extracted with dichloromethane. The organic phases were combined, dried over anhydrous sodium sulfate, filtered, and the filtrate was concentrated under reduced pressure. The residue was purified by column chromatography to obtain the solid intermediate.

Step 2: to a solution of the solid intermediate from Step 1 (1.0 equiv.) in dichloromethane was added a solution of HCl in 1,4-dioxane. The reaction mixture was stirred at room temperature for 1 hour. After monitoring the reaction via TLC and confirming its completion, the reaction mixture was concentrated under reduced pressure to obtain the target product.

Scheme 3:

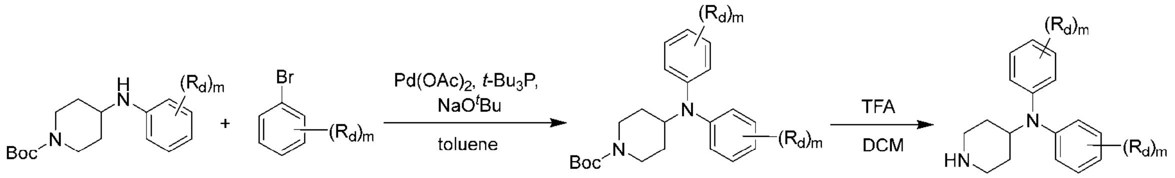


Scheme 3

In Scheme 3, $(R_d)_m$ indicates that the benzene ring is substituted by m $R_d$ groups, where each $R_d$ may be identical or different and independently represents deuterium, hydroxy, amino, mercapto, halogen, cyano, optionally deuterated $C_{1-6}$ alkyl, optionally deuterated $C_{3-6}$ cycloalkyl, optionally deuterated $C_{1-6}$ alkoxy, halogenated $C_{1-6}$ alkyl, halogenated $C_{1-6}$ alkoxy or $-C_{1-6}$ alkylene-$NH_2$; and m represents an integer of 0 to 10.

The coupling reaction in Step 1 of Scheme 3 may be performed using conventional techniques and methods well-known to those skilled in the art, such as in the presence of $Pd_2(dba)_3$ and X-Phos, or $Pd(OAc)_2$ and Xantphos. The Boc deprotection reaction in Step 2 of Scheme 3 may also employ standard techniques and methods familiar to those skilled in the art, for example using a solution of HCl in 1,4-dioxane.

For illustration, Scheme 3 may be executed as follows:

Step 1: to a solution of the halide substrate (1.0 equiv.) and amine substrate (1.0 equiv.) in anhydrous toluene were sequentially added $Pd(OAc)_2$ (0.1 eq.), t-$Bu_3P$ (0.5 eq.), and sodium tert-butoxide (2.25 equiv.). Under an $N_2$ atmosphere, the reaction mixture was heated to 100°C and stirred for 18 hours. After monitoring the reaction via TLC and confirming its completion, the reaction solution was cooled to room temperature, washed with water, and extracted with ethyl acetate. The organic phases were combined, dried over anhydrous $Na_2SO_4$, filtered, and the filtrate was concentrated under reduced pressure. The residue was purified by column chromatography to afford the solid intermediate.

Step 2: to a solution of the solid intermediate from Step 1 (1.0 equiv.) in dichloromethane was added trifluoroacetic acid (2 mL). The reaction mixture was stirred at room temperature for 1 hour. After monitoring the reaction via TLC and confirming its completion, the reaction solution was concentrated under reduced pressure to obtain the target product.

Scheme 4:

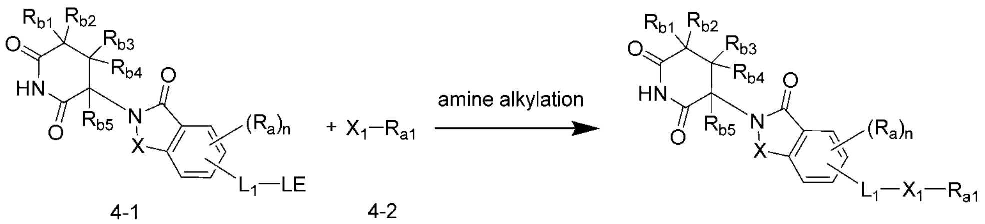


4-1

4-2

Scheme 4

In Scheme 4, $Rb_1$, $Rb_2$, $Rb_3$, $Rb_4$, $Rb_5$, $(R_a)_n$, X, $L_1$, $X_1$, and $R_{a1}$ are as defined in the compound of Formula (I) of the present disclosure and its various sub-embodiments. LE represents Cl, Br, I, OMs, OTs or ONs, and $X_1$ represents optionally substituted nitrogen-containing heterocyclyl, optionally substituted aryl-$NH_2$, optionally substituted heteroaryl-$NH_2$, or optionally substituted cycloalkyl-$NH_2$.

The amine alkylation reaction in Scheme 4 may be performed using conventional techniques well-known to those skilled in the art. For example, the amine alkylation may be conducted in the presence of DIEA and sodium iodide, or triethylamine and sodium iodide, at temperatures ranging from room temperature to 80°C (e.g., 40-60°C, 40-50°C or 50-60°C). The molar ratio of substrate 1 to substrate 2 may be, for example, 1:1.1-2, 1:1.1-1.5, 1:1.1-1.2, or 1:1.2-1.3.

For illustration, Scheme 4 may be specifically performed as follows:

To a solution of the mesylate substrate 4-1 (1.3 equiv.) and amine substrate 4-2 (1.0 equiv.) in anhydrous DMF (3 mL) were added triethylamine (3.0 equiv.) and sodium iodide (1.0 equiv.). The reaction mixture was stirred at 50°C for 2 hours. After monitoring the reaction via LCMS and confirming its completion, the reaction mixture was filtered, and the filtrate was purified by HPLC to obtain the target compound.

Scheme 5:

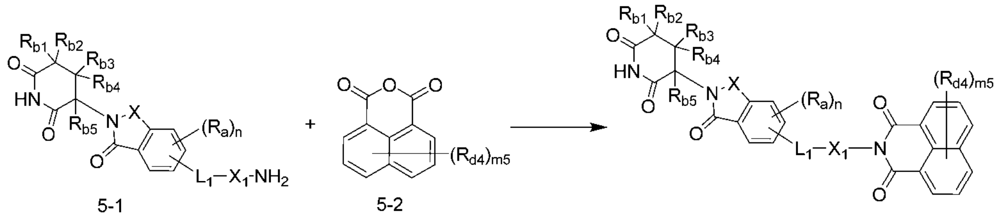

Scheme 5

In Scheme 5, $R_{b1}$, $R_{b2}$, $R_{b3}$, $Rb_4$, $Rb_5$, $(R_a)_n$, X, $L_1$, and $(R_{d4})_{m5}$ are as defined in the compound of Formula (I) of the present disclosure and its various sub-embodiments. $X_1$ represents optionally substituted heterocyclyl (e.g., nitrogen-containing heterocyclyl), optionally substituted aryl, optionally substituted heteroaryl or optionally substituted cycloalkyl, or $X_1$ represents a bond.

Scheme 5 may be specifically performed as follows:

To a solution of substrate 5-1 (1.0 equiv.) and the corresponding anhydride substrate 5-2 (1.3 equiv.) in anhydrous NMP (3 mL) was added triethylamine (3.0 equiv.). The reaction mixture was stirred at 80°C for 16 hours. After monitoring the reaction via LCMS and confirming its completion, the reaction mixture was filtered, and the filtrate was purified by HPLC to obtain the target compound.

Scheme 6:

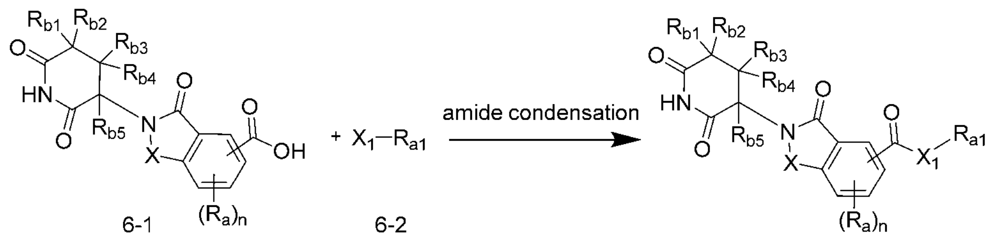


Scheme 6

In Scheme 6, $R_{b1}$, $Rb_2$, $R_{b3}$, $Rb_4$, $R_{b5}$, $(R_a)_n$, X, $X_1$, and $R_{a1}$ are as defined in the compound of Formula (I) of the present disclosure and its various sub-embodiments. $X_1$ represents optionally substituted nitrogen-containing heterocyclyl, optionally substituted aryl-$NH_2$, optionally substituted heteroaryl-$NH_2$ or optionally substituted cycloalkyl-$NH_2$.

Scheme 6 may be specifically performed as follows:

To a solution of carboxylic acid substrate 6-1 (1.1 equiv.) and amine substrate 6-2 (1.0 equiv.) in anhydrous DMF (2 mL) were added triethylamine (3.0 equiv.) and HATU (1.3 equiv.). The reaction mixture was stirred at room temperature for 2 hours. After monitoring the reaction via LCMS and confirming its completion, the reaction mixture was filtered, and the filtrate was purified by HPLC to obtain the target compound.

Scheme 7:

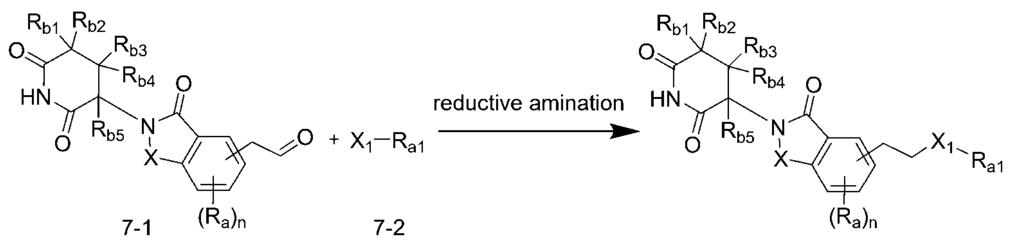


Scheme 7

In Scheme 7, $R_{b1}$, $Rb_2$, $R_{b3}$, $Rb_4$, $R_{b5}$, $(R_a)_n$, X, $X_1$, and $R_{a1}$ are as defined in the compound of Formula (I) of the present disclosure and its various sub-embodiments. $X_1$ represents optionally substituted nitrogen-containing heterocyclyl, optionally substituted aryl-$NH_2$, optionally substituted heteroaryl-$NH_2$ or optionally substituted cycloalkyl-$NH_2$.

Scheme 7 may be specifically performed as follows:

To a solution of the aldehyde substrate 7-1 (1.0 eq.) and the amine substrate 7-2 (1.1 eq.) in anhydrous DMF (2 mL) was added one drop of acetic acid. The reaction solution was stirred at 50 °C for 2 hours, followed by addition of sodium triacetoxyborohydride (2.0 equiv.). The reaction solution was then stirred at room temperature for 18 hours. After

monitoring the reaction via LCMS and confirming its completion, the reaction mixture was quenched with water, filtered, and the filtrate was purified by HPLC to obtain the target compound.

Scheme 8:

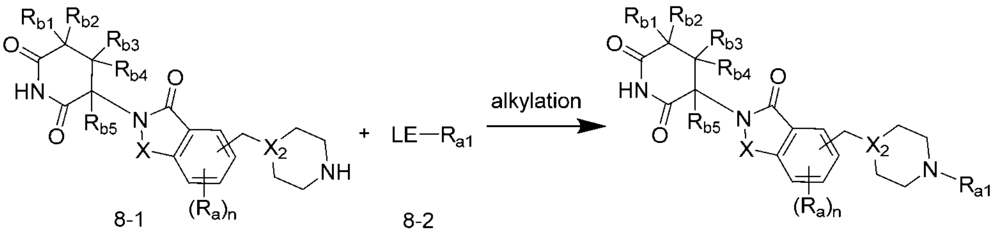


8-1

8-2

Scheme 8

In Scheme 8, $R_{b1}$, $Rb_2$, $Rb_3$, $Rb_4$, $Rb_5$, $(R_a)_n$, X, and $R_{a1}$ are as defined in the compound of Formula (I) of the present disclosure and its various sub-embodiments. LE represents Cl, Br, I, OMs, OTs or ONs, and $X_2$ represents CH or N.

Scheme 8 may be specifically performed as follows:

To a solution of nitrogen-containing heterocycle substrate 8-1 (1.0 equiv.) and halide substrate 8-2 (2.0 equiv.) in anhydrous DMF (2 mL) were added triethylamine (3.0 equiv.) and sodium iodide (1.0 equiv.). The reaction mixture was stirred at room temperature for 18 hours. After monitoring the reaction via LCMS and confirming its completion, the mixture was filtered and the filtrate was purified by HPLC to afford the target compound.

Depending upon the target compounds, the various schemes mentioned above and their reaction substrates, reaction conditions (including reaction dosage, temperature, duration, etc.), work up, etc. can be appropriately modified and adjusted by techniques and methods well known to those skilled in the art to obtain the desired target compounds. The obtained target compounds can be further modified by the substituents and the like to obtain subsequent target compounds through methods well known to those skilled in the art.

## Examples

**Intermediate example** 1: **preparation of (2-(2,6-dioxopiperidin-3-yl)-1-oxoisoindolin-5-yl)methyl methanesulfonate**

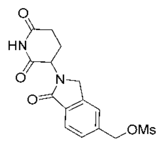

Referring to the method of Scheme 1, the target product (2-(2,6-dioxopiperidin-3-yl)-1-oxoisoindolin-5-yl)methyl methanesulfonate was prepared as grayish-white solid (20.0 g, yield 86%). LC/MS (ESI) m/z calcd for $C_{15}H_{17}N_2O_6S^+$ $[M+H]^+$, 353.08 ; found, 353.1.

**Intermediate** example 2: **preparation of (2-(2,6-dioxopiperidin-3-yl)-4-fluoro-1-oxoisoindolin-5-yl)methyl methanesulfonate**

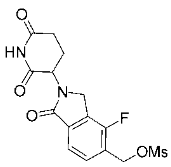

Referring to the method of Scheme 1, the target product (2-(2,6-dioxopiperidin-3-yl)-4-fluoro-1-oxoisoindolin-5-yl)methyl methanesulfonate was prepared as pale yellow solid (1.1 g, yield 86%). LC/MS (ESI) m/z calcd for $C_{15}H_{16}FN_2O_6S^+$ [M+H]+, 371.07 ; found, 371.1.

**Intermediate example 3: preparation of (2-(2,6-dioxopiperidin-3-yl)-6-fluoro-1-oxoisoindolin-5-yl)methyl methanesulfonate**

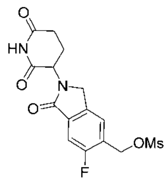

Referring to the method of Scheme 1, the target product (2-(2,6-dioxopiperidin-3-yl)-6-fluoro-1-oxoisoindolin-5-yl)methyl methanesulfonate was prepared as grayish-white solid (3.9 g, yield 78%). LC/MS (ESI) m/z calcd for $C_{15}H_{16}FN_2O_6S^+$ [M+H]+, 371.07 ; found, 371.1.

**Intermediate** example 4: **preparation** of **(2-(2,6-dioxopiperidin-3-yl)-7-fluoro-1-oxoisoindolin-5-yl)methyl methanesulfonate**

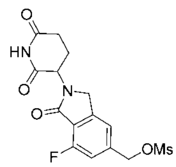

Referring to the method of Scheme 1, the target product (2-(2,6-dioxopiperidin-3-yl)-7-fluoro-1-oxoisoindolin-5-yl)methyl methanesulfonate was prepared as grayish-white solid (6.5 g, yield 82%). LC/MS (ESI) m/z calcd for $C_{15}H_{16}FN_2O_6S^+$ [M+H]+, 371.07 ; found, 371.1.

**Intermediate example** 5: **preparation of (2-(2,6-dioxopiperidin-3-yl)-1-oxoisoindolin-4-yl)methyl methanesulfonate**

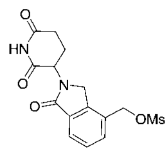

Referring to the method of Scheme 1, the target product (2-(2,6-dioxopiperidin-3-yl)-1-oxoisoindolin-4-yl)methyl methanesulfonate was prepared as grayish-white solid (4.5 g, yield 85%). LC/MS (ESI) m/z calcd for $C_{15}H_{17}N_2O_6S^+$ [M+H]+, 353.08 ; found, 353.1.

**Intermediate example 6: preparation of 3-benzhydryl-3,6-diazabicyclo[3.1.1]heptane**

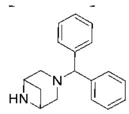

Referring to the method of Scheme 2, the target product 3-benzhydryl-3,6-diazabicyclo[3.1.1]heptane was prepared as grayish-white solid (560 mg, yield 88.%). $^{1}H$ NMR (400 MHz, DMSO) δ 9.18 (s, 1H), 7.71 (s, 1H), 7.52 (d, *J* = 7.2 Hz, 4H), 7.33 (t, *J* = 7.6 Hz, 4H), 7.22 (t, *J* = 7.2 Hz, 2H), 4.67 (s, 1H), 4.22 (s, 2H), 3.05 (d, *J* = 12.0 Hz, 2H), 2.81 (d, *J* = 12.0 Hz, 2H), 2.69 - 2.58 (m, 1H), 2.37 - 2.28 (m, 1H). LCMS (ESI) m/z calcd for $C_{18}H_{21}N_2^+$ [M+H]$^+$, 265.17; found, 265.2.

**Intermediate example 7: preparation of (1S,4S)-2-benzhydryl-2,5-diazabicyclo[2.2.1]heptane**

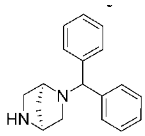

Referring to the method of Scheme 2, the target product (1S,4S)-2-benzhydryl-2,5-diazabicyclo[2.2.1]heptane was prepared as brown solid (312 mg, yield 82%). $^{1}H$ NMR (400 MHz, DMSO) δ 9.02 (brs, 1H), 8.84 (brs, 1H), 7.48 (d, J= 7.6 Hz, 4H), 7.31 (t, J= 7.6 Hz, 4H), 7.21 - 7.18 (m, 2H), 4.76 (s, 1H), 4.17 (s, 1H), 3.46 (s, 1H), 3.36 (s, 1H), 3.01 (brs, 1H), 2.68 - 2.64 (m, 2H), 2.12 (brs, 1H), 1.66 (d, *J* = 10.4 Hz, 1H). LCMS (ESI) m/z calcd for $C_{18}H_{21}N_2^+$ [M+H]$^+$, 265.17; found, 265.5.

**Intermediate example 8: preparation of (IR,4R)-2-benzhydryl-2,5-diazabicyclo[2.2.1]heptane**

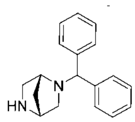

Referring to the method of Scheme 2, the target product (1R,4R)-2-benzhydryl-2,5-diazabicyclo[2.2.1]heptane was prepared as pale yellow solid (180 mg, yield 92%). LCMS (ESI) m/z calcd for $C_{18}H_{21}N_2^+$ [M+H]$^+$, 265.17; found, 265.2.

**Intermediate example** 9: **preparation of 3-benzhydryl-3,8-diazabicyclo[3.2.1]octane**

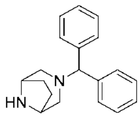

Referring to the method of Scheme 2, the target product 3-benzhydryl-3,8-diazabicyclo[3.2.1]octane was prepared as white solid (133 mg, yield 66 %). $^{1}H$ NMR (400 MHz, CDCl3) δ 7.43(s, 1H), 9.17 (s, 1H), 7.40 - 7.29 (m, 4H), 7.27 - 7.25 (m, 4H), 7.20 - 7.16(m, 2H), 4.43 (s, 1H), 3.81 (s, 1H), 2.75 - 2.72 (m, 2H), 2.51 - 2.48 (m, 2H), 2.20 - 2.11 (m, 1H). LCMS (ESI) m/z calcd for $C_{19}H_{23}N_2^+$ [M+H]$^+$, 279.19; found, 279.4.

**Intermediate example 10: preparation of 8-benzhydryl-3,8-diazabicyclo[3.2.1]octane**

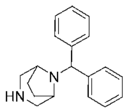

Referring to the method of Scheme 2, the target product 8-benzhydryl-3,8-diazabicyclo[3.2.1]octane was prepared as white solid (80 mg, yield 24%). $^{1}H$ NMR (400 MHz, DMSO) *δ* 8.99 (s, 1H), 8.40 (s, 1H), 7.55 - 7.51 (m, 4H), 7.33 - 7.29 (m, 4H), 7.22 - 7.18 (m, 2H), 4.54 (s, 1H), 3.20 - 2.99 (m, 6H), 2.11 (brs, 2H), 1.81 - 1.79 (m, 2H). LCMS (ESI)

m/z calcd for $C_{19}H_{23}N_2^+$ $[M+H]^+$, 279.19; found, 279.4.

**Intermediate example 11: preparation of (R)-1-benzhydryl-3-methylpiperazine**

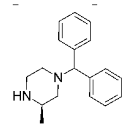

Referring to the method of Scheme 2, the target product (R)-1-benzhydryl-3-methylpiperazine was prepared as pale yellow solid (167 mg, yield 89%). LCMS (ESI) m/z calcd for $C_{18}H_{23}N_2^+$ $[M+H]^+$, 267.19; found, 267.2.

**Intermediate example 12: preparation of 1-benzhydryl-3,5-dimethylpiperazine**

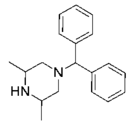

Referring to the method of Scheme 2, the target product 1-benzhydryl-3,5-dimethylpiperazine was prepared as white solid (295 mg, yield 91%). $^1$H NMR (400 MHz, DMSO) δ 9.03 (s, 1H), 8.20 (s, 1H), 7.42 - 7.40 (m, 4H), 7.34 - 7.30 (m, 4H), 7.24 - 7.21 (m, 2H), 4.51 (s, 1H), 3.39 (brs, 2H), 2.85 (d, $J$ = 11.6 Hz, 2H), 1.90 (t, $J$ = 11.6 Hz, 2H), 1.11 (d, $J$= 6.8 Hz, 6H). LCMS (ESI) m/z calcd for $C_{19}H_{25}N_2^+$ $[M+H]^+$, 281.20; found, 281.4.

**Intermediate example 13: preparation of 2-benzhydryl-2,5-diazabicyclo[2.2.2]octane**

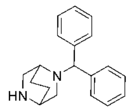

Referring to the method of Scheme 2, the target product 2-benzhydryl-2,5-diazabicyclo[2.2.2]octane was prepared as white solid (198 mg, yield 84%). $^1$H NMR (400 MHz, MeOD) δ 7.57 - 7.54 (m, 4H), 7.34 - 7.30 (m, 1H), 7.25 - 7.21 (m, 2H), 4.97 (s, 1H), 3.78 - 3.64 (m, 1H), 3.61 (brs, 1H), 3.33 (brs, 1H), 3.16 (d, $J$ = 12.4 Hz, 1H), 3.07 (s, 1H), 2.87 (d, $J$ = 13.2 Hz, 1H), 2.39 - 2.25 (m, 1H), 2.05 - 2.01 (m, 1H), 1.77 - 1.64 (m, 1H). LCMS (ESI) m/z calcd for $C_{19}H_{23}N_2^+$ $[M+H]^+$, 279.18; found, 279.2.

**Intermediate example 14: preparation of 1-benzhydryl-2,2-dimethylpiperazine**

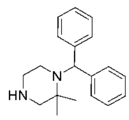

Referring to the method of Scheme 2, the target product 1-benzhydryl-2,2-dimethylpiperazine was prepared as white solid (170 mg, yield 82%). LCMS (ESI) m/z calcd for $C_{19}H_{25}N_2^+$ $[M+H]^+$, 281.20; found, 281.2.

**Intermediate example 15: preparation of 1-(bis(3-fluorophenyl)methyl)piperazine**

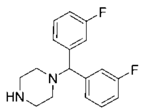

Referring to the method of Scheme 2, the target product 1-(bis(3-fluorophenyl)methyl)piperazine was prepared as white solid (700 mg, yield 97 %). $^1$H NMR (400 MHz, $D_2O$) $\delta$ 7.50 - 7.38 (m, 6H), 7.21 - 7.17 (m, 2H), 5.35 (s, 1H), 3.58 - 3.55 (m, 4H), 3.42 - 3.40 (m, 1H). LCMS (ESI) m/z calcd for $C_{17}H_{19}F_2N_2^+$ [M+H]$^+$, 289.15; found, 289.2.

**Intermediate example 16: preparation of N,N-diphenylpiperidin-4-amine**

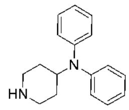

The target compound N,N-diphenylpiperidin-4-amine was prepared according to the method of Scheme 3.

Step 1: To a solution of tert-butyl 4-(phenylamino)piperidine-1-carboxylate (5 g, 18.09 mmol) and bromobenzene (1.91 ml, 18.09 mmol) in anhydrous toluene (50 mL) were added sequentially $Pd(OAc)_2$ (0.41 g, 1.81 mmol), t-$Bu_3P$ (1.83 g, 9.046 mmol), and sodium tert-butoxide (3.91 g, 40.71 mmol). The reaction system was purged with $N_2$ three times, heated to 100°C, and stirred for 18 h. After monitoring the reaction via TLC and confirming its completion, the reaction mixture was cooled to room temperature, washed with water, and extracted with ethyl acetate. The organic phases were combined, dried over anhydrous $Na_2SO_4$, and filtered, and the filtrate was concentrated under reduced pressure. The residue was purified by column chromatography to afford tert-butyl 4-(diphenylamino)piperidine-1-carboxylate as a pale yellow solid (3 g, 47% yield). LCMS (ESI) m/z calcd for $C_{22}H_{29}N_2O_2^+$ [M+H]$^+$, 353.22; found, 353.2.

Step 2: to a solution of tert-butyl 4-(diphenylamino)piperidine-1-carboxylate (2.9 g, 8.23 mmol) in dichloromethane (10 mL) was added trifluoroacetic acid (2 mL). The reaction mixture was stirred at room temperature for 1 h. After monitoring the reaction via TLC and confirming its completion, the reaction mixture was concentrated under reduced pressure to give N,N-diphenylpiperidin-4-amine trifluoroacetate as a grayish-white solid (1.7 g, yield 82 %). $^1$H NMR (400 MHz, DMSO) $\delta$ 8.77 (brs, 1H), 8.16 (brs, 1H), 7.32 - 7.28 (m, 4H), 7.03 - 7.00 (m, 2H), 6.86 - 6.84 (m, 4H), 4.27 (t, *J* = 3.2 Hz, 1H), 3.31 (d, *J* = 12.4 Hz, 2H), 3.14 - 3.10 (m, 2H), 2.05 (d, *J* = 12.0 Hz, 2H), 1.51 - 1.47 (m, 2H). LCMS (ESI) m/z calcd for $C_{17}H_{21}N_2^+$ [M+H]$^+$, 253.17; found, 253.2.

**Example 1: preparation of 3-(5-((4-benzhydrylpiperazin-1-yl)methyl)-1-oxoisoindolin-2-yl)piperidine-2,6-dione (GT-03245)**

Referring to the method of Scheme 4, the target product GT-03245 was prepared as white solid (450 mg, yield 33%). $^1$H NMR (400 MHz, MeOD) $\delta$ 7.79 (d, *J* = 7.8 Hz, 1H), 7.72 (s, 1H), 7.61 (d, *J* = 7.8 Hz, 1H), 7.59 - 7.52 (m, 4H), 7.33 - 7.26 (m, 4H), 7.22 - 7.20 (m, 2H), 5.09 - 5.04 (m, 1H), 4.98 (brs, 1H), 4.56 - 4.35 (m, 4H), 3.53 - 3.41 (m, 4H), 3.17 - 2.99 (m, 4H), 2.87 - 2.75 (m, 1H), 2.73 - 2.63 (m, 1H), 2.46 - 2.35 (m, 1H), 2.14 - 2.01 (m, 1H). LC/MS (ESI) m/z calcd for $C_{31}H_{33}N_4O_3^+$ [M+H]$^+$, 509.25; found, 509.3.

**Example 2: preparation of 3-(5-((4-benzhydrylpiperazin-1-yl)methyl)-4-fluoro-1-oxoisoindolin-2-yl)piperidine-2,6-dione (GT-03466)**

Referring to the method of Scheme 4, the target product GT-03466 was prepared as white solid (40 mg, yield 64%). $^1$H NMR (400 MHz, MeOD) $\delta$ 7.84 - 7.78 (m, 1H), 7.75 (d, *J* = 7.8 Hz, 1H), 7.70 - 7.62 (m, 4H), 7.43 - 7.40 (m, 4H), 7.36 - 7.32 (m, 2H), 5.18 (dd, *J* = 13.2, 5.2 Hz, 1H), 5.06 (brs, 1H), 4.63 (q, *J* = 17.2 Hz, 2H), 4.56 (s, 2H), 3.58 (brs, 4H), 3.18 (brs, 4H), 3.01 - 2.87 (m, 1H), 2.84 - 2.77 (m, 1H), 2.59 - 2.48 (m, 1H), 2.23 - 2.18 (m, 1H). LC/MS (ESI) m/z calcd for $C_{31}H_{32}FN_4O_3^+$ [M+H]$^+$, 527.25; found, 527.3.

**Example 3: preparation of 3-(5-((4-benzhydrylpiperazin-1-yl)methyl)-6-fluoro-1-oxoisoindolin-2-yl)piperidine-2,6-dione (GT-03700)**

Referring to the method of Scheme 4, the target product GT-03700 was prepared as white solid (42 mg, yield 67%). $^1$H NMR (400 MHz, MeOD) $\delta$ 7.90 (d, *J* = 6.0 Hz, 1H), 7.71 (d, *J* = 7.6 Hz, 4H), 7.66 (d, *J* = 8.6 Hz, 1H), 7.44 (t, *J* = 7.6 Hz, 4H), 7.37 (t, *J* = 8.6 Hz, 2H), 5.18 (dd, *J* = 13.2, 5.2 Hz, 2H), 4.68 - 4.48 (m, 4H), 3.65 (s, 4H), 3.29 (s, 4H), 2.98 - 2.89 (m,

1H), 2.86 - 2.73 (m, 1H), 2.58 - 2.38 (m, 1H), 2.29 - 2.08 (m, 1H). LC/MS (ESI) m/z calcd for $C_{31}H_{32}FN_4O_3^+$ [M+H]+, 527.25; found, 527.3.

**Example 4: preparation of 3-(5-((4-benzhydrylpiperazin-1-yl)methyl)-7-fluoro-1-oxoisoindolin-2-yl)piperidine-2,6-dione (GT-03467)**

Referring to the method of Scheme 4, the target product GT-03467 was prepared as white solid (30 mg, yield 48%). $^1H$ NMR (400 MHz, DMSO) δ 11.02 (s, 1H), 7.63-7.56 (m, 1H), 7.54-7.38 (m, 4H), 7.34-7.29 (m, 4H), 7.27-7.20 (m, 2H), 5.12-5.07 (m, 1H), 4.51 (d, *J* = 18.0 Hz, 2H), 4.38 (d, *J* = 18.0 Hz, 2H), 3.21-3.08 (m, 4H), 2.95-2.81 (m, 4H), 2.73-2.62 (m, 2H), 2.42-2.33 (m, 4H), 2.06-1.92 (m, 1H), 1.29-1.23 (m, 1H). LC/MS (ESI) m/z calcd for $C_{31}H_{32}FN_4O_3^+$ [M+H]+, 527.25; found, 527.3.

**Example 5: preparation of 3-(4-((4-benzhydrylpiperazin-1-yl)methyl)-1-oxoisoindolin-2-yl)piperidine-2,6-dione (GT-03646)**

Referring to the method of Scheme 4, the target product GT-03646 was prepared as white solid (25 mg, yield 34%). $^1H$ NMR (400 MHz, DMSO) δ 11.11 (s, 1H), 7.97 (d, *J* = 7.2 Hz, 1H), 7.86 (d, *J* = 7.2 Hz, 1H), 7.74 (brs, 3H), 7.66 (t, *J* = 7.6 Hz, 1H), 7.45-7.42 (m, 5H), 7.36-7.33 (m, 2H), 5.24-5.20 (m, 1H), 4.91 (d, *J* = 17.2 Hz, 1H), 4.57 (d, *J* = 17.2 Hz, 1H), 4.44 (brs, 2H), 3.47-3.38 (m, 4H), 3.17-3.05 (m, 3H), 3.03-2.96 (m, 2H), 2.73-2.62 (m, 1H), 2.46-2.35 (m, 1H), 2.11-2.08 (m, 1H). LC/MS (ESI) m/z calcd for $C_{31}H_{33}N_4O_3^+$ [M+H]+, 509.25; found, 509.3.

**Example 6: preparation of 3-(5-((4-benzhydryl-1,4-diazepan-1-yl)methyl)-1-oxoisoindolin-2-yl)piperidine-2,6-dione (GT-03994)**

Referring to the method of Scheme 4, the target product GT-03994 was prepared as white solid (20 mg, yield 28%). $^1H$ NMR (400 MHz, MeOD) δ 7.81 (d, *J* = 7.8 Hz, 1H), 7.72 (s, 1H), 7.62 - 7.59 (m, 4H), 7.35 - 7.32 (m, 4H), 7.28 - 7.25 (m, 2H), 5.33 (brs, 1H), 5.08 (dd, *J* = 13.2, 5.2 Hz, 1H), 4.58 - 4.36 (m, 4H), 3.77 - 3.64 (m, 2H), 3.53 - 3.39 (m, 4H), 3.28 - 3.24 (m, 2H), 2.87 - 2.77 (m, 1H), 2.76 - 2.63 (m, 1H), 2.47 - 2.36 (m, 1H), 2.28 - 2.16 (m, 2H), 2.14 - 2.00 (m, 1H). LC/MS (ESI) m/z calcd for $C_{32}H_{35}N_4O_3^+$ [M+H]+, 523.27; found, 523.3.

**Example 7: preparation of 3-(5-((4-benzhydryl-1,4-diazepan-1-yl)methyl)-4-fluoro-1-oxoisoindolin-2-yl)piperidine-2,6-dione (GT-04164)**

Referring to the method of Scheme 4, the target product GT-04164 was prepared as white solid (24 mg, yield 39%). $^1H$ NMR (400 MHz, MeOD) δ 7.76 - 7.70 (m, 1H), 7.69 - 7.62 (m, 5H), 7.39 - 7.34 (m, 4H), 7.31 - 7.27 (m, 2H), 5.45 (brs, 1H), 5.08 (dd, *J* = 13.2, 5.2 Hz, 1H), 4.58 - 4.46 (m, 4H), 3.83 (brs, 2H), 3.66 - 3.46 (m, 4H), 3.30 (brs, 2H), 2.87 - 2.78 (m, 1H), 2.75 - 2.63 (m, 1H), 2.49 - 2.37 (m, 1H), 2.28 (brs, 2H), 2.15 - 2.01 (m, 1H). LC/MS (ESI) m/z calcd for $C_{32}H_{34}FN_4O_3^+$ [M+H]+, 541.26; found, 541.3.

**Example 8: preparation of 3-(5-((3-benzhydryl-3,6-diazabicyclo[3.1.1]heptan-6-yl)methyl)-1-oxoisoindolin-2-yl)piperidine-2,6-dione (GT-03569)**

Referring to the method of Scheme 4, the target product GT-03569 was prepared as white solid (12 mg, yield 24%). $^1H$ NMR (400 MHz, MeOD) δ 7.73 - 7.70 (m, 1H), 7.47 (s, 1H), 7.44 - 7.38 (m, 3H), 7.36 - 7.29 (m, 8H), 5.39 (d, *J* = 9.2 Hz, 1H), 5.12 - 5.06 (m, 1H), 4.45 - 4.39 (m, 3H), 3.73 (brs, 2H), 3.48 (brs, 1H), 2.99 - 2.62 (m, 6H), 2.51 - 2.34 (m, 2H), 2.12 - 2.08 (m, 2H). LC/MS (ESI) m/z calcd for $C_{32}H_{33}N_4O_3^+$ [M+H]+, 521.25; found, 521.3.

**Example 9: preparation of 3-(5-((3-benzhydryl-3,6-diazabicyclo[3.1.1]heptan-6-yl)methyl)-4-fluoro-1-oxoisoindolin-2-yl)piperidine-2,6-dione (GT-03572)**

Referring to the method of Scheme 4, the target product GT-03572 was prepared as white solid (10 mg, yield 20%). LC/MS (ESI) m/z calcd for $C_{32}H_{32}FN_4O_3^+$ [M+H]+, 539.25; found, 539.3.

**Example 10: preparation of 3-(5-((3-benzhydryl-3,6-diazabicyclo[3.1.1]heptan-6-yl)methyl)-7-fluoro-1-oxoisoindolin-2-yl)piperidine-2,6-dione (GT-03575)**

Referring to the method of Scheme 4, the target product GT-03575 was prepared as white solid (16 mg, yield 36%). $^1H$ NMR (400 MHz, DMSO-d6) δ 11.02 (s, 1H), 7.68 - 7.63 (m, 4H), 7.49 - 7.36 (m, 6H), 7.34 - 7.31 (m, 1H), 7.24 - 7.20

(m, 1H), 5.74 (s, 1H), 5.13 - 5.08 (m, 1H), 4.67 (s, 1H), 4.48 - 4.31 (m, 2H), 3.78 - 3.66 (m, 2H), 3.07 - 2.85 (m, 3H), 2.79 - 2.73 (m, 1H), 2.64 - 2.60 (m, 2H), 2.44 - 2.30 (m, 3H), 2.27 - 2.13 (m, 1H), 2.05 - 1.99 (m, 1H). LC/MS (ESI) m/z calcd for $C_{32}H_{32}FN_4O_3^+$ $[M+H]^+$, 539.25; found, 539.3.

**Example 11: preparation of 3-(5-(((1R,4R)-5-benzhydryl-2,5-diazabicyclo[2.2.1]heptan-2-yl)methyl)-1-oxoisoindolin-2-yl)piperidine-2,6-dione (GT-03570)**

Referring to the method of Scheme 4, the target product GT-03570 was prepared as white solid (14 mg, yield 32%). $^1H$ NMR (400 MHz, MeOD) δ 7.80 (d, *J* = 7.8 Hz, 1H), 7.76 (s, 1H), 7.70 (d, *J* = 7.8 Hz, 2H), 7.67 - 7.63 (m, 1H), 7.59 - 7.53 (m, 3H), 7.49 (d, *J* = 7.8 Hz, 2H), 7.30 - 7.24 (m, 2H), 7.22 - 7.17 (m, 1H), 5.08 - 5.04 (m, 2H), 4.67 (s, 4H), 4.47 - 4.36 (m, 4H), 2.85 - 2.75 (m, 2H), 2.72 - 2.66 (m, 2H), 2.46 - 2.35 (m, 2H), 2.12 - 2.05 (m, 2H). LC/MS (ESI) m/z calcd for $C_{32}H_{33}N_4O_3^+$ $[M+H]^+$, 521.25; found, 521.3.

**Example 12: preparation of 3-(5-(((1R,4R)-5-benzhydryl-2,5-diazabicyclo[2.2.1]heptan-2-yl)methyl)-4-fluoro-1-oxoisoindolin-2-yl)piperidine-2,6-dione (GT-03573)**

Referring to the method of Scheme 4, the target product GT-03573 was prepared as white solid (16 mg, yield 34%). $^1H$ NMR (400 MHz, MeOD) δ 7.77 - 7.72 (m, 1H), 7.67 - 7.63 (m, 1H), 7.60 - 7.46 (m, 4H), 7.34 - 7.14 (m, 6H), 5.10 - 5.05 (m, 2H), 4.58 - 4.42 (m, 5H), 4.24 (s, 1H), 3.99 - 3.71 (m, 2H), 3.03 - 2.95 (m, 1H), 2.92 - 2.75 (m, 2H), 2.74 - 2.64 (m, 1H), 2.47 - 2.38 (m, 2H), 2.31 - 2.21 (m, 1H), 2.17 - 2.01 (m, 1H). LC/MS (ESI) m/z calcd for $C_{32}H_{32}FN_4O_3^+$ $[M+H]^+$, 539.25; found, 539.3.

**Example 13: preparation of 3-(5-(((1R,4R)-5-benzhydryl-2,5-diazabicyclo[2.2.1]heptan-2-yl)methyl)-7-fluoro-1-oxoisoindolin-2-yl)piperidine-2,6-dione (GT-03576)**

Referring to the method of Scheme 4, the target product GT-03576 was prepared as white solid (10 mg, yield 22%). $^1H$ NMR (400 MHz, MeOD) δ 7.70 - 7.49 (m, 5H), 7.45 - 7.38 (m, 1H), 7.35 - 7.26 (m, 4H), 7.24 - 7.19 (m, 2H), 5.31 - 5.14 (m, 1H), 5.06 - 5.02 (m, 1H), 4.65 - 4.52 (m, 1H), 4.49 - 4.44 (m, 2H), 4.42 - 4.33 (m, 1H), 4.31 - 4.26 (m, 1H), 4.14 - 3.76 (m, 2H), 3.16 - 3.01 (m, 1H), 2.90 - 2.77 (m, 2H), 2.73 - 2.65 (m, 1H), 2.63 - 2.52 (m, 1H), 2.49 - 2.21 (m, 2H), 2.14 - 2.01 (m, 1H). LC/MS (ESI) m/z calcd for $C_{32}H_{32}FN_4O_3^+$ $[M+H]^+$, 539.25; found, 539.3.

**Example 14: preparation of 3-(5-(((1S,4S)-5-benzhydryl-2,5-diazabicyclo[2.2.1]heptan-2-yl)methyl)-1-oxoisoindolin-2-yl)piperidine-2,6-dione (GT-03721)**

Referring to the method of Scheme 4, the target product GT-03721 was prepared as white solid (32 mg, yield 54%). $^1H$ NMR (400 MHz, MeOD) δ 7.81 - 7.78 (m, 2H), 7.66 (d, *J* = 7.6 Hz, 1H), 7.57 (brs, 4H), 7.28 (t, *J* = 7.6 Hz, 4H), 7.23 - 7.19 (m, 2H), 5.33 - 5.14 (m, 1H), 5.07 (dd, *J* = 13.2, 5.2 Hz, 1H), 4.62 - 4.59 (m, 1H), 4.51 - 4.40 (m, 3H), 4.31 (s, 1H), 4.09 - 3.73 (m, 2H), 3.30 - 3.22 (m, 2H), 3.12 - 3.04 (m, 1H), 2.89 - 2.75 (m, 1H), 2.71 - 2.67 (m, 1H), 2.62 - 2.57 (m, 1H), 2.49 - 2.18 (m, 2H), 2.15 - 2.02 (m, 1H). LC/MS (ESI) m/z calcd for $C_{32}H_{33}N_4O_3^+$ $[M+H]^+$, 521.25; found, 521.3.

**Example 15: preparation of 3-(5-(((1S,4S)-5-benzhydryl-2,5-diazabicyclo[2.2.1]heptan-2-yl)methyl)-4-fluoro-1-oxoisoindolin-2-yl)piperidine-2,6-dione (GT-03724)**

Referring to the method of Scheme 4, the target product GT-03724 was prepared as white solid (26 mg, yield 45%). $^1H$ NMR (400 MHz, MeOD) δ 7.79 -7.74 (m, 1H), 7.65 (d, *J* = 7.6 Hz, 1H), 7.61 - 7.52 (m, 4H), 7.34 - 7.27 (m, 4H), 7.23 - 7.19 (m, 2H), 5.27 - 5.13 (m, 1H), 5.07 (dd, *J* = 13.2, 5.2 Hz, 1H), 4.65 - 4.39 (m, 5H), 4.28 (s, 1H), 4.09 - 3.91 (m, 2H), 3.30 - 3.27 (m, 1H), 3.13 - 2.96 (m, 1H), 2.91 - 2.76 (m, 1H), 2.77 - 2.67 (m, 1H), 2.65 - 2.50 (m, 1H), 2.50 - 2.37 (m, 1H), 2.31 - 2.23 (m, 1H), 2.16 - 2.00 (m, 1H). LC/MS (ESI) m/z calcd for $C_{32}H_{32}FN_4O_3^+$ $[M+H]^+$, 539.25; found, 539.3.

**Example 16: preparation of 3-(5-((3-benzhydryl-3,8-diazabicyclo[3.2.1]octan-8-yl)methyl)-1-oxoisoindolin-2-yl) piperidine-2,6-dione (GT-03837)**

Referring to the method of Scheme 4, the target product GT-03837 was prepared as white solid (20 mg, yield 58%). $^1H$ NMR (400 MHz, MeOD) δ 7.90 (d, *J* = 7.8 Hz, 1H), 7.86 (s, 1H), 7.75 (d, *J* = 7.8 Hz, 1H), 7.55 - 7.48 (m, 4H), 7.37 - 7.31 (m, 4H), 7.25 - 7.22 (m, 2H), 5.18 (dd, *J* = 13.2, 5.2 Hz, 1H), 4.66 - 4.48 (m, 3H), 4.39 (brs, 2H), 3.95 (brs, 2H), 2.97 - 2.88 (m, 3H), 2.83 - 2.77 (m, 1H), 2.62 - 2.32 (m, 7H), 2.22 - 2.16 (m, 1H). LC/MS (ESI) m/z calcd for $C_{33}H_{35}N_4O_3^+$ $[M+ H]^+$, 535.27; found, 535.3.

**Example 17: preparation of 3-(5-((3-benzhydryl-3,8-diazabicyclo[3.2.1]octan-8-yl)methyl)-4-fluoro-1-oxoisoindolin-2-yl)piperidine-2,6-dione (GT-03838)**

Referring to the method of Scheme 4, the target product GT-03838 was prepared as white solid (20 mg, yield 57%). $^1$H NMR (400 MHz, MeOD) δ 7.88 (t, *J*= 7.2 Hz, 1H), 7.75 (d, *J*= 7.8 Hz, 1H), 7.59 - 7.48 (m, 4H), 7.35 - 7.32 (m, 4H), 7.25 - 7.21 (m, 2H), 5.18 (dd, *J*= 13.2, 5.2 Hz, 1H), 4.68 - 4.57 (m, 3H), 4.47 (s, 2H), 4.07 (s, 2H), 3.07 - 2.86 (m, 3H), 2.83 - 2.71 (m 1H), 2.71 - 2.63 (m, 1H), 2.56 - 2.42 (m, 6H), 2.21 - 2.17 (m, 1H). LC/MS (ESI) m/z calcd for $C_{33}H_{34}FN_4O_3^+$ [M+H]$^+$, 553.26; found, 553.3.

**Example 18: preparation of 3-(5-((3-benzhydryl-3,8-diazabicyclo[3.2.1]octan-8-yl)methyl)-6-fluoro-1-oxoisoindolin-2-yl)piperidine-2,6-dione (GT-03839)**

Referring to the method of Scheme 4, the target product GT-03839 was prepared as white solid (18 mg, yield 51%). $^1$H NMR (400 MHz, MeOD) δ 7.97 (d, *J* = 6.0 Hz, 1H), 7.65 (d, *J* = 8.8 Hz, 1H), 7.57 - 7.52 (m, 4H), 7.34 (t, *J* = 7.6 Hz, 4H), 7.26 - 7.22 (m, 2H), 5.17 (dd, *J* = 13.2, 5.2 Hz, 1H), 4.56 (q, *J= 17.2* Hz, 3H), 4.45 (brs, 2H), 4.05 (brs, 2H), 3.00 - 2.86 (m, 3H), 2.83 - 2.77 (m, 1H), 2.72 - 2.62 (m, 1H), 2.53 - 2.46 (m, 6H), 2.22 - 2.16 (m, 1H). LC/MS (ESI) m/z calcd for $C_{33}H_{34}FN_4O_3^+$ [M+H]$^+$, 553.26; found, 553.3.

**Example 19: preparation of 3-(5-((3-benzhydryl-3,8-diazabicyclo[3.2.1]octan-8-yl)methyl)-7-fluoro-1-oxoisoindolin-2-yl)piperidine-2,6-dione (GT-03840)**

Referring to the method of Scheme 4, the target product GT-03840 was prepared as white solid (24 mg, yield 68%). $^1$H NMR (400 MHz, MeOD) δ 7.62 (s, 1H), 7.53 - 7.46 (m, 5H), 7.35 - 7.31 (m, 4H), 7.25 - 7.21 (m, 2H), 5.15 (dd, *J* = 13.2, 5.2 Hz, 1H), 4.65 - 4.50 (m, 2H), 4.47 (s, 1H), 4.36 (s, 2H), 3.95 (s, 2H), 2.97 - 2.88 (m, 3H), 2.85 - 2.72 (m, 1H), 2.58 - 2.41 (m, 7H), 2.21 - 2.16 (m, 1H). LC/MS (ESI) m/z calcd for $C_{33}H_{34}FN_4O_3^+$ [M+H]$^+$, 553.26; found, 553.3.

**Example 20: preparation of 3-(5-((8-benzhydryl-3,8-diazabicyclo[3.2.1]octan-3-yl)methyl)-1-oxoisoindolin-2-yl) piperidine-2,6-dione (GT-03722)**

Referring to the method of Scheme 4, the target product GT-03722 was prepared as white solid (24 mg, yield 39%). $^1$H NMR (400 MHz, MeOD) δ 7.72 (d, *J* = 7.6 Hz, 1H), 7.65 - 7.49 (m, 6H), 7.41 - 7.23 (m, 6H), 5.27 (brs, 1H), 5.06 (dd, *J* = 13.2, 5.2 Hz, 1H), 4.53 - 4.32 (m, 2H), 4.20 - 3.48 (m, 4H), 3.14 - 2.74 (m, 4H), 2.75 - 2.62 (m, 1H), 2.51 - 2.26 (m, 3H), 2.25 - 1.96 (m, 3H). LC/MS (ESI) m/z calcd for $C_{33}H_{35}N_4O_3^+$ [M+ H]$^+$, 535.27; found, 535.3.

**Example 21: preparation of 3-(5-((8-benzhydryl-3,8-diazabicyclo[3.2.1]octan-3-yl)methyl)-4-fluoro-1-oxoisoindolin-2-yl)piperidine-2,6-dione (GT-03725)**

Referring to the method of Scheme 4, the target product GT-03725 was prepared as white solid (20 mg, yield 33%). $^1$H NMR (400 MHz, MeOD) δ 7.64 - 7.57 (m, 4H), 7.55 (brs, 2H), 7.42 - 7.31 (m, 6H), 5.32 - 5.19 (m, 1H), 5.06 (dd, *J* = 13.2, 5.2 Hz, 1H), 4.47 (q, *J* = 17.2 Hz, 2H), 3.87 (brs, 2H), 3.75 (brs, 2H), 3.05 - 2.75 (m, 5H), 2.72 - 2.66 (m, 1H), 2.51 - 2.37 (m, 1H), 2.37 - 2.25 (m, 2H), 2.21 - 2.02 (m, 3H). LC/MS (ESI) m/z calcd for $C_{33}H_{34}FN_4O_3^+$ [M+H]$^+$, 553.26; found, 553.3.

**Example 22: preparation of 3-(5-(((R)-4-benzhydryl-2-methylpiperazin-1-yl)methyl)-1-oxoisoindolin-2-yl)piperidine-2,6-dione (GT-03571)**

Referring to the method of Scheme 4, the target product GT-03571 was prepared as white solid (14 mg, yield 45%). $^1$H NMR (400 MHz, MeOD) δ 7.91 (d, *J* = 7.8 Hz, 1H), 7.83 (s, 1H), 7.73 (d, *J* = 7.8 Hz, 1H), 7.68 - 7.59 (m, 5H), 7.43 - 7.30 (m, 5H), 5.27 - 5.12 (m, 2H), 4.78 (s, 2H), 4.60 - 4.45 (m, 3H), 4.36 - 4.32 (m, 1H), 3.90 (brs, 1H), 3.49 (brs, 1H), 3.01 - 2.88 (m, 4H), 2.83 - 2.77 (m, 1H), 2.54 - 2.50 (m, 1H), 2.20 - 2.17(m, 2H), 1.58 (d, *J* = 6.4 Hz, 3H). LC/MS (ESI) m/z calcd for $C_{32}H_{35}N_4O_3^+$ [M+H]$^+$, 523.27; found, 523.3.

**Example 23: preparation of 3-(5-(((R)-4-benzhydryl-2-methylpiperazin-1-yl)methyl)-4-fluoro-1-oxoisoindolin-2-yl)piperidine-2,6-dione (GT-03574)**

Referring to the method of Scheme 4, the target product GT-03574 was prepared as white solid (20 mg, yield 40%). $^1$H NMR (400 MHz, MeOD) δ 7.67 - 7.62 (m, 2H), 7.50 - 7.41 (m, 4H), 7.29 - 7.23 (m, 4H), 7.22 - 7.12 (m, 2H), 5.09 - 5.05 (m, 1H), 4.58 - 4.46 (m, 2H), 4.37 - 4.15 (m, 1H), 3.77 - 3.59 (m, 1H), 3.31 - 3.28 (m, 4H), 3.04 - 2.77 (m, 2H), 2.85 - 2.75 (m, 1H), 2.71 - 2.54 (m, 3H), 2.50 - 2.34 (m, 1H), 2.17 - 2.02 (m, 1H), 1.43 (brs, 3H). LC/MS (ESI) m/z calcd for

$C_{32}H_{34}FN_4O_3^+$ [M+H]+, 541.26; found, 541.3.

**Example 24: preparation of 3-(5-(((R)-4-benzhydryl-2-methylpiperazin-1-yl)methyl)-7-fluoro-1-oxoisoindolin-2-yl)piperidine-2,6-dione (GT-03577)**

Referring to the method of Scheme 4, the target product GT-03577 was prepared as white solid (18 mg, yield 41%). $^1$H NMR (400 MHz, MeOD) δ 7.59 - 7.45 (m, 4H), 7.40 - 7.09 (m, 8H), 5.03 (dd, *J* = 13.2, 5.2 Hz, 1H), 4.91 - 4.81 (m, 1H), 4.55 - 4.32 (m, 3H), 4.14 (brs, 1H), 3.62 (brs, 1H), 3.18 - 3.06 (m, 4H), 2.90 - 2.57 (m, 4H), 2.44 - 2.33 (m, 1H), 2.14 - 1.99 (m, 1H), 1.42 (brs, 3H). LC/MS (ESI) m/z calcd for $C_{32}H_{34}FN_4O_3^+$ [M+H]+, 541.26; found, 541.3.

**Example 25: preparation of 3-(5-((4-benzhydryl-2,6-dimethylpiperazin-1-yl)methyl)-1-oxoisoindolin-2-yl)piperidine-2,6-dione (GT-03723)**

Referring to the method of Scheme 4, the target product GT-03723 was prepared as white solid (15 mg, yield 24%). $^1$H NMR (400 MHz, MeOD) δ 7.95 (d, *J* = 8.0 Hz, 1H), 7.83 (s, 1H), 7.75 (d, *J* = 8.0 Hz, 1H), 7.55 - 7.48 (m, 4H), 7.38 - 7.33 (m, 4H), 7.30 - 7.26 (m, 2H), 5.22 (dd, *J* = 13.2, 5.2 Hz, 1H), 4.73 (s, 2H), 4.66 - 4.55 (m, 2H), 3.74 (brs, 1H), 3.64 (brs, 2H), 3.15 - 3, 12 (m, 2H), 3.00 - 2.91 (m, 1H), 2.84 - 2.80 (m, 1H), 2.74 - 2.32 (m, 4H), 2.28 - 2.19 (m, 1H), 1.51 (d, *J* = 6.0 Hz, 6H). LC/MS (ESI) m/z calcd for $C_{33}H_{37}N_4O_3^+$ [M+H]+, 537.29; found, 537.3.

**Example 26: preparation of 3-(5-((4-benzhydryl-2,6-dimethylpiperazin-1-yl)methyl)-4-fluoro-1-oxoisoindolin-2-yl)piperidine-2,6-dione (GT-03726)**

Referring to the method of Scheme 4, the target product GT-03726 was prepared as white solid (10 mg, yield 17%). $^1$H NMR (400 MHz, MeOD) δ 7.74 (t, *J*= 8.0 Hz, 1H), 7.64 (d, *J* = 8.0 Hz, 1H), 7.47 - 7.40 (m, 4H), 7.29 - 7.25 m, 4H), 7.22 - 7.20 (m, 2H), 5.09 (dd, *J* = 13.2, 5.2 Hz, 1H), 4.53 (q, *J* = 17.2 Hz, 4H), 3.59 - 3.42 (m, 2H), 3.12 - 2.95 (m, 2H), 2.91 - 2.77 (m, 1H), 2.73 - 2.68 (m, 1H), 2.61 - 2.52 (m, 1H), 2.52 - 2.38 (m, 2H), 2.18 - 2.03 (m, 1H), 1.29 (brs, 6H). LC/MS (ESI) m/z calcd for $C_{33}H_{36}FN_4O_3^+$ [M+H]+, 555.28; found, 555.3.

**Example 27: preparation of 3-(5-((4-benzhydryl-2,6-dimethylpiperazin-1-yl)methyl)-4-fluoro-1-oxoisoindolin-2-yl)piperidine-2,6-dione (GT-03873)**

Referring to the method of Scheme 4, the target product GT-03873 was prepared as white solid (34 mg, yield 43%). LC/MS (ESI) m/z calcd for $C_{33}H_{36}FN_4O_3^+$ [M+H]+, 555.28; found, 555.3.

**Example 28: preparation of 3-(5-((4-benzhydryl-2,6-dimethylpiperazin-1-yl)methyl)-7-fluoro-1-oxoisoindolin-2-yl)piperidine-2,6-dione (GT-03874)**

Referring to the method of Scheme 4, the target product GT-03874 was prepared as white solid (30 mg, yield 43%). $^1$H NMR (400 MHz, MeOD) δ 7.54 - 7.39 (m, 5H), 7.35 - 7.24 (m, 5H), 7.22 - 7.18 (m, 2H), 5.06 (dd, *J* = 13.2, 5.2 Hz, 1H), 4.56 - 4.37 (m, 4H), 3.56 - 3.40 (m, 2H), 3.08 - 3.01 (m, 2H), 2.94 - 2.75 (m, 2H), 2.72 - 2.65 (m, 1H), 2.62 - 2.52 (m, 1H), 2.46 - 2.34 (m, 2H), 2.15 - 2.02 (m, 1H), 1.29 (d, *J* = 3.6 Hz, 6H). LC/MS (ESI) m/z calcd for $C_{33}H_{36}FN_4O_3^+$ [M+H]+, 555.28; found, 555.3.

**Example 29: preparation of 3-(5-((5-benzhydryl-2,5-diazabicyclo[2.2.2]octan-2-yl)methyl)-1-oxoisoindolin-2-yl) piperidine-2,6-dione (GT-03993)**

Referring to the method of Scheme 4, the target product GT-03993 was prepared as white solid (25 mg, yield 36%). $^1$H NMR (400 MHz, MeOD) δ 7.81 (d, *J* = 8.0 Hz, 1H), 7.77 (brs, 1H), 7.71 - 7.62 (m, 1H), 7.34 - 7.24 (m, 4H), 7.36 - 7.23 (m, 4H), 7.21 - 7.14 (m, 2H), 5.08 (dd, *J* = 13.2, 5.2 Hz, 1H), 4.58 - 4.52 (m, 2H), 4.49 - 4.43 (m, 2H), 3.58 - 3.44 (m, 1H), 2.90 - 2.76 (m, 2H), 2.75 - 2.62 (m, 2H), 2.49 - 2.37 (m, 2H), 2.35 - 2.22 (m, 2H), 2.14 - 2.05 (m, 2H), 2.02 - 1.91 (m, 2H), 1.79 - 1.63 (m, 2H). LC/MS (ESI) m/z calcd for $C_{33}H_{35}N_4O_3^+$ [M+H]+, 535.27; found, 535.3.

**Example 30: preparation of 3-(5-((5-benzhydryl-2,5-diazabicyclo[2.2.2]octan-2-yl)methyl)-4-fluoro-1-oxoisoindolin-2-yl)piperidine-2,6-dione (GT-04163)**

Referring to the method of Scheme 4, the target product GT-04163 was prepared as white solid (33 mg, yield 52%). $^1$H NMR (400 MHz, MeOD) δ 8.00 - 7.88 (m, 1H), 7.88 - 7.62 (m, 5H), 7.54 - 7.38 (m, 4H), 7.39 - 7.31 (m, 2H), 5.19 (ddd, *J* = 13.2, 5.2, 1H), 4.84 - 4.71 (m, 2H), 4.70 - 4.54 (m, 2H), 4.31 - 3.95 (m, 1H), 3.79 - 3.61 (m, 2H), 3.54 - 3.44 (m, 2H),

3.22 - 3.09 (m, 1H), 3.00 - 2.87 (m, 1H), 2.87 - 2.75 (m, 1H), 2.64 - 2.27 (m, 3H), 2.26 - 2.02 (m, 3H), 1.98 - 1.82 (m, 1H). LC/MS (ESI) m/z calcd for $C_{33}H_{34}FN_4O_3^+$ [M+H]$^+$, 553.26; found, 553.3.

**Example 31: preparation of 3-(5-((4-benzhydryl-3,3-dimethylpiperazin-1-yl)methyl)-1-oxoisoindolin-2-yl)piperidine-2,6-dione (GT-04165)**

Referring to the method of Scheme 4, the target product GT-04165 was prepared as white solid (10 mg, yield 15%). LC/MS (ESI) m/z calcd for $C_{33}H_{37}N_4O_3^+$ [M+H]$^+$, 537.29; found, 537.3.

**Example 32: preparation of 3-(5-((4-benzhydryl-3,3-dimethylpiperazin-1-yl)methyl)-4-fluoro-1-oxoisoindolin-2-yl)piperidine-2,6-dione (GT-04166)**

Referring to the method of Scheme 4, the target product GT-04166 was prepared as white solid (6 mg, yield 10%). $^1$H NMR (400 MHz, MeOD) δ 7.69 - 7.61 (m, 2H), 7.32 - 7.10 (m, 10H), 5.46 (brs, 1H), 5.39 (s, 1H), 5.07 (dd, *J*= 13.2, 5.2 Hz, 1H), 4.54 - 4, 41 (m, 4H), 3.08 - 2.95 (m, 2H), 2.91 - 2.75 (m, 3H), 2.75 - 2.64 (m, 2H), 2.56 - 2.34 (m, 2H), 2.17 - 2.01 (m, 2H), 1.29 - 1.21 (m, 2H), 1.04 - 0.96 (m, 2H). LC/MS (ESI) m/z calcd for $C_{33}H_{36}FN_4O_3^+$ [M+H]$^+$, 555.28; found, 555.3.

**Example 33: preparation of 3-(4-fluoro-1-oxo-5-((4-(phenyl(pyridin-2-yl)methyl)piperazin-1-yl)methyl)isoindolin-2-yl)piperidine-2,6-dione (GT-03832)**

Referring to the method of Scheme 4, the target product GT-03832 was prepared as white solid (11 mg, yield 26%). $^1$H NMR (400 MHz, MeOD) δ 8.76 (dd, *J* = 6.0, 0.8 Hz, 1H), 8.44 (td, *J* = 8.0, 1.6 Hz, 1H), 8.01 (d, *J* = *8.0* Hz, 1H), 7.90 - 7.87 (m, 1H), 7.77 - 7.70 (m, 1H), 7.64 (d, *J*= 8.0 Hz, 1H), 7.42 - 7.27 (m, 5H), 5.20 (s, 1H), 5.08 (dd, *J*= 13.2, 5.2 Hz, 1H), 4.64 - 4.44 (m, 4H), 3.46 (t, *J* = 4.8 Hz, 4H), 3.07 - 2.74 (m, 4H), 2.72 - 2.67 (m, 2H), 2.45 - 2.41 (m, 1H), 2.12 - 2.08 (m, 1H). LC/MS (ESI) m/z calcd for $C_{30}H_{31}FN_5O_3^+$ [M+H]$^+$, 528.24; found, 528.2.

**Example 34: preparation of 3-(6-fluoro-1-oxo-5-((4-(phenyl(pyridin-2-yl)methyl)piperazin-1-yl)methyl)isoindolin-2-yl)piperidine-2,6-dione (GT-03833)**

Referring to the method of Scheme 4, the target product GT-03833 was prepared as white solid (26 mg, yield 36%). $^1$H NMR (400 MHz, MeOD) δ 8.88 (dd, *J* = 6.0, 0.8 Hz, 1H), 8.58 (td, *J* = 8.0, 1.6 Hz, 1H), 8.14 (d, *J* = 8.0 Hz, 1H), 8.04 - 8.00 (m, 1H), 7.96 (d, *J* = 6.0 Hz, 1H), 7.67 (d, *J* = 8.4 Hz, 1H), 7.54 - 7.40 (m, 5H), 5.32 (s, 1H), 5.18 (dd, *J* = 13.2, 5.2 Hz, 1H), 4.63 (s, 2H), 4.62 - 4.52 (m, 2H), 3.64 - 3.54 (m, 4H), 2.98 -2.82 (m, 4H), 2.80 - 2.77 (m, 2H), 2.59 - 2.48 (m, 1H), 2.24 - 2.18 (m, 1H). LC/MS (ESI) m/z calcd for $C_{30}H_{31}FN_5O_3^+$ [M+H]$^+$, 528.24; found, 528.2.

**Example 35: preparation of 3-(7-fluoro-1-oxo-5-((4-(phenyl(pyridin-2-yl)methyl)piperazin-1-yl)methyl)isoindolin-2-yl)piperidine-2,6-dione (GT-03834)**

Referring to the method of Scheme 4, the target product GT-03834 was prepared as white solid (39 mg, yield 49%). $^1$H NMR (400 MHz, MeOD) δ 8.89 (d, *J*= 5.6 Hz, 1H), 8.57 (t, *J*= 7.6 Hz, 1H), 8.14 (d, *J* = *8.0* Hz, 1H), 8.05 - 7.96 (m, 1H), 7.69 (s, 1H), 7.56 - 7.38 (m, 6H), 5.32 (s, 1H), 5.15 (dd, *J*= 13.2, 5.2 Hz, 1H), 4.72 - 4.49 (m, 4H), 3.54 (s, 4H), 3.20 - 2.85 (m, 4H), 2.82 - 2.77 (m, 1H), 2.62 - 2.36 (m, 2H), 2.34 - 2.13 (m, 1H). LC/MS (ESI) m/z calcd for $C_{30}H_{31}FN_5O_3^+$ [M+H]$^+$, 528.24; found, 528.2.

**Example 36: preparation of 3-(1-oxo-5-((4-(phenyl(pyridin-2-yl)methyl)piperazin-1-yl)methyl)isoindolin-2-yl)piperidine-2,6-dione (GT-03835)**

Referring to the method of Scheme 4, the target product GT-03835 was prepared as white solid (23 mg, yield 32%). $^1$H NMR (400 MHz, MeOD) δ 8.88 (dd, *J* = 6.0, 0.8 Hz, 1H), 8.57 (td, *J* = 8.0, 1.6 Hz, 1H), 8.14 (d, *J* = 8.0 Hz, 1H), 8.03 - 8.01 (m, 1H), 7.94 - 7.85 (m, 2H), 7.80 - 7.73 (m, 1H), 7.55 - 7.39 (m, 5H), 5.32 (s, 1H), 5.19 (dd, *J* = 13.2, 5.2 Hz, 1H), 4.70 - 4.49 (m, 4H), 3.54 (s, 4H), 3.21 - 3.08 (m, 1H), 2.97 - 2.90 (m, 3H), 2.83 - 2.77 (m, 1H), 2.75 - 2.35 (m, 2H), 2.23 - 2.17 (m, 1H). LC/MS (ESI) m/z calcd for $C_{30}H_{32}N_5O_3^+$ [M+H]$^+$, 510.25; found, 510.3.

**Example 37: preparation of 3-(1-oxo-4-((4-(phenyl(pyridin-2-yl)methyl)piperazin-1-yl)methyl)isoindolin-2-yl)piperidine-2,6-dione (GT-03836)**

Referring to the method of Scheme 4, the target product GT-03836 was prepared as white solid (28 mg, yield 34%). $^1$H NMR (400 MHz, MeOD) δ 8.87 (d, *J* = 5.2 Hz, 1H), 8.56 - 8.41 (m, 1H), 8.11 - 7.99 (m, 1H), 7.93 (d, *J* = 7.6 Hz, 2H),

7.87 (d, $J$ = 7.6 Hz, 1H), 7.68 (t, $J$ = 7.6 Hz, 1H), 7.55 - 7.39 (m, 5H), 5.32 (s, 1H), 5.23 (dd, $J$ = 13.2, 5.2 Hz, 1H), 4.82 - 4.63 (m, 2H), 4.49 (s, 2H), 3.53 (s, 4H), 3.14 - 2.89 (m, 4H), 2.89 - 2.75 (m, 2H), 2.65 - 2.54 (m, 1H), 2.31 - 2.14 (m, 1H). LC/MS (ESI) m/z calcd for $C_{30}H_{32}N_5O_3^+$ [M+H]$^+$, 510.25; found, 510.3.

**Example 38: preparation of 3-(5-((4-(bis(4-fluorophenyl)methyl)piperazin-1-yl)methyl)-1-oxoisoindolin-2-yl)piperidine-2,6-dione (GT-03386)**

Referring to the method of Scheme 4, the target product GT-03386 was prepared as white solid (11 mg, yield 21%). $^1$H NMR (400 MHz, DMSO) *6* 11.01 (s, 1H), 7.83-7.80 (m, 2H), 7.72 (d, $J$= 8.0 Hz, 1H), 7.52 (brs, 4H), 7.18 (t, $J$ = 8.0 Hz, 4H), 5.16-5.11 (m, 1H), 4.51-4.34 (m, 4H), 3.34-3.30 (m, 4H), 3.21-3.11 (m, 2H), 2.97-2.88 (m, 3H), 2.63-2.59 (m, 1H), 2.45-2.40 (m, 2H), 2.02-1.99 (m, 1H). LC/MS (ESI) m/z calcd for $C_{31}H_{31}F_2N_4O_3^+$ [M+H]$^+$, 545.24; found, 545.3.

**Example 39: preparation of 3-(5-((4-(bis(4-fluorophenyl)methyl)piperazin-1-yl)methyl)-4-fluoro-1-oxoisoindolin-2-yl)piperidine-2,6-dione (GT-03564)**

Referring to the method of Scheme 4, the target product GT-03564 was prepared as white solid (22 mg, yield 48%). $^1$H NMR (400 MHz, MeOD) δ 8.20 (s, 1H), 7.94 (dd, $J$ = 8.4, 1.2 Hz, 1H), 7.69 - 7.61 (m, 1H), 7.59 (d, $J$ = 8.4 Hz, 1H), 7.19 (dd, $J$ = 10.8, 2.4 Hz, 1H), 7.12 (t, $J$ = 8.4 Hz, 1H), 7.09 - 7.01 (m, 2H), 6.66 (d, $J$= 8.4 Hz, 1H), 5.29 - 5.24 (m, 1H), 5.05 - 5.03 (m, 1H), 4.90 - 4.85 (m, 1H), 4.73 - 4.69 (m, 1H), 4.66 - 4.60 (m, 1H), 4.49 - 4.43 (m, 1H), 4.02 - 4.89 (m, 2H), 2.90 - 2.70 (m, 3H), 2.62 - 2.47 (m, 1H), 2.44 - 2.39 (m, 2H), 2.06 - 1.96 (m, 2H), 1.85 - 1.69 (m, 2H). LC/MS (ESI) m/z calcd for $C_{31}H_{30}F_3N_4O_3^+$ [M+H]$^+$, 563.23; found, 563.3.

**Example 40: preparation of 3-(5-((4-(bis(4-fluorophenyl)methyl)piperazin-1-yl)methyl)-6-fluoro-1-oxoisoindolin-2-yl)piperidine-2,6-dione (GT-03698)**

Referring to the method of Scheme 4, the target product GT-03698 was prepared as white solid (22 mg, yield 33%). $^1$H NMR (400 MHz, MeOD) δ 7.88 (d, $J$ = 6.0 Hz, 1H), 7.67 (d, $J$ = 8.4 Hz, 1H), 7.65 - 7.54 (m, 4H), 7.12 (t, $J$ = 8.4 Hz, 4H), 5.18 (dd, $J$ = 13.2, 5.2 Hz, 1H), 4.90 (brs, 1H), 4.64 - 4.49 (m, 4H), 3.53 (brs, 4H), 3.18 - 2.72 (m, 6H), 2.58 - 2.47 (m, 1H), 2.27 - 2.14 (m, 1H). LC/MS (ESI) m/z calcd for $C_{31}H_{30}F_3N_4O_3^+$ [M+H]$^+$, 563.23; found, 563.3.

**Example 41: preparation of 3-(5-((4-(bis(4-fluorophenyl)methyl)piperazin-1-yl)methyl)-7-fluoro-1-oxoisoindolin-2-yl)piperidine-2,6-dione (GT-03565)**

Referring to the method of Scheme 4, the target product GT-03565 was prepared as white solid (25 mg, yield 55%). $^1$H NMR (400 MHz, MeOD) δ 7.57 (s, 1H), 7.53 - 7.49 (m, 4H), 7.42 (d, $J$= 9.8 Hz, 1H), 7.11 - 7.07 (m, 4H), 5.18 - 5.13 (m, 1H), 4.63 - 4.52 (m, 3H), 4.45 (brs, 2H), 3.37 - 3.34 (m, 4H), 3.22 - 2.69 (m, 5H), 2.56 - 2.45 (m, 2H), 2.28 - 2.12 (m, 1H). LC/MS (ESI) m/z calcd for $C_{31}H_{30}F_3N_4O_3^+$ [M+H]$^+$, 563.23; found, 563.3.

**Example 42: preparation of 3-(4-((4-(bis(4-fluorophenyl)methyl)piperazin-1-yl)methyl)-1-oxoisoindolin-2-yl)piperidine-2,6-dione (GT-03647)**

Referring to the method of Scheme 4, the target product GT-03647 was prepared as white solid (30 mg, yield 39%). $^1$H NMR (400 MHz, DMSO) δ 11.06 (s, 1H), 7.95-7.87 (m, 1H), 7.81 (d, $J$= 7.2 Hz, 1H), 7.61 (t, $J$ = 7.2 Hz, 1H), 7.49-7.41 (m, 4H), 7.23-7.13 (m, 4H), 5.19-5.15 (m, 1H), 4.80-4.75 (m, 1H), 4.53-4.48 (m, 1H), 4.41-4.30 (m, 2H), 3.29-3.14 (m, 4H), 2.95-2.78 (m, 4H), 2.67-2.63 (m, 1H), 2.40-2.28 (m, 2H), 2.08-2.03 (m, 1H), 1.30-1.26 (m, 1H). LC/MS (ESI) m/z calcd for $C_{31}H_{31}F_2N_4O_3^+$ [M+H]$^+$, 545.24; found, 545.2.

**Example 43: preparation of 3-(5-((4-(bis(3-fluorophenyl)methyl)piperazin-1-yl)methyl)-6-fluoro-1-oxoisoindolin-2-yl)piperidine-2,6-dione (GT-03701)**

Referring to the method of Scheme 4, the target product GT-03701 was prepared as white solid (23 mg, yield 39%). $^1$H NMR (400 MHz, MeOD) δ 7.88 (d, $J$ = 6.0 Hz, 1H), 7.68 (d, $J$ = 8.6 Hz, 1H), 7.43 - 7.27 (m, 6H), 7.02 (t, $J$ = 8.6 Hz, 2H), 5.18 (dd, $J$ = 13.2, 5.2 Hz, 1H), 4.70 (s, 1H), 4.61 - 4.51 (m, 4H), 3.51 (s, 4H), 3.12 - 2.64 (m, 6H), 2.58 - 2.47 (m, 1H), 2.27 - 2.13 (m, 1H). LC/MS (ESI) m/z calcd for $C_{31}H_{30}F_3N_4O_3^+$ [M+H]$^+$, 563.23; found, 563.3.

**Example 44: preparation of 3-(5-((4-(bis(3-fluorophenyl)methyl)piperazin-1-yl)methyl)-1-oxoisoindolin-2-yl)piperidine-2,6-dione (GT-03702)**

Referring to the method of Scheme 4, the target product GT-03702 was prepared as white solid (23 mg, yield 39%). $^1$H NMR (400 MHz, MeOD) δ 7.92 (d, *J* = 7.8 Hz, 1H), 7.81 (s, 1H), 7.71 (d, *J* = 7.8 Hz, 1H), 7.40 - 7.29 (m, 6H), 7.02 (t, *J* = 7.8 Hz, 2H), 5.19 (dd, *J* = 13.2, 5.2 Hz, 1H), 4.67 (s, 1H), 4.61 - 4.49 (m, 4H), 3.44 (s, 4H), 2.98 - 2.78 (m, 7H), 2.26 - 2.14 (m, 1H). LC/MS (ESI) m/z calcd for $C_{31}H_{31}F_2N_4O_3^+$ [M+H]$^+$, 545.24; found, 545.3.

**Example 45: preparation of 3-(5-((4-(bis(3-fluorophenyl)methyl)piperazin-1-yl)methyl)-4-fluoro-1-oxoisoindolin-2-yl)piperidine-2,6-dione (GT-03703)**

Referring to the method of Scheme 4, the target product GT-03703 was prepared as white solid (32 mg, yield 55%). $^1$H NMR (400 MHz, MeOD) δ 7.83 - 7.73 (m, 2H), 7.42 - 7.34 (m, 2H), 7.33 - 7.27 (m, 4H), 7.02 (t, *J* = 7.6 Hz, 2H), 5.19 (dd, *J*= 13.2, 5.2 Hz, 1H), 4.70 - 4.58 (m, 5H), 3.48 (s, 4H), 2.98 - 2.50 (m, 7H), 2.25 - 2.15 (m, 1H). LC/MS (ESI) m/z calcd for $C_{31}H_{30}F_3N_4O_3^+$ [M+H]$^+$, 563.23; found, 563.3.

**Example 46: preparation of 3-(5-((4-(bis(3-fluorophenyl)methyl)piperazin-1-yl)methyl)-7-fluoro-1-oxoisoindolin-2-yl)piperidine-2,6-dione (GT-03704)**

Referring to the method of Scheme 4, the target product GT-03704 was prepared as white solid (25 mg, yield 43%). $^1$H NMR (400 MHz, MeOD) δ 7.49 (s, 1H), 7.39 - 7.31 (m, 1H), 7.30 - 7.23 (m, 2H), 7.23 - 7.14 (m, 4H), 6.90 (t, *J = 7.6* Hz, 2H), 5.03 (dd, *J*= 13.2, 5.2 Hz, 1H), 4.54 (s, 1H), 4.51 - 4.35 (m, 5H), 3.31 (s, 4H), 2.87 - 2.33 (m, 6H), 2.13 - 2.02 (m, 1H). LC/MS (ESI) m/z calcd for $C_{31}H_{30}F_3N_4O_3^+$ [M+H]$^+$, 563.23; found, 563.3.

**Example 47: preparation of 3-(4-((4-(bis(3-fluorophenyl)methyl)piperazin-1-yl)methyl)-1-oxoisoindolin-2-yl)piperidine-2,6-dione (GT-03875)**

Referring to the method of Scheme 4, the target product GT-03875 was prepared as white solid (35 mg, yield 52%). $^1$H NMR (400 MHz, MeOD) *δ* 7.83 (d, *J* = 7.6 Hz, 1H), 7.75 (d, *J* = 7.6 Hz, 1H), 7.57 (t, *J* = 7.6 Hz, 1H), 7.30-7.18 (m, 6H), 6.93-6.89 (m, 2H), 5.13-5.09 (m, 1H), 4.93-4.81 (m, 2H), 4.66 (d, *J* = 17.2 Hz, 1H), 4.55 (d, *J* = 17.2 Hz, 1H), 4.37 (brs, 2H), 3.37 (brs, 4H), 3.29-3.26 (m, 1H), 2.89-2.79 (m, 2H), 2.74-2.67 (m, 2H), 2.48-2.38 (m, 1H), 2.17-2.10 (m, 1H). LC/MS (ESI) m/z calcd for $C_{31}H_{31}F_2N_4O_3^+$ [M+H]$^+$, 545.24; found, 545.3.

**Example 48: preparation of 3-(5-((4-(bis(4-chlorophenyl)methyl)piperazin-1-yl)methyl)-1-oxoisoindolin-2-yl)piperidine-2,6-dione (GT-03566)**

Referring to the method of Scheme 4, the target product GT-03566 was prepared as white solid (22 mg, yield 45%). $^1$H NMR (400 MHz, MeOD) δ 7.92 (d, *J* = 7.6 Hz, 1H), 7.78 (s, 1H), 7.71 (t, *J*= 7.6 Hz, 1H), 7.47 (d, *J*= 8.4 Hz, 4H), 7.36 (d, *J* = 8.4 Hz, 4H), 5.21-5.19 (m, 1H), 4.63-4.56 (m, 3H), 4.51 (s, 2H), 3.52-3.37 (m, 4H), 3.28-3.24 (m, 1H), 3.02-2.86 (m, 3H), 2.83-2.79 (m, 1H), 2.58-2.48 (m, 2H), 2.26-2.15 (m, 1H). LC/MS (ESI) m/z calcd for $C_{31}H_{31}Cl_2N_4O_3^+$ [M+H]$^+$, 577.18; found, 577.2.

**Example 49: preparation of 3-(5-((4-(bis(4-chlorophenyl)methyl)piperazin-1-yl)methyl)-4-fluoro-1-oxoisoindolin-2-yl)piperidine-2,6-dione (GT-03567)**

Referring to the method of Scheme 4, the target product GT-03567 was prepared as white solid (25 mg, yield 46%). $^1$H NMR (400 MHz, MeOD) δ 7.83 - 7.72 (m, 2H), 7.50 (d, *J* = 8.4 Hz, 4H), 7.37 (d, *J*= 8.4 Hz, 4H), 5.19 (dd, *J* = 13.2, 4.8 Hz, 1H), 4.73 - 4.53 (m, 5H), 3.47 (brs, 4H), 3.14 - 2.87 (m, 3H), 2.87 - 2.74 (m, 2H), 2.60 - 2.52 (m, 2H), 2.27 - 2.14 (m, 1H). LC/MS (ESI) m/z calcd for $C_{31}H_{30}Cl_2FN_4O_3^+$ [M+H]$^+$, 595.17; found, 595.2.

**Example 50: preparation of 3-(5-((4-(bis(4-chlorophenyl)methyl)piperazin-1-yl)methyl)-6-fluoro-1-oxoisoindolin-2-yl)piperidine-2,6-dione (GT-03699)**

Referring to the method of Scheme 4, the target product GT-03699 was prepared as white solid (22 mg, yield 34%). $^1$H NMR (400 MHz, MeOD) δ 7.74 (d, *J* = 6.0 Hz, 1H), 7.56 (d, *J* = 8.4 Hz, 1H), 7.37 (d, *J* = 8.4 Hz, 4H), 7.25 (d, *J* = 8.4 Hz, 4H), 5.07 (dd, *J* = 13.2, 5.2 Hz, 1H), 4.49 - 4.44 (m, 5H), 3.34 (s, 4H), 2.99 - 2.30 (m, 7H), 2.15 - 1.97 (m, 1H). LC/MS (ESI) m/z calcd for $C_{31}H_{30}Cl_2FN_4O_3^+$ [M+H]$^+$, 595.17; found, 595.2.

**Example 51: preparation of 3-(5-((4-(bis(4-chlorophenyl)methyl)piperazin-1-yl)methyl)-7-fluoro-1-oxoisoindolin-2-yl)piperidine-2,6-dione (GT-03568)**

Referring to the method of Scheme 4, the target product GT-03568 was prepared as white solid (23 mg, yield 48%). $^1$H NMR (400 MHz, MeOD) δ 7.57 (s, 1H), 7.48-7.41 (m, 5H), 7.38-7.33 (m, 4H), 5.18-5.13 (m, 1H), 4.64-4.51 (m, 3H), 4.48 (s, 2H), 3.47-3.34 (m, 4H), 3.30-3.23 (m, 1H), 3.18-2.87 (m, 3H), 2.82-2.78 (m, 1H), 2.56-2.46 (m, 2H), 2.25-2.16 (m, 1H). LC/MS (ESI) m/z calcd for $C_{31}H_{30}Cl_2FN_4O_3^+$ [M+H]$^+$, 595.17; found, 595.2.

**Example 52: preparation of 3-(4-((4-(bis(4-chlorophenyl)methyl)piperazin-1-yl)methyl)-1-oxoisoindolin-2-yl)piperidine-2,6-dione (GT-03648)**

Referring to the method of Scheme 4, the target product GT-03648 was prepared as white solid (36 mg, yield 39%). $^1$H NMR (400 MHz, DMSO) *δ* 11.06 (s, 1H), 7.91 (d, *J* = 7.2 Hz, 1H), 7.81 (d, *J* = 7.2 Hz, 1H), 7.61 (t, *J*= 7.2 Hz, 1H), 7.55-7.45 (m, 4H), 7.43-7.39 (m, 4H), 5.19-5.15 (m, 1H), 4.81 (d, *J* = 17.6 Hz, 1H), 4.51 (d, *J* = 17.6 Hz, 1H), 4.37 (brs, 2H), 3.42-3.20 (m, 4H), 2.98-2.84 (m, 4H), 2.65 (d, *J= 16.8* Hz, 2H), 2.37-2.33 (m, 2H), 2.05-1.99 (m, 1H), 1.24-1.16 (m, 1H). LC/MS (ESI) m/z calcd for $C_{31}H_{31}Cl_2N_4O_3^+$ [M+H]$^+$, 577.18; found, 577.2.

**Example 53: preparation of 3-(1-oxo-5-((4-(phenyl(pyridin-3-yl)methyl)piperazin-1-yl)methyl)isoindolin-2-yl)piperidine-2,6-dione (GT-04249)**

Referring to the method of Scheme 4, the target product GT-04249 was prepared as white solid (20 mg, yield 33%). $^1$H NMR (400 MHz, MeOD) δ 9.11 (s, 1H), 8.85 (d, *J*= 8.2 Hz, 1H), 8.80 (d, *J = 5.6* Hz, 1H), 8.11 (dd, *J = 8.2,* 5.6 Hz, 1H), 7.91 - 7.88 (m, 2H), 7.76 - 7.73 (m, 1H), 7.52 - 7.50 (m, 2H), 7.44 - 7.40 (m, 2H), 7.37 -7.33 (m, 1H), 5.18 (dd, *J* = 13.2, 5.2 Hz, 1H), 5.05 (s, 1H), 4.60 - 4.57 (m, 4H), 3.48 (s, 4H), 3.16 - 2.66 (m, 5H), 2.63 - 2.35 (m, 3H), 2.23 - 2.18 (m, 1H). LC/MS (ESI) m/z calcd for $C_{30}H_{32}N_5O_3^+$ [M+H]$^+$, 510.25; found, 510.3.

**Example 54: preparation of 3-(4-fluoro-1-oxo-5-((4-(phenyl(pyridin-3-yl)methyl)piperazin-1-yl)methyl)isoindolin-2-yl)piperidine-2,6-dione (GT-04250)**

Referring to the method of Scheme 4, the target product GT-04250 was prepared as white solid (23 mg, yield 35%). $^1$H NMR (400 MHz, MeOD) δ 9.09 (s, 1H), 8.83 (d, *J*= 8.0 Hz, 1H), 8.80 (d, *J = 5.6* Hz, 1H), 8.11 (dd, *J* = 8.2, 5.6 Hz, 1H), 7.90 - 7.82 (m, 1H), 7.76 (d, *J* = 7.6 Hz, 1H), 7.51 - 7.48 (m, 2H), 7.44 - 7.40 (m, 2H), 7.38 - 7.30 (m, 1H), 5.19 (dd, *J* = 13.2, 5.2 Hz, 1H), 5.01 (s, 1H), 4.75 - 4.55 (m, 4H), 3.54 (s, 4H), 3.20 - 2.65 (m, 5H), 2.60 - 2.49 (m, 2H), 2.24 - 2.19 (m, 1H). LC/MS (ESI) m/z calcd for $C_{30}H_{31}FN_5O_3^+$ [M+H]$^+$, 528.24; found, 528.3.

**Example 55: preparation of 3-(6-fluoro-1-oxo-5-((4-(phenyl(pyridin-3-yl)methyl)piperazin-1-yl)methyl)isoindolin-2-yl)piperidine-2,6-dione (GT-04251)**

Referring to the method of Scheme 4, the target product GT-04251 was prepared as white solid (25 mg, yield 38%). $^1$H NMR (400 MHz, MeOD) δ 8.97 (s, 1H), 8.72 - 8.67 (m, 2H), 7.99 (dd, *J* = 8.0, 5.5 Hz, 1H), 7.84 (d, *J* = 6.0 Hz, 1H), 7.56 (d, *J* = 8.0 Hz, 1H), 7.37 (d, *J* = 7.2 Hz, 2H), 7.30 (t, *J* = 7.2 Hz, 2H), 7.25 - 7.21 (m, 1H), 5.07 (dd, *J*= 13.2, 5.2 Hz, 1H), 4.88 (s, 1H), 4.55 - 4.37 (m, 4H), 3.41 (s, 4H), 2.91 - 2.77 (m, 3H), 2.75 - 2.64 (m, 1H), 2.58 (s, 1H), 2.47 - 2.36 (m, 2H), 2.16 - 1.98 (m, 1H). LC/MS (ESI) m/z calcd for $C_{30}H_{31}FN_5O_3^+$ [M+H]$^+$, 528.24; found, 528.3.

**Example 56: preparation of 3-(1-oxo-5-((4-(phenyl(pyridin-4-yl)methyl)piperazin-1-yl)methyl)isoindolin-2-yl)piperidine-2,6-dione (GT-04205)**

Referring to the method of Scheme 4, the target product GT-04205 was prepared as white solid (20 mg, yield 26%). $^1$H NMR (400 MHz, MeOD) δ 9.11 (s, 1H), 8.84 (d, *J*= 8.0 Hz, 1H), 8.80 (d, *J* = 5.6 Hz, 1H), 8.13 - 8.09 (m, 1H), 7.93 - 7.87 (m, 2H), 7.76 (d, *J* = 8.0 Hz, 1H), 7.56 - 7.50 (m, 2H), 7.42 (t, *J* = 7.2 Hz, 2H), 7.36 - 7.33 (m, 1H), 5.18 (dd, *J* = 13.2, 5.2 Hz, 1H), 5.05 (s, 1H), 4.70 - 4.47 (m, 4H), 3.48 (s, 4H), 3.20 - 2.66 (m, 5H), 2.65 - 2.33 (m, 2H), 2.32 - 2.13 (m, 1H). LC/MS (ESI) m/z calcd for $C_{30}H_{32}N_5O_3^+$ [M+H]$^+$, 510.25; found, 510.3.

**Example 57: preparation of 3-(4-fluoro-1-oxo-5-((4-(phenyl(pyridin-4-yl)methyl)piperazin-1-yl)methyl)isoindolin-2-yl)piperidine-2,6-dione (GT-04247)**

Referring to the method of Scheme 4, the target product GT-04247 was prepared as white solid (24 mg, yield 37%). $^1$H NMR (400 MHz, MeOD) δ 9.11 (s, 1H), 8.85 (d, *J* = 8.0 Hz, 1H), 8.80 (d, *J* = 5.6 Hz, 1H), 8.11 (dd, *J* = 8.0, 5.6 Hz,

1H), 7.89 - 7.83 (m, 1H), 7.76 - 7.73 (m, 1H), 7.52 - 7.50 (m, 2H), 7.42 (t, *J* = 7.2 Hz, 2H), 7.39 - 7.31 (m, 1H), 5.19 (dd, *J*= 13.2, 5.2 Hz, 1H), 5.05 (s, 1H), 4.76 - 4.56 (m, 4H), 3.55 (s, 4H), 3.15 - 2.67 (m, 5H), 2.64 - 2.38 (m, 2H), 2.25 - 2.19 (m, 1H). LC/MS (ESI) m/z calcd for $C_{30}H_{31}FN_5O_3^+$ $[M+H]^+$, 528.24; found, 528.3.

**Example 58: preparation of 3-(6-fluoro-1-oxo-5-((4-(phenyl(pyridin-4-yl)methyl)piperazin-1-yl)methyl)isoindolin-2-yl)piperidine-2,6-dione (GT-04248)**

Referring to the method of Scheme 4, the target product GT-04248 was prepared as white solid (26 mg, yield 40%). $^1H$ NMR (400 MHz, MeOD) δ 9.13 (s, 1H), 8.87 (d, *J*= 8.4 Hz, 1H), 8.82 (d, *J* = 5.6 Hz, 1H), 8.12 (dd, *J* = 8.0, 5.6 Hz, 1H), 7.98 (d, *J* = 6.0 Hz, 1H), 7.66 (d, *J* = 8.4 Hz, 1H), 7.53 (d, *J* = 7.2 Hz, 2H), 7.43 (t, *J* = 7.2 Hz, 2H), 7.36 (t, *J* = 7.2 Hz, 1H), 5.18 (dd, *J* = 13.2, 5.2 Hz, 1H), 5.10 (s, 1H), 4.63 (s, 2H), 4.56 (t, *J* = 14.8 Hz, 2H), 3.56 (s, 4H), 3.16 - 2.64 (m, 5H), 2.63 - 2.35 (m, 2H), 2.30 - 2.14 (m, 1H). LC/MS (ESI) m/z calcd for $C_{30}H_{31}FN_5O_3^+$ $[M+H]^+$, 528.24; found, 528.3.

**Example 59: preparation of 2-((2-(2,6-dioxopiperidin-3-yl)-1-oxoisoindolin-5-yl)methyl)-1H-benzo[de]isoquinoline-1,3(2H)-dione (GT-04244)**

Referring to the method of Scheme 5, the target product GT-04244 was prepared as white solid (22 mg, yield 50%). $^1H$ NMR (400 MHz, DMSO) δ 8.48 (dd, *J* = 8.4, 0.8 Hz, 2H), 8.41 (dd, *J* = 8.4, 0.8 Hz, 2H), 7.82 (t, *J* = 8.4, 2H), 7.62 (d, *J* = 8.0 Hz, 1H), 7.52 (s, 1H), 7.48 (d, *J* = 8.0 Hz, 1H), 5.33 (s, 2H), 5.02 (dd, *J* = 13.2, 5.2 Hz, 1H), 4.28 (q, *J* = 17.2 Hz, 2H), 2.86 - 2.77 (m, 1H), 2.59 - 2.49 (m, 1H), 2.35 - 2.24 (m, 1H), 2.00 - 1.86 (m, 1H). LC/MS (ESI) m/z calcd for $C_{26}H_{20}N_3O_5$+ $[M+H]^+$, 454.14; found, 454.2.

**Example 60: preparation of 3-(5-((4-(diphenylamino)piperidin-1-yl)methyl)-1-oxoisoindolin-2-yl)piperidine-2,6-dione (GT-04225)**

Referring to the method of Scheme 4, the target product GT-04225 was prepared as white solid (41 mg, yield 69%). $^1H$ NMR (400 MHz, MeOD) δ 7.79 (d, *J* = 7.8 Hz, 1H), 7.63 (s, 1H), 7.54 (d, *J* = 7.8 Hz, 1H), 7.23 - 7.14 (m, 4H), 6.94 - 6.90 (m, 2H), 6.80 - 6.76 (m, 4H), 5.07 (dd, *J* = 13.2, 5.2 Hz, 1H), 4.52 - 4.39 (m, 2H), 4.33 (s, 2H), 4.25 - 4.19 (m, 1H), 3.46 - 3.43 (m, 2H), 3.20 - 3.02 (m, 2H), 2.88 - 2.75 (m, 1H), 2.75 - 2.63 (m, 1H), 2.46 - 2.30 (m, 1H), 2.31 - 2.01 (m, 4H), 1.65 - 1.55 (m, 2H). LC/MS (ESI) m/z calcd for $C_{31}H_{33}N_4O_3^+$ $[M+H]^+$, 509.25; found, 509.3.

**Example 61: preparation of 3-(5-((4-(diphenylamino)piperidin-1-yl)methyl)-4-fluoro-1-oxoisoindolin-2-yl)piperidine-2,6-dione (GT-04226)**

Referring to the method of Scheme 4, the target product GT-04226 was prepared as white solid (32 mg, yield 52%). $^1H$ NMR (400 MHz, MeOD) δ 7.66 - 7.58 (m, 2H), 7.20 -7.16 (m, 4H), 6.92 (t, *J* = 7.6 Hz, 2H), 6.80 - 6.74 (m, 4H), 5.08 (dd, *J* = 13.2, 5.2 Hz, 1H), 4.52 (q, *J* = 17.2 Hz, 2H), 4.41 (s, 2H), 4.26 - 4.18 (m, 1H), 3.55 - 3.47 (m, 2H), 3.30 - 3.23 (m, 2H), 2.86 - 2.77 (m, 1H), 2.72 - 2.66 (m, 1H), 2.48 - 2.37 (m, 1H), 2.19 - 2.11 (m, 2H), 2.09 - 2.06 (m, 1H), 1.68 - 1.57 (m, 2H). LC/MS (ESI) m/z calcd for $C_{31}H_{32}FN_4O_3^+$ $[M+H]^+$, 527.25; found, 527.25.

**Example 62: preparation of 3-(5-((4-(diphenylamino)piperidin-1-yl)methyl)-6-fluoro-1-oxoisoindolin-2-yl)piperidine-2,6-dione (GT-04227)**

Referring to the method of Scheme 4, the target product GT-04227 was prepared as white solid (33 mg, yield 54%). $^1H$ NMR (400 MHz, MeOD) δ 7.82 (d, *J* = 6.0 Hz, 1H), 7.68 (d, *J* = 8.6 Hz, 1H), 7.37 - 7.26 (m, 4H), 7.04 (t, *J* = 7.6 Hz, 2H), 6.90 (d, *J* = 7.6 Hz, 4H), 5.19 (dd, *J* = 13.2, 5.3 Hz, 1H), 4.61 - 4.55 (m, 2H), 4.51 (s, 2H), 4.39 - 4.32 (m, 1H), 3.71 - 3.58 (m, 2H), 3.42 - 3.36 (m, 2H), 2.94 - 2.91 (m, 1H), 2.89 - 2.75 (m, 1H), 2.57 - 2.58 (m, 1H), 2.44 - 2.27 (m, 2H), 2.26 - 2.14 (m, 1H), 1.81 - 1.69 (m, 2H). LC/MS (ESI) m/z calcd for $C_{31}H_{32}FN_4O_3^+$ $[M+H]^+$, 527.25; found, 527.25.

**Example 63: preparation of 3-(6-((4-benzhydrylpiperazin-1-yl)methyl)-1-oxoisoindolin-2-yl)piperidine-2,6-dione (GT-05686)**

Referring to the method of Scheme 4, the target product (GT-05686) was prepared as white solid (19 mg, yield 29%). $^1H$ NMR (400 MHz, DMSO-d6) δ 11.01 (s, 1H), 10.37 (s, 1H), 7.96 (s, 1H), 7.81 (d, J = 8.0 Hz, 1H), 7.70 (d, J = 7.5 Hz, 1H), 7.51 - 7.44 (m, 4H), 7.38 - 7.28 (m, 4H), 7.28 - 7.13 (m, 2H), 5.12 (dd, J = 13.3, 4.8 Hz, 1H), 4.63 - 4.31 (m, 5H), 3.22 - 3.09 (m, 2H), 3.00 - 2.78 (m, 4H), 2.66 (d, J = 17.7 Hz, 2H), 2.44 - 2.34 (m, 3H), 2.08 - 1.95 (m, 1H). LCMS (ESI) calcd for $C_{31}H_{33}N_4O_3^+$ $[M+H]^+$: 509.25, found, 509.3.

**Example 64: preparation of 3-(7-((4-benzhydrylpiperazin-1-yl)methyl)-1-oxoisoindolin-2-yl)piperidine-2,6-dione (GT-05687)**

Referring to the method of Scheme 4, the target product (GT-05687) was prepared as white solid (44 mg, yield 68%). $^1$H NMR (400 MHz, DMSO-d6) δ 10.97 (s, 1H), 10.31 (s, 1H), 7.65 (s, 3H), 7.47 - 7.35 (m, 4H), 7.32 - 7.21 (m, 4H), 7.17 - 7.12 (m, 2H), 5.07 (dd, J = 13.2, 5.1 Hz, 1H), 4.81 (d, J = 11.8 Hz, 1H), 4.67 (d, J = 11.2 Hz, 1H), 4.38 (dd, J = 43.9, 17.9 Hz, 3H), 3.46 - 3.40 (m, 4H), 3.25 - 3.03 (m, 2H), 2.94 - 2.68 (m, 3H), 2.55 (d, J = 17.4 Hz, 1H), 2.40 - 2.32 (m, 1H), 1.96 - 1.91 (m, 1H). LCMS (ESI) calcd for $C_{31}H_{33}N_4O_3^+$ [M+H]$^+$: 509.25, found, 509.3.

**Example 65: preparation of 3-(5-(2-(4-benzhydrylpiperazin-1-yl)ethyl)-1-oxoisoindolin-2-yl)piperidine-2,6-dione (GT-05696)**

Referring to the method of Scheme 7, the target product (GT-05696) was prepared as white solid (12 mg, yield 15%). $^1$H NMR (400 MHz, DMSO-d6) δ 10.99 (s, 1H), 7.71 (d, *J* = 7.8 Hz, 1H), 7.56 - 7.37 (m, 6H), 7.34 (t, *J* = 7.3 Hz, 4H), 7.26 - 7.20 (m, 2H), 5.11 (dd, *J* = 13.1, 4.9 Hz, 1H), 4.56 - 4.24 (m, 3H), 3.57 - 3.51 (m, 2H), 3.19 - 3.10 (m, 4H), 2.96 - 2.83 (m, 4H), 2.63 - 2.51 (m, 2H), 2.46 - 2.25 (m, 3H), 2.03 - 1.96 (m, 1H). LCMS (ESI) calcd for $C_{32}H_{35}N_4O_3^+$ [M+H]$^+$: 523.27, found, 523.3.

**Example 66: preparation of 3-(5-(3-(4-benzhydrylpiperazin-1-yl)propyl)-1-oxoisoindolin-2-yl)piperidine-2,6-dione (GT-05688)**

Referring to the method of Scheme 4, the target product (GT-05688) was prepared as white solid (39 mg, yield 52%). $^1$H NMR (400 MHz, DMSO-d6) δ 10.99 (s, 1H), 7.67 (d, J = 7.8 Hz, 1H), 7.63 - 7.53 (m, 2H), 7.48 (s, 2H), 7.40 - 7.35 (m, 6H), 7.32 - 7.23 (m, 2H), 5.11 (dd, J = 13.3, 5.1 Hz, 1H), 4.43 (d, J = 17.3 Hz, 1H), 4.31 (d, J = 17.3 Hz, 1H), 3.47 - 3.42 (m, 4H), 3.10 (brs, 4H), 2.99 - 2.84 (m, 3H), 2.78 (t, J = 7.5 Hz, 3H), 2.60 (d, J = 16.8 Hz, 1H), 2.40 (dd, J = 13.1, 4.4 Hz, 1H), 2.09 - 1.98 (m, 4H). LCMS (ESI) calcd for $C_{33}H_{37}N_4O_3^+$ [M+H]$^+$: 537.29, found, 537.3.

**Example 67: preparation of 3-(5-(3-(4-benzhydrylpiperazin-1-yl)propyl)-1-oxoisoindolin-2-yl)piperidine-2,6-dione (GT-05867)**

Referring to the method of Scheme 8, the target product (GT-05867) was prepared as white solid (7 mg, yield 10%). $^1$H NMR (400 MHz, DMSO) δ 11.01 (s, 1H), 7.93 (s, 1H), 7.88 - 7.79 (m, 4H), 7.68 (d, *J*= 7.5 Hz, 2H), 7.51 - 7.38 (m, 4H), 7.32 (t, *J* = 7.3 Hz, 4H), 7.24 - 7.20 (m, 2H), 5.15 (dd, *J* = 13.5, 5.1 Hz, 1H), 4.55 - 4.38 (m, 5H), 3.28 - 3.09 (m, 4H), 3.04 - 2.79 (m, 4H), 2.62 (d, *J* = 17.3 Hz, 1H), 2.36 - 2.26 (m, 2H), 2.09 - 1.98 (m, 1H). LCMS (ESI) calcd for $C_{37}H_{37}N_4O_3^+$ [M+H]$^+$: 585.29, found, 585.3.

**Example 68: preparation of 3-(5-((1-benzhydrylpiperidin-4-yl)methyl)-1-oxoisoindolin-2-yl)piperidine-2,6-dione (GT-05744)**

Referring to the method of Scheme 8, the target product (GT-05744) was prepared as white solid (9 mg, yield 13%). $^1$H NMR (400 MHz, DMSO) δ 10.98 (s, 1H), 7.88 (d, *J* = 7.6 Hz, 1H), 7.78 (d, *J*= 7.6 Hz, 3H), 7.71 - 7.61 (m, 1H), 7.54 - 7.24 (m, 9H), 5.10 (dd, *J* = 13.6, 4.8 Hz, 1H), 4.45 - 4.39 (m, 1H), 4.34 - 4.26 (m, 1H), 3.22 - 3.12 (m, 2H), 2.95 - 2.82 (m, 3H), 2.68 - 2.66 (m, 2H), 2.64 - 2.56 (m, 2H), 2.44 - 2.31 (m, 1H), 2.10 - 1.93 (m, 2H), 1.88 - 1.65 (m, 4H). LCMS (ESI) calcd for $C_{32}H_{34}N_3O_3^+$ [M+H]$^+$: 508.26, found, 508.3.

**Example 69: preparation of 3-(5-((1-benzhydrylpiperidin-4-yl)amino)-1-oxoisoindolin-2-yl)piperidine-2,6-dione (GT-05864)**

Referring to the method of Scheme 8, the target product (GT-05864) was prepared as white solid (4 mg, yield 5%). $^1$H NMR (400 MHz, DMSO-d6) δ 11.23 (s, 1H), 10.92 (s, 1H), 7.86 (d, *J* = 7.6 Hz, 4H), 7.47 (t, *J* = 7.6 Hz, 4H), 7.39 (q, *J* = 7.5 Hz, 3H), 6.79 - 6.66 (m, 2H), 5.01 (dd, *J* = 13.3, 5.1 Hz, 1H), 4.27 (d, *J* = 17.0 Hz, 1H), 4.14 (d, *J* = 16.9 Hz, 1H), 3.30 - 3.23 (m, 3H), 3.22 - 3.03 (m, 3H), 2.97 - 2.82 (m, 1H), 2.59 - 2.55(m, 1H), 2.39 - 2.31 (m, 1H), 2.15 - 2.12 (m, 2H), 2.03 - 1.87 (m, 3H). LCMS (ESI) calcd for $C_{31}H_{33}N_4O_3^+$ [M+H]$^+$: 509.25, found, 509.3.

**Example 70: preparation of 3-(5-((4-benzhydrylpiperazin-1-yl)amino)-1-oxoisoindolin-2-yl)piperidine-2,6-dione (GT-05743)**

Referring to the method of Scheme 8, the target product (GT-05743) was prepared as white solid (10 mg, yield

17%). $^1$H NMR (400 MHz, DMSO-d6) δ 11.42 (s, 1H), 10.93 (s, 1H), 7.84 (d, *J* = 6.9 Hz, 3H), 7.69 (s, 1H), 7.47 - 7.39 (m, 7H), 6.93 (s, 1H), 6.86 (d, *J* = 8.5 Hz, 1H), 5.62 (d, *J* = 8.0 Hz, 1H), 5.03 (d, *J* = 8.6 Hz, 1H), 4.31 - 4.14 (m, 2H), 3.23 - 3.19 (m, 2H), 3.07 (brs, 4H), 2.95 - 2.87 (m, 1H), 2.58 (d, *J* = 18.3 Hz, 1H), 2.39 - 2.30 (m, 1H), 2.02 - 1.87 (m, 1H). LCMS (ESI) calcd for $C_{30}H_{32}N_5O_3^+$ $[M+H]^+$: 510.25, found, 510.3.

**Example 71: preparation of 3-(5-((1-benzhydrylazetidin-3-yl)methyl)-1-oxoisoindolin-2-yl)piperidine-2,6-dione (GT-05859)**

Referring to the method of Scheme 8, the target product (GT-05859) was prepared as white solid (6 mg, yield 7%). $^1$H NMR (400 MHz, DMSO-d6) δ 10.97 (s, 1H), 7.73 - 7.53 (m, 5H), 7.53 - 7.27 (m, 8H), 5.16 - 5.07 (m, 1H), 4.41 (d, *J* = 16.9 Hz, 1H), 4.28 (d, *J* = 17.2 Hz, 2H), 4.04 - 3.90 (m, 2H), 3.07 (s, 1H), 2.97 - 2.84 (m, 2H), 2.74 - 2.61 (m, 5H), 2.43 - 2.30 (m, 1H), 2.06 - 1.92 (m, 1H). LCMS (ESI) calcd for $C_{30}H_{30}N_3O_3^+$ $[M+H]^+$: 480.23, found, 480.2.

**Example 72: preparation of 3-(5-((1-benzhydrylazetidin-3-ylidene)methyl)-1-oxoisoindolin-2-yl)piperidine-2,6-dione (GT-05745)**

Referring to the method of Scheme 8, the target product (GT-05745) was prepared as white solid (11 mg, yield 13%). $^1$H NMR (400 MHz, DMSO-d6) δ 10.98 (s, 1H), 7.76 - 7.61 (m, 5H), 7.54 - 7.32 (m, 10H), 6.64 (brs, 1H), 6.15 - 5.93 (m, 1H), 5.28 - 5.02 (m, 3H), 5.00 - 4.75 (m, 2H), 4.41 (d, *J* = 17.6 Hz, 1H), 4.29 (d, *J* = 17.2 Hz, 1H), 2.94 - 2.87 (m, 1H), 2.70 - 2.61 (m, 2H), 2.42 - 2.28 (m, 1H), 2.05 - 1.88 (m, 1H). LCMS (ESI) calcd for $C_{30}H_{28}N_3O_3^+$ $[M+H]^+$: 478.21, found, 478.2.

**Example 73: preparation of3-(5-(1-benzhydrylpiperidin-4-yl)-1-oxoisoindolin-2-yl)piperidine-2,6-dione (GT-05868)**

Referring to the method of Scheme 8, the target product (GT-05868) was prepared as white solid (7 mg, yield 7%). $^1$H NMR (400 MHz, DMSO-d6) δ 10.98 (s, 1H), 7.86 (d, *J* = 7.6 Hz, 3H), 7.70 (d, *J* = 7.9 Hz, 1H), 7.49 - 7.46 (m, 6H), 7.43 - 7.37 (m, 3H), 5.58 (d, *J* = 9.3 Hz, 1H), 5.10 (dd, *J* = 13.3, 5.2 Hz, 1H), 4.45 (d, *J*= 17.1 Hz, 1H), 4.31 (d, *J* = 17.2 Hz, 1H), 3.18 - 3.10 (m, 2H), 3.00 - 2.87 (m, 2H), 2.62 - 2.58 (m, 2H), 2.45 - 2.19 (m, 4H), 2.06 - 1.94 (m, 3H). LCMS (ESI) calcd for $C_{31}H_{32}N_3O_3^+$ $[M+H]^+$: 494.24, found, 494.3.

**Example 74: preparation of 3-(5-(1-benzhydrylpiperidin-4-yl)-7-fluoro-1-oxoisoindolin-2-yl)piperidine-2,6-dione (GT-06198)**

Referring to the method of Scheme 8, the target product (GT-06198) was prepared as white solid (16 mg, yield 20%). $^1$H NMR (400 MHz, DMSO-d6) δ 11.24 (s, 1H), 10.93 (s, 1H), 7.87 - 7.80 (m, 4H), 7.45 - 7.38 (m, 4H), 7.35 - 7.28 (m, 2H), 7.26 (s, 1H), 7.13 (d, J = 10.9 Hz, 1H), 5.51 (d, J = 9.3 Hz, 1H), 4.99 (dd, J = 13.3, 4.7 Hz, 1H), 4.40 (d, J = 17.9 Hz, 1H), 4.28 (d, J = 17.9 Hz, 1H), 3.12 - 2.96 (m, 3H), 2.95 - 2.76 (m, 3H), 2.53 (d, J = 17.7 Hz, 2H), 2.37 - 2.17 (m, 3H), 2.05 - 1.86 (m, 3H). LCMS (ESI) calcd for $C_{31}H_{31}FN_3O_3^+$ $[M+H]^+$: 512.23, found, 512.3.

**Example 75: preparation of 3-(5-(1-benzhydrylpiperidin-4-yl)-4-fluoro-1-oxoisoindolin-2-yl)piperidine-2,6-dione (GT-06136)**

Referring to the method of Scheme 8, the target product (GT-06136) was prepared as white solid (11 mg, yield 14%). $^1$H NMR (400 MHz, DMSO-d6) δ 11.04 (s, 1H), 10.93 (s, 1H), 7.85 (d, J = 7.3 Hz, 1H), 7.81 - 7.77 (m, 3H), 7.55 (d, J = 7.9 Hz, 1H), 7.45 - 7.40 (m, 5H), 7.37 - 7.28 (m, 2H), 5.48 (d, J = 8.5 Hz, 1H), 5.04 (dd, J = 13.2, 5.1 Hz, 1H), 4.48 (d, J = 17.4 Hz, 1H), 4.31 (d, J = 17.5 Hz, 1H), 3.21 - 3.05 (m, 4H), 3.10 - 3.02 (m, 1H), 2.88 - 2.81 (m, 1H), 2.53 (d, J = 16.6 Hz, 1H), 2.39 - 2.21 (m, 3H), 1.98 - 1.89 (m, 3H). LCMS (ESI) calcd for $C_{31}H_{31}FN_3O_3^+$ $[M+H]^+$: 512.23, found, 512.3.

**Example 76: preparation of 3-(5-(1-benzhydryl-1,2,3,6-tetrahydropyridin-4-yl)-4-fluoro-1-oxoisoindolin-2-yl)piperidine-2,6-dione (GT-06072)**

Referring to the method of Scheme 8, the target product (GT-06072) was prepared as white solid (14 mg, yield 18%). $^1$H NMR (400 MHz, DMSO-d6) δ 11.57 (s, 1H), 11.01 (s, 1H), 7.90 - 7.80 (m, 4H), 7.57 - 7.34 (m, 8H), 6.14 (s, 1H), 5.71 (d, J = 11.8 Hz, 1H), 5.12 (dd, J = 13.2, 5.0 Hz, 1H), 4.58 (d, J = 17.3 Hz, 1H), 4.40 (d, J = 17.4 Hz, 1H), 3.89 - 3.70 (m, 2H), 3.15 - 2.98 (m, 2H), 2.99 - 2.79 (m, 2H), 2.70 - 2.56 (m, 2H), 2.46 - 2.37 (m, 1H), 2.09 - 1.91 (m, 1H). LCMS (ESI) calcd for $C_{31}H_{29}FN_3O_3^+$ $[M+H]^+$: 510.22, found, 510.2.

**Example 77: preparation of 3-(5-(8-benzhydryl-3,8-diazabicyclo[3.2.1]octan-3-yl)-1-oxoisoindolin-2-yl)piperidine-2,6-dione (GT-05865)**

Referring to the method of Scheme 8, the target product (GT-05865) was prepared as white solid (7 mg, yield 12%). $^1$H NMR (400 MHz, DMSO) δ 10.94 (s, 1H), 7.97 (d, *J* = 7.4 Hz, 3H), 7.64 - 7.38 (m, 7H), 7.30 (t, *J* = 6.3 Hz, 1H), 7.02 - 6.93 (m, 2H), 5.05 (dd, *J* = 11.3, 2.8 Hz, 1H), 4.35 (d, *J* = 17.1 Hz, 1H), 4.22 (d, *J* = 17.6 Hz, 1H), 3.83 - 3.78 (m, 3H), 3.66 (d, *J* = 11.1 Hz, 2H), 3.20 - 3.16 (m, 1H), 3.06 - 2.99 (m, 1H), 2.97 - 2.83 (m, 1H), 2.60 - 2.56(m, 2H), 2.42 - 2.39 (m, 2H), 2.35 - 2.32 (m, 1H), 2.11 - 1.89 (m, 3H). LCMS (ESI) calcd for $C_{32}H_{33}N_4O_3^+$ [M+H]$^+$: 521.25, found, 521.3.

**Example 78: preparation of 3-(5-(3-benzhydryl-3,8-diazabicyclo[3.2.1]octan-8-yl)-1-oxoisoindolin-2-yl)piperidine-2,6-dione (GT-05866)**

Referring to the method of Scheme 8, the target product (GT-05866) was prepared as white solid (16 mg, yield 19%). $^1$H NMR (400 MHz, DMSO) δ 10.93 (s, 1H), 7.92 - 7.88 (m, 1H), 7.52 - 7.38 (m, 5H), 7.27 (t, *J* = 7.4 Hz, 3H), 7.16 (t, *J* = 7.2 Hz, 2H), 6.91 - 6.88 (m, 2H), 5.08 - 4.97 (m, 1H), 4.34 (brs, 2H), 4.31 - 4.13 (m, 3H), 3.21 - 3.00 (m, 1H), 2.94 - 2.86 (m, 1H), 2.67 - 2.56 (m, 2H), 2.41 - 2.31 (m, 2H), 2.22 - 2.19 (m, 2H), 2.14 - 2.12 (m, 2H), 2.02 - 1.84 (m, 3H). LCMS (ESI) calcd for $C_{32}H_{33}N_4O_3^+$ [M+H]$^+$: 521.25, found, 521.3.

**Example 79: preparation of 3-(5-((4-benzhydryl-1,4-diazepan-1-yl)methyl)-6-fluoro-1-oxoisoindolin-2-yl)piperidine-2,6-dione (GT-05738)**

Referring to the method of Scheme 4, the target product (GT-05738) was prepared as white solid (14 mg, yield 46%). $^1$H NMR (400 MHz, DMSO) δ 11.02 (s, 1H), 7.98 - 7.88 (m, 1H), 7.91 - 7.49 (m, 5H), 7.49 - 7.13 (m, 6H), 5.14 (dd, *J* = 13.3, 5.0 Hz, 1H), 4.50 - 4.33 (m, 4H), 4.08 - 3.93 (m, 1H), 3.76 - 3.66 (m, 1H), 3.54 - 3.46 (m, 6H), 3.27 - 3.16 (m, 2H), 3.00 - 2.84 (m, 2H), 2.60 (d, *J* = 17.9 Hz, 1H), 2.46 - 2.38 (m, 1H), 2.05 - 1.95 (m, 1H). LCMS (ESI) calcd for $C_{32}H_{34}FN_4O_3^+$ [M+H]$^+$: 541.26, found, 541.3.

**Example 80: preparation of 3-(5-((7-benzhydryl-2,7-diazaspiro[3.5]nonan-2-yl)methyl)-1-oxoisoindolin-2-yl)piperidine-2,6-dione (GT-05901)**

Referring to the method of Scheme 4, the target product (GT-05901) was prepared as white solid (8 mg, yield 9%). $^1$H NMR (400 MHz, DMSO-d6) δ 11.79 (s, 1H), 11.25 (s, 1H), 11.01 (s, 1H), 7.89 - 7.84 (m, 5H), 7.82 - 7.80 (m, 2H), 7.68 (d, *J* = 7.8 Hz, 1H), 7.46 - 7.42 (m, 5H), 7.38 - 7.36 (m, 1H), 5.45 (d, *J* = 9.0 Hz, 1H), 5.14 (dd, *J* = 13.3, 5.0 Hz, 1H), 4.58 - 4.31 (m, 4H), 4.05 - 3.98 (m, 2H), 3.90 - 3.86 (m, 2H), 3.79 - 3.74 (m, 1H), 3.14 - 3.07 (m, 3H), 3.02 - 2.85 (m, 3H), 2.61 (d, *J* = 16.9 Hz, 1H), 2.45 - 2.41 (m, 1H), 2.25 2.20 (m, 2H), 2.08 - 1.96 (m, 1H). LCMS (ESI) calcd for $C_{34}H_{37}N_4O_3^+$ [M+H]$^+$: 549.29, found, 549.3.

**Example 81: preparation of 3-(5-((7-benzhydryl-2,7-diazaspiro[3.5]nonan-2-yl)methyl)-4-fluoro-1-oxoisoindolin-2-yl)piperidine-2,6-dione (GT-05902)**

Referring to the method of Scheme 4, the target product (GT-05902) was prepared as white solid (16 mg, yield 17%). $^1$H NMR (400 MHz, DMSO-d6) δ 11.86(s, 1H), 11.26 (s, 1H), 11.03 (s, 1H), 7.88 - 7.84 (m, 5H), 7.44 - 7.38 (m, 5H), 7.36 - 7.32 (m, 2H), 5.53 - 5.41 (m, 1H), 5.14 (dd, *J* = 13.0, 4.9 Hz, 1H), 4.65 - 4.59 (m, 3H), 4.43 (dd, *J* = 17.9, 5.6 Hz, 1H), 4.06 - 3.97 (m, 4H), 3.09 - 3.04 (m, 4H), 2.97 - 2.90 (m, 2H), 2.61 (d, *J* = 17.3 Hz, 1H), 2.30 - 2.24 (m, 4H), 2.09 - 1.95 (m, 1H). LCMS (ESI) calcd for $C_{34}H_{36}FN_4O_3^+$ [M+H]$^+$: 567.28, found, 567.3.

**Example 82: preparation of 3-(5-((7-benzhydryl-2,7-diazaspiro[3.5]nonan-2-yl)methyl)-6-fluoro-1-oxoisoindolin-2-yl)piperidine-2,6-dione (GT-05903)**

Referring to the method of Scheme 4, the target product (GT-05903) was prepared as white solid (17 mg, yield 19%). $^1$H NMR (400 MHz, DMSO-d6) δ 11.88 (s, 1H), 11.30 (s, 1H), 11.01 (s, 1H), 7.93-4.84 (m, 5H), 7.48 - 7.42 (m, 5H), 7.36 - 7.31 (m, 2H), 5.58 - 5.44 (m, 1H), 5.19 - 5.06 (m, 1H), 4.65 - 4.60 (m, 2H), 4.50 - 4.43 (m, 1H), 4.34 - 4.30 (m, 1H), 4.12 - 3.99 (m, 3H), 3.85 - 3.68 (m, 1H), 3.13 - 3.04 (m, 3H), 2.99 - 2.82 (m, 2H), 2.61 (d, J = 16.0 Hz, 1H), 2.47 - 2.34 (m, 2H), 2.27 - 2.18 (m, 3H), 2.04 - 2.00 (m, 1H). LCMS (ESI) calcd for $C_{34}H_{36}FN_4O_3^+$ [M+H]$^+$: 567.28, found, 567.3.

**Example 83: preparation of 3-(5-((7-benzhydryl-2,7-diazaspiro[3.5]nonan-2-yl)methyl)-7-fluoro-1-oxoisoindolin-2-yl)piperidine-2,6-dione (GT-05904)**

Referring to the method of Scheme 4, the target product (GT-05904) was prepared as white solid (16 mg, yield 17%). $^1$H NMR (400 MHz, DMSO-d6) δ 11.87 (s, 1H), 11.52 (s, 1H), 11.03 (s, 1H), 7.94 - 7.80 (m, 5H), 7.45 - 7.42 (M, 5H), 7.39 - 7.33 (m, 2H), 5.47 (d, J = 8.7 Hz, 1H), 5.10 (dd, J = 13.2, 5.0 Hz, 1H), 4.56 - 4.48 (m, 2H), 4.37 (d, J = 18.1 Hz, 1H), 4.04 - 4.01 (m, 2H), 3.93 - 3.87 (m, 2H), 3.82 - 3.72 (m, 1H), 3.16 - 3.06 (m, 3H), 2.97 - 2.89 (m, 2H), 2.68 - 2.56 (m, 1H), 2.41 - 2.34 (m, 1H), 2.31 - 2.15 (m, 4H), 2.08 - 1.96 (m, 1H). LCMS (ESI) calcd for $C_{34}H_{36}FN_4O_3^+$ [M+H]$^+$: 567.28, found, 567.3.

**Example 84: preparation of 3-(4-((7-benzhydryl-2,7-diazaspiro[3.5]nonan-2-yl)methyl)-1-oxoisoindolin-2-yl)piperidine-2,6-dione (GT-05905)**

Referring to the method of Scheme 4, the target product (GT-05905) was prepared as white solid (11 mg, yield 12%). $^1$H NMR (400 MHz, DMSO-d6) δ 11.80 (s, 1H), 11.20 (s, 1H), 11.05 (s, 1H), 7.92 - 7.83 (m, 5H), 7.81 (d, *J* = 7.5 Hz, 1H), 7.61 (t, *J* = 7.5 Hz, 1H), 7.49 - 7.42 (m, 4H), 7.38 - 7.33 (m, 2H), 5.53 - 5.46 (m, 1H), 5.17 (dd, *J* = 15.4, 3.9 Hz, 1H), 4.80 (dd, *J* = 17.4, 10.5 Hz, 1H), 4.51 - 5.46 (m, 3H), 4.17 - 4.01 (m, 3H), 3.99 - 3.91 (m, 2H), 3.16 - 3.10 (m, 3H), 3.00 - 2.87 (m, 2H), 2.64 (d, *J* = 16.5 Hz, 1H), 2.38 - 2.35 (m, 1H), 2.31 - 2.26 (m, 3H), 2.04 - 1.99 (m, 1H). LCMS (ESI) calcd for $C_{34}H_{37}N_4O_3^+$ [M+H]$^+$: 549.29, found, 549.3.

**Example 85: preparation of 3-(4-((7-benzhydryl-2,7-diazaspiro[3.5]nonan-2-yl)methyl)-1-oxoisoindolin-2-yl)piperidine-2,6-dione (GT-05969)**

Referring to the method of Scheme 4, the target product (GT-05969) was prepared as white solid (19 mg, yield 36%). $^1$H NMR (400 MHz, DMSO-d6) δ 11.82 (s, 1H), 11.01 (s, 1H), 8.00 - 7.72 (m, 7H), 7.47 - 7.43 (m, 4H), 7.37 - 7.33 (m, 2H), 5.65 - 5.58 (m, 1H), 5.15 (dd, J = 13.0, 5.4 Hz, 1H), 4.68 - 4.30 (m, 4H), 3.30 - 3.17 (m, 2H), 3.16 - 3.00 (m, 4H), 2.97 - 2.89 (m, 2H), 2.62 (d, J = 16.6 Hz, 1H), 2.48 - 2.36 (m, 1H), 2.34 - 1.75 (m, 8H). LCMS (ESI) calcd for $C_{35}H_{39}N_4O_3^+$ [M+H]$^+$: 563.30, found, 563.3.

**Example 86: preparation of 3-(5-((8-benzhydryl-2,8-diazaspiro[4.5]decan-2-yl)methyl)-4-fluoro-1-oxoisoindolin-2-yl)piperidine-2,6-dione (GT-05970)**

Referring to the method of Scheme 4, the target product (GT-05970) was prepared as white solid (22 mg, yield 41%). $^1$H NMR (400 MHz, DMSO-d6) δ 11.85 (s, 1H), 11.05 (s, 1H), 7.91 (s, 4H), 7.77 - 7.65 (m, 1H), 7.48 - 7.42 (m, 4H), 7.37 - 7.31 (m, 2H), 5.72 - 5.54 (m, 1H), 5.17 - 5.13 (m, 1H), 4.69 - 4.36 (m, 4H), 3.65 - 3.48 (m, 2H), 3.18 - 3.05 (m, 5H), 2.99 - 2.83 (m, 2H), 2.62 (d, J = 16.2 Hz, 1H), 2.48 - 2.37 (m, 1H), 2.27 - 2.12 (m, 3H), 2.08 - 1.97 (m, 2H), 1.91 - 1.84 (m, 2H). LCMS (ESI) calcd for $C_{35}H_{38}FN_4O_3^+$ [M+H]$^+$: 581.39, found, 581.3.

**Example 87: preparation of 3-(5-((8-benzhydryl-2,8-diazaspiro[4.5]decan-2-yl)methyl)-6-fluoro-1-oxoisoindolin-2-yl)piperidine-2,6-dione (GT-05971)**

Referring to the method of Scheme 4, the target product (GT-05971) was prepared as white solid (27 mg, yield 50%). $^1$H NMR (400 MHz, DMSO-d6) δ 11.83 (s, 1H), 11.03 (s, 1H), 8.14 - 8.00 (m, 1H), 7.97 - 7.82 (m, 4H), 7.70 - 7.65 (m, 1H), 7.45 - 7.37 (m, 4H), 7.35 - 7.31(m, 2H), 5.63 (d, J = 9.5 Hz, 1H), 5.14 (dd, J = 13.2, 5.0 Hz, 1H), 4.67 - 4.31 (m, 4H), 3.65 - 3.58 (m, 2H), 3.14 - 3.06 (m, 4H), 3.00 - 2.84 (m, 2H), 2.61 (d, J = 16.4 Hz, 1H), 2.47 - 2.36 (m, 1H), 2.29 - 2.18 (m, 2H), 2.15 - 1.94 (m, 4H), 1.91 - 1.84 (m, 2H). LCMS (ESI) calcd for $C_{35}H_{38}FN_4O_3^+$ [M+H]$^+$: 581.39, found, 581.3.

**Example 88: preparation of 3-(5-((8-benzhydryl-2,8-diazaspiro[4.5]decan-2-yl)methyl)-7-fluoro-1-oxoisoindolin-2-yl)piperidine-2,6-dione (GT-05972)**

Referring to the method of Scheme 4, the target product (GT-05972) was prepared as white solid (24 mg, yield 45%). $^1$H NMR (400 MHz, DMSO-d6) δ 11.68 (s, 1H), 11.06 (s, 1H), 7.93 - 7.85 (m, 4H), 7.78 - 7.56 (m, 2H), 7.45 - 7.42 (m, 4H), 7.39 - 7.32 (m, 2H), 5.60 (dd, J = 19.2, 9.0 Hz, 1H), 5.12 (dd, J = 16.3, 10.1 Hz, 1H), 4.61 - 4.37 (m, 4H), 3.65 - 3.47 (m, 1H), 3.19 - 2.98 (m, 5H), 2.93 - 2.88 (m, 2H), 2.61 (d, J = 16.6 Hz, 1H), 2.47 - 2.34 (m, 1H), 2.20 - 1.88(m, 8H). LCMS (ESI) calcd for $C_{35}H_{38}FN_4O_3^+$ [M+H]$^+$: 581.39, found, 581.3.

**Example 89: preparation of 3-(4-((8-benzhydryl-2,8-diazaspiro[4.5]decan-2-yl)methyl)-1-oxoisoindolin-2-yl)piperidine-2,6-dione (GT-05973)**

Referring to the method of Scheme 4, the target product (GT-05973) was prepared as white solid (25 mg, yield 48%). $^1$H NMR (400 MHz, DMSO-d6) δ 11.72 (s, 1H), 11.06 (s, 1H), 8.02 (dd, J = 40.5, 7.5 Hz, 1H), 7.91 - 7.80 (m, 5H), 7.66 - 7.60 (m, 1H), 7.48 - 7.41 (m, 4H), 7.39 - 7.34 (m, 2H), 5.68 - 5.50 (m, 1H), 5.20 - 5.15 (m, 1H), 4.97 - 4.75 (m, 1H), 4.63 - 4.34 (m, 3H), 3.63 - 3.48 (m, 2H), 3.21 - 2.83 (m, 7H), 2.64 (d, J = 17.0 Hz, 1H), 2.38 - 2.33 (m, 1H), 2.26 - 2.12 (m, 3H), 2.09 - 1.96 (m, 3H), 1.92 - 1.84 (m, 1H). LCMS (ESI) calcd for $C_{35}H_{39}N_4O_3^+$ [M+H]$^+$: 563.30, found, 563.3.

**Example 90: preparation of 3-(5-((9-benzhydryl-3,9-diazaspiro[5.5]undecan-3-yl)methyl)-1-oxoisoindolin-2-yl) piperidine-2,6-dione (GT-06002)**

Referring to the method of Scheme 4, the target product (GT-06002) was prepared as white solid (46 mg, yield 54%). $^1$H NMR (400 MHz, DMSO-d6) δ 11.68 (s, 1H), 11.01 (s, 1H), 10.73 (s, 1H), 7.95 - 7.87 (m, 4H), 7.86 (s, 1H), 7.81 (d, J = 7.8 Hz, 1H), 7.73 (d, J = 7.9 Hz, 1H), 7.45 - 7.42 (m, 4H), 7.38 - 7.33 (m, 2H), 5.66 (d, J = 9.4 Hz, 1H), 5.14 (dd, J = 13.2, 5.0 Hz, 1H), 4.56 - 4.31 (m, 4H), 3, 24 - 2.88 (m, 9H), 2.61 (d, J = 16.9 Hz, 1H), 2.42 (dd, J = 13.2, 4.4 Hz, 1H), 2.25 - 2.13 (m, 1H), 2.16 - 1.86 (m, 4H), 1.84 - 1.77 (m, 1H), 1.67 - 1.61 (m, 1H), 1.57 - 1.39 (m, 2H). LCMS (ESI) calcd for $C_{36}H_{41}N_4O_3^+$ [M+H]$^+$: 577.32, found, 577.3.

**Example 91: preparation of 3-(5-((9-benzhydryl-3,9-diazaspiro[5.5]undecan-3-yl)methyl)-4-fluoro-1-oxoisoindolin-2-yl)piperidine-2,6-dione (GT-06024)**

Referring to the method of Scheme 4, the target product (GT-06024) was prepared as white solid (42 mg, yield 48%). $^1$H NMR (400 MHz, DMSO-d6) δ 11.70 (s, 1H), 11.03 (s, 1H), 10.69 (s, 1H), 7.95 - 7.89 (m, 5H), 7.68 (d, J = 7.7 Hz, 1H), 7.45 - 7.42 (m, 4H), 7.37 - 7.34 (m, 2H), 5.67 (d, J = 9.1 Hz, 1H), 5.15 (dd, J = 13.2, 5.1 Hz, 1H), 4.69 - 4.52 (m, 1H), 4.53 - 4.36 (m, 3H), 3.26 - 2.88 (m, 9H), 2.61 (d, J = 17.1 Hz, 1H), 2.49 - 2.37 (m, 1H), 2.28 - 2.14 (m, 2H), 2.11 - 1.97 (m, 2H), 1.94 - 1.82(m, 2H), 1.67 - 1.60(m, 1H), 1.52 - 1.45 (m, 2H). LCMS (ESI) calcd for $C_{36}H_{40}FN_4O_3^+$ [M+H]$^+$: 595.31, found, 595.4.

**Example 92: preparation of 3-(5-((9-benzhydryl-3,9-diazaspiro[5.5]undecan-3-yl)methyl)-6-fluoro-1-oxoisoindolin-2-yl)piperidine-2,6-dione (GT-06025)**

Referring to the method of Scheme 4, the target product (GT-06025) was prepared as white solid (53 mg, yield 60%). $^1$H NMR (400 MHz, DMSO-d6) δ 11.64 (s, 1H), 11.03 (s, 1H), 10.64 (s, 1H), 8.01 (d, J = 6.0 Hz, 1H), 7.93 - 7.83 (m, 4H), 7.67 (d, J = 8.5 Hz, 1H), 7.50 - 7.40 (m, 4H), 7.38 - 7.34 (m, 2H), 5.65 (d, J = 9.4 Hz, 1H), 5.14 (dd, J = 13.3, 5.1 Hz, 1H), 4.52 - 4.29 (m, 4H), 3.20 - 2.85 (m, 9H), 2.61 (d, J = 16.5 Hz, 1H), 2.42 (dd, J = 17.4, 8.9 Hz, 1H), 2.27 - 2.23 (m, 1H), 2.17 - 2.14 (m, 1H), 2.11 - 1.96 (m, 2H), 1.96 - 1.73 (m, 2H), 1.65 - 1.59 (m, 1H), 1.52 - 1.45 (m, 2H). LCMS (ESI) calcd for $C_{36}H_{40}FN_4O_3^+$ [M+H]$^+$: 595.31, found, 595.3.

**Example 93: preparation of 3-(5-((9-benzhydryl-3,9-diazaspiro[5.5]undecan-3-yl)methyl)-7-fluoro-1-oxoisoindolin-2-yl)piperidine-2,6-dione (GT-06026)**

Referring to the method of Scheme 4, the target product (GT-06026) was prepared as white solid (35 mg, yield 40%). $^1$H NMR (400 MHz, DMSO-d6) δ 11.61 (s, 1H), 11.02 (s, 1H), 10.84 (s, 1H), 7.89 (t, J = 6.5 Hz, 4H), 7.71 - 7.55 (m, 2H), 7.46 - 7.42 (m, 4H), 7.37 - 7.34 (m, 2H), 5.65 (d, J = 9.3 Hz, 1H), 5.10 (dd, J = 13.3, 5.1 Hz, 1H), 4.56 - 4.31 (m, 4H), 3.20 - 3.16 (m, 1H), 3.13 - 2.93 (m, 7H), 2.91 - 2.88 (m, 1H), 2.61 (d, J = 16.8 Hz, 1H), 2.42 - 2.35 (m, 1H), 2.23 (d, J = 14.4 Hz, 1H), 2.16 - 1.96 (m, 3H), 1.95 - 1.74 (m, 2H), 1.65 (t, J = 12.6 Hz, 1H), 1.51 - 1.48 (m, 2H). LCMS (ESI) calcd for $C_{36}H_{40}FN_4O_3^+$ [M+H]$^+$: 595.31, found, 595.3.

**Example 94: preparation of 3-(4-((9-benzhydryl-3,9-diazaspiro[5.5]undecan-3-yl)methyl)-1-oxoisoindolin-2-yl) piperidine-2,6-dione (GT-06027)**

Referring to the method of Scheme 4, the target product (GT-06027) was prepared as white solid (46 mg, yield 54%). $^1$H NMR (400 MHz, DMSO-d6) δ 11.62 (s, 1H), 11.06 (s, 1H), 10.56 (s, 1H), 7.95 (d, J = 7.6 Hz, 1H), 7.89 (d, J = 7.2 Hz, 4H), 7.83 (d, J = 7.5 Hz, 1H), 7.63 (t, J = 7.5 Hz, 1H), 7.47 - 7.42 (m, 4H), 7.39 - 7.34 (m, 2H), 5.64 (d, J = 8.6 Hz, 1H), 5.17 (dd, J = 13.1, 5.1 Hz, 1H), 4.80 (dd, J = 17.4, 9.1 Hz, 1H), 4.49 (d, J = 15.3 Hz, 1H), 4.36 (s, 2H), 3.21 - 3.04 (m, 8H), 2.98 - 2.88 (m, 1H), 2.64 (d, J = 16.6 Hz, 1H), 2.37 - 2.34 (m, 1H), 2.28 - 2.12 (m, 2H), 2.09 - 2.02 (m, 2H), 1.98 - 1.78 (m, 2H), 1.74 - 1.58 (m, 1H), 1.49 (t, J = 14.7 Hz, 2H). LCMS (ESI) calcd for $C_{36}H_{41}N_4O_3^+$ [M+H]$^+$: 577.32, found, 577.3.

**Example 95: preparation of 3-(5-((5-benzhydrylhexahydropyrrolo[3,4-c]pyrrol-2(1H)-yl)methyl)-1-oxoisoindolin-2-yl)piperidine-2,6-dione (GT-06050)**

Referring to the method of Scheme 4, the target product (GT-06050) was prepared as white solid (47 mg, yield 52%). $^1$H NMR (400 MHz, DMSO-d6) δ 11.01 (s, 1H), 8.05 - 7.89 (m, 2H), 7.82 - 7.76 (m, 5H), 7.54 - 7.28 (m, 6H), 5.65 (dd, J = 53.7, 8.7 Hz, 1H), 5.13 (dd, J = 13.2, 4.8 Hz, 1H), 4.67 - 4.31 (m, 4H), 3.99 - 3.93 (m, 1H), 3.72 - 3.66 (m, 1H), 3.50 - 3.43 (m, 4H), 3.29 - 3.24 (m, 4H), 3.02 - 2.84 (m, 1H), 2.61 (d, J = 16.8 Hz, 1H), 2.43 (dd, J = 13.0, 4.8 Hz, 1H), 2.05 - 2.00 (m, 1H). LCMS (ESI) calcd for $C_{33}H_{35}N_4O_3^+$ [M+H]$^+$: 535.27, found, 535.3.

**Example 96: preparation of 3-(5-((5-benzhydrylhexahydropyrrolo[3,4-c]pyrrol-2(1H)-yl)methyl)-4-fluoro-1-oxoisoindolin-2-yl)piperidine-2,6-dione (GT-06044)**

Referring to the method of Scheme 4, the target product (GT-06044) was prepared as white solid (35 mg, yield 37%). $^1$H NMR (400 MHz, DMSO-d6) δ 11.03 (s, 1H), 8.00 - 7.95 (m, 2H), 7.85 - 7.77 (m, 3H), 7.68 (d, J = 7.6 Hz, 1H), 7.55 - 7.25 (m, 6H), 5.79 - 5.49 (m, 1H), 5.14 (dd, J = 13.2, 4.9 Hz, 1H), 4.63 - 4.41 (m, 4H), 4.04 - 3.96 (m, 1H), 3.70 - 3.66 (m, 1H), 3.64 - 3.51 (m, 2H), 3.38 - 3.29 (m, 6H), 2.99 - 2.85 (m, 1H), 2.61 (d, J = 17.4 Hz, 1H), 2.48 - 2.41 (m, 1H), 2.03 - 2.00 (m, 1H). LCMS (ESI) calcd for $C_{33}H_{34}FN_4O_3^+$ [M+H]$^+$: 553.26, found, 553.3.

**Example 97: preparation of 3-(5-((5-benzhydrylhexahydropyrrolo[3,4-c]pyrrol-2(1H)-yl)methyl)-6-fluoro-1-oxoisoindolin-2-yl)piperidine-2,6-dione (GT-06051)**

Referring to the method of Scheme 4, the target product (GT-06051) was prepared as white solid (46 mg, yield 49%). $^1$H NMR (400 MHz, DMSO-d6) δ 11.03 (s, 1H), 8.11 - 7.99 (m, 2H), 7.84 - 7.66 (m, 4H), 7.54 - 7.29 (m, 6H), 5.63 (dd, J = 48.9, 9.3 Hz, 1H), 5.14 (dd, J = 12.8, 4.8 Hz, 1H), 4.72 - 4.28 (m, 4H), 4.08 - 3.96 (m, 1H), 3.78 - 3.63 (m, 1H), 3.60 - 3.50 (m, 4H), 3.34 - 3.16 (m, 4H), 2.96 - 2.89 (m, 1H), 2.61 (d, J = 17.1 Hz, 1H), 2.48 - 2.37 (m, 1H), 2.04 - 2.00 (m, 1H). LCMS (ESI) calcd for $C_{33}H_{34}FN_4O_3^+$ [M+H]$^+$: 553.26, found, 553.3.

**Example 98: preparation of 3-(5-((5-benzhydrylhexahydropyrrolo[3,4-c]pyrrol-2(1H)-yl)methyl)-7-fluoro-1-oxoisoindolin-2-yl)piperidine-2,6-dione (GT-06045)**

Referring to the method of Scheme 4, the target product (GT-06045) was prepared as white solid (25 mg, yield 27%). $^1$H NMR (400 MHz, DMSO-d6) δ 11.02 (s, 1H), 7.99 (d, J = 7.6 Hz, 1H), 7.82 - 7.71 (m, 4H), 7.62 - 7.50 (m, 1H), 7.50 - 7.28 (m, 6H), 5.72 - 5.56 (m, 1H), 5.10 (dd, J = 13.3, 5.0 Hz, 1H), 4.68 - 4.33 (m, 4H), 3.98 - 3.74 (m, 2H), 3.60 - 3.40 (m, 4H), 3.29 - 3.16 (m, 4H), 3.01 - 2.87 (m, 1H), 2.60 (d, J = 16.9 Hz, 1H), 2.46 - 2.32 (m, 1H), 2.03 - 1.99 (m, 1H). LCMS (ESI) calcd for $C_{33}H_{34}FN_4O_3^+$ [M+H]$^+$: 553.26, found, 553.3.

**Example 99: preparation of 3-(4-((5-benzhydrylhexahydropyrrolo[3,4-c]pyrrol-2(1H)-yl)methyl)-1-oxoisoindolin-2-yl)piperidine-2,6-dione (GT-06052)**

Referring to the method of Scheme 4, the target product (GT-06052) was prepared as white solid (35 mg, yield 39%). $^1$H NMR (400 MHz, DMSO-d6) δ 11.07 (s, 1H), 8.02 - 7.95 (m, 2H), 7.93 - 7.68 (m, 4H), 7.64 - 7.61 (m, 1H), 7.44 - 7.35 (m, 6H), 5.75 - 5.56 (m, 1H), 5.19 (dd, J = 12.8, 5.3 Hz, 1H), 5.04 (dd, J = 26.3, 18.1 Hz, 1H), 4.64 - 4.45 (m, 2H), 3.99 - 3.94 (m, 1H), 3.87 - 3.57 (m, 2H), 3.53 - 3.47 (m, 4H), 3.33 - 3.27 (m, 4H), 3.03 - 2.91 (m, 1H), 2.66 (d, J = 17.0 Hz, 1H), 2.45 - 2.26 (m, 1H), 2.12 - 1.96 (m, 1H). LCMS (ESI) calcd for $C_{33}H_{35}N_4O_3^+$ [M+H]$^+$: 535.27, found, 535.3.

**Example 100: preparation of 3-(5-((3-benzhydryl-3,6-diazabicyclo[3.1.1]heptan-6-yl)methyl)-6-fluoro-1-oxoisoindolin-2-yl)piperidine-2,6-dione (GT-05737)**

Referring to the method of Scheme 4, the target product (GT-05737) was prepared as white solid (17 mg, yield 37%). $^1$H NMR (400 MHz, DMSO-d6) δ 11.02 (s, 1H), 7.88 - 7.83 (m, 1H), 7.72 - 7.61 (m, 1H), 7.57 - 7.47 (m, 4H), 7.35 - 7.30 (m, 4H), 7.26 - 7.19 (m, 2H), 5.14 (dd, *J*= 13.3, 5.9 Hz, 1H), 4.79 - 4.57 (m, 2H), 4.44 - 4.23 (m, 5H), 3.47 - 3.41 (m, 1H), 3.13 - 3.11 (m, 1H), 3.05 - 2.85 (m, 2H), 2.70 - 2.57 (m, 2H), 2.44 - 2.40 (m, 2H), 2.32 - 2.26 (m, 1H), 2.11 - 1.94 (m, 1H). LCMS (ESI) calcd for $C_{32}H_{32}FN_4O_3^+$ [M+H]$^+$: 539.25, found, 539.3.

**Example 101: preparation of 3-(5-(((1R,4R)-5-benzhydryl-2,5-diazabicyclo[2.2.1]heptan-2-yl)methyl)-6-fluoro-1-oxoisoindolin-2-yl)piperidine-2,6-dione (GT-05958)**

Referring to the method of Scheme 4, the target product (GT-05958) was prepared as white solid (11 mg, yield

12%). $^1H$ NMR (400 MHz, DMSO-d6) δ 11.02 (s, 1H), 8.04 - 7.98 (m, 1H), 7.90 - 7.48 (m, 5H), 7.48 - 7.16 (m, 6H), 5.14 (dd, J = 13.1, 5.3 Hz, 1H), 4.75 - 4.52 (m, 1H), 4.1 - 4.34 (m, 3H), 4.26 - 4.16 (m, 1H), 3.26 - 3.11 (m, 4H), 2.97 - 2.88 (m, 2H), 2.71 - 2.54 (m, 3H), 2.45 - 2.37 (m, 1H), 2.33 - 2.27 (m, 1H), 2.05 - 1.99 (m, 1H). LCMS (ESI) calcd for $C_{32}H_{32}FN_4O_3^+$ [M+H]$^+$: 539.25, found, 539.3.

**Example 102: preparation of 3-(5-(((1S,4S)-5-benzhydryl-2,5-diazabicyclo[2.2.1]heptan-2-yl)methyl)-6-fluoro-1-oxoisoindolin-2-yl)piperidine-2,6-dione (GT-05894)**

Referring to the method of Scheme 4, the target product (GT-05894) was prepared as white solid (16 mg, yield 17%). $^1H$ NMR (400 MHz, DMSO-d6) δ 11.02 (s, 1H), 8.07 (s, 1H), 8.01 - 7.73 (m, 2H), 7.66 (d, *J* = 8.4 Hz, 2H), 7.60 - 7.50 (m, 1H), 7.47 - 7.09 (m, 6H), 5.14 (dd, *J* = 13.3, 5.1 Hz, 1H), 4.67 - 4.58 (m, 1H), 4.49 (d, *J* = 17.5 Hz, 2H), 4.36 (d, *J* = 17.4 Hz, 2H), 3.67 - 3.60 (m, 4H), 3.49 - 3.42 (m, 4H), 2.97 - 2.86 (m, 1H), 2.61 (d, *J* = 17.2 Hz, 1H), 2.45 - 2.39(m, 1H), 2.08 - 1.96 (m, 1H). LCMS (ESI) calcd for $C_{32}H_{32}FN_4O_3^+$ [M+H]$^+$: 539.25, found, 539.3.

**Example 103: preparation of 3-(5-(((1S,4S)-5-benzhydryl-2,5-diazabicyclo[2.2.1]heptan-2-yl)methyl)-7-fluoro-1-oxoisoindolin-2-yl)piperidine-2,6-dione (GT-05895)**

Referring to the method of Scheme 4, the target product (GT-05895) was prepared as white solid (22 mg, yield 23%). $^1H$ NMR (400 MHz, DMSO-d6) δ 11.02 (s, 1H), 7.75 - 7.66 (m, 3H), 7.59 - 7.47 (m, 3H), 7.39 - 7.07 (m, 6H), 5.10 (dd, *J* = 13.1, 5.2 Hz, 1H), 4.65 - 4.54 (m, 1H), 4.54 - 4.49 (m, 2H), 4.44 - 4.30 (m, 2H), 3.57 - 3.48 (m, 4H), 3.34 - 3.25(m, 4H), 2.97 - 2.88 (m, 2H), 2.60 (d, *J* = 18.1 Hz, 2H), 2.43 - 2.33 (m, 1H), 2.08 - 1.96 (m, 1H). LCMS (ESI) calcd for $C_{32}H_{32}FN_4O_3^+$ [M+H]$^+$: 539.25, found, 539.3.

**Example 104: preparation of 3-(5-((8-benzhydryl-3,8-diazabicyclo[3.2.1]octan-3-yl)methyl)-6-fluoro-1-oxoisoindolin-2-yl)piperidine-2,6-dione (GT-05896)**

Referring to the method of Scheme 4, the target product (GT-05896) was prepared as white solid (21 mg, yield 22%). $^1H$ NMR (400 MHz, DMSO-d6) δ 11.01 (s, 1H), 8.04 - 7.99 (m, 3H), 7.87 - 7.72 (m, 1H), 7.61 - 7.54 (m, 2H), 7.51 - 7.22 (m, 6H), 5.41 (d, *J* = 7.9 Hz, 1H), 5.12 (dd, *J* = 13.2, 4.9 Hz, 1H), 4.46 (d, *J* = 17.2 Hz, 1H), 4.33 (d, *J* = 17.1 Hz, 1H), 3.92 (brs, 2H), 3.70 (brs, 4H), 3.32 - 3.19 (m, 2H), 3.01 - 2.82 (m, 2H), 2.60 (d, *J* = 17.7 Hz, 1H), 2.46 - 2.26 (m, 3H), 2.14 (brs, 2H), 2.05 - 1.94 (m, 1H). LCMS (ESI) calcd for $C_{33}H_{34}FN_4O_3^+$ [M+H]$^+$: 553.26, found, 553.3.

**Example** 105: **preparation of 3-(5-((8-benzhydryl-3,8-diazabicyclo[3.2.1]octan-3-yl)methyl)-7-fluoro-1-oxoisoindolin-2-yl)piperidine-2,6-dione (GT-05897)**

Referring to the method of Scheme 4, the target product (GT-05897) was prepared as white solid (20 mg, yield 21%). $^1H$ NMR (400 MHz, DMSO-d6) δ 11.00 (s, 1H), 8.00 - 7.95 (m, 3H), 7.80 - 7.78(m, 1H), 7.57 - 7.53 (m, 2H), 7.45 - 7.31 (m, 6H), 5.48 - 5.32 (m, 1H), 5.12 - 5.04 (m, 1H), 4.56 - 4.31 (m, 4H), 3.29 - 3.07 (m, 4H), 2.99 - 2.84 (m, 2H), 2.60 (d, *J* = 16.8 Hz, 1H), 2.43 - 2.29 (m, 3H), 2.06 - 1.98 (m, 4H). LCMS (ESI) calcd for $C_{33}H_{34}FN_4O_3^+$ [M+H]$^+$: 553.26, found, 553.3.

**Example 106: preparation of 3-(5-(((R)-4-benzhydryl-2-methylpiperazin-1-yl)methyl)-6-fluoro-1-oxoisoindolin-2-yl)piperidine-2,6-dione (GT-06046)**

Referring to the method of Scheme 4, the target product (GT-06046) was prepared as white solid (46 mg, yield 48%). $^1H$ NMR (400 MHz, DMSO-d6) δ 11.02 (s, 1H), 7.95 (s, 1H), 7.65 (d, J = 8.5 Hz, 1H), 7.60 (brs, 4H), 7.38 - 7.32 (m, 4H), 7.28 - 7.25 (m, 2H), 5.13 (dd, J = 13.3, 5.1 Hz, 1H), 4.77 - 4.62 (m, 1H), 4.50 - 4.31 (m, 3H), 4.25 - 4.16 (m, 1H), 3.70 - 3.60 (m, 2H), 3.22 - 3.14 (m, 3H), 3.06 - 2.81 (m, 3H), 2.60 (d, J = 16.8 Hz, 1H), 2.42 (dd, J = 13.1, 4.3 Hz, 1H), 2.01 - 1.97 (m, 1H), 1.41 (s, 3H). LCMS (ESI) calcd for $C_{32}H_{34}FN_4O_3^+$ [M+H]$^+$: 541.26, found, 541.3.

**Example 107: preparation of 3-(5-((4-benzhydryl-3,5-dimethylpiperazin-1-yl)methyl)-1-oxoisoindolin-2-yl)piperidine-2,6-dione (GT-03872)**

Referring to the method of Scheme 4, the target product (GT-03872) was prepared as white solid (24 mg, yield 27%). $^1H$ NMR (400 MHz, DMSO-d6) δ 11.01 (s, 1H), 9.75 (s, 1H), 7.82 - 7.77 (m, 2H), 7.71 - 7.65 (m, 1H), 7.36 (t, J = 7.2 Hz, 4H), 7.29 (t, J = 7.6 Hz, 4H), 7.19 (t, J = 7.2 Hz, 2H), 5.69 (s, 1H), 5.13 (dd, J = 13.2, 5.1 Hz, 1H), 4.49 (d, J = 17.5 Hz, 2H), 4.36 (d, J = 17.4 Hz, 2H), 4.29 - 4.02 (m, 2H), 3.24 - 3.15 (m, 4H), 3.00 - 2.85 (m, 2H), 2.63 - 2.59 (m, 2H), 2.46 - 2.37 (m, 1H), 2.03 - 1.99 (m, 1H), 1.26 (s, 3H), 1.25 (s, 3H). LCMS (ESI) calcd for $C_{33}H_{37}N_4O_3^+$ [M+H]$^+$: 537.29, found, 537.3.

**Example 108: preparation of 3-(5-((4-benzhydryl-3,5-dimethylpiperazin-1-yl)methyl)-4-fluoro-1-oxoisoindolin-2-yl)piperidine-2,6-dione (GT-05965)**

Referring to the method of Scheme 4, the target product (GT-05965) was prepared as white solid (27 mg, yield 29%). $^1$H NMR (400 MHz, DMSO-d6) δ 11.03 (s, 1H), 9.60 (s, 1H), 7.80 - 7.69 (m, 1H), 7.64 (d, J = 7.6 Hz, 1H), 7.36 (d, J = 7.3 Hz, 4H), 7.29 (t, J = 7.6 Hz, 4H), 7.19 (t, J = 7.2 Hz, 2H), 5.69 (s, 1H), 5.14 (dd, J = 13.2, 5.0 Hz, 1H), 4.59 (d, J = 17.6 Hz, 1H), 4.43 (d, J = 17.5 Hz, 1H), 4.08 (brs, 2H), 3.23 - 3.12 (m, 4H), 2.99 - 2.85 (m, 2H), 2.63 - 2.59 (m, 2H), 2.48 - 2.39 (m, 2H), 2.04 - 2.00 (m, 1H), 1.25 (s, 3H), 1.23 (s, 3H). LCMS (ESI) calcd for $C_{33}H_{36}FN_4O_3^+$ [M+H]$^+$: 555.28, found, 555.3.

**Example 109: preparation of 3-(5-((4-benzhydryl-3,5-dimethylpiperazin-1-yl)methyl)-6-fluoro-1-oxoisoindolin-2-yl)piperidine-2,6-dione (GT-05966)**

Referring to the method of Scheme 4, the target product (GT-05966) was prepared as white solid (17 mg, yield 18%). $^1$H NMR (400 MHz, DMSO-d6) δ 11.02 (s, 1H), 7.78 (s, 1H), 7.58 (d, J = 8.5 Hz, 1H), 7.37 (d, J = 7.3 Hz, 4H), 7.29 (t, J = 7.5 Hz, 4H), 7.19 (t, J = 7.2 Hz, 2H), 5.13 (dd, J = 13.3, 5.0 Hz, 1H), 4.45 (d, J = 17.4 Hz, 1H), 4.34 (d, J = 17.4 Hz, 1H), 3.96 (brs, 2H), 3.23 - 3.09 (m, 4H), 2.99 - 2.84 (m, 2H), 2.61 (d, J = 17.2 Hz, 1H), 2.47 - 2.33 (m, 3H), 2.07 - 1.95 (m, 2H), 1.24 (s, 3H), 1.22 (s, 3H). LCMS (ESI) calcd for $C_{33}H_{36}FN_4O_3^+$ [M+H]$^+$: 555.28, found, 555.3.

**Example 110: preparation of 3-(5-((4-benzhydryl-3,5-dimethylpiperazin-1-yl)methyl)-7-fluoro-1-oxoisoindolin-2-yl)piperidine-2,6-dione (GT-05967)**

Referring to the method of Scheme 4, the target product (GT-05967) was prepared as white solid (22 mg, yield 24%). $^1$H NMR (400 MHz, DMSO-d6) δ 11.01 (s, 1H), 8.15 - 8.08 (m, 1H), 7.56 - 7.50 (m, 2H), 7.37 - 7.34 (m, 4H), 7.31 - 7.27 (m, 4H), 7.21 - 7.17 (m, 2H), 5.11 - 5.07 (m, 2H), 4.52 - 4.34 (m, 4H), 3.24 - 3.13 (m, 4H), 2.98 - 2.83 (m, 2H), 2.60 (d, J = 17.4 Hz, 1H), 2.44 - 2.35 (m, 2H), 2.03 - 1.99 (m, 1H), 1.24 (s, 3H), 1.22 (s, 3H). LCMS (ESI) calcd for $C_{33}H_{36}FN_4O_3^+$ [M+H]$^+$: 555.28, found, 555.3.

**Example 111: preparation of 3-(5-((5-benzhydryl-2,5-diazabicyclo[2.2.2]octan-2-yl)methyl)-6-fluoro-1-oxoisoindolin-2-yl)piperidine-2,6-dione (GT-05963)**

Referring to the method of Scheme 4, the target product (GT-05963) was prepared as white solid (16 mg, yield 17%). $^1$H NMR (400 MHz, DMSO-d6) δ 11.02 (s, 1H), 10.45 (s, 1H), 8.11 - 7.99 (m, 1H), 7.71 - 7.65 (m, 1H), 7.63 - 7.47 (m, 3H), 7.44 - 7.28 (m, 5H), 7.23 - 7.16 (m, 2H), 5.14 (dd, J = 13.3, 5.1 Hz, 1H), 4.75 - 4.61 (m, 1H), 4.52 - 4.35 (m, 3H), 3.59 - 3.44 (m, 4H), 3.28 - 3.14 (m, 2H), 3.00 - 2.79 (m, 2H), 2.61 (d, J = 18.1 Hz, 1H), 2.46 - 2.40 (m, 1H), 2.18 - 2.09 (m, 1H), 2.05 - 2.00 (m, 1H), 1.94 - 1.86 (m, 1H), 1.80 - 1.48 (m, 1H). LCMS (ESI) calcd for $C_{33}H_{34}FN_4O_3^+$ [M+H]$^+$: 553.26, found, 553.3.

**Example 112: preparation of 3-(5-((5-benzhydryl-2,5-diazabicyclo[2.2.2]octan-2-yl)methyl)-7-fluoro-1-oxoisoindolin-2-yl)piperidine-2,6-dione (GT-05964)**

Referring to the method of Scheme 4, the target product (GT-05964) was prepared as white solid (43 mg, yield 46%). $^1$H NMR (400 MHz, DMSO-d6) δ 11.02 (s, 1H), 8.16 - 7.89 (m, 1H), 7.88 - 7.66 (m, 3H), 7.65 - 7.57 (m, 2H), 7.46 - 7.07 (m, 6H), 5.10 (dd, J = 13.2, 5.0 Hz, 1H), 4.55 - 4.49 (m, 3H), 4.42 - 4.39 (m, 1H), 3.62 - 3.51 (m, 4H), 3.36 - 3.13 (m, 4H), 2.95 - 2.85 (m, 1H), 2.61 (d, J = 17.0 Hz, 1H), 2.56 - 2.51 (m, 1H), 2.43 - 2.38 (m, 1H), 2.31 - 2.14 (m, 1H), 2.09 - 1.94 (m, 1H), 1.89 - 1.69 (m, 1H). LCMS (ESI) calcd for $C_{33}H_{34}FN_4O_3^+$ [M+H]$^+$: 553.26, found, 553.3.

**Example 113: preparation of 3-(5-((4-benzhydryl-3,3-dimethylpiperazin-1-yl)methyl)-6-fluoro-1-oxoisoindolin-2-yl)piperidine-2,6-dione (GT-05960)**

Referring to the method of Scheme 4, the target product (GT-05960) was prepared as white solid (18 mg, yield 19%). $^1$H NMR (400 MHz, DMSO-d6) δ 11.02 (s, 1H), 9.90 (s, 1H), 8.03 - 7.99 (m, 1H), 7.69 - 7.67 (m, 1H), 7.42 - 7.27 (m, 8H), 7.23 - 7.18 (m, 2H), 5.16 - 5.13 (m, 1H), 4.51 - 4.35 (m, 4H), 3.21 - 3.14 (m, 4H), 3.00 - 2.86 (m, 2H), 2.82 - 2.71 (m, 2H), 2.61 (d, J = 16.5 Hz, 2H), 2.41 - 2.23 (m, 2H), 2.03 - 2.00(m, 2H), 1.23 (s, 3H), 1.01 (s, 3H). LCMS (ESI) calcd for $C_{33}H_{36}FN_4O_3^+$ [M+H]$^+$: 553.28, found, 555.3.

**Example 114: preparation of 3-(5-((4-benzhydryl-3,3-dimethylpiperazin-1-yl)methyl)-7-fluoro-1-oxoisoindolin-2-yl)piperidine-2,6-dione (GT-05961)**

Referring to the method of Scheme 4, the target product (GT-05961) was prepared as white solid (19 mg, yield

20%). $^1$H NMR (400 MHz, DMSO-d6) δ 11.02 (s, 1H), 10.15 (s, 1H), 7.70 - 7.59 (m, 2H), 7.44 - 7.34 (m, 4H), 7.33 - 7.27 (m, 4H), 7.25 - 7.20 (m, 2H), 5.48 (s, 1H), 5.13 - 5.06 (m, 1H), 4.55 - 4.37 (m, 4H), 3.16 - 3.09 (m, 1H), 3.01 - 2.96 (m, 2H), 2.92 - 2.87 (m, 2H), 2.77 - 2.69 (m, 2H), 2.60 (d, J = 16.4 Hz, 1H), 2.40 - 2.38 (m, 1H), 2.07 - 1.95 (m, 1H), 1.21 (s, 3H), 1.02 (s, 3H). LCMS (ESI) calcd for $C_{33}H_{36}FN_4O_3^+$ [M+H]$^+$: 553.28, found, 555.3.

**Example 115: preparation of 3-(7-fluoro-1-oxo-5-((4-(phenyl(pyridin-3-yl)methyl)piperazin-1-yl)methyl)isoindolin-2-yl)piperidine-2,6-dione (GT-06111)**

Referring to the method of Scheme 4, the target product (GT-06111) was prepared as white solid (34 mg, yield 44%). LCMS (ESI) calcd for $C_{30}H_{31}FN_5O_3^+$ [M+H]$^+$: 528.24, found, 528.3.

**Example 116: preparation of 3-(1-oxo-4-((4-(phenyl(pyridin-3-yl)methyl)piperazin-1-yl)methyl)isoindolin-2-yl) piperidine-2,6-dione (GT-06113)**

Referring to the method of Scheme 4, the target product (GT-06113) was prepared as white solid (25 mg, yield 34%). $^1$H NMR (400 MHz, DMSO-d6) δ 11.06 (s, 1H), 8.97 (s, 1H), 8.78 (d, J = 5.3 Hz, 1H), 8.53 (d, J = 8.0 Hz, 1H), 7.98 - 7.93 (m, 2H), 7.81 (d, J = 7.4 Hz, 1H), 7.61 (t, J = 7.6 Hz, 1H), 7.49 (d, J = 7.5 Hz, 2H), 7.39 (t, J = 7.5 Hz, 2H), 7.30 (t, J = 7.3 Hz, 1H), 5.17 (dd, J = 13.2, 5.0 Hz, 1H), 5.00 (s, 1H), 4.89 (dd, J = 17.6, 4.0 Hz, 1H), 4.52 (d, J = 17.5 Hz, 1H), 4.39 (s, 2H), 3.36 - 3.24 (m, 4H), 3.02 - 2.90 (m, 2H), 2.86 - 2.81 (m, 2H), 2.65 (d, J = 17.3 Hz, 2H), 2.36 - 2.30 (m, 1H), 2.07 - 2.02 (m, 1H). LCMS (ESI) calcd for $C_{30}H_{32}N_5O_3^+$ [M+H]$^+$: 510.25, found, 510.3.

**Example 117: preparation of 3-(7-fluoro-1-oxo-5-((4-(phenyl(pyridin-4-yl)methyl)piperazin-1-yl)methyl)isoindolin-2-yl)piperidine-2,6-dione (GT-06135)**

Referring to the method of Scheme 4, the target product (GT-06135) was prepared as white solid (17 mg, yield 17%). $^1$H NMR (400 MHz, DMSO-d6) δ 11.50 (s, 1H), 11.02 (s, 1H), 8.84 (d, J = 6.3 Hz, 1H), 8.10 (d, J = 6.4 Hz, 1H), 7.76 - 7.53 (m, 3H), 7.46 (d, J = 7.6 Hz, 2H), 7.42 - 7.34 (m, 3H), 7.33 - 7.21 (m, 1H), 5.10 (dd, J = 13.3, 5.1 Hz, 1H), 4.99 (s, 1H), 4.62 - 4.33 (m, 5H), 3.11 - 3.01 (m, 2H), 2.95 - 2.87 (m, 2H), 2.79 - 2.76 (m, 2H), 2.64 - 2.60(m, 2H), 2.41 - 2.31 (m, 1H), 2.28 - 2.25 (m, 1H), 2.09 - 1.95 (m, 1H). LCMS (ESI) calcd for $C_{30}H_{31}FN_5O_3^+$ [M+H]$^+$: 528.24, found, 528.3.

**Example 118: preparation of 3-(1-oxo-4-((4-(phenyl(pyridin-4-yl)methyl)piperazin-1-yl)methyl)isoindolin-2-yl) piperidine-2,6-dione (GT-04945)**

Referring to the method of Scheme 4, the target product (GT-04945) was prepared as white solid (32 mg, yield 34%). $^1$H NMR (400 MHz, DMSO-d6) δ 11.06 (s, 1H), 8.87 (d, J = 6.4 Hz, 2H), 8.14 (d, J = 6.3 Hz, 2H), 8.00 (dd, J = 7.5, 4.5 Hz, 1H), 7.81 (d, J = 7.5 Hz, 1H), 7.61 (t, J = 7.6 Hz, 1H), 7.48 (d, J = 7.5 Hz, 2H), 7.39 (t, J = 7.5 Hz, 2H), 7.31 (t, J = 7.3 Hz, 1H), 5.17 (dd, J = 12.9, 4.9 Hz, 1H), 5.04 (s, 1H), 4.93 (dd, J = 17.6, 4.1 Hz, 1H), 4.52 (d, J = 17.7 Hz, 1H), 4.47 - 4.35 (m, 2H), 3.33 - 3.22 (m, 4H), 3.06 - 2.90 (m, 2H), 2.81 - 2.72 (m, 2H), 2.65 (d, J = 17.6 Hz, 2H), 2.43 - 2.29 (m, 1H), 2.09 - 2.02 (m, 1H). LCMS (ESI) calcd for $C_{30}H_{32}N_5O_3^+$ [M+H]$^+$: 510.25, found, 510.3.

**Example 119: preparation of 3-(5-((4-(di(pyridin-2-yl)methyl)piperazin-1-yl)methyl)-4-fluoro-1-oxoisoindolin-2-yl)piperidine-2,6-dione (GT-05713)**

Referring to the method of Scheme 4, the target product (GT-05713) was prepared as white solid (38 mg, yield 62%). $^1$H NMR (400 MHz, DMSO-d6) δ 11.04 (s, 1H), 8.71 (d, *J* = 5.1 Hz, 2H), 8.16 (t, J = 7.8 Hz, 2H), 7.99 - 7.92 (m, 1H), 7.84 (d, *J*= 7.9 Hz, 2H), 7.71 - 7.60 (m, 3H), 5.84 (s, 1H), 5.15 (dd, *J* = 13.2, 5.1 Hz, 2H), 4.62 - 4.40 (m, 5H), 3.51 - 3.43 (m, 5H), 3.08 - 2.85 (m, 5H), 2.61 (d, *J* = 17.2 Hz, 1H), 2.49 - 2.44 (m, 1H), 2.07 - 1.94 (m, 1H). LCMS (ESI) calcd for $C_{20}H_{30}FN_6O_3^+$ [M+H]$^+$: 529.24, found, 529.3.

**Example 120: preparation of 3-(5-((4-(di(pyridin-2-yl)methyl)piperazin-1-yl)methyl)-7-fluoro-1-oxoisoindolin-2-yl)piperidine-2,6-dione (GT-05714)**

Referring to the method of Scheme 4, the target product (GT-05714) was prepared as white solid (33 mg, yield 54%). $^1$H NMR (400 MHz, DMSO-d6) δ 11.02 (s, 1H), 8.70 (d, *J* = 4.3 Hz, 2H), 8.18 - 8.10 (m, 2H), 7.83 (d, *J* = 7.9 Hz, 2H), 7.70 - 7.67 (m, 2H), 7.64 - 7.60 (m, 2H), 5.80 (s, 1H), 5.10 (dd, *J* = 13.3, 5.1 Hz, 1H), 4.56 - 4.42 (m, 3H), 3.52 - 3.42 (m, 1H), 3.37 (s, 4H), 2.99 (s, 4H), 2.94 - 2.82 (m, 1H), 2.60 (d, *J* = 16.8 Hz, 1H), 2.47 - 2.34 (m, 1H), 2.08 - 1.99 (m, 1H). LCMS (ESI) calcd for $C_{29}H_{30}FN_6O_3^+$ [M+H]$^+$: 529.24, found, 529.3.

**Example 121: preparation of 3-(4-((4-(di(pyridin-2-yl)methyl)piperazin-1-yl)methyl)-1-oxoisoindolin-2-yl)piperidine-2,6-dione (GT-05715)**

Referring to the method of Scheme 4, the target product (GT-05715) was prepared as white solid (38 mg, yield 64%). $^1$H NMR (400 MHz, DMSO-d6) δ 11.06 (s, 1H), 8.71 (d, *J* = 5.1 Hz, 2H), 8.17 - 8.13 (m, 2H), 7.98 (d, *J* = 7.5 Hz, 1H), 7.86 - 7.81 (m, 3H), 7.67 - 7.57 (m, 3H), 5.86 (s, 1H), 5.17 (dd, *J* = 13.2, 5.2 Hz, 1H), 4.95 (d, *J* = 17.7 Hz, 1H), 4.67 (d, *J* = 20.6 Hz, 3H), 3.61 - 3.24 (m, 5H), 3.00 (s, 4H), 2.98 - 2.87 (m, 1H), 2.65 (d, *J* = 16.7 Hz, 1H), 2.40 - 2.29 (m, 1H), 2.08 - 2.02 (m, 1H). LCMS (ESI) calcd for $C_{29}H_{31}N_6O_3^+$ [M+H]$^+$: 511.25, found, 511.3.

**Example 122: preparation of 3-(5-((4-(di(pyridin-3-yl)methyl)piperazin-1-yl)methyl)-4-fluoro-1-oxoisoindolin-2-yl)piperidine-2,6-dione (GT-05716)**

Referring to the method of Scheme 4, the target product (GT-05716) was prepared as white solid (15 mg, yield 19%). $^1$H NMR (400 MHz, DMSO) δ 11.03 (s, 1H), 8.87 (s, 2H), 8.71 (d, *J* = 5.0 Hz, 2H), 8.27 (d, *J* = 7.0 Hz, 2H), 7.95 - 7.84 (m, 1H), 7.78 - 7.74 (m, 2H), 7.73 - 7.67 (m, 1H), 5.17 - 5.15 (m, 2H), 4.61 - 4.46 (m, 4H), 3.29 - 3.19 (m, 4H), 3.01 - 2.79 (m, 4H), 2.61 (d, *J* = 17.3 Hz, 2H), 2.35 - 2.22 (m, 1H), 2.07 - 1.95 (m, 1H). LCMS (ESI) calcd for $C_{29}H_{30}FN_6O_3^+$ [M+H]$^+$: 529.24, found, 529.3.

**Example 123: preparation of 3-(5-((4-(di(pyridin-3-yl)methyl)piperazin-1-yl)methyl)-7-fluoro-1-oxoisoindolin-2-yl)piperidine-2,6-dione (GT-05717)**

Referring to the method of Scheme 4, the target product (GT-05717) was prepared as white solid (27 mg, yield 35%). $^1$H NMR (400 MHz, DMSO-d6) δ 11.01 (s, 1H), 9.39 (s, 1H), 9.14 (d, *J* = 6.0 Hz, 1H), 8.75 (s, 1H), 8.66 (d, *J* = 6.2 Hz, 2H), 8.22 - 8.13 (m, 1H), 8.00 (d, *J* = 8.5 Hz, 1H), 7.66 - 7.50 (m, 3H), 5.24 (s, 1H), 5.09 (dd, *J* = 13.2, 5.1 Hz, 1H), 4.43 (dd, *J* = 53.7, 17.8 Hz, 2H), 3.64 - 3.60 (m, 4H), 3.17 - 3.13 (m, 4H), 2.62 - 2.59 (m, 4H), 2.44 - 2.36 (m, 1H), 2.09 - 1.90 (m, 1H). LCMS (ESI) calcd for $C_{20}H_{30}FN_6O_3^+$ [M+H]$^+$: 529.24, found, 529.3.

**Example 124: preparation of 3-(4-((4-(di(pyridin-3-yl)methyl)piperazin-1-yl)methyl)-1-oxoisoindolin-2-yl)piperidine-2,6-dione (GT-05718)**

Referring to the method of Scheme 4, the target product (GT-05718) was prepared as white solid (40 mg, yield 53%). $^1$H NMR (400 MHz, DMSO-d6) δ 11.06 (s, 1H), 9.34 (s, 1H), 9.05 (d, *J* = 6.0 Hz, 1H), 8.84 (brs, 2H), 8.22 - 8.14 (m, 1H), 7.96 (s, 2H), 7.82 (d, *J*= 7.2 Hz, 1H), 7.63 - 7.49 (m, 3H), 5.21 - 5.13 (m, 1H), 4.62 - 4.40 (m, 1H), 3.52 - 3.47 (m, 4H), 3.32 - 3.29(m, 4H), 3.11 - 3.08(m, 4H), 2.96 - 2.93 (m, 1H), 2.67 (dd, *J* = 18.2, 4.6 Hz, 1H), 2.39 - 2.32 (m, 1H), 2.07 - 1.96 (m, 1H). LCMS (ESI) calcd for $C_{29}H_{31}N_6O_3^+$ [M+H]$^+$: 511.25, found, 511.3.

**Example 125: preparation of 3-(5-((4-((4-bromophenyl)(phenyl)methyl)piperazin-1-yl)methyl)-1-oxoisoindolin-2-yl)piperidine-2,6-dione (GT-06640)**

Referring to the method of Scheme 4, the target product (GT-06640) was prepared as white solid (157 mg, yield 74%). $^1$H NMR (400 MHz, DMSO-d6) δ 11.01 (s, 1H), 7.85 (s, 1H), 7.80 (d, J = 7.8 Hz, 1H), 7.73 (d, J = 7.8 Hz, 1H), 7.53 (d, J = 7.1 Hz, 2H), 7.48 - 7.39 (m, 3H), 7.34 (t, J = 7.1 Hz, 3H), 7.26 (d, J = 6.8 Hz, 1H), 5.14 (dd, J = 13.3, 5.1 Hz, 1H), 4.61 - 4.28 (m, 5H), 3.52 - 3.42 (m, 2H), 3.39 - 3.23 (m, 3H), 3.23 - 3.04 (m, 2H), 2.94 - 2.88 (m, 2H), 2.61 (d, J = 17.3 Hz, 1H), 2.48 - 2.40 (m, 1H), 2.03 - 1.99 (m, 1H). LCMS (ESI) calcd for $C_{31}H_{32}BrN_4O_3^+$ [M+H]$^+$: 587.17, found, 587.2.

**Example 126: preparation of 3-(6-((4-(bis(4-fluorophenyl)methyl)piperazin-1-yl)methyl)-1-oxoisoindolin-2-yl)piperidine-2,6-dione (GT-05719)**

Referring to the method of Scheme 4, the target product (GT-05719) was prepared as white solid (72 mg, yield 89%). $^1$H NMR (400 MHz, DMSO-d6) δ 11.00 (s, 1H), 7.97 (s, 1H), 7.83 (d, *J* = 7.9 Hz, 1H), 7.69 (d, *J* = 7.8 Hz, 1H), 7.48 (s, 4H), 7.17 (t, *J* = 8.3 Hz, 4H), 5.12 (dd, *J* = 13.3, 5.1 Hz, 1H), 4.56 - 4.32 (m, 4H), 3.47 - 3.42 (m, 2H), 3.33 - 3.27 (m, 2H), 3.19 - 3.09 (m, 2H), 2.99 - 2.78 (m, 3H), 2.61 (d, *J* = 17.1 Hz, 1H), 2.47 - 2.36 (m, 2H), 2.08 - 1.91 (m, 1H). LCMS (ESI) calcd for $C_{31}H_{31}F_2N_4O_3^+$ [M+H]$^+$: 545.24, found, 545.3.

**Example 127: preparation of 3-(7-((4-(bis(4-fluorophenyl)methyl)piperazin-1-yl)methyl)-1-oxoisoindolin-2-yl)piperidine-2,6-dione (GT-05720)**

Referring to the method of Scheme 4, the target product (GT-05720) was prepared as white solid (28 mg, yield

35%). $^1$H NMR (400 MHz, DMSO-d6) δ 11.04 (s, 1H), 7.72 (s, 3H), 7.49 (s, 4H), 7.17 (t, *J* = 8.4 Hz, 4H), 5.14 (dd, *J* = 13.2, 5.2 Hz, 1H), 4.87 (d, *J* = 12.6 Hz, 1H), 4.73 (d, *J* = 12.3 Hz, 1H), 4.45 (dd, *J* = 44.0, 17.9 Hz, 2H), 3.45 - 3.34 (m, 4H), 3.27 - 3.12 (m, 2H), 3.01 - 2.74 (m, 3H), 2.64 (t, *J* = 14.4 Hz, 1H), 2.46 - 2.33 (m, 2H), 2.09 - 1.94 (m, 1H). LCMS (ESI) calcd for $C_{31}H_{31}F_2N_4O_3^+$ [M+H]+: 545.24, found, 545.3.

**Example 128: preparation of 3-(6-((4-(bis(3-fluorophenyl)methyl)piperazin-1-yl)methyl)-1-oxoisoindolin-2-yl)piperidine-2,6-dione (GT-06116)**

Referring to the method of Scheme 4, the target product (GT-06116) was prepared as white solid (71 mg, yield 79%). $^1$H NMR (400 MHz, DMSO-d6) δ 11.06 (s, 1H), 10.64 (s, 1H), 8.03 (s, 1H), 7.89 (dd, J = 7.9, 1.3 Hz, 1H), 7.76 (d, J = 7.8 Hz, 1H), 7.49 - 7.40 (m, 2H), 7.36 (t, J = 8.5 Hz, 4H), 7.13 (td, J = 8.6, 2.0 Hz, 2H), 5.18 (dd, J = 13.2, 5.1 Hz, 1H), 4.71 (brs, 1H), 4.64 - 4.38 (m, 4H), 3.23 - 3.18 (m, 4H), 3.02 - 2.84 (m, 3H), 2.67 (d, J = 16.5 Hz, 1H), 2.54 - 2.36 (m, 3H), 2.15 - 1.99 (m, 1H). LCMS (ESI) calcd for $C_{31}H_{31}F_2N_4O_3^+$ [M+H]+: 545.24, found, 545.3.

**Example 129: preparation of 3-(7-((4-(bis(3-fluorophenyl)methyl)piperazin-1-yl)methyl)-1-oxoisoindolin-2-yl)piperidine-2,6-dione (GT-06117)**

Referring to the method of Scheme 4, the target product (GT-06117) was prepared as white solid (45 mg, yield 50%). $^1$H NMR (400 MHz, DMSO-d6) δ 11.05 (s, 1H), 10.43 (s, 1H), 7.82 - 7.66 (m, 3H), 7.47 - 7.23 (m, 6H), 7.10 - 7.05 (m, 2H), 5.15 (dd, J = 13.3, 5.0 Hz, 1H), 4.89 (d, J = 12.8 Hz, 1H), 4.74 (d, J = 12.9 Hz, 1H), 4.68 (s, 1H), 4.46 (dd, J = 44.4, 17.9 Hz, 2H), 3.31 - 3.19 (m, 4H), 3.02 - 2.78 (m, 3H), 2.63 (d, J = 16.9 Hz, 1H), 2.48 - 2.37 (m, 3H), 2.04 - 2.00 (m, 1H). LCMS (ESI) calcd for $C_{31}H_{31}F_2N_4O_3^+$ [M+H]+: 545.24, found, 545.3.

**Example 130: preparation of 3-(5-((4-(bis(2-fluorophenyl)methyl)piperazin-1-yl)methyl)-1-oxoisoindolin-2-yl)piperidine-2,6-dione (GT-06084)**

Referring to the method of Scheme 4, the target product (GT-06084) was prepared as white solid (54 mg, yield 60%). $^1$H NMR (400 MHz, DMSO-d6) δ 11.01 (s, 1H), 7.84 (s, 1H), 7.80 (d, J = 7.8 Hz, 1H), 7.73 (t, J = 7.3 Hz, 1H), 7.58 - 7.55 (m, 2H), 7.35 - 7.30 (m, 2H), 7.24 - 7.14 (m, 4H), 5.16 - 5.11 (m, 2H), 4.55 - 4.30 (m, 4H), 3.39 - 3.31 (m, 4H), 3.17 - 3.12 (m, 2H), 2.99 - 2.77 (m, 3H), 2.61 (d, J = 16.7 Hz, 2H), 2.46 - 2.36 (m, 1H), 2.03 - 1.99 (m, 1H). LCMS (ESI) calcd for $C_{31}H_{31}F_2N_4O_3^+$ [M+H]+: 545.24, found, 545.3.

**Example 131: preparation of 3-(5-((4-(bis(2-fluorophenyl)methyl)piperazin-1-yl)methyl)-4-fluoro-1-oxoisoindolin-2-yl)piperidine-2,6-dione (GT-06085)**

Referring to the method of Scheme 4, the target product (GT-06085) was prepared as white solid (46 mg, yield 50%). $^1$H NMR (400 MHz, DMSO-d6) δ 11.03 (s, 1H), 7.93 - 7.82 (m, 1H), 7.68 (d, J = 7.7 Hz, 1H), 7.63 - 7.50 (m, 2H), 7.35 - 7.31 (m, 2H), 7.24 - 7.15 (m, 4H), 5.14 (dd, J = 13.2, 4.9 Hz, 2H), 4.68 - 4.37 (m, 4H), 3.42 - 3.31 (m, 4H), 3.27 - 3.15 (m, 2H), 2.92 - 2.81 (m, 2H), 2.61 (d, J = 17.7 Hz, 2H), 2.48 - 2.36 (m, 1H), 2.10 - 1.92 (m, 1H). LCMS (ESI) calcd for $C_{31}H_{30}F_3N_4O_3^+$ [M+H]+: 563.23, found, 563.3.

**Example 132: preparation of 3-(5-((4-(bis(2-fluorophenyl)methyl)piperazin-1-yl)methyl)-6-fluoro-1-oxoisoindolin-2-yl)piperidine-2,6-dione (GT-06086)**

Referring to the method of Scheme 4, the target product (GT-06086) was prepared as white solid (26 mg, yield 28%). $^1$H NMR (400 MHz, DMSO-d6) δ 11.02 (s, 1H), 7.98 (d, J = 6.0 Hz, 1H), 7.66 (d, J = 8.4 Hz, 1H), 7.60 - 7.57 (m, 2H), 7.35 - 7.31 (m, 2H), 7.24 - 7.15 (m, 4H), 5.14 (dd, J = 13.0, 5.1 Hz, 2H), 4.55 - 4.29 (m, 4H), 3.45 - 3.30 (m, 4H), 3.27 - 3.17 (m, 2H), 2.96 - 2.77 (m, 3H), 2.61 (d, J = 17.3 Hz, 1H), 2.46 - 2.38 (m, 1H), 2.04 - 2.00 (m, 1H). LCMS (ESI) calcd for $C_{31}H_{30}F_3N_4O_3^+$ [M+H]+: 563.23, found, 563.3.

**Example 133: preparation of 3-(5-((4-(bis(2-fluorophenyl)methyl)piperazin-1-yl)methyl)-7-fluoro-1-oxoisoindolin-2-yl)piperidine-2,6-dione (GT-06087)**

Referring to the method of Scheme 4, the target product (GT-06087) was prepared as white solid (49 mg, yield 53%). $^1$H NMR (400 MHz, DMSO-d6) δ 11.02 (s, 1H), 7.65 (s, 1H), 7.62 - 7.56 (m, 3H), 7.35 - 7.30 (m, 2H), 7.24 - 7.15 (m, 4H), 5.12 - 5.07 (m, 2H), 4.58 - 4.33 (m, 4H), 3.36 - 3.27 (m, 4H), 3.16 - 3.08 (m, 2H), 2.98 - 2.80 (m, 3H), 2.62 - 2.58 (m, 1H), 2.45 - 2.34 (m, 1H), 2.03 - 1.99 (m, 1H). LCMS (ESI) calcd for $C_{31}H_{30}F_3N_4O_3^+$ [M+H]+: 563.23, found, 563.2.

**Example 134: preparation of 3-(5-((4-(bis(4-(trifluoromethyl)phenyl)methyl)piperazin-1-yl)methyl)-1-oxoisoindolin-2-yl)piperidine-2,6-dione (GT-06199)**

Referring to the method of Scheme 4, the target product (GT-06199) was prepared as white solid (50 mg, yield 62%). $^1$H NMR (400 MHz, DMSO-d6) δ 11.01 (s, 1H), 10.71 (s, 1H), 7.85 - 7.78 (m, 2H), 7.73 - 7.67 (m, 9H), 5.14 (dd, J = 13.2, 5.1 Hz, 1H), 4.85 (s, 1H), 4.55 - 4.31 (m, 4H), 3.24 - 3.06 (m, 3H), 3.00 - 2.87 (m, 2H), 2.83 - 2.80 (m, 2H), 2.61 (d, J = 16.4 Hz, 1H), 2.48 - 2.36 (m, 3H), 2.04 - 2.00 (m, 1H). LCMS (ESI) calcd for $C_{33}H_{31}F_6N_4O_3^+$ [M+H]$^+$: 645.23, found, 645.3.

**Example 135: preparation of 3-(5-((4-(bis(4-(trifluoromethyl)phenyl)methyl)piperazin-1-yl)methyl)-4-fluoro-1-oxoisoindolin-2-yl)piperidine-2,6-dione (GT-06200)**

Referring to the method of Scheme 4, the target product (GT-06200) was prepared as white solid (50 mg, yield 61%). $^1$H NMR (400 MHz, DMSO-d6) δ 11.04 (s, 1H), 10.61 (s, 1H), 7.84 (t, J = 7.0 Hz, 1H), 7.77 - 7.63 (m, 10H), 5.15 (dd, J = 13.2, 5.1 Hz, 1H), 4.86 (s, 1H), 4.64 - 4.39 (m, 4H), 3.32 - 3.15 (m, 4H), 2.97 - 2.90 (m, 1H), 2.85 - 2.82 (m, 2H), 2.62 (d, J = 16.6 Hz, 1H), 2.49 - 2.34 (m, 3H), 2.07 - 1.95 (m, 1H). LCMS (ESI) calcd for $C_{33}H_{30}F_7N_4O_3^+$ [M+H]$^+$: 663.22, found, 663.2.

**Example 136: preparation of 3-(5-((4-(bis(4-(trifluoromethyl)phenyl)methyl)piperazin-1-yl)methyl)-6-fluoro-1-oxoisoindolin-2-yl)piperidine-2,6-dione (GT-06201)**

Referring to the method of Scheme 4, the target product (GT-06201) was prepared as white solid (33 mg, yield 40%). $^1$H NMR (400 MHz, DMSO-d6) δ 11.03 (s, 1H), 10.62 (s, 1H), 7.92 (d, J = 6.0 Hz, 1H), 7.71 - 7.61(m, 10H), 5.14 (dd, J = 13.3, 5.1 Hz, 1H), 4.85 (s, 1H), 4.51 - 4.34 (m, 4H), 3.28 - 3.10 (m, 4H), 3.00 - 2.76 (m, 3H), 2.61 (d, J = 16.9 Hz, 1H), 2.45 - 2.36 (m, 3H), 2.07 - 2.01 (m, 1H). LCMS (ESI) calcd for $C_{33}H_{30}F_7N_4O_3^+$ [M+H]$^+$: 663.22, found, 663.2.

**Example 137: preparation of 3-(5-((4-(bis(4-(trifluoromethyl)phenyl)methyl)piperazin-1-yl)methyl)-7-fluoro-1-oxoisoindolin-2-yl)piperidine-2,6-dione (GT-06202)**

Referring to the method of Scheme 4, the target product (GT-06202) was prepared as white solid (44 mg, yield 53%). $^1$H NMR (400 MHz, DMSO-d6) δ 11.02 (s, 1H), 10.93 (s, 1H), 7.73 - 7.65 (m, 9H), 7.64 (s, 1H), 7.59 (d, J = 10.0 Hz, 1H), 5.10 (dd, J = 13.2, 5.1 Hz, 1H), 4.86 (s, 1H), 4.58 - 4.31 (m, 4H), 3.31 - 3.15 (m, 4H), 2.95 - 2.81 (m, 3H), 2.61 (d, J = 17.0 Hz, 1H), 2.48 - 2.31 (m, 3H), 2.08 - 1.92 (m, 1H). LCMS (ESI) calcd for $C_{33}H_{30}F_7N_4O_3^+$ [M+H]$^+$: 663.22, found, 663.2.

**Example 138: preparation of 3-(5-((4-(bis(3,5-difluorophenyl)methyl)piperazin-1-yl)methyl)-1-oxoisoindolin-2-yl)piperidine-2,6-dione (GT-06387)**

Referring to the method of Scheme 4, the target product (GT-06387) was prepared as white solid (24 mg, yield 40%). $^1$H NMR (400 MHz, DMSO-d6) δ 10.94 (s, 1H), 10.45 (s, 1H), 7.78 - 7.72 (m, 2H), 7.63 (d, J = 8.2 Hz, 1H), 7.15 - 7.12 (m, 4H), 7.09 - 7.04 (m, 2H), 5.07 (dd, J = 13.4, 5.1 Hz, 1H), 4.58 (s, 1H), 4.49 - 4.26 (m, 4H), 3.22 - 3.00 (m, 4H), 2.81 - 2.76 (m, 3H), 2.54 (d, J = 16.4 Hz, 1H), 2.39 - 2.26 (m, 3H), 2.00 - 1.88 (m, 1H). LCMS (ESI) calcd for $C_{31}H_{29}F_4N_4O_3^+$ [M+H]$^+$: 581.22, found, 581.2.

**Example 139: preparation of 3-(6-((4-(bis(4-chlorophenyl)methyl)piperazin-1-yl)methyl)-1-oxoisoindolin-2-yl) piperidine-2,6-dione (GT-05735)**

Referring to the method of Scheme 4, the target product (GT-05735) was prepared as white solid (73 mg, yield 95%). $^1$H NMR (400 MHz, DMSO-d6) δ 11.00 (s, 1H), 7.96 (s, 1H), 7.82 (d, *J* = 7.9 Hz, 1H), 7.70 (d, *J* = 7.8 Hz, 1H), 7.49 - 7.35 (m, 8H), 5.12 (dd, *J* = 13.3, 5.1 Hz, 1H), 4.60 (brs, 1H), 4.56 - 4.32 (m, 4H), 3.31 - 3.28 (m, 2H), 3.13 (brs, 2H), 2.99 - 2.78 (m, 3H), 2.61 (d, *J* = 16.0 Hz, 1H), 2.47 - 2.28 (m, 3H), 2.03 - 1.99 (m, 1H). LCMS (ESI) calcd for $C_{31}H_{31}Cl_2N_4O_3^+$ [M+H]$^+$: 577.18, found, 577.2.

**Example 140: preparation of 3-(7-((4-(bis(4-chlorophenyl)methyl)piperazin-1-yl)methyl)-1-oxoisoindolin-2-yl) piperidine-2,6-dione (GT-05736)**

Referring to the method of Scheme 4, the target product (GT-05736) was prepared as white solid (25 mg, yield 33%). $^1$H NMR (400 MHz, DMSO-d6) δ 11.04 (s, 1H), 10.04 (s, 1H), 7.77 - 7.69 (m, 2H), 7.69 - 7.61 (m, 1H), 7.45 - 7.38 (m, 8H), 5.14 (dd, *J*= 13.2, 5.0 Hz, 1H), 4.87 (d, *J* = 12.3 Hz, 1H), 4.73 (d, *J* = 13.7 Hz, 1H), 4.59 (s, 1H), 4.53 - 4.38 (m, 2H), 3.30 - 3.12 (m, 4H), 2.98 - 2.86 (m, 1H), 2.81 - 2.76 (m, 2H), 2.65 - 2.59 (m, 1H), 2.44 - 2.40 (m, 1H), 2.38 - 2.23 (m, 2H), 2.03 - 1.97 (m, 1H). LCMS (ESI) calcd for $C_{31}H_{31}Cl_2N_4O_3^+$ [M+H]$^+$: 577.18, found, 577.2.

**Example 141: preparation of 3-(5-((4-(2,7-dichloro-9H-fluoren-9-yl)piperazin-1-yl)methyl)-1-oxoisoindolin-2-yl)piperidine-2,6-dione (GT-06048)**

Referring to the method of Scheme 4, the target product (GT-06048) was prepared as white solid (44 mg, yield 51%). $^1$H NMR (400 MHz, DMSO-d6) δ 11.01 (s, 1H), 7.91 (d, J = 8.1 Hz, 2H), 7.82 (d, J = 7.6 Hz, 1H), 7.77 (s, 1H), 7.67 (d, J = 8.4 Hz, 1H), 7.61 (s, 2H), 7.51 (d, J = 8.1 Hz, 2H), 5.12 (d, J = 11.9 Hz, 2H), 4.60 - 4.32 (m, 4H), 3.13 - 3.04 (m, 3H), 2.95 - 2.84 (m, 4H), 2.78 - 2.72 (m, 2H), 2.63 - 2.59 (m, 1H), 2.45 - 2.34 (m, 1H), 2.03 - 1.99 (m, 1H). LCMS (ESI) calcd for $C_{31}H_{29}Cl_2N_4O_3^+$ [M+H]$^+$: 575.16, found, 575.2.

**Example 142: preparation of 3-(5-((4-(9H-fluoren-9-yl)piperazin-1-yl)methyl)-1-oxoisoindolin-2-yl)piperidine-2,6-dione (GT-05907)**

Referring to the method of Scheme 4, the target product (GT-05907) was prepared as white solid (18 mg, yield 19%). $^1$H NMR (400 MHz, DMSO-d6) δ 11.00 (s, 1H), 7.88 (d, J = 7.5 Hz, 2H), 7.82 (s, 1H), 7.79 (d, J = 7.8 Hz, 1H), 7.75 (d, J = 6.3 Hz, 2H), 7.71 (d, J = 8.0 Hz, 1H), 7.47 (t, J = 7.4 Hz, 2H), 7.37 (t, J = 7.2 Hz, 2H), 5.28 (s, 1H), 5.13 (dd, J = 13.3, 5.1 Hz, 1H), 4.54 - 4.30 (m, 4H), 3.34 - 3.32 (m, 2H), 3.27 - 3.17 (m, 2H), 3.12 - 3.02 (m, 2H), 3.00 - 2.84 (m, 3H), 2.60 (d, J = 17.2 Hz, 1H), 2.42 (dd, J = 13.2, 4.5 Hz, 1H), 2.03 - 1.98 (m, 1H). LCMS (ESI) calcd for $C_{31}H_{31}N_4O_3^+$ [M+H]$^+$: 507.24, found, 507.2.

**Example 143: preparation of 3-(5-((4-(bis(4-(benzyloxy)phenyl)methyl)piperazin-1-yl)methyl)-1-oxoisoindolin-2-yl)piperidine-2,6-dione (GT-06424)**

Referring to the method of Scheme 4, the target product (GT-06424) was prepared as white solid (46 mg, yield 61%). $^1$H NMR (400 MHz, DMSO-d6) δ 10.94 (s, 1H), 7.80 - 7.67 (m, 2H), 7.66 - 7.55 (m, 1H), 7.35 - 7.27 (m, 10H), 7.25 - 7.21 (m, 3H), 7.16 (d, J = 8.6 Hz, 1H), 6.95 - 6.90 (m, 3H), 6.85 (d, J = 8.7 Hz, 1H), 5.06 (dd, J = 13.2, 5.1 Hz, 1H), 4.99 (s, 4H), 4.46 - 4.25 (m, 4H), 3.17 - 2.97 (m, 4H), 2.95 - 2.69 (m, 3H), 2.54 (d, J = 16.2 Hz, 1H), 2.42 - 2.34 (m, 4H), 2.00 - 1.90 (m, 1H). LCMS (ESI) calcd for $C_{45}H_{44}N_4NaO_5^+$ [M+Na]$^+$: 743.32, found, 743.4.

**Example 144: preparation of 3-(5-((4-(diphenylamino)piperidin-1-yl)methyl)-7-fluoro-1-oxoisoindolin-2-yl)piperidine-2,6-dione (GT-06133)**

Referring to the method of Scheme 4, the target product (GT-06133) was prepared as white solid (35 mg, yield 57%). $^1$H NMR (400 MHz, DMSO-d6) δ 10.95 (s, 1H), 9.88 (s, 1H), 7.51 (s, 1H), 7.45 (d, J = 10.1 Hz, 1H), 7.23 (t, J = 7.9 Hz, 4H), 6.94 (t, J = 7.3 Hz, 2H), 6.75 (d, J = 7.6 Hz, 4H), 5.03 (dd, J = 13.3, 5.1 Hz, 1H), 4.50 - 4.23 (m, 4H), 4.18 - 4.12 (m, 1H), 3.20 - 3.05 (m, 3H), 2.92 - 2.75 (m, 1H), 2.54 (d, J = 17.1 Hz, 1H), 2.42 - 2.25 (m, 2H), 2.01 (d, J = 13.1 Hz, 2H), 1.97 - 1.86 (m, 1H), 1.65 - 1.47 (m, 2H). LCMS (ESI) calcd for $C_{31}H_{32}FN_4O_3^+$ [M+H]$^+$: 527.25, found, 527.2.

**Example 145: preparation of 3-(4-((4-(diphenylamino)piperidin-1-yl)methyl)-1-oxoisoindolin-2-yl)piperidine-2,6-dione (GT-04992)**

Referring to the method of Scheme 4, the target product (GT-04992) was prepared as white solid (15 mg, yield 20%). $^1$H NMR (400 MHz, DMSO) δ 11.07 (s, 1H), 9.70 (s, 1H), 7.86 - 7.80 (m, 2H), 7.63 (t, J = 7.6 Hz, 1H), 7.32 - 7.28 (m, 6H), 6.83 - 6.80 (m, 4H), 5.18 (dd, J = 13.2, 5.1 Hz, 2H), 4.66 (d, J = 17.3 Hz, 1H), 4.53 (d, J = 17.3 Hz, 1H), 4.47 (d, J = 7.8 Hz, 1H), 4.33 (d, J = 4.8 Hz, 2H), 4.26 - 4.21 (m, 1H), 2.91 - 2.81 (m, 2H), 2.65 (d, J = 15.8 Hz, 2H), 2.38 - 2.30 (m, 1H), 2.11 - 2.03 (m, 4H), 1.67 - 1.58 (m, 2H). LCMS (ESI) calcd for $C_{31}H_{33}N_4O_3^+$ [M+H]$^+$: 509.25, found, 509.3.

**Example 146: preparation of 3-(1-oxo-5-((4-(phenyl(pyridin-2-yl)amino)piperidin-1-yl)methyl)isoindolin-2-yl)piperidine-2,6-dione (GT-04525)**

Referring to the method of Scheme 4, the target product (GT-04525) was prepared as white solid (53 mg, yield 56%). $^1$H NMR (400 MHz, DMSO-d6) δ 11.01 (s, 1H), 10.59 (s, 1H), 8.09 (dd, *J* = 5.8, 1.2 Hz, 1H), 7.83 (s, 1H), 7.80 (d, *J* = 7.8 Hz, 1H), 7.72 (d, *J* = 7.7 Hz, 2H), 7.62 - 7.58 (m, 2H), 7.56 - 7.52 (m, 1H), 7.30 (d, *J* = 7.3 Hz, 2H), 7.00 - 6.80 (m, 1H), 6.29 (s, 1H), 5.13 (dd, *J*= 13.3, 5.1 Hz, 1H), 4.93 (t, *J* = 11.2 Hz, 1H), 4.51 - 4.34 (m, 4H), 3.55 - 3.41 (m, 2H), 3.26 - 3.18 (m, 3H), 3.02 - 2.83 (m, 1H), 2.61 (d, *J* = 16.9 Hz, 1H), 2.44 - 2.40 (m, 1H), 2.15 - 2.12 (m, 2H), 2.02 - 1.99 (m, 1H), 1.81 - 1.72 (m, 2H). LCMS (ESI) calcd for $C_{30}H_{32}N_5O_3^+$ [M+H]$^+$: 510.25, found, 510.3.

**Example 147: preparation of 3-(4-fluoro-1-oxo-5-((4-(phenyl(pyridin-2-yl)amino)piperidin-1-yl)methyl)isoindolin-2-yl)piperidine-2,6-dione (GT-05856)**

Referring to the method of Scheme 4, the target product (GT-05856) was prepared as white solid (56 mg, yield 57%). $^1H$ NMR (400 MHz, DMSO-d6) δ 11.03 (s, 1H), 10.44 (s, 1H), 8.09 (d, *J* = 5.3 Hz, 1H), 7.90 - 7.80 (m, 1H), 7.68 (d, *J* = 7.6 Hz, 2H), 7.60 (t, *J* = 7.5 Hz, 2H), 7.56 - 7.49 (m, 1H), 7.29 (d, *J* = 7.5 Hz, 2H), 6.88 (s, 1H), 6.28 (s, 1H), 5.15 (dd, *J* = 13.2, 5.0 Hz, 1H), 5.02 - 4.83 (m, 1H), 4.67 - 4.34 (m, 4H), 3.33 - 3.20 (m, 4H), 2.99 - 2.85 (m, 1H), 2.61 (d, *J* = 16.7 Hz, 2H), 2.48 - 2.36 (m, 1H), 2.15 - 2.12 (m, 2H), 2.07 - 1.95 (m, 1H), 1.78 - 1.69 (m, 2H). LCMS (ESI) calcd for $C_{30}H_{31}FN_5O_3^+$ [M+H]$^+$: 528.24, found, 528.3.

**Example 148: preparation of 3-(6-fluoro-1-oxo-5-((4-(phenyl(pyridin-2-yl)amino)piperidin-1-yl)methyl)isoindolin-2-yl)piperidine-2,6-dione (GT-05857)**

Referring to the method of Scheme 4, the target product (GT-05857) was prepared as white solid (57 mg, yield 58%). $^1H$ NMR (400 MHz, DMSO-d6) δ 11.02 (s, 1H), 10.52 (s, 1H), 8.09 (d, *J* = 5.5 Hz, 1H), 7.95 (d, *J* = 5.9 Hz, 1H), 7.67 (d, *J*= 8.4 Hz, 2H), 7.59 (t, *J*= 7.5 Hz, 2H), 7.56 - 7.50 (m, 1H), 7.29 (d, *J*= 7.5 Hz, 2H), 6.88 (s, 1H), 6.29 (s, 1H), 5.14 (dd, *J* = 13.2, 5.1 Hz, 1H), 4.98 - 4.90 (m, 1H), 4.57 - 4.29 (m, 4H), 3.31 - 3.20 (m, 4H), 2.99 - 2.83 (m, 1H), 2.61 (d, *J* = 16.4 Hz, 1H), 2.44 - 2.40 (m, 1H), 2.15 - 2.12 (m, 2H), 2.08 - 1.94 (m, 1H), 1.78 - 1.69 (m, 2H). LCMS (ESI) calcd for $C_{30}H_{31}FN_5O_3^+$ [M+H]$^+$: 528.24, found, 528.3.

**Example 149: preparation of 3-(7-fluoro-1-oxo-5-((4-(phenyl(pyridin-2-yl)amino)piperidin-1-yl)methyl)isoindolin-2-yl)piperidine-2,6-dione (GT-05858)**

Referring to the method of Scheme 4, the target product (GT-05858) was prepared as white solid (61 mg, yield 62%). $^1H$ NMR (400 MHz, DMSO-d6) δ 11.02 (s, 1H), 10.36 (s, 1H), 8.10 (d, *J* = 4.4 Hz, 1H), 7.74 - 7.47 (m, 6H), 7.28 (d, *J* = 7.3 Hz, 2H), 6.84 (s, 1H), 6.21 (s, 1H), 5.10 (dd, *J* = 13.3, 5.1 Hz, 1H), 4.94 (t, *J* = 9.8 Hz, 1H), 4.59 - 4.33 (m, 4H), 3.26 - 3.17 (m, 4H), 2.96 - 2.87 (m, 1H), 2.60 (d, *J* = 17.0 Hz, 1H), 2.46 - 2.34 (m, 1H), 2.12 (d, *J* = 13.2 Hz, 2H), 2.06 - 1.94 (m, 1H), 1.77 - 1.68 (m, 2H). LCMS (ESI) calcd for $C_{30}H_{31}FN_5O_3^+$ [M+H]$^+$: 528.24, found, 528.3.

**Example 150: preparation of 3-(1-oxo-4-((4-(phenyl(pyridin-2-yl)amino)piperidin-1-yl)methyl)isoindolin-2-yl)piperidine-2,6-dione (GT-04948)**

Referring to the method of Scheme 4, the target product (GT-04948) was prepared as white solid (65 mg, yield 69%). $^1H$ NMR (400 MHz, DMSO-d6) δ 11.06 (s, 1H), 10.66 (s, 1H), 8.13 - 8.07 (m, 1H), 7.93 (d, *J* = 7.6 Hz, 1H), 7.81 (d, *J*= 7.2 Hz, 1H), 7.79 - 7.71 (m, 1H), 7.65 - 7.51 (m, 4H), 7.31 (d, *J* = 7.3 Hz, 2H), 6.93 (s, 1H), 6.32 (s, 1H), 5.18 (dd, *J*= 13.2, 5.2 Hz, 1H), 4.92 (t, *J*= 9.6 Hz, 1H), 4.79 (t, *J* = 14.2 Hz, 1H), 4.49 (d, *J* = 17.5 Hz, 1H), 4.36 (d, *J* = 4.5 Hz, 2H), 3.47 - 3.43 (m, 2H), 3.37 - 3.34 (m, 2H), 2.99 - 2.89 (m, 1H), 2.64 (d, *J* = 16.9 Hz, 1H), 2.36 - 2.30 (m, 1H), 2.15 (d, *J* = 12.2 Hz, 2H), 2.04 - 2.00 (m, 1H), 1.93 - 1.72 (m, 2H). LCMS (ESI) calcd for $C_{30}H_{32}N_5O_3^+$ [M+H]$^+$: 510.25, found, 510.3.

**Example 151: preparation of 3-(5-((4-(diphenylamino)cyclohexyl)amino)-1-oxoisoindolin-2-yl)piperidine-2,6-dione (GT-06806)**

Referring to the method of Scheme 4, the target product (GT-06806) was prepared as white solid (14 mg, yield 14%). $^1H$ NMR (400 MHz, DMSO-d6) δ 10.84 (s, 1H), 7.30 - 7.25 (m, 1H), 7.25 - 7.21 (m, 2H), 6.95 - 6.87 (m, 4H), 6.78 - 6.70 (m, 6H), 4.92 (dd, J = 13.2, 5.0 Hz, 1H), 4.19 - 4.13 (m, 1H), 4.06 - 4.01 (m, 1H), 3.89 - 3.83 (m, 1H), 3.81 - 3.68 (m, 2H), 3.19 - 3.13 (m, 1H), 2.90 - 2.74 (m, 1H), 2.52 - 2.44 (m, 1H), 2.29 - 2.20 (m, 1H), 1.98 - 1.92 (m, 2H), 1.74 - 1.67 (m, 2H), 1.42 - 1.36 (m, 2H), 1.20 - 1.17 (m, 1H). LCMS (ESI) calcd for $C_{31}H_{33}N_4O_3^+$ [M+H]$^+$: 509.25, found, 509.3.

**Example 152: preparation of 3-(5-(4-benzhydrylpiperazine-1-carbonyl)-1-oxoisoindolin-2-yl)piperidine-2,6-dione (GT-05689)**

Referring to the method of Scheme 6, the target product (GT-05689) was prepared as white solid (65 mg, yield 73%). $^1H$ NMR (400 MHz, DMSO-d6) δ 11.00 (s, 1H), 7.91 - 7.70 (m, 3H), 7.70 - 7.60 (m, 1H), 7.60 - 7.12 (m, 9H), 5.13 (dd, J = 13.1, 5.2 Hz, 1H), 4.49 (d, J = 17.7 Hz, 1H), 4.37 (d, J = 17.8 Hz, 1H), 3.79 - 3.61 (m, 2H), 3.35 - 3.25 (m, 4H), 3.21 - 3.12 (m, 2H), 2.92 - 2.89(m, 1H), 2.61 (d, J = 17.5 Hz, 1H), 2.44 - 2.32 (m, 3H), 2.03 - 1.99 (m, 1H). LCMS (ESI) calcd for $C_{31}H_{31}N_4O_4^+$ [M+H]$^+$: 523.23, found, 523.3.

**Example 153: preparation of 3-(4-(4-benzhydrylpiperazine-1-carbonyl)-1-oxoisoindolin-2-yl)piperidine-2,6-dione (GT-05721)**

Referring to the method of Scheme 6, the target product (GT-05721) was prepared as white solid (48 mg, yield 54%). $^1$H NMR (400 MHz, DMSO-d6) δ 11.01 (s, 1H), 7.84 - 7.75 (m, 4H), 7.64 - 7.58 (m, 3H), 7.49 - 7.32 (m, 6H), 5.14 (dd, *J* = 13.3, 5.1 Hz, 1H), 4.49 - 4.35 (m, 3H), 3.48 - 3.43 (m, 4H), 3.33 - 3.05 (m, 4H), 3.00 - 2.84 (m, 1H), 2.60 (d, *J* = 17.6 Hz, 1H), 2.46 - 2.38 (m, 1H), 2.09 - 1.95 (m, 1H). LCMS (ESI) calcd for $C_{31}H_{31}N_4O_4^+$ [M+H]$^+$: 523.23, found, 523.2.

**Example 154: preparation of 3-(4-(4-benzhydrylpiperazine-1-carbonyl)-1-oxoisoindolin-2-yl)piperidine-2,6-dione (GT-06597)**

Referring to the method of Scheme 6, the target product (GT-06597) was prepared as white solid (63 mg, yield 72%). $^1$H NMR (400 MHz, DMSO-d6) δ 11.01 (s, 1H), 7.77 (d, J = 7.8 Hz, 1H), 7.63 (s, 1H), 7.51 (d, J = 8.0 Hz, 1H), 7.40 - 7.22 (m, 4H), 7.15 (t, J = 8.7 Hz, 2H), 5.13 (dd, J = 13.3, 5.1 Hz, 1H), 4.69 (s, 1H), 4.49 (d, J = 17.6 Hz, 1H), 4.37 (d, J = 17.7 Hz, 1H), 3.75 (brs, 2H), 3.31 - 3.22 (m, 4H), 2.98 - 2.85 (m, 1H), 2.61 (d, J = 17.0 Hz, 1H), 2.45 - 2.35 (m, 3H), 2.01 - 1.99 (m, 1H). LCMS (ESI) calcd for $C_{31}H_{27}F_4N_4O_4^+$ [M+H]$^+$: 595.20, found, 595.2.

**Example 155: preparation of 3-(4-(4-benzhydrylpiperazine-1-carbonyl)-1-oxoisoindolin-2-yl)piperidine-2,6-dione (GT-05690)**

Referring to the method of Scheme 4, the target product (GT-05690) was prepared as white solid (58 mg, yield 65%). $^1$H NMR (400 MHz, DMSO-d6) δ 11.07 (s, 1H), 8.12 (brs, 1H), 7.99 (brs, 1H), 7.93 - 7.88 (m, 1H), 7.71 - 7.32 (m, 4H), 7.27 (brs, 4H), 7.18 (brs, 2H), 5.10 (dd, J = 12.8, 5.4 Hz, 1H), 4.43 (brs, 2H), 3.37 - 3.24 (m, 4H), 3.14 - 3.10 (m, 2H), 2.90 - 2.73 (m, 3H), 2.54 (d, J = 18.4 Hz, 1H), 2.52 - 2.44 (m, 2H), 2.07 - 1.90 (m, 1H). LCMS (ESI) calcd for $C_{31}H_{31}N_4O_4^+$ [M+H]$^+$: 523.23, found, 523.2.

**Example 156: preparation of 4-((4-benzhydrylpiperazin-1-yl)methyl)-2-(2,6-dioxopiperidin-3-yl)isoindoline-1,3-dione (GT-05722)**

Referring to the method of Scheme 4, the target product (GT-05722) was prepared as white solid (45 mg, yield 54%). $^1$H NMR (400 MHz, DMSO) δ 11.15 (s, 1H), 8.08 (brs, 1H), 7.98 - 7.89 (m, 2H), 7.65 - 7.03 (m, 10H), 5.17 (dd, *J* = 12.7, 5.2 Hz, 1H), 4.72 (brs, 1H), 4.49 (bsr, 1H), 3.28 - 3.21 (m, 4H), 3.06 - 2.68 (m, 4H), 2.61 (d, *J* = 17.8 Hz, 1H), 2.55 - 2.51 (m, 1H), 2.44 - 2.33 (m, 2H), 2.08 - 2.02 (m, 1H). LCMS (ESI) calcd for $C_{31}H_{31}N_4O_4^+$ [M+H]$^+$: 523.23, found, 523.2.

**Example 157: preparation of 5-((4-benzhydryl-1,4-diazepan-1-yl)methyl)-2-(2,6-dioxopiperidin-3-yl)isoindoline-1,3-dione (GT-05739)**

Referring to the method of Scheme 4, the target product (GT-05739) was prepared as white solid (18 mg, yield 59%). $^1$H NMR (400 MHz, DMSO) δ 11.14 (s, 1H), 8.19 (s, 1H), 8.08 - 7.94 (m, 2H), 7.97 - 7.70 (m, 2H), 7.69 - 7.48 (m, 2H), 7.48 - 7.18 (m, 6H), 5.18 (dd, *J* = 12.8, 5.4 Hz, 1H), 4.67 - 4.37 (m, 2H), 3.59 - 3.40 (m, 6H), 3.28 - 3.13 (m, 4H), 2.97 - 2.82 (m, 2H), 2.61 (d, *J* = 19.2 Hz, 2H), 2.49 - 2.33 (m, 1H), 2.13 - 1.95 (m, 2H). LCMS (ESI) calcd for $C_{32}H_{32}N_4O_4^+$ [M+H]$^+$: 537.25, found, 537.3.

**Example 158: preparation of 5-((7-benzhydryl-2,7-diazaspiro[3.5]nonan-2-yl)methyl)-2-(2,6-dioxopiperidin-3-yl) isoindoline-1,3-dione (GT-05906)**

Referring to the method of Scheme 4, the target product (GT-05906) was prepared as white solid (12 mg, yield 13%). $^1$H NMR (400 MHz, DMSO-d6) δ 11.56 (s, 1H), 11.15 (s, 1H), 11.08 (s, 1H), 8.15 (s, 1H), 8.03 (s, 2H), 7.91 - 7.75 (m, 4H), 7.53 - 7.41 (m, 4H), 7.41 - 7.30 (m, 2H), 5.43 (d, J = 9.6 Hz, 1H), 5.18 (dd, J = 12.8, 5.4 Hz, 1H), 4.66 - 4.54 (m, 2H), 4.11 - 4.04 (m, 2H), 3.94 - 3.89 (m, 2H), 3.18 - 3.09 (m, 2H), 2.96 - 2.87 (m, 2H), 2.67 - 2.57 (m, 3H), 2.37 - 2.16 (m, 4H), 2.14 - 2.01 (m, 1H). LCMS (ESI) calcd for $C_{34}H_{35}N_4O_4^+$ [M+H]$^+$: 563.27, found, 563.3.

**Example 159: preparation of 5-((8-benzhydryl-2,8-diazaspiro[4.5]decan-2-yl)methyl)-2-(2,6-dioxopiperidin-3-yl) isoindoline-1,3-dione (GT-05974)**

Referring to the method of Scheme 4, the target product (GT-05974) was prepared as white solid (31 mg, yield 58%). $^1$H NMR (400 MHz, DMSO-d6) δ 11.58 (s, 1H), 11.17 (s, 1H), 8.29 - 8.19 (m, 1H), 8.18 - 7.99 (m, 2H), 7.92 - 7.76 (m, 4H), 7.49 - 7.40 (m, 4H), 7.40 - 7.32 (m, 2H), 5.58 (dd, J = 29.0, 9.2 Hz, 1H), 5.26 - 5.11 (m, 1H), 4.71 - 4.49 (m, 2H), 3.63 -

3.47 (m, 1H), 3.13 - 3.04 (m, 4H), 3.02 - 2.82 (m, 3H), 2.69 - 2.53 (m, 2H), 2.27 - 1.77 (m, 7H). LCMS (ESI) calcd for $C_{35}H_{37}N_4O_4^+$ [M+H]$^+$: 577.28, found, 577.3.

**Example 160: preparation of 5-((9-benzhydryl-3,9-diazaspiro[5.5]undecan-3-yl)methyl)-2-(2,6-dioxopiperidin-3-yl)isoindoline-1,3-dione (GT-06028)**

Referring to the method of Scheme 4, the target product (GT-06028) was prepared as white solid (52 mg, yield 59%). $^1$H NMR (400 MHz, DMSO-d6) δ 11.57 (s, 1H), 11.15 (s, 1H), 10.75 (s, 1H), 8.20 (s, 1H), 8.08 - 8.01 (m, 2H), 7.94 - 7.82 (m, 4H), 7.46 - 7.42 (m, 4H), 7.39 - 7.34 (m, 2H), 5.64 (d, J = 9.4 Hz, 1H), 5.18 (dd, J = 12.8, 5.4 Hz, 1H), 4.50 (s, 2H), 3.31 - 3.19 (m, 2H), 3.15 - 2.98 (m, 6H), 2.97 - 2.84 (m, 1H), 2.64 - 2.51 (m, 2H), 2.23 (d, J = 13.5 Hz, 1H), 2.17 - 1.97 (m, 3H), 1.89 - 1.85 (m, 1H), 1.80 - 1.74 (m, 1H), 1.61 - 1.58 (m, 1H), 1.50 (t, J = 11.8 Hz, 2H). LCMS (ESI) calcd for $C_{36}H_{39}N_4O_4^+$ [M+H]$^+$: 591.30, found, 591.3.

**Example 161: preparation of 5-((5-benzhydrylhexahydropyrrolo[3,4-c]pyrrol-2(1H)-yl)methyl)-2-(2,6-dioxopiperidin-3-yl)isoindoline-1,3-dione (GT-06053)**

Referring to the method of Scheme 4, the target product (GT-06053) was prepared as white solid (29 mg, yield 31%). $^1$H NMR (400 MHz, DMSO-d6) δ 11.15 (s, 1H), 8.35 - 8.12 (m, 1H), 8.10 - 7.91 (m, 3H), 7.84 - 7.76 (m, 3H), 7.59 - 7.25 (m, 6H), 5.71 - 5.57 (m, 1H), 5.18 (dd, J = 12.7, 5.2 Hz, 1H), 4.76 - 4.50 (m, 2H), 4.04 - 3.84 (m, 1H), 3.73 - 3.61 (m, 2H), 3.59 - 3.44 (m, 4H), 3.32 - 3.14 (m, 4H), 2.93 - 2.87 (m, 1H), 2.61 (d, J = 17.6 Hz, 1H), 2.11 - 2.06 (m, 1H). LCMS (ESI) calcd for $C_{33}H_{33}N_4O_4^+$ [M+H]$^+$: 549.25, found, 549.3.

**Example 162: preparation of 5-((3-benzhydryl-3,6-diazabicyclo[3.1.1]heptan-6-yl)methyl)-2-(2,6-dioxopiperidin-3-yl)isoindoline-1,3-dione (GT-05728)**

Referring to the method of Scheme 4, the target product (GT-05728) was prepared as white solid (21 mg, yield 28%). $^1$H NMR (400 MHz, DMSO-d6) δ 11.15 (s, 1H), 8.11 (s, 1H), 8.04 - 7.91 (m, 2H), 7.60 - 7.42 (m, 4H), 7.39 - 7.29 (m, 4H), 7.25 - 7.20 (m, 2H), 5.18 (dd, *J* = 12.8, 4.9 Hz, 1H), 4.79 - 4.66 (m, 1H), 4.44 - 4.21 (m, 2H), 3.64 - 3.52 (m, 2H), 3.17 - 2.98 (m, 2H), 2.90 - 2.87 (m, 2H), 2.67 - 2.60 (m, 3H), 2.35 - 2.31 (m, 1H), 2.27 - 2.24 (m, 1H), 2.16 - 2.01 (m, 1H). LCMS (ESI) calcd for $C_{32}H_{31}N_4O_4^+$ [M+H]$^+$: 535.23, found, 535.3.

**Example 163: preparation of 5-(((1R,4R)-5-benzhydryl-2,5-diazabicyclo[2.2.1]heptan-2-yl)methyl)-2-(2,6-dioxopiperidin-3-yl)isoindoline-1,3-dione (GT-05959)**

Referring to the method of Scheme 4, the target product (GT-05959) was prepared as white solid (20 mg, yield 21%). $^1$H NMR (400 MHz, DMSO-d6) δ 11.15 (s, 1H), 8.26 (s, 1H), 8.14 (s, 1H), 8.03 (d, J = 7.5 Hz, 1H), 7.61 - 7.45 (m, 3H), 7.37 - 7.29 (m, 5H), 7.27 - 7.19 (m, 2H), 5.18 (dd, J = 12.9, 5.4 Hz, 1H), 4.71 - 4.63 (m, 1H), 4.47 - 4.37 (m, 1H), 4.24 - 4.15 (m, 1H), 3.83 - 3.61 (m, 1H), 3.33 - 3.20 (m, 3H), 3.11 - 3.05 (m, 1H), 2.93 - 2.87 (m, 2H), 2.65 - 2.61 (m, 1H), 2.60 - 2.57 (m, 1H), 2.57 - 2.54 (m, 1H), 2.35 - 2.31 (m, 1H), 2.09 - 2.04 (m, 1H). LCMS (ESI) calcd for $C_{32}H_{31}N_4O_4^+$ [M+H]$^+$: 535.23, found, 535.3.

**Example 164: preparation of 5-(((1S,4S)-5-benzhydryl-2,5-diazabicyclo[2.2.1]heptan-2-yl)methyl)-2-(2,6-dioxopiperidin-3-yl)isoindoline-1,3-dione (GT-05729)**

Referring to the method of Scheme 4, the target product (GT-05729) was prepared as white solid (31 mg, yield 41%). $^1$H NMR (400 MHz, DMSO-d6) δ 11.15 (s, 1H), 8.29 - 8.24 (m, 1H), 8.16 - 8.10 (m, 1H), 8.04 - 8.02 (m, 1H), 7.65 - 7.13 (m, 10H), 5.18 (dd, *J* = 12.7, 5.5 Hz, 1H), 4.91 - 4.73 (m, 1H), 4.70 - 4.61 (m, 1H), 4.51 - 4.33 (m, 1H), 4.30 - 4.12 (m, 1H), 3.78 - 3.57 (m, 2H), 3.12 - 3.06 (m, 1H), 2.98 - 2.82 (m, 2H), 2.70 - 2.58 (m, 2H), 2.54 - 2.51 (m, 2H), 2.34 - 2.27 (m, 1H), 2.10 - 2.04 (m, 1H). LCMS (ESI) calcd for $C_{32}H_{31}N_4O_4^+$ [M+H]$^+$: 535.23, found, 535.3.

**Example 165: preparation of 5-((3-benzhydryl-3,8-diazabicyclo[3.2.1]octan-8-yl)methyl)-2-(2,6-dioxopiperidin-3-yl)isoindoline-1,3-dione (GT-05899)**

Referring to the method of Scheme 4, the target product (GT-05899) was prepared as white solid (16 mg, yield 17%). $^1$H NMR (400 MHz, DMSO-d6) δ 11.14 (s, 1H), 10.46 (s, 1H), 8.28 (s, 1H), 8.17 (d, *J* = 7.7 Hz, 1H), 8.01 (d, *J* = 7.7 Hz, 1H), 7.46 (d, *J* = 7.3 Hz, 4H), 7.31 (t, *J* = 7.6 Hz, 4H), 7.20 (t, *J* = 7.3 Hz, 2H), 5.17 (dd, *J* = 12.8, 5.5 Hz, 1H), 4.43 (s, 1H), 4.39 (d, *J* = 6.0 Hz, 2H), 3.84 (s, 2H), 2.97 - 2.82 (m, 1H), 2.68 - 2.54 (m, 5H), 2.51 - 2.49 (m, 1H), 2.32 - 2.22 (m, 4H), 2.12 - 1.93 (m, 1H). LCMS (ESI) calcd for $C_{33}H_{33}N_4O_4^+$ [M+H]$^+$: 549.25, found, 549.3.

**Example 166: preparation of 5-((8-benzhydryl-3,8-diazabicyclo[3.2.1]octan-3-yl)methyl)-2-(2,6-dioxopiperidin-3-yl)isoindoline-1,3-dione (GT-05898)**

Referring to the method of Scheme 4, the target product (GT-05898) was prepared as white solid (22 mg, yield 24%). $^{1}H$ NMR (400 MHz, DMSO-d6) δ 11.13 (s, 1H), 8.03 - 7.84 (m, 5H), 7.57 - 7.52 (m, 1H), 7.47 - 7.30 (m, 6H), 7.24 - 7.16 (m, 1H), 5.41 (d, *J* = 9.1 Hz, 1H), 5.16 (dd, *J* = 10.7, 3.1 Hz, 1H), 4.69 - 4.52 (m, 1H), 3.86 - 3.80 (m, 1H), 3.68 (brs, 1H), 3.33 - 3.22 (m, 1H), 3.20 - 3.06 (m, 2H), 2.95 - 2.84 (m, 2H), 2.81 - 2.69 (m, 1H), 2.60 (d, *J* = 17.9 Hz, 1H), 2.41 - 2.23 (m, 2H), 2.15 - 1.94 (m, 4H). LCMS (ESI) calcd for $C_{33}H_{33}N_4O_4^+$ [M+H]$^+$: 549.25, found, 549.3.

**Example 167: preparation of 5-(((R)-4-benzhydryl-2-methylpiperazin-1-yl)methyl)-2-(2,6-dioxopiperidin-3-yl) isoindoline-1,3-dione (GT-06047)**

Referring to the method of Scheme 4, the target product (GT-06047) was prepared as white solid (44 mg, yield 47%). $^{1}H$ NMR (400 MHz, DMSO-d6) δ 11.14 (s, 1H), 8.17 (s, 1H), 8.05 - 7.99 (m, 2H), 7.51 - 7.43 (m, 4H), 7.36 - 7.30 (m, 4H), 7.28 - 7.06 (m, 2H), 5.17 (dd, J = 12.7, 5.3 Hz, 1H), 4.95 - 4.80 (m, 1H), 4.69 - 4.49 (m, 1H), 4.44 - 4.29 (m, 1H), 3.21 - 2.98 (m, 3H), 2.99 - 2.78 (m, 3H), 2.61 (d, J = 18.9 Hz, 1H), 2.55 - 2.51 (m, 1H), 2.37 - 2.33 (m, 2H), 2.12 - 2.03 (m, 1H), 1.41 (s, 3H). LCMS (ESI) calcd for $C_{32}H_{33}N_4O_4^+$ [M+H]$^+$: 537.25, found, 537.3.

**Example 168: preparation of 5-((4-benzhydryl-2,6-dimethylpiperazin-1-yl)methyl)-2-(2,6-dioxopiperidin-3-yl) isoindoline-1,3-dione (GT-05740)**

Referring to the method of Scheme 4, the target product (GT-05740) was prepared as white solid (8 mg, yield 14%). $^{1}H$ NMR (400 MHz, DMSO-d6) δ 11.16 (s, 1H), 8.05 (s, 1H), 7.92 - 7.75 (m, 2H), 7.54 - 7.13 (m, 10H), 5.26 - 5.07 (m, 1H), 4.76 (s, 1H), 3.30 - 3.21 (m, 2H), 3.07 - 2.77 (m, 5H), 2.70 - 2.56 (m, 2H), 2.27 - 2.20 (m, 1H), 2.12 - 2.05 (m, 2H), 1.42 (brs, 4H), 0.89 (brs, 2H). LCMS (ESI) calcd for $C_{33}H_{35}N_4O_4^+$ [M+H]$^+$: 551.27, found, 551.3.

**Example 169: preparation of 5-((4-benzhydryl-3,5-dimethylpiperazin-1-yl)methyl)-2-(2,6-dioxopiperidin-3-yl) isoindoline-1,3-dione (GT-05968)**

Referring to the method of Scheme 4, the target product (GT-05968) was prepared as white solid (28 mg, yield 30%). $^{1}H$ NMR (400 MHz, DMSO-d6) δ 11.14 (s, 1H), 8.22 - 8.14 (m, 1H), 8.05 - 7.93 (m, 2H), 7.90 (s, 1H), 7.53 - 7.48 (m, 1H), 7.46 - 7.34 (m, 4H), 7.29 (t, J = 7.6 Hz, 3H), 7.19 (t, J = 7.2 Hz, 1H), 5.17 (dd, J = 12.7, 5.3 Hz, 1H), 4.23 - 4.06 (m, 1H), 3.28 - 3.12 (m, 4H), 3.09 - 3.07 (m, 1H), 2.97 - 2.88 (m, 2H), 2.67 - 2.56 (m, 2H), 2.08 - 2.05 (m, 1H), 1.56 - 1.39 (m, 2H), 1.25 (s, 3H), 1.23 (s, 3H). LCMS (ESI) calcd for $C_{33}H_{35}N_4O_4^+$ [M+H]$^+$: 551.27, found, 551.3.

**Example 170: preparation of 5-((5-benzhydryl-2,5-diazabicyclo[2.2.2]octan-2-yl)methyl)-2-(2,6-dioxopiperidin-3-yl)isoindoline-1,3-dione (GT-05730)**

Referring to the method of Scheme 4, the target product (GT-05730) was prepared as white solid (25 mg, yield 34%). $^{1}H$ NMR (400 MHz, DMSO) δ 11.15 (s, 1H), 8.39 - 8.35 (m, 1H), 8.26 - 8.19 (m, 1H), 8.11 - 8.00 (m, 1H), 7.67 - 7.10 (m, 10H), 5.18 (dd, *J* = 12.8, 5.3 Hz, 1H), 4.93 - 4.90 (m, 1H), 4.69 - 4.45 (m, 2H), 3.72 - 3.58 (m, 3H), 3.23 - 3.20 (m, 1H), 2.98 - 2.74 (m, 2H), 2.70 - 2.58 (m, 2H), 2.56 - 2.51 (m, 2H), 2.15 - 1.99 (m, 2H), 1.96 - 1.84 (m, 1H), 1.73 - 1.50 (m, 1H). LCMS (ESI) calcd for $C_{33}H_{33}N_4O_4^+$ [M+H]$^+$: 549.25, found, 549.3.

**Example 171: preparation of 5-((4-benzhydryl-3,3-dimethylpiperazin-1-yl)methyl)-2-(2,6-dioxopiperidin-3-yl) isoindoline-1,3-dione (GT-05962)**

Referring to the method of Scheme 4, the target product (GT-05962) was prepared as white solid (11 mg, yield 12%). $^{1}H$ NMR (400 MHz, DMSO-d6) δ 11.13 (s, 1H), 8.13 - 8.00 (m, 1H), 7.95 - 7.84 (m, 2H), 7.37 - 7.21 (m, 10H), 5.23 - 5.10 (m, 1H), 4.51 (brs, 1H), 3.29 - 3.23 (m, 4H), 3.20 - 3.13 (m, 2H), 3.06 - 2.98 (m, 1H), 2.93 - 2.85 (m, 1H), 2.68 - 2.55 (m, 3H), 2.12 - 2.01 (m, 1H), 1.21 (s, 3H), 1.00 (s, 3H). LCMS (ESI) calcd for $C_{33}H_{35}N_4O_4^+$ [M+H]$^+$: 551.27, found, 551.3.

**Example 172: preparation of 2-(2,6-dioxopiperidin-3-yl)-5-((4-(phenyl(pyridin-2-yl)methyl)piperazin-1-yl) methyl)isoindoline-1,3-dione (GT-05723)**

Referring to the method of Scheme 4, the target product (GT-05723) was prepared as white solid (49 mg, yield 57%). $^{1}H$ NMR (400 MHz, DMSO-d6) δ 11.14 (s, 1H), 8.67 (d, *J* = 4.5 Hz, 1H), 8.19 (s, 1H), 8.08 (d, *J* = 7.3 Hz, 1H), 8.02 (t, *J* = 6.9 Hz, 2H), 7.73 (d, *J* = 7.8 Hz, 1H), 7.57 (d, *J* = 7.3 Hz, 2H), 7.53 - 7.46 (m, 1H), 7.39 (t, *J*= 7.3 Hz, 2H), 7.33 (t, *J* = 7.2 Hz,

1H), 5.38 (s, 1H), 5.18 (dd, *J*= 12.8, 5.4 Hz, 1H), 4.47 (s, 2H), 3.28 (s, 4H), 3.09 - 2.91 (m, 4H), 2.90 - 2.80 (m, 1H), 2.61 (d, *J* = 18.4 Hz, 1H), 2.58 - 2.53 (m, 1H), 2.15 - 1.98 (m, 1H). LCMS (ESI) calcd for $C_{30}H_{30}N_5O_4^+$ [M+H]+: 524.23, found, 524.3.

**Example 173: preparation of 2-(2,6-dioxopiperidin-3-yl)-5-((4-(phenyl(pyridin-3-yl)methyl)piperazin-1-yl) methyl)isoindoline-1,3-dione (GT-06114)**

Referring to the method of Scheme 4, the target product (GT-06114) was prepared as white solid (35 mg, yield 46%). $^1$H NMR (400 MHz, DMSO-d6) δ 11.15 (s, 1H), 9.01 (s, 1H), 8.81 (d, J = 5.4 Hz, 1H), 8.62 (d, J = 8.1 Hz, 1H), 8.26 (s, 1H), 8.15 (d, J = 7.9 Hz, 1H), 8.07 - 7.95 (m, 2H), 7.51 (d, J = 7.3 Hz, 2H), 7.39 (t, J = 7.5 Hz, 2H), 7.31 (t, J = 7.3 Hz, 1H), 5.18 (dd, J = 12.8, 5.4 Hz, 1H), 5.10 (brs, 1H), 4.56 (s, 2H), 3.36 - 3.25 (m, 4H), 3.00 - 2.83 (m, 3H), 2.81 - 2.68 (m, 1H), 2.61 (d, J = 18.9 Hz, 2H), 2.55 - 2.51 (m, 1H), 2.14 - 2.00 (m, 1H). LCMS (ESI) calcd for $C_{30}H_{30}N_5O_4^+$ [M+H]+: 524.23, found, 524.3.

**Example 174: preparation of 2-(2,6-dioxopiperidin-3-yl)-5-((4-(phenyl(pyridin-4-yl)methyl)piperazin-1-yl) methyl)isoindoline-1,3-dione (GT-06115)**

Referring to the method of Scheme 4, the target product (GT-06115) was prepared as white solid (33 mg, yield 34%). $^1$H NMR (400 MHz, DMSO-d6) δ 11.15 (s, 1H), 8.84 (d, J = 6.4 Hz, 2H), 8.26 (s, 1H), 8.14 (d, J = 7.9 Hz, 1H), 8.10 (d, J = 6.3 Hz, 2H), 8.02 (d, J = 7.6 Hz, 1H), 7.46 (d, J = 7.4 Hz, 2H), 7.38 (t, J = 7.5 Hz, 2H), 7.31 (d, J = 7.3 Hz, 1H), 5.18 (dd, J = 12.7, 5.4 Hz, 1H), 5.01 (s, 1H), 4.55 (s, 2H), 3.27 - 3.13 (m, 4H), 2.99 - 2.85 (m, 2H), 2.84 - 2.71 (m, 2H), 2.68 - 2.64 (m, 1H), 2.59 - 2.55 (m, 2H), 2.13 - 1.92 (m, 1H). LCMS (ESI) calcd for $C_{30}H_{30}N_5O_4^+$ [M+H]+: 524.23, found, 524.3.

**Example 175: preparation of 5-((4-(di(pyridin-2-yl)methyl)piperazin-1-yl)methyl)-2-(2,6-dioxopiperidin-3-yl)isoindoline-1,3-dione (GT-05724)**

Referring to the method of Scheme 4, the target product (GT-05724) was prepared as white solid (43 mg, yield 50%). $^1$H NMR (400 MHz, DMSO) δ 11.15 (s, 1H), 8.75 - 8.65 (m, 2H), 8.27 (s, 1H), 8.18 - 8.14 (m, 3H), 8.02 (d, *J* = 7.7 Hz, 1H), 7.86 (d, *J*= 7.9 Hz, 2H), 7.68 - 7.56 (m, 2H), 5.86 (s, 1H), 5.19 (dd, *J* = 12.8, 5.4 Hz, 1H), 4.59 (s, 2H), 3.39 (s, 4H), 2.98 (s, 4H), 2.95 - 2.82 (m, 1H), 2.61 (d, *J* = 18.3 Hz, 1H), 2.56 - 2.52 (m, 1H), 2.09 - 2.06 (m, 1H). LCMS (ESI) calcd for $C_{29}H_{29}N_6O_4^+$ [M+H]+: 525.22, found, 525.3.

**Example 176: preparation of 5-((4-(di(pyridin-3-yl)methyl)piperazin-1-yl)methyl)-2-(2,6-dioxopiperidin-3-yl)isoindoline-1,3-dione (GT-05725)**

Referring to the method of Scheme 4, the target product (GT-05725) was prepared as white solid (4 mg, yield 4%). $^1$H NMR (400 MHz, DMSO-d6) δ 11.14 (s, 1H), 9.46 (s, 1H), 9.18 (d, *J* = 6.1 Hz, 1H), 8.76 - 8.71 (m, 3H), 8.70 - 8.62 (m, 2H), 8.20 - 8.16 (m, 2H), 8.04 - 8.02 (m, 2H), 5.25 (s, 1H), 5.17 (dd, *J*= 12.8, 5.4 Hz, 1H), 3.65 (s, 2H), 3.41 - 3.38 (m, 4H), 3.25 - 3.19 (m, 4H), 2.92 - 2.84 (m, 1H), 2.57 - 2.55 (m, 1H), 2.49 - 2.45 (m, 1H), 2.13 - 2.00 (m, 1H). LCMS (ESI) calcd for $C_{29}H_{29}N_6O_4^+$ [M+H]+: 525.22, found, 525.2.

**Example 177: preparation of 5-((4-((4-bromophenyl)(phenyl)methyl)piperazin-1-yl)methyl)-2-(2,6-dioxopiperidin-3-yl)isoindoline-1,3-dione (GT-06639)**

Referring to the method of Scheme 4, the target product (GT-06639) was prepared as white solid (168 mg, yield 92%). $^1$H NMR (400 MHz, DMSO-d6) δ 11.15 (s, 1H), 10.81 (s, 1H), 8.18 (s, 1H), 8.05-8.01 (m, 2H), 7.53 (d, J = 7.0 Hz, 2H), 7.46 - 7.39 (m, 3H), 7.36 - 7.31 (m, 3H), 7.28 - 7.22 (m, 1H), 5.18 (dd, J = 12.8, 5.4 Hz, 1H), 4.53 (s, 2H), 3.46 - 3.39 (m, 2H), 3.38 - 3.24 (m, 2H), 3.21 - 3.11 (m, 2H), 2.94 - 2.77 (m, 3H), 2.64 - 2.59 (m, 1H), 2.55 - 2.52 (m, 1H), 2.43 - 2.34 (m, 1H), 2.13 - 2.02 (m, 1H). LCMS (ESI) calcd for $C_{31}H_{30}B_rN_4O_4^+$ [M+H]+: 601.14, found, 601.1.

**Example 178: preparation of 5-((4-(bis(4-fluorophenyl)methyl)piperazin-1-yl)methyl)-2-(2,6-dioxopiperidin-3-yl) isoindoline-1,3-dione (GT-05726)**

Referring to the method of Scheme 4, the target product (GT-05726) was prepared as white solid (92 mg, yield 89%). $^1$H NMR (400 MHz, DMSO-d6) δ 11.14 (s, 1H), 8.19 (s, 1H), 8.07 (d, *J* = 7.3 Hz, 1H), 8.01 (d, *J* = 7.6 Hz, 1H), 7.50 (brs, 4H), 7.18 (t, *J* = 7.8 Hz, 4H), 5.18 (dd, *J* = 12.7, 5.4 Hz, 1H), 4.50 (brs, 2H), 3.46 - 3.38 (m, 2H), 3.33 (brs, 3H), 3.23 - 3.11 (m, 2H), 3.02 - 2.76 (m, 3H), 2.61 (d, *J* = 18.4 Hz, 1H), 2.55 - 2.51 (m, 1H), 2.10 - 2.04 (m, 1H). LCMS (ESI) calcd for $C_{31}H_{29}F_2N_4O_4^+$ [M+H]+: 559.22, found, 559.3.

**Example 179: preparation of 5-((4-(bis(3-fluorophenyl)methyl)piperazin-1-yl)methyl)-2-(2,6-dioxopiperidin-3-yl) isoindoline-1,3-dione (GT-06118)**

Referring to the method of Scheme 4, the target product (GT-06118) was prepared as white solid (74 mg, yield 81%). $^1$H NMR (400 MHz, DMSO-d6) δ 11.15 (s, 1H), 10.68 (s, 1H), 8.18 (s, 1H), 8.04 (q, J = 7.5 Hz, 2H), 7.47 - 7.31 (m, 2H), 7.33 - 7.26 (m, 4H), 7.09 (t, J = 7.4 Hz, 2H), 5.18 (dd, J = 12.7, 5.4 Hz, 1H), 4.67 (s, 1H), 4.52 (s, 2H), 3.27 - 3.11 (m, 4H), 2.93 - 2.81 (m, 4H), 2.61 (d, J = 19.3 Hz, 2H), 2.44 - 2.36 (m, 1H), 2.15 - 1.95 (m, 1H). LCMS (ESI) calcd for $C_{31}H_{29}F_2N_4O_4^+$ $[M+H]^+$: 559.22, found, 559.2.

**Example 180: preparation of 5-((4-(bis(2-fluorophenyl)methyl)piperazin-1-yl)methyl)-2-(2,6-dioxopiperidin-3-yl) isoindoline-1,3-dione (GT-06088)**

Referring to the method of Scheme 4, the target product (GT-06088) was prepared as white solid (47 mg, yield 51%). $^1$H NMR (400 MHz, DMSO-d6) δ 11.15 (s, 1H), 10.77 (s, 1H), 8.18 (s, 1H), 8.04 (dd, J = 16.0, 7.7 Hz, 2H), 7.56 - 7.53 (m, 2H), 7.35 - 7.30 (m, 2H), 7.24 - 7.14 (m, 4H), 5.18 (dd, J = 12.7, 5.4 Hz, 1H), 5.10 (s, 1H), 4.53 (s, 2H), 3.32 - 3.16 (m, 4H), 2.96 - 2.79 (m, 3H), 2.63 - 2.55 (m, 2H), 2.48 - 2.36 (m, 2H), 2.10 - 2.05 (m, 1H). LCMS (ESI) calcd for $C_{31}H_{29}F_2N_4O_4^+$ $[M+H]^+$: 559.22, found, 559.2.

**Example 181: preparation of 5-((4-(bis(4-(trifluoromethyl)phenyl)methyl)piperazin-1-yl)methyl)-2-(2,6-dioxopiperidin-3-yl)isoindoline-1,3-dione (GT-06203)**

Referring to the method of Scheme 4, the target product (GT-06203) was prepared as white solid (52 mg, yield 63%). $^1$H NMR (400 MHz, DMSO-d6) δ 11.15 (s, 1H), 10.80 (s, 1H), 8.18 (s, 1H), 8.07 - 8.02 (m, 2H), 7.73 - 8.67 (m, 8H), 5.18 (dd, J = 12.8, 5.4 Hz, 1H), 4.85 (s, 1H), 4.54 (s, 2H), 3.22 - 3.13 (m, 4H), 2.97 - 2.76 (m, 3H), 2.61 (d, J = 19.4 Hz, 1H), 2.55 - 2.51 (m, 1H), 2.44 - 2.34 (m, 2H), 2.10 - 2.04 (m, 1H). LCMS (ESI) calcd for $C_{33}H_{29}F_6N_4O_4^+$ $[M+H]^+$: 659.21, found, 659.3.

**Example 182: preparation of 5-((4-(bis(3,5-difluorophenyl)methyl)piperazin-1-yl)methyl)-2-(2,6-dioxopiperidin-3-yl)isoindoline-1,3-dione (GT-06596)**

Referring to the method of Scheme 4, the target product (GT-06596) was prepared as white solid (58 mg, yield 66%). $^1$H NMR (400 MHz, DMSO-d6) δ 11.15 (s, 1H), 10.96 (s, 1H), 8.21 (s, 1H), 8.10 - 8.02 (m, 2H), 7.24 - 7.21 (m, 4H), 7.17 - 7.12 (m, 2H), 5.19 (dd, J = 12.7, 5.4 Hz, 1H), 4.68 (s, 1H), 4.53 (s, 2H), 3.21 - 3.12 (m, 4H), 3.00 - 2.77 (m, 3H), 2.62 (d, J = 18.9 Hz, 1H), 2.45 - 2.40 (m, 3H), 2.14 - 1.98 (m, 1H). LCMS (ESI) calcd for $C_{31}H_{27}F_4N_4O_4^+$ $[M+H]^+$: 595.20, found, 595.2.

**Example 183: preparation of 5-((4-(bis(4-chlorophenyl)methyl)piperazin-1-yl)methyl)-2-(2,6-dioxopiperidin-3-yl)isoindoline-1,3-dione (GT-05727)**

Referring to the method of Scheme 4, the target product (GT-05727) was prepared as white solid (48 mg, yield 49%). $^1$H NMR (400 MHz, DMSO) δ 11.14 (s, 1H), 8.18 (s, 1H), 8.07 - 8.01 (m, 2H), 7.53 - 7.32 (m, 8H), 5.18 (dd, *J* = 12.7, 5.4 Hz, 1H), 4.61 (brs, 1H), 4.52 (brs, 2H), 3.37 - 3.22 (m, 4H), 3.19 - 3.09 (m, 3H), 2.86 - 2.77 (m, 2H), 2.61 (d, *J* = 14.4 Hz, 2H), 2.43 - 2.32 (m, 2H), 2.11 - 1.99 (m, 1H). LCMS (ESI) calcd for $C_{31}H_{29}Cl_2N_4O_4^+$ $[M+H]^+$: 591.16, found, 591.2.

**Example 184: preparation of 5-((4-(2,7-dichloro-9H-fluoren-9-yl)piperazin-1-yl)methyl)-2-(2,6-dioxopiperidin-3-yl)isoindoline-1,3-dione (GT-06049)**

Referring to the method of Scheme 4, the target product (GT-06049) was prepared as white solid (65 mg, yield 74%). $^1$H NMR (400 MHz, DMSO-d6) δ 11.15 (s, 1H), 8.16 (s, 1H), 8.03 (s, 2H), 7.92 (d, J = 8.2 Hz, 2H), 7.63 (s, 2H), 7.52 (d, J = 8.1 Hz, 2H), 5.22 - 5.13 (m, 2H), 4.52 (s, 2H), 3.30 - 3.27 (m, 2H), 3.16 - 3.09 (m, 2H), 2.96 - 2.69 (m, 6H), 2.61 (d, J = 19.0 Hz, 1H), 2.09 - 2.05 (m, 1H). LCMS (ESI) calcd for $C_{31}H_{27}Cl_2N_4O_4^+$ $[M+H]^+$: 589.14, found, 589.2.

**Example 185: preparation of 5-((4-(9H-fluoren-9-yl)piperazin-1-yl)methyl)-2-(2,6-dioxopiperidin-3-yl)isoindoline-1,3-dione (GT-05908)**

Referring to the method of Scheme 4, the target product (GT-05908) was prepared as white solid (15 mg, yield 15%). $^1$H NMR (400 MHz, DMSO-d6) δ 11.14 (s, 1H), 8.17 (s, 1H), 8.06 (d, J = 7.7 Hz, 1H), 8.00 (d, J = 7.6 Hz, 1H), 7.88 (d, J = 7.5 Hz, 2H), 7.72 (s, 2H), 7.46 (t, J = 7.5 Hz, 2H), 7.37 (t, J = 7.4 Hz, 2H), 5.25 (s, 1H), 5.17 (dd, J = 12.8, 5.4 Hz, 1H), 4.49

(s, 2H), 3.29 (s, 2H), 3.18 (s, 2H), 3.07 - 2.83 (m, 5H), 2.61 (d, J = 18.7 Hz, 1H), 2.55 - 2.50 (m, 1H), 2.09 - 2.05 (m, 1H). LCMS (ESI) calcd for $C_{31}H_{29}N_4O_4^+$ $[M+H]^+$: 521.22, found, 521.3.

**Example 186: preparation of 5-((4-(bis(4-(benzyloxy)phenyl)methyl)piperazin-1-yl)methyl)-2-(2,6-dioxopiperidin-3-yl)isoindoline-1,3-dione (GT-06425)**

Referring to the method of Scheme 4, the target product (GT-06425) was prepared as white solid (41 mg, yield 53%). $^1H$ NMR (400 MHz, DMSO-d6) δ 11.15 (s, 1H), 8.18 - 8.13 (m, 1H), 8.09 - 7.93 (m, 2H), 7.72 - 7.49 (m, 2H), 7.44 - 7.35 (m, 8H), 7.36 - 7.28 (m, 2H), 7.23 (d, J = 8.6 Hz, 1H), 7.07 - 7.00 (m, 3H), 6.93 (d, J = 8.7 Hz, 1H), 5.18 (dd, J = 12.8, 5.4 Hz, 1H), 5.07 (s, 4H), 4.39 (brs, 2H), 3.33 - 3.03 (m, 8H), 2.93 - 2.87 (m, 2H), 2.64 - 2.54 (m, 1H), 2.57 - 2.52 (m, 1H), 2.14 - 1.87 (m, 1H). LCMS (ESI) calcd for $C_{45}H_{42}N_4NaO_6^+$ $[M+Na]^+$: 757.30, found, 757.3.

**Example 187: preparation of 2-(2,6-dioxopiperidin-3-yl)-5-((4-(diphenylamino)piperidin-1-yl)methyl)isoindoline-1,3-dione (GT-05741)**

Referring to the method of Scheme 4, the target product (GT-05741) was prepared as white solid (12 mg, yield 26%). $^1H$ NMR (400 MHz, DMSO) δ 11.15 (s, 1H), 9.83 (s, 1H), 8.12 (s, 1H), 8.05 - 7.99 (m, 2H), 7.29 (t, *J* = 7.9 Hz, 4H), 7.01 (t, *J* = 7.3 Hz, 2H), 6.82 (d, *J* = 7.7 Hz, 4H), 5.18 (dd, *J* = 12.8, 5.3 Hz, 1H), 4.47 (d, *J* = 4.1 Hz, 2H), 4.23 (t, *J* = 9.5 Hz, 1H), 3.24 - 3.16 (m, 4H), 2.93 - 2.86 (m, 1H), 2.61 (d, *J* = 19.6 Hz, 1H), 2.55 - 2.51 (m, 1H), 2.11 - 2.05 (m, 3H), 1.68 - 1.51 (m, 2H). LCMS (ESI) calcd for $C_{31}H_{31}N_4O_4^+$ $[M+H]^+$: 523.23, found, 523.3.

**Example 188: preparation of 2-(2,6-dioxopiperidin-3-yl)-5-((4-(phenyl(pyridin-2-yl)amino)piperidin-1-yl)methyl) isoindoline-1,3-dione (GT-05742)**

Referring to the method of Scheme 4, the target product (GT-05742) was prepared as white solid (32 mg, yield 33%). $^1H$ NMR (400 MHz, DMSO-d6) δ 11.15 (s, 1H), 10.36 (s, 1H), 8.17 (s, 1H), 8.12 (d, *J* = 8.8 Hz, 1H), 8.06 - 8.00 (m, 2H), 7.60 - 7.52 (m, 3H), 7.54 - 7.48 (m, 1H), 7.26 (d, *J* = 7.4 Hz, 2H), 6.80 (brs, 1H), 6.15 (brs, 1H), 5.18 (dd, *J* = 12.7, 5.4 Hz, 1H), 4.99 - 4.93 (m, 1H), 4.49 (s, 2H), 3.34 - 3.11 (m, 4H), 3.00 - 2.82 (m, 1H), 2.61 (d, *J* = 19.1 Hz, 2H), 2.57 - 2.53 (m, 1H), 2.16 - 2.05 (m, 3H), 1.75 - 1.66 (m, 2H). LCMS (ESI) calcd for $C_{30}H_{30}N_5O_4^+$ $[M+H]^+$: 524.23, found, 524.3.

**Example 189: preparation of 5-(4-benzhydrylpiperazine-1-carbonyl)-2-(2,6-dioxopiperidin-3-yl)isoindoline-1,3-dione (GT-05691)**

Referring to the method of Scheme 6, the target product (GT-05691) was prepared as white solid (56 mg, yield 61%). $^1H$ NMR (400 MHz, DMSO-d6) δ 12.23 (s, 1H), 11.07 (s, 1H), 8.03 - 7.65 (m, 6H), 7.56 - 7.05 (m, 7H), 5.44 (s, 1H), 5.11 (dd, J = 12.7, 5.4 Hz, 1H), 4.43 (brs, 1H), 3.81 - 3.73 (m, 1H), 3.71 - 3.58 (m, 2H), 3.19 - 3.14 (m, 2H), 3.08 - 3.03 (m, 1H), 2.91 - 2.76 (m, 1H), 2.56 - 2.51 (m, 1H), 2.51 - 2.45 (m, 2H), 2.03 - 1.98 (m, 1H). LCMS (ESI) calcd for $C_{31}H_{29}N_4O_5^+$ $[M+H]^+$: 537.21, found, 537.2.

**Example 190: preparation of 5-(4-(bis(3,5-difluorophenyl)methyl)piperazine-1-carbonyl)-2-(2,6-dioxopiperidin-3-yl)isoindoline-1,3-dione (GT-06598)**

Referring to the method of Scheme 6, the target product (GT-06598) was prepared as white solid (69 mg, yield 77%). $^1H$ NMR (400 MHz, DMSO-d6) δ 11.15 (s, 1H), 7.98 (d, J = 7.6 Hz, 1H), 7.94 - 7.83 (m, 2H), 7.34 (s, 4H), 7.16 (t, J = 8.4 Hz, 2H), 5.18 (dd, J = 12.8, 5.4 Hz, 1H), 4.72 (s, 1H), 3.77 (s, 2H), 3.30 - 3.14 (m, 4H), 2.97 - 2.83 (m, 1H), 2.61 - 2.55 (m, 4H), 2.09 - 2.04 (m, 1H). LCMS (ESI) calcd for $C_{31}H_{25}F_4N_4O_5^+$ $[M+H]^+$: 609.18, found, 609.2.

**Example 191: preparation of 3-(5-((4-(di(pyridin-2-yl)methyl)piperazin-1-yl)methyl)-1-oxoisoindolin-2-yl)piperidine-2,6-dione (GT-04383)**

Referring to the method of Scheme 4, the target product (GT-04383) was prepared as white solid (26 mg, yield 29%). $^1H$ NMR (400 MHz, DMSO-d6) δ 11.01 (s, 1H), 8.70 (d, J = 4.8 Hz, 2H), 8.15 (dd, J = 12.1, 4.5 Hz, 2H), 7.91 (s, 1H), 7.85 (d, J = 7.9 Hz, 2H), 7.78 (d, J = 8.3 Hz, 2H), 7.67 - 7.59 (m, 2H), 5.87 (s, 1H), 5.13 (dd, J = 13.2, 5.1 Hz, 1H), 4.87 - 4.76 (m, 1H), 3.47 (brs, 1H), 3.38 (brs, 4H), 3.28 - 3.15 (m, 1H), 3.01 (brs, 4H), 2.94 - 2.85 (m, 1H), 2.61 (d, J = 16.8 Hz, 1H), 2.45 - 2.41 (m, 1H), 2.34 - 2.21 (m, 1H), 2.04 - 1.99 (m, 1H). LCMS (ESI) calcd for $C_{29}H_{31}N_6O_3^+$ $[M+H]^+$: 511.25, found, 511.3.

**Example 192: preparation of 3-(5-((4-(di(pyridin-2-yl)methyl)piperazin-1-yl)methyl)-6-fluoro-1-oxoisoindolin-2-yl)piperidine-2,6-dione (GT-04384)**

Referring to the method of Scheme 4, the target product (GT-04384) was prepared as white solid (38 mg, yield 41%). $^1$H NMR (400 MHz, DMSO-d6) δ 11.04 (s, 1H), 8.72 (d, J = 4.8 Hz, 2H), 8.22 - 8.14 (m, 3H), 8.04 (d, J = 6.1 Hz, 1H), 7.85 (d, J = 7.9 Hz, 2H), 7.70 - 7.63 (m, 3H), 5.83 (s, 1H), 5.13 (dd, J = 13.1, 5.2 Hz, 1H), 4.87 - 4.76 (m, 1H), 3.52 (brs, 1H), 3.44 (brs, 5H), 3.28 - 3.25 (m, 2H), 3.02 - 2.87 (m, 5H), 2.61 (d, J = 17.1 Hz, 1H), 2.48 - 2.38 (m, 1H), 2.31 - 2.24 (m, 1H), 2.03 - 1.99 (m, 1H). LCMS (ESI) calcd for $C_{29}H_{30}FN_6O_3^+$ [M+H]$^+$: 529.24, found, 529.3.

**Example 193: preparation of 3-(5-((4-(di(pyridin-3-yl)methyl)piperazin-1-yl)methyl)-1-oxoisoindolin-2-yl)piperidine-2,6-dione (GT-04385)**

Referring to the method of Scheme 4, the target product (GT-04385) was prepared as white solid (36 mg, yield 32%). $^1$H NMR (400 MHz, DMSO-d6) δ 9.18 (d, J = 6.2 Hz, 2H), 8.91 (s, 1H), 8.78 (d, J = 4.9 Hz, 1H), 8.66 (d, J = 8.1 Hz, 1H), 8.31 (d, J = 7.7 Hz, 1H), 8.17 (dd, J = 8.1, 6.2 Hz, 1H), 7.89 - 7.75 (m, 3H), 7.69 (d, J = 7.9 Hz, 1H), 6.05 (s, 2H), 5.37 (s, 1H), 4.81 (dd, J = 10.8, 3.8 Hz, 1H), 4.50 (d, J = 2.7 Hz, 2H), 3.17 (s, 4H), 2.59 (s, 4H), 2.31 - 2.22 (m, 3H), 2.08 - 2.04 (m, 1H). LCMS (ESI) calcd for $C_{29}H_{31}N_6O_3^+$ [M+H]$^+$: 511.25, found, 511.3.

**Example 194: preparation of 3-(5-((4-(di(pyridin-3-yl)methyl)piperazin-1-yl)methyl)-6-fluoro-1-oxoisoindolin-2-yl)piperidine-2,6-dione (GT-04386)**

Referring to the method of Scheme 4, the target product (GT-04386) was prepared as white solid (32 mg, yield 28%). $^1$H NMR (400 MHz, DMSO-d6) δ 11.04 (s, 1H), 9.38 (s, 1H), 9.07 (d, J = 6.0 Hz, 2H), 8.74 (s, 1H), 8.65 (t, J = 7.7 Hz, 2H), 8.21 - 8.13 (m, 1H), 7.97 (d, J = 7.9 Hz, 1H), 7.84 (t, J = 5.4 Hz, 1H), 7.67 (d, J = 8.9 Hz, 1H), 7.61 - 7.53 (m, 1H), 6.12 (s, 2H), 5.24 (s, 1H), 5.13 (dd, J = 13.4, 4.9 Hz, 1H), 4.42 (dd, J = 51.3, 17.4 Hz, 2H), 3.15 (brs, 4H), 2.97 - 2.85 (m, 1H), 2.66 - 2.61 (m, 1H), 2.57 (brs, 4H), 2.45 - 2.40 (m, 1H), 2.05 - 2.01 (m, 1H). LCMS (ESI) calcd for $C_{29}H_{30}FN_6O_3^+$ [M+H]$^+$: 529.24, found, 529.3.

**Example 195: preparation of 3-(5-(4-benzhydrylpiperazine-1-carbonyl)-7-fluoro-1-oxoisoindolin-2-yl)piperidine-2,6-dione (GT-05850)**

Referring to the method of Scheme 6, the target product (GT-05850) was prepared as white solid (48 mg, yield 68.18%). $^1$H NMR (400 MHz, DMSO) δ 12.18 (s, 1H), 11.01 (s, 1H), 7.84 (s, 3H), 7.41 (dd, *J* = 39.1, 13.0 Hz, 9H), 5.09 (dd, *J* = 13.3, 5.0 Hz, 1H), 4.45 (dd, *J* = 48.9, 18.1 Hz, 3H), 3.55 (s, 4H), 3.17 (s, 2H), 2.89 (dd, *J* = 21.6, 9.2 Hz, 1H), 2.60 (d, *J* = 17.1 Hz, 2H), 2.39 (dt, *J* = 26.0, 13.0 Hz, 2H), 2.05 - 1.93 (m, 1H). LCMS (ESI) calcd for $C_{31}H_{30}FN_4O_4^+$ [M+H]$^+$: 541.22, found, 541.2.

**Example 196: preparation of 3-(6-(4-benzhydrylpiperazine-1-carbonyl)-1-oxoisoindolin-2-yl)piperidine-2,6-dione (GT-05851)**

Referring to the method of Scheme 6, the target product (GT-05851) was prepared as white solid (41 mg, yield 60.11%). $^1$H NMR (400 MHz, DMSO-d6) δ 12.09 (s, 1H), 11.00 (s, 1H), 7.80 (d, J= 31.5 Hz, 4H), 7.69 (q, *J*= 8.0 Hz, 2H), 7.44 (s, 4H), 7.38 (s, 2H), 5.54 (s, 1H), 5.13 (dd, *J*= 13.3, 5.1 Hz, 1H), 4.51 (d, *J* = 17.8 Hz, 1H), 4.38 (d, *J* = 17.8 Hz, 1H), 3.62 (s, 4H), 3.20 (s, 3H), 2.94 - 2.84 (m, 1H), 2.63 (t, *J* = 17.7 Hz, 2H), 2.40 (dt, *J* = 13.2, 8.7 Hz, 1H), 2.06 - 1.95 (m, 1H). LCMS (ESI) calcd for $C_{31}H_{31}N_4O_4^+$ [M+H]$^+$: 523.23, found, 523.3.

**Example 197: preparation of 3-(7-(4-benzhydrylpiperazine-1-carbonyl)-1-oxoisoindolin-2-yl)piperidine-2,6-dione (GT-05852)**

Referring to the method of Scheme 6, the target product (GT-05852) was prepared as white solid (27 mg, yield 39.59%). $^1$H NMR (400 MHz, DMSO) δ 12.10 (s, 1H), 11.01 (s, 1H), 7.86 (d, *J* = 73.6 Hz, 3H), 7.67 (s, 2H), 7.38 (s, 7H), 5.49 (d, *J* = 66.6 Hz, 1H), 5.05 (d, *J* = 7.6 Hz, 1H), 4.43 (dd, *J* = 31.3, 14.4 Hz, 3H), 3.54 (s, 2H), 3.02 (s, 2H), 2.88 (d, *J* = 10.0 Hz, 2H), 2.68 (d, *J* = 39.7 Hz, 2H), 2.42 (s, 1H), 2.02 (s, 1H), 1.34 - 1.19 (m, 1H). LCMS (ESI) calcd for $C_{31}H_{31}N_4O_4^+$ [M+H]$^+$: 523.23, found, 523.3.

**Example 198: preparation of 4-(4-benzhydrylpiperazine-1-carbonyl)-2-(2,6-dioxopiperidin-3-yl)isoindoline-1,3-dione (GT-05853)**

Referring to the method of Scheme 6, the target product (GT-05853) was prepared as white solid (33 mg, yield 47.20%). $^1$H NMR (400 MHz, DMSO-d6) δ 12.07 (s, 1H), 11.16 (s, 1H), 7.96 (s, 3H), 7.77 (s, 3H), 7.39 (s, 8H), 5.14 (dd, *J* = 12.8, 5.3 Hz, 1H), 4.59 (s, 1H), 3.41 (s, 4H), 3.03 (s, 3H), 2.89 (t, *J* = 12.7 Hz, 2H), 2.69 (d, *J* = 36.2 Hz, 2H), 2.05 (d, *J* = 5.8 Hz, 1H). LCMS (ESI) calcd for $C_{31}H_{29}N_4O_5^+$ [M+H]$^+$: 537.21, found, 537.2.

**Example 199: preparation of 3-(1-oxo-5-(4-(phenyl(pyridin-2-yl)methyl)piperazine-1-carbonyl)isoindolin-2-yl) piperidine-2,6-dione (GT-05854)**

Referring to the method of Scheme 6, the target product (GT-05854) was prepared as white solid (23 mg, yield 33.66%). $^1$H NMR (400 MHz, DMSO) δ 11.01 (s, 1H), 8.76 (d, *J* = 4.1 Hz, 1H), 7.93 (dd, *J* = 7.7, 6.3 Hz, 1H), 7.80 (d, *J* = 7.8 Hz, 1H), 7.67 (t, *J* = 7.0 Hz, 4H), 7.56 (d, *J* = 7.6 Hz, 1H), 7.51 - 7.34 (m, 4H), 5.78 (s, 1H), 5.13 (dd, *J* = 13.2, 5.0 Hz, 1H), 4.44 (dd, *J* = 49.0, 17.6 Hz, 2H), 3.95 (s, 2H), 3.68 (s, 2H), 3.19 (s, 4H), 2.96 - 2.86 (m, 1H), 2.61 (d, *J* = 17.1 Hz, 1H), 2.44 - 2.32 (m, 1H), 2.00 (d, *J* = 8.1 Hz, 1H). LCMS (ESI) calcd for $C_{30}H_{30}N_5O_4^+$ [M+H]$^+$: 524.23, found, 524.3.

**Example 200: preparation of 3-(7-fluoro-1-oxo-5-(4-(phenyl(pyridin-2-yl)methyl)piperazine-1-carbonyl)isoindolin-2-yl)piperidine-2,6-dione (GT-05877)**

Referring to the method of Scheme 6, the target product (GT-05877) was prepared as white solid (25 mg, yield 35.45%). $^1$H NMR (400 MHz, DMSO-d6) δ 11.01 (s, 1H), 8.75 (s, 1H), 7.92 (t, *J* = 7.2 Hz, 1H), 7.62 (t, *J* = 7.3 Hz, 3H), 7.42 (ddd, *J* = 29.5, 14.4, 8.0 Hz, 6H), 5.64 (s, 1H), 5.09 (dd, *J* = 13.3, 5.1 Hz, 1H), 4.45 (dd, *J* = 49.2, 18.2 Hz, 2H), 3.88 (s, 2H), 3.62 (s, 2H), 3.08 - 2.86 (m, 4H), 2.60 (d, *J* = 18.4 Hz, 2H), 2.38 (dd, *J* = 13.2, 4.5 Hz, 1H), 2.03 - 1.96 (m, 1H). LCMS (ESI) calcd for $C_{30}H_{29}FN_5O_4^+$ [M+H]$^+$: 542.22, found, 542.2.

**Example 201: preparation of 2-(2,6-dioxopiperidin-3-yl)-5-(4-(phenyl(pyridin-2-yl)methyl)piperazine-1-carbonyl)isoindoline-1,3-dione (GT-05878)**

Referring to the method of Scheme 6, the target product (GT-05878) was prepared as brown solid (36 mg, yield 51.40%). $^1$H NMR (400 MHz, DMSO-d6) δ 11.15 (s, 1H), 8.75 (d, *J* = 4.4 Hz, 1H), 8.05 - 7.98 (m, 1H), 7.98 - 7.94 (m, 1H), 7.94 - 7.85 (m, 2H), 7.66 (d, *J* = 7.3 Hz, 3H), 7.51 - 7.37 (m, 4H), 5.71 (s, 1H), 5.18 (dd, *J* = 12.8, 5.4 Hz, 1H), 3.95 (s, 2H), 3.63 (s, 2H), 3.17 (s, 3H), 3.03 (s, 1H), 2.98 - 2.82 (m, 2H), 2.58 (dd, *J* = 24.7, 11.4 Hz, 2H). LCMS (ESI) calcd for $C_{30}H_{28}N_5O_5^+$ [M+H]$^+$: 538.21, found, 538.2.

**Example 202: preparation of 5-(4-(di(pyridin-2-yl)methyl)piperazine-1-carbonyl)-2-(2,6-dioxopiperidin-3-yl)isoindoline-1,3-dione (GT-05880)**

Referring to the method of Scheme 6, the target product (GT-05880) was prepared as white solid (17 mg, yield 26.88%). $^1$H NMR (400 MHz, DMSO-d6) δ 11.14 (s, 1H), 8.69 (d, *J* = 4.4 Hz, 2H), 8.08 - 7.98 (m, 2H), 7.98 - 7.86 (m, 4H), 7.72 (d, *J* = 7.9 Hz, 2H), 7.48 (dd, *J* = 7.2, 5.2 Hz, 2H), 5.90 (s, 1H), 5.18 (dd, *J* = 12.7, 5.3 Hz, 1H), 3.96 (s, 2H), 3.16 (s, 4H), 2.90 (s, 2H), 2.64 - 2.55 (m, 3H), 2.09 - 2.01 (m, 1H). LCMS (ESI) calcd for $C_{29}H_{27}N_6O_5^+$ [M+H]$^+$: 539.20, found, 539.3.

**Example 203: preparation of 3-(5-(4-(bis(4-fluorophenyl)methyl)piperazine-1-carbonyl)-1-oxoisoindolin-2-yl)piperidine-2,6-dione (GT-05881)**

Referring to the method of Scheme 6, the target product (GT-05881) was prepared as white solid (31 mg, yield 46.93%). $^1$H NMR (400 MHz, DMSO-d6) δ 12.17 (s, 1H), 11.00 (s, 1H), 7.84 (s, 1H), 7.79 (d, *J* = 7.7 Hz, 3H), 7.65 (s, 1H), 7.54 (d, *J* = 7.7 Hz, 1H), 7.27 (s, 4H), 5.13 (dd, *J* = 13.2, 5.1 Hz, 1H), 4.49 (d, *J* = 17.6 Hz, 1H), 4.37 (d, *J* = 17.6 Hz, 1H), 3.50 (s, 6H), 3.11 (s, 1H), 3.03 - 2.85 (m, 2H), 2.60 (d, *J* = 16.0 Hz, 2H), 2.44 - 2.35 (m, 1H), 2.05 - 1.97 (m, 1H). LCMS (ESI) calcd for $C_{31}H_{29}F_2N_4O_4^+$ [M+H]$^+$: 559.22, found, 559.2.

**Example 204: preparation of 3-(5-(4-(bis(4-fluorophenyl)methyl)piperazine-1-carbonyl)-7-fluoro-1-oxoisoindolin-2-yl)piperidine-2,6-dione (GT-05882)**

Referring to the method of Scheme 6, the target product (GT-05882) was prepared as white solid (24 mg, yield 35.27%). $^1$H NMR (400 MHz, DMSO-d6) δ 11.02 (s, 1H), 7.74 (s, 3H), 7.47 (s, 2H), 7.37 - 7.24 (m, 5H), 7.17 (t, *J* = 8.9 Hz, 1H), 5.09 (dd, *J* = 13.3, 5.1 Hz, 1H), 4.51 (d, *J* = 18.1 Hz, 1H), 4.39 (d, *J* = 18.1 Hz, 1H), 3.60 (s, 4H), 3.14 (s, 2H), 2.96 - 2.86

(m, 2H), 2.60 (d, *J* = 17.1 Hz, 2H), 2.42 - 2.33 (m, 1H), 2.04 - 1.95 (m, 1H). LCMS (ESI) calcd for $C_{31}H_{28}F_3N_4O_4^+$ [M+H]$^+$: 577.21, found, 577.2.

**Example 205: preparation of 3-(6-(4-(bis(4-fluorophenyl)methyl)piperazine-1-carbonyl)-1-oxoisoindolin-2-yl)piperidine-2,6-dione (GT-05883)**

Referring to the method of Scheme 6, the target product (GT-05883) was prepared as white solid (29 mg, yield 43.91%). $^1$H NMR (400 MHz, DMSO-d6) δ 12.26 (s, 1H), 11.00 (s, 1H), 7.86 (s, 2H), 7.77 - 7.59 (m, 4H), 7.27 (s, 4H), 5.12 (dd, *J* = 13.3, 5.1 Hz, 1H), 4.51 (d, *J* = 17.8 Hz, 1H), 4.38 (d, *J* = 17.8 Hz, 1H), 3.62 (s, 2H), 3.39 (s, 2H), 3.35 - 3.32 (m, 2H), 3.04 (d, *J* = 68.1 Hz, 3H), 2.95 - 2.86 (m, 1H), 2.61 (d, *J* = 16.8 Hz, 1H), 2.45 - 2.34 (m, 1H), 2.05 - 1.96 (m, 1H). LCMS (ESI) calcd for $C_{31}H_{29}F_2N_4O_4^+$ [M+H]$^+$: 559.22, found, 559.2.

**Example 206: preparation of 3-(7-(4-(bis(4-fluorophenyl)methyl)piperazine-1-carbonyl)-1-oxoisoindolin-2-yl)piperidine-2,6-dione (GT-05884)**

Referring to the method of Scheme 6, the target product (GT-05884) was prepared as white solid (24 mg, yield 36.34%). $^1$H NMR (400 MHz, DMSO-d6) δ 12.34 (s, 1H), 11.03 (s, 1H), 7.89 (d, *J* = 46.5 Hz, 3H), 7.67 (t, *J* = 6.9 Hz, 2H), 7.37 (d, *J* = 6.9 Hz, 1H), 7.27 (s, 4H), 5.46 (s, 1H), 5.05 (dd, *J* = 13.1, 5.2 Hz, 1H), 4.43 (dd, *J* = 34.6, 14.2 Hz, 2H), 3.51 (s, 2H), 3.34 - 3.25 (m, 2H), 3.15 (s, 2H), 2.89 (t, *J* = 13.3 Hz, 2H), 2.65 (s, 2H), 2.42 (d, *J*= 12.9 Hz, 1H), 2.02 (s, 1H). LCMS (ESI) calcd for $C_{31}H_{29}F_2N_4O_4^+$ [M+H]$^+$: 559.22, found, 559.2.

**Example 207: preparation of 5-(4-(bis(4-fluorophenyl)methyl)piperazine-1-carbonyl)-2-(2,6-dioxopiperidin-3-yl) isoindoline-1,3-dione (GT-05885)**

Referring to the method of Scheme 6, the target product (GT-05885) was prepared as white solid (24 mg, yield 35.50%). $^1$H NMR (400 MHz, DMSO-d6) δ 12.42 (s, 1H), 11.14 (s, 1H), 8.00 (d, *J* = 7.6 Hz, 1H), 7.96 - 7.62 (m, 5H), 7.60 - 7.10 (m, 5H), 5.18 (dd, *J* = 12.8, 5.4 Hz, 1H), 4.37 (d, *J* = 62.5 Hz, 1H), 3.62 (s, 2H), 3.35 - 3.27 (m, 2H), 3.15 (s, 2H), 3.04 - 2.83 (m, 2H), 2.70 - 2.52 (m, 3H), 2.16 - 1.96 (m, 1H). LCMS (ESI) calcd for $C_{31}H_{27}F_2N_4O_5^+$ [M+H]$^+$: 573.19, found, 573.2.

**Example 208: preparation of 3-(5-(4-(bis(4-chlorophenyl)methyl)piperazine-1-carbonyl)-1-oxoisoindolin-2-yl) piperidine-2,6-dione (GT-05886)**

Referring to the method of Scheme 6, the target product (GT-05886) was prepared as white solid (15 mg, yield 23.65%). $^1$H NMR (400 MHz, DMSO-d6) δ 11.00 (s, 1H), 7.78 (d, *J* = 7.9 Hz, 3H), 7.64 (s, 3H), 7.48 (dd, *J* = 34.5, 14.9 Hz, 6H), 5.13 (dd, *J* = 13.4, 5.1 Hz, 1H), 4.49 (d, *J* = 17.6 Hz, 1H), 4.42 - 4.29 (m, 1H), 3.61 (s, 2H), 3.30 - 3.21 (m, 2H), 3.12 (s, 1H), 2.96 - 2.83 (m, 2H), 2.60 (d, *J* = 16.7 Hz, 2H), 2.44 2.28 (m, 2H), 2.07 - 1.94 (m, 1H), 1.36 - 1.13 (m, 1H). LCMS (ESI) calcd for $C_{31}H_{29}Cl_2N_4O_4^+$ [M+H]$^+$: 591.16, found, 591.2.

**Example 209: preparation of 3-(5-(4-(bis(4-chlorophenyl)methyl)piperazine-1-carbonyl)-7-fluoro-1-oxoisoindolin-2-yl)piperidine-2,6-dione (GT-05887)**

Referring to the method of Scheme 6, the target product (GT-05887) was prepared as white solid (30 mg, yield 45.98%). $^1$H NMR (400 MHz, DMSO-d6) δ 11.01 (s, 1H), 7.66 (s, 4H), 7.46 (s, 6H), 7.34 (d, *J*= 9.4 Hz, 1H), 5.09 (dd, *J* = 13.3, 5.1 Hz, 1H), 4.50 (d, *J* = 17.9 Hz, 1H), 4.38 (d, *J* = 18.1 Hz, 1H), 3.03 (s, 2H), 2.96 - 2.85 (m, 2H), 2.60 (d, *J* = 17.2 Hz, 2H), 2.46 - 2.31 (m, 2H), 2.04 - 1.94 (m, 1H), 1.72 (d, *J* = 12.6 Hz, 1H), 1.62 (d, *J* = 12.3 Hz, 1H), 1.31 - 1.18 (m, 1H), 1.13 - 0.98 (m, 1H). LCMS (ESI) calcd for $C_{31}H_{28}Cl_2FN_4O_4^+$ [M+H]$^+$: 609.15, found, 609.2.

**Example 210: preparation of 3-(6-(4-(bis(4-chlorophenyl)methyl)piperazine-1-carbonyl)-1-oxoisoindolin-2-yl) piperidine-2,6-dione (GT-05888)**

Referring to the method of Scheme 6, the target product (GT-05888) was prepared as white solid (37 mg, yield 58.34%). $^1$H NMR (400 MHz, DMSO-d6) δ 11.00 (s, 1H), 7.89 - 7.55 (m, 7H), 7.48 (s, 4H), 5.12 (dd, *J* = 13.3, 5.2 Hz, 1H), 4.51 (d, *J* = 17.7 Hz, 1H), 4.38 (d, *J* = 17.7 Hz, 1H), 3.48 (s, 2H), 2.99 - 2.84 (m, 2H), 2.60 (d, *J* = 17.3 Hz, 2H), 2.41 (dt, *J* = 13.6, 11.2 Hz, 2H), 2.07 - 1.96 (m, 1H), 1.78 - 1.45 (m, 2H), 1.30 - 0.98 (m, 2H). LCMS (ESI) calcd for $C_{31}H_{29}Cl_2N_4O_4^+$ [M+H]$^+$: 591.16, found, 591.2.

**Example 211: preparation of 3-(7-(4-(bis(4-chlorophenyl)methyl)piperazine-1-carbonyl)-1-oxoisoindolin-2-yl) piperidine-2,6-dione (GT-05889)**

Referring to the method of Scheme 6, the target product (GT-05889) was prepared as white solid (21 mg, yield 33.11%). $^1$H NMR (400 MHz, DMSO-d6) δ 11.04 (s, 1H), 7.73 (dd, *J*= 44.2, 36.1 Hz, 6H), 7.44 (d, *J*= 21.5 Hz, 5H), 7.35 (d, *J* = 6.4 Hz, 1H), 5.05 (dd, *J* = 13.1, 5.3 Hz, 1H), 4.49 (dd, *J*= 17.5, 9.9 Hz, 1H), 4.38 (d, *J* = 17.7 Hz, 1H), 3.59 (s, 2H), 3.27 - 3.19 (m, 1H), 3.05 (s, 3H), 2.96 - 2.83 (m, 2H), 2.63 (d, *J* = 16.5 Hz, 2H), 2.48 - 2.28 (m, 2H), 2.00 (s, 1H). LCMS (ESI) calcd for $C_{31}H_{29}Cl_2N_4O_4^+$ $[M+H]^+$: 591.16, found, 591.2.

**Example 212: preparation of 5-(4-(bis(4-chlorophenyl)methyl)piperazine-1-carbonyl)-2-(2,6-dioxopiperidin-3-yl)isoindoline-1,3-dione (GT-05890)**

Referring to the method of Scheme 6, the target product (GT-05890) was prepared as white solid (26 mg, yield 40.10%). $^1$H NMR (400 MHz, DMSO-d6) δ 11.14 (s, 1H), 7.99 (d, *J* = 7.6 Hz, 1H), 7.90 *(dd, J*= 22.1, 12.0 Hz, 3H), 7.66 (s, 3H), 7.47 (s, 4H), 5.17 *(dd, J*= 12.7, 5.4 Hz, 1H), 3.53 (s, 2H), 3.33 - 3.27 (m, 2H), 3.08 (s, 2H), 2.95 - 2.85 (m, 2H), 2.73 (s, 1H), 2.66 - 2.52 (m, 3H), 2.11 - 2.00 (m, 1H). LCMS (ESI) calcd for $C_{31}H_{27}Cl_2N_4O_5^+$ $[M+H]^+$: 605.14, found, 605.1.

**Example 213: preparation of 3-(5-(3-benzhydryl-3,6-diazabicyclo[3.1.1]heptane-6-carbonyl)-1-oxoisoindolin-2-yl)piperidine-2,6-dione (GT-05891)**

Referring to the method of Scheme 6, the target product (GT-05891) was prepared as white solid (25 mg, yield 50.90%). $^1$H NMR (400 MHz, DMSO-d6) δ 12.39 (s, 1H), 11.02 (s, 1H), 8.01 (s, 1H), 7.97 (s, 2H), 7.83 (d, *J* = 7.6 Hz, 2H), 7.37 (s, 7H), 5.15 (dd, *J* = 13.1, 4.6 Hz, 1H), 4.55 (s, 2H), 4.51 (s, 1H), 4.42 (d, *J* = 17.7 Hz, 1H), 3.58 (s, 2H), 3.19 (s, 2H), 3.02 - 2.82 (m, 2H), 2.62 (d, *J* = 16.9 Hz, 3H), 2.48 - 2.39 (m, 1H), 2.09 - 1.97 (m, 1H). LCMS (ESI) calcd for $C_{32}H_{31}N_4O_4^+$ $[M+H]^+$: 535.23, found, 535.3.

**Example 214: preparation of 3-(5-(3-benzhydryl-3,6-diazabicyclo[3.1.1]heptane-6-carbonyl)-7-fluoro-1-oxoisoindolin-2-yl)piperidine-2,6-dione (GT-05916)**

Referring to the method of Scheme 6, the target product (GT-05916) was prepared as white solid (27 mg, yield 53.30%). $^1$H NMR (400 MHz, DMSO-d6) δ 11.03 (s, 1H), 7.75 (d, *J* = 8.3 Hz, 1H), 7.57 (d, *J* = 9.6 Hz, 5H), 7.30 (d, *J* = 33.9 Hz, 6H), 5.09 (d, *J* = 8.8 Hz, 1H), 4.54 (d, *J* = 19.3 Hz, 2H), 4.42 (d, *J* = 18.1 Hz, 2H), 3.54 (s, 2H), 3.36 (s, 2H), 2.98 - 2.82 (m, 2H), 2.76 - 2.55 (m, 3H), 2.42 - 2.32 (m, 1H), 2.07 - 2.00 (m, 1H). LCMS (ESI) calcd for $C_{32}H_{30}FN_4O_4^+$ $[M+H]^+$: 553.22, found, 553.3.

**Example 215: preparation of 5-(3-benzhydryl-3,6-diazabicyclo[3.1.1]heptane-6-carbonyl)-2-(2,6-dioxopiperidin-3-yl)isoindoline-1,3-dione (GT-05918)**

Referring to the method of Scheme 6, the target product (GT-05918) was prepared as white solid (22 mg, yield 43.72%). $^1$H NMR (400 MHz, DMSO-d6) δ 11.16 (s, 1H), 8.24 - 8.11 (m, 2H), 8.03 (d, J = 7.7 Hz, 1H), 7.58 (s, 3H), 7.36 (s, 4H), 7.28 (s, 3H), 5.19 (dd, *J* = 12.8, 5.4 Hz, 1H), 4.52 (s, 2H), 3.61 (s, 2H), 3.36 - 3.29 (m, 2H), 3.00 - 2.78 (m, 2H), 2.74 (s, 1H), 2.59 (dd, *J* = 24.1, 11.5 Hz, 3H), 2.13 - 2.03 (m, 1H). LCMS (ESI) calcd for $C_{32}H_{29}N_4O_5^+$ $[M+H]^+$: 549.21, found, 549.3.

**Example 216: preparation of 3-(5-(4-benzhydryl-1,4-diazepane-1-carbonyl)-1-oxoisoindolin-2-yl)piperidine-2,6-dione (GT-05892)**

Referring to the method of Scheme 6, the target product (GT-05892) was prepared as white solid (7.8 mg, yield 31.65%). $^1$H NMR (400 MHz, DMSO-d6) δ 11.79 (s, 1H), 11.01 (s, 1H), 7.93 - 7.74 (m, 4H), 7.64 (dd, *J* = 30.9, 13.9 Hz, 2H), 7.55 - 7.39 (m, 4H), 7.38 (d, *J* = 7.0 Hz, 2H), 5.68 (t, *J* = 23.7 Hz, 1H), 5.14 (dd, *J* = 12.8, 4.9 Hz, 1H), 4.63 - 4.45 (m, 1H), 4.44 - 4.30 (m, 1H), 4.19 (d, *J* = 12.6 Hz, 1H), 3.77 (d, *J* = 14.9 Hz, 2H), 3.51 (s, 2H), 3.22 (s, 2H), 2.92 (t, *J* = 12.7 Hz, 1H), 2.61 (d, *J* = 16.9 Hz, 2H), 2.49 - 2.31 (m, 2H), 2.10 (d, *J* = 68.4 Hz, 2H). LCMS (ESI) calcd for $C_{32}H_{33}N_4O_4^+$ $[M+H]^+$: 537.25, found, 537.3.

**Example 217: preparation of 5-(4-benzhydryl-1,4-diazepane-1-carbonyl)-2-(2,6-dioxopiperidin-3-yl)isoindoline-1,3-dione (GT-05919)**

Referring to the method of Scheme 6, the target product (GT-05919) was prepared as white solid (12 mg, yield 47.54%). $^1$H NMR (400 MHz, DMSO-d6) δ 11.75 (s, 1H), 11.15 (s, 1H), 8.03 (d, *J* = 7.2 Hz, 1H), 7.97 (d, *J* = 14.4 Hz, 1H),

7.91 (d, $J$ = 18.3 Hz, 4H), 7.55 - 7.43 (m, 3H), 7.38 (d, $J$ = 6.7 Hz, 3H), 5.67 (d, $J$ = 8.3 Hz, 1H), 5.19 (dd, $J$ = 12.7, 5.5 Hz, 1H), 3.73 (s, 2H), 3.50 (s, 1H), 3.32 - 3.18 (m, 4H), 2.90 (t, $J$ = 12.5 Hz, 1H), 2.58 (dd, $J$ = 23.1, 11.3 Hz, 3H), 2.49 - 2.41 (m, 1H), 2.11 (dd, $J$ = 31.5, 26.4 Hz, 2H). LCMS (ESI) calcd for $C_{32}H_{31}N_4O_5^+$ [M+H]$^+$: 551.23, found, 551.3.

**Example 218: preparation of 3-(5-(4-benzhydryl-2,6-dimethylpiperazine-1-carbonyl)-1-oxoisoindolin-2-yl)piperidine-2,6-dione (GT-05920)**

Referring to the method of Scheme 6, the target product (GT-05920) was prepared as white solid (10 mg, yield 30.41%). $^1$H NMR (400 MHz, DMSO-d6) δ 10.93 (s, 1H), 7.69 (d, $J$ = 7.8 Hz, 1H), 7.51 (s, 1H), 7.42 (dd, $J$ = 21.0, 7.3 Hz, 5H), 7.24 (s, 4H), 7.13 (d, $J$ = 6.8 Hz, 2H), 5.04 (dd, $J$ = 13.3, 5.0 Hz, 1H), 4.41 (d, $J$ = 17.5 Hz, 1H), 4.32 - 4.20 (m, 2H), 3.19 - 3.07 (m, 1H), 2.88 - 2.79 (m, 1H), 2.53 (d, $J$ = 16.7 Hz, 4H), 2.32 (dd, $J$ = 19.8, 10.7 Hz, 1H), 1.96 (dd, $J$ = 25.3, 18.8 Hz, 3H), 1.31 (s, 6H). LCMS (ESI) calcd for $C_{33}H_{35}N_4O_4^+$ [M+H]$^+$: 551.27, found, 551.3.

**Example 219: preparation of 3-(5-(4-benzhydryl-2,6-dimethylpiperazine-1-carbonyl)-7-fluoro-1-oxoisoindolin-2-yl)piperidine-2,6-dione (GT-05921)**

Referring to the method of Scheme 6, the target product (GT-05921) was prepared as white solid (7.3 mg, yield 21.54%). $^1$H NMR (400 MHz, DMSO-d6) δ 11.00 (s, 1H), 7.51 (d, $J$ = 6.3 Hz, 4H), 7.41 (s, 1H), 7.30 (t, $J$ = 8.3 Hz, 5H), 7.19 (d, $J$ = 6.6 Hz, 2H), 5.08 (dd, $J$ = 13.2, 5.1 Hz, 1H), 4.49 (d, $J$ = 18.1 Hz, 1H), 4.40 - 4.28 (m, 2H), 3.46 (d, $J$ = 9.1 Hz, 2H), 2.95 - 2.85 (m, 1H), 2.73 - 2.53 (m, 3H), 2.41 - 2.32 (m, 1H), 2.08 (d, $J$ = 7.7 Hz, 2H), 2.03 - 1.96 (m, 1H), 1.30 (d, $J$ = 54.3 Hz, 6H). LCMS (ESI) calcd for $C_{33}H_{34}FN_4O_4^+$ [M+H]$^+$: 569.26, found, 569.3.

**Example 220: preparation of 5-(4-benzhydryl-2,6-dimethylpiperazine-1-carbonyl)-2-(2,6-dioxopiperidin-3-yl) isoindoline-1,3-dione (GT-05922)**

Referring to the method of Scheme 6, the target product (GT-05922) was prepared as white solid (4.3 mg, yield 12.77%). $^1$H NMR (400 MHz, DMSO-d6) δ 11.07 (s, 1H), 7.89 (d, $J$ = 7.6 Hz, 1H), 7.82 - 7.73 (m, 2H), 7.44 (s, 4H), 7.24 (s, 4H), 7.12 (s, 2H), 5.09 (dd, $J$ = 12.8, 5.4 Hz, 1H), 4.25 (s, 2H), 2.93 - 2.71 (m, 2H), 2.70 - 2.47 (m, 4H), 2.01 (dd, $J$ = 20.3, 14.8 Hz, 3H), 1.30 (s, 6H). LCMS (ESI) calcd for $C_{33}H_{33}N_4O_5^+$ [M+H]$^+$: 565.24, found, 565.3.

**Example 221: preparation of 3-(1-oxo-5-(4-(phenyl(pyridin-2-yl)amino)piperidine-1-carbonyl)isoindolin-2-yl)piperidine-2,6-dione (GT-05923)**

Referring to the method of Scheme 6, the target product (GT-05923) was prepared as white solid (27 mg, yield 37.54%). $^1$H NMR (400 MHz, DMSO-d6) δ 11.00 (s, 1H), 8.08 (d, $J$ = 5.0 Hz, 1H), 7.75 (d, $J$ = 7.7 Hz, 1H), 7.62 (s, 1H), 7.57 (dd, $J$ = 18.3, 7.2 Hz, 4H), 7.43 (d, $J$ = 8.0 Hz, 1H), 7.29 (d, $J$ = 7.4 Hz, 2H), 6.84 (s, 1H), 6.33 (s, 1H), 5.12 (dd, $J$ = 13.2, 5.1 Hz, 1H), 4.87 (s, 1H), 4.58 (s, 1H), 4.48 (d, $J$ = 17.5 Hz, 1H), 4.34 (d, $J$ = 17.6 Hz, 1H), 3.58 (s, 1H), 2.97 - 2.84 (m, 2H), 2.60 (d, $J$ = 17.0 Hz, 1H), 2.47 - 2.27 (m, 2H), 1.98 (dd, $J$ = 23.6, 18.4 Hz, 3H), 1.29 - 1.15 (m, 2H). LCMS (ESI) calcd for $C_{30}H_{30}N_5O_4^+$ [M+H]$^+$: 524.23, found, 524.2.

**Example 222: preparation of 3-(7-fluoro-1-oxo-5-(4-(phenyl(pyridin-2-yl)amino)piperidine-1-carbonyl)isoindolin-2-yl)piperidine-2,6-dione (GT-05924)**

Referring to the method of Scheme 6, the target product (GT-05924) was prepared as white solid (24 mg, yield 32.26%). $^1$H NMR (400 MHz, DMSO-d6) δ 11.01 (s, 1H), 8.07 (d, $J$ = 4.9 Hz, 1H), 7.78 (s, 1H), 7.59 (dq, $J$ = 14.3, 7.1 Hz, 3H), 7.39 (s, 1H), 7.31 (dd, $J$ = 12.9, 8.5 Hz, 3H), 6.92 (s, 1H), 6.43 (s, 1H), 5.09 (dd, $J$ = 13.2, 5.1 Hz, 1H), 4.87 (s, 1H), 4.55 (s, 1H), 4.49 (d, $J$ = 18.0 Hz, 1H), 4.36 (d, $J$ = 18.0 Hz, 1H), 3.37 - 3.26 (m, 2H), 3.00 (s, 1H), 2.95 - 2.81 (m, 1H), 2.60 (d, $J$ = 16.8 Hz, 1H), 2.44 - 2.30 (m, 1H), 2.08 (s, 1H), 1.98 (dd, $J$ = 16.8, 11.7 Hz, 2H), 1.25 (s, 2H). LCMS (ESI) calcd for $C_{30}H_{29}FN_5O_4^+$ [M+H]$^+$: 542.22, found, 542.3.

**Example 223: preparation of 2-(2,6-dioxopiperidin-3-yl)-5-(4-(phenyl(pyridin-2-yl)amino)piperidine-1-carbonyl) isoindoline-1,3-dione (GT-05925)**

Referring to the method of Scheme 6, the target product (GT-05925) was prepared as white solid (32 mg, yield 43.34%). $^1$H NMR (400 MHz, DMSO-d6) δ 11.15 (d, $J$ = 6.8 Hz, 2H), 8.41 (dd, $J$ = 7.8, 1.3 Hz, 1H), 8.28 (s, 1H), 8.07 (t, $J$ = 7.6 Hz, 2H), 7.95 (d, $J$ = 7.6 Hz, 1H), 7.81 (dd, $J$ = 14.1, 6.5 Hz, 2H), 7.61 (t, $J$ = 7.3 Hz, 2H), 7.57 - 7.50 (m, 1H), 7.30 (d, $J$ = 7.3 Hz, 2H), 5.18 (td, $J$ = 13.3, 5.4 Hz, 2H), 4.87 (s, 1H), 3.01 (d, $J$ = 9.4 Hz, 1H), 2.94 - 2.85 (m, 2H), 2.67 - 2.56 (m, 3H), 2.12 - 2.02 (m, 3H), 1.29 (s, 2H). LCMS (ESI) calcd for $C_{30}H_{28}N_5O_5^+$ [M+H]$^+$: 538.21, found, 538.2.

**Example 224: preparation of 3-(5-(4-(diphenylamino)piperidine-1-carbonyl)-1-oxoisoindolin-2-yl)piperidine-2,6-dione (GT-05926)**

Referring to the method of Scheme 6, the target product (GT-05926) was prepared as white solid (18 mg, yield 48.28%). $^1$H NMR (400 MHz, DMSO-d6) δ 11.00 (s, 1H), 7.74 (d, *J* = 7.8 Hz, 1H), 7.55 (s, 1H), 7.41 (d, *J* = 8.0 Hz, 1H), 7.32 - 7.25 (m, 4H), 6.99 (t, *J* = 7.3 Hz, 2H), 6.83 (d, *J* = 7.6 Hz, 4H), 5.12 (dd, *J* = 13.3, 5.1 Hz, 1H), 4.61 - 4.17 (m, 4H), 3.57 (s, 1H), 3.32 - 3.21 (m, 1H), 3.05 - 2.82 (m, 2H), 2.60 (d, *J* = 17.6 Hz, 1H), 2.39 (dd, *J* = 13.0, 4.5 Hz, 1H), 2.07 - 1.94 (m, 2H), 1.89 (s, 1H), 1.20 (s, 2H). LCMS (ESI) calcd for $C_{31}H_{31}N_4O_4^+$ $[M+H]^+$: 523.23, found, 523.2.

**Example 225: preparation of 3-(5-(4-(diphenylamino)piperidine-1-carbonyl)-7-fluoro-1-oxoisoindolin-2-yl)piperidine-2,6-dione (GT-05927)**

Referring to the method of Scheme 6, the target product (GT-05927) was prepared as white solid (17 mg, yield 44.09%). $^1$H NMR (400 MHz, DMSO-d6) δ 11.01 (s, 1H), 7.37 (s, 1H), 7.28 (dd, *J* = 13.4, 5.9 Hz, 5H), 6.99 (t, *J* = 7.3 Hz, 2H), 6.82 (d, *J* = 8.2 Hz, 4H), 5.08 (dd, *J* = 13.4, 5.1 Hz, 1H), 4.42 (dd, *J* = 52.9, 18.0 Hz, 3H), 4.23 (t, *J* = 11.4 Hz, 1H), 3.55 (s, 1H), 2.91 (ddd, *J* = 18.5, 13.5, 6.7 Hz, 2H), 2.59 (d, *J* = 17.3 Hz, 2H), 2.41 - 2.27 (m, 1H), 2.02 (dd, *J* = 21.6, 14.2 Hz, 2H), 1.88 (s, 1H), 1.21 (s, 2H). LCMS (ESI) calcd for $C_{31}H_{30}FN_4O_4^+$ $[M+H]^+$: 541.22, found, 541.3.

**Example 226: preparation of 2-(2,6-dioxopiperidin-3-yl)-5-(4-(diphenylamino)piperidine-1-carbonyl)isoindoline-1,3-dione (GT-05928)**

Referring to the method of Scheme 6, the target product (GT-05928) was prepared as white solid (7 mg, yield 18.29%). $^1$H NMR (400 MHz, DMSO-d6) δ 11.14 (s, 1H), 7.95 (d, *J* = 7.6 Hz, 1H), 7.84 - 7.75 (m, 2H), 7.31 - 7.26 (m, 4H), 6.99 (t, *J* = 7.3 Hz, 2H), 6.82 (t, *J* = 6.8 Hz, 4H), 5.16 (dd, *J* = 12.8, 5.4 Hz, 1H), 4.56 (d, *J* = 12.2 Hz, 1H), 4.23 (t, *J* = 11.6 Hz, 1H), 3.49 (s, 1H), 3.32 - 3.29 (m, 1H), 2.90 (ddd, *J* = 21.0, 17.0, 13.9 Hz, 2H), 2.57 (dd, *J* = 25.1, 12.2 Hz, 2H), 2.11 - 1.99 (m, 2H), 1.90 (d, *J* = 15.5 Hz, 1H), 1.24 (s, 2H). LCMS (ESI) calcd for $C_{31}H_{29}N_4O_5^+$ $[M+H]^+$: 537.21, found, 537.2.

**Example 227: preparation of 3-(1-oxo-4-(4-(phenyl(pyridin-2-yl)methyl)piperazine-1-carbonyl)isoindolin-2-yl) piperidine-2,6-dione (GT-05929)**

Referring to the method of Scheme 6, the target product (GT-05929) was prepared as white solid (36 mg, yield 46.24%). $^1$H NMR (400 MHz, DMSO-d6) δ 11.01 (d, *J* = 5.2 Hz, 1H), 8.75 (d, *J* = 3.9 Hz, 1H), 7.94 - 7.78 (m, 2H), 7.74 - 7.54 (m, 6H), 7.45 (dt, *J* = 13.8, 7.1 Hz, 4H), 5.19 - 5.13 (m, 1H), 4.74 - 4.34 (m, 3H), 3.71 (s, 4H), 2.98 (dd, *J* = 33.8, 28.5 Hz, 4H), 2.63 (t, *J* = 18.3 Hz, 2H), 2.42 (d, *J* = 9.0 Hz, 1H), 2.02 (d, *J* = 5.9 Hz, 1H). LCMS (ESI) calcd for $C_{30}H_{30}N_5O_4^+$ $[M+H]^+$: 524.23, found, 524.3.

**Example 228: preparation of 3-(4-(4-(bis(4-fluorophenyl)methyl)piperazine-1-carbonyl)-1-oxoisoindolin-2-yl)piperidine-2,6-dione (GT-05930)**

Referring to the method of Scheme 6, the target product (GT-05930) was prepared as white solid (45 mg, yield 54.41%). $^1$H NMR (400 MHz, DMSO-d6) δ 12.32 (s, 1H), 11.00 (s, 1H), 7.82 (d, *J* = 7.4 Hz, 3H), 7.60 (dd, *J* = 14.3, 7.0 Hz, 3H), 7.27 (s, 5H), 5.14 (dd, *J* = 13.2, 5.1 Hz, 1H), 4.37 (dd, *J*= 39.3, 21.7 Hz, 2H), 3.60 (s, 2H), 3.12 (s, 2H), 2.93 - 2.86 (m, 1H), 2.62 (t, *J* = 19.7 Hz, 2H), 2.45 - 2.37 (m, 1H), 2.07 - 1.91 (m, 1H). LCMS (ESI) calcd for $C_{31}H_{29}F_2N_4O_4^+$ $[M+H]^+$: 559.22, found, 559.2.

**Example 229: preparation of 5-(8-benzhydryl-3,8-diazabicyclo[3.2.1]octan-3-yl)-2-(2,6-dioxopiperidin-3-yl)-6-fluoroisoindoline-1,3-dione (GT-05931)**

Referring to the method of Scheme 8, the target product (GT-05931) was prepared as yellow solid (10 mg, yield 14.03%). $^1$H NMR (400 MHz, DMSO-d6) δ 11.34 (s, 1H), 11.11 (s, 1H), 8.04 (d, *J* = 6.7 Hz, 3H), 7.79 (d, *J* = 11.2 Hz, 1H), 7.63 - 7.14 (m, 8H), 5.47 (d, *J* = 10.2 Hz, 1H), 5.11 (dd, *J* = 12.5, 5.1 Hz, 1H), 4.06 (d, *J* = 12.2 Hz, 1H), 3.83 (s, 1H), 3.56 (d, *J* = 11.7 Hz, 1H), 3.15 (s, 3H), 2.98 - 2.81 (m, 1H), 2.59 (d, *J* = 19.2 Hz, 2H), 2.46 (s, 2H), 2.15 - 1.94 (m, 3H). LCMS (ESI) calcd for $C_{32}H_{30}FN_4O_4^+$ $[M+H]^+$: 553.22, found, 553.2.

**Example 230: preparation of 5-(8-benzhydryl-3,8-diazabicyclo[3.2.1]octan-3-yl)-2-(2,6-dioxopiperidin-3-yl)-6-fluoroisoindoline-1,3-dione (GT-05932)**

Referring to the method of Scheme 8, the target product (GT-05932) was prepared as yellow solid (17 mg, yield

23.85%). $^1$H NMR (400 MHz, DMSO-d6) δ 11.10 (s, 1H), 7.92 (s, 1H), 7.65 (d, *J* = 12.5 Hz, 1H), 7.45 (d, *J* = 7.1 Hz, 5H), 7.29 (t, *J* = 7.0 Hz, 3H), 7.18 (d, *J* = 6.7 Hz, 2H), 5.14 - 4.98 (m, 1H), 4.50 (s, 2H), 4.28 (d, *J* = 13.2 Hz, 1H), 3.15 (s, 1H), 2.89 (dd, *J* = 21.6, 9.0 Hz, 1H), 2.58 (d, *J* = 11.5 Hz, 3H), 2.33 (s, 1H), 2.16 (dd, *J* = 23.4, 8.2 Hz, 3H), 2.01 (dd, *J* = 20.5, 14.7 Hz, 3H). LCMS (ESI) calcd for $C_{32}H_{30}FN_4O_4^+$ [M+H]$^+$: 553.22, found, 553.2.

**Example 231: preparation of 5-(3-benzhydryl-3,6-diazabicyclo[3.1.1]heptan-6-yl)-2-(2,6-dioxopiperidin-3-yl)-6-fluoroisoindoline-1,3-dione (GT-05933)**

Referring to the method of Scheme 8, the target product (GT-05933) was prepared as yellow solid (16 mg, yield 23.00%). $^1$H NMR (400 MHz, DMSO-d6) δ 11.14 (s, 1H), 7.83 (d, *J* = 62.7 Hz, 3H), 7.29 (dd, *J* = 10.3, 4.8 Hz, 10H), 5.14 (dd, *J* = 12.9, 5.4 Hz, 1H), 4.55 (d, *J* = 28.7 Hz, 2H), 3.59 (s, 3H), 3.02 (s, 1H), 2.97 - 2.84 (m, 2H), 2.76 - 2.53 (m, 4H), 2.11 - 2.03 (m, 1H). LCMS (ESI) calcd for $C_{31}H_{28}FN_4O_4^+$ [M+H]$^+$: 539.21, found, 539.2.

**Example 232: preparation of 5-(6-benzhydryl-3,6-diazabicyclo[3.1.1]heptan-3-yl)-2-(2,6-dioxopiperidin-3-yl)-6-fluoroisoindoline-1,3-dione (GT-05934)**

Referring to the method of Scheme 8, the target product (GT-05934) was prepared as yellow solid (14 mg, yield 20.12%). $^1$H NMR (400 MHz, DMSO-d6) δ 11.11 (d, *J* = 7.1 Hz, 1H), 10.21 (s, 1H), 7.90 - 7.62 (m, 3H), 7.54 (s, 1H), 7.44 (dd, *J*= 20.0, 7.5 Hz, 4H), 7.28 (s, 2H), 7.17 (s, 1H), 5.24 - 5.03 (m, 1H), 4.81 (s, 1H), 4.35 - 4.04 (m, 1H), 3.67 (s, 2H), 3.50 (d, *J* = 24.3 Hz, 2H), 3.33 - 3.32 (m, 2H), 2.89 (dd, *J* = 21.9, 9.4 Hz, 1H), 2.65 - 2.52 (m, 3H), 2.05 (s, 1H). LCMS (ESI) calcd for $C_{31}H_{28}FN_4O_4^+$ [M+H]$^+$: 539.21, found, 539.2.

**Example 233: preparation of 5-(4-benzhydryl-3,5-dimethylpiperazin-1-yl)-2-(2,6-dioxopiperidin-3-yl)-6-fluoroisoindoline-1,3-dione (GT-05935)**

Referring to the method of Scheme 8, the target product (GT-05935) was prepared as yellow solid (4.6 mg, yield 6.25%). $^1$H NMR (400 MHz, DMSO-d6) δ 9.45 (d, *J* = 8.7 Hz, 1H), 8.87 (d, *J* = 10.8 Hz, 1H), 7.80 (d, *J* = 11.2 Hz, 1H), 7.63 (d, *J* = 7.4 Hz, 1H), 7.35 - 7.25 (m, 7H), 7.20 (d, *J* = 7.3 Hz, 2H), 7.01 (s, 1H), 5.39 (dd, *J* = 13.1, 5.4 Hz, 1H), 3.75 (d, *J* = 12.7 Hz, 2H), 3.50 (s, 3H), 3.22 - 3.12 (m, 1H), 3.04 - 2.93 (m, 2H), 2.80 (d, *J* = 16.8 Hz, 1H), 2.71 - 2.60 (m, 1H), 2.18 - 2.05 (m, 1H), 1.28 (t, *J* = 8.6 Hz, 6H). LCMS (ESI) calcd for $C_{32}H_{32}FN_4O_4^+$ [M+H]$^+$: 555.24, found, 555.3.

**Example 234: preparation of 5-(5-benzhydryl-2,5-diazabicyclo[2.2.2]octan-2-yl)-2-(2,6-dioxopiperidin-3-yl)-6-fluoroisoindoline-1,3-dione (GT-05936)**

Referring to the method of Scheme 8, the target product (GT-05936) was prepared as yellow solid (10 mg, yield 14.03%). $^1$H NMR (400 MHz, DMSO-d6) δ 11.65 (d, *J* = 81.0 Hz, 1H), 11.10 (s, 1H), 8.08 - 7.92 (m, 3H), 7.80 - 7.65 (m, 1H), 7.59 - 7.17 (m, 7H), 5.97 (dd, *J* = 29.8, 9.8 Hz, 1H), 5.17 - 5.00 (m, 1H), 4.58 (s, 1H), 4.31 (s, 1H), 3.68 (s, 1H), 3.61 - 3.47 (m, 3H), 3.23 - 3.03 (m, 1H), 2.87 (d, *J* = 13.6 Hz, 1H), 2.67 - 2.55 (m, 2H), 2.09 (d, *J* = 38.8 Hz, 3H), 1.84 (s, 1H). LCMS (ESI) calcd for $C_{32}H_{30}FN_4O_4^+$ [M+H]$^+$: 553.22, found, 553.3.

**Example 235: preparation of 5-(8-benzhydryl-3,8-diazabicyclo[3.2.1]octan-3-yl)-2-(2,6-dioxopiperidin-3-yl)isoindoline-1,3-dione (GT-05975)**

Referring to the method of Scheme 8, the target product (GT-05975) was prepared as yellow solid (10.2 mg, yield 14.76%). $^1$H NMR (400 MHz, DMSO-d6) δ 11.48 (s, 1H), 11.08 (s, 1H), 8.04 (d, *J* = 6.1 Hz, 3H), 7.75 (d, *J* = 7.9 Hz, 2H), 7.46 (dd, *J* = 21.9, 6.7 Hz, 5H), 7.32 (s, 2H), 7.18 (s, 1H), 5.08 (dd, *J* = 12.9, 5.1 Hz, 1H), 3.88 (dd, *J* = 29.0, 16.7 Hz, 4H), 3.45 (s, 1H), 3.33 - 3.26 (m, 2H), 3.19 (s, 1H), 2.88 (t, *J* = 13.0 Hz, 1H), 2.59 (d, *J* = 16.3 Hz, 2H), 2.44 (s, 1H), 2.02 (d, *J* = 9.3 Hz, 3H). LCMS (ESI) calcd for $C_{32}H_{31}N_4O_4^+$ [M+H]$^+$: 535.23, found, 535.3.

**Example 236: preparation of 5-(3-benzhydryl-3,8-diazabicyclo[3.2.1]octan-8-yl)-2-(2,6-dioxopiperidin-3-yl)isoindoline-1,3-dione (GT-05937)**

Referring to the method of Scheme 8, the target product (GT-05937) was prepared as yellow solid (19 mg, yield 27.50%). $^1$H NMR (400 MHz, DMSO-d6) δ 11.08 (s, 1H), 7.82 (d, *J* = 67.1 Hz, 1H), 7.62 (d, *J* = 7.7 Hz, 1H), 7.51 - 7.35 (m, 5H), 7.30 (dd, *J* = 7.6, 4.4 Hz, 4H), 7.23 - 7.15 (m, 2H), 7.11 (s, 1H), 5.06 (d, *J* = 8.5 Hz, 1H), 4.52 (s, 2H), 3.39 - 3.31 (m, 2H), 3.09 (s, 1H), 2.88 (t, *J* = 13.1 Hz, 1H), 2.59 (d, *J* = 16.9 Hz, 2H), 2.49 - 2.37 (m, 1H), 2.16 (s, 3H), 2.07 - 1.88 (m, 3H). LCMS (ESI) calcd for $C_{32}H_{31}N_4O_4^+$ [M+H]$^+$: 535.23, found, 535.3.

**Example 237: preparation of 5-(3-benzhydryl-3,6-diazabicyclo[3.1.1]heptan-6-yl)-2-(2,6-dioxopiperidin-3-yl)isoindoline-1,3-dione (GT-05938)**

Referring to the method of Scheme 8, the target product (GT-05938) was prepared as yellow solid (22 mg, yield 32.64%). $^1$H NMR (400 MHz, DMSO-d6) δ 11.09 (d, *J* = 21.2 Hz, 1H), 7.83 (d, *J* = 33.6 Hz, 3H), 7.41 - 7.26 (m, 5H), 7.26 - 7.05 (m, 5H), 6.97 (d, *J*= 18.0 Hz, 1H), 5.24 - 5.03 (m, 1H), 4.51 (d, *J* = 49.7 Hz, 2H), 3.45 (s, 2H), 3.36 - 3.30 (m, 2H), 2.96 - 2.82 (m, 2H), 2.62 (dd, *J*= 44.0, 25.4 Hz, 4H), 2.11 - 1.99 (m, 1H). LCMS (ESI) calcd for $C_{31}H_{29}N_4O_4^+$ [M+H]$^+$: 521.22, found, 521.2.

**Example 238: preparation of 5-(6-benzhydryl-3,6-diazabicyclo[3.1.1]heptan-3-yl)-2-(2,6-dioxopiperidin-3-yl)isoindoline-1,3-dione (GT-05939)**

Referring to the method of Scheme 8, the target product (GT-05939) was prepared as yellow solid (15 mg, yield 22.26%). $^1$H NMR (400 MHz, DMSO-d6) δ 11.09 (s, 1H), 7.79 (d, *J* = 8.1 Hz, 2H), 7.70 *(d, J*= 7.4 Hz, 1H), 7.48 *(dd, J*= 17.2, 9.6 Hz, 4H), 7.41 (d, *J* = 7.0 Hz, 1H), 7.30 (d, *J* = 15.5 Hz, 2H), 7.24 - 7.13 (m, 2H), 7.10 (d, *J* = 8.8 Hz, 1H), 5.09 (dd, *J*= 12.6, 5.0 Hz, 1H), 4.58 (d, *J* = 35.6 Hz, 1H), 4.04 (dd, *J* = 24.3, 11.9 Hz, 1H), 3.67 - 3.42 (m, 3H), 3.31 - 3.19 (m, 2H), 2.88 (d, *J*= 14.6 Hz, 1H), 2.68 - 2.53 (m, 3H), 2.33 (s, 1H), 2.03 (d, *J* = 5.1 Hz, 1H). LCMS (ESI) calcd for $C_{31}H_{29}N_4O_4^+$ [M+H]$^+$: 521.22, found, 521.2.

**Example 239: preparation of 5-(4-benzhydryl-3,5-dimethylpiperazin-1-yl)-2-(2,6-dioxopiperidin-3-yl)isoindoline-1,3-dione (GT-06205)**

Referring to the method of Scheme 8, the target product (GT-06205) was prepared as yellow solid (3.1 mg, yield 4.34%). $^1$H NMR (400 MHz, DMSO) δ 9.25 (s, 1H), 8.75 (s, 1H), 7.68 (d, *J* = 8.4 Hz, 1H), 7.46 (d, *J* = 2.0 Hz, 1H), 7.29 (t, *J* = 6.9 Hz, 7H), 7.13 (d, *J* = 7.3 Hz, 2H), 6.94 (s, 1H), 5.81 (s, 1H), 5.62 (s, 1H), 5.29 (dd, *J* = 13.0, 5.6 Hz, 1H), 4.18 (d, *J* = 12.5 Hz, 2H), 3.18 - 3.01 (m, 2H), 2.88 - 2.70 (m, 3H), 2.58 (d, *J* = 14.6 Hz, 1H), 2.03 (s, 1H), 1.22 (t, *J* = 7.2 Hz, 6H). LCMS (ESI) calcd for $C_{32}H_{33}N_4O_4^+$ [M+H]$^+$: 559.23, found, 559.3.

**Example 240: preparation of 5-(5-benzhydryl-2,5-diazabicyclo[2.2.2]octan-2-yl)-2-(2,6-dioxopiperidin-3-yl)isoindoline-1,3-dione (GT-05976)**

Referring to the method of Scheme 8, the target product (GT-05976) was prepared as yellow solid (3.9 mg, yield 5.64%). $^1$H NMR (400 MHz, DMSO-d6) δ 11.80 (s, 1H), 11.08 (s, 1H), 8.16 - 7.84 (m, 3H), 7.70 (d, *J* = 8.5 Hz, 1H), 7.53 - 7.30 (m, 6H), 7.29 - 6.76 (m, 3H), 5.97 (s, 1H), 5.07 (d, *J* = 5.3 Hz, 1H), 4.49 (d, *J* = 47.3 Hz, 1H), 3.69 - 3.45 (m, 2H), 3.31 - 3.23 (m, 2H), 3.10 (d, *J* = 33.7 Hz, 1H), 2.94 - 2.77 (m, 2H), 2.63 - 2.54 (m, 2H), 2.15 (s, 1H), 2.01 (s, 3H), 1.79 (s, 1H). LCMS (ESI) calcd for $C_{32}H_{31}N_4O_4^+$ [M+H]$^+$: 535.23, found, 535.3.

**Example 241: preparation of 3-(5-((1S,4S)-5-benzhydryl-2,5-diazabicyclo[2.2.1]heptane-2-carbonyl)-1-oxoisoindolin-2-yl)piperidine-2,6-dione (GT-05977)**

Referring to the method of Scheme 6, the target product (GT-05977) was prepared as white solid (27 mg, yield 40.07%). $^1$H NMR (400 MHz, DMSO-d6) δ 11.00 (s, 1H), 8.07 - 7.82 (m, 4H), 7.77 (t, *J* = 9.9 Hz, 2H), 7.64 (s, 1H), 7.42 (dd, *J* = 39.2, 17.4 Hz, 6H), 5.71 (d, *J* = 9.6 Hz, 1H), 5.21 - 5.06 (m, 1H), 4.41 (ddd, *J*= 23.9, 23.5, 16.7 Hz, 3H), 4.16 (d, *J*= 33.7 Hz, 1H), 3.67 (t, *J* = 51.7 Hz, 3H), 3.12 (d, *J* = 11.0 Hz, 1H), 2.91 *(t, J= 12.6* Hz, 1H), 2.65 (dd, *J* = 35.1, 17.1 Hz, 2H), 2.39 (dd, *J*= 28.7, 15.5 Hz, 1H), 2.11 (dd, *J* = 45.4, 16.9 Hz, 2H). LCMS (ESI) calcd for $C_{32}H_{31}N_4O_4^+$ [M+H]$^+$: 535.23, found, 535.3.

**Example 242: preparation of 3-(5-((1S,4S)-5-benzhydryl-2,5-diazabicyclo[2.2.1]heptane-2-carbonyl)-7-fluoro-1-oxoisoindolin-2-yl)piperidine-2,6-dione (GT-05978)**

Referring to the method of Scheme 6, the target product (GT-05978) was prepared as white solid (29 mg, yield 41.72%). $^1$H NMR (400 MHz, DMSO-d6) δ 11.01 (s, 1H), 8.14 - 7.68 (m, 4H), 7.59 - 7.23 (m, 8H), 5.72 (s, 1H), 5.10 (d, *J* = 5.5 Hz, 1H), 4.60 - 4.33 (m, 3H), 4.17 (d, *J* = 26.7 Hz, 1H), 3.68 (t, *J* = 47.3 Hz, 3H), 3.18 - 2.99 (m, 1H), 2.89 (d, *J* = 13.8 Hz, 1H), 2.73 (d, *J* = 10.7 Hz, 1H), 2.60 (d, *J* = 16.3 Hz, 1H), 2.40 (d, *J* = 13.1 Hz, 1H), 2.23 - 2.06 (m, 1H), 2.02 (s, 1H). LCMS (ESI) calcd for $C_{32}H_{30}FN_4O_4^+$ [M+H]$^+$: 553.22, found, 553.3.

**Example 243: preparation of 5-((1S,4S)-5-benzhydryl-2,5-diazabicyclo[2.2.1]heptane-2-carbonyl)-2-(2,6-dioxopiperidin-3-yl)isoindoline-1,3-dione (GT-05979)**

Referring to the method of Scheme 6, the target product (GT-05979) was prepared as white solid (20 mg, yield 28.97%). $^1$H NMR (400 MHz, DMSO-d6) δ 11.15 (d, *J* = 4.9 Hz, 1H), 8.15 - 7.74 (m, 7H), 7.57 - 7.04 (m, 7H), 5.70 (d, *J* = 9.8 Hz, 1H), 5.22 - 5.06 (m, 1H), 4.64 - 4.31 (m, 1H), 4.17 (d, *J* = 32.4 Hz, 1H), 3.65 (d, *J* = 33.9 Hz, 3H), 3.15 - 2.99 (m, 1H), 2.90 (t, *J* = 12.9 Hz, 1H), 2.72 (d, *J* = 11.0 Hz, 1H), 2.65 - 2.54 (m, 2H), 2.22 (d, *J* = 10.4 Hz, 1H), 2.08 (s, 1H). LCMS (ESI) calcd for $C_{32}H_{29}N_4O_5^+$ [M+H]$^+$: 549.21, found, 549.2.

**Example 244: preparation of 3-(5-(8-benzhydryl-3,8-diazabicyclo[3.2.1]octane-3-carbonyl)-1-oxoisoindolin-2-yl)piperidine-2,6-dione (GT-05980)**

Referring to the method of Scheme 6, the target product (GT-05980) was prepared as white solid (26 mg, yield 38.98%). $^1$H NMR (400 MHz, DMSO-d6) δ 11.63 (s, 1H), 11.00 (s, 1H), 8.01 (s, 3H), 7.85 - 7.64 (m, 2H), 7.48 (d, *J* = 43.2 Hz, 7H), 5.38 (s, 1H), 5.13 (dd, *J* = 13.2, 5.0 Hz, 1H), 4.43 (dd, *J* = 48.5, 17.7 Hz, 4H), 3.89 (d, *J* = 29.7 Hz, 1H), 3.62 (s, 1H), 3.40 (s, 2H), 2.96 - 2.85 (m, 1H), 2.60 (d, *J* = 17.4 Hz, 1H), 2.47 - 2.22 (m, 3H), 2.04 - 1.97 (m, 1H), 1.84 (d, *J* = 59.5 Hz, 2H). LCMS (ESI) calcd for $C_{33}H_{33}N_4O_4^+$ [M+H]$^+$: 549.25, found, 549.3.

**Example 245: preparation of 3-(5-(8-benzhydryl-3,8-diazabicyclo[3.2.1]octane-3-carbonyl)-7-fluoro-1-oxoisoindolin-2-yl)piperidine-2,6-dione (GT-05981)**

Referring to the method of Scheme 6, the target product (GT-05981) was prepared as white solid (34 mg, yield 49.45%). $^1$H NMR (400 MHz, DMSO-d6) δ 11.47 (s, 1H), 11.01 (s, 1H), 7.98 (s, 3H), 7.46 (d, *J* = 28.6 Hz, 9H), 5.40 (s, 1H), 5.09 (dd, *J* = 13.2, 5.1 Hz, 1H), 4.44 (dd, *J* = 48.7, 17.8 Hz, 3H), 4.21 (s, 1H), 3.85 (s, 1H), 3.59 (s, 2H), 3.34 - 3.31 (m, 2H), 2.94 - 2.84 (m, 1H), 2.62 (t, *J* = 19.1 Hz, 2H), 2.44 - 2.31 (m, 2H), 2.02 - 1.95 (m, 1H), 1.80 (s, 1H). LCMS (ESI) calcd for $C_{33}H_{32}FN_4O_4{}^+$ [M+H]$^+$: 567.24, found, 567.3.

**Example 246: preparation of 5-(8-benzhydryl-3,8-diazabicyclo[3.2.1]octane-3-carbonyl)-2-(2,6-dioxopiperidin-3-yl)isoindoline-1,3-dione (GT-05982)**

Referring to the method of Scheme 6, the target product (GT-05982) was prepared as white solid (21 mg, yield 30.75%). $^1$H NMR (400 MHz, DMSO-d6) δ 11.39 (s, 1H), 11.14 (s, 1H), 7.95 (d, *J* = 26.5 Hz, 5H), 7.44 (s, 7H), 5.18 (dd, *J* = 12.8, 5.4 Hz, 1H), 4.33 (d, *J* = 93.7 Hz, 2H), 3.86 (s, 1H), 3.53 (d, *J* = 50.9 Hz, 2H), 3.35 - 3.33 (m, 2H), 3.11 (s, 1H), 2.91 (dd, *J* = 22.5, 8.5 Hz, 1H), 2.69 - 2.53 (m, 2H), 2.33 (s, 1H), 2.14 - 2.03 (m, 1H), 2.03 - 1.68 (m, 2H). LCMS (ESI) calcd for $C_{33}H_{31}N_4O_5^+$ [M+H]$^+$: 563.23, found, 563.2.

**Example 247: preparation of 3-(5-(3-benzhydryl-3,8-diazabicyclo[3.2.1]octane-8-carbonyl)-1-oxoisoindolin-2-yl)piperidine-2,6-dione (GT-05983)**

Referring to the method of Scheme 6, the target product (GT-05983) was prepared as white solid (22 mg, yield 32.98%). $^1$H NMR (400 MHz, DMSO-d6) δ 11.00 (s, 1H), 7.79 (dd, *J* = 59.8, 53.0 Hz, 3H), 7.38 (dd, J=109.9, 42.6 Hz, 10H), 5.12 (dd, *J* = 13.3, 5.1 Hz, 1H), 4.49 (dd, *J* = 54.2, 37.6 Hz, 3H), 3.96 (s, 1H), 3.48 (s, 2H), 2.97 - 2.84 (m, 1H), 2.70 (d, *J* = 23.4 Hz, 1H), 2.60 (d, *J* = 16.3 Hz, 2H), 2.44 - 2.32 (m, 1H), 2.07 (d, *J* = 46.9 Hz, 4H), 1.84 (s, 2H). LCMS (ESI) calcd for $C_{33}H_{33}N_4O_4^+$ [M+H]$^+$: 549.25, found, 549.3.

**Example 248: preparation of 3-(5-(3-benzhydryl-3,8-diazabicyclo[3.2.1]octane-8-carbonyl)-7-fluoro-1-oxoisoindolin-2-yl)piperidine-2,6-dione (GT-06055)**

Referring to the method of Scheme 6, the target product (GT-06055) was prepared as white solid (15 mg, yield 21.82%). $^1$H NMR (400 MHz, DMSO-d6) δ 11.01 (s, 1H), 7.97 (s, 1H), 7.48 (d, *J* = 10.5 Hz, 5H), 7.25 (d, *J* = 51.4 Hz, 6H), 5.09 (dd, *J* = 13.1, 4.7 Hz, 1H), 4.49 (dd, *J* = 51.8, 33.4 Hz, 4H), 3.96 (s, 1H), 3.38 - 3.30 (m, 2H), 2.89 (dd, *J* = 21.7, 8.8 Hz, 1H), 2.70 (s, 1H), 2.59 (d, *J* = 15.0 Hz, 2H), 2.37 (d, *J* = 11.9 Hz, 1H), 1.85 (s, 2H), 1.72 (d, *J* = 11.6 Hz, 2H), 1.50 (d, J= 12.6 Hz, 1H). LCMS (ESI) calcd for $C_{33}H_{32}FN_4O_4{}^+$ [M+H]$^+$: 567.24, found, 567.3.

**Example 249: preparation of 5-(3-benzhydryl-3,8-diazabicyclo[3.2.1]octane-8-carbonyl)-2-(2,6-dioxopiperidin-3-yl)isoindoline-1,3-dione (GT-05984)**

Referring to the method of Scheme 6, the target product (GT-05984) was prepared as white solid (11 mg, yield

17.4 %). $^1$H NMR (400 MHz, DMSO-d6) δ 11.14 (s, 1H), 7.93 (dd, *J* = 27.9, 8.8 Hz, 4H), 7.46 (s, 4H), 7.24 (d, *J* = 47.7 Hz, 6H), 5.17 (dd, *J* = 12.7, 5.3 Hz, 1H), 4.60 (s, 1H), 4.39 (s, 1H), 3.88 (s, 1H), 2.95 -2.84 (m, 1H), 2.70 (s, 1H), 2.57 (dd, *J* = 24.8, 11.8 Hz, 3H), 2.20 (s, 1H), 2.15 - 1.95 (m, 4H), 1.88 (s, 2H). LCMS (ESI) calcd for $C_{33}H_{31}N_4O_5^+$ [M+H]$^+$: 563.23, found, 563.3.

**Example 250: preparation of 3-(4-(4-(bis(4-chlorophenyl)methyl)piperazine-1-carbonyl)-1-oxoisoindolin-2-yl) piperidine-2,6-dione (GT-05985)**

Referring to the method of Scheme 6, the target product (GT-05985) was prepared as white solid (28 mg, yield 44.15%). $^1$H NMR (400 MHz, DMSO-d6) δ 10.93 (s, 1H), 7.75 (d, *J* = 7.2 Hz, 3H), 7.64 - 7.16 (m, 9H), 5.07 (dd, *J* = 13.2, 5.1 Hz, 1H), 4.34 (dd, *J*= 40.7, 17.6 Hz, 3H), 3.43 (s, 5H), 3.27 - 3.19 (m, 2H), 2.83 (dd, *J* = 21.7, 9.2 Hz, 1H), 2.53 (d, *J* = 16.9 Hz, 2H), 2.38 - 2.30 (m, 1H), 1.97 - 1.88 (m, 1H). LCMS (ESI) calcd for $C_{31}H_{29}Cl_2N_4O_4^+$ [M+H]$^+$: 591.16, found, 591.2.

**Example 251: preparation of 3-(5-(4-(9H-fluoren-9-yl)piperazine-1-carbonyl)-1-oxoisoindolin-2-yl)piperidine-2,6-dione (GT-05986)**

Referring to the method of Scheme 6, the target product (GT-05986) was prepared as white solid (42 mg, yield 61.30%). $^1$H NMR (400 MHz, DMSO-d6) δ 11.00 (s, 1H), 8.21 - 7.87 (m, 4H), 7.75 (d, *J* = 7.8 Hz, 1H), 7.64 (s, 1H), 7.54 (t, *J* = 9.0 Hz, 3H), 7.43 (t, *J* = 7.4 Hz, 2H), 5.73 (s, 1H), 5.12 (dd, *J* = 13.2, 5.0 Hz, 1H), 4.47 (d, *J* = 17.6 Hz, 1H), 4.35 (d, *J* = 17.6 Hz, 1H), 3.65 (s, 3H), 3.37 - 3.35 (m, 2H), 3.16 - 2.82 (m, 4H), 2.60 (d, *J* = 17.6 Hz, 1H), 2.39 (qd, *J* = 13.4, 4.5 Hz, 1H), 2.05 - 1.96 (m, 1H). LCMS (ESI) calcd for $C_{31}H_{29}N_4O_4^+$ [M+H]$^+$: 521.22, found, 521.3.

**Example 252: 5-(4-(9H-fluoren-9-yl)piperazine-1-carbonyl)-2-(2,6-dioxopiperidin-3-yl)isoindoline-1,3-dione (GT-05987)**

Referring to the method of Scheme 6, the target product (GT-05987) was prepared as white solid (44 mg, yield 62.65%). $^1$H NMR (400 MHz, DMSO-d6) δ 11.07 (s, 1H), 7.88 (dd, *J* = 10.9, 7.0 Hz, 5H), 7.85 - 7.77 (m, 2H), 7.47 (s, 2H), 7.36 (t, *J* = 7.2 Hz, 2H), 5.60 (s, 1H), 5.10 (dd, *J* = 12.8, 5.4 Hz, 1H), 3.49 (s, 3H), 3.26 - 3.13 (m, 2H), 2.93 (s, 2H), 2.90 - 2.77 (m, 2H), 2.50 (dd, *J* = 24.9, 12.1 Hz, 2H), 2.04 - 1.93 (m, 1H). LCMS (ESI) calcd for $C_{31}H_{27}N_4O_5^+$ [M+H]$^+$: 535.20, found, 535.2.

**Example 253: preparation of 3-(5-(7-benzhydryl-2,7-diazaspiro[3.5]nonane-2-carbonyl)-1-oxoisoindolin-2-yl)piperidine-2,6-dione (GT-05988)**

Referring to the method of Scheme 6, the target product (GT-05988) was prepared as white solid (29 mg, yield 44.41%). $^1$H NMR (400 MHz, DMSO-d6) δ 11.02 (s, 1H), 10.10 (s, 1H), 7.81 (ddd, *J* = 24.4, 14.5, 7.4 Hz, 3H), 7.66 (dd, *J* = 13.6, 7.7 Hz, 3H), 7.55 - 7.35 (m, 6H), 5.14 (d, *J* = 13.2 Hz, 1H), 4.53 - 4.33 (m, 2H), 4.11 (d, *J* = 16.5 Hz, 2H), 3.88 (d, *J*= 34.3 Hz, 2H), 3.15 - 2.89 (m, 6H), 2.61 (d, J = 16.9 Hz, 1H), 2.41 (d, *J*= 13.2 Hz, 1H), 2.14 (s, 2H), 1.99 (d, *J* = 33.1 Hz, 3H). LCMS (ESI) calcd for $C_{34}H_{35}N_4O_4^+$ [M+H]$^+$: 563.27, found, 563.3.

**Example 254: preparation of 5-(7-benzhydryl-2,7-diazaspiro[3.5]nonane-2-carbonyl)-2-(2,6-dioxopiperidin-3-yl) isoindoline-1,3-dione (GT-05989)**

Referring to the method of Scheme 6, the target product (GT-05989) was prepared as white solid (15 mg, yield 22.45%). $^1$H NMR (400 MHz, DMSO-d6) δ 11.40 (s, 1H), 11.16 (d, *J*= 7.9 Hz, 1H), 8.09 (dd, *J* = 16.6, 10.4 Hz, 2H), 8.01 (dd, *J* = 15.3, 7.6 Hz, 1H), 7.83 (dd, *J* = 14.2, 7.6 Hz, 3H), 7.50 - 7.40 (m, 4H), 7.37 (d, *J* = 6.4 Hz, 2H), 5.54 (dd, *J* = 54.7, 9.4 Hz, 1H), 5.20 (dd, *J*= 12.4, 6.0 Hz, 1H), 4.15 (d, *J*= 17.7 Hz, 2H), 3.89 (d, *J* = 34.7 Hz, 2H), 3.16 - 3.06 (m, 2H), 2.97 (dd, *J* = 28.9, 16.2 Hz, 3H), 2.66 - 2.55 (m, 2H), 2.14 (dd, *J* = 28.0, 12.9 Hz, 5H). LCMS (ESI) calcd for $C_{34}H_{33}N_4O_5^+$ [M+H]$^+$: 577.24, found, 577.3.

**Example 255: preparation of 3-(5-((1R,4R)-5-benzhydryl-2,5-diazabicyclo[2.2.1]heptane-2-carbonyl)-1-oxoisoindolin-2-yl)piperidine-2,6-dione (GT-05990)**

Referring to the method of Scheme 6, the target product (GT-05990) was prepared as white solid (40 mg, yield 59.36%). $^1$H NMR (400 MHz, DMSO-d6) δ 10.93 (s, 1H), 7.81 (dt, *J* = 14.5, 7.2 Hz, 4H), 7.74 - 7.63 (m, 2H), 7.58 (s, 1H), 7.49 - 7.13 (m, 6H), 5.64 (d, *J*= 9.0 Hz, 1H), 5.14 - 4.98 (m, 1H), 4.49 - 4.28 (m, 3H), 4.09 (d, *J* = 33.9 Hz, 1H), 3.53 (d, *J* = 34.5 Hz, 3H), 3.06 (d, *J* = 13.4 Hz, 1H), 2.85 (t, J = 13.0 Hz, 1H), 2.67 - 2.50 (m, 2H), 2.32 (d, *J*= 13.4 Hz, 1H), 2.15 - 1.99 (m, 1H), 1.96 (s, 1H). LCMS (ESI) calcd for $C_{32}H_{31}N_4O_4^+$ [M+H]$^+$: 535.23, found, 535.2.

**Example 256: preparation of 3-(5-((1R,4R)-5-benzhydryl-2,5-diazabicyclo[2.2.1]heptane-2-carbonyl)-7-fluoro-1-oxoisoindolin-2-yl)piperidine-2,6-dione (GT-05991)**

Referring to the method of Scheme 6, the target product (GT-05991) was prepared as white solid (32 mg, yield 46.04%). $^1$H NMR (400 MHz, DMSO-d6) δ 11.02 (s, 1H), 7.92 (dd, *J* = 24.6, 7.3 Hz, 4H), 7.59 - 7.31 (m, 8H), 5.71 (d, *J* = 9.6 Hz, 1H), 5.11 (d, *J* = 5.7 Hz, 1H), 4.60 - 4.36 (m, 3H), 4.17 (d, *J* = 29.1 Hz, 1H), 3.60 (d, *J* = 34.2 Hz, 3H), 3.16 - 2.84 (m, 2H), 2.73 (d, *J* = 11.2 Hz, 1H), 2.61 (d, *J* = 16.8 Hz, 1H), 2.44 - 2.31 (m, 1H), 2.22 - 2.06 (m, 1H), 2.03 (s, 1H). LCMS (ESI) calcd for $C_{32}H_{30}FN_4O_4^+$ [M+H]$^+$: 553.22, found, 553.3.

**Example 257: preparation of 5-((1R,4R)-5-benzhydryl-2,5-diazabicyclo[2.2.1]heptane-2-carbonyl)-2-(2,6-dioxopiperidin-3-yl)isoindoline-1,3-dione (GT-05992)**

Referring to the method of Scheme 6, the target product (GT-05992) was prepared as white solid (36 mg, yield 52.15%). $^1$H NMR (400 MHz, DMSO-d6) δ 11.15 (d, *J* = 5.2 Hz, 1H), 8.11 - 7.74 (m, 7H), 7.51 - 7.30 (m, 6H), 5.71 (d, *J*= 9.7 Hz, 1H), 5.28 - 5.14 (m, 1H), 4.60 - 4.30 (m, 1H), 4.18 (d, *J* = 33.0 Hz, 1H), 3.65 (d, *J* = 31.7 Hz, 3H), 3.12 (d, *J* = 11.9 Hz, 1H), 2.89 (d, *J* = 13.2 Hz, 1H), 2.75 - 2.55 (m, 3H), 2.26 - 2.03 (m, 2H). LCMS (ESI) calcd for $C_{32}H_{29}N_4O_5^+$ [M+H]$^+$: 549.21, found, 549.2.

Example 258: preparation of 3-(5-(5-benzhydryl-2,5-diazabicyclo[2.2.2]octane-2-carbonyl)-1-oxoisoindolin-2-yl)piperidine-2,6-dione (GT-06056)

Referring to the method of Scheme 6, the target product (GT-06056) was prepared as white solid (26 mg, yield 38.98%). $^1$H NMR (400 MHz, DMSO-d6) δ 11.00 (s, 1H), 7.89 (dd, *J* = 84.4, 33.5 Hz, 5H), 7.68 - 7.28 (m, 8H), 5.96 (d, *J* = 42.2 Hz, 1H), 5.12 (d, *J* = 11.2 Hz, 1H), 4.42 (ddd, *J*= 24.2, 17.8, 7.4 Hz, 3H), 3.88 (s, 1H), 3.61 (s, 2H), 3.28 (dd, *J* = 14.0, 7.2 Hz, 2H), 2.97 - 2.86 (m, 1H), 2.61 (dd, *J* = 33.1, 15.3 Hz, 2H), 2.45 - 2.31 (m, 1H), 2.12 - 1.73 (m, 4H). LCMS (ESI) calcd for $C_{33}H_{33}N_4O_4^+$ [M+H]$^+$: 549.25, found, 549.3.

**Example 259: preparation of 3-(5-(5-benzhydryl-2,5-diazabicyclo[2.2.2]octane-2-carbonyl)-7-fluoro-1-oxoisoindolin-2-yl)piperidine-2,6-dione (GT-06057)**

Referring to the method of Scheme 6, the target product (GT-06057) was prepared as white solid (26 mg, yield 37.82%). $^1$H NMR (400 MHz, DMSO-d6) δ 11.73 (d, *J* = 49.5 Hz, 1H), 11.01 (s, 1H), 8.04 (d, *J*= 60.1 Hz, 4H), 7.41 (dd, *J* = 36.6, 17.8 Hz, 8H), 5.95 (d, *J* = 26.5 Hz, 1H), 5.10 (d, *J*= 8.0 Hz, 1H), 4.46 (ddd, *J* = 42.2, 28.7, 26.5 Hz, 4H), 3.65 (s, 3H), 2.92 (dd, *J*= 21.6, 9.2 Hz, 2H), 2.60 (d, *J* = 16.5 Hz, 2H), 2.42 - 2.30 (m, 1H), 1.92 (d, *J* = 72.9 Hz, 4H). LCMS (ESI) calcd for $C_{33}H_{32}FN_4O_4^+$ [M+H]$^+$: 567.24, found, 567.3.

**Example 260: preparation of 5-(5-benzhydryl-2,5-diazabicyclo[2.2.2]octane-2-carbonyl)-2-(2,6-dioxopiperidin-3-yl)isoindoline-1,3-dione (GT-06058)**

Referring to the method of Scheme 6, the target product (GT-06058) was prepared as white solid (21 mg, yield 30.75%). $^1$H NMR (400 MHz, DMSO-d6) δ 11.65 (d, *J* = 53.5 Hz, 1H), 11.14 (s, 1H), 8.12 - 7.79 (m, 6H), 7.41 (s, 6H), 5.94 (d, *J* = 31.7 Hz, 1H), 5.17 (d, *J* = 7.5 Hz, 1H), 4.66 - 4.23 (m, 1H), 3.84 - 3.55 (m, 2H), 3.42 (s, 1H), 3.33 - 3.20 (m, 2H), 2.94 (t, *J* = 40.5 Hz, 2H), 2.60 (d, *J* = 17.7 Hz, 2H), 1.95 *(d, J*= 96.7 Hz, 4H). LCMS (ESI) calcd for $C_{33}H_{31}N_4O_5^+$ [M+H]$^+$: 563.23, found, 563.3.

**Example 261: preparation of 3-(5-(4-benzhydryl-3,5-dimethylpiperazine-1-carbonyl)-1-oxoisoindolin-2-yl)piperidine-2,6-dione (GT-06059)**

Referring to the method of Scheme 6, the target product (GT-06059) was prepared as white solid (20 mg, yield 30.15%). $^1$H NMR (400 MHz, DMSO-d6) δ 11.00 (s, 1H), 9.96 - 9.36 (m, 1H), 8.22 (s, 2H), 7.81 - 7.50 (m, 3H), 7.33 (dt, *J* = 15.1, 10.0 Hz, 7H), 5.94 (d, *J* = 8.8 Hz, 1H), 5.12 (dd, *J* = 13.3, 5.0 Hz, 1H), 4.42 (d, *J* = 30.5 Hz, 2H), 4.07 (s, 1H), 3.49 (s, 2H), 2.96 (ddd, *J* = 31.0, 30.2, 18.4 Hz, 3H), 2.60 (d, *J* = 17.3 Hz, 1H), 2.46 - 2.30 (m, 1H), 2.06 - 1.92 (m, 1H), 1.54 - 1.03 (m, 7H). LCMS (ESI) calcd for $C_{33}H_{35}N_4O_4^+$ [M+H]$^+$: 551.27, found, 551.3.

**Example 262: preparation of 3-(5-(4-benzhydryl-3,5-dimethylpiperazine-1-carbonyl)-7-fluoro-1-oxoisoindolin-2-yl)piperidine-2,6-dione (GT-06060)**

Referring to the method of Scheme 6, the target product (GT-06060) was prepared as white solid (13 mg, yield

19.01%). $^{1}H$ NMR (400 MHz, DMSO-d6) δ 11.01 (s, 1H), 8.18 (s, 2H), 7.52 (s, 3H), 7.45 - 7.22 (m, 7H), 7.20 (d, *J*= 7.2 Hz, 1H), 5.08 (dd, *J* = 13.2, 5.0 Hz, 1H), 4.46 (t, *J* = 24.6 Hz, 2H), 3.48 (s, 2H), 2.95 (dd, *J* = 56.8, 43.8 Hz, 4H), 2.59 (d, *J* = 16.1 Hz, 1H), 2.43 - 2.32 (m, 1H), 2.00 (d, *J* = 5.8 Hz, 1H), 1.46 - 0.85 (m, 8H). LCMS (ESI) calcd for $C_{33}H_{34}FN_4O_4^+$ [M+H]$^+$: 569.26, found, 569.3.

**Example 263: preparation of 5-(4-benzhydryl-3,5-dimethylpiperazine-1-carbonyl)-2-(2,6-dioxopiperidin-3-yl) isoindoline-1,3-dione (GT-06386)**

Referring to the method of Scheme 6, the target product (GT-06386) was prepared as white solid (41 mg, yield 60.36%). $^{1}H$ NMR (400 MHz, DMSO-d6) δ 11.16 (s, 1H), 8.06 (d, *J* = 49.4 Hz, 5H), 7.41 (s, 7H), 5.18 (dd, *J*= 12.8, 5.4 Hz, 1H), 4.38 (d, *J* = 63.3 Hz, 1H), 3.58 (s, 1H), 3.42 (s, 2H), 3.29 - 3.05 (m, 2H), 2.95 - 2.84 (m, 1H), 2.57 (dd, *J*= 25.8, 12.3 Hz, 2H), 2.50 - 2.46 (m, 1H), 2.16 - 1.95 (m, 1H), 1.35 (d, *J* = 78.8 Hz, 4H), 0.95 (d, *J*= 71.9 Hz, 2H). LCMS (ESI) calcd for $C_{33}H_{33}N_4O_5^+$ [M+H]$^+$: 565.24, found, 565.2.

**Example 264: preparation of 3-(5-(4-benzhydryl-3,3-dimethylpiperazine-1-carbonyl)-1-oxoisoindolin-2-yl)piperidine-2,6-dione (GT-06061)**

Referring to the method of Scheme 6, the target product (GT-06061) was prepared as white solid (36 mg, yield 54.26%). $^{1}H$ NMR (400 MHz, DMSO-d6) δ 11.86 (s, 1H), 11.00 (s, 1H), 8.00 (d, *J* = 21.4 Hz, 2H), 7.87 - 7.48 (m, 3H), 7.44 - 7.19 (m, 8H), 5.55 (d, *J*= 54.0 Hz, 1H), 5.12 (dd, *J*= 13.2, 4.9 Hz, 1H), 4.48 (d, *J*= 17.3 Hz, 1H), 4.37 (d, *J*= 17.6 Hz, 1H), 3.67 (s, 2H), 3.17 (s, 2H), 2.97 - 2.85 (m, 1H), 2.60 (d, *J* = 16.6 Hz, 2H), 2.49 - 2.30 (m, 2H), 2.05 - 1.92 (m, 1H), 1.47 (d, *J* = 69.3 Hz, 2H), 1.11 - 0.78 (m, 4H). LCMS (ESI) calcd for $C_{33}H_{35}N_4O_4^+$ [M+H]$^+$: 551.27, found, 551.3.

**Example 265: preparation of 3-(5-(4-benzhydryl-3,3-dimethylpiperazine-1-carbonyl)-7-fluoro-1-oxoisoindolin-2-yl)piperidine-2,6-dione (GT-06062)**

Referring to **the method of Scheme** 6, **the** target product (GT-06062) was prepared as white solid (28 mg, yield 40.95%). $^{1}H$ NMR (400 MHz, DMSO-d6) δ 11.81 (s, 1H), 11.01 (s, 1H), 8.00 (d, *J* = 32.3 Hz, 2H), 7.56 - 7.15 (m, 10H), 5.54 (d, *J*= 53.2 Hz, 1H), 5.09 (dd, *J*= 13.1, 4.7 Hz, 1H), 4.44 (dd, *J* = 48.0, 18.0 Hz, 2H), 4.04 - 3.42 (m, 3H), 3.15 (s, 2H), 2.95 - 2.83 (m, 1H), 2.59 (d, *J* = 17.2 Hz, 1H), 2.38 (dd, *J* = 26.3, 12.8 Hz, 2H), 2.04 - 1.94 (m, 1H), 1.47 (d, *J* = 73.9 Hz, 2H), 1.12 - 0.82 (m, 4H). LCMS (ESI) calcd for $C_{33}H_{34}FN_4O_4^+$ [M+H]$^+$: 569.26, found, 569.2.

**Example 266: preparation of 5-(4-benzhydryl-3,3-dimethylpiperazine-1-carbonyl)-2-(2,6-dioxopiperidin-3-yl) isoindoline-1,3-dione (GT-06278)**

Referring to the method of Scheme 6, the target product (GT-06278) was prepared as white solid (21 mg, yield 30.92%). $^{1}H$ NMR (400 MHz, DMSO-d6) δ 11.14 (s, 1H), 7.90 (d, *J* = 57.4 Hz, 5H), 7.55 - 7.16 (m, 9H), 5.73 - 5.36 (m, 1H), 5.17 (dd, *J* = 12.8, 5.1 Hz, 1H), 3.67 (s, 2H), 3.35 - 3.31 (m, 2H), 3.14 (s, 1H), 2.94 - 2.84 (m, 1H), 2.60 (dd, *J* = 34.8, 15.6 Hz, 3H), 2.14 - 1.97 (m, 1H), 1.48 (d, *J* = 82.6 Hz, 1H), 0.98 *(d, J* = 87.7 Hz, 5H). LCMS (ESI) calcd for $C_{33}H_{33}N_4O_5^+$ [M+H]$^+$: 565.24, found, 565.2.

Example 267: preparation of 3-(5-(8-benzhydryl-2,8-diazaspiro[4.5]decane-2-carbonyl)-1-**oxoisoindolin-2-yl)piperidine-2,6-dione (GT-06063)**

Referring to the method of Scheme 6, the target product (GT-06063) was prepared as white solid (21 mg, yield 43.35%). $^{1}H$ NMR (400 MHz, DMSO-d6) δ 11.33 (s, 1H), 11.02 (d, *J* = 16.1 Hz, 1H), 7.95 - 7.71 (m, 6H), 7.50 - 7.32 (m, 6H), 5.71 - 5.49 (m, 1H), 5.14 (dd, *J* = 18.5, 10.0 Hz, 1H), 4.50 (dd, *J* = 21.4, 12.4 Hz, 1H), 4.38 (t, *J*= 16.1 Hz, 1H), 3.57 (t, *J* = 9.7 Hz, 2H), 3.10 (d, *J* = 22.5 Hz, 3H), 2.99 - 2.80 (m, 2H), 2.60 (d, *J* = 14.5 Hz, 2H), 2.45 - 2.31 (m, 1H), 2.04 (d, *J* = 6.8 Hz, 3H), 1.87 - 1.68 (m, 3H). LCMS (ESI) calcd for $C_{35}H_{37}N_4O_4^+$ [M+H]$^+$: 577.28, found, 577.3.

**Example 268: preparation of 5-(8-benzhydryl-2,8-diazaspiro[4.5]decane-2-carbonyl)-2-(2,6-dioxopiperidin-3-yl) isoindoline-1,3-dione (GT-06064)**

Referring to the method of Scheme 6, the target product (GT-06064) was prepared as white solid (16 mg, yield 32.29%). $^{1}H$ NMR (400 MHz, DMSO-d6) δ 11.39 - 11.02 (m, 2H), 8.04 - 7.90 (m, 3H), 7.88 - 7.71 (m, 4H), 7.54 - 7.19 (m, 7H), 5.73 - 5.40 (m, 1H), 5.27 - 5.11 (m, 1H), 3.64 - 3.42 (m, 3H), 3.21 - 2.98 (m, 4H), 2.88 (dd, *J* = 20.7, 9.2 Hz, 2H), 2.68 - 2.54 (m, 2H), 2.14 - 1.85 (m, 5H), 1.76 (dd, *J*= 18.0, 10.6 Hz, 2H). LCMS (ESI) calcd for $C_{35}H_{35}N_4O_5^+$ [M+H]$^+$: 591.26, found, 591.2.

**Example 269: preparation of 3-(5-(9-benzhydryl-3,9-diazaspiro[5.5]undecane-3-carbonyl)-1-oxoisoindolin-2-yl) piperidine-2,6-dione (GT-06065)**

Referring to the method of Scheme 6, the target product (GT-06065) was prepared as white solid (23 mg, yield 36.31%). $^1$H NMR (400 MHz, DMSO-d6) δ 11.01 (s, 1H), 10.17 (s, 1H), 7.78 (d, *J* = 7.5 Hz, 1H), 7.70 (d, *J* = 6.5 Hz, 4H), 7.62 (s, 1H), 7.48 (t, *J* = 7.7 Hz, 5H), 7.40 (t, *J* = 7.1 Hz, 2H), 5.65 (s, 1H), 5.13 (dd, *J* = 13.1, 4.9 Hz, 1H), 4.49 (d, *J* = 17.5 Hz, 1H), 4.37 (d, *J* = 17.7 Hz, 1H), 3.62 (s, 3H), 3.05 (s, 3H), 2.93 (dd, *J* = 22.3, 8.8 Hz, 2H), 2.61 (d, *J* = 17.9 Hz, 2H), 2.41 (d, *J* = 13.3 Hz, 1H), 2.02 (d, *J*= 5.9 Hz, 1H), 1.91 (d, *J* = 14.1 Hz, 2H), 1.71 (s, 4H), 1.41 (d, *J* = 47.1 Hz, 2H). LCMS (ESI) calcd for $C_{36}H_{39}N_4O_4^+$ [M+H]$^+$: 591.30, found, 591.3.

**Example 270: preparation of 5-(9-benzhydryl-3,9-diazaspiro[5.5]undecane-3-carbonyl)-2-(2,6-dioxopiperidin-3-yl)isoindoline-1,3-dione (GT-06066)**

Referring to the method of Scheme 6, the target product (GT-06066) was prepared as white solid (21 mg, yield 32.43%). $^1$H NMR (400 MHz, DMSO-d6) δ 11.15 (s, 1H), 9.95 (s, 1H), 7.98 (s, 1H), 7.92 - 7.81 (m, 2H), 7.66 (s, 4H), 7.48 (t, *J* = 7.2 Hz, 4H), 7.44 - 7.38 (m, 2H), 5.65 (s, 1H), 5.18 (dd, *J* = 12.5, 5.3 Hz, 1H), 3.63 (s, 2H), 3.21 - 3.14 (m, 2H), 3.06 (s, 3H), 2.93 - 2.85 (m, 1H), 2.58 (dd, *J* = 24.5, 11.7 Hz, 3H), 2.12 - 2.04 (m, 1H), 1.92 (d, *J* = 14.5 Hz, 2H), 1.67 (d, *J* = 48.6 Hz, 4H), 1.44 (d, *J* = 49.5 Hz, 2H). LCMS (ESI) calcd for $C_{36}H_{37}N_4O_5^+$ [M+H]$^+$: 605.28, found, 605.3.

**Example 271: preparation of 3-(5-(5-benzhydryloctahydropyrrolo[3,4-c]pyrrole-2-carbonyl)-1-oxoisoindolin-2-yl)piperidine-2,6-dione (GT-06067)**

Referring to the method of Scheme 6, the target product (GT-06067) was prepared as white solid (18 mg, yield 26.99%). $^1$H NMR (400 MHz, DMSO-d6) δ 11.01 (s, 2H), 7.80 (t, *J*= 7.2 Hz, 1H), 7.73 (d, *J*= 7.2 Hz, 1H), 7.65 (d, *J* = 7.6 Hz, 5H), 7.44 (d, *J* = 25.0 Hz, 6H), 5.73 (dd, *J* = 22.2, 9.4 Hz, 1H), 5.14 (dd, *J*= 13.2, 5.0 Hz, 1H), 4.51 (d, *J* = 16.9 Hz, 1H), 4.38 (d, *J* = 17.9 Hz, 1H), 3.79 (s, 1H), 3.56 (s, 2H), 3.08 (s, 2H), 2.99 - 2.88 (m, 2H), 2.61 (d, *J* = 18.0 Hz, 1H), 2.42 (dd, *J* = 13.1, 4.2 Hz, 1H), 2.07 - 1.98 (m, 1H). LCMS (ESI) calcd for $C_{33}H_{33}N_4O_4^+$ [M+H]$^+$: 549.25, found, 549.3.

**Example 272: preparation of 5-(5-benzhydryloctahydropyrrolo[3,4-c]pyrrole-2-carbonyl)-2-(2,6-dioxopiperidin-3-yl)isoindoline-1,3-dione (GT-06068)**

Referring to the method of Scheme 6, the target product (GT-06068) was prepared as white solid (18 mg, yield 26.36%). $^1$H NMR (400 MHz, DMSO-d6) δ 11.15 (s, 1H), 10.98 (s, 1H), 8.05 - 7.93 (m, 3H), 7.65 (s, 4H), 7.43 (d, *J* = 30.6 Hz, 6H), 5.73 (d, *J* = 10.1 Hz, 1H), 5.19 (dd, *J* = 12.7, 5.3 Hz, 1H), 3.82 (s, 1H), 3.55 (s, 2H), 3.09 (s, 2H), 2.96 - 2.85 (m, 2H), 2.59 (dd, *J*= 24.2, 12.1 Hz, 2H), 2.12 - 2.03 (m, 1H). LCMS (ESI) calcd for $C_{33}H_{31}N_4O_5{}^+$ [M+H]$^+$: 563.23, found, 563.3.

**Example 273: preparation of 3-(5-(4-(2,7-dichloro-9H-fluoren-9-yl)piperazine-1-carbonyl)-1-oxoisoindolin-2-yl) piperidine-2,6-dione (GT-06069)**

Referring to the method of Scheme 6, the target product (GT-06069) was prepared as white solid (34 mg, yield 53.78%). $^1$H NMR (400 MHz, DMSO-d6) δ 11.00 (s, 1H), 7.94 (d, *J* = 8.2 Hz, 4H), 7.76 (d, *J* = 7.8 Hz, 1H), 7.64 (s, 1H), 7.55 (dd, *J* = 13.3, 8.0 Hz, 3H), 5.43 (s, 1H), 5.12 (dd, *J* = 13.3, 5.1 Hz, 1H), 4.48 (d, *J* = 17.5 Hz, 1H), 4.35 (d, *J* = 17.6 Hz, 1H), 3.65 (s, 2H), 3.34 - 3.23 (m, 2H), 3.02 - 2.66 (m, 5H), 2.60 (d, *J* = 16.5 Hz, 1H), 2.39 (qd, *J* = 13.2, 4.3 Hz, 1H), 2.04 - 1.97 (m, 1H). LCMS (ESI) calcd for $C_{31}H_{27}Cl_2N_4O_4{}^+$ [M+H]$^+$: 589.14, found, 589.2.

**Example 274: preparation of 5-(4-(2,7-dichloro-9H-fluoren-9-yl)piperazine-1-carbonyl)-2-(2,6-dioxopiperidin-3-yl)isoindoline-1,3-dione (GT-06070)**

Referring to the method of Scheme 6, the target product (GT-06070) was prepared as white solid (38 mg, yield 58.79%). $^1$H NMR (400 MHz, DMSO-d6) δ 11.14 (s, 1H), 7.96 (t, *J* = 6.5 Hz, 5H), 7.89 (d, *J*= 8.4 Hz, 2H), 7.58 (d, *J* = 7.7 Hz, 2H), 5.47 (s, 1H), 5.17 (dd, *J* = 12.7, 5.4 Hz, 1H), 3.70 (s, 2H), 3.37 - 3.25 (m, 2H), 2.99 - 2.78 (m, 4H), 2.57 (dd, *J* = 24.0, 11.6 Hz, 2H), 2.10 - 2.01 (m, 1H). LCMS (ESI) calcd for $C_{31}H_{25}Cl_2N_4O_5^+$ [M+H]$^+$: 603.12, found, 603.1.

Example 275: preparation of 3-(5-(4-benzhydryl-2-(trifluoromethyl)piperazine-1-carbonyl)-1-oxoisoindolin-2-yl)piperidine-2,6-dione (GT-06071)

Referring to the method of Scheme 6, the target product (GT-06071) was prepared as white solid (3.1 mg, yield 6.50%). $^1$H NMR (400 MHz, DMSO-d6) δ 11.00 (s, 1H), 7.78 (t, *J* = 10.1 Hz, 1H), 7.70 (d, *J* = 26.9 Hz, 1H), 7.60 - 7.52 (m,

1H), 7.43 (dd, *J* = 17.4, 7.6 Hz, 4H), 7.35 - 7.24 (m, 4H), 7.24 - 7.15 (m, 2H), 5.37 (s, 1H), 5.12 (d, *J* = 9.8 Hz, 1H), 4.49 (d, *J* = 18.4 Hz, 1H), 4.37 (d, *J* = 23.0 Hz, 2H), 3.52 (s, 1H), 3.20 (d, *J* = 12.4 Hz, 1H), 2.93 (dd, *J* = 32.8, 19.6 Hz, 2H), 2.64 (dd, *J* = 35.3, 14.2 Hz, 2H), 2.40 (d, *J* = 9.4 Hz, 1H), 2.25 (d, *J* = 10.5 Hz, 1H), 1.97 (d, *J* = 34.0 Hz, 2H). LCMS (ESI) calcd for $C_{32}H_{30}F_3N_4O_4^+$ [M+H]$^+$: 591.22, found, 591.2.

**Example 276: preparation of 3-(5-(4-benzhydryl-2-(trifluoromethyl)piperazine-1-carbonyl)-7-fluoro-1-oxoisoindolin-2-yl)piperidine-2,6-dione (GT-06090)**

Referring to the method of Scheme 6, the target product (GT-06090) was prepared as white solid (3.1 mg, yield 6.32 %). $^1$H NMR (400 MHz, DMSO-d6) δ 11.01 (s, 1H), 7.49 - 7.39 (m, 5H), 7.36 - 7.27 (m, 5H), 7.20 (dt, *J* = 14.5, 7.1 Hz, 2H), 5.32 (s, 1H), 5.15 - 4.90 (m, 1H), 4.50 (d, *J* = 18.0 Hz, 1H), 4.42 - 4.31 (m, 2H), 3.52 (s, 1H), 3.19 (d, *J* = 12.8 Hz, 1H), 2.89 (dd, *J* = 21.4, 8.8 Hz, 2H), 2.64 (dd, *J* = 33.0, 13.5 Hz, 2H), 2.31 (dd, *J* = 53.2, 11.4 Hz, 2H), 2.00 (d, *J* = 11.8 Hz, 2H). LCMS (ESI) calcd for $C_{32}H_{29}F_4N_4O_4^+$ [M+H]$^+$: 609.21, found, 609.3.

**Example 277: preparation of 5-(4-benzhydryl-2-(trifluoromethyl)piperazine-1-carbonyl)-2-(2,6-dioxopiperidin-3-yl)isoindoline-1,3-dione (GT-06091)**

Referring to the method of Scheme 6, the target product (GT-06091) was prepared as white solid (6.4 mg, yield 13.14%). $^1$H NMR (400 MHz, DMSO-d6) δ 11.14 (s, 1H), 7.99 (d, *J* = 7.4 Hz, 1H), 7.88 (d, *J* = 8.8 Hz, 2H), 7.43 (dd, *J* = 18.2, 7.4 Hz, 4H), 7.31 (dt, *J* = 14.8, 7.6 Hz, 5H), 7.24 - 7.16 (m, 2H), 5.33 (s, 1H), 5.18 (dd, *J* = 12.7, 5.3 Hz, 1H), 4.33 (d, *J* = 18.7 Hz, 2H), 3.62 - 3.45 (m, 2H), 3.19 (d, *J* = 12.9 Hz, 1H), 2.88 (dd, *J* = 20.9, 8.9 Hz, 1H), 2.70 - 2.56 (m, 2H), 2.29 (d, *J* = 10.7 Hz, 1H), 2.04 (dd, *J* = 21.4, 16.9 Hz, 2H). LCMS (ESI) calcd for $C_{32}H_{28}F_3N_4O_5^+$ [M+H]$^+$: 605.20, found, 605.2.

**Example 278: preparation of 3-(5-((R)-4-benzhydryl-2-methylpiperazine-1-carbonyl)-1-oxoisoindolin-2-yl)piperidine-2,6-dione (GT-06092)**

Referring to the method of Scheme 6, the target product (GT-06092) was prepared as white solid (34 mg, yield 50.28%). $^1$H NMR (400 MHz, DMSO-d6) δ 11.00 (s, 1H), 7.82 (d, *J* = 41.2 Hz, 3H), 7.64 - 7.15 (m, 10H), 5.12 (dd, *J* = 13.2, 5.0 Hz, 1H), 4.48 (d, *J* = 17.6 Hz, 1H), 4.36 (d, *J* = 18.0 Hz, 2H), 3.61 (s, 1H), 3.32 (s, 2H), 3.08 (d, *J* = 23.2 Hz, 1H), 2.89 (dd, *J* = 11.8, 6.4 Hz, 1H), 2.62 (t, *J* = 18.8 Hz, 2H), 2.46 - 2.33 (m, 1H), 2.16 - 1.83 (m, 2H), 1.40 (s, 3H). LCMS (ESI) calcd for $C_{32}H_{33}N_4O_4^+$ [M+H]$^+$: 537.25, found, 537.3.

**Example 279: preparation of 3-(5-((R)-4-benzhydryl-2-methylpiperazine-1-carbonyl)-7-fluoro-1-oxoisoindolin-2-yl)piperidine-2,6-dione (GT-06093)**

Referring to the method of Scheme 6, the target product (GT-06093) was prepared as white solid (9.4 mg, yield 13.48%). $^1$H NMR (400 MHz, DMSO-d6) δ 11.01 (s, 1H), 7.87 (s, 2H), 7.37 (d, *J* = 44.7 Hz, 11H), 5.09 (dd, *J* = 13.2, 4.9 Hz, 1H), 4.58 - 4.25 (m, 3H), 3.32 (s, 2H), 2.95 (ddd, *J* = 38.4, 18.2, 12.6 Hz, 3H), 2.67 - 2.55 (m, 2H), 2.38 (dt, *J* = 23.3, 11.6 Hz, 1H), 2.14 - 1.86 (m, 2H), 1.38 (dd, *J* = 83.3, 49.0 Hz, 4H). LCMS (ESI) calcd for $C_{32}H_{32}FN_4O_4^+$ [M+H]$^+$: 555.24, found, 555.2.

**Example 280: preparation of 5-((R)-4-benzhydryl-2-methylpiperazine-1-carbonyl)-2-(2,6-dioxopiperidin-3-yl) isoindoline-1,3-dione (GT-06094)**

Referring to the method of Scheme 6, the target product (GT-06094) was prepared as grey solid (27 mg, yield 38.97%). $^1$H NMR (400 MHz, DMSO-d6) δ 11.14 (s, 1H), 7.92 (d, *J*= 44.0 Hz, 5H), 7.39 (d, *J* = 48.1 Hz, 9H), 5.17 (dd, *J*= 12.7, 5.3 Hz, 1H), 4.29 (s, 2H), 3.51 (s, 2H), 3.19 (s, 2H), 2.88 (dd, *J*= 17.0, 5.1 Hz, 1H), 2.57 (dd, *J*= 24.2, 11.5 Hz, 3H), 2.02 (dd, *J*= 34.9, 29.9 Hz, 2H), 1.40 (s, 3H). LCMS (ESI) calcd for $C_{32}H_{31}N_4O_5^+$ [M+H]$^+$: 551.23, found, 551.3.

**Example 281: preparation of 3-(5-((4-benzhydryl-2-(trifluoromethyl)piperazin-1-yl)methyl)-1-oxoisoindolin-2-yl) piperidine-2,6-dione (GT-06095)**

Referring to the method of Scheme 4, the target product (GT-06095) was prepared as white solid (16 mg, yield 32.41%). $^1$H NMR (400 MHz, DMSO-d6) δ 10.98 (s, 1H), 7.68 (d, *J* = 7.7 Hz, 1H), 7.54 (s, 1H), 7.46 (d, *J* = 7.9 Hz, 5H), 7.32 (s, 4H), 7.21 (s, 2H), 5.10 (dd, *J* = 13.2, 5.0 Hz, 1H), 4.43 (d,*J* = 17.0 Hz, 1H), 4.30 (d, *J* = 17.3 Hz, 1H), 3.99 (d, *J* = 18.2 Hz, 2H), 3.51 (s, 1H), 3.41 - 3.33 (m, 2H), 3.00 - 2.84 (m, 3H), 2.59 (d, *J* = 17.7 Hz, 2H), 2.38 (dt, *J* = 13.4, 9.0 Hz, 2H), 2.02 (dd, *J* = 35.1, 29.4 Hz, 2H). LCMS (ESI) calcd for $C_{32}H_{32}F_3N_4O_3^+$ [M+H]$^+$: 577.24, found, 577.2.

**Example 282: preparation of 3-(5-((4-benzhydryl-2-(trifluoromethyl)piperazin-1-yl)methyl)-4-fluoro-1-oxoisoindolin-2-yl)piperidine-2,6-dione (GT-06096)**

Referring to the method of Scheme 4, the target product (GT-06096) was prepared as white solid (12 mg, yield 23.65%). $^1$H NMR (400 MHz, DMSO-d6) δ 10.99 (s, 1H), 7.63 - 7.53 (m, 2H), 7.42 (s, 4H), 7.30 (t, *J* = 6.7 Hz, 4H), 7.22 - 7.15 (m, 2H), 5.10 (dd, *J* = 13.3, 5.0 Hz, 1H), 4.54 (dd, *J* = 17.4, 2.6 Hz, 1H), 4.36 (dd, *J*= 25.5, 8.3 Hz, 2H), 4.02 (q, *J*= 15.1 Hz, 2H), 3.60 (s, 1H), 3.35 - 3.31 (m, 2H), 3.03 - 2.83 (m, 3H), 2.59 (d, *J* = 18.3 Hz, 1H), 2.47 - 2.30 (m, 2H), 2.02 (dd, *J* = 27.2, 20.1 Hz, 2H). LCMS (ESI) calcd for $C_{32}H_{31}F_4N_4O_3^+$ [M+H]$^+$: 595.23, found, 595.2.

**Example 283: preparation of 3-(5-((4-benzhydryl-2-(trifluoromethyl)piperazin-1-yl)methyl)-6-fluoro-1-oxoisoindolin-2-yl)piperidine-2,6-dione (GT-06097)**

Referring to the method of Scheme 4, the target product (GT-06097) was prepared as white solid (15 mg, yield 23.91%). $^1$H NMR (400 MHz, DMSO-d6) δ 10.92 (s, 1H), 7.59 (d, *J* = 6.1 Hz, 1H), 7.42 (d, *J* = 8.9 Hz, 2H), 7.37 (s, 2H), 7.24 (s, 4H), 7.14 (s, 2H), 5.03 (dd, *J*= 13.2, 5.0 Hz, 1H), 4.36 (d, *J* = 17.5 Hz, 1H), 4.23 (d, *J* = 17.2 Hz, 2H), 3.93 (s, 2H), 3.44 (s, 2H), 2.98 - 2.77 (m, 3H), 2.53 (d, *J* = 17.6 Hz, 2H), 2.38 - 2.21 (m, 2H), 2.05 (s, 1H), 1.98 - 1.88 (m, 1H). LCMS (ESI) calcd for $C_{32}H_{31}F_4N_4O_3^+$ [M+H]$^+$: 595.23, found, 595.3.

**Example 284: preparation of 3-(5-((4-benzhydryl-2-(trifluoromethyl)piperazin-1-yl)methyl)-7-fluoro-1-oxoisoindolin-2-yl)piperidine-2,6-dione (GT-06098)**

Referring to the method of Scheme 4, the target product (GT-06098) was prepared as white solid (7.3 mg, yield 11.63%). $^1$H NMR (400 MHz, DMSO-d6) δ 10.92 (s, 1H), 7.37 (s, 3H), 7.31 (s, 2H), 7.24 (s, 4H), 7.15 (d, *J* = 10.5 Hz, 3H), 4.99 (dd, *J* = 13.3, 5.1 Hz, 1H), 4.38 (dd, *J* = 17.8, 2.7 Hz, 1H), 4.24 (d, *J* = 16.0 Hz, 2H), 3.95 (s, 1H), 3.87 (d, *J* = 13.1 Hz, 1H), 3.51 (s, 2H), 2.96 - 2.78 (m, 3H), 2.52 (d, *J* = 16.6 Hz, 2H), 2.37 - 2.21 (m, 2H), 2.04 (s, 1H), 1.96 - 1.85 (m, 1H). LCMS (ESI) calcd for $C_{32}H_{31}F_4N_4O_3$ $^+$ [M+H]$^+$: 595.23, found, 595.3.

**Example 285: preparation of 5-((4-benzhydryl-2-(trifluoromethyl)piperazin-1-yl)methyl)-2-(2,6-dioxopiperidin-3-yl)isoindoline-1,3-dione (GT-06099)**

Referring to the method of Scheme 4, the target product (GT-06099) was prepared as white solid (12.5 mg, yield 20.04%). $^1$H NMR (400 MHz, DMSO-d6) δ 11.05 (s, 1H), 7.84 - 7.78 (m, 2H), 7.77 - 7.71 (m, 1H), 7.36 (s, 4H), 7.24 (s, 4H), 7.13 (s, 2H), 5.07 (dd, *J* = 12.7, 5.2 Hz, 1H), 4.25 (s, 1H), 3.99 (dd, *J* = 44.2, 14.8 Hz, 2H), 3.55 (s, 2H), 2.84 (dd, *J* = 32.7, 16.0 Hz, 3H), 2.53 (d, *J* = 17.9 Hz, 3H), 2.31 (s, 1H), 2.11 - 1.92 (m, 2H). LCMS (ESI) calcd for $C_{32}H_{30}F_3N_4I_4$ $^+$ [M+H]$^+$: 591.22, found, 591.3.

**Example 286: preparation of 3-(5-(4-(bis(2-fluorophenyl)methyl)piperazine-1-carbonyl)-1-oxoisoindolin-2-yl)piperidine-2,6-dione (GT-06104)**

Referring to the method of Scheme 6, the target product (GT-06104) was prepared as white solid (30 mg, yield 45.42%). $^1$H NMR (400 MHz, DMSO-d6) δ 10.93 (s, 1H), 7.91 (s, 1H), 7.71 (d, *J* = 7.8 Hz, 1H), 7.59 (s, 1H), 7.48 (d, *J* = 7.5 Hz, 1H), 7.28 (d, *J* = 40.7 Hz, 7H), 5.06 (dd, *J* = 13.3, 5.1 Hz, 1H), 4.42 (d, *J* = 17.6 Hz, 1H), 4.30 (d, *J* = 17.7 Hz, 1H), 3.45 (s, 5H), 3.35 - 3.30 (m, 2H), 2.92 - 2.73 (m, 2H), 2.53 (d, *J* = 17.0 Hz, 2H), 2.34 (dt, *J* = 13.4, 8.8 Hz, 1H), 1.94 (dd, *J* = 9.0, 3.5 Hz, 1H). LCMS (ESI) calcd for $C_{31}H_{29}F_2N_4O_4^+$ [M+H]$^+$: 559.22, found, 559.2.

**Example 287: preparation of 5-(4-(bis(2-fluorophenyl)methyl)piperazine-1-carbonyl)-2-(2,6-dioxopiperidin-3-yl) isoindoline-1,3-dione (GT-06141)**

Referring to the method of Scheme 6, the target product (GT-06141) was prepared as white solid (19 mg, yield 28.11%). $^1$H NMR (400 MHz, DMSO-d6) δ 11.14 (s, 1H), 8.20 - 7.94 (m, 3H), 7.89 (d, *J* = 7.6 Hz, 2H), 7.39 (s, 2H), 7.35 - 7.19 (m, 4H), 5.18 (dd, *J*= 12.8, 5.4 Hz, 1H), 3.51 (s, 4H), 3.32 - 3.20 (m, 2H), 3.15 - 3.00 (m, 1H), 2.96 - 2.82 (m, 2H), 2.58 (dd, *J* = 24.1, 11.7 Hz, 3H), 2.11 - 2.02 (m, 1H). LCMS (ESI) calcd for $C_{31}H_{27}F_2N_4O_5^+$ [M+H]$^+$: 573.19, found, 573.3.

**Example 288: preparation of 3-(1-oxo-5-(4-(phenyl(pyridin-3-yl)methyl)piperazine-1-carbonyl)isoindolin-2-yl) piperidine-2,6-dione (GT-06142)**

Referring to the method of Scheme 6, the target product (GT-06142) was prepared as white solid (29 mg, yield 58.84%). $^1$H NMR (400 MHz, DMSO-d6) δ 11.00 (s, 1H), 9.06 (s, 1H), 8.71 (d, *J* = 4.8 Hz, 1H), 8.65 (d, *J* = 7.5 Hz, 1H), 7.79

(d, *J* = 7.8 Hz, 4H), 7.66 (s, 1H), 7.55 (d, *J* = 7.8 Hz, 1H), 7.45 (t, *J* = 7.4 Hz, 2H), 7.37 (t, *J* = 7.0 Hz, 1H), 5.48 (s, 1H), 5.13 (dd, *J* = 13.3, 5.1 Hz, 1H), 4.49 (d, *J* = 17.6 Hz, 1H), 4.37 (d, *J* = 17.5 Hz, 1H), 3.74 (s, 2H), 3.38 - 3.27 (m, 2H), 2.97 (s, 2H), 2.95 - 2.85 (m, 2H), 2.63 (t, *J* = 18.2 Hz, 2H), 2.46 - 2.32 (m, 1H), 2.06 - 1.94 (m, 1H). LCMS (ESI) calcd for $C_{30}H_{30}N_5O_4^+$ $[M+H]^+$: 524.23, found, 524.3.

**Example 289: preparation of 2-(2,6-dioxopiperidin-3-yl)-5-(4-(phenyl(pyridin-3-yl)methyl)piperazine-1-carbonyl)isoindoline-1,3-dione (GT-06143)**

Referring to the method of Scheme 6, the target product (GT-06143) was prepared as white solid (5.1 mg, yield 10.10%). $^1$H NMR (400 MHz, DMSO-d6) δ 11.14 (s, 1H), 9.03 (s, 1H), 8.71 (d, *J* = 4.5 Hz, 1H), 8.60 (d, *J* = 7.6 Hz, 1H), 7.99 (d, *J* = 7.6 Hz, 1H), 7.94 - 7.87 (m, 2H), 7.76 (s, 3H), 7.44 (t, *J* = 7.3 Hz, 2H), 7.37 (d, *J* = 7.4 Hz, 1H), 5.20 (s, 1H), 5.18 (dd, *J*= 12.7, 5.4 Hz, 1H), 3.66 (s, 2H), 3.33 - 3.27 (m, 2H), 3.23 - 3.07 (m, 2H), 2.88 (dd, *J* = 21.3, 9.4 Hz, 2H), 2.72 - 2.52 (m, 3H), 2.10 - 1.99 (m, 1H). LCMS (ESI) calcd for $C_{30}H_{28}N_5O_5^+$ $[M+H]^+$: 538.21, found, 538.3.

**Example 290: preparation of 3-(1-oxo-5-(4-(phenyl(pyridin-4-yl)methyl)piperazine-1-carbonyl)isoindolin-2-yl) piperidine-2,6-dione (GT-06144)**

Referring to the method of Scheme 6, the target product (GT-06144) was prepared as white solid (19 mg, yield 27.58%). $^1$H NMR (400 MHz, DMSO-d6) δ 11.00 (s, 1H), 8.84 (d, *J* = 6.4 Hz, 2H), 8.22 (d, *J* = 4.6 Hz, 2H), 7.78 (d, *J* = 7.8 Hz, 1H), 7.74 - 7.57 (m, 3H), 7.53 (d, *J* = 7.8 Hz, 1H), 7.42 (t, *J* = 7.5 Hz, 2H), 7.35 (t, *J* = 7.3 Hz, 1H), 5.13 (dd, *J* = 13.3, 5.1 Hz, 2H), 4.49 (d, *J* = 17.1 Hz, 1H), 4.41 - 4.33 (m, 1H), 3.87 (s, 1H), 3.23 - 3.09 (m, 2H), 2.98 - 2.86 (m, 2H), 2.75 (s, 2H), 2.60 (d, *J* = 16.4 Hz, 2H), 2.48 - 2.29 (m, 2H), 2.04 - 1.95 (m, 1H). LCMS (ESI) calcd for $C_{30}H_{30}N_5O_4^+$ $[M+H]^+$: 524.23, found, 524.3.

**Example 291: preparation of 2-(2,6-dioxopiperidin-3-yl)-5-(4-(phenyl(pyridin-4-yl)methyl)piperazine-1-carbonyl)isoindoline-1,3-dione (GT-06145)**

Referring to the method of Scheme 6, the target product (GT-06145) was prepared as white solid (23 mg, yield 32.57%). $^1$H NMR (400 MHz, DMSO-d6) δ 11.14 (s, 1H), 8.81 (d, *J* = 6.4 Hz, 2H), 8.16 (d, *J* = 5.7 Hz, 2H), 7.98 (d, *J* = 7.6 Hz, 1H), 7.92 - 7.85 (m, 2H), 7.62 (d, *J* = 7.9 Hz, 2H), 7.41 (t, *J* = 7.4 Hz, 2H), 7.34 (d, *J* = 7.1 Hz, 1H), 5.17 (dd, *J* = 12.8, 5.4 Hz, 1H), 3.75 (s, 3H), 3.35 - 3.33 (m, 2H), 3.15 - 3.02 (m, 2H), 2.94 - 2.82 (m, 2H), 2.65 - 2.54 (m, 3H), 2.11 - 2.02 (m, 1H). LCMS (ESI) calcd for $C_{30}H_{28}N_5O_5^+$ $[M+H]^+$: 538.21, found, 538.2.

**Example 292: preparation of 3-(5-(4-(bis(3-fluorophenyl)methyl)piperazine-1-carbonyl)-1-oxoisoindolin-2-yl)piperidine-2,6-dione (GT-06146)**

Referring to the method of Scheme 6, the target product (GT-06146) was prepared as white solid (40 mg, yield 64.64%). $^1$H NMR (400 MHz, DMSO-d6) δ 11.00 (s, 1H), 7.78 (d, *J* = 7.7 Hz, 1H), 7.64 (s, 2H), 7.53 (d, *J* = 7.7 Hz, 2H), 7.46 (s, 4H), 7.16 (s, 2H), 5.15 - 5.06 (m, 1H), 4.49 (d, *J* = 17.6 Hz, 1H), 4.37 (d, *J* = 17.7 Hz, 1H), 3.52 (s, 4H), 3.35 - 3.31 (m, 2H), 3.09 - 2.83 (m, 3H), 2.60 (d, *J* = 16.8 Hz, 2H), 2.43 - 2.35 (m, 1H), 2.03 - 1.95 (m, 1H). LCMS (ESI) calcd for $C_{31}H_{29}F_2N_4O_4^+$ $[M+H]^+$: 559.22, found, 559.2.

**Example 293: preparation of 3-(5-(4-(bis(3-fluorophenyl)methyl)piperazine-1-carbonyl)-7-fluoro-1-oxoisoindolin-2-yl)piperidine-2,6-dione (GT-06147)**

Referring to the method of Scheme 6, the target product (GT-06147) was prepared as white solid (40 mg, yield 62.74%). $^1$H NMR (400 MHz, DMSO-d6) δ 11.01 (s, 1H), 7.54 (s, 3H), 7.47 (s, 4H), 7.35 (d, *J* = 9.4 Hz, 1H), 7.17 (s, 2H), 5.09 (dd, *J* = 13.3, 5.0 Hz, 1H), 4.51 (d, *J* = 18.1 Hz, 1H), 4.38 (d, *J* = 18.0 Hz, 1H), 3.60 (s, 3H), 3.34 - 3.27 (m, 2H), 3.01 (s, 2H), 2.97 - 2.83 (m, 2H), 2.62 (t, *J* = 19.3 Hz, 2H), 2.38 (dt, *J* = 13.9, 9.3 Hz, 1H), 2.05 - 1.94 (m, 1H). LCMS (ESI) calcd for $C_{31}H_{28}F_3N_4O_4^+$ $[M+H]^+$: 577.21, found, 577.2.

**Example 294: preparation of 3-(4-(4-(bis(3-fluorophenyl)methyl)piperazine-1-carbonyl)-1-oxoisoindolin-2-yl)piperidine-2,6-dione (GT-06148)**

Referring to the method of Scheme 6, the target product (GT-06148) was prepared as white solid (36 mg, yield 58.17%). $^1$H NMR (400 MHz, DMSO-d6) δ 11.00 (s, 1H), 7.82 (d, *J* = 7.2 Hz, 1H), 7.60 (dt, *J* = 14.9, 7.3 Hz, 3H), 7.45 (s, 5H), 7.15 (s, 2H), 5.14 (dd, *J* = 13.3, 5.1 Hz, 1H), 4.46 (d, *J* = 18.0 Hz, 1H), 4.36 (d, *J* = 17.7 Hz, 1H), 3.59 (s, 3H), 3.34 - 3.27 (m, 2H), 3.07 - 2.84 (m, 3H), 2.60 (d, *J* = 18.4 Hz, 2H), 2.48 - 2.31 (m, 2H), 2.05 - 1.93 (m, 1H). LCMS (ESI) calcd for $C_{31}H_{29}F_2N_4O_4^+$ $[M+H]^+$: 559.22, found, 559.2.

**Example 295: preparation of 3-(6-(4-(bis(3-fluorophenyl)methyl)piperazine-1-carbonyl)-1-oxoisoindolin-2-yl)piperidine-2,6-dione (GT-06149)**

Referring to the method of Scheme 6, the target product (GT-06149) was prepared as white solid (43 mg, yield 69.48%). $^1H$ NMR (400 MHz, DMSO-d6) δ 11.00 (s, 1H), 7.77 - 7.64 (m, 4H), 7.46 (s, 6H), 7.17 (s, 2H), 5.12 (dd, *J* = 13.3, 5.1 Hz, 1H), 4.51 (d, *J* = 17.8 Hz, 1H), 4.38 (d, *J* = 17.7 Hz, 1H), 3.55 (s, 3H), 3.35 - 3.29 (m, 2H), 2.93 (ddd, *J* = 22.7, 18.7, 15.8 Hz, 4H), 2.61 (d, *J* = 16.9 Hz, 2H), 2.44 - 2.35 (m, 1H), 2.06 - 1.96 (m, 1H). LCMS (ESI) calcd for $C_{31}H_{29}F_2N_4O_4^+$ $[M+H]^+$: 559.22, found, 559.2.

**Example 296: preparation of 3-(7-(4-(bis(3-fluorophenyl)methyl)piperazine-1-carbonyl)-1-oxoisoindolin-2-yl)piperidine-2,6-dione (GT-06150)**

Referring to the method of Scheme 6, the target product (GT-06150) was prepared as white solid (32 mg, yield 51.71%). $^1H$ NMR (400 MHz, DMSO-d6) δ 11.03 (s, 1H), 7.67 (d, *J* = 6.4 Hz, 6H), 7.42 (d, *J* = 37.2 Hz, 4H), 7.18 (s, 2H), 5.04 (s, 2H), 4.63 - 4.18 (m, 3H), 3.52 (s, 3H), 2.89 (s, 4H), 2.65 (s, 2H), 2.41 (s, 1H), 2.02 (s, 1H). LCMS (ESI) calcd for $C_{31}H_{29}F_2N_4O_4^+$ $[M+H]^+$: 559.22, found, 559.2.

**Example 297: preparation of 5-(4-(bis(3-fluorophenyl)methyl)piperazine-1-carbonyl)-2-(2,6-dioxopiperidin-3-yl)isoindoline-1,3-dione (GT-06206)**

Referring to the method of Scheme 6, the target product (GT-06206) was prepared as white solid (31 mg, yield 48.94%). $^1H$ NMR (400 MHz, DMSO-d6) δ 11.14 (s, 1H), 8.00 (d, *J* = 7.6 Hz, 1H), 7.95 - 7.86 (m, 2H), 7.46 (s, 6H), 7.17 (s, 2H), 5.18 (dd, *J* = 12.7, 5.4 Hz, 1H), 4.30 (s, 1H), 3.64 (s, 4H), 3.14 (s, 2H), 3.10 - 2.85 (m, 2H), 2.58 (dd, *J* = 23.7, 11.5 Hz, 2H), 2.34 (s, 1H), 2.14 - 1.97 (m, 1H). LCMS (ESI) calcd for $C_{31}H_{27}F_2N_4O_5^+$ $[M+H]^+$: 573.19, found, 573.2.

**Example 298: preparation of 5-(4-benzhydrylpiperazin-1-yl)-2-(2,6-dioxopiperidin-3-yl)isoindoline-1,3-dione (GT-06208)**

Referring to the method of Scheme 8, the target product (GT-06208) was prepared as yellow solid (5.6 mg, yield 8.77%). $^1H$ NMR (400 MHz, DMSO-d6) δ 11.02 (s, 1H), 7.73 (d, *J* = 41.2 Hz, 4H), 7.53 - 7.12 (m, 9H), 5.09 - 4.95 (m, 1H), 4.09 (s, 1H), 3.58 (s, 3H), 3.09 (d, *J* = 41.1 Hz, 4H), 2.87 - 2.77 (m, 1H), 2.57 - 2.45 (m, 2H), 2.41 - 2.29 (m, 1H), 1.95 (d, *J* = 11.4 Hz, 1H). LCMS (ESI) calcd for $C_{30}H_{29}N_4O_4^+$ $[M+H]^+$: 509.22, found, 509.2.

**Example 299: preparation of 5-(4-benzhydrylpiperazin-1-yl)-2-(2,6-dioxopiperidin-3-yl)-6-fluoroisoindoline-1,3-dione (GT-06209)**

Referring to the method of Scheme 8, the target product (GT-06209) was prepared as yellow solid (15 mg, yield 24.00%). $^1H$ NMR (400 MHz, DMSO-d6) δ 12.03 (s, 1H), 11.04 (s, 1H), 7.91 - 7.62 (m, 4H), 7.40 (t, *J* = 28.4 Hz, 8H), 5.62 (s, 1H), 5.04 (dd, *J* = 12.7, 5.3 Hz, 1H), 3.66 (d, *J* = 36.9 Hz, 3H), 3.21 (d, *J* = 11.7 Hz, 4H), 2.88 - 2.75 (m, 1H), 2.49 (dd, *J* = 23.9, 11.5 Hz, 2H), 2.40 (s, 1H), 2.03 - 1.90 (m, 1H). LCMS (ESI) calcd for $C_{30}H_{28}FN_4O_4^+$ $[M+H]^+$: 527.21, found, 527.3.

**Example 300: preparation of 5-(4-benzhydrylpiperazin-1-yl-2,2,3,3,5,5,6,6-ds)-2-(2,6-dioxopiperidin-3-yl)-6-fluoroisoindoline-1,3-dione (GT-06210)**

Referring to the method of Scheme 8, the target product (GT-06210) was prepared as yellow solid, (13.5 mg, yield 21.69%). $^1H$ NMR (400 MHz, DMSO-d6) δ 11.93 (s, 1H), 11.04 (s, 1H), 7.92 - 7.61 (m, 4H), 7.40 (t, *J* = 29.2 Hz, 8H), 5.61 (s, 1H), 5.04 (dd, *J* = 12.6, 5.2 Hz, 1H), 2.87 - 2.74 (m, 1H), 2.62 - 2.45 (m, 2H), 2.04 - 1.89 (m, 1H). LCMS (ESI) calcd for $C_{30}H_{20}D_8FN_4O_4^+$ $[M+H]^+$: 535.26, found, 535.3.

**Example 301: preparation of 5-((1-benzhydryl-5-chloropiperidin-3-yl)amino)-2-(2,6-dioxopiperidin-3-yl)-6-fluoroisoindoline-1,3-dione (GT-06211)**

Referring to the method of Scheme 8, the target product (GT-06211) was prepared as white solid (13.5 mg, yield 22.53%). $^1H$ NMR (400 MHz, DMSO-d6) δ 11.02 (s, 1H), 7.54 (t, *J* = 12.2 Hz, 1H), 7.38 (d, *J* = 16.4 Hz, 3H), 7.25 (ddd, *J* = 50.3, 27.4, 20.3 Hz, 6H), 6.95 (s, 1H), 6.55 (s, 1H), 5.00 (dd, *J* = 12.9, 5.2 Hz, 1H), 4.58 (d, *J* = 44.2 Hz, 1H), 4.04 (s, 1H), 3.36 - 3.34 (m, 2H), 2.81 (dd, *J* = 22.2, 10.2 Hz, 1H), 2.68 - 2.44 (m, 4H), 2.23 - 1.90 (m, 3H). LCMS (ESI) calcd for $C_{31}H_{29}ClFN_4O_4^+$ $[M+H]^+$: 575.19, found, 575.3.

**Example 302: preparation of 3-(5-(4-(bis(4-(trifluoromethyl)phenyl)methyl)piperazine-1-carbonyl)-1-oxoisoindolin-2-yl)piperidine-2,6-dione (GT-06216)**

Referring to the method of Scheme 6, the target product (GT-06216) was prepared as white solid (35 mg, yield 53.57%). $^1$H NMR (400 MHz, DMSO-d6) δ 11.01 (s, 1H), 7.85 (s, 3H), 7.78 (d, *J* = 7.6 Hz, 6H), 7.64 (s, 1H), 7.53 (d, *J* = 7.8 Hz, 1H), 5.13 (dd, *J* = 13.2, 5.1 Hz, 1H), 4.43 (dd, *J* = 49.3, 17.6 Hz, 2H), 3.51 (s, 4H), 3.42 - 3.37 (m, 4H), 2.93 - 2.87 (m, 1H), 2.61 (d, *J* = 16.5 Hz, 2H), 2.43 - 2.33 (m, 1H), 2.04 - 1.96 (m, 1H). LCMS (ESI) calcd for $C_{33}H_{29}F_6N_4O_4^+$ [M+H]$^+$: 659.21, found, 659.2.

**Example 303: preparation of 5-(4-(bis(4-(trifluoromethyl)phenyl)methyl)piperazine-1-carbonyl)-2-(2,6-dioxopiperidin-3-yl)isoindoline-1,3-dione (GT-06217)**

Referring to the method of Scheme 6, the target product (GT-06217) was prepared as white solid (37 mg, yield 55.51%). $^1$H NMR (400 MHz, DMSO-d6) δ 11.14 (s, 1H), 7.98 (d, *J* = 7.6 Hz, 1H), 7.89 (d, *J* = 8.4 Hz, 2H), 7.81 (d, *J* = 39.5 Hz, 8H), 5.18 (dd, *J* = 12.8, 5.4 Hz, 1H), 3.63 (s, 5H), 3.36 - 3.31 (m, 2H), 2.91 - 2.72 (m, 2H), 2.64 - 2.55 (m, 3H), 2.13 - 2.00 (m, 1H). LCMS (ESI) calcd for $C_{33}H_{27}F_6N_4O_5^+$ [M+H]$^+$: 673.19, found, 673.2.

**Example 304: preparation of 3-(5-(4-(bis(4-(benzyloxy)phenyl)methyl)piperazine-1-carbonyl)-1-oxoisoindolin-2-yl)piperidine-2,6-dione (GT-06557)**

Referring to the method of Scheme 6, the target product (GT-06557) was prepared as white solid (27 mg, yield 43.76%). $^1$H NMR (400 MHz, DMSO-d6) δ 12.08 (s, 1H), 11.01 (s, 1H), 7.76 (dd, *J* = 36.9, 21.6 Hz, 5H), 7.56 (d, *J* = 7.7 Hz, 1H), 7.50 - 7.27 (m, 13H), 7.23 (d, *J* = 8.6 Hz, 1H), 7.15 - 6.90 (m, 5H), 5.50 (d, *J* = 69.1 Hz, 1H), 5.21 - 5.00 (m, 6H), 4.44 (dd, *J* = 48.3, 17.7 Hz, 2H), 3.74 (s, 2H), 3.48 (s, 2H), 3.12 (s, 3H), 2.97 - 2.85 (m, 1H), 2.61 (d, *J* = 17.2 Hz, 1H), 2.47 - 2.32 (m, 1H), 2.07 - 1.86 (m, 1H). LCMS (ESI) calcd for $C_{45}H_{42}N_4NaO_6^+$ [M+Na]$^+$: 757.30, found, 757.3.

**Example 305: preparation of 5-(4-(bis(4-(benzyloxy)phenyl)methyl)piperazine-1-carbonyl)-2-(2,6-dioxopiperidin-3-yl)isoindoline-1,3-dione (GT-06558)**

Referring to the method of Scheme 6, the target product (GT-06558) was prepared as white solid (25 mg, yield 39.79%). $^1$H NMR (400 MHz, DMSO-d6) δ 11.80 (s, 1H), 11.15 (s, 1H), 8.05 - 7.87 (m, 3H), 7.72 (s, 3H), 7.46 - 7.31 (m, 12H), 7.18 - 6.89 (m, 5H), 5.38 (s, 1H), 5.27 - 4.97 (m, 6H), 3.67 (s, 4H), 3.14 (s, 3H), 2.91 (dd, *J* = 22.7, 8.9 Hz, 1H), 2.70 - 2.54 (m, 2H), 2.05 (dd, *J* = 15.9, 10.7 Hz, 1H). LCMS (ESI) calcd for $C_{45}H_{40}N_4NaO_7^+$ [M+Na]$^+$: 771.28, found, 771.3.

**Biological activity assay**

**Reagents and Materials:**

| Reagents and materials | Suppliers or Source |
|---|---|
| Human T cell acute lymphoblastic leukemia cell line: CCRF-CEM | Dalian Meilun Biotech Co., Ltd. |
| Human acute lymphoblastic leukemia cell: Kasumi-1 | ATCC (American Type Culture Collection) |
| Human breast cancer cell: MDA-MB-231 | Dalian Meilun Biotech Co., Ltd. |
| Human multiple myeloma cell: MM.1S | ATCC |
| Human T lymphocyte leukemia cell: Jurkat | ATCC |
| Human multiple myeloma cell line resistant to dexamethasone: MM.1R | ATCC |
| Human diffuse large B-cell lymphoma cell line: TMD8 | Kyinno Biotechnology Co., Ltd |
| Human small cell lung cancer cell: H146 | Cell Bank of the Committee on Type Culture Collection of the Chinese Academy of Sciences |
| Human thyroid carcinoma cell: CAL-62 | Dalian Meilun Biotech Co., Ltd. |
| Human peripheral blood mononuclear cell: hPBMC | Milecell Biotechnologies Inc, Shanghai |
| RPMI-1640 Medium | Dalian Meilun Biotech Co., Ltd. |
| DMEM Medium | Dalian Meilun Biotech Co., Ltd. |
| DMEM Medium, high glucose | Dalian Meilun Biotech Co., Ltd. |

(continued)

| Reagents and materials | Suppliers or Source |
|---|---|
| Meilun Cell Counting Kit-8 | Dalian Meilun Biotech Co., Ltd. |
| Fetal bovine serum (FBS) | Sunrise Science Products Company |
| Penicillin-Streptomycin | Beijing TransGen Biotech Co., Ltd |
| Mycoplasma detection kit | Beijing TransGen Biotech Co., Ltd |
| STR genotype detection | Shanghai Biowing Applied Biotechnology Co. Ltd |

**Methods:**

**Cell cultures**

The tumor cells used herein were cultured in a cell culture medium listed in table 2 at 37°C in an incubator with 5% $CO_2$. Prior to experimentation, all cells used were correctly identified by STR profiling, and tested negative for mycoplasma through routine screenings.

Table 2. Tumor cells and cell culture medium used in the present disclosure

| Cell line | Cell culture medium used |
|---|---|
| CCRF-CEM | RPMI 1640 culture medium supplemented with 10% FBS, as well as penicillin at a final concentration of 100 U/mL and streptomycin at a final concentration of 100 μg/mL |
| Kasumi-1 | |
| MM.1S | |
| Jurkat | |
| MM.1R | |
| TMD8 | |
| H146 | |
| MDA-MB-231 | DMEM culture medium supplemented with 10% FBS, as well as penicillin at a final concentration of 100 U/mL and streptomycin at a final concentration of 100 μg/mL |
| CAL-62 | DMEM culture medium supplemented with 10% FBS, as well as penicillin at a final concentration of 100 U/mL and streptomycin at a final concentration of 100 μg/mL |

**I. Determination of CRBN-binding affinity of compounds of the present disclosure:**

The CEREBLON BINDING kits (specification: 10,000 tests; product number: Catalog # 64BDCRBNPEH; CISBIO company) was used to determine the CRBN binding ability of the compounds to be tested through a HTRF method (homogeneous time-resolved fluorescence). The specific methods are as follows:

**1.** According to the instructions of the CEREBLON BINDING kits, the compounds to be tested of the present disclosure and lenalidomide were serially diluted using diluent #9 (1X) solution to obtain a final concentration of 2 μM for both the tested compounds and lenalidomide solution.
**2.** 2.5 μL of the above 2 μM of the tested compounds and lenalidomide solution, as well as the same volume of diluent #9 (1X) solution (solvent control group, Std0) were added to each well of a 96-well plate, respectively. Then, 2.5 μL of human Cereblon WT GST-tagged protein solution was added to each well. Finally, 5 μL of the thoroughly mixed Thalidomide-Red reagent and GST Eu antibody working solution were added to each of the aforementioned wells. The final concentration of the tested compounds and lenalidomide in each well is 0.5 μM.
**3.** The blank control wells were sequentially added with 2.5 μL of diluent #9 (1X) solution, 2.5 μL of PROTAC binding buffer, and 5 μL of thoroughly mixed Thalidomide-Red reagent and GST Eu antibody working solution.
**4.** After sealing and incubating the solutions in the aforementioned wells at room temperature for 3 hours, the absorbance values at emission wavelengths of 620 nm and 665 nm were detected by the HTRF method using a Spark microplate reader (V3.1 SP1).

The corresponding CRBN binding inhibition rate was calculated using the following method:

$$R_{compound} = OD\ 665\ nm_{compound} / OD\ 620\ nm_{compound} - OD\ 665\ nm_{control} / OD\ 620\ nm_{control}$$

$$R_{Std0} = OD\ 665\ nm_{Std0} / OD\ 620\ nm_{Std0} - OD\ 665\ nm_{control} / OD\ 620\ nm_{control}$$

$$\text{inhibition rate (\%)} = (1 - R_{compound} / R_{Std0}) * 100$$

Note: OD 665 nm $_{compound}$ is the absorbance value of each well of the compounds to be tested of the present disclosure at an emission wavelength of 665 nm.
OD 620 nm $_{compound}$ is the absorbance value of each well of the compounds to be tested of the present disclosure at an emission wavelength of 620 nm.
OD 665 nm $_{control}$ is the absorbance value of the blank control well at an emission wavelength of 665 nm.
OD 620 nm $_{control}$ is the absorbance value of the blank control well at an emission wavelength of 620 nm.
OD 665 nm $_{Std0}$ is the absorbance value of the solvent control well at an emission wavelength of 665 nm.
OD 620 nm $_{Std0}$ is the absorbance value of the solvent control well at an emission wavelength of 620 nm.

The test results were shown in Table 3 below.

Table 3. HTRF inhibitory activity ($IC_{50}$, μM) of the compounds of the present disclosure (including but not limited to the compounds in Tables 1 and 2, and the Examples 1-305 compounds)

| Comp. No. | HTRF $IC_{50}$ (μM) | Comp. No. | HTRF $IC_{50}$ (μM) | Comp. No. | HTRF $IC_{50}$ (μM) | Comp. No. | HTRF $IC_{50}$ (μM) |
|---|---|---|---|---|---|---|---|
| lenalidomide | d | GT-05894 | a | GT-05716 | a | GT-05980 | b |
| GT-03245 | b | GT-05895 | a | GT-03386 | b | GT-05981 | b |
| GT-03466 | a | GT-05896 | b | GT-03564 | a | GT-06092 | a |
| GT-03646 | b | GT-05897 | b | GT-03565 | a | GT-06093 | b |
| GT-05686 | c | GT-03571 | a | GT-03647 | b | GT-05920 | a |
| GT-05696 | a | GT-03574 | b | GT-05719 | b | GT-05921 | b |
| GT-05688 | c | GT-06046 | b | GT-05720 | c | GT-06059 | a |
| GT-05867 | c | GT-03577 | a | GT-03875 | b | GT-06060 | b |
| GT-05744 | a | GT-03873 | a | GT-06116 | b | GT-06056 | a |
| GT-05864 | a | GT-03874 | a | GT-06117 | b | GT-06057 | a |
| GT-05743 | c | GT-03872 | c | GT-06084 | a | GT-06090 | c |
| GT-05859 | a | GT-05965 | c | GT-06085 | b | GT-06061 | b |
| GT-05745 | a | GT-05966 | c | GT-06086 | b | GT-06062 | b |
| GT-05868 | b | GT-05967 | c | GT-06087 | a | GT-05854 | c |
| GT-06198 | a | GT-03993 | a | GT-03566 | c | GT-05877 | c |
| GT-06136 | a | GT-04163 | b | GT-03567 | c | GT-05929 | b |
| GT-06072 | a | GT-05963 | b | GT-03568 | c | GT-06142 | b |
| GT-05865 | b | GT-05964 | b | GT-05735 | c | GT-06144 | b |
| GT-05866 | b | GT-06095 | b | GT-05736 | c | GT-05881 | b |
| GT-03994 | a | GT-06096 | b | GT-06048 | a | GT-05882 | b |
| GT-04164 | b | GT-06097 | b | GT-05907 | a | GT-05930 | a |
| GT-05738 | c | GT-06098 | a | GT-04244 | a | GT-06146 | a |
| GT-05901 | a | GT-04165 | a | GT-04225 | a | GT-06147 | a |
| GT-05902 | b | GT-04166 | b | GT-04226 | a | GT-06148 | a |
| GT-05903 | b | GT-05960 | c | GT-04227 | b | GT-06149 | b |

(continued)

| Comp. No. | HTRF $IC_{50}$ (μM) | Comp. No. | HTRF $IC_{50}$ (μM) | Comp. No. | HTRF $IC_{50}$ (μM) | Comp. No. | HTRF $IC_{50}$ (μM) |
|---|---|---|---|---|---|---|---|
| GT-05904 | b | GT-05961 | b | GT-06133 | a | GT-06150 | b |
| GT-05905 | b | GT-03835 | b | GT-04525 | a | GT-06104 | a |
| GT-05969 | c | GT-04523 | b | GT-05856 | a | GT-05886 | c |
| GT-05970 | c | GT-04524 | b | GT-05857 | a | GT-05887 | c |
| GT-05971 | c | GT-03832 | b | GT-05858 | b | GT-05985 | b |
| GT-05972 | c | GT-03833 | c | GT-04948 | b | GT-05888 | c |
| GT-05973 | c | GT-04249 | a | GT-04992 | a | GT-06069 | b |
| GT-06002 | b | GT-04250 | a | GT-05689 | b | GT-05986 | a |
| GT-06024 | b | GT-04251 | a | GT-05850 | c | GT-05926 | a |
| GT-06025 | b | GT-06111 | a | GT-05721 | b | GT-05927 | a |
| GT-06026 | b | GT-06113 | b | GT-05988 | a | GT-05923 | a |
| GT-06027 | c | GT-04205 | a | GT-06063 | b | GT-05924 | b |
| GT-06050 | a | GT-04247 | a | GT-06065 | a | GT-05728 | c |
| GT-06044 | a | GT-04248 | b | GT-06067 | b | GT-05730 | c |
| GT-06051 | b | GT-06135 | a | GT-05916 | a | GT-06114 | c |
| GT-06045 | a | GT-04945 | b | GT-05990 | a | GT-05918 | c |
| GT-06052 | a | GT-04383 | a | GT-05991 | a | GT-05922 | c |
| GT-03569 | b | GT-05713 | b | GT-05977 | b | GT-06206 | c |
| GT-05737 | b | GT-04384 | b | GT-05978 | c | GT-06141 | b |
| GT-03575 | b | GT-05714 | c | GT-05983 | b | GT-05976 | a |
| GT-03570 | a | GT-05715 | c | GT-06055 | a | GT-05936 | b |
| GT-03573 | a | GT-05958 | b | GT-03576 | a | GT-03700 | b |
| GT-03467 | b | GT-03721 | a | GT-03724 | a | GT-03837 | a |
| GT-03838 | b | GT-03839 | c | GT-03840 | c | GT-03722 | a |
| GT-03725 | b | GT-03723 | b | GT-03726 | a | GT-03834 | a |
| GT-03836 | b | GT-06640 | b | GT-03698 | b | GT-03702 | a |
| GT-03703 | a | GT-03701 | b | GT-03704 | a | GT-06387 | c |
| GT-06806 | c | GT-06071 | b | GT-06597 | b | | |
| In Table 3, the symbols a, b, and c are defined as follows: 0<a≤0.5uM, 0.5μM <b≤1μM, 1μM <c<1.9μM, 1.9μM ≤ d. | | | | | | | |

The results show that the compounds of the present disclosure (including the compounds listed in Table 1, and the Examples 1-305 compounds) exhibit strong binding affinity to CRBN.

## II. Western-blot assay

The effect of the compounds of the present disclosure on the expression of target proteins in cells was detected using conventional Western blot analysis.

Human peripheral blood mononuclear cells (hPBMCs) at a density of $1\times10^6$ cells/mL were thawed according to the PBMC Recovery Protocol (Milecell Biotechnologies Inc, Shanghai). The cells were then treated with the compounds of the present disclosure (including the compounds listed in Table 1, and the Examples 1-305 compounds) at concentration of 500 nM. Controls included a negative control (DMSO) and a positive control (the commercial immunomodulatory drug

lenalidomide), both processed following the identical experimental protocol as the compounds of the present disclosure. After 24 hours of compounds treatment, cells were harvested and lysed in RIPA protein lysate containing phosphatase inhibitors on ice for 30-60 min, followed by centrifugation. The supernatant was aspirated as the extracted total cell protein. The protein concentration of the supernatant was determined using the conventional BCA (Bicinchoninic Acid) assay. The supernatant was then mixed with 4X SDS sample loading buffer, denatured at 95°C for 5 minutes, and then subjected to SDS-PAGE electrophoresis on a polyacrylamide gel. Proteins were then transferred to a nitrocellulose (NC) membrane, blocked at room temperature for 1-2 h, and processed for antibody incubation and detection according to the manufacturer's instructions (Cell Signaling Technology).

Half-Maximal Degradation Concentration value (the drug concentration required for degrading proteins by 50%, abbreviated as $DC_{50}$) reads method: comparing the grayscale values of Western blot bands from drug-treated samples (protein degrader compounds) with those from blank (DMSO-treated) controls, and identifying the drug concentration range at which the grayscale value is half that of the DMSO-treated control bands.

$DC_{50}$ values can be calculated using ImageJ software to measure grayscale values of Western blot bands from drug-treated samples. The relationship curve between drug concentration and grayscale values is fitted to estimate the drug concentration corresponding to half the grayscale value.

Target protein remaining (%) refers to the percentage of target protein remaining in hPBMC cells after treatment with protein degrader compounds.

Calculation method for protein remaining (%): Use ImageJ software to measure grayscale values of Western blot bands from drug (protein degrader)-treated samples. The grayscale value of drug-treated bands is divided by that of internal control protein bands, and the resulting quotient is normalized to obtain the relative percentage of remaining target protein after protein degrader treatment.

Target protein degradation rate (%) = 100% - target protein remaining (%).

The effects of the compounds of the present disclosure (including the compounds listed in Table 1, and the Examples 1-305 compounds) on substrate proteins expression in hPBMC cells were shown in Table 4 below.

Table 4: Degradation effects (substrate protein remaining %) of the compounds of the present disclosure on substrate proteins at a concentration level of 500 nM

| Comp. No. | **IKZF1** | **IKZF3** | **GSPT1** | Comp. No. | **IKZF1** | **IKZF3** | **GSPT1** | Comp. No. | **IKZF1** | **IKZF3** | **GSPT1** |
|---|---|---|---|---|---|---|---|---|---|---|---|
| lenalid omide | B | B | B | GT-04249 | A | A | A | GT-05854 | B | B | A |
| GT-03245 | A | A | A | GT-04250 | A | A | A | GT-05877 | B | B | A |
| GT-03466 | A | A | A | GT-04251 | A | A | A | GT-05929 | A | A | A |
| GT-03700 | A | B | A | GT-04205 | A | A | A | GT-06142 | B | B | A |
| GT-05696 | A | A | A | GT-04247 | A | A | A | GT-05881 | B | B | A |
| GT-05688 | A | A | B | GT-04248 | A | B | A | GT-05882 | B | B | A |
| GT-05744 | B | B | A | GT-04945 | B | B | A | GT-05930 | B | B | A |
| GT-05859 | A | B | A | GT-04383 | B | B | A | GT-06146 | A | A | A |
| GT-05868 | A | A | A | GT-05713 | B | B | A | GT-06147 | B | B | A |
| GT-03833 | B | A | A | GT-04384 | B | B | A | GT-06148 | B | B | A |
| GT-05866 | B | A | A | GT-05920 | B | B | A | GT-06104 | A | B | A |
| GT-03994 | B | B | A | GT-06071 | B | B | A | GT-05938 | B | B | A |
| GT-04164 | B | B | A | GT-04386 | B | B | A | GT-06597 | A | A | A |
| GT-05738 | B | B | A | GT-06640 | A | A | A | GT-05886 | B | A | A |
| GT-04523 | A | A | A | GT-03386 | A | A | A | GT-05986 | B | B | A |
| GT-04524 | A | A | A | GT-03564 | A | A | A | GT-06090 | B | B | A |
| GT-05905 | B | B | A | GT-03698 | A | A | A | GT-05926 | B | B | A |
| GT-05970 | B | B | A | GT-03565 | B | B | A | GT-05927 | B | B | A |
| GT-05971 | B | B | A | GT-03647 | B | B | A | GT-05690 | A | A | A |
| GT-05973 | B | A | A | GT-03702 | A | A | A | GT-05976 | B | B | A |

(continued)

| Comp. No. | **IKZ F1** | **IKZ F3** | **GSP T1** | Comp. No. | **IKZ F1** | **IKZ F3** | **GSP T1** | Comp. No. | **IKZ F1** | **IKZ F3** | **GSP T1** |
|---|---|---|---|---|---|---|---|---|---|---|---|
| GT-03832 | B | A | A | GT-03703 | A | A | A | GT-05739 | B | B | A |
| GT-06027 | B | B | A | GT-03701 | A | A | A | GT-06053 | B | B | A |
| GT-06050 | B | B | A | GT-03704 | B | A | A | GT-05959 | B | B | A |
| GT-06044 | B | A | A | GT-06117 | B | B | A | GT-06047 | B | B | A |
| GT-06051 | B | B | A | GT-06084 | A | A | A | GT-05968 | B | B | A |
| GT-06045 | B | B | A | GT-06085 | A | A | A | GT-05730 | B | B | A |
| GT-06052 | B | B | A | GT-06086 | A | A | A | GT-06099 | B | B | A |
| GT-03569 | B | A | A | GT-06087 | B | B | A | GT-05934 | B | A | A |
| GT-03572 | B | A | A | GT-06199 | A | A | A | GT-05723 | B | B | A |
| GT-05737 | B | B | A | GT-06200 | B | A | A | GT-06114 | B | B | A |
| GT-03575 | B | B | A | GT-06201 | B | A | A | GT-06115 | B | B | A |
| GT-03570 | B | B | A | GT-03836 | B | B | A | GT-06639 | A | A | A |
| GT-05907 | A | A | A | GT-06387 | B | A | A | GT-05726 | B | A | A |
| GT-03721 | B | A | A | GT-03566 | A | A | A | GT-06118 | A | A | A |
| GT-03724 | A | A | A | GT-03567 | B | A | A | GT-06088 | B | B | A |
| GT-05894 | A | B | A | GT-03699 | B | A | A | GT-06203 | B | A | A |
| GT-03837 | B | A | A | GT-03648 | B | B | A | GT-06596 | A | A | A |
| GT-03838 | A | A | A | GT-06048 | A | A | A | GT-05727 | B | A | A |
| GT-03839 | B | A | A | GT-06424 | B | A | A | GT-06049 | B | A | A |
| GT-03722 | A | A | A | GT-04225 | A | A | A | GT-05908 | A | A | A |
| GT-03725 | A | A | A | GT-04226 | A | A | A | GT-06425 | B | A | A |
| GT-03571 | B | B | A | GT-04227 | A | A | A | GT-05741 | A | A | A |
| GT-03574 | B | B | A | GT-06133 | B | B | A | GT-05742 | A | A | A |
| GT-06046 | B | B | A | GT-04525 | A | A | A | GT-06068 | B | B | A |
| GT-05932 | B | B | A | GT-05856 | A | A | A | GT-05925 | B | B | A |
| GT-03723 | B | B | A | GT-05857 | A | A | A | GT-05982 | B | B | A |
| GT-03726 | B | B | A | GT-05858 | B | B | A | GT-06094 | B | B | A |
| GT-03873 | A | B | A | GT-04948 | B | B | A | GT-05922 | B | B | A |
| GT-06093 | B | B | A | GT-04992 | B | B | A | GT-06061 | B | B | A |
| GT-03872 | B | A | A | GT-05937 | B | B | A | GT-06278 | B | B | A |
| GT-05965 | A | A | A | GT-05689 | B | B | A | GT-05880 | B | B | A |
| GT-05966 | B | B | A | GT-05850 | A | B | A | GT-05924 | B | B | A |
| GT-05967 | B | A | A | GT-05923 | B | B | A | GT-05885 | B | B | A |
| GT-03993 | B | A | A | GT-05851 | A | B | A | GT-06206 | B | B | A |
| GT-04163 | B | A | A | GT-05892 | B | B | A | GT-06141 | B | B | A |
| GT-05963 | B | B | A | GT-05988 | B | B | A | GT-06217 | B | B | A |
| GT-06095 | B | B | A | GT-06063 | B | B | A | GT-06598 | B | B | A |
| GT-06096 | B | A | A | GT-06065 | B | B | A | GT-05890 | B | B | A |
| GT-06097 | B | B | A | GT-06067 | B | B | A | GT-06558 | A | B | A |

(continued)

| Comp. No. | **IKZF1** | **IKZF3** | **GSPT1** | Comp. No. | **IKZF1** | **IKZF3** | **GSPT1** | Comp. No. | **IKZF1** | **IKZF3** | **GSPT1** |
|---|---|---|---|---|---|---|---|---|---|---|---|
| GT-05960 | B | A | A | GT-05891 | B | B | A | GT-05928 | B | B | A |
| GT-05961 | B | A | A | GT-05980 | A | B | A | GT-06092 | B | B | A |
| GT-03835 | A | A | A | | | | | | | | |
| In Table 4, A represents 0% ≤ substrate protein remaining % ≤ 50%, B represents substrate protein remaining % >50%. | | | | | | | | | | | |

Experimental results demonstrated that the compounds of the present invention (including those listed in Table 1 and Examples 1-305) effectively degraded substrate proteins (e.g., IKZF1, IKZF3, GSPT1) at a concentration of 500 nM.

The present inventors further evaluated the effects of the compounds of the present disclosure on target protein expression in hPBMCs at low concentration levels. Western blot results were shown in Figure 1. As shown in Figure 1, lenalidomide failed to degrade GSPT1 protein at concentrations of 0-1000 nM, and could not degrade IKZF1 or IKZF3 proteins at either 50 nM or 500 nM concentrations. In contrast, the compounds of the present disclosure significantly degraded IKZF1, IKZF3 or GSPT1 proteins (Figure 1). Notably, the compounds GT-05688, GT-05689, GT-05690, GT-04525, GT-05856, GT-05857, GT-05858 and GT-05742 demonstrated marked degradation effects at concentrations below 50 nM. Specifically: compound GT-05688 selectively and significantly degraded IKZF1 and IKZF3 proteins at <50 nM; compounds GT-05689 and GT-05858 selectively and significantly degraded GSPT1 protein at <50 nM; compound GT-05690 clearly degraded GSPT1 at <50 nM and significantly degraded IKZF1, IKZF3 and GSPT1 proteins at <500 nM; compounds GT-04525, GT-05856, GT-05857 and GT-05742 significantly degraded IKZF1, IKZF3 and GSPT1 proteins at <50 nM; and compound GT-04948 selectively and significantly degraded GSPT1 protein at <500 nM. The Western blot results demonstrated that, compared to lenalidomide, the compounds of the present disclosure exhibited significantly improved efficiency in inducing substrate protein degradation and were capable of selectively inducing degradation of substrate proteins. In summary, the structural diversity of the compounds of the present disclosure enables effective degradation of diverse substrate proteins, making them suitable for treating indications associated with these substrate proteins.

**III. Determination of half inhibitory concentration ($IC_{50}$) of the compounds of the present disclosure against tumor cells:**

$IC_{50}$ values of the compounds of the present disclosure (including the compounds in Table 1 and the Examples 1-305 compounds) were measured using the Meilun Cell Counting Kit-8 kit from Dalian Meilun Biotech Co., Ltd. Assay details are as follows: tumor cells were seeded in a 96-well plate at a density listed in Table 5. After 24h, 100 μL of the inoculated cells were treated with 0.5μL of the compounds of the present disclosure (including the compounds in Table 1 and the Examples 1-305 compounds) at 10 different successively decreasing concentrations (starting at the highest concentration of 10μM; 5-fold serial dilutions). After the cells were treated with the compounds of the present disclosure for a period of time, the cell viability detection reagent was added to the culture medium for cell viability assessment according to the instructions provided with the CCK-8 kit. DMSO served as the negative control, and corresponding commercial inhibitors immunomodulatory agents (including lenalidomide and pomalidomide) were used as the positive control, both of which treated the cells using the same protocol as that of the compounds of the present disclosure. The growth inhibition of the compounds of the present disclosure on cells was plotted by Prism Graphpad software, and the $IC_{50}$ values of the compounds of the present disclosure were calculated therefrom. Results were shown in Table 6 below.

Table 5. Seeding protocol and treatment time for tumor cells

| Cells | Cells seeding protocol | The times for treating cells with the compounds of the present disclosure (hours) |
|---|---|---|
| Kasumi-1 | cells were seeded at a density of 10,000 cells per well in 100 μL of serum-containing RPMI-1640 medium per well | 96 h |
| MM.1S | | |
| MM.1R | | |
| TMD8 | | |
| Jurkat | | |

(continued)

| Cells | Cells seeding protocol | The times for treating cells with the compounds of the present disclosure (hours) |
|---|---|---|
| H146 | cells were seeded at a density of 10,000 cells per well in 100 μL of serum-containing RPMI-1640 medium per well | 72 h |
| CCRF-CEM | cells were seeded at a density of 4,000 cells per well in 100 μL of serum-containing RPMI-1640 medium per well | 96 h |
| MDA-MB-231 | cells were seeded at a density of 4,000 cells per well in 100 μL of serum-containing DMEM medium per well | 96 h |
| CAL-62 | cells were seeded at a density of 2,000 cells per well in 100 μL of serum-containing high-glucose DMEM medium per well | 96 h |

Results of inhibiting tumor cell proliferation were shown in Table 6 below.

Table 6. The inhibitory activity ($IC_{50}$, nM) of the compounds of the present disclosure designed based on immunomodulatory drugs against tumor cells proliferation

| Comp. No./Name | MM.1S | MM.1 R | H146 | TMD8 | CCRF-CEM | MDA -MB-231 | Kasumi -1 | Jurkat | CAL-62 |
|---|---|---|---|---|---|---|---|---|---|
| lenalidomide | F | F | F | F | F | F | F | F | F |
| pomalidomid e | C | F | F | E | E | F | F | F | F |
| GT-03245 | A++ | A+++ | A+ | A+ | A+++ | A++ | A+++ | A++ | A+++ |
| GT-03466 | A++ | A++ | A+ | A+ | A+++ | A++ | A++ | A++ | A++ |
| GT-03700 | A++ | A++ | A+ | A+ | A++ | A++ | A++ | A++ | A++ |
| GT-03467 | B | - | - | - | A+ | B | A | - | - |
| GT-05696 | A | - | - | - | - | - | - | - | - |
| GT-05866 | A++ | - | A+ | A | - | A+ | - | - | - |
| GT-06051 | A | - | B | - | - | - | - | - | - |
| GT-03724 | A+ | A+ | - | - | A+ | - | - | - | - |
| GT-05894 | A++ | - | A+ | C | - | A | - | - | - |
| GT-03837 | A+ | C | - | - | - | - | - | - | - |
| GT-03839 | A+ | A+ | C | - | A+ | A | A+ | - | A+ |
| GT-03840 | - | - | - | - | A+ | - | - | - | - |
| GT-03835 | A++ | A++ | A | B | A++ | A+ | A++ | A++ | A++ |
| GT-04523 | A++ | A++ | A+ | C | - | A | - | A | A++ |
| GT-04524 | A+ | A+ | B | - | - | C | - | A+ | A+ |
| GT-03832 | A++ | A++ | A | B | A++ | A+ | A+ | A++ | A++ |
| GT-03833 | A++ | A++ | A+ | A+ | A+++ | A+ | A++ | A++ | A++ |
| GT-04249 | C | - | - | B | A+ | - | - | - | A+ |
| GT-04250 | B | - | - | - | A | - | - | - | A |
| GT-04251 | A+ | - | B | B | A+ | B | - | A | A+ |
| GT-04205 | B | - | - | A | A | - | - | C | A+ |
| GT-04247 | B | - | - | - | A | - | - | - | A |

(continued)

| Comp. No./Name | MM.1S | MM.1 R | H146 | TMD8 | CCRF-CEM | MDA -MB-231 | Kasumi -1 | Jurkat | CAL-62 |
|---|---|---|---|---|---|---|---|---|---|
| GT-04248 | A | - | B | - | A+ | B | - | A | A+ |
| GT-03386 | A++ | A++ | A | A | A++ | A+ | A+ | A+ | A++ |
| GT-03564 | A++ | A+ | A+ | A+ | A++ | A+ | A++ | A+ | A++ |
| GT-03698 | A++ | A++ | A+ | B | A+++ | A+ | A++ | A++ | A++ |
| GT-03702 | A+++ | A++ | A+ | A+ | A+++ | A++ | A++ | A++ | A+++ |
| GT-03703 | A++ | A++ | A+ | A+ | A+++ | A++ | A++ | A++ | A++ |
| GT-03701 | A++ | A++ | A | B | A++ | A+ | A+ | A+ | A++ |
| GT-03704 | A+ | A+ | B | - | A++ | A | A | B | A+ |
| GT-06084 | A++ | - | A+ | A+ | - | A+ | - | - | - |
| GT-06085 | A++ | - | A+ | A+ | - | A+ | - | - | - |
| GT-06086 | A++ | - | A+ | A+ | - | A+ | - | - | - |
| GT-06087 | A+ | - | A | B | - | B | - | - | - |
| GT-06199 | A+ | - | - | - | - |  | - | - | - |
| GT-06387 | A+++ | - | A++ | A++ | - | A++ | - | - | - |
| GT-03566 | C | B | - | - | A+ | - | - | - | - |
| GT-03699 | A | A | - | - | A+ | - | - | - | A |
| GT-05907 | A++ | - | A+ | A+ | - | A+ | - | - | - |
| GT-04225 | A+++ | - | A++ | A++ | A+++ | A++ | - | A+++ | A+++ |
| GT-04226 | A+++ | - | A++ | A++ | A+++ | A++ | - | A+++ | A+++ |
| GT-04227 | A+++ | - | A++ | A++ | A+++ | A++ | - | A+++ | A+++ |
| GT-06133 | A+ | - | B | - | - | C | - | - | - |
| GT-04525 | A+++ | - | A++ | A++ | - | A++ | - | - | - |
| GT-05856 | A+++ | - | A++ | A++ | - | A++ | - | - | - |
| GT-05857 | A+++ | - | A++ | A++ | - | A++ | - | - | - |
| GT-06146 | A+ | - | B | C | - | B | - | - | - |
| GT-06104 | A+ | - | B | - | - | C | - | - | - |
| GT-06597 | A+ | - | A+ | B | - | A | - | - | - |
| GT-06118 | A | - | B | C | - | - | - | - | - |
| GT-06596 | A | - | B | C | - | B | - | - | - |
| GT-05741 | A+ | - | A | - | - | B | - | - | - |
| Note: In Table 6, the symbols A+++, A++, A+, A, B, C, D, E, and F are defined as follows: 0 < A+++ ≤ 1 nM; 1 nM < A++ ≤ 10 nM; 10 nM < A+ ≤ 50 nM; 50 nM < A ≤ 100 nM; 100 nM < B ≤ 500 nM; 500 nM < C ≤ 1000 nM; 1000 nM < D ≤ 5000 nM; 5000 nM < E ≤ 10000 nM; F > 10000 nM. The symbol "-" indicates "not detected". | | | | | | | | | |

The results showed that the compounds of the present disclosure significantly inhibited the proliferation of MM.1S cells (human multiple myeloma cells), MM.1R cells (dexamethasone-resistant human multiple myeloma cells), H146 cells (human small cell lung carcinoma cells), TMD8 cells (human diffuse large B-cell lymphoma cells), MDA-MB-231 cells (human breast adenocarcinoma cells), Jurkat cells (human T-cell leukemia cells), Kasumi-1 (human acute myeloid leukemia cells), CAL-62 (human thyroid carcinoma cells) and CCRF-CEM (human acute lymphoblastic leukemia T-cells), with superior efficacy compared to the corresponding positive control drugs (Table 6).

**IV. TNF-α Activity Inhibition Assay**

PBMCs were cultured at 37°C with 5% $CO_2$ and seeded in 96-well plates at 1×10^7 cells/well. The compounds to be tested (including those listed in Table 1 and Examples 1-305) were dissolved in DMSO and diluted to appropriate concentrations, with the final DMSO concentration in cell culture media maintained below 0.5%. After 1-hour incubation in medium with or without the compounds, the cells were stimulated with LPS (1 ng/ml) for 18-20 hours. Culture supernatants were then collected, diluted with serum-free medium, and analyzed for TNF-α levels using an ELISA kit. $IC_{50}$ values were calculated using GraphPad Prism 7.0.
TNF-α downregulation is a key mechanism of immunomodulatory agents' antitumor effects. The compounds of the present disclosure demonstrated dose-dependent inhibition of TNF-α levels.

**V. Pharmacokinetic Study of the compounds of the present disclosure**

The pharmacokinetic properties of the compounds of the present disclosure were evaluated using conventional pharmacokinetic experimental methods.
The test compounds of the present disclosure (including the compounds in Table 1 and the Examples 1-305 compounds) were orally administered to experimental animals to evaluate their pharmacokinetic properties. The experimental animals used may be those commonly used in pharmacokinetic experiments, e.g., adult and healthy rodents (e.g., mice, rats (such as Sprague-Dawley (SD) rats), guinea pigs) or non-rodents (rabbits, dogs, and monkeys). In the present disclosure, mice were used as the experimental animals.
The experimental animals were divided into two groups: a control group (for blank plasma collection) and an oral administration group (dosed at 10 mg/kg or 30 mg/kg). Blood samples were collected at predetermined time points after administration, including: 0.25h, 0.5h, 1h, 2h, 4h, 8h, and 24h post-dose. Blank plasma was collected from the control group.
Prior to analysis, plasma samples were processed as follows: To 10 μL of plasma sample were added 10 μL of 50% acetonitrile and 200 μL of internal standard solution (containing 50 ng/mL dexamethasone in acetonitrile). For blank control plasma samples, an equivalent volume of acetonitrile was added in place of the internal standard. The processed samples were vortex-mixed, centrifuged, and the supernatant was subjected to LC-MS/MS analysis.
After preparation of calibration curves and QC samples, the concentrations of compounds in plasma samples were determined by LC-MS/MS analysis. The pharmacokinetic parameters of the test compounds were calculated using the pharmacokinetic calculation software WinNonlin v8.1 with non-compartmental analysis based on the plasma concentration data obtained from LC-MS/MS.
The results demonstrated that the compounds of the present disclosure administered via oral route exhibited favorable drug metabolism and pharmacokinetic (DMPK) properties, indicating their potential as therapeutic agents for cancer patients.

**VI. Effects of the compounds of the present disclosure on TNF-α, IL-10, IL-6, IL-1β, IFN-γ, GM-CSF, IL-2, and IL-12p40 release from PBMCs induced by Lipopolysaccharide or Phytohemagglutinin**

The following materials were used in this study:

VI.1 Reagents and Consumable materials

| Name | Supplier or Source |
|---|---|
| Human PBMC cell | Shanghai Sai Li |
| Lipopolysaccharide (LPS) | Sigma |
| Phytohemagglutinin (PHA) | MCE |
| RPMI 1640 Medium | Gibco |
| Human TNF-alpha DuoSet ELISA | R&D Systems |
| Human IL-6 DuoSet ELISA | R&D Systems |
| Human IL-10 DuoSet ELISA | R&D Systems |
| Human IFN-gamma DuoSet ELISA | R&D Systems |
| Human IL-2 DuoSet ELISA | R&D Systems |
| Human IL-1 beta ELISA Kit | Abclonal |

(continued)

| Name | Supplier or Source |
|---|---|
| Human GM-CSF ELISA Kit | Abclonal |
| Human IL-12/IL-23 p40 ELISA Kit | Abclonal |

VI.2 Methods

VI.2.1 Cell Thawing and Plating

A complete medium was prepared and pre-warmed to 37°C. Cryovials containing cells were retrieved from liquid nitrogen, and the area immediately below the cap-notch interface was firmly gripped with forceps. The cryovials were immersed in a 37°C water bath, and shook occasionally to facilitate thawing. The cell suspension was aspirated and transferred to a centrifuge tube containing 10 mL complete medium, followed by thorough mixing and centrifugation at 400 $\times$ g for 10 min at room temperature. The supernatant was discarded, and the pellet was resuspended in fresh complete medium. After cell counting and the cell density adjustment, the cell suspension was seeded in 96-well plates at a density of $1\times10^5$ cells/well (75 μL/well) and incubated for 2 h at 37°C in an incubator with 5% $CO_2$ under high humidity conditions.

VI.2.2 Compound Preparation and Addition to Cell Plates

1) The test compounds were prepared as 10 mM stock solutions in DMSO.
2) The test compounds were serially diluted 5-fold in DMSO starting from 1 mM (highest concentration) to generate 9 concentration gradients.
3) The test compounds were further diluted in culture medium to five times the final working concentration intended for use.
4) The diluted compounds (25 μL/well) were dispensed into designated wells according to the plate layout, generating final concentrations ranging from 1 μM with 5-fold serial dilution (totaling 9 concentration points).
5) After 1 h incubation, LPS or PHA (25 μL/well) was added to the cell wells to reach final concentrations of: 1 μg/mL LPS (for TNF-α, IL-10, IL-6, GM-CSF and IL-12p40), 5 μg/mL LPS (IL-1β), 50 μg/mL LPS (IFN-γ), or 1 μg/mL PHA (IL-2).
6) The plates were incubated in a incubator for 24 h at 37°C with 5% $CO_2$ under high humidity.

Cell plate layout:

| | 1 | 2 | 3 | 4 | 5 | 6 | 7 | 8 | 9 | 10 | 11 | 12 |
|---|---|---|---|---|---|---|---|---|---|---|---|---|
| A | $H_2O$ | $H_2O$ | $H_2O$ | $H_2O$ | $H_2O$ | $H_2O$ | $H_2O$ | $H_2O$ | $H_2O$ | $H_2O$ | $H_2O$ | $H_2O$ |
| B | $H_2O$ | Cmpd 1# (1 μM Starting, 5-fold dilution, 9 points) | | | | | | | | | LPS/PHA | $H_2O$ |
| C | $H_2O$ | | | | | | | | | | | $H_2O$ |
| D | $H_2O$ | Cmpd 2# (1 μM Starting, 5-fold dilution, 9 points) | | | | | | | | | | $H_2O$ |
| E | $H_2O$ | | | | | | | | | | | $H_2O$ |
| F | $H_2O$ | Cmpd 3# (1 μM Starting, 5-fold dilution, 9 points) | | | | | | | | | Blank | $H_2O$ |
| G | $H_2O$ | | | | | | | | | | | $H_2O$ |
| H | $H_2O$ | $H_2O$ | $H_2O$ | $H_2O$ | $H_2O$ | $H_2O$ | $H_2O$ | $H_2O$ | $H_2O$ | $H_2O$ | $H_2O$ | $H_2O$ |

VI.2.3 Detection

(1) The cell plate was centrifuged and the culture supernatant was aspirated. TNF-α, IL-10, IL-6, IL-1β, IFN-γ, GM-CSF, IL-2, and IL-12p40 cytokines in the culture supernatant were detected according to the ELISA kit instructions. The concentrations of TNF-$\alpha$, IL-10, IL-6, IL-1$\beta$, IFN-$\gamma$, GM-CSF, IL-2, and IL-12p40 in the samples were calculated based on the standard curve. Inhibition rate% = [(Ac-As)/(Ac-Ab)]$\times$100%

As: OA of the samples (cells + LPS/PHA + test compound)
Ac: OA of positive cell control (cells + LPS/PHA + DMSO)
Ab: OA of blank control (cells + culture medium + DMSO)

(2) $IC_{50}$ value was calculated as follows: using GraphPad Prism 6 software and applying the calculation formula XY-analysis/Nonlinear regression (curve fit)/Dose response-Inhibition/log (inhibitor) vs. response-Variable slope (four parameters) to perform $IC_{50}$ curve fitting and determine the $IC_{50}$ values.

The experimental results demonstrated that the compounds of the present disclosure can modulate the production levels of cytokines associated with autoimmune diseases and may be used for the treatment of autoimmune diseases.

**VII. Efficacy experiment of the compounds of the present disclosure on Psoriasis-like skin lesions induced by Imiquimod in Mice**

The compounds of the present disclosure (test compounds) were administered orally via gavage once daily for 7 consecutive days to mice with psoriasis-like skin lesions induced by imiquimod, to investigate their efficacy on such lesions.

A total of 60 female C57BL/6J mice (purchased from Shanghai SLAC Laboratory Animal Co., Ltd.) were randomly divided into 6 groups (n=10 per group) based on body weight:

| Group | Drug | Number of Animals | Dose (mg/kg) | Administration volume (mL/kg) | Administration concentration (mg/mL) | Route and Duration |
|---|---|---|---|---|---|---|
| 1 | Control: Vehicle | 10 | NA | 10 | NA | ig. qd. 7 days |
| 2 | Model: Vehicle | 10 | NA | 10 | NA | ig. qd. 7 days |
| 3 | Positive Control | 10 | 3 | 10 | 0.3 | sc. qd. 7 days |
| 4 | Low-dose test compounds group | 10 | 3 | 10 | 0.3 | ig. qd. 7 days |
| 5 | Medium-dose test compounds group | 10 | 10 | 10 | 1 | ig. qd. 7 days |
| 6 | High-dose test compounds group | 10 | 30 | 10 | 3 | ig. qd. 7 days |
| Note: ig., oral gavage; sc., subcutaneous injection; qd., once daily. | | | | | | |

After grouping the experimental animals, the backs of all mice were shaved. Except for the Control group, the remaining groups were topically treated with imiquimod cream once daily for 7 consecutive days. Simultaneously, each group was administered with a solvent control, a positive control drug, or the test compounds, according to the above table, once daily for 7 consecutive days.

All clinical symptoms of each animal were observed at the beginning and throughout the experiment. The animals' weights were measured and recorded every 2 days.

Scoring

Gross Observation Scoring of Skin Lesions (PASI Method): during the last 3 days of modeling and drug administration, the changes in skin lesions of mice in each group were observed once daily. PASI Method: the erythema, scaling, and thickening/infiltration of the lesions were scored separately, and the scores were summed to obtain the total

score. PASI Scoring Criteria: 0 = none; 1 = mild; 2 = moderate; 3 = severe; 4 = very severe.

The skin lesions were photographed once daily during the last 3 days of modeling and drug administration.

After the experiment, the mice were euthanized by $CO_2$ inhalation. Three different areas of skin from the lesion site on the back were collected using a 6 mm punch, weighed, and recorded, and the average weight was calculated.

The skin tissue from the lesion site was homogenized and the levels of IL-23p19, IL-12p40, IL-17, and TNF-$\alpha$ were determined.

The local lesion tissue was fixed, stained with HE, and subjected to pathological scoring (based on epidermal thickness, degree of epidermal keratinization, thickness of the basal layer, and degree of dermal inflammatory cell infiltration). Scoring Criteria: 0 = none; 1 = mild; 2 = moderate; 3 = severe; 4 = very severe.

Experimental data were presented as Mean $\pm$ SD. Statistical analysis between two groups was performed using SPSS, with $p < 0.05$ considered statistically significant.

The experimental results demonstrated that the compounds of the present disclosure improve psoriasis-like skin lesions on the back of mice induced by imiquimod and may be used for the treatment of psoriasis.

**VIII. Efficacy Experiment of the compounds of the present disclosure on the CIA Model in Rats Induced by Type II Collagen**

The compounds of the present disclosure (test compounds) were administered orally via gavage once daily for 21 consecutive days to Wistar rats with collagen-induced arthritis (CIA) that was induced using bovine type II collagen (Chondrex, Inc.), in order to investigate their efficacy in the rat arthritis model.

Preparation of the Modeling Agent:

10 mg of CII (Immunization Grade Bovine Type II Collagen, purchased from Chondrex, Inc.) was dissolved in 5 mL of 0.05M acetic acid solution, allowing it to be fully dissolved, thereby preparing a solution with a concentration of 2 mg/mL, which was then stored at 4°C in the dark overnight. On the day of the experiment, the 2 mg/mL CII solution was mixed with an equal volume of a 4 mg/mL solution of Complete Freund's Adjuvant (CFA, also purchased from Chondrex, Inc.) on ice, and was thoroughly emulsified into an emulsion.

On day 0, 10 Wistar rats (purchased from Beijing Vital River Laboratory Animal Technology Co., Ltd.) were randomly selected as the blank control group and were not immunized. The remaining 100 Wistar rats were intradermally injected at the base of the tail with an emulsion prepared by mixing equal volumes of CII (2 mg/mL) and CFA (4 mg/mL) for the first immunization. A second booster immunization was administered to them on day 7. Between days 10 and 14, i.e., 3 to 7 days after the second booster immunization, 50 rats with an arthritis index (AI) score ranging from 1 to 2 were selected from the modeled animals and were divided into 5 groups based on their body weight, foot volume, and AI score, with 10 rats per group:

| Group | Drug | Number of Animals | Dose (mg/kg) | Administration volume (mL/kg) | Administration concentration (mg/mL) | Route and Duration |
|---|---|---|---|---|---|---|
| 1 | Control: Vehicle | 10 | NA | 10 | NA | ig. qd. 21 days |
| 2 | Model: Vehicle | 10 | NA | 10 | NA | ig. qd. 21 days |
| 3 | Positive Control | 10 | 0.1 | 10 | 0.01 | sc. qd. 21 days |
| 4 | Low-dose test compounds group | 10 | 3 | 10 | 0.3 | ig. qd. 21 days |
| 5 | Medium-dose test compounds group | 10 | 10 | 10 | 1 | ig. qd. 21 days |
| 6 | High-dose test compounds group | 10 | 30 | 10 | 3 | ig. qd. 21 days |
| Note: ig. = oral gavage; sc. = subcutaneous injection | | | | | | |

Between 3 and 7 days after the second booster immunization, when the AI score of the affected feet reached 1 to

2, a solvent control, a positive control drug, or the test compounds were administered orally via gavage (or subcutaneously) to the animals in each group, according to the above table, once daily for 21 consecutive days.

Starting 3 days after the second booster immunization, the volume of both hind paws (or toes) was measured twice a week and recorded.

Foot Volume Measurement: Before the measurement, a line was marked on the rat's ankle joint with a marker pen to indicate the position. After clean water was added to the instrument, the instrument's reading was reset to zero in preparation for the measurement. The hind limb of the rat was placed in the water so that the marked line at the ankle joint was at the surface of the liquid. The reading obtained by pressing the foot pedal at this time was the foot volume of the rat. After the measurement was completed, the foot pedal was pressed again to reset the instrument to zero, preparing it for the measurement of the next rat.

Scoring

Arthritis Index (AI): The foot volume was measured twice a week, and the swelling of the four toes was scored simultaneously. Each foot was scored on a scale from 0 to 4, with a maximum score of 16 for each rat.

Arthritis Index (AI) Scoring Criteria:

| Score | Degree |
|---|---|
| 0 | No swelling, normal appearance |
| 1 | Mild swelling or redness of the ankle, wrist, or finger joints |
| 2 | Moderate swelling or redness of the ankle, wrist, or finger joints |
| 3 | Severe swelling or redness of the ankle, wrist, or finger joints |
| 4 | Very severe swelling or redness of the ankle, wrist, or finger joints |

The day of starting drug administration is recorded as Day 0. Five days after starting drug administration, i.e., 2 hours after Day 5 administration, blood was collected from the jugular vein of the experimental animals, allowed to stand for more than 30 minutes, centrifuged to separate the serum, which was stored at -80°C. The levels of TNF-$\alpha$, IL-1$\beta$, and IL-6 in serum were measured by ELISA.

After the experiment, the animals were euthanized by $CO_2$ inhalation. The spleen and thymus of the animals were collected and weighed, and the organ coefficients were calculated.

Pathological Examination: One side of the foot joint tissue was fixed in 10% neutral formalin solution, followed by decalcification and paraffin embedding to prepare pathological sections. The sections were then stained with hematoxylin and eosin (HE) for pathological observation.

Observation Indices:

1. Infiltration of synovial inflammatory cells (lymphocytes, plasma cells);
2. Synovial tissue hyperplasia;
3. Synovial pannus;
4. Fibrinoid necrosis on the synovial surface.

Scoring and Counting Method: 0, 1, 2, 3, and 4 levels, where "0" indicates no observation; "1" is mild; "2" is moderate; "3" is severe; and "4" is extremely severe.

Experimental data were presented as Mean $\pm$ SEM. Data between two groups were analyzed using Excel-T test, with $p<0.05$ considered statistically significant. Scoring data were analyzed using the non-parametric Mann-Whitney U test in SPSS, with $p<0.05$ considered statistically significant.

The experimental results demonstrated that the compounds of the present disclosure can regulate the serum levels of inflammatory cytokines in rats with collagen-induced arthritis (CIA) model that was induced using bovine type II collagen, and significantly improve the swelling of rat limbs, and thus may be used for the treatment of arthritis.

The above descriptions represent only preferred embodiments of the present invention, but the scope of

protection is not limited thereto. Any person skilled in the art, within the technical scope disclosed by the invention, may make equivalent replacements or modifications based on the technical solutions and inventive concepts of the present invention, and such changes shall fall within the protection scope of the invention.

## Claims

**1.** A compound of Formula (I)

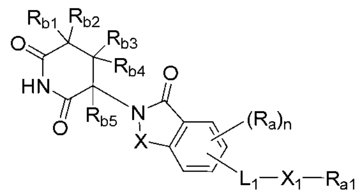

Formula (I)

or salts, enantiomers, stereoisomers, solvates, or polymorphs thereof,

wherein $R_{b1}$, $R_{b2}$, $R_{b3}$, $R_{b4}$, and $R_{b5}$ each independently represent H, D or $C_{1-3}$ alkyl;
X represents C(O) or $CH_2$;
$(R_a)_n$ indicates that the benzene ring in Formula (I) is optionally substituted with n $R_a$, where $R_a$ represents deuterium, halogen, hydroxy, mercapto, nitro, amino, cyano, optionally deuterated $C_{1-6}$ alkyl, optionally deuterated $C_{1-6}$ alkoxy, halogenated $C_{1-6}$ alkoxy or halogenated $C_{1-6}$ alkyl, and n represents an integer of 0, 1, 2 or 3; and
$L_1$ represents C(O), alkynylene, alkenylene, optionally substituted $C_{1-5}$ alkylene, -CH= or $N(R_{c1})$, where $R_{c1}$ represents H or $C_{1-3}$ alkyl; or
$L_1$ represents Formula $-L_2-L_3-*$, where $L_2$ represents optionally substituted phenylene, $L_3$ represents optionally substituted $C_{1-2}$ alkylene, and symbol * indicates the point of attachment to Xi; or
$L_1$ represents a bond;
$X_1$ represents optionally substituted cycloalkylene, optionally substituted heterocyclylene, optionally substituted heteroarylene, or $N(R_{c2})$, where $R_{c2}$ represents H or $C_{1-3}$ alkyl; or
$X_1$ represents a bond;
$R_{a1}$ represents the structure of Formula (II):

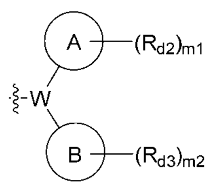

Formula (II)

wherein W represents N or $CR_{d1}$, where $R_{d1}$ represents H, halogen, $C_{1-3}$ alkyl or halogenated $C_{1-3}$ alkyl;

ring A represents aryl, cycloalkyl, heterocyclyl or heteroaryl, and $(R_{d2})_{m1}$ indicates that ring A is optionally substituted with m1 $R_{d2}$, where m1 represents an integer of 0 to 10, and each $R_{d2}$ independently represents deuterium, hydroxy, amino, mercapto, halogen, cyano, optionally deuterated $C_{1-6}$ alkyl, optionally deuterated $C_{3-6}$ cycloalkyl, optionally deuterated $C_{1-6}$ alkoxy, halogenated $C_{1-6}$ alkyl, halogenated $C_{1-6}$ alkoxy or $-C_{1-6}$ alkylene-$NH_2$; and
ring B represents aryl, cycloalkyl, heterocyclyl or heteroaryl, and $(R_{d3})_{m2}$ indicates that ring B is optionally substituted with m2 $R_{d3}$, where m2 represents an integer of 0 to 10, and each $R_{d3}$ independently represents deuterium, hydroxy, amino, mercapto, halogen, cyano, optionally deuterated $C_{1-6}$ alkyl, optionally deuterated $C_{3-6}$ cycloalkyl, optionally deuterated $C_{1-6}$ alkoxy, halogenated $C_{1-6}$ alkyl, halogenated $C_{1-6}$ alkoxy or $-C_{1-6}$ alkylene-$NH_2$; or

$R_{a1}$ represents the structure of Formula (III):

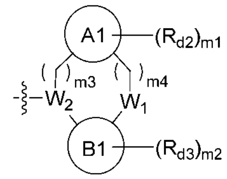

Formula (III)

wherein m3 and m4 each independently represent an integer of 0, 1 or 2, $W_1$ represents O, S or NH, or $W_1$ represents a bond;

$W_2$ represents N or $CR_{d1}$, where $R_{d1}$ represents H, halogen, $C_{1-3}$ alkyl or halogenated $C_{1-3}$ alkyl;

ring A1 represents an aryl ring, a cycloalkyl ring, a heterocyclyl ring or a heteroaryl ring, and $(R_{d2})_{m1}$ indicates that ring A is optionally substituted with m1 $R_{d2}$, where m1 represents an integer of 0 to 10, and each $R_{d2}$ independently represents deuterium, hydroxy, amino, mercapto, halogen, cyano, optionally deuterated $C_{1-6}$ alkyl, optionally deuterated $C_{3-6}$ cycloalkyl, optionally deuterated $C_{1-6}$ alkoxy, halogenated $C_{1-6}$ alkyl, halogenated $C_{1-6}$ alkoxy or -$C_{1-6}$ alkylene-$NH_2$; and

ring B1 represents an aryl ring, a cycloalkyl ring, a heterocyclyl ring or a heteroaryl ring, and $(R_{d3})_{m2}$ indicates that ring B is optionally substituted with m2 $R_{d3}$, where m2 represents an integer of 0 to 10, and each $R_{d3}$ independently represents deuterium, hydroxy, amino, mercapto, halogen, cyano, optionally deuterated $C_{1-6}$ alkyl, optionally deuterated $C_{3-6}$ cycloalkyl, optionally deuterated $C_{1-6}$ alkoxy, halogenated $C_{1-6}$ alkyl, halogenated $C_{1-6}$ alkoxy or -$C_{1-6}$ alkylene-$NH_2$; or

$R_{a1}$ represents the structure of Formula (IV):

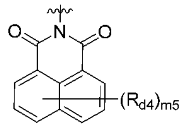

Formula (IV) ;

wherein $(R_{d4})_{m5}$ indicates that the structure of Formula (IV) is optionally substituted with m5 $R_{d4}$, where m5 represents an integer of 0 to 6, and each $R_{d4}$ independently represents hydroxy, amino, mercapto, halogen, cyano, optionally deuterated $C_{1-6}$ alkyl, optionally deuterated $C_{3-6}$ cycloalkyl, optionally deuterated $C_{1-6}$ alkoxy, halogenated $C_{1-6}$ alkyl, halogenated $C_{1-6}$ alkoxy or -$C_{1-6}$ alkylene-$NH_2$;

with the provisos that:

$L_1$ and $X_1$ are not simultaneously bonds; and

when W represents N and $X_1$ represents $N(R_{c2})$, $L_1$ is not $N(R_{c1})$.

2. The compound of Formula (I) or salts, enantiomers, stereoisomers, solvates, or polymorphs thereof as claimed in claim 1, wherein the compound of Formula (I) is also of Formula (I-1) or Formula (I-2):

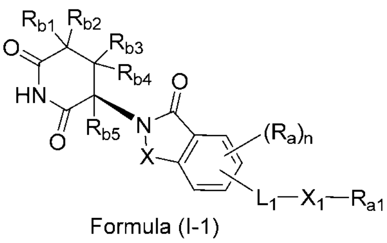

Formula (I-1)

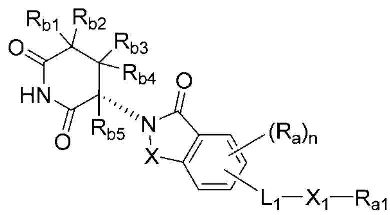

Formula (I-2)

wherein $R_{b1}$, $R_{b2}$, $R_{b3}$, $R_{b4}$, $R_{b5}$, $(R_a)_n$, X, $L_1$, $X_1$, and $R_{a1}$ are as defined in claim 1.

3. The compound of Formula (I) or salts, enantiomers, stereoisomers, solvates, or polymorphs thereof as claimed in claim 1 or 2, wherein

(i) $R_{b1}$, $R_{b2}$, $R_{b3}$ and $R_{b4}$ each independently represent H, $R_{b5}$ represents H or D; and/or
(ii) X represents C(O); or
X represents $CH_2$; and/or
(iii) $L_1$ represents C(O), $C_{2-6}$ alkynylene (e.g., $C_{2-4}$ alkynylene), $C_{2-6}$ alkenylene (e.g., $C_{2-4}$ alkenylene), optionally substituted $C_{1-5}$ alkylene (e.g., optionally substituted $C_{1-3}$ alkylene), -CH= or $N(R_{c1})$, where $R_{c1}$ represents H or $C_{1-3}$ alkyl; or

$L_1$ represents Formula $-L_2-L_3-*$, wherein $L_2$ represents optionally substituted phenylene, $L_3$ represents optionally substituted $C_{1-2}$ alkylene (e.g., optionally substituted methylene), and symbol * indicates the point of attachment to Xi; or
$L_1$ represents a bond; and/or

(iv) $X_1$ represents optionally substituted $C_{3-30}$ cycloalkylene (e.g., optionally substituted $C_{3-20}$ cycloalkylene), optionally substituted 4- to 30-membered heterocyclylene (e.g., optionally substituted 4- to 20-membered heterocyclylene), optionally substituted 5- to 30-membered heteroarylene (e.g., optionally substituted 5- to 20-membered heteroarylene), or $N(R_{c2})$, where $R_{c2}$ represents H or $C_{1-3}$ alkyl; or

$X_1$ represents a bond.

**4.** The compound of Formula (I) or salts, enantiomers, stereoisomers, solvates, or polymorphs thereof as claimed in claim 1 or 2, wherein

(i) $L_1$ represents C(O), ethynylene, vinylene, methylene, ethylene, propylene, -CH(OH)-, -CH= or NH; or

$L_1$ represents -phenylene-methylene-*, where symbol * indicates the point of attachment to $X_1$; or
$L_1$ represents a bond; and/or

(ii) $X_1$ represents the following bivalent groups:

cyclopropylene, cyclobutylene, cyclopentylene, cyclopentenylene, cyclohexylene, cyclohexenylene, cycloheptylene, cyclooctylene, decalinylene, octahydropentalenylene, octahydro-1H-indenylene, spiro-cycloalkylene (e.g., $C_5$-$C_{15}$ spiro-cycloalkylene, such as spiro[3.3]heptylene, spiro[2.5]octylene, spiro[3.5]nonylene, spiro[4.4]nonylene, spiro[4.5]decylene, and spiro[5.5]undecylene), p-menthanylene, m-menthanylene, or bridged cycloalkylene (e.g., $C_6$-$C_{15}$ bridged cycloalkylene, such as adamantanylene, noradamantanylene, bornylene, bicyclo[2.2.1]heptanylene, 2-oxobicyclo[2.2.1]heptanylene, or bicyclo[2.2.1]heptentylene), with each group being optionally substituted with one or more substituents selected from the group consisting of D, optionally deuterated $C_{1-4}$ alkyl, hydroxy, amino, mercapto, halogen, cyano, oxo, $C_{1-4}$ alkoxy, $C_{1-4}$ alkyl-NH-, halogenated $C_{1-4}$ alkyl, $NH_2$-$C_{1-4}$ alkylene-, $C_{1-4}$ alkyl-NHC(O)-, $C_{1-4}$ alkyl-C(O)NH- and any combination thereof; or
azetidinylene, pyrrolidinylene, imidazolidylene, pyrazolidylene, oxazolidinylene, thiazolidinylene, piperidinylene, piperazinylene, tetrahydropyridylene, dihydroxypiperidinylene, difluoropiperidinylene, morpholinylene, thiomorpholinylene, azacycloheptanylene, azacyclooctylene, diazacycloheptanylene, diazacyclooctylene, bridged heterocyclylene (e.g., 6- to 15-membered bridged heterocyclylene, such as 3-azabicyclo[3.1.0]hexylene, 3-azabicyclo[3.1.1]heptanylene, 2-azabicyclo[2.2.1]heptanylene, 6-azabicyclo[3.1.1]heptanylene, 2-azabicyclo[2.2.2]octanylene, 2,5-diazabicyclo[2.2.1]heptanylene, 3,6-diazabicyclo[3.1.1]heptanylene, 3-azabicyclo[3.2.1]octanylene, 3,8-diazabicyclo[3.2.1]octanylene, 2,5-diazabicyclo[2.2.2]octanylene, and quinuclidinylene) or spiro-heterocyclylene (e.g., 5- to 15-membered spiro-heterocyclylene, such as 2,6-diazaspiro[3.3]heptanylene, 2,7-diazaspiro[3.5]nonylene, 2,8-diazaspiro[4.5]decylene, 3,9-diazaspiro[5.5]undecylene, 3-azaspiro[5.5]undecylene, and 7-azaspiro[3.5]nonylene) or octahydropyrrolo[3,4-c]pyrrolylene, with each group being optionally substituted with one or more substituents selected from the group consisting of D, optionally deuterated $C_{1-4}$ alkyl, hydroxy, amino, mercapto, halogen, cyano, oxo, $C_{1-4}$ alkoxy, $C_{1-4}$ alkyl-NH-, halogenated $C_{1-4}$ alkyl, $NH_2$-$C_{1-4}$ alkylene-, $C_{1-4}$ alkyl-NHC(O)-, $C_{1-4}$ alkyl-C(O)NH- and any combination thereof; or
furanylene, oxazolylene, isoxazolylene, oxadiazolylene, thienylene, thiazolylene, isothiazolylene, thiadiazolylene, pyrrolylene, imidazolylene, pyrazolylene, triazolylene, pyridylene, pyrimidinylene, pyridazinylene, pyrazinylene, indolylene, isoindolylene, benzofuranylene, isobenzofuranylene, benzothienylene, indazolylene, benzimidazolylene, benzoxazolylene, benzisoxazolylene, benzothiazolylene, benzisothiazolylene,

benzotriazolylene, benzo[2,1,3]oxadiazolylene, benzo[2,1,3]thiadiazolylene, benzo[1,2,3]thiadiazolylene, quinolinylene, isoquinolinylene, naphthyridinylene, cinnolinylene, quinazolinylene, quinoxalinylene, phthalazinylene, pyrazolo[1,5-a]pyridylene, pyrazolo[1,5-a]pyrimidinylene, imidazo[1,2-a]pyridylene, 1H-pyrrolo[3,2-b]pyridylene, 1H-pyrrolo[2,3-b]pyridylene, 4H-fluoro[3,2-b]pyrrolylene, pyrrolo[2,1-b]thiazolylene, or imidazo[2,1-b]thiazolylene, with each group being optionally substituted with one or more substituents selected from the group consisting of D, optionally deuterated $C_{1-4}$ alkyl, hydroxy, amino, mercapto, halogen, cyano, $C_{1-4}$ alkoxy, $C_{1-4}$ alkyl-NH-, halogenated $C_{1-4}$ alkyl, $NH_2$-$C_{1-4}$ alkylene-, $C_{1-4}$ alkyl-NHC(O)-, $C_{1-4}$ alkyl-C(O)NH- and any combination thereof; or

$X_1$ represents NH or $N(CH_3)$; or
$X_1$ represents a bond.

**5.** The compound of Formula (I) or salts, enantiomers, stereoisomers, solvates, or polymorphs thereof as claimed in claim 1 or 4, wherein $X_1$ represents the following bivalent groups:

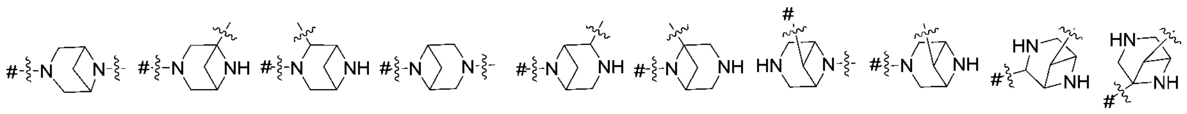

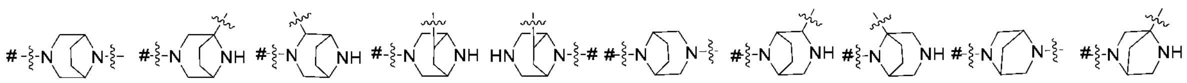

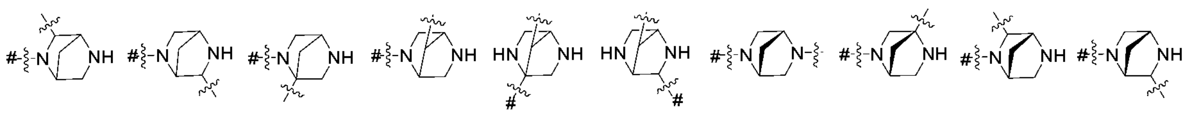

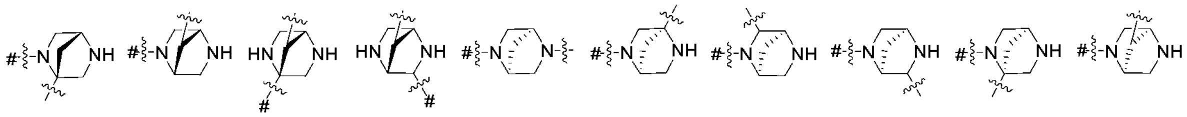

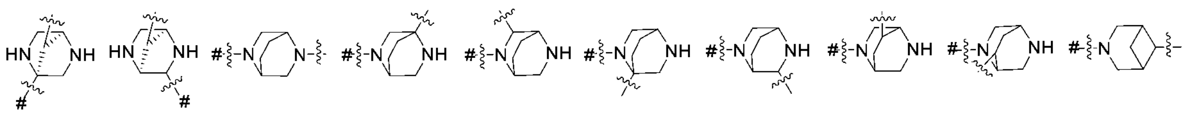

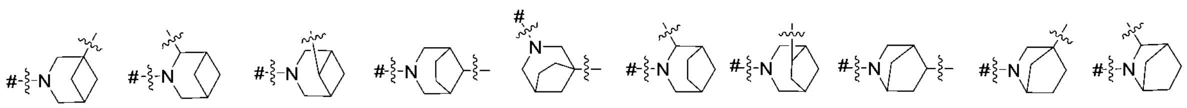

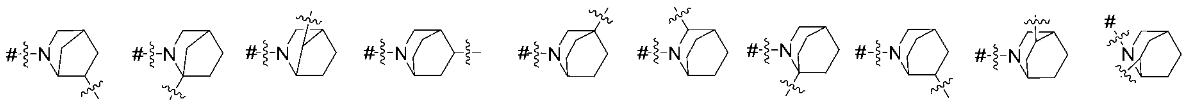

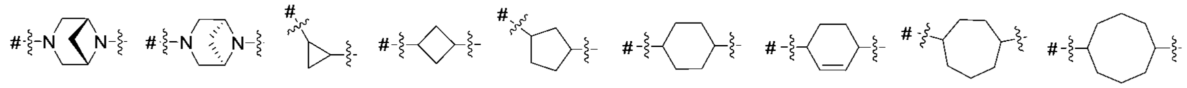

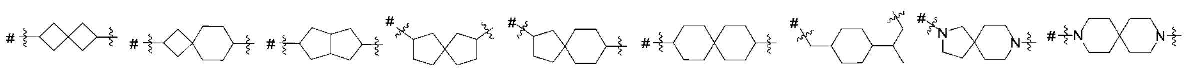

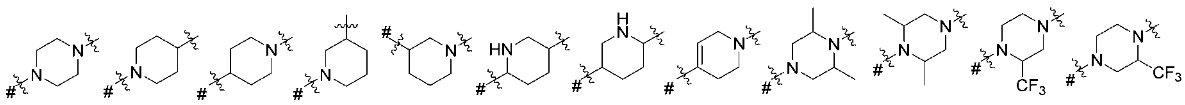

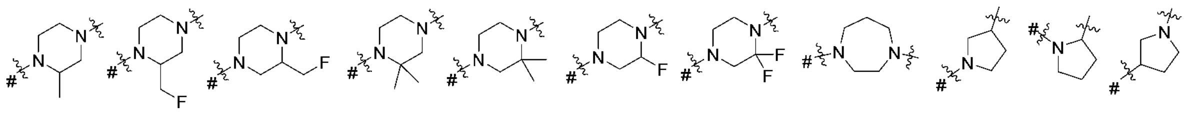

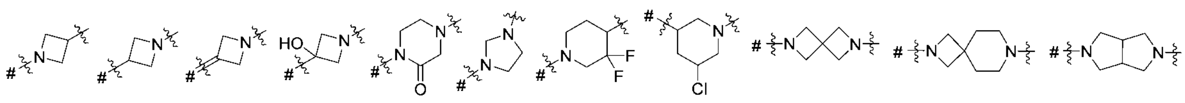

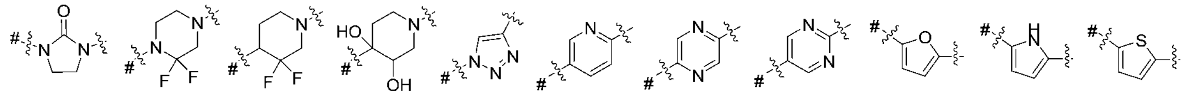

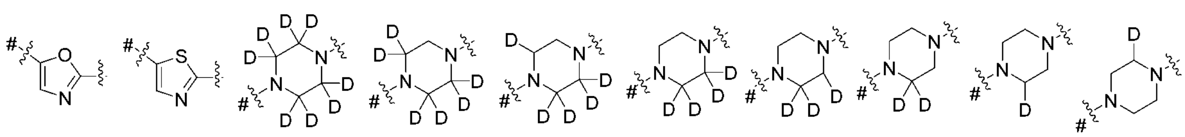

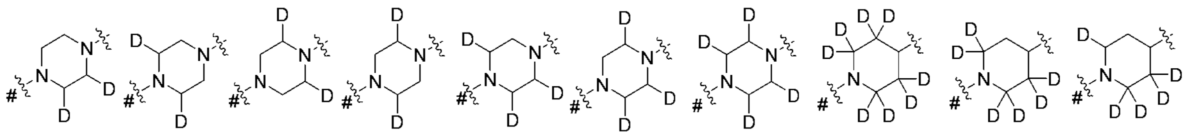

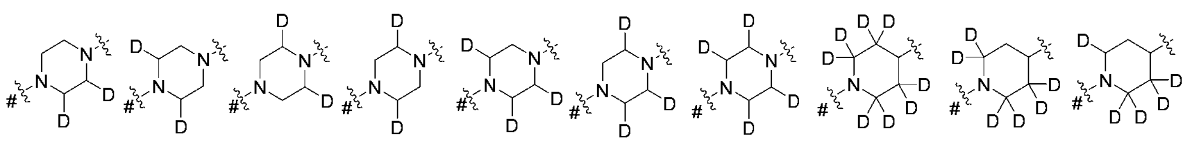

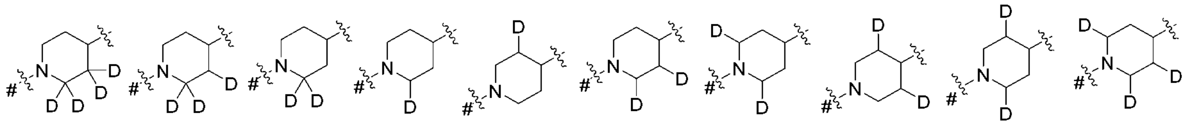

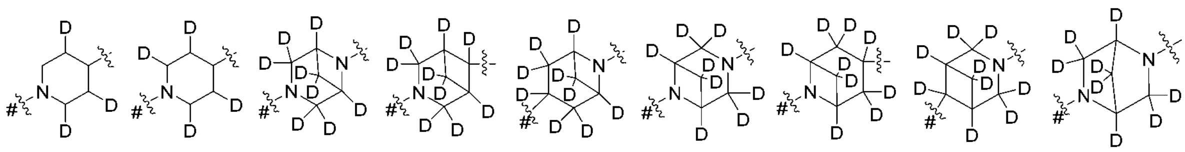

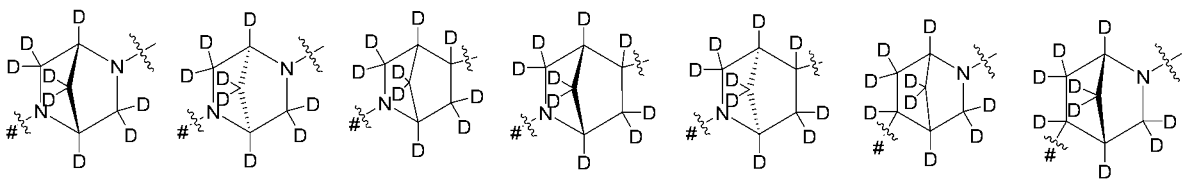

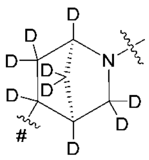 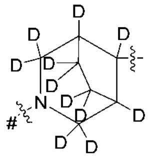 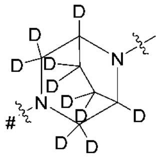 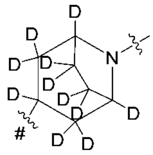 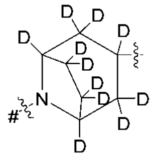 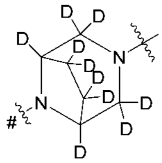 或 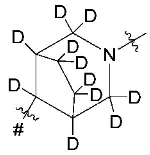 ,

wherein symbol # indicates the point of attachment to $L_1$.

**6.** The compound of Formula (I) or salts, enantiomers, stereoisomers, solvates, or polymorphs thereof as claimed in any one of claims 1-5, wherein

(i) $R_{a1}$ represents the structure of Formula (II):

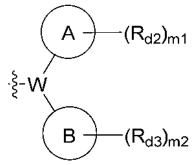

Formula (II)

wherein W represents N or $CR_{d1}$, where $R_{d1}$ represents H, halogen, $C_{1-3}$ alkyl or halogenated $C_{1-3}$ alkyl;

ring A represents $C_{5-30}$ aryl (e.g., $C_{5-20}$ aryl, or $C_{5-15}$ aryl), $C_{3-30}$ cycloalkyl (e.g., $C_{3-20}$ cycloalkyl, or $C_{3-15}$ cycloalkyl), 4- to 30-membered heterocyclyl (e.g., 4- to 20-membered heterocyclyl, or 4- to 15-membered heterocyclyl) or 5- to 30-membered heteroaryl (e.g., 5- to 20-membered heteroaryl, or 5- to 15-membered heteroaryl), and $(R_{d2})_{m1}$ indicates that ring A is optionally substituted with m1 $R_{d2}$, where m1 represents an integer of 0 to 10, and each $R_{d2}$ independently represents deuterium, hydroxy, amino, mercapto, halogen, cyano, optionally deuterated $C_{1-6}$ alkyl, optionally deuterated $C_{3-6}$ cycloalkyl, optionally deuterated $C_{1-6}$ alkoxy, halogenated $C_{1-6}$ alkyl, halogenated $C_{1-6}$ alkoxy or $-C_{1-6}$ alkylene-$NH_2$; and

ring B represents $C_{5-30}$ aryl (e.g., $C_{5-20}$ aryl, or $C_{5-15}$ aryl), $C_{3-30}$ cycloalkyl (e.g., $C_{3-20}$ cycloalkyl, or $C_{3-15}$ cycloalkyl), 4- to 30-membered heterocyclyl (e.g., 4- to 20-membered heterocyclyl, or 4- to 15-membered heterocyclyl) or 5- to 30-membered heteroaryl (e.g., 5- to 20-membered heteroaryl, or 5- to 15-membered heteroaryl), and $(R_{d3})_{m2}$ indicates that ring B is optionally substituted with m2 $R_{d3}$, where m2 represents an integer of 0 to 10, and each $R_{d3}$ independently represents deuterium, hydroxy, amino, mercapto, halogen, cyano, optionally deuterated $C_{1-6}$ alkyl, optionally deuterated $C_{3-6}$ cycloalkyl, optionally deuterated $C_{1-6}$ alkoxy, halogenated $C_{1-6}$ alkyl, halogenated $C_{1-6}$ alkoxy or $-C_{1-6}$ alkylene-$NH_2$; or

(ii) $R_{a1}$ represents the structure of Formula (III):

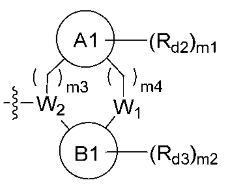

Formula (III)

wherein m3 and m4 each independently represent an integer of 0, 1 or 2, $W_1$ represents O, S or NH, or $W_1$ represents a bond; preferably, m3 and m4 each independently represent an integer of 0 or 1, $W_1$ represents O, S or NH, or $W_1$ represents a bond;

$W_2$ represents N or $CR_{d1}$, where $R_{d1}$ represents H, halogen, $C_{1-3}$ alkyl or halogenated $C_{1-3}$ alkyl;

ring A1 represents $C_{5-30}$ aryl ring (e.g., $C_{5-20}$ aryl ring, or $C_{5-15}$ aryl ring), $C_{3-30}$ cycloalkyl ring (e.g., $C_{3-20}$ cycloalkyl ring, or $C_{3-15}$ cycloalkyl ring), 4- to 30-membered heterocyclyl ring (e.g., 4-to 20-membered heterocyclyl ring, or 4- to 15-membered heterocyclyl ring) or 5- to 30-membered heteroaryl ring (e.g., 5- to 20-membered heteroaryl ring, or 5- to 15-membered heteroaryl ring), and $(R_{d2})_{m1}$ indicates that ring A1 is optionally substituted with m1 $R_{d2}$, where m1 represents an integer of 0 to 10, and each $R_{d2}$ independently

represents deuterium, hydroxy, amino, mercapto, halogen, cyano, optionally deuterated $C_{1-6}$ alkyl, optionally deuterated $C_{3-6}$ cycloalkyl, optionally deuterated $C_{1-6}$ alkoxy, halogenated $C_{1-6}$ alkyl, halogenated $C_{1-6}$ alkoxy or -$C_{1-6}$ alkylene-$NH_2$; and

ring B1 represents $C_{5-30}$ aryl ring (e.g., $C_{5-20}$ aryl ring, or $C_{5-15}$ aryl ring), $C_{3-30}$ cycloalkyl ring (e.g., $C_{3-20}$ cycloalkyl ring, or $C_{3-15}$ cycloalkyl ring), 4- to 30-membered heterocyclyl ring (e.g., 4-to 20-membered heterocyclyl ring, or 4- to 15-membered heterocyclyl ring) or 5- to 30-membered heteroaryl ring (e.g., 5- to 20-membered heteroaryl ring, or 5- to 15-membered heteroaryl ring), and $(R_{d3})_{m2}$ indicates that ring B1 is optionally substituted with m2 $R_{d3}$, where m2 represents an integer of 0 to 10, and each $R_{d3}$ independently represents deuterium, hydroxy, amino, mercapto, halogen, cyano, optionally deuterated $C_{1-6}$ alkyl, optionally deuterated $C_{3-6}$ cycloalkyl, optionally deuterated $C_{1-6}$ alkoxy, halogenated $C_{1-6}$ alkyl, halogenated $C_{1-6}$ alkoxy or -$C_{1-6}$ alkylene-$NH_2$; or

(iii) $R_{a1}$ represents the structure of Formula (IV):

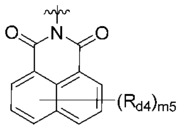

Formula (IV)

wherein $(R_{d4})_{m5}$ indicates that the structure of Formula (IV) is optionally substituted with m5 $R_{d4}$, where m5 represents an integer of 0 to 6 (e.g., an integer of 0 to 3), and each $R_{d4}$ independently represents hydroxy, amino, mercapto, halogen, cyano, optionally deuterated $C_{1-6}$ alkyl(e.g., $C_{1-6}$ alkyl), optionally deuterated $C_{3-6}$ cycloalkyl (e.g., $C_{3-6}$ cycloalkyl), optionally deuterated $C_{1-6}$ alkoxy (e.g., $C_{1-6}$ alkoxy), halogenated $C_{1-6}$ alkyl, halogenated $C_{1-6}$ alkoxy or -$C_{1-6}$ alkylene-$NH_2$.

**7.** The compound of Formula (I) or salts, enantiomers, stereoisomers, solvates, or polymorphs thereof as claimed in claim 1 or 6, wherein

(i) the ring A and ring B independently represent one of the following groups:

phenyl or naphthyl; or

cyclopropyl, cyclobutyl, cyclopentyl, cyclopentenyl, cyclohexyl, cyclohexenyl, cycloheptyl, cyclooctyl, decalinyl, octahydropentalenyl, octahydro-1H-indenyl, spiro-cycloalkyl (e.g., $C_5$-$C_{15}$ spiro-cycloalkyl, such as spiro[3.3]heptanyl, spiro[2.5]octyl, spiro[3.5]nonyl, spiro[4.4]nonyl, spiro[4.5]decyl, and spiro[5.5]undecyl), p-menthanyl, m-menthanyl, or bridged cycloalkyl (e.g., $C_6$-$C_{15}$ bridged cycloalkyl, such as adamantanyl, noradamantanyl, bornyl, bicyclo[2.2.1]heptanyl, 2-oxobicyclo[2.2.1]heptanyl, or bicyclo[2.2.1heptentyl); or

azetidinyl, pyrrolidinyl, imidazolidinyl, pyrazolidyl, oxazolidinyl, thiazolidinyl, piperidinyl, piperazinyl, tetrahydropyridyl, dihydroxypiperidinyl, difluoropiperidinyl, morpholinyl, thiomorpholinyl, azacycloheptanyl, azacyclooctyl, diazacycloheptanyl, diazacyclooctyl, bridged heterocyclyl (e.g., 6- to 15-membered bridged heterocyclyl, such as 3-azabicyclo[3.1.0]hexyl, 3-azabicyclo[3.1.1]heptanyl, 2-azabicyclo[2.2.1]heptanyl, 6-azabicyclo[3.1.1]heptanyl, 2-azabicyclo[2.2.2]octanyl, 2,5-diazabicyclo[2.2.1]heptanyl, 3,6-diazabicyclo [3.1.1]heptanyl, 3-azabicyclo[3.2.1]octanyl, 3,8-diazabicyclo[3.2.1]octanyl, 2,5-diazabicyclo[2.2.2]octanyl, and quinuclidinyl) or spiro-heterocyclyl (e.g., 5- to 15-membered spiro-heterocyclyl, such as 2,6-diazaspiro [3.3]heptanyl, 2,7-diazaspiro[3.5]nonyl, 2,8-diazaspiro[4.5]decyl, 3,9-diazaspiro[5.5]undecyl, 3-azaspiro [5.5]undecyl, and 7-azaspiro[3.5]nonyl) or octahydropyrrolo[3,4-c]pyrrolyl; or

furanyl, oxazolyl, isoxazolyl, oxadiazolyl, thienyl, thiazolyl, isothiazolyl, thiadiazolyl, pyrrolyl, imidazolyl, pyrazolyl, triazolyl, pyridyl, pyrimidinyl, pyridazinyl, pyrazinyl, indolyl, isoindolyl, benzofuranyl, isobenzofuranyl, benzothienyl, indazolyl, benzimidazolyl, benzoxazolyl, benzisoxazolyl, benzothiazolyl, benzisothiazolyl, benzotriazolyl, benzo[2,1,3]oxadiazolyl, benzo[2,1,3]thiadiazolyl, benzo[1,2,3]thiadiazolyl, quinolinyl, isoquinolinyl, naphthyridinyl, cinnolinyl, quinazolinyl, quinoxalinyl, phthalazinyl, pyrazolo[1,5-a]pyridyl, pyrazolo[1,5-a]pyrimidinyl, imidazo[1,2-a]pyridyl, 1H-pyrrolo[3,2-b]pyridyl, 1H-pyrrolo[2,3-b]pyridyl, 4H-fluoro [3,2-b]pyrrolyl, pyrrolo[2,1-b]thiazolyl, or imidazo[2,1-b]thiazolyl;

wherein the ring A and ring B are each independently optionally substituted with 1 to 10 substituents selected from the group consisting of deuterium, hydroxy, amino, mercapto, halogen, cyano, optionally deuterated $C_{1-6}$ alkyl, optionally deuterated $C_{3-6}$ cycloalkyl, optionally deuterated $C_{1-6}$ alkoxy, halogenated $C_{1-6}$ alkyl, halogenated $C_{1-6}$ alkoxy, -$C_{1-6}$ alkylene-$NH_2$ or any combination thereof; or

(ii) $R_{a1}$ represents the structure of Formula (III):

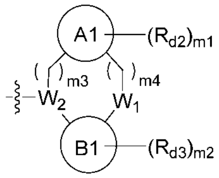

Formula (III)

wherein m3 and m4 each independently represent an integer of 0, 1 or 2, $W_1$ represents O, S or NH, or $W_1$ represents a bond; preferably, m3 and m4 each independently represent an integer of 0 or 1, $W_1$ represents NH, or $W_1$ represents a bond;
$W_2$ represents N or $CR_{d1}$, where $R_{d1}$ represents H, halogen, $C_{1-3}$ alkyl or halogenated $C_{1-3}$ alkyl;
ring A1 and ring B1 each independently represent one of the following groups:

phenyl ring or naphthyl ring; or
cyclopropyl ring, cyclobutyl ring, cyclopentyl ring, cyclopentenyl ring, cyclohexyl ring, cyclohexenyl ring, cycloheptyl ring, cyclooctyl ring, decalinyl ring, octahydropentalenyl ring, octahydro-1H-indenyl ring, spiro-cycloalkyl ring (e.g., $C_5$-$C_{15}$ spiro-cycloalkyl ring, such as spiro[3.3]heptanyl ring, spiro[2.5]octyl ring, spiro[3.5]nonyl ring, spiro[4.4]nonyl ring, spiro[4.5]decyl ring, and spiro[5.5]undecyl ring), p-menthanyl ring, m-menthanyl ring, or bridged cycloalkyl ring (e.g., $C_5$-$C_{15}$ bridged cycloalkyl ring, such as adamantanyl ring, noradamantanyl ring, bornyl ring, bicyclo[2.2.1]heptanyl ring, 2-oxobicyclo[2.2.1]heptanyl ring, or bicyclo[2.2.1]heptentyl ring); or
azetidinyl ring, pyrrolidinyl ring, imidazolidinyl ring, pyrazolidyl ring, oxazolidinyl ring, thiazolidinyl ring, piperidinyl ring, piperazinyl ring, tetrahydropyridyl ring, dihydroxypiperidinyl ring, difluoropiperidinyl ring, morpholinyl ring, thiomorpholinyl ring, azacycloheptanyl ring, azacyclooctyl ring, diazacycloheptanyl ring, diazacyclooctyl ring, bridged heterocyclyl ring (e.g., 6-to 15-membered bridged heterocyclyl ring, such as 3-azabicyclo[3.1.0]hexyl ring, 3-azabicyclo[3.1.1]heptanyl ring, 2-azabicyclo[2.2.1]heptanyl ring, 6-azabicyclo[3.1.1]heptanyl ring, 2-azabicyclo[2.2.2]octanyl ring, 2,5-diazabicyclo[2.2.1]heptanyl ring, 3,6-diazabicyclo[3.1.1]heptanyl ring, 3-azabicyclo[3.2.1]octanyl ring, 3,8-diazabicyclo[3.2.1]octanyl ring, 2,5-diazabicyclo[2.2.2]octanyl ring, or quinuclidinyl ring), spiro-heterocyclyl ring (e.g., 5- to 15-membered spiro-heterocyclyl ring, such as 2,6-diazaspiro[3.3]heptanyl ring, 2,7-diazaspiro[3.5]nonyl ring, 2,8-diazaspiro[4.5]decyl ring, 3,9-diazaspiro[5.5]undecyl ring, 3-azaspiro[5.5]undecyl ring, or 7-azaspiro[3.5]nonyl ring) or octahydropyrrolo[3,4-c]pyrrolyl ring; or
furanyl ring, oxazolyl ring, isoxazolyl ring, oxadiazolyl ring, thienyl ring, thiazolyl ring, isothiazolyl ring, thiadiazolyl ring, pyrrolyl ring, imidazolyl ring, pyrazolyl ring, triazolyl ring, pyridyl ring, pyrimidinyl ring, pyridazinyl ring, pyrazinyl ring, indolyl ring, isoindolyl ring, benzofuranyl ring, isobenzofuranyl ring, benzothienyl ring, indazolyl ring, benzimidazolyl ring, benzoxazolyl ring, benzisoxazolyl ring, benzothiazolyl ring, benzisothiazolyl ring, benzotriazolyl ring, benzo[2,1,3]oxadiazolyl ring, benzo[2,1,3]thiadiazolyl ring, benzo[1,2,3]thiadiazolyl ring, quinolinyl ring, isoquinolinyl ring, naphthyridinyl ring, cinnolinyl ring, quinazolinyl ring, quinoxalinyl ring, phthalazinyl ring, pyrazolo[1,5-a]pyridyl ring, pyrazolo[1,5-a]pyrimidinyl ring, imidazo[1,2-a]pyridyl ring, 1H-pyrrolo[3,2-b]pyridyl ring, 1H-pyrrolo[2,3-b]pyridyl ring, pyrrolo[2,1-b]thiazolyl ring, or imidazo[2,1-b]thiazolyl ring; or

wherein the ring A1 and ring B1 are each independently optionally substituted with 1 to 10 substituents selected from the group consisting of deuterium, hydroxy, amino, mercapto, halogen, cyano, optionally deuterated $C_{1-6}$ alkyl, optionally deuterated $C_{3-6}$ cycloalkyl, optionally deuterated $C_{1-6}$ alkoxy, halogenated $C_{1-6}$ alkyl, halogenated $C_{1-6}$ alkoxy, $-C_{1-6}$ alkylene-$NH_2$ or any combination thereof.

**8.** The compound of Formula (I) or salts, enantiomers, stereoisomers, solvates, or polymorphs thereof as claimed in claim 1 or 6, wherein $R_{a1}$ represents the following groups:

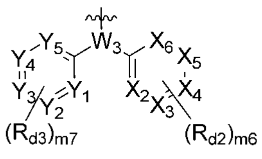 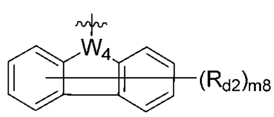 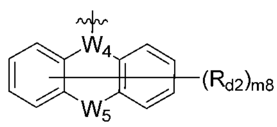 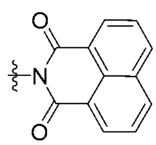

wherein $W_3$ and $W_4$ independently represent CH or N, and $W_5$ represents O, S, NH or $CH_2$;
any one or two of $X_2$, $X_3$, $X_4$, $X_5$, and $X_6$ represent CH or N, and the remaining ones represent CH;
any one or two of $Y_1$, $Y_2$, $Y_3$, $Y_4$, and $Y_5$ represent CH or N, and the remaining ones represent CH;
$(R_{d2})_{m6}$ represents m6 substituents $R_{d2}$, where m6 represents an integer of 0 to 5, and each $R_{d2}$ independently represents hydroxy, amino, mercapto, halogen, cyano, optionally deuterated $C_{1-6}$ alkyl, optionally deuterated $C_{3-6}$ cycloalkyl, optionally deuterated $C_{1-6}$ alkoxy, halogenated $C_{1-6}$ alkyl, halogenated $C_{1-6}$ alkoxy or -$C_{1-6}$ alkylene-$NH_2$;
$(R_{d3})_{m7}$ represents m7 substituents $R_{d3}$, where m7 represents an integer of 0 to 5, and each $R_{d3}$ independently represents hydroxy, amino, mercapto, halogen, cyano, optionally deuterated $C_{1-6}$ alkyl, optionally deuterated $C_{3-6}$ cycloalkyl, optionally deuterated $C_{1-6}$ alkoxy, halogenated $C_{1-6}$ alkyl, halogenated $C_{1-6}$ alkoxy or -$C_{1-6}$ alkylene-$NH_2$; and
each $(R_{d2})_{m8}$ independently represents m8 substituents $R_{d2}$, where m8 represents an integer of 0 to 8, and each $R_{d2}$ independently represents hydroxy, amino, mercapto, halogen, cyano, optionally deuterated $C_{1-6}$ alkyl, optionally deuterated $C_{3-6}$ cycloalkyl, optionally deuterated $C_{1-6}$ alkoxy, halogenated $C_{1-6}$ alkyl, halogenated $C_{1-6}$ alkoxy or -$C_{1-6}$ alkylene-$NH_2$.

**9.** The compound of Formula (I) or salts, enantiomers, stereoisomers, solvates, or polymorphs thereof as claimed in claim 1, which is selected from the group consisting of:

3-(5-((4-benzhydrylpiperazin-1-yl)methyl)-1-oxoisoindolin-2-yl)piperidine-2,6-dione;
3-(5-((4-benzhydrylpiperazin-1-yl)methyl)-4-fluoro-1-oxoisoindolin-2-yl)piperidine-2,6-dione;
3-(5-((4-benzhydrylpiperazin-1-yl)methyl)-6-fluoro-1-oxoisoindolin-2-yl)piperidine-2,6-dione;
3-(5-((4-benzhydrylpiperazin-1-yl)methyl)-7-fluoro-1-oxoisoindolin-2-yl)piperidine-2,6-dione;
3-(4-((4-benzhydrylpiperazin-1-yl)methyl)-1-oxoisoindolin-2-yl)piperidine-2,6-dione;
3-(6-((4-benzhydrylpiperazin-1-yl)methyl)-1-oxoisoindolin-2-yl)piperidine-2,6-dione;
3-(7-((4-benzhydrylpiperazin-1-yl)methyl)-1-oxoisoindolin-2-yl)piperidine-2,6-dione;
3-(5-(2-(4-benzhydrylpiperazin-1-yl)ethyl)-1-oxoisoindolin-2-yl)piperidine-2,6-dione;
3-(5-(3-(4-benzhydrylpiperazin-1-yl)propyl)-1-oxoisoindolin-2-yl)piperidine-2,6-dione;
3-(5-(4-((4-benzhydrylpiperazin-1-yl)methyl)phenyl)-1-oxoisoindolin-2-yl)piperidine-2,6-dione;
3-(5-((1-benzhydrylpiperidin-4-yl)methyl)-1-oxoisoindolin-2-yl)piperidine-2,6-dione;
3-(5-((1-benzhydrylpiperidin-4-yl)amino)-1-oxoisoindolin-2-yl)piperidine-2,6-dione;
3-(5-((4-benzhydrylpiperazin-1-yl)amino)-1-oxoisoindolin-2-yl)piperidine-2,6-dione;
3-(5-(((S)-1-benzhydryl-3,3-difluoropiperidin-4-yl)amino)-1-oxoisoindolin-2-yl)piperidine-2,6-dione;
3-(5-((1-benzhydrylazetidin-3-yl)methyl)-1-oxoisoindolin-2-yl)piperidine-2,6-dione;
3-(5-((R)-(1-benzhydryl-3-hydroxyazetidin-3-yl)(hydroxy)methyl)-1-oxoisoindolin-2-yl)piperidine-2,6-dione;
3-(5-((1-benzhydrylazetidin-3-ylidene)methyl)-1-oxoisoindolin-2-yl)piperidine-2,6-dione;
3-(5-(1-benzhydryl-1,2,3,6-tetrahydropyridin-4-yl)-1-oxoisoindolin-2-yl)piperidine-2,6-dione;
3-(5-(1-benzhydrylpiperidin-4-yl)-1-oxoisoindolin-2-yl)piperidine-2,6-dione;
3-(5-((3S,4S)-1-benzhydryl-3,4-dihydroxypiperidin-4-yl)-1-oxoisoindolin-2-yl)piperidine-2,6-dione;
3-(5-(1-benzhydrylpiperidin-4-yl)-7-fluoro-1-oxoisoindolin-2-yl)piperidine-2,6-dione;
3-(5-(1-benzhydrylpiperidin-4-yl)-4-fluoro-1-oxoisoindolin-2-yl)piperidine-2,6-dione;
3-(5-(1-benzhydryl-1,2,3,6-tetrahydropyridin-4-yl)-4-fluoro-1-oxoisoindolin-2-yl)piperidine-2,6-dione;
3-(5-(8-benzhydryl-3,8-diazabicyclo[3.2.1]octan-3-yl)-1-oxoisoindolin-2-yl)piperidine-2,6-dione;
3-(5-(3-benzhydryl-3,8-diazabicyclo[3.2.1]octan-8-yl)-1-oxoisoindolin-2-yl)piperidine-2,6-dione;
3-(5-((3-benzhydrylimidazolidin-1-yl)methyl)-1-oxoisoindolin-2-yl)piperidine-2,6-dione;
3-(5-((3-benzhydrylimidazolidin-1-yl)methyl)-4-fluoro-1-oxoisoindolin-2-yl)piperidine-2,6-dione;
3-(5-((3-benzhydrylimidazolidin-1-yl)methyl)-6-fluoro-1-oxoisoindolin-2-yl)piperidine-2,6-dione;
3-(5-((3-benzhydrylimidazolidin-1-yl)methyl)-7-fluoro-1-oxoisoindolin-2-yl)piperidine-2,6-dione;
3-(4-((3-benzhydrylimidazolidin-1-yl)methyl)-1-oxoisoindolin-2-yl)piperidine-2,6-dione;
3-(5-((4-benzhydryl-1,4-diazepan-1-yl)methyl)-1-oxoisoindolin-2-yl)piperidine-2,6-dione;
3-(5-((4-benzhydryl-1,4-diazepan-1-yl)methyl)-4-fluoro-1-oxoisoindolin-2-yl)piperidine-2,6-dione;
3-(5-((4-benzhydryl-1,4-diazepan-1-yl)methyl)-6-fluoro-1-oxoisoindolin-2-yl)piperidine-2,6-dione;
3-(5-((4-benzhydryl-1,4-diazepan-1-yl)methyl)-7-fluoro-1-oxoisoindolin-2-yl)piperidine-2,6-dione;
3-(5-(((R)-4-benzhydryl-3,3-difluoropiperidin-1-yl)methyl)-1-oxoisoindolin-2-yl)piperidine-2,6-dione;
3-(5-(((R)-4-(bis(4-fluorophenyl)methyl)-3,3-difluoropiperidin-1-yl)methyl)-1-oxoisoindolin-2-yl)piperidine-2,6-dione;
3-(5-(((R)-4-(bis(4-chlorophenyl)methyl)-3,3-difluoropiperidin-1-yl)methyl)-1-oxoisoindolin-2-yl)piperidine-2,6-dione;

3-(5-(((R)-4-(9H-fluoren-9-yl)-3,3-difluoropiperidin-1-yl)methyl)-1-oxoisoindolin-2-yl)piperidine-2,6-dione;
3-(5-((7-benzhydryl-2,7-diazaspiro[3.5]nonan-2-yl)methyl)-1-oxoisoindolin-2-yl)piperidine-2,6-dione;
3-(5-((7-benzhydryl-2,7-diazaspiro[3.5]nonan-2-yl)methyl)-4-fluoro-1-oxoisoindolin-2-yl)piperidine-2,6-dione;
3-(5-((7-benzhydryl-2,7-diazaspiro[3.5]nonan-2-yl)methyl)-6-fluoro-1-oxoisoindolin-2-yl)piperidine-2,6-dione;
3-(5-((7-benzhydryl-2,7-diazaspiro[3.5]nonan-2-yl)methyl)-7-fluoro-1-oxoisoindolin-2-yl)piperidine-2,6-dione;
3-(4-((7-benzhydryl-2,7-diazaspiro[3.5]nonan-2-yl)methyl)-1-oxoisoindolin-2-yl)piperidine-2,6-dione;
3-(5-((8-benzhydryl-2,8-diazaspiro[4.5]decan-2-yl)methyl)-1-oxoisoindolin-2-yl)piperidine-2,6-dione;
3-(5-((8-benzhydryl-2,8-diazaspiro[4.5]decan-2-yl)methyl)-4-fluoro-1-oxoisoindolin-2-yl)piperidine-2,6-dione;
3-(5-((8-benzhydryl-2,8-diazaspiro[4.5]decan-2-yl)methyl)-6-fluoro-1-oxoisoindolin-2-yl)piperidine-2,6-dione;
3-(5-((8-benzhydryl-2,8-diazaspiro[4.5]decan-2-yl)methyl)-7-fluoro-1-oxoisoindolin-2-yl)piperidine-2,6-dione;
3-(4-((8-benzhydryl-2,8-diazaspiro[4.5]decan-2-yl)methyl)-1-oxoisoindolin-2-yl)piperidine-2,6-dione;
3-(5-((9-benzhydryl-3,9-diazaspiro[5.5]undecan-3-yl)methyl)-1-oxoisoindolin-2-yl)piperidine-2,6-dione;
3-(5-((9-benzhydryl-3,9-diazaspiro[5.5]undecan-3-yl)methyl)-4-fluoro-1-oxoisoindolin-2-yl)piperidine-2,6-dione;
3-(5-((9-benzhydryl-3,9-diazaspiro[5.5]undecan-3-yl)methyl)-6-fluoro-1-oxoisoindolin-2-yl)piperidine-2,6-dione;
3-(5-((9-benzhydryl-3,9-diazaspiro[5.5]undecan-3-yl)methyl)-7-fluoro-1-oxoisoindolin-2-yl)piperidine-2,6-dione;
3-(4-((9-benzhydryl-3,9-diazaspiro[5.5]undecan-3-yl)methyl)-1-oxoisoindolin-2-yl)piperidine-2,6-dione;
3-(5-((5-benzhydrylhexahydropyrrolo[3,4-c]pyrrol-2(1H)-yl)methyl)-1-oxoisoindolin-2-yl)piperidine-2,6-dione;
3-(5-((5-benzhydrylhexahydropyrrolo[3,4-c]pyrrol-2(1H)-yl)methyl)-4-fluoro-1-oxoisoindolin-2-yl)piperidine-2,6-dione;
3-(5-((5-benzhydrylhexahydropyrrolo[3,4-c]pyrrol-2(1H)-yl)methyl)-6-fluoro-1-oxoisoindolin-2-yl)piperidine-2,6-dione;
3-(5-((5-benzhydrylhexahydropyrrolo[3,4-c]pyrrol-2(1H)-yl)methyl)-7-fluoro-1-oxoisoindolin-2-yl)piperidine-2,6-dione;
3-(4-((5-benzhydrylhexahydropyrrolo[3,4-c]pyrrol-2(1H)-yl)methyl)-1-oxoisoindolin-2-yl)piperidine-2,6-dione;
3-(5-((3-benzhydryl-3,6-diazabicyclo[3.1.1]heptan-6-yl)methyl)-1-oxoisoindolin-2-yl)piperidine-2,6-dione;
3-(5-((3-benzhydryl-3,6-diazabicyclo[3.1.1]heptan-6-yl)methyl)-4-fluoro-1-oxoisoindolin-2-yl)piperidine-2,6-dione;
3-(5-((3-benzhydryl-3,6-diazabicyclo[3.1.1]heptan-6-yl)methyl)-6-fluoro-1-oxoisoindolin-2-yl)piperidine-2,6-dione;
3-(5-((3-benzhydryl-3,6-diazabicyclo[3.1.1]heptan-6-yl)methyl)-7-fluoro-1-oxoisoindolin-2-yl)piperidine-2,6-dione;
3-(5-(((1R,4R)-5-benzhydryl-2,5-diazabicyclo[2.2.1]heptan-2-yl)methyl)-1-oxoisoindolin-2-yl)piperidine-2,6-dione;
3-(5-(((1R,4R)-5-benzhydryl-2,5-diazabicyclo[2.2.1]heptan-2-yl)methyl)-4-fluoro-1-oxoisoindolin-2-yl)piperidine-2,6-dione;
3-(5-(((1R,4R)-5-benzhydryl-2,5-diazabicyclo[2.2.1]heptan-2-yl)methyl)-6-fluoro-1-oxoisoindolin-2-yl)piperidine-2,6-dione;
3-(5-(((1R,4R)-5-benzhydryl-2,5-diazabicyclo[2.2.1]heptan-2-yl)methyl)-7-fluoro-1-oxoisoindolin-2-yl)piperidine-2,6-dione;
3-(5-(((1S,4S)-5-benzhydryl-2,5-diazabicyclo[2.2.1]heptan-2-yl)methyl)-1-oxoisoindolin-2-yl)piperidine-2,6-dione;
3-(5-(((1S,4S)-5-benzhydryl-2,5-diazabicyclo[2.2.1]heptan-2-yl)methyl)-4-fluoro-1-oxoisoindolin-2-yl)piperidine-2,6-dione;
3-(5-(((1S,4S)-5-benzhydryl-2,5-diazabicyclo[2.2.1]heptan-2-yl)methyl)-6-fluoro-1-oxoisoindolin-2-yl)piperidine-2,6-dione;
3-(5-(((1S,4S)-5-benzhydryl-2,5-diazabicyclo[2.2.1]heptan-2-yl)methyl)-7-fluoro-1-oxoisoindolin-2-yl)piperidine-2,6-dione;
3-(5-((3-benzhydryl-3,8-diazabicyclo[3.2.1]octan-8-yl)methyl)-1-oxoisoindolin-2-yl)piperidine-2,6-dione;
3-(5-((3-benzhydryl-3,8-diazabicyclo[3.2.1]octan-8-yl)methyl)-4-fluoro-1-oxoisoindolin-2-yl)piperidine-2,6-dione;
3-(5-((3-benzhydryl-3,8-diazabicyclo[3.2.1]octan-8-yl)methyl)-6-fluoro-1-oxoisoindolin-2-yl)piperidine-2,6-dione;
3-(5-((3-benzhydryl-3,8-diazabicyclo[3.2.1]octan-8-yl)methyl)-7-fluoro-1-oxoisoindolin-2-yl)piperidine-2,6-dione;
3-(5-((8-benzhydryl-3,8-diazabicyclo[3.2.1]octan-3-yl)methyl)-1-oxoisoindolin-2-yl)piperidine-2,6-dione;

3-(5-((8-benzhydryl-3,8-diazabicyclo[3.2.1]octan-3-yl)methyl)-4-fluoro-1-oxoisoindolin-2-yl)piperidine-2,6-dione;
3-(5-((8-benzhydryl-3,8-diazabicyclo[3.2.1]octan-3-yl)methyl)-6-fluoro-1-oxoisoindolin-2-yl)piperidine-2,6-dione;
3-(5-((8-benzhydryl-3,8-diazabicyclo[3.2.1]octan-3-yl)methyl)-7-fluoro-1-oxoisoindolin-2-yl)piperidine-2,6-dione;
3-(5-(((R)-4-benzhydryl-2-methylpiperazin-1-yl)methyl)-1-oxoisoindolin-2-yl)piperidine-2,6-dione;
3-(5-(((R)-4-benzhydryl-2-methylpiperazin-1-yl)methyl)-4-fluoro-1-oxoisoindolin-2-yl)piperidine-2,6-dione;
3-(5-(((R)-4-benzhydryl-2-methylpiperazin-1-yl)methyl)-6-fluoro-1-oxoisoindolin-2-yl)piperidine-2,6-dione;
3-(5-(((R)-4-benzhydryl-2-methylpiperazin-1-yl)methyl)-7-fluoro-1-oxoisoindolin-2-yl)piperidine-2,6-dione;
3-(5-((4-benzhydryl-2,6-dimethylpiperazin-1-yl)methyl)-1-oxoisoindolin-2-yl)piperidine-2,6-dione;
3-(5-((4-benzhydryl-2,6-dimethylpiperazin-1-yl)methyl)-4-fluoro-1-oxoisoindolin-2-yl)piperidine-2,6-dione;
3-(5-((4-benzhydryl-2,6-dimethylpiperazin-1-yl)methyl)-6-fluoro-1-oxoisoindolin-2-yl)piperidine-2,6-dione;
3-(5-((4-benzhydryl-2,6-dimethylpiperazin-1-yl)methyl)-7-fluoro-1-oxoisoindolin-2-yl)piperidine-2,6-dione;
3-(5-((4-benzhydryl-3,5-dimethylpiperazin-1-yl)methyl)-1-oxoisoindolin-2-yl)piperidine-2,6-dione;
3-(5-((4-benzhydryl-3,5-dimethylpiperazin-1-yl)methyl)-4-fluoro-1-oxoisoindolin-2-yl)piperidine-2,6-dione;
3-(5-((4-benzhydryl-3,5-dimethylpiperazin-1-yl)methyl)-6-fluoro-1-oxoisoindolin-2-yl)piperidine-2,6-dione;
3-(5-((4-benzhydryl-3,5-dimethylpiperazin-1-yl)methyl)-7-fluoro-1-oxoisoindolin-2-yl)piperidine-2,6-dione;
3-(5-((5-benzhydryl-2,5-diazabicyclo[2.2.2]octan-2-yl)methyl)-1-oxoisoindolin-2-yl)piperidine-2,6-dione;
3-(5-((5-benzhydryl-2,5-diazabicyclo [2.2.2] octan-2-yl)methyl)-4-fluoro-1-oxoisoindolin-2-yl)piperidine-2,6-dione;
3-(5-((5-benzhydryl-2,5-diazabicyclo [2.2.2] octan-2-yl)methyl)-6-fluoro-1-oxoisoindolin-2-yl)piperidine-2,6-dione;
3-(5-((5-benzhydryl-2,5-diazabicyclo[2.2.2]octan-2-yl)methyl)-7-fluoro-1-oxoisoindolin-2-yl)piperidine-2,6-dione;
3-(5-((4-benzhydryl-2-(fluoromethyl)piperazin-1-yl)methyl)-1-oxoisoindolin-2-yl)piperidine-2,6-dione;
3-(5-((4-benzhydryl-2-(fluoromethyl)piperazin-1-yl)methyl)-4-fluoro-1-oxoisoindolin-2-yl)piperidine-2,6-dione;
3-(5-((4-benzhydryl-2-(fluoromethyl)piperazin-1-yl)methyl)-6-fluoro-1-oxoisoindolin-2-yl)piperidine-2,6-dione;
3-(5-((4-benzhydryl-2-(fluoromethyl)piperazin-1-yl)methyl)-7-fluoro-1-oxoisoindolin-2-yl)piperidine-2,6-dione;
3-(5-((4-benzhydryl-2-(trifluoromethyl)piperazin-1-yl)methyl)-1-oxoisoindolin-2-yl)piperidine-2,6-dione;
3-(5-((4-benzhydryl-2-(trifluoromethyl)piperazin-1-yl)methyl)-4-fluoro-1-oxoisoindolin-2-yl)piperidine-2,6-dione;
3-(5-((4-benzhydryl-2-(trifluoromethyl)piperazin-1-yl)methyl)-6-fluoro-1-oxoisoindolin-2-yl)piperidine-2,6-dione;
3-(5-((4-benzhydryl-2-(trifluoromethyl)piperazin-1-yl)methyl)-7-fluoro-1-oxoisoindolin-2-yl)piperidine-2,6-dione;
3-(5-((4-benzhydryl-3,3-dimethylpiperazin-1-yl)methyl)-1-oxoisoindolin-2-yl)piperidine-2,6-dione;
3-(5-((4-benzhydryl-3,3-dimethylpiperazin-1-yl)methyl)-4-fluoro-1-oxoisoindolin-2-yl)piperidine-2,6-dione;
3-(5-((4-benzhydryl-3,3-dimethylpiperazin-1-yl)methyl)-6-fluoro-1-oxoisoindolin-2-yl)piperidine-2,6-dione;
3-(5-((4-benzhydryl-3,3-dimethylpiperazin-1-yl)methyl)-7-fluoro-1-oxoisoindolin-2-yl)piperidine-2,6-dione;
3-(5-((3-benzhydryl-2-oxoimidazolidin-1-yl)methyl)-1-oxoisoindolin-2-yl)piperidine-2,6-dione;
3-(5-((3-benzhydryl-2-oxoimidazolidin-1-yl)methyl)-4-fluoro-1-oxoisoindolin-2-yl)piperidine-2,6-dione;
3-(5-((3-benzhydryl-2-oxoimidazolidin-1-yl)methyl)-6-fluoro-1-oxoisoindolin-2-yl)piperidine-2,6-dione;
3-(5-((3-benzhydryl-2-oxoimidazolidin-1-yl)methyl)-7-fluoro-1-oxoisoindolin-2-yl)piperidine-2,6-dione;
3-(5-((4-benzhydryl-2-oxopiperazin-1-yl)methyl)-1-oxoisoindolin-2-yl)piperidine-2,6-dione;
3-(1-oxo-5-((4-(phenyl(pyridin-2-yl)methyl)pip erazin-1-yl)methyl)isoindolin-2-yl)piperidine-2,6-dione;
3-(1-oxo-5-((4-((R)-phenyl(pyridin-2-yl)methyl)piperazin-1-yl)methyl)isoindolin-2-yl)piperidine-2,6-dione;
3-(1-oxo-5-((4-((S)-phenyl(pyridin-2-yl)methyl)piperazin-1-yl)methyl)isoindolin-2-yl)piperidine-2,6-dione;
3-(4-fluoro-1-oxo-5-((4-(phenyl(pyridin-2-yl)methyl)piperazin-1-yl)methyl)isoindolin-2-yl)piperidine-2,6-dione;
3-(6-fluoro-1-oxo-5-((4-(phenyl(pyridin-2-yl)methyl)piperazin-1-yl)methyl)isoindolin-2-yl)piperidine-2,6-dione;
3-(7-fluoro-1-oxo-5-((4-(phenyl(pyridin-2-yl)methyl)piperazin-1-yl)methyl)isoindolin-2-yl)piperidine-2,6-dione;
3-(1-oxo-4-((4-(phenyl(pyridin-2-yl)methyl)pip erazin-1-yl)methyl)isoindolin-2-yl)piperidine-2,6-dione;
3-(1-oxo-5-((4-(phenyl(pyridin-3-yl)methyl)piperazin-1-yl)methyl)isoindolin-2-yl)piperidine-2,6-dione;
3-(4-fluoro-1-oxo-5-((4-(phenyl(pyridin-3-yl)methyl)piperazin-1-yl)methyl)isoindolin-2-yl)piperidine-2,6-dione;
3-(6-fluoro-1-oxo-5-((4-(phenyl(pyridin-3-yl)methyl)piperazin-1-yl)methyl)isoindolin-2-yl)piperidine-2,6-dione;
3-(7-fluoro-1-oxo-5-((4-(phenyl(pyridin-3-yl)methyl)piperazin-1-yl)methyl)isoindolin-2-yl)piperidine-2,6-dione;
3-(1-oxo-4-((4-(phenyl(pyridin-3-yl)methyl)piperazin-1-yl)methyl)isoindolin-2-yl)piperidine-2,6-dione;
3-(1-oxo-5-((4-(phenyl(pyridin-4-yl)methyl)piperazin-1-yl)methyl)isoindolin-2-yl)piperidine-2,6-dione;

3-(4-fluoro-1-oxo-5-((4-(phenyl(pyridin-4-yl)methyl)piperazin-1-yl)methyl)isoindolin-2-yl)piperidine-2,6-dione;
3-(6-fluoro-1-oxo-5-((4-(phenyl(pyridin-4-yl)methyl)piperazin-1-yl)methyl)isoindolin-2-yl)piperidine-2,6-dione;
3-(7-fluoro-1-oxo-5-((4-(phenyl(pyridin-4-yl)methyl)piperazin-1-yl)methyl)isoindolin-2-yl)piperidine-2,6-dione;
3-(1-oxo-4-((4-(phenyl(pyridin-4-yl)methyl)pip erazin-1-yl)methyl)isoindolin-2-yl)piperidine-2,6-dione;
3-(5-((4-(di(pyridin-2-yl)methyl)piperazin-1-yl)methyl)-1-oxoisoindolin-2-yl)piperidine-2,6-dione;
3-(5-((4-(di(pyridin-2-yl)methyl)piperazin-1-yl)methyl)-4-fluoro-1-oxoisoindolin-2-yl)piperidine-2,6-dione;
3-(5-((4-(di(pyridin-2-yl)methyl)piperazin-1-yl)methyl)-6-fluoro-1-oxoisoindolin-2-yl)piperidine-2,6-dione;
3-(5-((4-(di(pyridin-2-yl)methyl)piperazin-1-yl)methyl)-7-fluoro-1-oxoisoindolin-2-yl)piperidine-2,6-dione;
3-(4-((4-(di(pyridin-2-yl)methyl)piperazin-1-yl)methyl)-1-oxoisoindolin-2-yl)piperidine-2,6-dione;
3-(5-((4-(di(pyridin-3-yl)methyl)piperazin-1-yl)methyl)-1-oxoisoindolin-2-yl)piperidine-2,6-dione;
3-(5-((4-(di(pyridin-3-yl)methyl)piperazin-1-yl)methyl)-4-fluoro-1-oxoisoindolin-2-yl)piperidine-2,6-dione;
3-(5-((4-(di(pyridin-3-yl)methyl)piperazin-1-yl)methyl)-6-fluoro-1-oxoisoindolin-2-yl)piperidine-2,6-dione;
3-(5-((4-(di(pyridin-3-yl)methyl)piperazin-1-yl)methyl)-7-fluoro-1-oxoisoindolin-2-yl)piperidine-2,6-dione;
3-(4-((4-(di(pyridin-3-yl)methyl)piperazin-1-yl)methyl)-1-oxoisoindolin-2-yl)piperidine-2,6-dione;
3-(5-((4-(di(pyridin-4-yl)methyl)piperazin-1-yl)methyl)-1-oxoisoindolin-2-yl)piperidine-2,6-dione;
3-(5-((4-(di(pyridin-4-yl)methyl)piperazin-1-yl)methyl)-4-fluoro-1-oxoisoindolin-2-yl)piperidine-2,6-dione;
3-(5-((4-(di(pyridin-4-yl)methyl)piperazin-1-yl)methyl)-6-fluoro-1-oxoisoindolin-2-yl)piperidine-2,6-dione;
3-(5-((4-(di(pyridin-4-yl)methyl)piperazin-1-yl)methyl)-7-fluoro-1-oxoisoindolin-2-yl)piperidine-2,6-dione;
3-(4-((4-(di(pyridin-4-yl)methyl)piperazin-1-yl)methyl)-1-oxoisoindolin-2-yl)piperidine-2,6-dione;
3-(5-((3-(bis(4-fluorophenyl)methyl)-2-oxoimidazolidin-1-yl)methyl)-1-oxoisoindolin-2-yl)piperidine-2,6-dione;
3-(5-((3-(bis(4-fluorophenyl)methyl)-2-oxoimidazolidin-1-yl)methyl)-4-fluoro-1-oxoisoindolin-2-yl)piperidine-2,6-dione;
3-(5-((3-(bis(4-fluorophenyl)methyl)-2-oxoimidazolidin-1-yl)methyl)-6-fluoro-1-oxoisoindolin-2-yl)piperidine-2,6-dione;
3-(5-((3-(bis(4-fluorophenyl)methyl)-2-oxoimidazolidin-1-yl)methyl)-7-fluoro-1-oxoisoindolin-2-yl)piperidine-2,6-dione;
3-(5-((3-(bis(3-fluorophenyl)methyl)-2-oxoimidazolidin-1-yl)methyl)-1-oxoisoindolin-2-yl)piperidine-2,6-dione;
3-(5-((3-(bis(3-fluorophenyl)methyl)-2-oxoimidazolidin-1-yl)methyl)-4-fluoro-1-oxoisoindolin-2-yl)piperidine-2,6-dione;
3-(5-((3-(bis(3-fluorophenyl)methyl)-2-oxoimidazolidin-1-yl)methyl)-6-fluoro-1-oxoisoindolin-2-yl)piperidine-2,6-dione;
3-(5-((3-(bis(3-fluorophenyl)methyl)-2-oxoimidazolidin-1-yl)methyl)-7-fluoro-1-oxoisoindolin-2-yl)piperidine-2,6-dione;
3-(5-((3-(bis(2-fluorophenyl)methyl)-2-oxoimidazolidin-1-yl)methyl)-1-oxoisoindolin-2-yl)piperidine-2,6-dione;
3-(5-((3-(bis(2-fluorophenyl)methyl)-2-oxoimidazolidin-1-yl)methyl)-4-fluoro-1-oxoisoindolin-2-yl)piperidine-2,6-dione;
3-(5-((3-(bis(2-fluorophenyl)methyl)-2-oxoimidazolidin-1-yl)methyl)-6-fluoro-1-oxoisoindolin-2-yl)piperidine-2,6-dione;
3-(5-((3-(bis(2-fluorophenyl)methyl)-2-oxoimidazolidin-1-yl)methyl)-7-fluoro-1-oxoisoindolin-2-yl)piperidine-2,6-dione;
3-(5-((3-(bis(4-chlorophenyl)methyl)-2-oxoimidazolidin-1-yl)methyl)-1-oxoisoindolin-2-yl)piperidine-2,6-dione;
3-(5-((3-(bis(4-chlorophenyl)methyl)-2-oxoimidazolidin-1-yl)methyl)-4-fluoro-1-oxoisoindolin-2-yl)piperidine-2,6-dione;
3-(5-((3-(bis(4-chlorophenyl)methyl)-2-oxoimidazolidin-1-yl)methyl)-6-fluoro-1-oxoisoindolin-2-yl)piperidine-2,6-dione;
3-(5-((3-(bis(4-chlorophenyl)methyl)-2-oxoimidazolidin-1-yl)methyl)-7-fluoro-1-oxoisoindolin-2-yl)piperidine-2,6-dione;
3-(5-((4-((4-bromophenyl)(phenyl)methyl)piperazin-1-yl)methyl)-1-oxoisoindolin-2-yl)piperidine-2,6-dione;
3-(5-((4-(bis(4-fluorophenyl)methyl)piperazin-1-yl)methyl)-1-oxoisoindolin-2-yl)piperidine-2,6-dione;
3-(5-((4-(bis(4-fluorophenyl)methyl)piperazin-1-yl)methyl)-4-fluoro-1-oxoisoindolin-2-yl)piperidine-2,6-dione;
3-(5-((4-(bis(4-fluorophenyl)methyl)piperazin-1-yl)methyl)-6-fluoro-1-oxoisoindolin-2-yl)piperidine-2,6-dione;
3-(5-((4-(bis(4-fluorophenyl)methyl)piperazin-1-yl)methyl)-7-fluoro-1-oxoisoindolin-2-yl)piperidine-2,6-dione;
3-(4-((4-(bis(4-fluorophenyl)methyl)piperazin-1-yl)methyl)-1-oxoisoindolin-2-yl)piperidine-2,6-dione;
3-(6-((4-(bis(4-fluorophenyl)methyl)piperazin-1-yl)methyl)-1-oxoisoindolin-2-yl)piperidine-2,6-dione;
3-(7-((4-(bis(4-fluorophenyl)methyl)piperazin-1-yl)methyl)-1-oxoisoindolin-2-yl)piperidine-2,6-dione;
3-(5-((4-(bis(3-fluorophenyl)methyl)piperazin-1-yl)methyl)-1-oxoisoindolin-2-yl)piperidine-2,6-dione;
3-(5-((4-(bis(3-fluorophenyl)methyl)piperazin-1-yl)methyl)-4-fluoro-1-oxoisoindolin-2-yl)piperidine-2,6-dione;
3-(5-((4-(bis(3-fluorophenyl)methyl)piperazin-1-yl)methyl)-6-fluoro-1-oxoisoindolin-2-yl)piperidine-2,6-dione;

3-(5-((4-(bis(3-fluorophenyl)methyl)piperazin-1-yl)methyl)-7-fluoro-1-oxoisoindolin-2-yl)piperidine-2,6-dione;
3-(4-((4-(bis(3-fluorophenyl)methyl)piperazin-1-yl)methyl)-1-oxoisoindolin-2-yl)piperidine-2,6-dione;
3-(6-((4-(bis(3-fluorophenyl)methyl)piperazin-1-yl)methyl)-1-oxoisoindolin-2-yl)piperidine-2,6-dione;
3-(7-((4-(bis(3-fluorophenyl)methyl)piperazin-1-yl)methyl)-1-oxoisoindolin-2-yl)piperidine-2,6-dione;
3-(5-((4-(bis(2-fluorophenyl)methyl)piperazin-1-yl)methyl)-1-oxoisoindolin-2-yl)piperidine-2,6-dione;
3-(5-((4-(bis(2-fluorophenyl)methyl)piperazin-1-yl)methyl)-4-fluoro-1-oxoisoindolin-2-yl)piperidine-2,6-dione;
3-(5-((4-(bis(2-fluorophenyl)methyl)piperazin-1-yl)methyl)-6-fluoro-1-oxoisoindolin-2-yl)piperidine-2,6-dione;
3-(5-((4-(bis(2-fluorophenyl)methyl)piperazin-1-yl)methyl)-7-fluoro-1-oxoisoindolin-2-yl)piperidine-2,6-dione;
3-(5-((4-(bis(4-(trifluoromethyl)phenyl)methyl)piperazin-1-yl)methyl)-1-oxoisoindolin-2-yl)piperidine-2,6-dione;
3-(5-((4-(bis(4-(trifluoromethyl)phenyl)methyl)piperazin-1-yl)methyl)-4-fluoro-1-oxoisoindolin-2-yl)piperidine-2,6-dione;
3-(5-((4-(bis(4-(trifluoromethyl)phenyl)methyl)piperazin-1-yl)methyl)-6-fluoro-1-oxoisoindolin-2-yl)piperidine-2,6-dione;
3-(5-((4-(bis(4-(trifluoromethyl)phenyl)methyl)piperazin-1-yl)methyl)-7-fluoro-1-oxoisoindolin-2-yl)piperidine-2,6-dione;
3-(5-((4-(bis(2,4-difluorophenyl)methyl)piperazin-1-yl)methyl)-1-oxoisoindolin-2-yl)piperidine-2,6-dione;
3-(5-((4-((2,3-difluorophenyl)(3,4-difluorophenyl)methyl)piperazin-1-yl)methyl)-1-oxoisoindolin-2-yl)piperidine-2,6-dione;
3-(5-((4-(bis(3,5-difluorophenyl)methyl)piperazin-1-yl)methyl)-1-oxoisoindolin-2-yl)piperidine-2,6-dione;
3-(5-((4-(bis(perfluorophenyl)methyl)piperazin-1-yl)methyl)-1-oxoisoindolin-2-yl)piperidine-2,6-dione;
3-(5-((4-(bis(perfluorophenyl)methyl)piperazin-1-yl)methyl)-4-fluoro-1-oxoisoindolin-2-yl)piperidine-2,6-dione;
3-(5-((4-(bis(perfluorophenyl)methyl)piperazin-1-yl)methyl)-6-fluoro-1-oxoisoindolin-2-yl)piperidine-2,6-dione;
3-(5-((4-(bis(perfluorophenyl)methyl)piperazin-1-yl)methyl)-7-fluoro-1-oxoisoindolin-2-yl)piperidine-2,6-dione;
3-(5-((4-(bis(4-chlorophenyl)methyl)piperazin-1-yl)methyl)-1-oxoisoindolin-2-yl)piperidine-2,6-dione;
3-(5-((4-(bis(4-chlorophenyl)methyl)piperazin-1-yl)methyl)-4-fluoro-1-oxoisoindolin-2-yl)piperidine-2,6-dione;
3-(5-((4-(bis(4-chlorophenyl)methyl)piperazin-1-yl)methyl)-6-fluoro-1-oxoisoindolin-2-yl)piperidine-2,6-dione;
3-(5-((4-(bis(4-chlorophenyl)methyl)piperazin-1-yl)methyl)-7-fluoro-1-oxoisoindolin-2-yl)piperidine-2,6-dione;
3-(4-((4-(bis(4-chlorophenyl)methyl)piperazin-1-yl)methyl)-1-oxoisoindolin-2-yl)piperidine-2,6-dione;
3-(6-((4-(bis(4-chlorophenyl)methyl)piperazin-1-yl)methyl)-1-oxoisoindolin-2-yl)piperidine-2,6-dione;
3-(7-((4-(bis(4-chlorophenyl)methyl)piperazin-1-yl)methyl)-1-oxoisoindolin-2-yl)piperidine-2,6-dione;
3-(5-((4-(3,6-dichloro-9H-fluoren-9-yl)piperazin-1-yl)methyl)-1-oxoisoindolin-2-yl)piperidine-2,6-dione;
3-(5-((4-(2,7-dichloro-9H-fluoren-9-yl)piperazin-1-yl)methyl)-1-oxoisoindolin-2-yl)piperidine-2,6-dione;
3-(5-((4-(9H-fluoren-9-yl)piperazin-1-yl)methyl)-1-oxoisoindolin-2-yl)piperidine-2,6-dione;
2-((2-(2,6-dioxopiperidin-3-yl)-1-oxoisoindolin-5-yl)methyl)-1H-benzo[de]isoquinoline-1,3(2H)-dione;
2-(1-((2-(2,6-dioxopiperidin-3-yl)-1-oxoisoindolin-5-yl)methyl)piperidin-4-yl)-1H-benzo[de]isoquinoline-1,3(2H)-dione;
3-(5-((4-(bis(4-hydroxyphenyl)methyl)piperazin-1-yl)methyl)-1-oxoisoindolin-2-yl)piperidine-2,6-dione;
3-(5-((4-(bis(4-hydroxyphenyl)methyl)piperazin-1-yl)methyl)-4-fluoro-1-oxoisoindolin-2-yl)piperidine-2,6-dione;
3-(5-((4-(bis(4-hydroxyphenyl)methyl)piperazin-1-yl)methyl)-6-fluoro-1-oxoisoindolin-2-yl)piperidine-2,6-dione;
3-(5-((4-(bis(4-hydroxyphenyl)methyl)piperazin-1-yl)methyl)-7-fluoro-1-oxoisoindolin-2-yl)piperidine-2,6-dione;
3-(4-((4-(bis(4-hydroxyphenyl)methyl)piperazin-1-yl)methyl)-1-oxoisoindolin-2-yl)piperidine-2,6-dione;
3-(6-((4-(bis(4-hydroxyphenyl)methyl)piperazin-1-yl)methyl)-1-oxoisoindolin-2-yl)piperidine-2,6-dione;
3-(7-((4-(bis(4-hydroxyphenyl)methyl)piperazin-1-yl)methyl)-1-oxoisoindolin-2-yl)piperidine-2,6-dione;
3-(5-((4-(bis(4-(benzyloxy)phenyl)methyl)piperazin-1-yl)methyl)-1-oxoisoindolin-2-yl)piperidine-2,6-dione;
3-(5-((4-(bis(4-methoxyphenyl)methyl)piperazin-1-yl)methyl)-1-oxoisoindolin-2-yl)piperidine-2,6-dione;
3-(5-((4-(bis(4-methoxyphenyl)methyl)piperazin-1-yl)methyl)-4-fluoro-1-oxoisoindolin-2-yl)piperidine-2,6-dione;
3-(5-((4-(bis(4-methoxyphenyl)methyl)piperazin-1-yl)methyl)-6-fluoro-1-oxoisoindolin-2-yl)piperidine-2,6-dione;
3-(5-((4-(bis(4-methoxyphenyl)methyl)piperazin-1-yl)methyl)-7-fluoro-1-oxoisoindolin-2-yl)piperidine-2,6-dione;
3-(4-((4-(bis(4-methoxyphenyl)methyl)piperazin-1-yl)methyl)-1-oxoisoindolin-2-yl)piperidine-2,6-dione;
3-(6-((4-(bis(4-methoxyphenyl)methyl)piperazin-1-yl)methyl)-1-oxoisoindolin-2-yl)piperidine-2,6-dione;
3-(7-((4-(bis(4-methoxyphenyl)methyl)piperazin-1-yl)methyl)-1-oxoisoindolin-2-yl)piperidine-2,6-dione;
3-(5-((4-(diphenylamino)piperidin-1-yl)methyl)-1-oxoisoindolin-2-yl)piperidine-2,6-dione;

3-(5-((4-(diphenylamino)piperidin-1-yl)methyl)-4-fluoro-1-oxoisoindolin-2-yl)piperidine-2,6-dione;
3-(5-((4-(diphenylamino)piperidin-1-yl)methyl)-6-fluoro-1-oxoisoindolin-2-yl)piperidine-2,6-dione;
3-(5-((4-(diphenylamino)piperidin-1-yl)methyl)-7-fluoro-1-oxoisoindolin-2-yl)piperidine-2,6-dione;
3-(4-((4-(diphenylamino)piperidin-1-yl)methyl)-1-oxoisoindolin-2-yl)piperidine-2,6-dione;
3-(1-oxo-5-((4-(phenyl(pyridin-2-yl)amino)piperidin-1-yl)methyl)isoindolin-2-yl)piperidine-2,6-dione;
3-(4-fluoro-1-oxo-5-((4-(phenyl(pyridin-2-yl)amino)piperidin-1-yl)methyl)isoindolin-2-yl)piperidine-2,6-dione;
3-(6-fluoro-1-oxo-5-((4-(phenyl(pyridin-2-yl)amino)piperidin-1-yl)methyl)isoindolin-2-yl)piperidine-2,6-dione;
3-(7-fluoro-1-oxo-5-((4-(phenyl(pyridin-2-yl)amino)piperidin-1-yl)methyl)isoindolin-2-yl)piperidine-2,6-dione;
3-(1-oxo-4-((4-(phenyl(pyridin-2-yl)amino)piperidin-1-yl)methyl)isoindolin-2-yl)piperidine-2,6-dione;
3-(5-((4-(diphenylamino)cyclohexyl)amino)-1-oxoisoindolin-2-yl)piperidine-2,6-dione;
3-(5-(4-benzhydrylpiperazine-1-carbonyl)-1-oxoisoindolin-2-yl)piperidine-2,6-dione;
3-(5-(4-benzhydrylpiperazine-1-carbonyl)-4-fluoro-1-oxoisoindolin-2-yl)piperidine-2,6-dione;
3-(5-(4-benzhydrylpiperazine-1-carbonyl)-6-fluoro-1-oxoisoindolin-2-yl)piperidine-2,6-dione;
3-(5-(4-benzhydrylpiperazine-1-carbonyl)-7-fluoro-1-oxoisoindolin-2-yl)piperidine-2,6-dione;
3-(4-(4-benzhydrylpiperazine-1-carbonyl)-1-oxoisoindolin-2-yl)piperidine-2,6-dione;
3-(6-(4-benzhydrylpiperazine-1-carbonyl)-1-oxoisoindolin-2-yl)piperidine-2,6-dione;
3-(7-(4-benzhydrylpiperazine-1-carbonyl)-1-oxoisoindolin-2-yl)piperidine-2,6-dione;
3-(5-(3-benzhydrylimidazolidine-1-carbonyl)-1-oxoisoindolin-2-yl)piperidine-2,6-dione;
3-(5-(3-benzhydryl-2-oxoimidazolidine-1-carbonyl)-1-oxoisoindolin-2-yl)piperidine-2,6-dione;
3-(5-(4-benzhydryl-1,4-diazepane-1-carbonyl)-1-oxoisoindolin-2-yl)piperidine-2,6-dione;
3-(5-(4-benzhydryl-1,4-diazepane-1-carbonyl)-4-fluoro-1-oxoisoindolin-2-yl)piperidine-2,6-dione;
3-(5-(4-benzhydryl-1,4-diazepane-1-carbonyl)-6-fluoro-1-oxoisoindolin-2-yl)piperidine-2,6-dione;
3-(5-(4-benzhydryl-1,4-diazepane-1-carbonyl)-7-fluoro-1-oxoisoindolin-2-yl)piperidine-2,6-dione;
3-(5-(7-benzhydryl-2,7-diazaspiro[3.5]nonane-2-carbonyl)-1-oxoisoindolin-2-yl)piperidine-2,6-dione;
3-(5-(8-benzhydryl-2,8-diazaspiro[4.5]decane-2-carbonyl)-1-oxoisoindolin-2-yl)piperidine-2,6-dione;
3-(5-(9-benzhydryl-3,9-diazaspiro[5.5]undecane-3-carbonyl)-1-oxoisoindolin-2-yl)piperidine-2,6-dione;
3-(5-(5-benzhydryloctahydropyrrolo[3,4-c]pyrrole-2-carbonyl)-1-oxoisoindolin-2-yl)piperidine-2,6-dione;
3-(5-(3-benzhydryl-3,6-diazabicyclo[3.1.1]heptane-6-carbonyl)-1-oxoisoindolin-2-yl)piperidine-2,6-dione;
3-(5-(3-benzhydryl-3,6-diazabicyclo[3.1.1]heptane-6-carbonyl)-4-fluoro-1-oxoisoindolin-2-yl)piperidine-2,6-dione;
3-(5-(3-benzhydryl-3,6-diazabicyclo[3.1.1]heptane-6-carbonyl)-6-fluoro-1-oxoisoindolin-2-yl)piperidine-2,6-dione;
3-(5-(3-benzhydryl-3,6-diazabicyclo[3.1.1]heptane-6-carbonyl)-7-fluoro-1-oxoisoindolin-2-yl)piperidine-2,6-dione;
3-(5-((1R,4R)-5-benzhydryl-2,5-diazabicyclo[2.2.1]heptane-2-carbonyl)-1-oxoisoindolin-2-yl)piperidine-2,6-dione;
3-(5-((1R,4R)-5-benzhydryl-2,5-diazabicyclo[2.2.1]heptane-2-carbonyl)-4-fluoro-1-oxoisoindolin-2-yl)piperidine-2,6-dione;
3-(5-((1R,4R)-5-benzhydryl-2,5-diazabicyclo[2.2.1]heptane-2-carbonyl)-6-fluoro-1-oxoisoindolin-2-yl)piperidine-2,6-dione;
3-(5-((1R,4R)-5-benzhydryl-2,5-diazabicyclo[2.2.1]heptane-2-carbonyl)-7-fluoro-1-oxoisoindolin-2-yl)piperidine-2,6-dione;
3-(5-((1S,4S)-5-benzhydryl-2,5-diazabicyclo[2.2.1]heptane-2-carbonyl)-1-oxoisoindolin-2-yl)piperidine-2,6-dione;
3-(5-((1S,4S)-5-benzhydryl-2,5-diazabicyclo[2.2.1]heptane-2-carbonyl)-4-fluoro-1-oxoisoindolin-2-yl)piperidine-2,6-dione;
3-(5-((1S,4S)-5-benzhydryl-2,5-diazabicyclo[2.2.1]heptane-2-carbonyl)-6-fluoro-1-oxoisoindolin-2-yl)piperidine-2,6-dione;
3-(5-((1S,4S)-5-benzhydryl-2,5-diazabicyclo[2.2.1]heptane-2-carbonyl)-7-fluoro-1-oxoisoindolin-2-yl)piperidine-2,6-dione;
3-(5-(3-benzhydryl-3,8-diazabicyclo[3.2.1]octane-8-carbonyl)-1-oxoisoindolin-2-yl)piperidine-2,6-dione;
3-(5-(3-benzhydryl-3,8-diazabicyclo[3.2.1]octane-8-carbonyl)-4-fluoro-1-oxoisoindolin-2-yl)piperidine-2,6-dione;
3-(5-(3-benzhydryl-3,8-diazabicyclo[3.2.1]octane-8-carbonyl)-6-fluoro-1-oxoisoindolin-2-yl)piperidine-2,6-dione;
3-(5-(3-benzhydryl-3,8-diazabicyclo[3.2.1]octane-8-carbonyl)-7-fluoro-1-oxoisoindolin-2-yl)piperidine-2,6-dione;
3-(5-(8-benzhydryl-3,8-diazabicyclo[3.2.1]octane-3-carbonyl)-1-oxoisoindolin-2-yl)piperidine-2,6-dione;

3-(5-(8-benzhydryl-3,8-diazabicyclo[3.2.1]octane-3-carbonyl)-4-fluoro-1-oxoisoindolin-2-yl)piperidine-2,6-dione;
3-(5-(8-benzhydryl-3,8-diazabicyclo[3.2.1]octane-3-carbonyl)-6-fluoro-1-oxoisoindolin-2-yl)piperidine-2,6-dione;
3-(5-(8-benzhydryl-3,8-diazabicyclo[3.2.1]octane-3-carbonyl)-7-fluoro-1-oxoisoindolin-2-yl)piperidine-2,6-dione;
3-(5-((R)-4-benzhydryl-2-methylpiperazine-1-carbonyl)-1-oxoisoindolin-2-yl)piperidine-2,6-dione;
3-(5-((R)-4-benzhydryl-2-methylpiperazine-1-carbonyl)-4-fluoro-1-oxoisoindolin-2-yl)piperidine-2,6-dione;
3-(5-((R)-4-benzhydryl-2-methylpiperazine-1-carbonyl)-6-fluoro-1-oxoisoindolin-2-yl)piperidine-2,6-dione;
3-(5-((R)-4-benzhydryl-2-methylpiperazine-1-carbonyl)-7-fluoro-1-oxoisoindolin-2-yl)piperidine-2,6-dione;
3-(5-(4-benzhydryl-2,6-dimethylpiperazine-1-carbonyl)-1-oxoisoindolin-2-yl)piperidine-2,6-dione;
3-(5-(4-benzhydryl-2,6-dimethylpiperazine-1-carbonyl)-4-fluoro-1-oxoisoindolin-2-yl)piperidine-2,6-dione;
3-(5-(4-benzhydryl-2,6-dimethylpiperazine-1-carbonyl)-6-fluoro-1-oxoisoindolin-2-yl)piperidine-2,6-dione;
3-(5-(4-benzhydryl-2,6-dimethylpiperazine-1-carbonyl)-7-fluoro-1-oxoisoindolin-2-yl)piperidine-2,6-dione;
3-(5-(4-benzhydryl-3,5-dimethylpiperazine- 1 -carbonyl)- 1-oxoisoindolin-2-yl)piperidine-2,6-dione;
3-(5-(4-benzhydryl-3,5-dimethylpiperazine-1-carbonyl)-7-fluoro-1-oxoisoindolin-2-yl)piperidine-2,6-dione;
3-(5-(5-benzhydryl-2,5-diazabicyclo [2.2.2] octane-2-carbonyl)-1-oxoisoindolin-2-yl)piperidine-2,6-dione;
3-(5-(5-benzhydryl-2,5-diazabicyclo[2.2.2]octane-2-carbonyl)-4-fluoro-1-oxoisoindolin-2-yl)piperidine-2,6-dione;
3-(5-(5-benzhydryl-2,5-diazabicyclo[2.2.2]octane-2-carbonyl)-6-fluoro-1-oxoisoindolin-2-yl)piperidine-2,6-dione;
3-(5-(5-benzhydryl-2,5-diazabicyclo[2.2.2]octane-2-carbonyl)-7-fluoro-1-oxoisoindolin-2-yl)piperidine-2,6-dione;
3-(5-(4-benzhydryl-2-(fluoromethyl)piperazine-1-carbonyl)-1-oxoisoindolin-2-yl)piperidine-2,6-dione;
3-(5-(4-benzhydryl-2-(fluoromethyl)piperazine-1-carbonyl)-4-fluoro-1-oxoisoindolin-2-yl)piperidine-2,6-dione;
3-(5-(4-benzhydryl-2-(fluoromethyl)piperazine-1-carbonyl)-6-fluoro-1-oxoisoindolin-2-yl)piperidine-2,6-dione;
3-(5-(4-benzhydryl-2-(fluoromethyl)piperazine-1-carbonyl)-7-fluoro-1-oxoisoindolin-2-yl)piperidine-2,6-dione;
3-(5-(4-benzhydryl-2-(trifluoromethyl)piperazine-1-carbonyl)-1-oxoisoindolin-2-yl)piperidine-2,6-dione;
3-(5-(4-benzhydryl-2-(trifluoromethyl)piperazine-1-carbonyl)-4-fluoro-1-oxoisoindolin-2-yl)piperidine-2,6-dione;
3-(5-(4-benzhydryl-2-(trifluoromethyl)piperazine- 1-carbonyl)-6-fluoro- 1-oxoisoindolin-2-yl)piperidine-2,6-dione;
3-(5-(4-benzhydryl-2-(trifluoromethyl)piperazine-1-carbonyl)-7-fluoro-1-oxoisoindolin-2-yl)piperidine-2,6-dione;
3-(5-(4-benzhydryl-3,3-dimethylpiperazine-1-carbonyl)-1-oxoisoindolin-2-yl)piperidine-2,6-dione;
3-(5-(4-benzhydryl-3,3-dimethylpiperazine-1-carbonyl)-4-fluoro-1-oxoisoindolin-2-yl)piperidine-2,6-dione;
3-(5-(4-benzhydryl-3,3-dimethylpiperazine-1-carbonyl)-6-fluoro-1-oxoisoindolin-2-yl)piperidine-2,6-dione;
3-(5-(4-benzhydryl-3,3-dimethylpiperazine-1-carbonyl)-7-fluoro-1-oxoisoindolin-2-yl)piperidine-2,6-dione;
3-(1-oxo-5-(4-(phenyl(pyridin-2-yl)methyl)piperazine-1-carbonyl)isoindolin-2-yl)piperidine-2,6-dione;
3-(4-fluoro-1-oxo-5-(4-(phenyl(pyridin-2-yl)methyl)piperazine-1-carbonyl)isoindolin-2-yl)piperidine-2,6-dione;
3-(6-fluoro-1-oxo-5-(4-(phenyl(pyridin-2-yl)methyl)piperazine-1-carbonyl)isoindolin-2-yl)piperidine-2,6-dione;
3-(7-fluoro-1-oxo-5-(4-(phenyl(pyridin-2-yl)methyl)piperazine-1-carbonyl)isoindolin-2-yl)piperidine-2,6-dione;
3-(1-oxo-4-(4-(phenyl(pyridin-2-yl)methyl)piperazine-1-carbonyl)isoindolin-2-yl)piperidine-2,6-dione;
3-(1-oxo-5-(4-(phenyl(pyridin-3-yl)methyl)piperazine-1-carbonyl)isoindolin-2-yl)piperidine-2,6-dione;
3-(1-oxo-5-(4-(phenyl(pyridin-4-yl)methyl)piperazine-1-carbonyl)isoindolin-2-yl)piperidine-2,6-dione;
3-(5-(4-(bis(4-fluorophenyl)methyl)piperazine-1-carbonyl)-1-oxoisoindolin-2-yl)piperidine-2,6-dione;
3-(5-(4-(bis(4-fluorophenyl)methyl)piperazine-1-carbonyl)-4-fluoro-1-oxoisoindolin-2-yl)piperidine-2,6-dione;
3-(5-(4-(bis(4-fluorophenyl)methyl)piperazine-1-carbonyl)-6-fluoro-1-oxoisoindolin-2-yl)piperidine-2,6-dione;
3-(5-(4-(bis(4-fluorophenyl)methyl)piperazine-1-carbonyl)-7-fluoro-1-oxoisoindolin-2-yl)piperidine-2,6-dione;
3-(4-(4-(bis(4-fluorophenyl)methyl)piperazine-1-carbonyl)-1-oxoisoindolin-2-yl)piperidine-2,6-dione;
3-(6-(4-(bis(4-fluorophenyl)methyl)piperazine-1-carbonyl)-1-oxoisoindolin-2-yl)piperidine-2,6-dione;
3-(7-(4-(bis(4-fluorophenyl)methyl)piperazine-1-carbonyl)-1-oxoisoindolin-2-yl)piperidine-2,6-dione;
3-(5-(4-(bis(3-fluorophenyl)methyl)piperazine-1-carbonyl)-1-oxoisoindolin-2-yl)piperidine-2,6-dione;
3-(5-(4-(bis(3-fluorophenyl)methyl)piperazine-1-carbonyl)-4-fluoro-1-oxoisoindolin-2-yl)piperidine-2,6-dione;
3-(5-(4-(bis(3-fluorophenyl)methyl)piperazine-1-carbonyl)-6-fluoro-1-oxoisoindolin-2-yl)piperidine-2,6-dione;
3-(5-(4-(bis(3-fluorophenyl)methyl)piperazine-1-carbonyl)-7-fluoro-1-oxoisoindolin-2-yl)piperidine-2,6-dione;
3-(4-(4-(bis(3-fluorophenyl)methyl)piperazine-1-carbonyl)-1-oxoisoindolin-2-yl)piperidine-2,6-dione;
3-(6-(4-(bis(3-fluorophenyl)methyl)piperazine-1-carbonyl)-1-oxoisoindolin-2-yl)piperidine-2,6-dione;

3-(7-(4-(bis(3-fluorophenyl)methyl)piperazine-1-carbonyl)-1-oxoisoindolin-2-yl)piperidine-2,6-dione;
3-(5-(4-(bis(2-fluorophenyl)methyl)piperazine-1-carbonyl)-1-oxoisoindolin-2-yl)piperidine-2,6-dione;
3-(5-(4-(bis(4-(trifluoromethyl)phenyl)methyl)piperazine-1-carbonyl)-1-oxoisoindolin-2-yl)piperidine-2,6-dione;
3-(5-(4-(bis(2,4-difluorophenyl)methyl)piperazine-1-carbonyl)-1-oxoisoindolin-2-yl)piperidine-2,6-dione;
3-(5-(4-((2,3-difluorophenyl)(3,4-difluorophenyl)methyl)piperazine-1-carbonyl)-1-oxoisoindolin-2-yl)piperidine-2,6-dione;
3-(5-(4-(bis(3,5-difluorophenyl)methyl)piperazine-1-carbonyl)-1-oxoisoindolin-2-yl)piperidine-2,6-dione;
3-(5-(4-(bis(perfluorophenyl)methyl)piperazine-1-carbonyl)-1-oxoisoindolin-2-yl)piperidine-2,6-dione;
3-(5-(4-(bis(4-chlorophenyl)methyl)piperazine-1-carbonyl)-1-oxoisoindolin-2-yl)piperidine-2,6-dione;
3-(5-(4-(bis(4-chlorophenyl)methyl)piperazine-1-carbonyl)-4-fluoro-1-oxoisoindolin-2-yl)piperidine-2,6-dione;
3-(5-(4-(bis(4-chlorophenyl)methyl)piperazine-1-carbonyl)-6-fluoro-1-oxoisoindolin-2-yl)piperidine-2,6-dione;
3-(5-(4-(bis(4-chlorophenyl)methyl)piperazine-1-carbonyl)-7-fluoro-1-oxoisoindolin-2-yl)piperidine-2,6-dione;
3-(4-(4-(bis(4-chlorophenyl)methyl)piperazine-1-carbonyl)-1-oxoisoindolin-2-yl)piperidine-2,6-dione;
3-(6-(4-(bis(4-chlorophenyl)methyl)piperazine-1-carbonyl)-1-oxoisoindolin-2-yl)piperidine-2,6-dione;
3-(7-(4-(bis(4-chlorophenyl)methyl)piperazine-1-carbonyl)-1-oxoisoindolin-2-yl)piperidine-2,6-dione;
3-(5-(4-(3,6-dichloro-9H-fluoren-9-yl)piperazine-1-carbonyl)-1-oxoisoindolin-2-yl)piperidine-2,6-dione;
3-(5-(4-(2,7-dichloro-9H-fluoren-9-yl)piperazine-1-carbonyl)-1-oxoisoindolin-2-yl)piperidine-2,6-dione;
3-(5-(4-(9H-fluoren-9-yl)piperazine-1-carbonyl)-1-oxoisoindolin-2-yl)piperidine-2,6-dione;
2-(1-(2-(2,6-dioxopiperidin-3-yl)-1-oxoisoindoline-5-carbonyl)piperidin-4-yl)-1H-benzo[de]isoquinoline-1,3(2H)-dione;
3-(5-(4-(bis(4-hydroxyphenyl)methyl)piperazine-1-carbonyl)-1-oxoisoindolin-2-yl)piperidine-2,6-dione;
3-(5-(4-(bis(4-hydroxyphenyl)methyl)piperazine-1-carbonyl)-4-fluoro-1-oxoisoindolin-2-yl)piperidine-2,6-dione;
3-(5-(4-(bis(4-hydroxyphenyl)methyl)piperazine-1-carbonyl)-6-fluoro-1-oxoisoindolin-2-yl)piperidine-2,6-dione;
3-(5-(4-(bis(4-hydroxyphenyl)methyl)piperazine-1-carbonyl)-7-fluoro-1-oxoisoindolin-2-yl)piperidine-2,6-dione;
3-(4-(4-(bis(4-hydroxyphenyl)methyl)piperazine-1-carbonyl)-1-oxoisoindolin-2-yl)piperidine-2,6-dione;
3-(6-(4-(bis(4-hydroxyphenyl)methyl)piperazine-1-carbonyl)-1-oxoisoindolin-2-yl)piperidine-2,6-dione;
3-(7-(4-(bis(4-hydroxyphenyl)methyl)piperazine-1-carbonyl)-1-oxoisoindolin-2-yl)piperidine-2,6-dione;
3-(5-(4-(bis(4-(benzyloxy)phenyl)methyl)piperazine-1-carbonyl)-1-oxoisoindolin-2-yl)piperidine-2,6-dione;
3-(5-(4-(bis(4-methoxyphenyl)methyl)piperazine-1-carbonyl)-1-oxoisoindolin-2-yl)piperidine-2,6-dione;
3-(5-(4-(bis(4-methoxyphenyl)methyl)piperazine-1-carbonyl)-4-fluoro-1-oxoisoindolin-2-yl)piperidine-2,6-dione;
3-(5-(4-(bis(4-methoxyphenyl)methyl)piperazine-1-carbonyl)-6-fluoro-1-oxoisoindolin-2-yl)piperidine-2,6-dione;
3-(5-(4-(bis(4-methoxyphenyl)methyl)piperazine-1-carbonyl)-7-fluoro-1-oxoisoindolin-2-yl)piperidine-2,6-dione;
3-(4-(4-(bis(4-methoxyphenyl)methyl)piperazine-1-carbonyl)-1-oxoisoindolin-2-yl)piperidine-2,6-dione;
3-(6-(4-(bis(4-methoxyphenyl)methyl)piperazine-1-carbonyl)-1-oxoisoindolin-2-yl)piperidine-2,6-dione;
3-(7-(4-(bis(4-methoxyphenyl)methyl)piperazine-1-carbonyl)-1-oxoisoindolin-2-yl)piperidine-2,6-dione;
3-(5-(4-(diphenylamino)piperidine-1-carbonyl)-1-oxoisoindolin-2-yl)piperidine-2,6-dione;
3-(5-(4-(diphenylamino)piperidine-1-carbonyl)-4-fluoro-1-oxoisoindolin-2-yl)piperidine-2,6-dione;
3-(5-(4-(diphenylamino)piperidine-1-carbonyl)-6-fluoro-1-oxoisoindolin-2-yl)piperidine-2,6-dione;
3-(5-(4-(diphenylamino)piperidine-1-carbonyl)-7-fluoro-1-oxoisoindolin-2-yl)piperidine-2,6-dione;
3-(1-oxo-5-(4-(phenyl(pyridin-2-yl)amino)piperidine-1-carbonyl)isoindolin-2-yl)piperidine-2,6-dione;
3-(7-fluoro-1-oxo-5-(4-(phenyl(pyridin-2-yl)amino)piperidine-1-carbonyl)isoindolin-2-yl)piperidine-2,6-dione;
5-((4-benzhydrylpiperazin-1-yl)methyl)-2-(2,6-dioxopiperidin-3-yl)isoindoline-1,3-dione;
5-((4-benzhydrylpiperazin-1-yl)methyl)-2-(2,6-dioxopiperidin-3-yl)-4-fluoroisoindoline-1,3-dione;
5-((4-benzhydrylpiperazin-1-yl)methyl)-2-(2,6-dioxopiperidin-3-yl)-6-fluoroisoindoline-1,3-dione;
6-((4-benzhydrylpiperazin-1-yl)methyl)-2-(2,6-dioxopiperidin-3-yl)-4-fluoroisoindoline-1,3-dione;
4-((4-benzhydrylpiperazin-1-yl)methyl)-2-(2,6-dioxopiperidin-3-yl)isoindoline-1,3-dione;
5-((3-benzhydrylimidazolidin-1-yl)methyl)-2-(2,6-dioxopiperidin-3-yl)isoindoline-1,3-dione;
5-((4-benzhydryl-1,4-diazepan-1-yl)methyl)-2-(2,6-dioxopiperidin-3-yl)isoindoline-1,3-dione;
5-((7-benzhydryl-2,7-diazaspiro[3.5]nonan-2-yl)methyl)-2-(2,6-dioxopiperidin-3-yl)isoindoline-1,3-dione;
5-((8-benzhydryl-2,8-diazaspiro[4.5]decan-2-yl)methyl)-2-(2,6-dioxopiperidin-3-yl)isoindoline-1,3-dione;
5-((9-benzhydryl-3,9-diazaspiro[5.5]undecan-3-yl)methyl)-2-(2,6-dioxopiperidin-3-yl)isoindoline-1,3-dione;
5-((5-benzhydrylhexahydropyrrolo[3,4-c]pyrrol-2(1H)-yl)methyl)-2-(2,6-dioxopiperidin-3-yl)isoindoline-1,3-dione;
5-((3-benzhydryl-3,6-diazabicyclo[3.1.1]heptan-6-yl)methyl)-2-(2,6-dioxopiperidin-3-yl)isoindoline-1,3-dione;
5-(((1R,4R)-5-benzhydryl-2,5-diazabicyclo[2.2.1]heptan-2-yl)methyl)-2-(2,6-dioxopiperidin-3-yl)isoindoline-1,3-dione;
5-(((1S,4S)-5-benzhydryl-2,5-diazabicyclo[2.2.1]heptan-2-yl)methyl)-2-(2,6-dioxopiperidin-3-yl)isoindoline-1,3-dione;
5-((3-benzhydryl-3,8-diazabicyclo[3.2.1]octan-8-yl)methyl)-2-(2,6-dioxopiperidin-3-yl)isoindoline-1,3-dione;
5-((8-benzhydryl-3,8-diazabicyclo[3.2.1]octan-3-yl)methyl)-2-(2,6-dioxopiperidin-3-yl)isoindoline-1,3-dione;
5-(((R)-4-benzhydryl-2-methylpiperazin-1-yl)methyl)-2-(2,6-dioxopiperidin-3-yl)isoindoline-1,3-dione;
5-((4-benzhydryl-2,6-dimethylpiperazin-1-yl)methyl)-2-(2,6-dioxopiperidin-3-yl)isoindoline-1,3-dione;
5-((4-benzhydryl-3,5-dimethylpiperazin-1-yl)methyl)-2-(2,6-dioxopiperidin-3-yl)isoindoline-1,3-dione;
5-((5-benzhydryl-2,5-diazabicyclo[2.2.2]octan-2-yl)methyl)-2-(2,6-dioxopiperidin-3-yl)isoindoline-1,3-dione;
5-((4-benzhydryl-2-(fluoromethyl)piperazin-1-yl)methyl)-2-(2,6-dioxopiperidin-3-yl)isoindoline-1,3-dione;
5-((4-benzhydryl-2-(trifluoromethyl)piperazin-1-yl)methyl)-2-(2,6-dioxopiperidin-3-yl)isoindoline-1,3-dione;
5-((4-benzhydryl-3,3-dimethylpiperazin-1-yl)methyl)-2-(2,6-dioxopiperidin-3-yl)isoindoline-1,3-dione;
5-((3-benzhydryl-2-oxoimidazolidin-1-yl)methyl)-2-(2,6-dioxopiperidin-3-yl)isoindoline-1,3-dione;
2-(2,6-dioxopiperidin-3-yl)-5-((4-(phenyl(pyridin-2-yl)methyl)piperazin-1-yl)methyl)isoindoline-1,3-dione;
2-(2,6-dioxopiperidin-3-yl)-5-((4-(phenyl(pyridin-3-yl)methyl)piperazin-1-yl)methyl)isoindoline-1,3-dione;
2-(2,6-dioxopiperidin-3-yl)-5-((4-(phenyl(pyridin-4-yl)methyl)piperazin-1-yl)methyl)isoindoline-1,3-dione;
5-((4-(di(pyridin-2-yl)methyl)piperazin-1-yl)methyl)-2-(2,6-dioxopiperidin-3-yl)isoindoline-1,3-dione;
5-((4-(di(pyridin-3-yl)methyl)piperazin-1-yl)methyl)-2-(2,6-dioxopiperidin-3-yl)isoindoline-1,3-dione;
5-((4-((4-bromophenyl)(phenyl)methyl)piperazin-1-yl)methyl)-2-(2,6-dioxopiperidin-3-yl)isoindoline-1,3-dione;
5-((4-(bis(4-fluorophenyl)methyl)piperazin-1-yl)methyl)-2-(2,6-dioxopiperidin-3-yl)isoindoline-1,3-dione;
5-((4-(bis(3-fluorophenyl)methyl)piperazin-1-yl)methyl)-2-(2,6-dioxopiperidin-3-yl)isoindoline-1,3-dione;
5-((4-(bis(2-fluorophenyl)methyl)piperazin-1-yl)methyl)-2-(2,6-dioxopiperidin-3-yl)isoindoline-1,3-dione;
5-((4-(bis(4-(trifluoromethyl)phenyl)methyl)piperazin-1-yl)methyl)-2-(2,6-dioxopiperidin-3-yl)isoindoline-1,3-dione;
5-((4-(bis(2,4-difluorophenyl)methyl)piperazin-1-yl)methyl)-2-(2,6-dioxopiperidin-3-yl)isoindoline-1,3-dione;
5-((4-(bis(3,5-difluorophenyl)methyl)piperazin-1-yl)methyl)-2-(2,6-dioxopiperidin-3-yl)isoindoline-1,3-dione;
5-((4-(bis(perfluorophenyl)methyl)piperazin-1-yl)methyl)-2-(2,6-dioxopiperidin-3-yl)isoindoline-1,3-dione;
5-((4-(bis(4-chlorophenyl)methyl)piperazin-1-yl)methyl)-2-(2,6-dioxopiperidin-3-yl)isoindoline-1,3-dione;
5-((4-(3,6-dichloro-9H-fluoren-9-yl)piperazin-1-yl)methyl)-2-(2,6-dioxopiperidin-3-yl)isoindoline-1,3-dione;
5-((4-(2,7-dichloro-9H-fluoren-9-yl)piperazin-1-yl)methyl)-2-(2,6-dioxopiperidin-3-yl)isoindoline-1,3-dione;
5-((4-(9H-fluoren-9-yl)piperazin-1-yl)methyl)-2-(2,6-dioxopiperidin-3-yl)isoindoline-1,3-dione;
2-((2-(2,6-dioxopiperidin-3-yl)-1,3-dioxoisoindolin-5-yl)methyl)-1H-benzo[de]isoquinoline-1,3(2H)-dione;
2-(1-((2-(2,6-dioxopiperidin-3-yl)-1,3-dioxoisoindolin-5-yl)methyl)piperidin-4-yl)-1H-benzo[de]isoquinoline-1,3(2H)-dione;
5-((4-(bis(4-hydroxyphenyl)methyl)piperazin-1-yl)methyl)-2-(2,6-dioxopiperidin-3-yl)isoindoline-1,3-dione;
5-((4-(bis(4-(benzyloxy)phenyl)methyl)piperazin-1-yl)methyl)-2-(2,6-dioxopiperidin-3-yl)isoindoline-1,3-dione;
5-((4-(bis(4-methoxyphenyl)methyl)piperazin-1-yl)methyl)-2-(2,6-dioxopiperidin-3-yl)isoindoline-1,3-dione;
2-(2,6-dioxopiperidin-3-yl)-5-((4-(diphenylamino)piperidin-1-yl)methyl)isoindoline-1,3-dione;
2-(2,6-dioxopiperidin-3-yl)-5-((4-(phenyl(pyridin-2-yl)amino)piperidin-1-yl)methyl)isoindoline-1,3-dione;
5-(4-benzhydrylpiperazine-1-carbonyl)-2-(2,6-dioxopiperidin-3-yl)isoindoline-1,3-dione;
5-(4-benzhydrylpiperazine-1-carbonyl)-2-(2,6-dioxopiperidin-3-yl)-4-fluoroisoindoline-1,3-dione;
5-(4-benzhydrylpiperazine-1-carbonyl)-2-(2,6-dioxopiperidin-3-yl)-6-fluoroisoindoline-1,3-dione;
6-(4-benzhydrylpiperazine-1-carbonyl)-2-(2,6-dioxopiperidin-3-yl)-4-fluoroisoindoline-1,3-dione;
4-(4-benzhydrylpiperazine-1-carbonyl)-2-(2,6-dioxopiperidin-3-yl)isoindoline-1,3-dione;
5-(3-benzhydrylimidazolidine-1-carbonyl)-2-(2,6-dioxopiperidin-3-yl)isoindoline-1,3-dione;
5-(4-benzhydryl-1,4-diazepane-1-carbonyl)-2-(2,6-dioxopiperidin-3-yl)isoindoline-1,3-dione;
5-(7-benzhydryl-2,7-diazaspiro[3.5]nonane-2-carbonyl)-2-(2,6-dioxopiperidin-3-yl)isoindoline-1,3-dione;
5-(8-benzhydryl-2,8-diazaspiro[4.5]decane-2-carbonyl)-2-(2,6-dioxopiperidin-3-yl)isoindoline-1,3-dione;
5-(9-benzhydryl-3,9-diazaspiro[5.5]undecane-3-carbonyl)-2-(2,6-dioxopiperidin-3-yl)isoindoline-1,3-dione;
5-(5-benzhydryloctahydropyrrolo[3,4-c]pyrrole-2-carbonyl)-2-(2,6-dioxopiperidin-3-yl)isoindoline-1,3-dione;
5-(3-benzhydryl-3,6-diazabicyclo[3.1.1]heptane-6-carbonyl)-2-(2,6-dioxopiperidin-3-yl)isoindoline-1,3-dione;
5-((1R,4R)-5-benzhydryl-2,5-diazabicyclo[2.2.1]heptane-2-carbonyl)-2-(2,6-dioxopiperidin-3-yl)isoindoline-1,3-dione;
5-((1S,4S)-5-benzhydryl-2,5-diazabicyclo[2.2.1]heptane-2-carbonyl)-2-(2,6-dioxopiperidin-3-yl)isoindoline-1,3-dione;

5-(3-benzhydryl-3,8-diazabicyclo[3.2.1]octane-8-carbonyl)-2-(2,6-dioxopiperidin-3-yl)isoindoline-1,3-dione;
5-(8-benzhydryl-3,8-diazabicyclo[3.2.1]octane-3-carbonyl)-2-(2,6-dioxopiperidin-3-yl)isoindoline-1,3-dione;
5-((R)-4-benzhydryl-2-methylpiperazine-1-carbonyl)-2-(2,6-dioxopiperidin-3-yl)isoindoline-1,3-dione;
5-(4-benzhydryl-2,6-dimethylpiperazine-1-carbonyl)-2-(2,6-dioxopiperidin-3-yl)isoindoline-1,3-dione;
5-(4-benzhydryl-3,5-dimethylpiperazine-1-carbonyl)-2-(2,6-dioxopiperidin-3-yl)isoindoline-1,3-dione;
5-(5-benzhydryl-2,5-diazabicyclo[2.2.2]octane-2-carbonyl)-2-(2,6-dioxopiperidin-3-yl)isoindoline-1,3-dione;
5-(4-benzhydryl-2-(fluoromethyl)piperazine-1-carbonyl)-2-(2,6-dioxopiperidin-3-yl)isoindoline-1,3-dione;
5-(4-benzhydryl-2-(trifluoromethyl)piperazine-1-carbonyl)-2-(2,6-dioxopiperidin-3-yl)isoindoline-1,3-dione;
5-(4-benzhydryl-3,3-dimethylpiperazine-1-carbonyl)-2-(2,6-dioxopiperidin-3-yl)isoindoline-1,3-dione;
2-(2,6-dioxopiperidin-3-yl)-5-(4-(phenyl(pyridin-2-yl)methyl)piperazine-1-carbonyl)isoindoline-1,3-dione;
2-(2,6-dioxopiperidin-3-yl)-5-(4-(phenyl(pyridin-3-yl)methyl)piperazine-1-carbonyl)isoindoline-1,3-dione;
2-(2,6-dioxopiperidin-3-yl)-5-(4-(phenyl(pyridin-4-yl)methyl)piperazine-1-carbonyl)isoindoline-1,3-dione;
5-(4-(di(pyridin-2-yl)methyl)piperazine-1-carbonyl)-2-(2,6-dioxopiperidin-3-yl)isoindoline-1,3-dione;
5-(4-(di(pyridin-3-yl)methyl)piperazine-1-carbonyl)-2-(2,6-dioxopiperidin-3-yl)isoindoline-1,3-dione;
5-(4-(bis(4-fluorophenyl)methyl)piperazine-1-carbonyl)-2-(2,6-dioxopiperidin-3-yl)isoindoline-1,3-dione;
5-(4-(bis(3-fluorophenyl)methyl)piperazine-1-carbonyl)-2-(2,6-dioxopiperidin-3-yl)isoindoline-1,3-dione;
5-(4-(bis(2-fluorophenyl)methyl)piperazine-1-carbonyl)-2-(2,6-dioxopiperidin-3-yl)isoindoline-1,3-dione;
5-(4-(bis(4-(trifluoromethyl)phenyl)methyl)piperazine-1-carbonyl)-2-(2,6-dioxopiperidin-3-yl)isoindoline-1,3-dione;
5-(4-(bis(2,4-difluorophenyl)methyl)piperazine-1-carbonyl)-2-(2,6-dioxopiperidin-3-yl)isoindoline-1,3-dione;
5-(4-(bis(3,5-difluorophenyl)methyl)piperazine-1-carbonyl)-2-(2,6-dioxopiperidin-3-yl)isoindoline-1,3-dione;
5-(4-(bis(perfluorophenyl)methyl)piperazine-1-carbonyl)-2-(2,6-dioxopiperidin-3-yl)isoindoline-1,3-dione;
5-(4-(bis(4-chlorophenyl)methyl)piperazine-1-carbonyl)-2-(2,6-dioxopiperidin-3-yl)isoindoline-1,3-dione;
5-(4-(3,6-dichloro-9H-fluoren-9-yl)piperazine-1-carbonyl)-2-(2,6-dioxopiperidin-3-yl)isoindoline-1,3-dione;
5-(4-(2,7-dichloro-9H-fluoren-9-yl)piperazine-1-carbonyl)-2-(2,6-dioxopiperidin-3-yl)isoindoline-1,3-dione;
5-(4-(9H-fluoren-9-yl)piperazine-1-carbonyl)-2-(2,6-dioxopiperidin-3-yl)isoindoline-1,3-dione;
2-(1-(2-(2,6-dioxopiperidin-3-yl)-1,3-dioxoisoindoline-5-carbonyl)piperidin-4-yl)-1H-benzo[de]isoquinoline-1,3(2H)-dione;
5-(4-(bis(4-hydroxyphenyl)methyl)piperazine-1-carbonyl)-2-(2,6-dioxopiperidin-3-yl)isoindoline-1,3-dione;
5-(4-(bis(4-(benzyloxy)phenyl)methyl)piperazine-1-carbonyl)-2-(2,6-dioxopiperidin-3-yl)isoindoline-1,3-dione;
5-(4-(bis(4-methoxyphenyl)methyl)piperazine-1-carbonyl)-2-(2,6-dioxopiperidin-3-yl)isoindoline-1,3-dione;
2-(2,6-dioxopiperidin-3-yl)-5-(4-(diphenylamino)piperidine-1-carbonyl)isoindoline-1,3-dione;
2-(2,6-dioxopiperidin-3-yl)-5-(4-(phenyl(pyridin-2-yl)amino)piperidine-1-carbonyl)isoindoline-1,3-dione;
5-(4-benzhydrylpiperazin-1-yl)-2-(2,6-dioxopiperidin-3-yl)isoindoline-1,3-dione;
5-(4-benzhydrylpiperazin-1-yl)-2-(2,6-dioxopiperidin-3-yl)-6-fluoroisoindoline-1,3-dione;
5-(3-benzhydryl-3,8-diazabicyclo[3.2.1]octan-8-yl)-2-(2,6-dioxopiperidin-3-yl)isoindoline-1,3-dione;
5-(8-benzhydryl-3,8-diazabicyclo[3.2.1]octan-3-yl)-2-(2,6-dioxopiperidin-3-yl)isoindoline-1,3-dione;
5-(3-benzhydryl-3,6-diazabicyclo[3.1.1]heptan-6-yl)-2-(2,6-dioxopiperidin-3-yl)isoindoline-1,3-dione;
5-(6-benzhydryl-3,6-diazabicyclo[3.1.1]heptan-3-yl)-2-(2,6-dioxopiperidin-3-yl)isoindoline-1,3-dione;
5-(4-benzhydryl-3,5-dimethylpiperazin-1-yl)-2-(2,6-dioxopiperidin-3-yl)isoindoline-1,3-dione;
5-(5-benzhydryl-2,5-diazabicyclo[2.2.2]octan-2-yl)-2-(2,6-dioxopiperidin-3-yl)isoindoline-1,3-dione;
5-(3-benzhydryl-3,8-diazabicyclo[3.2.1]octan-8-yl)-2-(2,6-dioxopiperidin-3-yl)-6-fluoroisoindoline-1,3-dione;
5-(3-benzhydryl-3,6-diazabicyclo[3.1.1]heptan-6-yl)-2-(2,6-dioxopiperidin-3-yl)-6-fluoroisoindoline-1,3-dione;
5-((1-benzhydryl-5-chloropiperidin-3-yl)amino)-2-(2,6-dioxopiperidin-3-yl)-6-fluoroisoindoline-1,3-dione;
5-(6-benzhydryl-3,6-diazabicyclo[3.1.1]heptan-3-yl)-2-(2,6-dioxopiperidin-3-yl)-6-fluoroisoindoline-1,3-dione;
5-(8-benzhydryl-3,8-diazabicyclo[3.2.1]octan-3-yl)-2-(2,6-dioxopiperidin-3-yl)-6-fluoroisoindoline-1,3-dione;
5-(4-benzhydryl-3,5-dimethylpiperazin-1-yl)-2-(2,6-dioxopiperidin-3-yl)-6-fluoroisoindoline-1,3-dione;
5-(5-benzhydryl-2,5-diazabicyclo[2.2.2]octan-2-yl)-2-(2,6-dioxopiperidin-3-yl)-6-fluoroisoindoline-1,3-dione;
and
5-(4-benzhydrylpiperazin-1-yl-2,2,3,3,5,5,6,6-ds)-2-(2,6-dioxopiperidin-3-yl)-6-fluoroisoindoline-1,3-dione.

**10.** The compound of Formula (I) or salts, enantiomers, stereoisomers, solvates, or polymorphs thereof as claimed in any one of claims 1-9, which is hydrochlorides, sulfates, citrates, maleates, sulfonates, citrates, lactates, tartrates, fumarates, phosphates, dihydrophosphates, pyrophosphates, metaphosphates, oxalates, malonates, benzoates, mandelates, succinates, trifluoroacetates, hydroxyacetates, or *p*-toluenesulfonates of the compound of Formula (I).

**11.** A pharmaceutical composition comprising the compound of Formula (I) or pharmaceutically acceptable salts thereof as claimed in any one of claims 1-9, and at least one pharmaceutically acceptable carrier or excipient.

**12.** The pharmaceutical composition as claimed in claim 11, further comprising a second therapeutic agent, e.g., an anticancer agent.

**13.** The compound of Formula (I) or pharmaceutically acceptable salts thereof as claimed in any one of claims 1-9, for use in the prevention and/or treatment of diseases or disorders associated with cereblon protein.

**14.** The compound of Formula (I) or pharmaceutically acceptable salts thereof as claimed in claim 13, wherein the diseases or disorders associated with cereblon protein are selected from the group consisting of: tumor, infectious disease, inflammatory disease, autoimmune disease, anemia, hemorrhagic shock, transplant rejection, multiple organ dysfunction syndrome (MODS), sarcoidosis, adult respiratory distress syndrome, cardiovascular disease, Richter syndrome (RS), acute liver failure, or diabetes.

**15.** The compound of Formula (I) or pharmaceutically acceptable salts thereof as claimed in claim 13, wherein the diseases or disorders associated with cereblon protein are selected from the group consisting of: myeloma, including multiple myeloma, plasma cell myeloma, smoldering myeloma, smoldering multiple myeloma; myelofibrosis; bone marrow disease; myelodysplastic syndrome (MDS); previously treated myelodysplastic syndrome; transplantation-related cancer; neutropenia; leukemia, including acute myeloid leukemia, chronic myelogenous leukemia, B-cell chronic lymphocytic leukemia, leukemia-associated anemia, acute myeloid leukemia (AML), acute lymphoblastic leukemia (ALL), acute B-cell lymphoblastic leukemia, acute T-cell lymphoblastic leukemia, chronic lymphocytic leukemia, lymphoma cell leukemia, T-cell lymphoblastic leukemia, monocytic leukemia, myelomonocytic leukemia; lymphoma, including diffuse large B-cell lymphoma, non-Hodgkin's lymphoma, Hodgkin's lymphoma, anaplastic lymphoma, anaplastic large cell lymphoma, CD20 positive lymphoma, mantle cell lymphoma, follicular lymphoma (FL), Burkitt lymphoma, primary lymphoma, B-cell lymphoma, recurrent B-cell non-Hodgkin's lymphoma, recurrent diffuse large B-cell lymphoma, recurrent mediastinal (thymic) large B-cell lymphoma, primary mediastinal (thymic) large B-cell lymphoma, recurrent transformed non-Hodgkin's lymphoma, refractory B-cell non-Hodgkin's lymphoma, refractory diffuse large B-cell lymphoma, refractory primary mediastinal (thymic) large B-cell lymphoma, and refractory transformed non-Hodgkin's lymphoma; thyroid cancer; melanoma; lung cancer, including lung adenocarcinoma, lung squamous cell carcinoma, non-small cell lung cancer, and small cell lung cancer; inflammatory myofibroblastoma; colorectal cancer; intestinal cancer; brain glioma; astroblastoma; ovarian cancer; bronchial cancer; prostate cancer; breast cancer, including triple negative breast cancer, sporadic breast cancer, and patients with Cowden syndrome; pancreatic cancer; central nervous system tumor; neuroblastoma; neuroglioma; peripheral neuroepithelioma; extramedullary plasmacytoma; plasmacytoma; gastric cancer; gastrointestinal stromal tumors; esophageal cancer; colorectal adenocarcinoma; esophageal squamous cell carcinoma; liver cancer; renal cell carcinoma; bladder cancer; endometrial cancer; metrocarcinoma; head and neck cancer; brain cancer; oral cancer; sarcoma, including rhabdomyosarcoma, various lipogenic tumors, Ewing's sarcoma/primitive neuroectodermal tumors (Ewing/PNETs), and leiomyosarcoma; urothelial carcinoma; basal cell carcinoma; oral squamous cell carcinoma; cholangiocarcinoma; bone cancer; cervical cancer; skin cancer; Richter syndrome (RS); sepsis syndrome; autoimmune diseases, including rheumatoid arthritis, autoimmune encephalomyelitis, ankylosing spondylitis, psoriasis, systemic lupus erythematosus, multiple sclerosis, recurrent oral ulcers, Kawasaki disease, polymyositis/dermatomyositis, Sjogren's syndrome, and atopic dermatitis; keratoconjunctivitis; arthritis; osteoarthritis; inflammatory diseases, including Crohn's disease and ulcerative colitis, pneumonia, synovitis, systemic inflammatory response syndrome, airway inflammation, and bronchitis; cerebral malaria; infectious diseases, including viral pneumonia, Acquired immunodeficiency syndrome (AIDS), COVID-19 novel coronavirus infection, gram-negative bacteria infection, gram-positive bacteria infection, tuberculosis, etc.; septic shock; tuberculosis; bacterial meningitis; chronic obstructive pulmonary disease; asthma; hemorrhagic shock; organ (including kidney, heart, lung) or tissue transplantation rejection; diabetes; sarcoidosis; adult respiratory distress syndrome; anemia; pediatric aplastic anemia; cardiovascular diseases (e.g., coronary heart disease, congestive heart failure, myocardial infarction, atherosclerosis); multiple organ dysfunction caused by cachexia and septic shock; or acute liver failure.

**16.** Use of the compound of Formula (I) or pharmaceutically acceptable salts thereof as claimed in any one of claims 1-9, or the pharmaceutical composition as claimed in claim 11 or 12 for the manufacture of a medicament for the prevention and/or treatment of diseases or disorders associated with cereblon protein.

**17.** A method for treating or preventing diseases or disorders associated with cereblon protein, comprising administering to a subject a therapeutically effective amount of the compound of Formula (I) or pharmaceutically acceptable salts thereof as claimed in any one of claims 1-9, or the pharmaceutical composition as claimed in claim 11 or 12.

**18.** The use of claim 16 or the method of claim 17, wherein the diseases or disorders associated with cereblon protein are

selected from the group consisting of: tumor, infectious disease, inflammatory disease, autoimmune disease, anemia, hemorrhagic shock, transplant rejection, multiple organ dysfunction syndrome (MODS), sarcoidosis, adult respiratory distress syndrome, cardiovascular disease, Richter syndrome (RS), acute liver failure, or diabetes.

**19.** The use of claim 16 or the method of claim 17, wherein the diseases or disorders associated with cereblon protein are selected from the group consisting of: myeloma, including multiple myeloma, plasma cell myeloma, smoldering myeloma, smoldering multiple myeloma; myelofibrosis; bone marrow disease; myelodysplastic syndrome (MDS); previously treated myelodysplastic syndrome; transplantation-related cancer; neutropenia; leukemia, including acute myeloid leukemia, chronic myelogenous leukemia, B-cell chronic lymphocytic leukemia, leukemia-associated anemia, acute myeloid leukemia (AML), acute B-cell lymphoblastic leukemia, acute T-cell lymphoblastic leukemia, chronic lymphocytic leukemia, lymphoma cell leukemia, T-cell lymphoblastic leukemia, monocytic leukemia, myelomonocytic leukemia; lymphoma, including diffuse large B-cell lymphoma, non-Hodgkin's lymphoma, Hodgkin's lymphoma, anaplastic lymphoma, anaplastic large cell lymphoma, CD20 positive lymphoma, mantle cell lymphoma, follicular lymphoma (FL), primary lymphoma, B-cell lymphoma, recurrent B-cell non-Hodgkin's lymphoma, recurrent diffuse large B-cell lymphoma, recurrent mediastinal (thymic) large B-cell lymphoma, primary mediastinal (thymic) large B-cell lymphoma, recurrent transformed non-Hodgkin's lymphoma, refractory B-cell non-Hodgkin's lymphoma, refractory diffuse large B-cell lymphoma, refractory primary mediastinal (thymic) large B-cell lymphoma, and refractory transformed non-Hodgkin's lymphoma; thyroid cancer; melanoma; lung cancer, including lung adenocarcinoma, lung squamous cell carcinoma, non-small cell lung cancer, and small cell lung cancer; inflammatory myofibroblastoma; colorectal cancer; intestinal cancer; brain glioma; astroblastoma; ovarian cancer; bronchial cancer; prostate cancer; breast cancer, including triple negative breast cancer, sporadic breast cancer, and patients with Cowden syndrome; pancreatic cancer; central nervous system tumor; neuroblastoma; neuroglioma; peripheral neuroepithelioma; extramedullary plasmacytoma; plasmacytoma; gastric cancer; gastrointestinal stromal tumors; esophageal cancer; colorectal adenocarcinoma; esophageal squamous cell carcinoma; liver cancer; renal cell carcinoma; bladder cancer; endometrial cancer; metrocarcinoma; head and neck cancer; brain cancer; oral cancer; sarcoma, including rhabdomyosarcoma, various lipogenic tumors, Ewing's sarcoma/primitive neuroectodermal tumors (Ewing/PNETs), and leiomyosarcoma; urothelial carcinoma; basal cell carcinoma; oral squamous cell carcinoma; cholangiocarcinoma; bone cancer; cervical cancer; skin cancer; Richter syndrome (RS); sepsis syndrome; autoimmune diseases, including rheumatoid arthritis, autoimmune encephalomyelitis, ankylosing spondylitis, psoriasis, systemic lupus erythematosus, multiple sclerosis, recurrent oral ulcers, Kawasaki disease, polymyositis/dermatomyositis, Sjogren's syndrome, and atopic dermatitis; keratoconjunctivitis; inflammatory diseases, including Crohn's disease and ulcerative colitis, pneumonia, osteoarthritis, synovitis, systemic inflammatory response syndrome, airway inflammation, and bronchitis; cerebral malaria; infectious diseases, including viral pneumonia, Acquired immunodeficiency syndrome (AIDS), COVID-19 novel coronavirus infection, gram-negative bacteria infection, gram-positive bacteria infection, tuberculosis, etc.; septic shock; tuberculosis; bacterial meningitis; chronic obstructive pulmonary disease; asthma; hemorrhagic shock; organ (including kidney, heart, lung) or tissue transplantation rejection; diabetes; sarcoidosis; adult respiratory distress syndrome; anemia; pediatric aplastic anemia; cardiovascular diseases (e.g., coronary heart disease, congestive heart failure, myocardial infarction, atherosclerosis); multiple organ dysfunction caused by cachexia and septic shock; or acute liver failure.

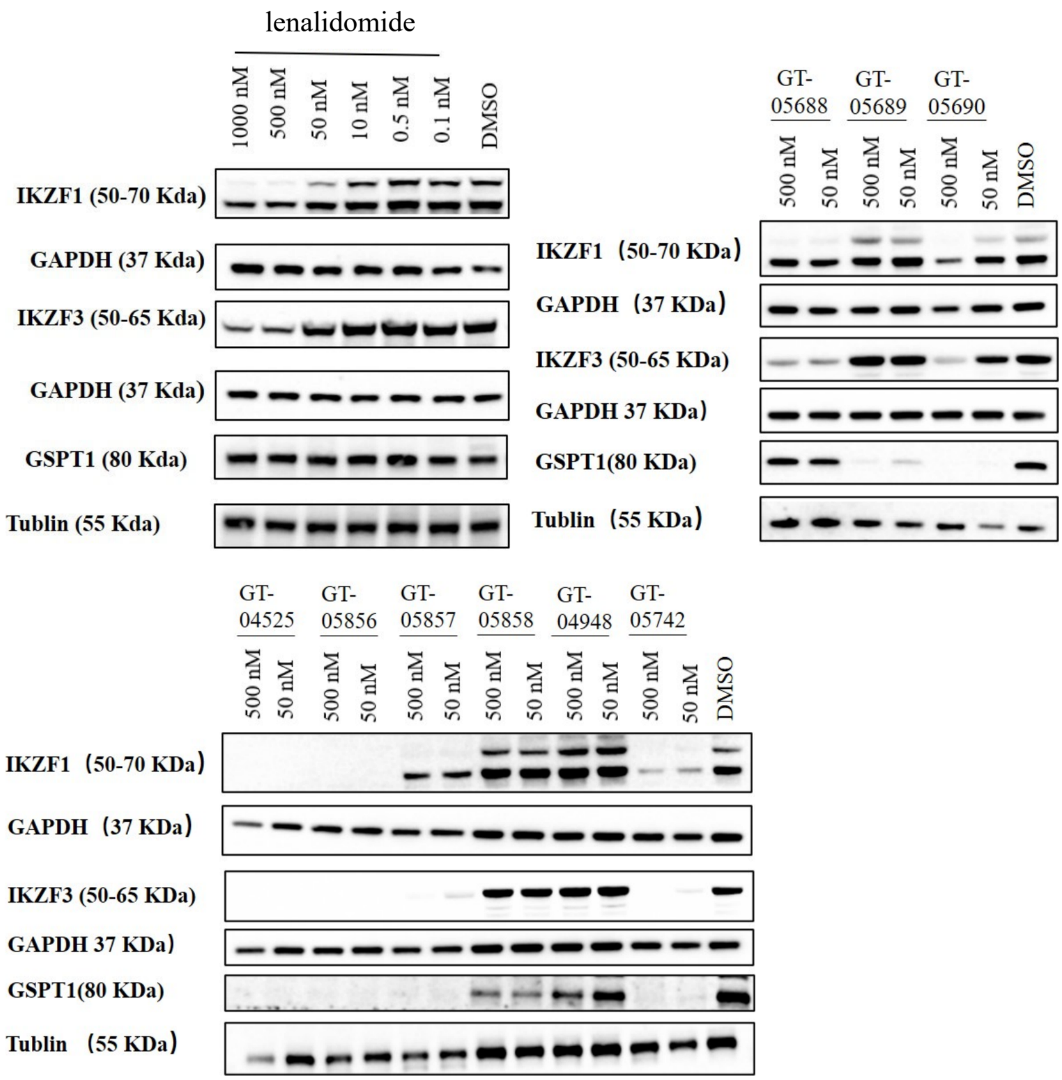
lenalidomide
1000 nM
500 nM
50 nM
10 nM
0.5 nM
0.1 nM
DMSO
IKZF1 (50-70 Kda)
GAPDH (37 Kda)
IKZF3 (50-65 Kda)
GAPDH (37 Kda)
GSPT1 (80 Kda)
Tublin (55 Kda)
GT-05688
GT-05689
GT-05690
500 nM
50 nM
DMSO
IKZF1（50-70 KDa）
GAPDH（37 KDa）
IKZF3 (50-65 KDa)
GAPDH 37 KDa)
GSPT1(80 KDa)
Tublin（55 KDa）
GT-04525
GT-05856
GT-05857
GT-05858
GT-04948
GT-05742
500 nM
50 nM
DMSO
IKZF1（50-70 KDa）
GAPDH（37 KDa）
IKZF3 (50-65 KDa)
GAPDH 37 KDa)
GSPT1(80 KDa)
Tublin（55 KDa）

Figure 1

**INTERNATIONAL SEARCH REPORT**

International application No.
**PCT/CN2023/141786**

**A. CLASSIFICATION OF SUBJECT MATTER**

C07D 401/14(2006.01)i; C07D401/ 04(2006.01)i; C07D 413/14(2006.01)i; C07D 491/107(2006.01)i; C07D 487/10(2006.01)i; C07D 498/10(2006.01)i; C07D 419/14(2006.01)i; C07D 417/14(2006.01)i; C07D 409/14(2006.01)i; C07D 405/14(2006.01)i; A61K 31/45(2006.01)i; A61K 31/454(2006.01)i; A61K 31/4545(2006.01)i; A61K 31/497(2006.01)i; A61K 31/538(2006.01)i; A61P 35/00(2006.01)i; A61P 37/00(2006.01)i; A61P 25/00(2006.01)i; A61P 29/00(2006.01)i

According to International Patent Classification (IPC) or to both national classification and IPC

**B. FIELDS SEARCHED**

Minimum documentation searched (classification system followed by classification symbols)

IPC:C07D,A61K,A61P

Documentation searched other than minimum documentation to the extent that such documents are included in the fields searched

Electronic data base consulted during the international search (name of data base and, where practicable, search terms used)

CNTXT, WPABS, WPABSC, CNABS, CNKI, REGISTRY(STN), CAPLUS(STN): 标新生物医药科技(上海)有限公司, 异吲哚啉, 邻苯二甲酰胺, 酮, 哌啶, 二氢, 异吲哚, 酰胺, 羟脑苷酯, 小脑蛋白, CRBN, cereblon, isoindoline, isoindol, phthalimide, dioxo, oxo, one, dione, piperidin, dihydrogen, amide, ligase?, 结构式检索, structural formula search

**C. DOCUMENTS CONSIDERED TO BE RELEVANT**

| Category* | Citation of document, with indication, where appropriate, of the relevant passages | Relevant to claim No. |
|---|---|---|
| X | WO 2022257897 A1 (HANGZHOU GLUBIO PHARMACEUTICAL CO., LTD.) 15 December 2022 (2022-12-15)<br>claims 1-42, and description, page 2, paragraph 2, pages 46-54 and 62-65, specific compounds, pages 133-134, table 7, and page 136, table 8 | 1-4, 6-8, 10-19 |
| A | CN 111902141 A (C4 THERAPEUTICS INC.) 06 November 2020 (2020-11-06)<br>claims 1-51, and description, page 13, paragraph [0106], and page 162, compound 203 | 1-19 |
| A | CN 110963994 A (SHANGHAI INSTITUTE OF MATERIA MEDICA, CHINESE ACADEMY OF SCIENCES) 07 April 2020 (2020-04-07)<br>claims 1-66, and description, pages 74-104, table 1, compounds | 1-19 |
| A | WO 2022235715 A1 (NURIX THERAPEUTICS, INC.) 10 November 2022 (2022-11-10)<br>claims 1-66, and description, pages 115-146, table 1, compounds | 1-19 |

☐ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **19 March 2024** | **29 March 2024** |
| Name and mailing address of the ISA/CN<br>**China National Intellectual Property Administration (ISA/CN)**<br>**China No. 6, Xitucheng Road, Jimenqiao, Haidian District, Beijing 100088** | Authorized officer<br><br>Telephone No. |

Form PCT/ISA/210 (second sheet) (July 2022)

**INTERNATIONAL SEARCH REPORT**

International application No.
**PCT/CN2023/141786**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **17-19**
because they relate to subject matter not required to be searched by this Authority, namely:

Claims 17-19 relate to a method for preventing or treating diseases. The present search report is provided on the basis of the use of a compound or a pharmaceutically acceptable salt thereof in the preparation of a drug for preventing or treating diseases.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (July 2022)

**INTERNATIONAL SEARCH REPORT**
**Information on patent family members**

International application No.
**PCT/CN2023/141786**

| Patent document cited in search report | Publication date (day/month/year) | Patent family member(s) | Publication date (day/month/year) |
|---|---|---|---|
| WO 2022257897 A1 | 15 December 2022 | AU 2022289605 A1 | 04 January 2024 |
| | | CA 3221549 A1 | 15 December 2022 |
| | | KR 20240019272 A | 14 February 2024 |
| | | CN 117561244 A | 13 February 2024 |
| CN 111902141 A | 06 November 2020 | EP 3773576 A1 | 17 February 2021 |
| | | EP 3773576 A4 | 29 December 2021 |
| | | US 2021009559 A1 | 14 January 2021 |
| | | US 11753397 B2 | 12 September 2023 |
| | | WO 2019191112 A1 | 03 October 2019 |
| | | JP 2021519337 A | 10 August 2021 |
| | | KR 20210018199 A | 17 February 2021 |
| CN 110963994 A | 07 April 2020 | BR 112021006458 A2 | 06 July 2021 |
| | | KR 20210069085 A | 10 June 2021 |
| | | CA 3121667 A1 | 02 April 2020 |
| | | AU 2019348094 A1 | 27 May 2021 |
| | | AU 2019348094 B2 | 22 December 2022 |
| | | WO 2020064002 A1 | 02 April 2020 |
| | | EP 3862348 A1 | 11 August 2021 |
| | | EP 3862348 A4 | 22 June 2022 |
| | | US 2022041576 A1 | 10 February 2022 |
| | | JP 2022503942 A | 12 January 2022 |
| | | JP 7168773 B2 | 09 November 2022 |
| WO 2022235715 A1 | 10 November 2022 | AU 2022269594 A1 | 16 November 2023 |
| | | CA 3217542 A1 | 10 November 2022 |
| | | CN 117693502 A | 12 March 2024 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- Clinical Pharmacology. People's Public Health Press, 2008
- *CHEMICAL ABSTRACTS*, 768-94-5